# EUROPEAN PATENT APPLICATION

(11) **EP 4 800 012 A1**
(43) Date of publication of application: **02.09.2026**
(21) Application number: 24881743.9
(22) Date of filing: 25.10.2024
(51) Int. Cl.: C07D 403/10, C07D 403/12, C07D 403/14, C07D 401/10, C07D 401/12, C07D 401/14, C07D 413/14, C07D 417/10, C07D 417/12, C07D 417/14, A61K 31/501, A61K 31/506, A61P 35/00, A61P 37/00, A61P 29/00, A61P 31/12

(54) **COMPOUND ACTING AS PARP7 INHIBITOR**

(30) Priority: 26.10.2023 CN 202311406408; 06.06.2024 CN 202410735258
(71) Applicant: Dovetree Medicines Unus, Inc., Prides Crossing, MA 01965 (US)
(72) Inventor: GAO, Fang, Shenzhen, Guangdong 518017 (CN); FANG, Xiaoniu, Shenzhen, Guangdong 518017 (CN); NIU, Chunyi, Shenzhen, Guangdong 518017 (CN); HUA, Zihao, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN)
(74) Representative: Latscha Schöllhorn Partner AG
(86) International application number: PCT/CN2024/127356
(87) International publication number: WO 2025/087383

(57) **Abstract**

The present invention relates to a biaromatic compound acting as a PARP7 inhibitor. Specifically, the present invention relates to a compound of formula (I), or an enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof. The present invention also relates to a use of the compound for treating or preventing PARP7-mediated diseases, such as cancer, immune diseases, inflammation or viral infections.

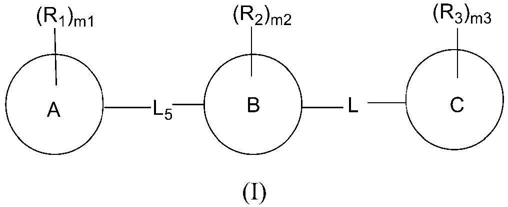

## Description

### Reference to Related Applications

This application claims priority to Chinese patent application CN202311406408.4 filed on October 26, 2023 and Chinese patent application CN202410735258.X filed on June 6, 2024, the contents of which are incorporated herein by reference in their entirety and for all purposes.

### Field of the Invention

The present invention relates to the field of pharmaceutical chemistry, specifically to a compound acting as a PARP7 inhibitor.

### Background

Epigenetic inheritance is an important mechanism linking environmental stress to gene expression, and is a collective term for heritable or inheritable mechanisms that regulate gene expression without altering the gene's DNA sequence, including DNA methylation, histone modification, chromatin remodeling, non-coding RNA, etc. Epigenetic inheritance participates in the regulation of numerous biological processes, including cell differentiation, individual development, tumorigenesis and progression, etc. ADP-ribosylation (ADP-ribose, ADPr) modification is an epigenetic inheritance mechanism mediated by the PARPs family. PARPs can use NAD+ as a substrate to perform a post-translational modification on a protein to alter its function, and this process is reversible. ADPr modification participates in various biological processes in cells: 1) under a non-stress condition, cell division, transcription, and chromatin structure regulation all require ADPr, making it crucial for cellular physiological processes. 2) ADPr regulation is also required under a cellular stress condition, such as DNA damage, apoptosis, heat shock, cytoplasmic stress, and unfolded protein response, etc. 3) ADPr also plays an important role in immune cell signaling and activation, and can regulate the cellular response to cytokines as well as the expression and secretion of cytokines.

The PARP family currently has 17 members, all containing one conserved catalytic domain, but with limited sequence similarity. The functions of PARPs family members are not fully understood. Currently, with the exception of a few special PARPs (such as PARP13, without enzymatic activity), other PARPs can be divided into monoPARPs and polyPARPs, which complete MARylation (Mono-ADP-ribosylation) or PARylation (poly-ADP-ribosylation), respectively, by transferring single or multiple ADP-ribosyl groups. PARP1 is a polyPARP that has been relatively well studied to date. PARP7 is a monoPARP that can cleave one NAD+ molecule into one ADP-ribose and nicotinamide, catalyze the transfer of a single ADP-ribose unit onto proteins to alter their functions, and release nicotinamide. PARP7 is normally located in the cell nucleus, but can translocate to the cytoplasm in response to viral infection, reactive oxygen species (ROS), and mitochondrial damage.

When cells are stimulated by an environmental toxin such as a cigarette smoke toxin, viral infection, and a steroid hormone, the ligand-dependent transcription factor aryl hydrocarbon receptor (AHR) is activated, and AHR can upregulate PARP7. During viral infection, AHR-induced PARP7 can inactivate the kinase TBK1 by mono-ADP-ribosylation, preventing TBK1 from phosphorylating IRF3 and thus inhibiting the type I IFN immune response. TBK1 is a major kinase activated when the pathogen-associated molecular pattern pathway is turned on, which can activate the type I IFN response and antiviral immunity. A similar mechanism also exists in tumors. Due to genomic instability, tumor cells often carry abnormal cytoplasmic nucleic acids. These nucleic acids can activate the sensing mechanisms of cGAS/STING and RIG-I, which are pattern recognition receptors (PRRs) pathways. By activating TBK1 and phosphorylating IRF3, they can activate the innate immune response induced by type I IFNs. Tumor cells can evade the host's immune system by overexpressing or activating PARP7 and inhibiting TBK1 activation, thereby inhibiting the type I IFN response and CD8+T cell-mediated anti-tumor immunity. Inhibiting PARP7 can directly inhibit cancer cell proliferation by restoring the response of type I IFN signaling to abnormal nucleic acids, while simultaneously inducing tumor-specific adaptive immunological memory, leading to tumor regression and durable immunity. In some tumor types, the copy number of the PARP7 gene on chromosome 3 (3q25) is amplified, especially in squamous tissues of primary tumors (SCCL, HNSC) and some ovarian cancers, where the corresponding PARP7 mRNA levels are also increased. The tumor types with high PARP7 mRNA expression also express higher levels of baseline IFN-stimulated genes ISGs, and these cancer cell lines are more sensitive to the inhibition/knockout of PARP7.

Therefore, there is a need to develop small molecule inhibitors targeting PARP7 to provide more possibilities for the treatment of PARP7-mediated diseases (such as related sensitive cancer types).

### Summary of the Invention

The main object of the present invention is to provide a compound acting as a PARP7 inhibitor, and to develop a small molecule inhibitor targeting PARP7 to provide more possibilities for the treatment of PARP7-mediated diseases (such as related sensitive cancer types). The above object of the present invention is achieved by the following specific solutions.

In a first aspect of the present invention, there is provided a compound of formula I, or an enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof: 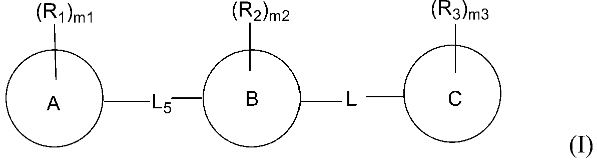 wherein,
ring A is selected from C6-10 aryl, 5-10-membered heteroaryl, 4-10-membered heterocycloalkyl;
ring B is selected from C6-10 aryl, 5-10-membered heteroaryl, 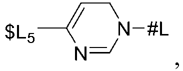 5-6-membered heterocycloalkyl-fused-5-6-membered heterocycloalkyl, C6-10 aryl-fused-5-10-membered heteroaryl, C6-10 aryl-fused-5-6-membered heterocycloalkyl, C6-10 aryl-fused-C5-6 cycloalkyl, 5-6-membered heteroaryl-fused-5-6-membered heterocycloalkyl, 5-6-membered heteroaryl-fused-C5-6 cycloalkyl; $L₅ represents the site of attachment to L₅, #L represents the site of attachment to L;
ring C is selected from: 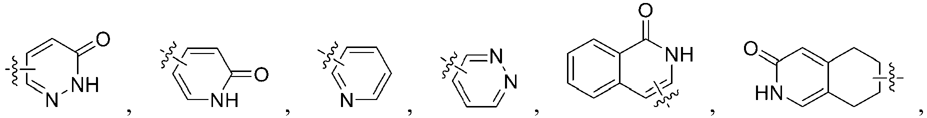 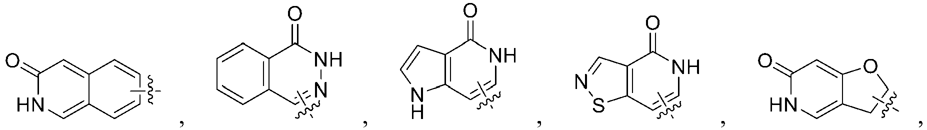 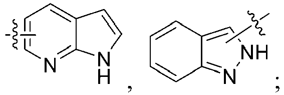 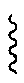 terminus is attached to L;
L is a linking group between ring B and ring C, and the number of main chain atoms of the linking group is between 2-7, preferably between 4-7;
L₅ is selected from a bond, $B-(CH₂)s-O-#A, $B-O-(CH₂)s-#A, $B-(CH₂)s-NRd-#A, $B-NRd-(CH₂)s-#A, wherein, s is 0, 1, or 2, the $B-(CH₂)s-O-#A, $B-O-(CH₂)s-#A, $B-(CH₂)s-NRd-#A, $B-NRd-(CH₂)s-#A are optionally substituted by one or more substituents selected from halogen, hydroxyl, C1-6 alkyl, C1-6 alkoxy, C1-6 haloalkyl, C1-6 haloalkoxy, $B represents the site of attachment to ring B, #A represents the site of attachment to ring A;
each R₁, R₂, and R₃ is independently selected from H, D, halogen, hydroxyl, -CN, -NReRf, -NReC(=O)Rf, -C(=O)-NReRf, C1-6 alkyl, C2-6 alkenyl, C2-6 alkynyl, C1-6 alkoxy, C1-6 haloalkyl, C1-6 haloalkoxy, C3-6 cycloalkyl, -C1-6 alkylene-C3-6 cycloalkyl, 5-6-membered heteroaryl, -C1-6 alkylene-5-6-membered heteroaryl, 4-7-membered heterocycloalkyl, -C1-6 alkylene-4-7-membered heterocycloalkyl, wherein, the C3-6 cycloalkyl, -C1-6 alkylene-C3-6 cycloalkyl, 5-6-membered heteroaryl, -C1-6 alkylene-5-6-membered heteroaryl, 4-7-membered heterocycloalkyl, -C1-6 alkylene-4-7-membered heterocycloalkyl are optionally substituted by 1 or more substituents selected from halogen, hydroxyl, C1-6 alkyl, C1-6 alkoxy, C1-6 haloalkyl, C1-6 haloalkoxy; optionally, two R₁ or R₂ attached to the same carbon atom form C=O with that carbon atom;
Rd is selected from H, C1-6 alkyl, C1-6 haloalkyl;
Re and Rf are each independently selected from H, C1-6 alkyl, C1-6 haloalkyl, C3-6 cycloalkyl, 4-7-membered heterocycloalkyl, or Re and Rf together with the atoms that they are attached to, form 4-7-membered heterocycloalkyl, and the 4-7-membered heterocycloalkyl is optionally substituted by 1 or more substituents selected from halogen, hydroxyl, cyano, C1-6 alkyl, C1-6 haloalkyl, C1-6 alkoxy, C1-6 haloalkoxy;
m1, m2, and m3 each independently represent an integer of 0-4.

In a second aspect of the present invention, there is provided a pharmaceutical composition, comprising the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, and a pharmaceutically acceptable carrier.

In a third aspect of the present invention, there is provided use of the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect , or the pharmaceutical composition of the second aspect in the preparation of a medicament for inhibiting PARP7 in a subject in need thereof, or for treating a PARP7-mediated disease; preferably, the PARP7-mediated disease is selected from cancer, immune diseases, inflammation, or viral infections.

In a fourth aspect of the present invention, there is provided a method for treating a PARP7-mediated disease, comprising administering to a subject in need thereof a therapeutically effective amount of the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, or the pharmaceutical composition of the second aspect, wherein the PARP7-mediated disease is selected from cancer, immune diseases, inflammation, or viral infections.

In a fifth aspect of the present invention, there is provided the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, or the pharmaceutical composition of the second aspect, for use in inhibiting PARP7 in a subject in need thereof, or for treating a PARP7-mediated disease; preferably, the PARP7-mediated disease is selected from cancer, immune diseases, inflammation, or viral infections.

### Advantageous Effects

The compounds of the present invention exhibit excellent inhibitory activity against PARP7, which can inhibit the binding of PARP7 to RBN011147, and also exert good inhibitory activity on the proliferation of NCI-H1373 cells. This provides more possibilities for developing small molecule inhibitors targeting PARP7 to treat PARP7-mediated diseases (such as related sensitive cancer types).

### Detailed Description

To make the objects, technical solutions, and advantages of the present invention clearer, the present invention will be further described in detail below with reference to examples. The particular examples described herein are intended only to explain the present invention and are not intended to constitute any limitation to the present invention. Furthermore, descriptions of well-known structures and techniques are omitted in the following description to avoid unnecessarily obscuring the concepts of the present disclosure. Such structures and techniques have also been described in many publications.

### Definitions

Unless otherwise defined, all technical and scientific terms used in the present invention have the same meanings as commonly used in the field to which the present invention pertains. For purposes of interpreting this specification, the following definitions will apply, and where appropriate, the terms used in the singular forms will also include the plural forms, and vice versa.

The expression "about" as used herein is as understood by one of ordinary skill in the art and varies within a certain rang depending on the context of its use. If one of ordinary skill in the art is unfamiliar with the use of this term in the context of its use, "about" will mean a particular value plus or minus at most 10%.

The term "alkyl" itself, or as part of another substituent, refers to a straight or branched chain saturated hydrocarbon group having a specified number of carbon atoms (i.e., C1-6 refers to a straight or branched chain hydrocarbon group having one to six carbon atoms). Preferably, an alkyl generally contains 1-6 carbon atoms, i.e., C1-6 alkyl. Examples of alkyl include, but are not limited to, methyl, ethyl, n-propyl, isopropyl, n-butyl, tert-butyl, isobutyl, sec-butyl, n-pentyl, n-hexyl, and similar alkyl thereof.

As used herein, the term "haloalkyl" refers to and includes a branched and straight chain saturated aliphatic hydrocarbon group having a specified number of carbon atoms and substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" of a branched and straight chain saturated aliphatic hydrocarbon group having a specified number of carbon atoms and substituted with 1 or more fluorine atoms.

The term "fluoroalkyl" or "fluorine-substituted alkyl" refer to an alkyl as defined above, wherein one or more hydrogen atoms are substituted by fluorine atoms.

The term "alkoxy" refers to a straight or branched chain alkyl linked by an ether oxygen, with its free valence bond originating from this ether oxygen. Representative examples include, but are not limited to, methoxy, ethoxy, propoxy, isopropoxy, and butoxy, etc. C1-3 alkoxy is preferred.

The term "haloalkoxy" refers to -O-haloalkyl, including a straight or branched chain haloalkoxy, and representative examples include (but are not limited to): fluoromethoxy, difluoromethoxy, trifluoromethoxy, trichloromethoxy, pentafluoroethoxy, pentachloroethoxy.

The term "cycloalkyl" or "carbocycle" refers to a cyclic alkyl including a saturated or partially unsaturated monocyclic, bicyclic or polycyclic ring, for example C3-8 or C3-12 cycloalkyl. C3-8 cycloalkyl refers to and includes C3, C4, C5, C6, C7, or C8 cycloalkyl. Cycloalkyl may also include a cycloalkyl with a structure such as spiro, bridged, fused ring, etc. Representative cycloalkyl groups of the present invention include, but are not limited to: cyclopropyl, cyclobutyl, cyclopentyl, cyclohexyl, and norbornyl. C5-12 fused bicyclic ring refers to and includes C5, C6, C7, C8, C9, C10, C11, C12 bicyclic alkyl, including but not limited to: 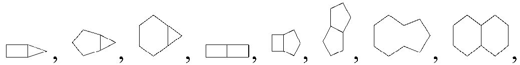 etc. C5-12 spiro bicyclic ring refers to and includes C5, C6, C7, C8, C9, C10, C11, C12 bicyclic alkyl, including but not limited to: 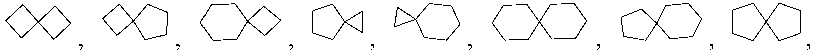 etc. In the present invention, cycloalkyl is preferably a monocyclic cycloalkyl containing 3 to 6 carbon atoms (i.e., C3-6), such as cyclopropyl, cyclobutyl, cyclopentyl, or cyclohexyl.

The term "aryl", alone or as part of a group such as "aralkyl", "aralkyloxy", or "aryloxyalkyl", refers to a monocyclic, bicyclic, or tricyclic carbocyclic system (preferably a 6-10-membered aromatic ring) having a total of 6 to 15 ring members (or ring atoms), wherein all rings in the system are aromatic and each ring in the system contains 3 to 7 ring members; when not all rings in the system are aromatic, they are considered as cycloalkyl. In certain embodiments of the present invention, "aryl" refers to an aromatic ring system, including but not limited to phenyl, biphenyl, 1-naphthalenyl, 2-naphthalenyl, anthracenyl.

The connecting lines drawn from the ring system indicate that one end of the bond can be connected to any suitable ring atom in the ring system that the bond passes through. If the bond passes through one ring, it means the connection can be at any optional position on that ring; if the bond passes through multiple rings, it means the connection can be at any optional position on those multiple rings. For example, the structure shown in the following formula (E) includes any possible connection mode shown in formulas (E1)-(E3):

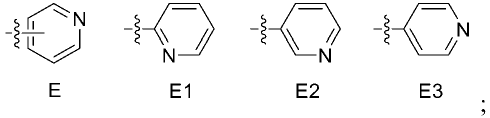

However, in this application, when ring C is the structure as shown in formula (F), it only includes the possible connection mode as shown in formula (F1) or (F2):

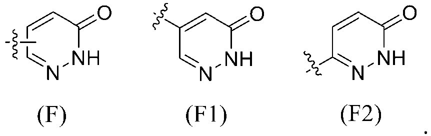

Or, in the structure as shown in the following formula (G), for example, the connecting line passes through two rings, and it includes any possible connection mode shown in formulas (G1)-(G5):

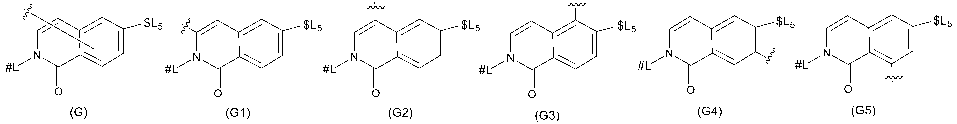

The term "heteroaryl" refers to a monocyclic or fused polycyclic aromatic heterocyclic system, which contains at least one ring atom selected from N, O, S, with the remaining ring atoms being C. Preferred heteroaryl is a monocyclic ring containing 5 to 10, especially 5 to 8, more preferably 5 or 6 ring atoms, or multiple fused rings containing 6 to 14, especially 6 to 10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, etc.

The term "heterocycloalkyl" refers to a cyclic group that is fully saturated or partially unsaturated and can exist as a monocyclic, bridged, or spiro ring, the heterocycloalkyl is typically a cycloalkyl containing 1 to 5, preferably 1 to 3 heteroatoms independently selected from N, O, and S (preferably 1 or 2 heteroatoms). The ring atom of the heterocycloalkyl is optionally substituted by oxo (=O). Non-limiting examples of heterocycloalkyl include oxiranyl, thiiranyl, aziridinyl, azetidinyl, oxetanyl, thietanyl, tetrahydrofuranyl, tetrahydrothienyl, pyrrolidinyl, isoxazolidinyl, oxazolidinyl, isothiazolidinyl, thiazolidinyl, imidazolidinyl, butyrolactam, valerolactam, imidazolidinone, hydantoin, dioxolane, phthalimide, tetrahydropyrazolyl, piperidinyl, tetrahydropyranyl, tetrahydrothiopyranyl, morpholinyl, thiomorpholine-S-oxide, thiomorpholine-S,S-oxide, pyranyl, pyridonyl, 3-pyrrolinyl, thiopyranyl, pyronyl, piperazinyl, 1,4-thioxanyl, 1,4-dioxanyl, thiomorpholinyl, 1,3-dithianyl, 1,4-dithianyl, azepanyl, oxepanyl, thiepanyl. Heterocycloalkyl can be attached to the rest of the molecule via a cyclic carbon or a heteroatom.

In the present invention, the term "substitution" refers to the substitution of one or more hydrogen atoms on a specific group by a specific substituent. The specific substituents are those correspondingly described hereinabove, or the substituents present in the respective examples. Unless otherwise specified, a certain substituted group may have a substituent selected from a specific group at any substitutable site of that group, and the substituents may be the same or different at each position. Those skilled in the art will understand that the combinations of substituents contemplated in the present invention are those combinations that can provide stable or chemically feasible compounds. Typical substitutions include, but are not limited to, one or more (for example 1-3) the following groups: such as hydrogen, deuterium, halogen, hydroxyl (-OH), nitrile group, nitro, oxo group (such as =O, where two H on a carbon atom are substituted by it to form a carbonyl), alkyl (C1-6 alkyl), cycloalkyl (for example C3-6 cycloalkyl), alkenyl, alkynyl, heterocycloalkyl (for example 4-10-membered heterocycloalkyl), aryl, heteroaryl (for example 5-6-membered heteroaryl), alkoxy (for example C1-6 alkoxy, such as methoxy, ethoxy), C1-6 haloalkyl (for example, mono-halogen substituted or poly-halogen substituted, the latter such as trifluoromethyl), C1-6 haloalkoxy (for example, mono-halogen substituted or poly-halogen substituted, the latter such as trifluoromethoxy), -OC(=O)NRᵢᵢRᵢᵢᵢ, ORᵢ, SRᵢ, S(=O)Rᵥ, S(=O)₂Rᵥ, P(=O)₂Rᵥ, S(=O)₂ORᵥ, P(=O)₂ORᵥ, NRᵢᵢRᵢᵢᵢ, NRᵢᵢS(=O)₂Rᵥ, NRᵢᵢP(=O)Rᵥ, S(=O)₂NRᵢᵢRᵢᵢᵢ, P(=O)₂NRᵢᵢRᵢᵢᵢ, C(=O)ORᵢᵥ, C(=O)Rᵢ, C(=O)NRᵢᵢRᵢᵢᵢ, OC(=O) Rᵢ, OC(=O)NRᵢᵢRᵢᵢᵢ, NRᵢᵢC(=O)ORᵥ, NRᵢᵥC(=O)NRᵢᵢRᵢᵢᵢ, NRᵢᵥS(=O)₂NRᵢᵢRᵢᵢᵢ, NRᵢᵥP(=O)₂NRᵢᵢRᵢᵢᵢ, NRᵢᵢC(=O)Rᵢ, or NRᵢᵢP(=O)₂Rᵥ, wherein, Rᵢ may independently represent hydrogen, deuterium, alkyl, cycloalkyl, alkenyl, alkynyl, heterocycle, or aromatic ring, Rᵢᵢ, Rᵢᵢᵢ, and Rᵢᵥ may independently represent hydrogen, deuterium, alkyl (for example C1-6 alkyl), cycloalkyl (for example C3-6 cycloalkyl), heterocycloalkyl, or aryl, or Rᵢᵢ and Rᵢᵢᵢ together with the atoms that they are attached to, may form heterocycloalkyl (for example C4-10 heterocycloalkyl), the heterocycloalkyl is optionally substituted by 1-3 substituents selected from halogen, -OH, C1-6 alkyl, C1-6 alkoxy, C1-6 haloalkyl, C1-6 haloalkoxy; Rᵥ may independently represent hydrogen, alkyl, cycloalkyl, alkenyl, alkynyl, heterocycle, or aromatic ring. The above typical substituents, such as alkyl, cycloalkyl, alkenyl, cycloalkenyl, alkynyl, heterocycle, or aromatic ring can be optionally substituted. The substituents include, for example (but are not limited to): halogen, hydroxyl, cyano, carboxyl (-COOH), C1-6 alkyl, C2-6 alkenyl, C2-6 alkynyl, C3-8 cycloalkyl, 3-12-membered heterocyclyl, aryl, heteroaryl, C1-8 aldehyde group, C2-10 acyl, C2-10 ester group, amine group, C1-6 alkoxy, C1-10 sulfonyl, and C1-6 ureido, etc.

The pharmaceutical composition of the present invention may contain a pharmaceutically acceptable excipient, including but not limited to: an ion exchanger, alumina, aluminum stearate, lecithin, a serum protein such as human serum albumin, a buffering substance such as a phosphate, glycerol, sorbic acid, potassium sorbate, a mixture of some glycerides of saturated vegetable fatty acids, water, a salt or an electrolyte such as protamine sulfate, disodium hydrogen phosphate, potassium hydrogen phosphate, sodium chloride, a zinc salt, colloidal silicon oxide, magnesium trisilicate, polyvinylpyrrolidone, a cellulose substance, polyethylene glycol, sodium carboxymethyl cellulose, polyacrylate, beeswax, lanolin, etc.

The pharmaceutical composition of the present invention can be prepared into various forms according to different routes of administration. For example, the pharmaceutical composition can be administered in any of the following ways: orally, by spray inhalation, rectal administration, nasal administration, buccal administration, vaginal administration, topical administration, parenteral administration such as subcutaneous, intravenous, intramuscular, intraperitoneal, intrathecal, intraventricular, intrasternal, and intracranial injection or infusion, or by means of an explantation reservoir administration. Among them, oral or intravenous administration is preferred.

The compounds of the present invention may optionally also be used in combination with one or more other active ingredients, and the respective dosage and proportion thereof can be adjusted by those skilled in the art according to the specific diseases and the patient's individual conditions, and clinical needs, etc. Combined use does not mean that the therapies or therapeutic agents must be administered simultaneously and/or formulated for co-delivery, although such delivery methods are within the scope of the present invention. The therapeutic agent used in combination may be administered simultaneously with, before, or after one or more other additional therapies or therapeutic agents. The therapeutic agent or treatment regimen may be administered in any order.

As used herein, unless otherwise stated, the term "prodrug" refers to a derivative that can be hydrolyzed, oxidized, or undergo other reactions under a biological condition (in vitro or in vivo) to provide the compound of the present invention. Prodrugs become active compounds only after undergoing the reaction under a biological condition, or they do not have or only have a lower activity in their unreacted forms. Prodrugs can usually be prepared using a well-known method, for example, those methods described in Burger's Medicinal Chemistry and Drug Discovery (1995) 172-178, 949-982 (edited by Manfred E. Wolff, 5th edition).

Unless otherwise specified, when a substituent is described by a conventional chemical formula written from left to right, the substituent only includes its form written from left to right, and is attached to the structure on the left side and the structure on the right side, respectively, of the corresponding group in the general structural formula.

The term "enantiomer" refers to two isomers of a compound that cannot superimposable but mirror-image each other.

The term "diastereomer" refers to stereoisomers that have two or more chiral centers and whose molecules are not mirror images of each other. Diastereomers have different physical properties, such as melting point, boiling point, spectral properties, and reactivity. The mixture of diastereomers can be separated by a high-resolution analytical operation such as electrophoresis and chromatography, for example, HPLC.

The term "racemate", "racemic compound", or "racemic mixture" refers to an equimolar mixture of two enantiomers lacking optical activity.

The term "tautomer" or "tautomeric form" refers to structural isomers with different energies that can be interconverted through a lowenergy barrier. If tautomerism is possible (e.g., in solution), a chemical equilibrium can be achieved for the tautomers. For example, protontautomers (also known as prototropic tautomers) involve interconversions via proton migration, such as keto-enol isomerization and imine-enamine isomerization. Valence tautomers involve interconversions via the rearrangement of some bonding electrons. A particular example of keto-enol tautomerism is the interconversion between pentan-2,4-dione and 4-hydroxypent-3-en-2-one tautomers. Another example of tautomerism is the phenol-keto tautomerism. A particular example of phenol-keto tautomerism is the interconversion between pyridin-4-ol and pyridin-4(1H)-one. Unless otherwise indicated, all tautomer forms of the compounds of the present invention are within the scope of the present invention.

The term "stereoisomer" refers to compounds having the same chemical construction but with different spatial arrangements of atoms or groups. Stereoisomers include enantiomers, diastereomers, conformational isomers (rotamers), geometric isomers (cis/trans isomers), atropisomers, etc.

The term "geometric isomer", also known as "cis/trans isomer", refers to isomers formed because a double bond (including a double bond in an alkene, a C=N double bond, and a N=N double bond) or a single bond between cyclic carbon atoms cannot rotate freely.

The stereochemical definitions and rules used in the present invention generally follow S.P.Parker, Ed.,McGraw-Hill Dictionary of Chemical Terms (1984) McGraw-Hill Book Company, New York; and Eliel, E.and Wilen, S, "Stereochemistry of Organic Compounds", John Wiley&Sons, Inc, New York, 1994. Many organic compounds exist in an optically active form, namely, they possess the ability to rotate the plane of plane-polarized light. When describing an optically active compound, the prefixes D and L or R and S are used to indicate the absolute configuration of the molecule with respect to one or more of its chiral centers. The prefixes d and l or (+) and (-) are symbols used to specify the rotation of plane-polarized light caused by the compound, where (-) or l indicates that the compound is levorotatory. The compound with the prefix (+) or d is dextrorotatory. A particular stereoisomer is an enantiomer, and a mixture of such isomers is called an enantiomer mixture. A 50:50 mixture of enantiomers is called a racemic mixture or racemate, which can occur when there is no stereoselectivity or stereospecificity in a chemical reaction or process.

Any asymmetric atom (e.g., carbon, etc.) of the compounds disclosed in the present invention can exist in a racemic or enantiomerically enriched form, for example in the (R)-, (S)-, or (R,S)-configuration forms. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess with respect to the (R)- or (S)-configuration.

Depending on the choice of starting materials and methods, the compounds of the present invention can exist as one of the possible isomers or mixtures thereof, for example mixtures of racemates and diastereomers (depending on the number of asymmetric carbon atoms). Optically active (R)- or (S)-isomers can be prepared using a chiral synthon or chiral reagent, or resolved using a conventional technique. If the compound contains a double bond, the substituents may have the E or Z configuration; if the compound contains a disubstituted cycloalkyl group, the substituents of the cycloalkyl group may have the cis or trans configuration.

Any mixture of the resulting stereoisomers can be separated into pure or substantially pure geometric isomers, enantiomers, and diastereomers based on differences in the physicochemical properties of the components, for example, by chromatography and/or fractional crystallization.

The racemates of any resulting end products or intermediates can be resolved into optical enantiomers by known methods familiar to those skilled in the art, such as by separating their diastereomeric salts obtained. Racemic products can also be separated by chiral chromatography, such as high performance liquid chromatography (HPLC) using chiral adsorbents. In particular, enantiomers can be prepared by asymmetric synthesis, for example, reference may be made to Jacques,et al., Enantiomers, Racematesand Resolutions (Wiley Interscience, New York, 1981); Principles of Asymmetric Synthesis (2nd Ed. Robert E. Gawley, Jeffrey Aube, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p.268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH&Co. KGaA, Weinheim, Germany, 2007).

The term "nitrogen oxide" refers to the formation of N-oxides by oxidizing one or more than one nitrogen atom when a compound contains several amine functional groups. Specific examples of N-oxides are N-oxides of tertiary amines or N-oxides of nitrogen atoms in nitrogen-containing heterocycles. The corresponding amines can be treated with an oxidizing agent, for example hydrogen peroxide, or a peracid (e.g., peroxycarboxylic acid) to form N-oxides (see Advanced Organic Chemistry, Wiley Interscience, 4th edition, Jerry March, pages). Especially, N-oxides can be prepared by the method of L.W.Deady (Syn.Comm. 1977, 7, 509-514), in which, for example, an amine compound is reacted with meta-chloroperbenzoic acid (MCPBA) in an inert solvent such as dichloromethane.

The compounds described herein include all possible isotope-labeled compounds of all compounds described herein. The term "isotope-labeled compound" refers to a compound obtained by replacing any atom in the compound with its isotopic atom. Examples of isotopes of the compounds that can be listed as those of the present invention include hydrogen, carbon, nitrogen, oxygen, phosphorus, sulfur, fluorine, and chlorine isotopes, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively.

"Solvated compound" or "solvate" can be used interchangeably, referring to a compound that exists in combination with certain solvent molecules. This combination may include a stoichiometric amount of a certain solvent; for example, when the solvent is water, a "hydrate" is formed, such as a monohydrate or dihydrate, or it may include any amount of water. As another example, when the solvent is an alcohol, such as methanol or ethanol, an "alcoholate" can be formed, which may also be stoichiometric or non-stoichiometric. As used herein, the term "solvate" refers to the solid form; that is, although a compound in a solvent solution may be solvated, it is not a solvate in the sense of the term used herein.

As used herein, the term "metabolite" refers to a derivative of a compound formed during the metabolism of the compound. The term "metabolism" refers to the sum of processes by which a specific substance is transformed by an organism (including, but not limited to, hydrolysis reactions and enzyme-catalyzed reactions).

As used herein, the term "ester" refers to an ester formed from -COOH present in the compounds provided by the present invention and a suitable alcohol, or an ester formed from - OH present in the compounds provided by the present invention and a suitable acid (e.g., a carboxylic acid or an oxygen-containing inorganic acid). Suitable ester groups include, but are not limited to, formate, acetate, propionate, butyrate, acrylate, ethyl succinate, stearate, or palmitate. Esters can undergo hydrolysis reactions in the presence of acids or bases to produce corresponding acids or alcohols.

As used herein, the term "pharmaceutically acceptable salt" refers to: (i) a salt formed by an acidic functional group (e.g., -COOH) present in the compounds provided by the present invention and a suitable inorganic or organic cation (base), including but not limited to, alkali metal salts such as sodium salt, potassium salt, lithium salt, etc.; alkaline earth metal salts such as calcium salt, magnesium salt, etc.; other metal salts such as aluminum salt, iron salt, zinc salt, copper salt, nickel salt, cobalt salt, etc.; inorganic base salts such as ammonium salt; organic base salts such as tert-octylamine salt, dibenzylamine salt, morpholine salt, glucosamine salt, phenylglycine alkyl ester salt, ethylenediamine salt, N-methylglucosamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenylethylamine salt, piperazine salt, tetramethylamine salt, tris(hydroxymethyl)aminomethane salt; and, (ii) a salt formed by a basic functional group (e.g., -NH2) present in the compounds provided by the present invention and a suitable inorganic or organic anion (acid), including but not limited to, hydrohalides, such as hydrofluoride, hydrochloride, hydrobromide, hydroiodide, etc.; inorganic acid salts, such as nitrate, perchlorate, sulfate, phosphate, etc.; lower alkanesulfonates, such as methanesulfonate, trifluoromethanesulfonate, ethanesulfonate, etc.; arylsulfonates, such as benzenesulfonate, p-toluenesulfonate, etc.; organic acid salts, such as acetate, malate, fumarate, succinate, citrate, tartrate, oxalate, maleate, etc.; amino acid salts, such as glycinate, trimethylglycinate, arginate, ornithinate, glutamate, aspartate, etc.

As used herein, the term "crystal form" refers to the crystal structure of a substance. The substance undergoes changes in an intramolecular or intermolecular bonding mode due to various factors during crystallization, resulting in different spatial arrangements of molecules or atoms in the crystal lattice and thus forming different crystal structures. The compounds of the present invention can exist in one crystal structure or multiple crystal structures, i.e., having "polymorphic form". The compounds of the present invention can exist in different crystal forms.

As described herein, the term "treatment" refers to reducing or improving the progression, severity, and/or duration of a condition, for example a proliferative condition, or improving one or more symptoms (preferably one or more identifiable symptoms) of a condition caused by the administration of one or more therapies. In a particular embodiment, the term "treatment" refers to an improvement in at least one measurable physical parameter of a proliferative condition in a patient, which may not be identifiable, for example the growth of a tumor. In other embodiments, the term "treatment" refers to physically or physiologically inhibiting the progression of a proliferative condition, for example, by stabilizing identifiable symptoms or stabilizing physical parameters, or both. In other embodiments, the term "treatment" refers to a reduction or stabilization in tumor size or cancer cell count.

The terms "subject" or "patient" refer to warm-blooded animals suffering from a specific disease, condition, or disorder, for example mammals, especially guinea pigs, dogs, cats, rats, mice, horses, cattle, sheep, and humans.

The term "cancer" refers to a disease characterized by the rapid and uncontrolled growth of abnormal cells. Cancer cells can spread locally or to other parts of the body through the bloodstream and lymphatic system. The terms "tumor" and "cancer" are used interchangeably herein; for example, both terms cover a solid tumor and a liquid tumor, for example a diffuse or circulating tumor. As used herein, the term "cancer" or "tumor" includes both pre-malignant and malignant cancers and tumors. As used herein, the term "cancer" is meant to include all types of cancerous growths or oncogenic processes, metastatic tissues, or malignantly transformed cells, tissues, or organs, regardless of the histopathological type or stage of invasiveness. Examples of cancerous conditions include, but are not limited to, breast cancer, central nervous system cancer, endometrial cancer, renal cancer, colorectal carcinoma, lung cancer, esophageal cancer, ovarian cancer, pancreatic cancer, prostate cancer, stomach cancer, head and neck cancer (upper aerodigestive tract cancer), urinary tract cancer, colon cancer, and cancers with amplified PARP7 expression, etc. In some embodiments, the cancers treatable according to the present invention include hematopoietic malignancies, for example leukemia and lymphoma. Exemplary lymphomas include Hodgkin lymphoma or non-Hodgkin lymphoma, multiple myeloma, B-cell lymphoma (e.g., diffuse large B-cell lymphoma (DLBCL)), chronic lymphocytic lymphoma (CLL), T-cell lymphoma, hairy cell lymphoma, and Burkitt's lymphoma. Examples of leukemia include acute lymphocytic leukemia (ALL), acute myelogenous leukemia (AMIL), chronic lymphocytic leukemia (CLL), and chronic myelogenous leukemia (CML).

Examples of autoimmune diseases that can be treated or prevented by administering the compounds of the present invention include, but are not limited to, rheumatoid arthritis, insulin-dependent diabetes mellitus, certain hemolytic anemias, rheumatic fever, thyroiditis, ulcerative colitis, myasthenia gravis, glomerulonephritis, allergic encephalomyelitis, continued damage to nerve and liver after viral hepatitis, multiple sclerosis, systemic lupus erythematosus, juvenile diabetes, autoimmune hemolytic anemia, psoriasis, idiopathic thrombocytopenic purpura, active chronic hepatitis, idiopathic leukopenia, primary biliary cirrhosis, thyrotoxicosis, dermatomyositis, discoid lupus erythematosus, psoriatic arthritis, regional enteritis, nephrotic syndrome, lupus nephritis, lupus hepatitis, Sjögren syndrome, Goodpasture syndrome, Wegener granulomatosis, scleroderma, sebaceous disease, uveitis and mumps mastitis, ulcerative colitis, Crohn's disease, multiple sclerosis, autoimmune liver disease, type I diabetes mellitus, bronchial asthma, systemic lupus erythematosus, rheumatoid arthritis, ankylosing spondylitis, juvenile idiopathic arthritis, psoriasis, polymyositis, and dermatomyositis.

Viruses and viral infections that can be treated or prevented by administering the compounds of the present invention include, but are not limited to, DNA viruses such as hepatitis B virus and hepatitis C virus; parvoviruses such as adeno-associated viruses and cytomegaloviruses; papillomaviruses such as papillomaviruses, polyomaviruses, and SV40; adenoviruses; herpesviruses such as herpes simplex virus type I (HSV-I), herpes simplex virus type II (HSV-II), and Epstein-Barr virus; poxviruses, such as variola virus (smallpox) virus and vaccinia virus; and RNA viruses such as human immunodeficiency virus type I (HIV-I), human immunodeficiency virus type II (HIV-II), human T-cell lymphotropic virus type I (HTLV-I), human T-cell lymphotropic virus type II (HTLV-II), influenza viruses, measles virus, rabies virus, Sendai virus, papillomaviruses such as polioviruses, coxsackieviruseses, rhinoviruses, reoviruses, togaviruses such as rubella virus (German measles) and Semliki Forest virus, dendritic virus, and hepatitis A virus.

Unless otherwise specified, the substitutions or combinations of groups involved in the Markush structures described herein are those substitutions or combinations that are stable or chemically feasible.

Those skilled in the art will understand that when referring to a ring or substituent that is clearly divalent according to the general formula, it is selected from divalent rings or groups, even if it is not explicitly stated that it may be or is selected from divalent candidates; wherein, a divalent group refers to a group formed after the corresponding group, such as an aryl group like a phenyl group, further loses a hydrogen atom. For example, ring B selected from phenyl and ring B selected from 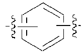 have the same meanings. For another example, L3 selected from C3-10 cycloalkyl indicates that L3 is selected from a group formed after the C3-10 cycloalkyl further loses a hydrogen atom.

Meanwhile, those skilled in the art will understand that when a single group connecting two groups is a bond, it indicates that the two groups connected by it are directly connected; when multiple consecutive groups connecting two groups are all bonds, it indicates that the two groups connected by them are directly connected.

### Compounds

In one aspect, the present invention provides a compound of formula I, or an enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof: 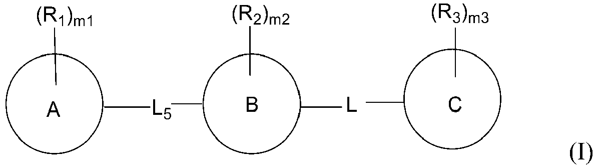 wherein,
ring A is selected from C6-10 aryl, 5-10-membered heteroaryl, 4-10-membered heterocycloalkyl;
ring B is selected from C6-10 aryl, 5-10-membered heteroaryl, 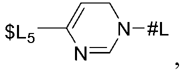 5-6-membered heterocycloalkyl-fused-5-6-membered heterocycloalkyl, C6-10 aryl-fused-5-10-membered heteroaryl, C6-10 aryl-fused-5-6-membered heterocycloalkyl, C6-10 aryl-fused-C5-6 cycloalkyl, 5-6-membered heteroaryl-fused-5-6-membered heterocycloalkyl, 5-6-membered heteroaryl-fused-C5-6 cycloalkyl; $L₅ represents the site of attachment to L₅, #L represents the site of attachment to L;
ring C is selected from: 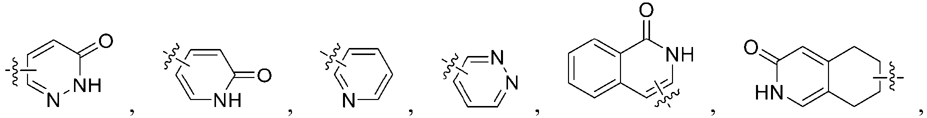 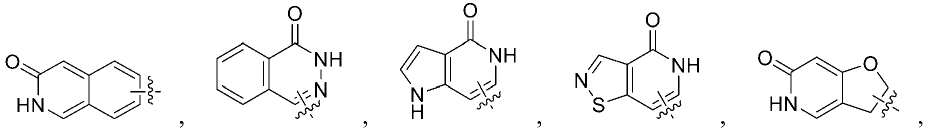 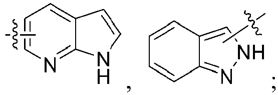 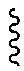 terminus is attached to L;
L is a linking group between ring B and ring C, and the number of main chain atoms of the linking group is between 2-7, preferably between 4-7;
L₅ is selected from a bond, $B-(CH₂)s-O-#A, $B-O-(CH₂)s-#A, $B-(CH₂)s-NRd-#A, $B-NRd-(CH₂)s-#A, wherein, s is 0, 1, or 2, the $B-(CH₂)s-O-#A, $B-O-(CH₂)s-#A, $B-(CH₂)s-NRd-#A, $B-NRd-(CH₂)s-#A are optionally substituted by one or more substituents selected from halogen, hydroxyl, C1-6 alkyl, C1-6 alkoxy, C1-6 haloalkyl, C1-6 haloalkoxy, $B represents the site of attachment to ring B, #A represents the site of attachment to ring A;
each R₁, R₂, and R₃ is independently selected from H, D, halogen, hydroxyl, -CN, -NReRf, -NReC(=O)Rf, -C(=O)-NReRf, C1-6 alkyl, C2-6 alkenyl, C2-6 alkynyl, C1-6 alkoxy, C1-6 haloalkyl, C1-6 haloalkoxy, C3-6 cycloalkyl, -C1-6 alkylene-C3-6 cycloalkyl, 5-6-membered heteroaryl, -C1-6 alkylene-5-6-membered heteroaryl, 4-7-membered heterocycloalkyl, -C1-6 alkylene-4-7-membered heterocycloalkyl, wherein, the C3-6 cycloalkyl, -C1-6 alkylene-C3-6 cycloalkyl, 5-6-membered heteroaryl, -C1-6 alkylene-5-6-membered heteroaryl, 4-7-membered heterocycloalkyl, -C1-6 alkylene-4-7-membered heterocycloalkyl are optionally substituted by 1 or more substituents selected from halogen, hydroxyl, C1-6 alkyl, C1-6 alkoxy, C1-6 haloalkyl, C1-6 haloalkoxy; optionally, two R₁ or R₂ attached to the same carbon atom form C=O with that carbon atom;
Rd is selected from H, C1-6 alkyl, C1-6 haloalkyl;
Re and Rf are each independently selected from H, C1-6 alkyl, C1-6 haloalkyl, C3-6 cycloalkyl, 4-7-membered heterocycloalkyl, or Re and Rf together with the atoms that they are attached to, form 4-7-membered heterocycloalkyl, and the 4-7-membered heterocycloalkyl is optionally substituted by 1 or more substituents selected from halogen, hydroxyl, cyano, C1-6 alkyl, C1-6 haloalkyl, C1-6 alkoxy, C1-6 haloalkoxy;
m1, m2, and m3 each independently represent an integer of 0-4.

In some embodiments, ring B is selected from C6-10 aryl, 5-10-membered heteroaryl, 5-6-membered heterocycloalkyl-fused-5-6-membered heterocycloalkyl, C6-10 aryl-fused-5-10-membered heteroaryl, C6-10 aryl-fused-5-6-membered heterocycloalkyl, C6-10 aryl-fused-C5-6 cycloalkyl, 5-6-membered heteroaryl-fused-5-6-membered heterocycloalkyl, 5-6-membered heteroaryl-fused-C5-6 cycloalkyl;
ring C is selected from: 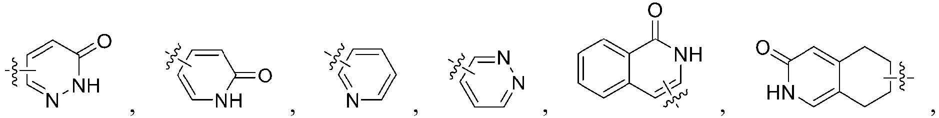 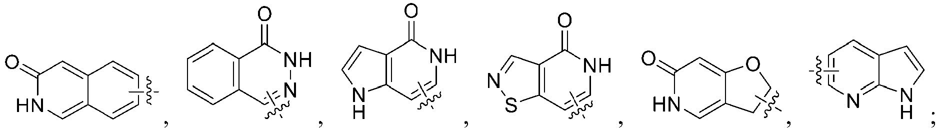 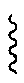 terminus is attached to L;
m1, m2, and m3 each independently represent an integer of 0-3.

In some embodiments, ring B is selected from C6-10 aryl, 5-10-membered heteroaryl, 5-6-membered heterocycloalkyl-fused-5-6-membered heterocycloalkyl, C6-10 aryl-fused-5-10-membered heteroaryl, C6-10 aryl-fused-5-6-membered heterocycloalkyl, C6-10 aryl-fused-C5-6 cycloalkyl, 5-6-membered heteroaryl-fused-5-6-membered heterocycloalkyl, 5-6-membered heteroaryl-fused-C5-6 cycloalkyl; any of the above heteroaryl groups has 1, 2, or 3 nitrogen heteroatoms and 0 or 1 heteroatom selected from O and S, any of the above heterocycloalkyl groups has 1 or 2 nitrogen heteroatoms and 0 or 1 heteroatom selected from O and S.

In some embodiments, ring A is selected from C6-10 aryl, 5-10-membered heteroaryl, 4-10-membered heterocycloalkyl; the 5-10-membered heteroaryl has 1, 2, 3, or more nitrogen heteroatoms and 0, 1, or 2 heteroatoms selected from O and S, the 4-10-membered heterocycloalkyl has 1, 2, or more nitrogen heteroatoms and 0, 1, or 2 heteroatoms selected from O and S.

In some embodiments, ring A is selected from 5-6-membered heteroaryl, 6-10-membered heterocycloalkyl; preferably, ring A is selected from 5-6-membered heteroaryl, 6-membered heterocycloalkyl, 7-10-membered spiro heterocycloalkyl; further preferably, ring A is selected from 5-6-membered heteroaryl.

In some embodiments, ring A is selected from 5-6-membered heteroaryl, 6-10-membered heterocycloalkyl; preferably, ring A is selected from 5-6-membered heteroaryl, 6-membered heterocycloalkyl, 7-10-membered spiro heterocycloalkyl; further preferably, ring A is selected from 5-6-membered heteroaryl; the heteroaryl has 1, 2, or 3 nitrogen heteroatoms and 0 or 1 heteroatom selected from O and S, the heterocycloalkyl has 1 or 2 nitrogen heteroatoms and 0 or 1 heteroatom selected from O and S. In some embodiments, ring A is selected from 5-6-membered heteroaryl, 6-membered monocyclic partially unsaturated heterocycloalkyl, 6-membered monocyclic saturated heterocycloalkyl, and 7-9-membered saturated spiro bicyclic heterocycloalkyl; the 5-6-membered heteroaryl has 1, 2, or 3 nitrogen heteroatoms and 0 or 1 heteroatom selected from O and S, the 6-membered monocyclic partially unsaturated heterocycloalkyl has 1 or 2 (preferably 2) nitrogen heteroatoms, the 6-membered monocyclic saturated heterocycloalkyl has 1 or 2 nitrogen heteroatoms and 0 or 1 oxygen heteroatom, the 7-9-membered saturated spiro bicyclic heterocycloalkyl has 1 or 2 (preferably 2) nitrogen heteroatoms.

In some embodiments, ring A is selected from 5-6-membered heteroaryl, the 5-6-membered heteroaryl has 1 or 2 nitrogen heteroatoms and 0 or 1 heteroatom selected from O and S, preferably has 1 or 2 nitrogen heteroatoms and 0 or 1 sulfur heteroatom.

In some embodiments, ring A is selected from pyrimidinyl, pyridinyl, 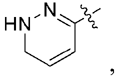 piperazinyl, morpholinyl, 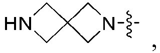 thiazolyl, isothiazolyl, imidazolyl, pyrazolyl, triazolyl (for example 1,2,4-triazolyl, 1,3,4-triazolyl), oxazolyl, isoxazolyl, 1,2,4-oxadiazole, 1,3,4-thiadiazole, 1,2,4-thiadiazole; 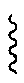 terminus is attached to L₅.

In some embodiments, ring A is selected from pyrimidinyl, thiazolyl, isothiazolyl, pyrazolyl, pyridinyl; further preferably, ring A is selected from pyrimidinyl (for example 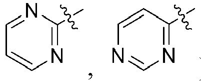 ), pyridinyl (for example 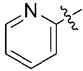 ), 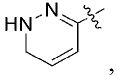 piperazinyl (for example 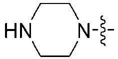 ), morpholinyl (for example 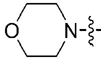 ), 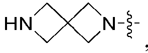 , thiazolyl (for example 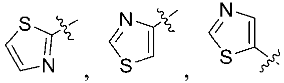 ), isothiazolyl (for example 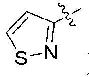 ), imidazolyl (for example 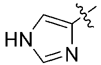 ), pyrazolyl (for example 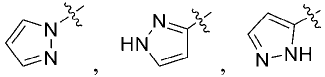 ), 1,2,4-triazolyl (for example 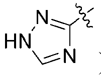 ), 1,3,4-triazolyl (for example 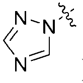 ), oxazolyl (for example 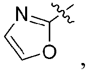 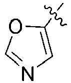 ), isoxazolyl (for example 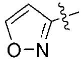 ), 1,2,4-oxadiazole (for example 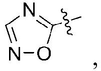 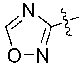 ), 1,3,4-thiadiazole ( 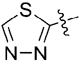 ), 1,2,4-thiadiazole (for example 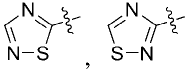 ); 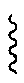 terminus is attached to L₅.

In some embodiments, ring A is selected from 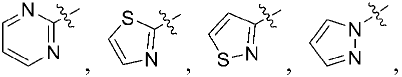 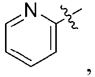 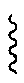 terminus is attached to L₅.

In some embodiments, each R₁ is independently selected from halogen, hydroxyl, -CN, C1-6 alkyl, C2-6 alkynyl, C1-6 alkoxy, C1-6 haloalkyl, C3-6 cycloalkyl, -C1-6 alkylene-C3-6 cycloalkyl, 4-7-membered heterocycloalkyl, -C1-6 alkylene-4-7-membered heterocycloalkyl, 5-6-membered heteroaryl, -NReRf, -C(=O)-NReRf, wherein, the C3-6 cycloalkyl, -C1-6 alkylene-C3-6 cycloalkyl, 4-7-membered heterocycloalkyl, -C1-6 alkylene-4-7-membered heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-6 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 substituents selected from halogen, C1-3 alkyl.

In some embodiments, each R₁ is independently selected from halogen, hydroxyl, -CN, C1-6 alkyl, C2-6 alkynyl, C1-6 alkoxy, C1-6 haloalkyl, C1-6 haloalkoxy, C3-6 cycloalkyl, -C1-6 alkylene-C3-6 cycloalkyl, 4-7-membered heterocycloalkyl, 5-6-membered heteroaryl, - NReRf; wherein, the C3-6 cycloalkyl, -C1-6 alkylene-C3-6 cycloalkyl, 4-7-membered heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-6 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 substituents selected from halogen, C1-3 alkyl.

In some embodiments, each R₁ is independently selected from halogen, hydroxyl, -CN, C1-6 alkyl, C2-6 alkynyl, C1-6 alkoxy, C1-6 haloalkyl, C1-6 haloalkoxy, C3-6 saturated cycloalkyl, -C1-6 alkylene-C3-6 saturated cycloalkyl, 4-7-membered saturated heterocycloalkyl, -C1-6 alkylene-4-7-membered saturated heterocycloalkyl, 5-6-membered heteroaryl, -NReRf; wherein, the C3-6 saturated cycloalkyl, -C1-6 alkylene-C3-6 saturated cycloalkyl, 4-7-membered saturated heterocycloalkyl, -C1-6 alkylene-4-7-membered saturated heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-6 alkyl, C3-6 saturated cycloalkyl, or Re and Rf together with the atoms that they are attached to, form 4-7-membered saturated heterocycloalkyl, and the 4-7-membered saturated heterocycloalkyl is optionally substituted by 1 to 3 substituents selected from halogen, C1-3 alkyl.

In some embodiments, each R₁ is independently selected from halogen, hydroxyl, -CN, C1-6 alkyl, C1-6 alkoxy, C1-6 haloalkyl, C1-6 haloalkoxy, C3-6 cycloalkyl, 4-7-membered heterocycloalkyl, 5-6-membered heteroaryl, -NReRf, -C(=O)-NReRf; Re and Rf are each independently selected from H, C1-6 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 substituents selected from halogen, C1-3 alkyl.

In some embodiments, each R₁ is independently selected from halogen, hydroxyl, -CN, C1-3 alkyl, C2-6 alkynyl, C1-3 alkoxy, C1-3 haloalkyl, C3-6 cycloalkyl, -C1-3 alkylene-C3-6 cycloalkyl, 4-7-membered heterocycloalkyl, -C1-3 alkylene-4-7-membered heterocycloalkyl, 5-6-membered heteroaryl, -NReRf, wherein, the C3-6 cycloalkyl, -C1-3 alkylene-C3-6 cycloalkyl, 4-7-membered heterocycloalkyl, -C1-3 alkylene-4-7-membered heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-3 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 halogens.

In some embodiments, each R₁ is independently selected from halogen, hydroxyl, -CN, C1-3 alkyl, C2-6 alkynyl, C1-3 alkoxy, C1-3 haloalkyl, C3-6 cycloalkyl, -C1-3 alkylene-C3-6 cycloalkyl, 4-7-membered heterocycloalkyl, 5-6-membered heteroaryl, -NReRf; wherein, the C3-6 cycloalkyl, -C1-3 alkylene-C3-6 cycloalkyl, 4-7-membered heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-3 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 halogens.

In some embodiments, each R₁ is independently selected from halogen, hydroxyl, -CN, C1-3 alkyl, C2-6 alkynyl, C1-3 alkoxy, C1-3 haloalkyl, C3-6 cycloalkyl, -C1-3 alkylene-C3-6 cycloalkyl, 4-7-membered heterocycloalkyl, -C1-3 alkylene-4-7-membered heterocycloalkyl, 5-6-membered heteroaryl.

In some embodiments, each R₁ is independently selected from F, Cl, -CN, methyl,-CH₂CH₂CH₃, 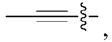 -CHF₂, -CF₃, methoxy, ethoxy, cyclopropyl, 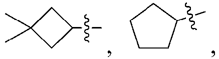 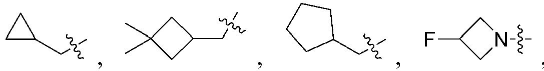 , piperidinyl (for example 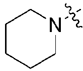 ), morpholinyl (for example 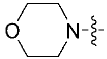 ), 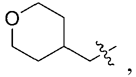 oxazolyl (for example 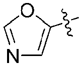 ), pyridinyl (for example 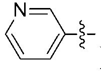 ), -N(CH₃)₂, 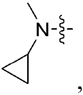 optionally, two R₁ attached to the same carbon atom form C=O with that carbon atom; 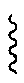 terminus is attached to ring A.

In some embodiments, each R₁ is independently selected from halogen, -CN, C1-3 alkyl, C1-3 alkoxy, C1-3 haloalkyl, C3-6 cycloalkyl, -C1-3 alkylene-C3-6 cycloalkyl, pyridinyl (for example 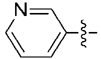 ), -NReRf; Re and Rf are each independently selected from C1-3 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 substituents selected from halogen, C1-3 alkyl; 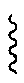 terminus is attached to ring A.

In some embodiments, each R₁ is independently selected from halogen, -CN, C1-3 alkyl, C1-3 haloalkyl, C3-6 cycloalkyl, -C1-3 alkylene-C3-6 cycloalkyl, pyridinyl (for example 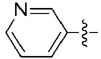 ), -NReRf; Re and Rf are each independently selected from C1-3 alkyl; 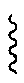 terminus is attached to ring A.

In some embodiments, R₁ is selected from F, -CN, methyl, -CH₂CH₂CH₃, -CF₃, cyclopropyl, 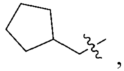 pyridinyl (for example 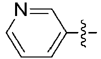 ), -N(CH₃)₂; 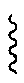 terminus is attached to ring A.

In some embodiments, R₁ is selected from F, -CN, methyl, -CHF₂, -CF₃, methoxy, cyclopropyl, 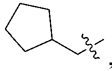 pyridinyl (for example 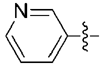 ), -N(CH₃)₂, 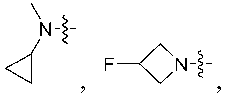 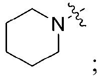 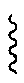 terminus is attached to ring A;

In some embodiments, R₁ is selected from -CF₃, 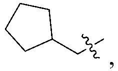 -N(CH₃)₂; 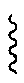 terminus is attached to ring A.

In some embodiments, any of the 4-7-membered heterocycloalkyl groups in R₁ is 4-7-membered saturated heterocycloalkyl.

In some embodiments, the 4-7-membered heterocycloalkyl in the -C1-6 alkylene-4-7-membered heterocycloalkyl or -C1-3 alkylene-4-7-membered heterocycloalkyl in R₁ is 4-7-membered saturated heterocycloalkyl.

In some embodiments, any of the C3-6 cycloalkyl groups in R₁ is C3-6 saturated cycloalkyl.

In some embodiments, the C3-6 cycloalkyl in the -C1-6 alkylene-C3-6 cycloalkyl or -C1-3 alkylene-C3-6 cycloalkyl in R₁ is C3-6 saturated cycloalkyl.

In some embodiments, the 4-7-membered heterocycloalkyl, 4-7-membered saturated heterocycloalkyl, and 5-6-membered heteroaryl in R₁ has 0, 1, 2, or more nitrogen heteroatoms and 0, 1 or 2 heteroatoms selected from O and S.

In some embodiments, the 5-6-membered heteroaryl in R₁ has 1, 2, or 3 nitrogen heteroatoms and 0 or 1 heteroatom selected from O and S, preferably has 1 nitrogen heteroatom and 0 or 1 heteroatom selected from O and S, more preferably has 1 nitrogen heteroatom and 0 or 1 oxygen heteroatom.

In some embodiments, the 4-7-membered heterocycloalkyl in R₁ has 0, 1, or 2 nitrogen heteroatoms and 0 or 1 heteroatom selected from O and S, preferably has 0 or 1 nitrogen heteroatom and 0 or 1 heteroatom selected from O and S, more preferably has 0 or 1 nitrogen heteroatom and 0 or 1 oxygen heteroatom.

In some embodiments, the 4-7-membered saturated heterocycloalkyl in R₁ has 0, 1, or 2 nitrogen heteroatoms and 0 or 1 heteroatom selected from O and S, preferably has 0 or 1 nitrogen heteroatom and 0 or 1 heteroatom selected from O and S, more preferably has 0 or 1 nitrogen heteroatom and 0 or 1 oxygen heteroatom.

In some embodiments, m1 is 0, 1, or 2.

In some embodiments, m1 is 1 or 2.

In some embodiments, m1 is 0 or 1.

In some embodiments, m1 is 1.

In some embodiments, 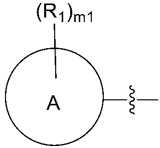 is selected from 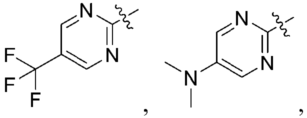 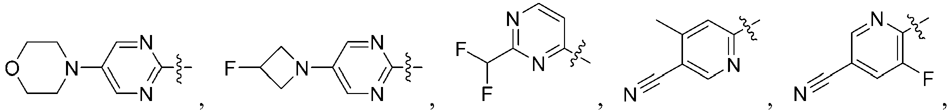 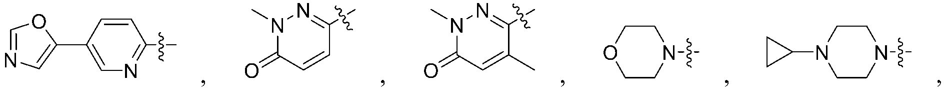 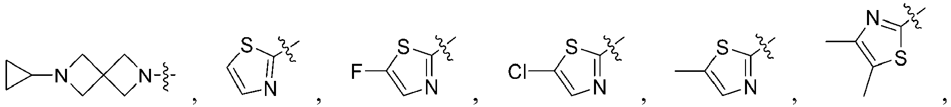 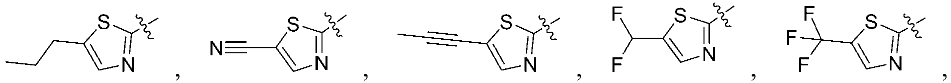 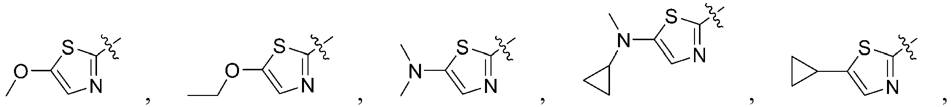 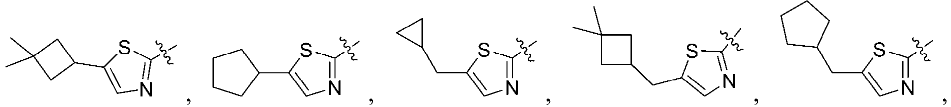 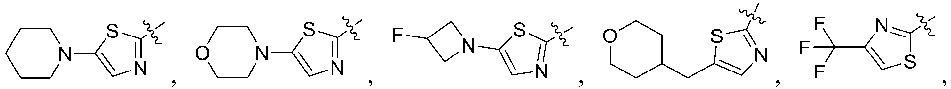 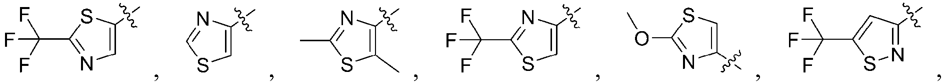 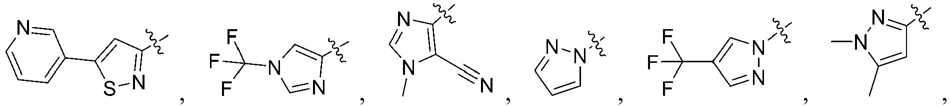 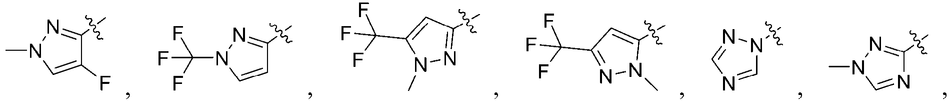 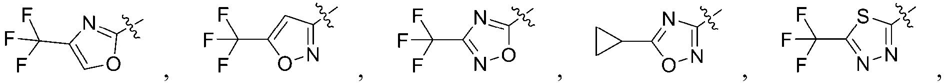 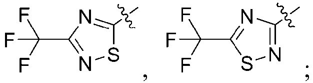 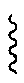 terminus is attached to L₅.

In some embodiments 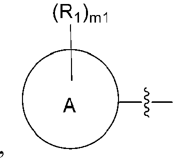 is selected from 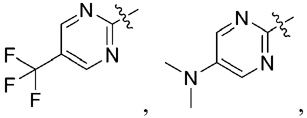 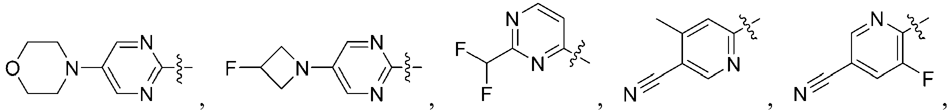 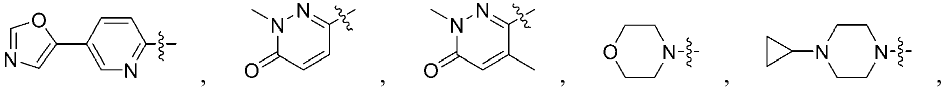 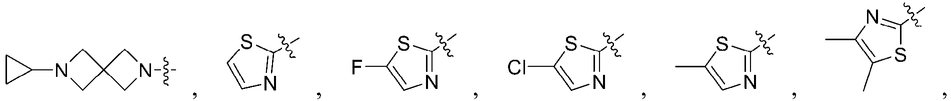 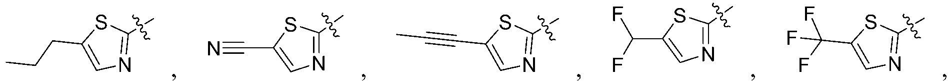 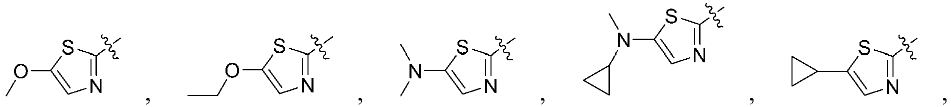 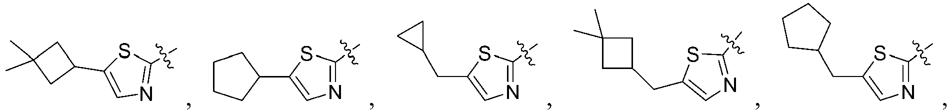 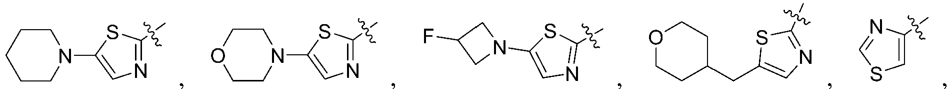 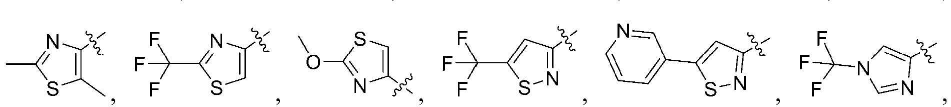 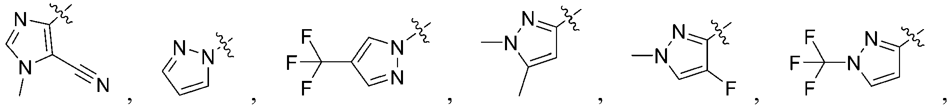 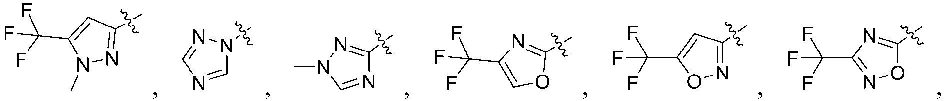 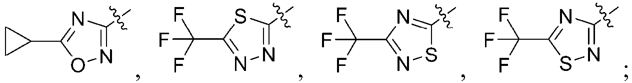 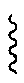 terminus is attached to L₅.

In some embodiments, 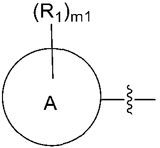 is selected from 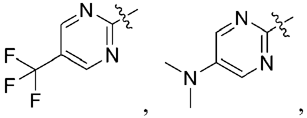 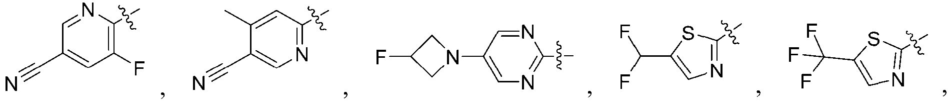 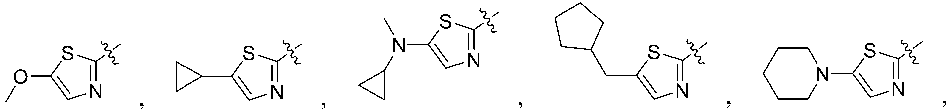 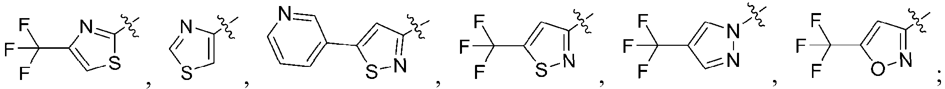 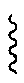 terminus is attached to L₅.

In some embodiments, 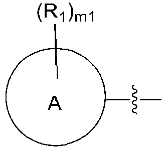 is selected from 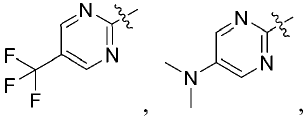 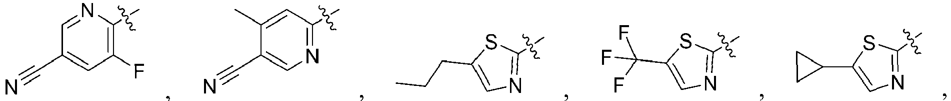 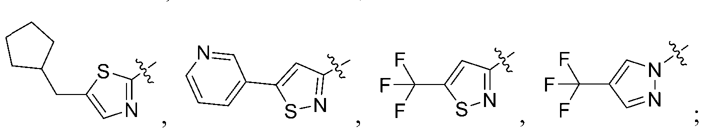 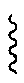 terminus is attached to L₅.

In some embodiments, 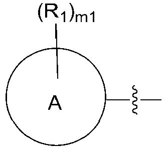 is selected from 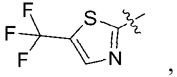 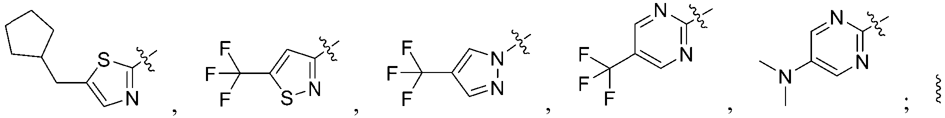 terminus is attached to L₅.

In some embodiments, 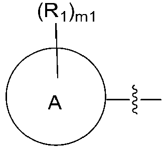 is selected from 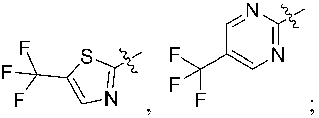 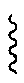 terminus is attached to L₅.

In some embodiments, with the proviso that: 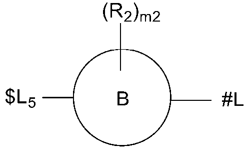 is not group E, the group E is selected from: 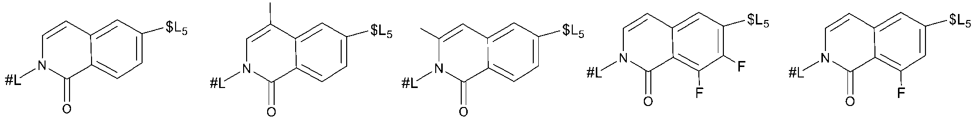 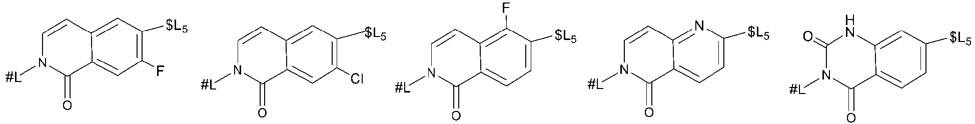 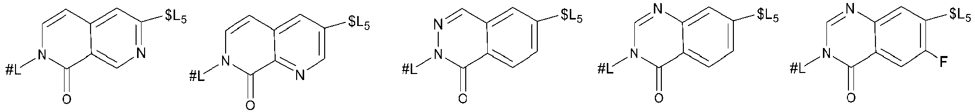 , wherein, $L₅ represents the point of attachment to L₅, #L represents the point of attachment to L.

In some embodiments, with the proviso that: 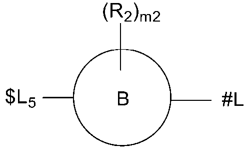 is not group E, the group E is selected from: 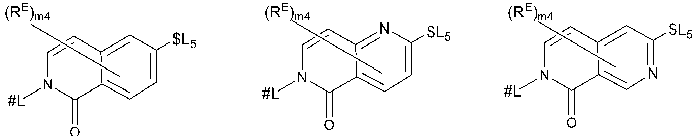 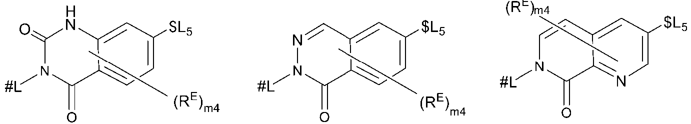 wherein, R^{E} is selected from C1-6 alkyl (preferably methyl), halogen (preferably fluorine, chlorine), m4 is selected from an integer of 0-5, $L₅ represents the point of attachment to L₅, #L represents the point of attachment to L.

In some embodiments, with the proviso that: 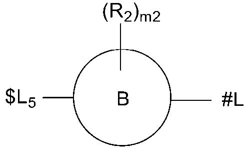 is not group E, the group E is selected from: 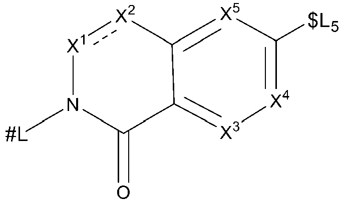
X¹ is selected from N, C=O, C-R^{F1}, C-(R^{F1})₂;
X² is selected from N, N-R^{F2}, C-R^{F3}, C-(R^{F3})₂;
X³ is selected from N, C-R^{F4};
X⁴ is selected from N, C-R^{F4};
X⁵ is selected from N, C-R^{F4};
wherein R^{F1} is selected from H, halogen, CH₃, CH₂F, CHF₂, CF₃, CH₂CF₃, OCH₃, OCF₃, OCHF₂, NO₂, CN, O-R^{F5}, C(O)-R^{F5}, C(O)-N(R^{F6})(R^{F7}), N(R^{F6})(R^{F7}), N(R^{F6})C(O)-R^{F5}, N(R^{F6})C(O)O-R^{F5}, N(R^{F6})S(O)₂(R^{F5}), -N(R^{F6})C(O)-N(R^{F7})(R^{F7}), S(O)₂R^{F5}, -SF₅, S(O)₂N(R^{F6})(R^{F7}), S(O)(NH)R^{F6}, S(O)(NR^{F6})NR^{F7}, C1-9 alkyl, C2-6 alkenyl, C2-6 alkynyl, C3-15 cycloalkyl, C6-10 aryl, 5-10-membered heterocycloalkyl, or 4-12-membered heterocycloalkyl, wherein any alkyl, alkenyl, alkynyl, cycloalkyl, aryl, heterocycloalkyl, or heterocycloalkyl is optionally substituted by one or more R^{F5};
R^{F2} is selected from H, C1-9 alkyl, C2-9 alkenyl, C2-9 alkynyl, C3-12 cycloalkyl, C6-10 aryl, 6-12-membered heteroaryl, or 4-12-membered heterocycloalkyl, wherein any alkyl, alkenyl, alkynyl, cycloalkyl, aryl, heteroaryl, or heterocycloalkyl is optionally substituted by one or more R^{F5};
R^{F3} is selected from H, halogen, CH₃, CH₂F, CHF₂, CF₃, CH₂CF₃, OCH₃, OCF₃, OCHF₂, NO₂, CN, O-R^{F5}, C(O)-R^{F5}, C(O)-N(R^{F6})(R^{F7}), N(R^{F6})(R^{F7}), N(R^{F6})C(O)-R^{F5}, N(R^{F6})C(O)OR^{F5}, N(R^{F6})S(O)₂(R^{F5}), -N(R^{F6})C(O)-N(R^{F7})(R^{F7}), S(O)₂R^{F5}, S(O)₂N(R^{F6}XR^{F7}), S(O)(NH)R^{F6}, S(O)(NR^{F6})NR^{F7}, C1-9 alkyl, C2-9 alkenyl, C2-9 alkynyl, C3-12 cycloalkyl, C6-10 aryl, 5-12-membered heteroaryl, or 4-12-membered heterocycloalkyl, wherein any alkyl, alkenyl, alkynyl, cycloalkyl, aryl, heteroaryl, or heterocycloalkyl is optionally substituted by one or more R^{F5};
R^{F4} is selected from H, halogen, CH₃, CH₂F, CHF₂, CF₃, CH₂CF₃, OCH₃, OCF₃, OCHF₂, NO₂, CN, O-R^{F5}, C(O)-R^{F5}, C(O)-N(R^{F6})(R^{F7}), N(R^{F6})(R^{F7}), N(R^{F6})C(O)-R^{F5}, N(R^{F6})C(O)O-R^{F5}, N(R^{F6})S(O)₂(R^{F5}), -N(R^{F6})C(O)-N(R^{F7})(R^{F7}), -SF₅, S(O)₂R^{F5}, S(O)₂N(R^{F6})(R^{F7}), S(O)(NH)R^{F6}, S(O)(NR^{F6})NR^{F7}, C1-9 alkyl, C2-9 alkenyl, C2-9 alkynyl, C3-12 cycloalkyl, C6-10 aryl, 5-12-membered heteroaryl, or 4-12-membered heterocycloalkyl, wherein any alkyl, alkenyl, alkynyl, cycloalkyl, aryl, heteroaryl, or heterocycloalkyl is optionally substituted by one or more R^{F5};
R^{F5} is independently selected from: H, C=O, halogen, -NO₂, -CN, C1-9 alkyl, C2-6 alkenyl, C2-6 alkynyl, C3-15 cycloalkyl, C1-8 haloalkyl, C6-10 aryl, 5-12-membered heterocyclic aryl, 4-12-membered heterocycloalkyl, -OH, -O(C1-9 alkyl), -O(C2-6 alkenyl), -O(C2-6 alkynyl),-O(C3-15 cycloalkyl), -O(C1-8 haloalkyl), -O(C6-10 aryl), -O(5-12-membered heterocyclic aryl), -O(4-12-membered heterocycloalkyl), -NH₂, -NH(C1-9 alkyl), -NH(C2-6 alkenyl),-NH(C2-6 alkynyl), -NH(C3-15 cycloalkyl), -NH(C1-8 haloalkyl), -NH(C6-10 aryl), -NH(5-12-membered heterocyclic aryl), -NH(4-12-membered heterocycloalkyl), -N(C1-9 alkyl)₂, -N(C3-15 cycloalkyl)₂, -N(C2-6 alkenyl)₂, -N(C2-6 alkynyl)₂, -N(C3-15 cycloalkyl)₂, -N(C1-8 haloalkyl)₂, -N(C6-10 aryl)₂, -N(5-12-membered heterocyclic aryl)₂, -N(4-12-membered heterocycloalkyl)₂, -N(C1-9 alkyl)(C3-15 cycloalkyl), -N(C1-9 alkyl)(C2-6 alkenyl), -N(C1-9 alkyl)(C2-6 alkynyl), -N(C1-9 alkyl)(C3-15 cycloalkyl), -N(C1-9 alkyl)(C1-8 haloalkyl), - N(C1-9 alkyl)(C6-10 aryl), -N(C1-9 alkyl)(5-12-membered heterocyclic aryl), -N(C1-9 alkyl)(4-12-membered heterocycloalkyl), -C(O)(C1-9 alkyl), -C(O)(C2-6 alkenyl), -C(O)(C2-6 alkynyl), -C(O)(C3-15 cycloalkyl), -C(O)(C1-8 haloalkyl), -C(O)(C6-10 aryl), -C(O)(5-12-membered heterocyclic aryl), -C(O)(4-12-membered heterocycloalkyl), -C(O)O(C1-9 alkyl), - C(O)O(C2-6 alkenyl), -C(O)O(C2-6 alkynyl), -C(O)O(C3-15 cycloalkyl), -C(O)O(C1-8 haloalkyl), -C(O)O(C6-10 aryl), -C(O)O(5-12-membered heterocyclic aryl), -C(O)O(4-12-membered heterocycloalkyl), -C(O)NH₂, -C(O)NH(C1-9 alkyl), -C(O)NH(C2-6 alkenyl),-C(O)NH(C2-6 alkynyl), -C(O)NH(C3-15 cycloalkyl), -C(O)NH(C1-8 haloalkyl),-C(O)NH(C6-10 aryl), -C(O)NH(5-12-membered heterocyclic aryl), -C(O)NH(4-12-membered heterocycloalkyl), -C(O)N(C1-9 alkyl)₂, -C(O)N(C3-15 cycloalkyl)₂, -C(O)N(C2-6 alkenyl)₂, -C(O)N(C2-6 alkynyl)₂, -C(O)N(C3-15 cycloalkyl)₂, -C(O)N(C1-8 haloalkyl)₂, -C(O)N(C6-10 aryl)₂, -C(O)N(5-12-membered heterocyclic aryl)₂, -C(O)N(4-12-membered heterocycloalkyl)₂, -NHC(O)(C1-9 alkyl), -NHC(O)(C2-6 alkenyl), -NHC(O)(C2-6 alkynyl), -NHC(O)(C3-15 cycloalkyl), -NHC(O)(C1-8 haloalkyl), -NHC(O)(C6-10 aryl), -NHC(O)(5-12-membered heterocyclic aryl), -NHC(O)(4-12-membered heterocycloalkyl), -NHC(O)O(C1-9 alkyl), - NHC(O)O(C2-6 alkenyl), -NHC(O)O(C2-6 alkynyl), -NHC(O)O(C3-15 cycloalkyl), - NHC(O)O(C1-8 haloalkyl), -NHC(O)O(C6-10 aryl), -NHC(O)O(5-12-membered heterocyclic aryl), -NHC(O)O(4-12-membered heterocycloalkyl), -NHC(O)NH(C1-9 alkyl), - NHC(O)NH(C2-6 alkenyl), -NHC(O)NH(C2-6 alkynyl), -NHC(O)NH(C3-15 cycloalkyl), - NHC(O)NH(C1-8 haloalkyl), -NHC(O)NH(C6-10 aryl), -NHC(O)NH(5-12-membered heterocyclic aryl), -NHC(O)NH(4-12-membered heterocycloalkyl), -SH, -S(C1-9 alkyl), - S(C2-6 alkenyl), -S(C2-6 alkynyl), -S(C3-15 cycloalkyl), -S(C1-8 haloalkyl), -S(C6-10 aryl),-S(5-12-membered heterocyclic aryl), -S(4-12-membered heterocycloalkyl), -NHS(O)(C1-9 alkyl), -N(C1-9 alkyl)(S(O)(C1-9 alkyl), -S(O)N(C1-9 alkyl)₂, -S(O)(C1-9 alkyl),-S(O)(NH)(C1-9 alkyl), -S(O)(NH)(C3-9 cycloalkyl), -S(O)(NC1-9 alkyl)(C1-9 alkyl),-S(O)(NH)(C6-10 aryl), -S(O)(NH)(5-12-membered heterocyclic aryl), -S(O)(C2-6 alkenyl),-S(O)(C2-6 alkynyl), -S(O)(C3-15 cycloalkyl), -S(O)(C1-8 haloalkyl), -S(O)(C6-10 aryl),-S(O)(5-12-membered heterocyclic aryl), -S(O)(4-12-membered heterocycloalkyl), -S(O)₂(C1-9 alkyl), -S(O)₂(C2-6 alkenyl), -S(O)₂(C2-6 alkynyl), -S(O)₂(C3-15 cycloalkyl), -S(O)₂(C1-8 haloalkyl), -S(O)₂(C6-10 aryl), -S(O)₂(5-12-membered heterocyclic aryl), -S(O)₂(4-12-membered heterocycloalkyl), -S(O)₂NH(C1-9 alkyl), or -S(O)₂N(C1-9 alkyl)₂; wherein any alkyl, cycloalkyl, aryl, heteroaryl, or heterocycloalkyl is optionally substituted by one or more of the following: halogen, C1-9 alkyl, C1-8 haloalkyl, -OH, -NH₂, -NH(C1-9 alkyl), -NH(C3-15 cycloalkyl), -NH(C1-8 haloalkyl), -NH(C6-10 aryl), -NH(5-12-membered heterocyclic aryl), -NH(4-12-membered heterocycloalkyl), -N(C1-9 alkyl)₂, -N(C3-15 cycloalkyl)₂,-NHC(O)(C3-15 cycloalkyl), -NHC(O)(C1-8 haloalkyl), -NHC(O)(C6-10 aryl), -NHC(O)(5-12-membered heterocyclic aryl), -NHC(O)(4-12-membered heterocycloalkyl), -NHC(O)O(C1-9 alkyl), -NHC(O)O(C2-6 alkynyl), -NHC(O)O(C3-15 cycloalkyl), -NHC(O)O(C1-8 haloalkyl), -NHC(O)O(C6-10 aryl), -NHC(O)O(5-12-membered heterocyclic aryl),-NHC(O)O(4-12-membered heterocycloalkyl), -NHC(O)NH(C1-9 alkyl), -S(O)(NH)(C1-9 alkyl), S(O)₂(C1-9 alkyl), -S(O)₂(C3-15 cycloalkyl), -S(O)₂(C1-8 haloalkyl), -S(O)₂(C6-10 aryl), -S(O)₂(5-12-membered heterocyclic aryl), -S(O)₂(4-12-membered heterocycloalkyl),-S(O)₂NH(C1-9 alkyl), -S(O)₂N(C1-9 alkyl)₂, -O(C3-15 cycloalkyl), -O(C1-8 haloalkyl), - O(C6-10 aryl), -O(5-12-membered heterocyclic aryl), -O(4-12-membered heterocycloalkyl), or -O(C1-9 alkyl); and
R^{F6} and R^{F7} are independently selected from: H, C1-9 alkyl, C2-6 alkenyl, C2-6 alkynyl, C3-15 cycloalkyl, C6-10 aryl, 5-12-membered heterocyclic aryl, or 4-12-membered heterocycloalkyl, wherein any alkyl, alkenyl, alkynyl, cycloalkyl, aryl, heteroaryl, or heterocycloalkyl is optionally substituted by one or more R^{F5};
wherein, $L₅ represents the point of attachment to L₅, #L represents the point of attachment to L.

In some embodiments, especially in preferred embodiments, with the proviso that: 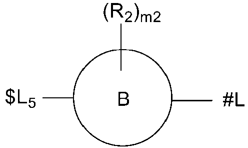 is not group E, the group E is selected from: 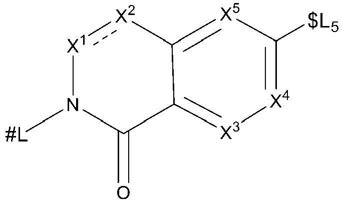
X¹ is selected from N, C=O, C-R^{F1}, C-(R^{F1})₂;
X² is selected from N, N-R^{F2}, C-R^{F3}, C-(R^{F3})₂;
X³ is selected from N, C-R^{F4};
X⁴ is selected from N, C-R^{F4};
X⁵ is selected from N, C-R^{F4};
wherein R^{F1} R^{F3}, and R^{F4} are each independently selected from H, halogen, CH₃, CH₂F, CHF₂, CF₃, CH₂CF₃, OCH₃, OCF₃, OCHF₂, NO₂, CN, O-R^{F5}, C(O)-R^{F5}, C(O)-N(R^{F6})(R^{F7}), N(R^{F6})(R^{F7}), N(R^{F6})C(O)-R^{F5}, N(R^{F6})C(O)O-R^{F5}, N(R^{F6})S(O)₂(R^{F5}), -N(R^{F6})C(O)-N(R^{F7})(R^{F7}), S(O)₂R^{F5}, -SF₅, S(O)₂N(R^{F6})(R^{F7}), S(O)(NH)R^{F6}, S(O)(NR^{F6})NR^{F7}, C1-9 alkyl, C2-9 alkenyl, C2-9 alkynyl, C3-15 cycloalkyl, C6-10 aryl, 5-12-membered heteroaryl, or 4-12-membered heterocycloalkyl, wherein any alkyl, alkenyl, alkynyl, cycloalkyl, aryl, heteroaryl, or heterocycloalkyl is optionally substituted by one or more R^{F5};
R^{F2}, R^{F6}, R^{F7}are each independently selected from H, C1-9 alkyl, C2-9 alkenyl, C2-9 alkynyl, C3-15 cycloalkyl, C6-10 aryl, 5-12-membered heteroaryl, or 4-12-membered heterocycloalkyl, wherein any alkyl, alkenyl, alkynyl, cycloalkyl, aryl, heteroaryl, or heterocycloalkyl is optionally substituted by one or more R^{F5};
R^{F5} is independently selected from: H, C=O, halogen, -NO₂, -CN, C1-9 alkyl, C2-6 alkenyl, C2-6 alkynyl, C3-15 cycloalkyl, C1-8 haloalkyl, C6-10 aryl, 5-12-membered heteroaryl, 4-12-membered heterocycloalkyl, -OH, -O(C1-9 alkyl), -O(C2-6 alkenyl), -O(C2-6 alkynyl), -O(C3-15 cycloalkyl), -O(C1-8 haloalkyl), -O(C6-10 aryl), -O(5-12-membered heteroaryl), -O(4-12-membered heterocycloalkyl), -NH₂, -NH(C1-9 alkyl), -NH(C2-6 alkenyl), -NH(C2-6 alkynyl), -NH(C3-15 cycloalkyl), -NH(C1-8 haloalkyl), -NH(C6-10 aryl), -NH(5-12-membered heteroaryl), -NH(4-12-membered heterocycloalkyl), -N(C1-9 alkyl)₂, -N(C3-15 cycloalkyl)₂,-N(C2-6 alkenyl)₂, -N(C2-6 alkynyl)₂, -N(C3-15 cycloalkyl)₂, -N(C1-8 haloalkyl)₂, -N(C6-10 aryl)₂, -N(5-12-membered heteroaryl)₂, -N(4-12-membered heterocycloalkyl)₂, -N(C1-9 alkyl)(C3-15 cycloalkyl), -N(C1-9 alkyl)(C2-6 alkenyl), -N(C1-9 alkyl)(C2-6 alkynyl), -N(C1-9 alkyl)(C3-15 cycloalkyl), -N(C1-9 alkyl)(C1-8 haloalkyl), -N(C1-9 alkyl)(C6-10 aryl),-N(C1-9 alkyl)(5-12-membered heteroaryl), -N(C1-9 alkyl)(4-12-membered heterocycloalkyl), -C(O)(C1-9 alkyl), -C(O)(C2-6 alkenyl), -C(O)(C2-6 alkynyl), -C(O)(C3-15 cycloalkyl),-C(O)(C1-8 haloalkyl), -C(O)(C6-10 aryl), -C(O)(5-12-membered heteroaryl), -C(O)(4-12-membered heterocycloalkyl), -C(O)O(C1-9 alkyl), -C(O)O(C2-6 alkenyl), -C(O)O(C2-6 alkynyl), -C(O)O(C3-15 cycloalkyl), -C(O)O(C1-8 haloalkyl), -C(O)O(C6-10 aryl), -C(O)O(5-12-membered heteroaryl), -C(O)O(4-12-membered heterocycloalkyl), -C(O)NH₂,-C(O)NH(C1-9 alkyl), -C(O)NH(C2-6 alkenyl), -C(O)NH(C2-6 alkynyl), -C(O)NH(C3-15 cycloalkyl), -C(O)NH(C1-8 haloalkyl), -C(O)NH(C6-10 aryl), -C(O)NH(5-12-membered heteroaryl), -C(O)NH(4-12-membered heterocycloalkyl), -C(O)N(C1-9 alkyl)₂, -C(O)N(C3-15 cycloalkyl)₂, -C(O)N(C2-6 alkenyl)₂, -C(O)N(C2-6 alkynyl)₂, -C(O)N(C3-15 cycloalkyl)₂,-C(O)N(C1-8 haloalkyl)₂, -C(O)N(C6-10 aryl)₂, -C(O)N(5-12-membered heteroaryl)₂,-C(O)N(4-12-membered heterocycloalkyl)₂, -NHC(O)(C1-9 alkyl), -NHC(O)(C2-6 alkenyl),-NHC(O)(C2-6 alkynyl), -NHC(O)(C3-15 cycloalkyl), -NHC(O)(C1-8 haloalkyl),-NHC(O)(C6-10 aryl), -NHC(O)(5-12-membered heteroaryl), -NHC(O)(4-12-membered heterocycloalkyl), -NHC(O)O(C1-9 alkyl), -NHC(O)O(C2-6 alkenyl), -NHC(O)O(C2-6 alkynyl), -NHC(O)O(C3-15 cycloalkyl), -NHC(O)O(C1-8 haloalkyl), -NHC(O)O(C6-10 aryl), -NHC(O)O(5-12-membered heteroaryl), -NHC(O)O(4-12-membered heterocycloalkyl),-NHC(O)NH(C1-9 alkyl), -NHC(O)NH(C2-6 alkenyl), -NHC(O)NH(C2-6 alkynyl),-NHC(O)NH(C3-15 cycloalkyl), -NHC(O)NH(C1-8 haloalkyl), -NHC(O)NH(C6-10 aryl),-NHC(O)NH(5-12-membered heteroaryl), -NHC(O)NH(4-12-membered heterocycloalkyl),-SH, -S(C1-9 alkyl), -S(C2-6 alkenyl), -S(C2-6 alkynyl), -S(C3-15 cycloalkyl), -S(C1-8 haloalkyl), -S(C6-10 aryl), -S(5-12-membered heteroaryl), -S(4-12-membered heterocycloalkyl), -NHS(O)(C1-9 alkyl), -N(C1-9 alkyl)(S(O)(C1-9 alkyl), -S(O)N(C1-9 alkyl)₂, -S(O)(C1-9 alkyl), -S(O)(NH)(C1-9 alkyl), -S(O)(NH)(C3-9 cycloalkyl), -S(O)(NC1-9 alkyl)(C1-9 alkyl), -S(O)(NH)(C6-10 aryl), -S(O)(NH)(5-12-membered heteroaryl), - S(O)(C2-6 alkenyl), -S(O)(C2-6 alkynyl), -S(O)(C3-15 cycloalkyl), -S(O)(C1-8 haloalkyl),-S(O)(C6-10 aryl), -S(O)(5-12-membered heteroaryl), -S(O)(4-12-membered heterocycloalkyl), -S(O)₂(C1-9 alkyl), -S(O)₂(C2-6 alkenyl), -S(O)₂(C2-6 alkynyl), -S(O)₂(C3-15 cycloalkyl),-S(O)₂(C1-8 haloalkyl), -S(O)₂(C6-10 aryl), -S(O)₂(5-12-membered heteroaryl), -S(O)₂(4-12-membered heterocycloalkyl), -S(O)₂NH(C1-9 alkyl), or -S(O)₂N(C1-9 alkyl)₂; wherein any alkyl, cycloalkyl, aryl, heteroaryl, or heterocycloalkyl is optionally substituted by one or more of the following: halogen, C1-9 alkyl, C1-8 haloalkyl, -OH, -NH₂, -NH(C1-9 alkyl), -NH(C3-15 cycloalkyl), -NH(C1-8 haloalkyl), -NH(C6-10 aryl), -NH(5-12-membered heteroaryl),-NH(4-12-membered heterocycloalkyl), -N(C1-9 alkyl)₂, -N(C3-15 cycloalkyl)₂, -NHC(O)(C3-15 cycloalkyl), -NHC(O)(C1-8 haloalkyl), -NHC(O)(C6-10 aryl), -NHC(O)(5-12-membered heteroaryl), -NHC(O)(4-12-membered heterocycloalkyl), -NHC(O)O(C1-9 alkyl),-NHC(O)O(C2-6 alkynyl), -NHC(O)O(C3-15 cycloalkyl), -NHC(O)O(C1-8 haloalkyl),-NHC(O)O(C6-10 aryl), -NHC(O)O(5-12-membered heteroaryl), -NHC(O)O(4-12-membered heterocycloalkyl), -NHC(O)NH(C1-9 alkyl), -S(O)(NH)(C1-9 alkyl), S(O)₂(C1-9 alkyl),-S(O)₂(C3-15 cycloalkyl), -S(O)₂(C1-8 haloalkyl), -S(O)₂(C6-10 aryl), -S(O)₂(5-12-membered heteroaryl), -S(O)₂(4-12-membered heterocycloalkyl), -S(O)₂NH(C1-9 alkyl), -S(O)₂N(C1-9 alkyl)₂, -O(C3-15 cycloalkyl), -O(C1-8 haloalkyl), -O(C6-10 aryl), -O(5-12-membered heteroaryl), -O(4-12-membered heterocycloalkyl), or -O(C1-9 alkyl);
wherein, $L₅ represents the point of attachment to L₅, #L represents the point of attachment to L.

In some embodiments, ring B is selected from phenyl, 5-6-membered heteroaryl, 5-6-membered heterocycloalkyl-fused-5-6-membered heterocycloalkyl, benzo-fused-5-6-membered heteroaryl, benzo-fused-5-6-membered heterocycloalkyl, 5-6-membered heteroaryl-fused-5-6-membered heterocycloalkyl.

In some embodiments, ring B is selected from phenyl, 5-6-membered heteroaryl, 5-6-membered heterocycloalkyl-fused-5-6-membered heterocycloalkyl, benzo-fused-5-6-membered heteroaryl, benzo-fused-5-6-membered heterocycloalkyl, 5-6-membered heteroaryl-fused-5-6-membered heterocycloalkyl ; wherein any of the heteroaryl groups each has 1, 2, or 3 nitrogen heteroatoms and 0 or 1 heteroatom selected from O and S, any of the heterocycloalkyl groups each has 1 or 2 nitrogen heteroatoms and 0 or 1 heteroatom selected from O and S, preferably has 1 or 2 nitrogen heteroatoms. In some embodiments, ring B is selected from phenyl, 5-6-membered heteroaryl, benzo-fused-5-6-membered heteroaryl, benzo-fused-5-6-membered saturated heterocycloalkyl, benzo-fused-5-6-membered partially unsaturated heterocycloalkyl, 5-6-membered heteroaryl-fused-5-6-membered saturated heterocycloalkyl; wherein any of the heteroaryl groups each has 1, 2, or 3 nitrogen heteroatoms and 0 or 1 heteroatom selected from O and S, any of the heterocycloalkyl groups each has 1 or 2 nitrogen heteroatoms and 0 or 1 heteroatom selected from O and S, preferably has 1 or 2 nitrogen heteroatoms.

In some embodiments, ring B is selected from phenyl, 5-6-membered heteroaryl, 5-6-membered heterocycloalkyl-fused-5-6-membered heterocycloalkyl, benzo-fused-5-6-membered heterocycloalkyl, 5-6-membered heteroaryl-fused-5-6-membered heterocycloalkyl.

In some embodiments, ring B is selected from phenyl, 5-6-membered heteroaryl, 5-6-membered heterocycloalkyl-fused-5-6-membered heterocycloalkyl, benzo-fused-5-6-membered heterocycloalkyl, 5-6-membered heteroaryl-fused-5-6-membered heterocycloalkyl; the heteroaryl has 1, 2, or 3 nitrogen heteroatoms and 0 or 1 heteroatom selected from O and S, the heterocycloalkyl has 1 or 2 nitrogen heteroatoms and 0 or 1 heteroatom selected from O and S, preferably has 1 or 2 nitrogen heteroatoms.

In some embodiments, ring B is selected from phenyl, pyridinyl, pyrazolyl, pyrazinyl, oxazolyl, isoxazolyl, benzopyrazolyl, thiazolyl, isothiazolyl, pyridazinyl, pyrimidinyl, triazolyl, thiadiazolyl, indolinyl, benzimidazolyl, dihydrobenzimidazolyl, indazolyl, isoindolinyl, pyridopyridinyl, pyrrolopyrimidinyl.

In some embodiments, ring B is selected from phenyl, pyrimidinyl, pyridinyl, pyrazinyl, pyridazinyl, pyrazolyl, thiazolyl, isothiazolyl, isoxazolyl, triazolyl (for example 1,2,3-triazolyl), thiadiazolyl (for example 1,3,4-thiadiazolyl), 5-membered nitrogen-containing heterocycloalkyl-fused-phenyl, phenyl-fused-pyrazolyl, phenyl-fused-5-membered nitrogen-containing heterocycloalkyl (for example phenyl-fused-tetrahydropyrrole), pyrimidinyl-fused-5-membered nitrogen-containing heterocycloalkyl, 6-membered nitrogen-containing heterocycloalkyl-fused-5-membered nitrogen-containing heterocycloalkyl, 6-membered nitrogen-containing heterocycloalkyl-fused-pyrazolyl.

In some embodiments, ring B is selected from phenyl, pyrimidinyl, pyridinyl, pyrazinyl, pyridazinyl, pyrazolyl, thiazolyl, isothiazolyl, isoxazolyl, triazolyl (for example 1,2,3-triazolyl), thiadiazolyl (for example 1,3,4-thiadiazolyl), 5-membered nitrogen-containing heterocycloalkyl-fused-phenyl, phenyl-fused-5-membered nitrogen-containing heterocycloalkyl, pyrimidinyl-fused-5-membered nitrogen-containing heterocycloalkyl, 6-membered nitrogen-containing heterocycloalkyl-fused-5-membered nitrogen-containing heterocycloalkyl.

In some embodiments, ring B is selected from phenyl, 5-6-membered heteroaryl, benzo-fused-5-6-membered heteroaryl.

In some embodiments, ring B is selected from phenyl, 5-6-membered heteroaryl, benzo-fused-5-6-membered heteroaryl, the heteroaryl has 1, 2, or 3 nitrogen heteroatoms and 0 or 1 heteroatom selected from O and S, preferably has 1 or 2 nitrogen heteroatoms and 0 or 1 heteroatom selected from O or S, more preferably has 1 or 2 nitrogen heteroatoms.

In some embodiments, ring B is selected from phenyl, 5-6-membered heteroaryl.

In some embodiments, ring B is selected from phenyl, 5-6-membered heteroaryl, the heteroaryl has 1, 2, or 3 nitrogen heteroatoms and 0 or 1 heteroatom selected from O and S, preferably has 1 or 2 nitrogen heteroatoms and 0 or 1 heteroatom selected from O or S, more preferably has 1 or 2 nitrogen heteroatoms.

In some embodiments, ring B is selected from phenyl, 6-membered heteroaryl.

In some embodiments, ring B is selected from phenyl, 6-membered heteroaryl, the heteroaryl has 1, 2, or 3 nitrogen heteroatoms and 0 or 1 heteroatom selected from O and S, preferably has 1 or 2 nitrogen heteroatoms and 0 or 1 heteroatom selected from O or S, more preferably has 1 or 2 nitrogen heteroatoms.

In some embodiments, ring B is selected from phenyl, pyrimidinyl, pyridinyl, pyrazinyl, pyridazinyl, pyrazolyl, thiazolyl, isothiazolyl, isoxazolyl, triazolyl (for example 1,2,3-triazolyl), thiadiazolyl (for example 1,3,4-thiadiazolyl).

In some embodiments, ring B is selected from phenyl, pyridinyl, pyrazinyl, isoxazolyl.

In some embodiments, ring B is selected from phenyl, pyridinyl, pyrazinyl.

In some embodiments, ring B is selected from the following groups: 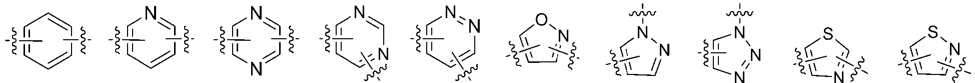 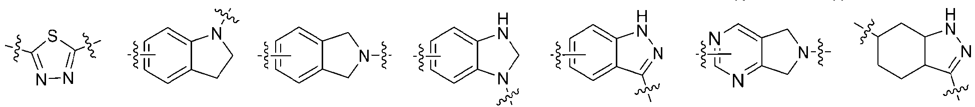 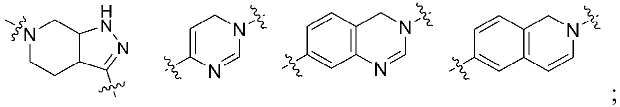 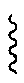 terminus is attached to L terminus or L₅.

In some embodiments, ring B is selected from the following groups: 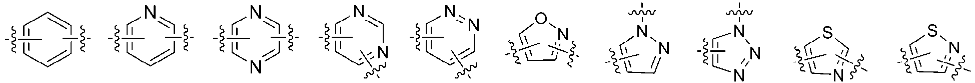 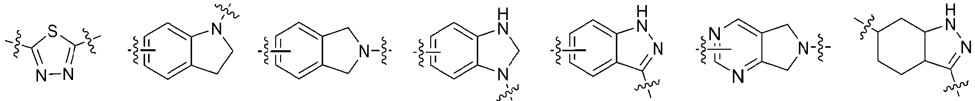 ; 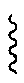 terminus is attached to L terminus or L₅.

In some embodiments, ring B is selected from 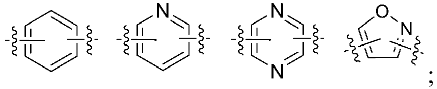 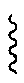 terminus is attached to L terminus or L₅.

In some embodiments, ring B is selected from 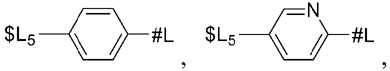 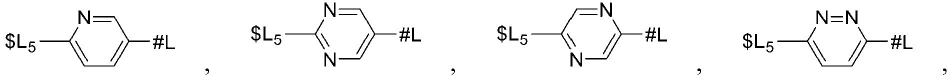 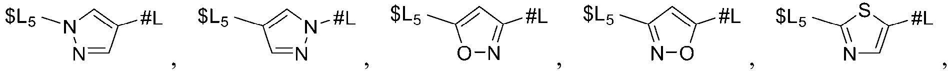 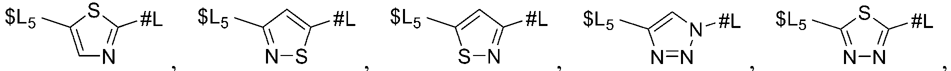 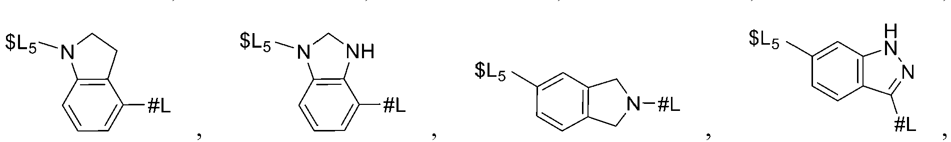 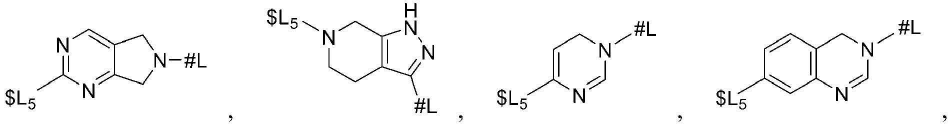 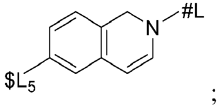 $L₅ represents the site of attachment to L₅, #L represents the site of attachment to L.

In some embodiments, ring B is selected from 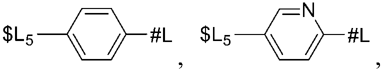 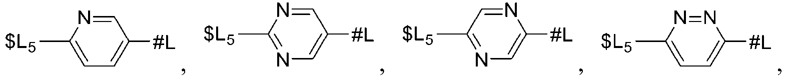 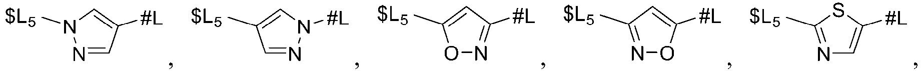 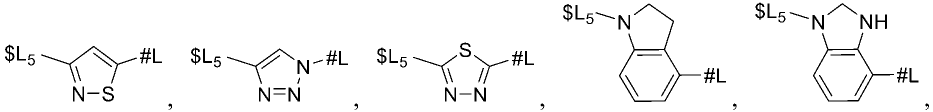 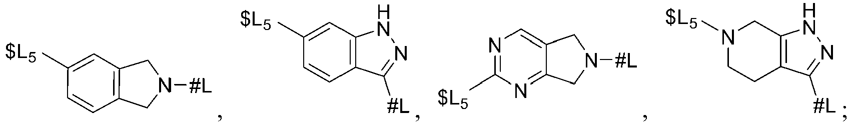 $L₅ represents the site of attachment to L₅, #L represents the site 0 of attachment to L.

In some embodiments, ring B is selected from 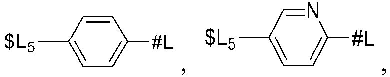 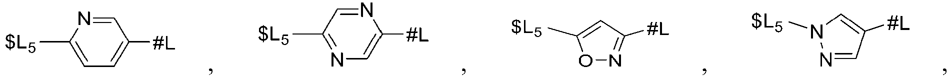 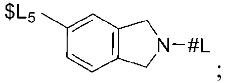 $L₅ represents the site of attachment to L₅, #L represents the site of attachment to L.

In some embodiments, ring B is selected from

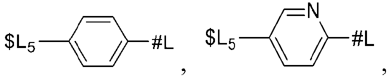

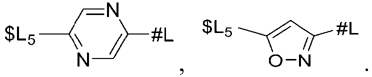

In some embodiments, ring B is selected from 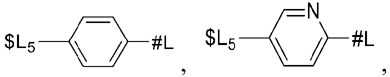 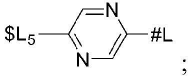 $L₅ represents the site of attachment to L₅, #L represents the site of attachment to L.

In some embodiments, R₂ is selected from halogen, hydroxyl, C1-3 alkyl, C1-3 haloalkyl, C1-3 alkoxy, C1-3 haloalkoxy, C3-4 cycloalkyl, -C1-2 alkylene-C3-4 cycloalkyl, -NReRf; Re and Rf are each independently selected from H and C1-3 alkyl; optionally, two R₂ attached to the same carbon atom form C=O with that carbon atom.

In some embodiments, R₂ is selected from F, Cl, hydroxyl, amino, methyl; optionally, two R₂ attached to the same carbon atom form C=O with that carbon atom; preferably, R₂ is F.

In some embodiments, R₂ is selected from F or hydroxyl.

In some embodiments, m2 is an integer of 0-2; preferably, m2 is 0 or 1; preferably, m2 is 0.

In some embodiments, 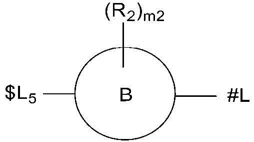 is selected from 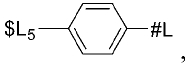 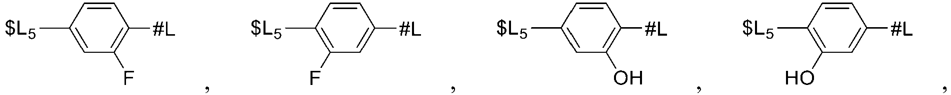 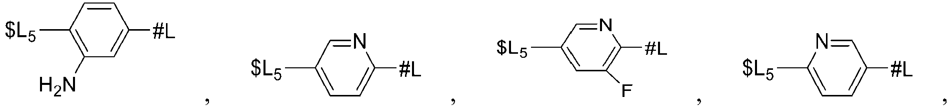 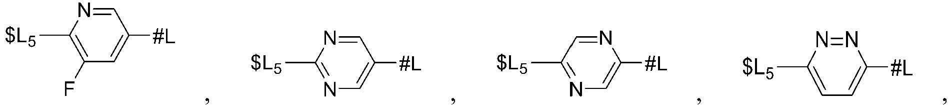 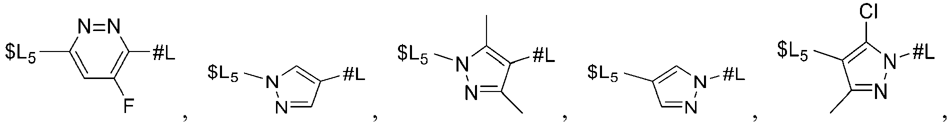 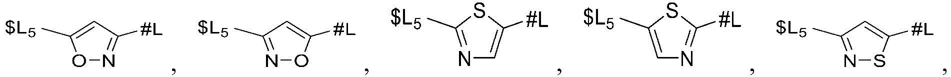 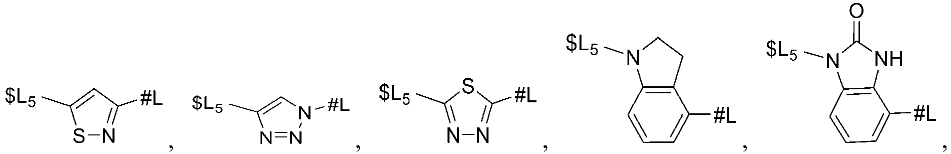 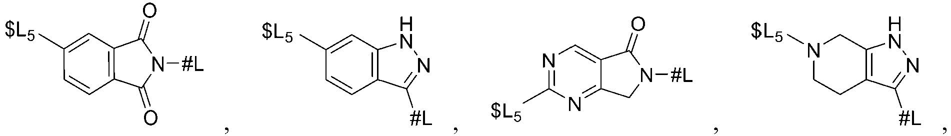 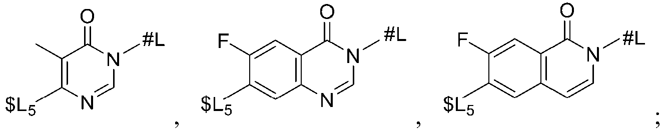 $L₅ represents the site of attachment to L₅, #L represents the site of attachment to L.

In some embodiments, 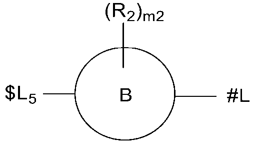 is selected from 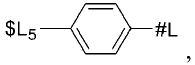 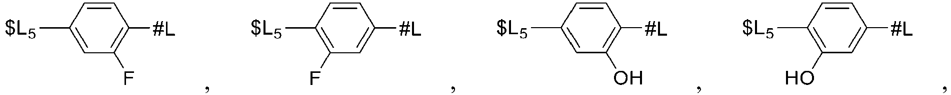 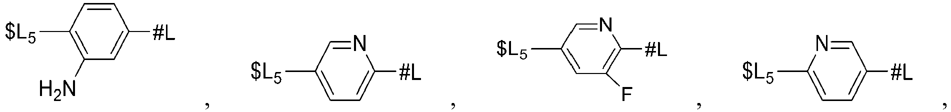 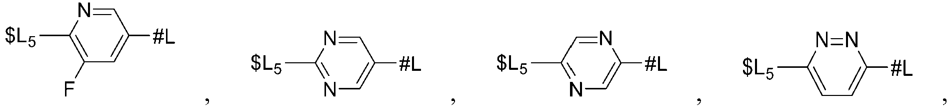 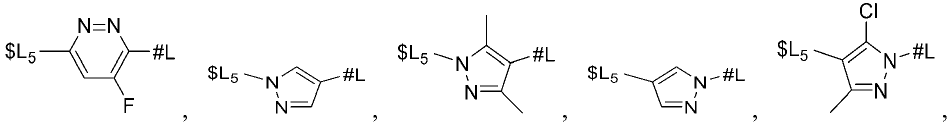 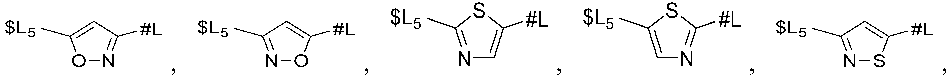 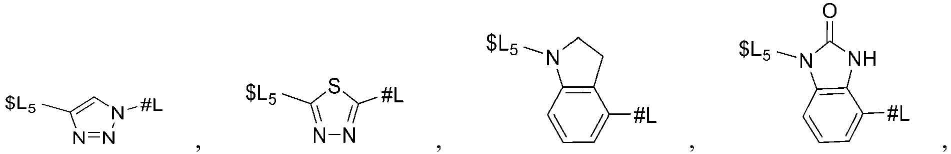 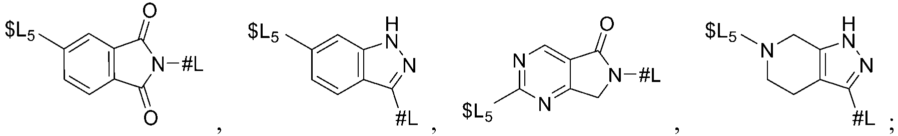 $L₅ represents the site of attachment to L₅, #L represents the site of attachment to L.

In some embodiments, 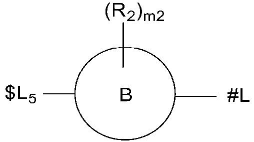 is selected from 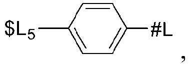 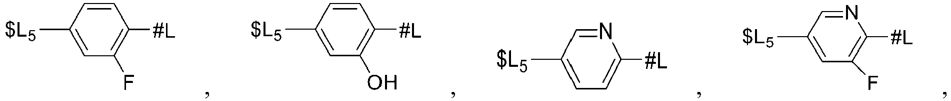 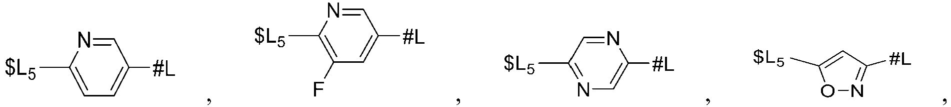 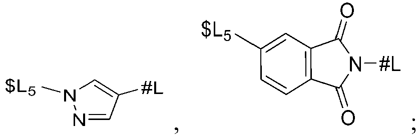 $L₅ represents the site of attachment to L₅, #L represents the site of attachment to L.

In some embodiments, 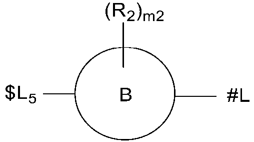 is selected from 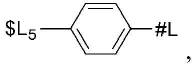 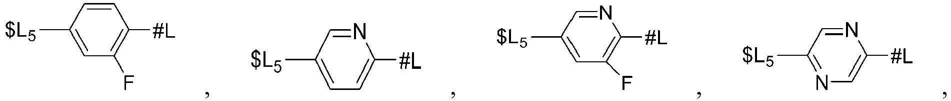 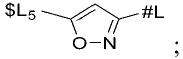 $L₅ represents the site of attachment to L₅, #L represents the site of attachment to L.

In some embodiments, 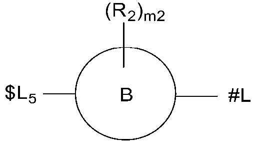 is selected from 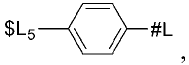 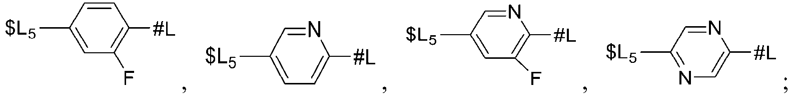 $L₅ represents the site of attachment to L₅, #L represents the site of attachment to L.

In some embodiments, 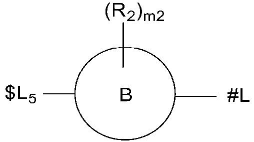 is selected from 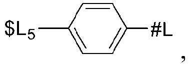 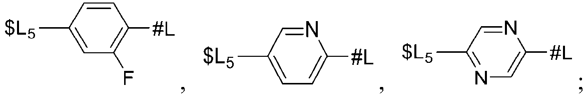 $L₅ represents the site of attachment to L₅, #L represents the site of attachment to L.

In some embodiments, L is the linking group represented by formula L: $B-L1-L2-X1-L3-L4-#C (L) ;
wherein, $B represents the site of attachment to ring B, #C represents the site of attachment to ring C;
wherein, L1 is selected from a bond, -C(=O)-, $B-C(=O)-NRa-#L2, -NRb-, $B-NRb-C(=O)-#L2, 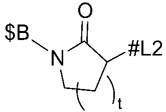 t is 1, 2, or 3, the 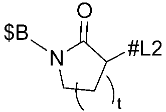 is optionally substituted by a substituent selected from halogen, hydroxyl, cyano, C1-6 alkyl, C1-6 alkoxy, C1-6 haloalkyl, C1-6 haloalkoxy; preferably, L1 is selected from a bond, -C(=O)-, $B-C(=O)NH-#L2, $B-C(=O)N(CH₃)-#L2, $B-NH(C=O)-#L2, -NH-, 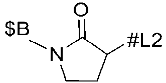 (for example 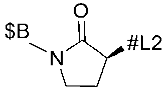 ); further preferably, L1 is selected from -C(=O)-, $B-C(=O)NH-#L2, $B-NH(C=O)-#L2; more preferably, L1 is selected from $B-C(=O)NH-#L2, $B-NH(C=O)-#L2;
Ra and Rb are each independently selected from H, D, C1-6 alkyl, C1-6 haloalkyl, C3-6 cycloalkyl, 4-7-membered heterocycloalkyl; $B represents the site of attachment to ring B, #L2 represents the site of attachment to L2; preferably, Ra and Rb are each independently selected from H, C1-6 alkyl; preferably, Ra and Rb are each independently selected from H, C1-3 alkyl;
L2 is selected from a bond, C1-6 alkylene; wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy;
preferably, L2 is selected from a bond, C1-6 alkylene; wherein, the C1-6 alkylene is optionally substituted by 1 to 3 substituents selected from halogen, oxo, C1-3 alkyl, C3-6 cycloalkyl, 4-7-membered heterocycloalkyl, -C(=O)-4-7-membered heterocycloalkyl, - C(=O)N(R_{L2})₂; the C3-6 cycloalkyl, 4-7-membered heterocycloalkyl, -C(=O)-4-7-membered heterocycloalkyl, as a substituent of C1-6 alkylene, are optionally substituted by 1 or more substituents selected from halogen, cyano, C1-3 alkyl, C1-3 alkoxy, C1-3 haloalkyl, C1-3 haloalkoxy; each R_{L2} is independently selected from H, D, C1-6 alkyl, C3-6 cycloalkyl, 4-7-membered heterocycloalkyl, phenyl, 5-6-membered heteroaryl, and the C1-6 alkyl, C3-6 cycloalkyl, 4-7-membered heterocycloalkyl, phenyl, 5-6-membered heteroaryl are optionally substituted by a substituent selected from halogen, hydroxyl, cyano, C1-3 alkyl, C1-3 alkoxy;
preferably, L2 is selected from a bond, C2-5 alkylene; wherein, the C2-5 alkylene is optionally substituted by 1 or 2 substituents selected from oxo, C1-3 alkyl, C3-6 cycloalkyl, 4-7-membered nitrogen-containing saturated heterocycloalkyl, -C(=O)-4-7-membered nitrogen-containing saturated heterocycloalkyl, -C(=O)N(R_{L2})₂, the 4-7-membered nitrogen-containing saturated heterocycloalkyl, -C(=O)-4-7-membered nitrogen-containing saturated heterocycloalkyl, as a substituent of C2-5 alkylene, are optionally substituted by a substituent selected from halogen, cyano, C1-3 alkyl; each R_{L2} is independently selected from H, C1-4 alkyl, C3-6 cycloalkyl, 4-7-membered heterocycloalkyl, 5-6-membered heteroaryl, and the C1-4 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy;
preferably, L2 is selected from a bond, C1-6 alkylene; wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, C1-6 alkyl, C1-6 alkoxy, C1-6 haloalkyl, C1-6 haloalkoxy;
preferably, L2 is selected from a bond, C1-6 alkylene; wherein, the C1-6 alkylene is optionally substituted by 1 to 3 substituents selected from halogen, oxo, C1-3 alkyl, C3-6 cycloalkyl, 4-7-membered heterocycloalkyl, -C(=O)-4-7-membered heterocycloalkyl; the C3-6 cycloalkyl, 4-7-membered heterocycloalkyl, -C(=O)-4-7-membered heterocycloalkyl, as a substituent of C1-6 alkylene, are optionally substituted by 1 or more substituents selected from halogen, C1-3 alkyl, C1-3 alkoxy, C1-3 haloalkyl, C1-3 haloalkoxy;
further preferably, L2 is selected from a bond, C1-5 alkylene, wherein, the C1-5 alkylene is optionally substituted by 1 or 2 substituents selected from oxo, C1-3 alkyl, C3-6 cycloalkyl, 4-7-membered nitrogen-containing saturated heterocycloalkyl, -C(=O)-4-7-membered nitrogen-containing saturated heterocycloalkyl, the 4-7-membered nitrogen-containing saturated heterocycloalkyl, -C(=O)-4-7-membered nitrogen-containing saturated heterocycloalkyl, as a substituent of C1-5 alkylene, are optionally substituted by a substituent selected from C1-3 alkyl;
further preferably, L2 is selected from a bond, C2-5 alkylene; wherein, the C2-5 alkylene is optionally substituted by a substituent selected from C1-3 alkyl, C3-6 cycloalkyl (for example cyclopropyl, cyclobutyl, cyclohexyl), 4-7-membered heterocycloalkyl (for example oxetanyl, oxanyl, 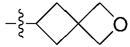 ), 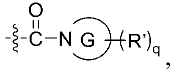 -C(=O)N(R₁₂)₂; wherein, ring G is 4-10-membered nitrogen-containing saturated heterocycloalkyl, R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 5-6-membered heteroaryl (for example pyridinyl), and the C1-3 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy;
more preferably, L2 is selected from C2-5 alkylene, the C2-5 alkylene is optionally substituted by a substituent selected from C1-3 alkyl, cyclopropyl, 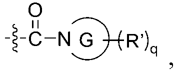 C(=O)N(R₁₂)₂; wherein, ring G is 4-10-membered nitrogen-containing saturated heterocycloalkyl (for example azetidinyl, azacyclopentyl, morpholinyl, 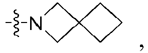 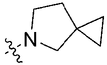 ), R' is selected from halogen (for example F, Cl), C1-3 alkyl (for example methyl, isopropyl), q is selected from 0, 1; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 6-membered heteroaryl (for example pyridinyl), and the 4-7-membered heterocycloalkyl, 6-membered heteroaryl are optionally substituted by a substituent selected from C1-3 alkyl;
Xl is selected from a bond, O, S; preferably, X1 is selected from a bond, O; further preferably, X1 is selected from a bond;
L3 is selected from a bond, C1-6 alkylene, C3-10 cycloalkyl-(CH₂)ₖ-#L4, 4-10-membered heterocycloalkyl-(CH₂)ₖ-#L4, 5-6-membered heteroaryl-(CH₂)ₖ-#L4; wherein, the C1-6 alkylene, C3-10 cycloalkyl-(CH₂)ₖ-#L4, 4-10-membered heterocycloalkyl-(CH₂)ₖ-#L4, 5-6-membered heteroaryl-(CH₂)ₖ-#L4 are optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; k is selected from 0, 1, 2, 3; #L4 represents the site of attachment to L4;
preferably, L3 is selected from a bond, C1-6 alkylene, C3-10 cycloalkyl, 4-10-membered heterocycloalkyl, 5-6-membered heteroaryl; wherein, the C1-6 alkylene, C3-10 cycloalkyl, 4-10-membered heterocycloalkyl, 5-6-membered heteroaryl are optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl;
preferably, preferably, L3 is selected from a bond, C1-3 alkylene, C3-6 cycloalkyl-(CH₂)ₖ-#L4, 4-6-membered monocyclic heterocycloalkyl-(CH₂)ₖ-#L4, 6-10-membered spiro heterocycloalkyl-(CH₂)ₖ-#L4, 6-10-membered bridged heterocycloalkyl-(CH₂)ₖ-#L4, 5-6-membered heteroaryl-(CH₂)ₖ-#L4; wherein, the C1-3 alkylene, C3-6 cycloalkyl-(CH₂)ₖ-#L4, 4-6-membered monocyclic heterocycloalkyl-(CH₂)ₖ-#L4, 6-10-membered spiro heterocycloalkyl-(CH₂)ₖ-#L4, 6-10-membered bridged heterocycloalkyl-(CH₂)ₖ-#L4, or 5-6-membered heteroaryl-(CH₂)ₖ-#L4 is optionally substituted by a substituent selected from halogen, hydroxyl, C1-3 alkyl, C1-3 haloalkyl, C1-3 alkoxy, C1-3 haloalkoxy, -O-C3-6 cycloalkyl;
preferably, L3 is selected from a bond, C1-3 alkylene, C3-6 cycloalkyl, 4-6-membered monocyclic heterocycloalkyl, 6-10-membered spiro heterocycloalkyl, 6-10-membered bridged heterocycloalkyl, 5-6-membered heteroaryl; wherein, the C1-3 alkylene, C3-6 cycloalkyl, 4-6-membered monocyclic heterocycloalkyl, 6-10-membered spiro heterocycloalkyl or 6-10-membered bridged heterocycloalkyl, 5-6-membered heteroaryl are optionally substituted by a substituent selected from halogen, hydroxyl, C1-3 alkyl, C1-3 halooxy, C1-3 alkoxy, C1-3 haloalkoxy, -O-C3-6 cycloalkyl;
preferably, L3 is selected from a bond, C1-3 alkylene, C3-6 cycloalkyl, 4-6-membered monocyclic heterocycloalkyl, 6-10-membered spiro heterocycloalkyl, pyrrolyl; wherein, the C1-3 alkylene, C3-6 cycloalkyl, 4-6-membered monocyclic heterocycloalkyl, 6-10-membered spiro heterocycloalkyl, pyrrolyl are optionally substituted by a substituent selected from C1-3 alkyl, C1-3 alkoxy, -O-C3-6 cycloalkyl;
further preferably, L3 is selected from a bond, C1-3 alkylene, 4-6-membered monocyclic heterocycloalkyl-(CH₂)ₖ-#L4, 6-10-membered spiro heterocycloalkyl-(CH₂)ₖ-#L4, pyrrolyl-(CH₂)ₖ-#L4; wherein, the C1-3 alkylene, 4-6-membered monocyclic heterocycloalkyl-(CH₂)ₖ-#L4, 6-10-membered spiro heterocycloalkyl-(CH₂)ₖ-#L4, pyrrolyl-(CH₂)ₖ-#L4 are optionally substituted by a substituent selected from C1-3 alkyl, C1-3 alkoxy, -O-C3-6 cycloalkyl;
more preferably, L3 is selected from a bond, C1-3 alkylene;
L4 is selected from a bond, O, S, -NRc-; Rc is selected from H, C1-6 alkyl, C1-6 haloalkyl, C3-6 cycloalkyl, 4-7-membered heterocycloalkyl;
preferably, L4 is selected from a bond, O, -NRc-; Rc is selected from H, C1-3 alkyl;
further preferably, L4 is selected from -NH-.

In some embodiments, when L1 is selected from $B-C(=O)-NRa-#L2, any one of the following conditions is satisfied:
(1) L2 is selected from C1-6 alkylene; wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl (preferably C3-10 saturated cycloalkyl), - O-C3-10 cycloalkyl, 4-10-membered heterocycloalkyl (preferably 4-10-membered saturated heterocycloalkyl, the 4-10-membered saturated heterocycloalkyl has 0, 1, or 2 nitrogen heteroatoms and 0 or 1 heteroatom selected from O and S, preferably has 1 oxygen heteroatom), -O-4-10-membered heterocycloalkyl, -C(=O)-4-10-membered heterocycloalkyl (preferably - C(=O)-4-10-membered saturated heterocycloalkyl, the 4-10-membered saturated heterocycloalkyl has 1 or 2 nitrogen heteroatoms and 0 or 1 heteroatom selected from O and S, preferably has 1 nitrogen heteroatom and 0 or 1oxygen heteroatom), -C(=O)N(R₁₂)₂; the C3-10 cycloalkyl (preferably C3-10 saturated cycloalkyl), -O-C3-10 cycloalkyl, 4-10-membered heterocycloalkyl (preferably 4-10-membered saturated heterocycloalkyl), -C(=O)-4-10-membered heterocycloalkyl (preferably -C(=O)-4-10-membered saturated heterocycloalkyl), as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl (preferably C3-10 saturated cycloalkyl), 4-10-membered heterocycloalkyl (preferably 4-10-membered saturated heterocycloalkyl, the 4-10-membered saturated heterocycloalkyl has 0, 1, or 2 nitrogen heteroatoms and 0 or 1 heteroatom selected from O and S, preferably has 1 oxygen heteroatom), C6-10 aryl, 5-10-membered heteroaryl (preferably 5-10-membered heteroaryl, the 5-10-membered heteroaryl has 0, 1, or 2 nitrogen heteroatoms and 0 or 1 heteroatom selected from O and S, preferably has 1 nitrogen heteroatom), and the C1-6 alkyl, C3-10 cycloalkyl (preferably C3-10 saturated cycloalkyl), 4-10-membered heterocycloalkyl (preferably 4-10-membered saturated heterocycloalkyl), C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from a bond, O, -NRc-, Rc is selected from H, C1-3 alkyl;
preferably, L2 is selected from C2-6 alkylene; wherein, the C2-6 alkylene is optionally substituted by a substituent of C1-3 alkyl, C3-6 cycloalkyl (preferably C3-6 saturated cycloalkyl), 4-7-membered heterocycloalkyl (preferably 4-7-membered saturated heterocycloalkyl, the 4-7-membered saturated heterocycloalkyl has 0, 1, or 2 nitrogen heteroatoms and 0 or 1 heteroatom selected from O and S, preferably has 1 oxygen heteroatom), 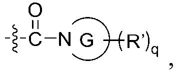 -C(=O)N(R₁₂)₂; wherein, ring G is 4-10-membered nitrogen-containing saturated heterocycloalkyl (preferably 4-10-membered nitrogen-containing saturated heterocycloalkyl, the 4-10-membered nitrogen-containing saturated heterocycloalkyl has 1 or 2 nitrogen heteroatoms and 0 or 1 heteroatom selected from O and S, preferably has 1 nitrogen heteroatom and 0 or 1 oxygen heteroatom), R' is selected from halogen, hydroxyl, cyano, C1-3 alkyl, C1-3 haloalkyl, C1-3 alkoxy, C1-3 haloalkoxy, q is selected from 0, 1, or 2; each R_{L2} is independently selected from H, methyl, ethyl, isopropyl, cyclopropyl, cyclobutyl, oxanyl, pyridinyl, and oxanyl is optionally substituted by a substituent selected from C1-3 alkyl;
preferably, L2 is selected from C2-6 alkylene; wherein, the C2-6 alkylene is optionally substituted by a substituent of C1-3 alkyl, C3-6 saturated cycloalkyl, 4-6-membered monocyclic saturated heterocycloalkyl, 7-membered saturated spiro heterocycloalkyl, 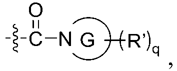 -C(=O)N(R₁₂)₂; wherein, ring G is 4-6-membered monocyclic nitrogen-containing saturated heterocycloalkyl, 7-membered nitrogen-containing saturated spiro heterocycloalkyl, R' is selected from halogen, cyano, C1-3 alkyl, C1-3 alkoxy, q is selected from 0, 1, or 2; each R_{L2} is independently selected from H, C1-4 alkyl, C3-6 saturated cycloalkyl, 4-6-membered monocyclic saturated heterocycloalkyl, 5-6-membered heteroaryl (preferably 6-membered heteroaryl), and the 4-6-membered monocyclic saturated heterocycloalkyl, 5-6-membered heteroaryl (preferably 6-membered heteroaryl) are optionally substituted by a substituent selected from C1-3 alkyl; wherein the 4-6-membered monocyclic saturated heterocycloalkyl and 7-membered saturated spiro heterocycloalkyl have 0, 1, or 2 nitrogen heteroatoms and 0 or 1 heteroatom selected from O and S, preferably have 1 oxygen heteroatom, the 4-6-membered monocyclic nitrogen-containing saturated heterocycloalkyl and 7-membered nitrogen-containing saturated spiro heterocycloalkyl have 1 or 2 nitrogen heteroatoms and 0 or 1 heteroatom selected from O and S, preferably have 1 nitrogen heteroatom and 0 or 1 oxygen heteroatom, the 5-6-membered heteroaryl (preferably 6-membered heteroaryl ) has 1, 2, or 3 nitrogen heteroatoms and 0 or 1 heteroatom selected from O and S, preferably has 1 nitrogen heteroatom.
(2) L2 is selected from a bond, C1-6 alkylene; X1 is selected from a bond; L3 is selected from C3-10 cycloalkyl-(CH₂)ₖ-#L4 (preferably C3-10 saturated cycloalkyl-(CH₂)ₖ-#L4), 4-10-membered heterocycloalkyl-(CH₂)ₖ-#L4 (preferably 4-10-membered saturated heterocycloalkyl-(CH₂)ₖ-#L4, the 4-10-membered saturated heterocycloalkyl has 1 or 2 nitrogen heteroatoms and 0 or 1 heteroatom selected from O and S, preferably has 1 nitrogen heteroatom), 5-6-membered heteroaryl-(CH₂)ₖ-#L4 (preferably 5-6-membered heteroaryl-(CH₂)ₖ-#L4, the 5-6-membered heteroaryl has 1, 2, or 3 nitrogen heteroatoms and 0, 1, or 2 heteroatoms selected from O and S, preferably has 1 nitrogen heteroatom), wherein, the C3-10 cycloalkyl-(CH₂)ₖ-#L4 (preferably C3-10 saturated cycloalkyl-(CH₂)ₖ-#L4), 4-10-membered heterocycloalkyl-(CH₂)ₖ-#L4 (preferably 4-10-membered saturated heterocycloalkyl-(CH₂)ₖ-#L4), 5-6-membered heteroaryl-(CH₂)ₖ-#L4 are optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl (preferably C3-6 saturated cycloalkyl), -O-C3-6 cycloalkyl (preferably -O-C3-6 saturated cycloalkyl), 4-10-membered heterocycloalkyl (preferably 4-10-membered saturated heterocycloalkyl), -O-4-10-membered heterocycloalkyl (preferably -O-4-10-membered saturated heterocycloalkyl); k is selected from 0, 1, 2, 3; #L4 represents the site of attachment to L4; L4 is selected from a bond or -NRc-;
preferably, L2 is selected from a bond, C1-3 alkylene; X1 is selected from a bond; L3 is selected from C3-6 cycloalkyl (preferably C3-6 saturated cycloalkyl), 4-10-membered heterocycloalkyl (preferably 4-10-membered saturated heterocycloalkyl, the 4-10-membered saturated heterocycloalkyl has 1 or 2 nitrogen heteroatoms and 0 or 1 heteroatom selected from O and S, preferably has 1 nitrogen heteroatom), 5-6-membered heteroaryl-(CH₂)ₖ-#L4 (preferably 5-6-membered heteroaryl-(CH₂)ₖ-#L4, the 5-6-membered heteroaryl has 1, 2, or 3 nitrogen heteroatoms and 0, 1, or 2 heteroatoms selected from O and S, preferably has 1 nitrogen heteroatom), wherein, the C3-6 cycloalkyl (preferably C3-6 saturated cycloalkyl), 4-10-membered heterocycloalkyl (preferably 4-10-membered saturated heterocycloalkyl), 5-6-membered heteroaryl-(CH₂)ₖ-#L4 are optionally substituted by 1 or more substituents selected from C1-3 alkyl, C1-3 alkoxy, -O-C3-6 cycloalkyl (preferably -O-C3-6 saturated cycloalkyl); k is selected from 0, 1, 2; #L4 represents the site of attachment to L4; L4 is selected from a bond or -NRc-;
preferably, preferably, L2 is selected from a bond, C1-3 alkylene; X1 is selected from a bond; L3 is selected from C3-6 saturated cycloalkyl, 4-6-membered monocyclic saturated heterocycloalkyl, 7-9-membered saturated spiro heterocycloalkyl, 8-10-membered saturated fused heterocycloalkyl, 5-membered heteroaryl-(CH₂)ₖ-#L4, the 4-6-membered monocyclic saturated heterocycloalkyl, 5-membered heteroaryl-(CH₂)ₖ-#L4 are optionally substituted by 1 substituent selected from C1-3 alkyl, C1-3 alkoxy, -O-C3-6 saturated cycloalkyl; wherein any of the heterocycloalkyl groups has 1 or 2 nitrogen heteroatoms and 0 or 1 heteroatom selected from O and S, preferably has 1 nitrogen heteroatom, the 5-membered heteroaryl has 1, 2, or 3 nitrogen heteroatoms and 0 or 1 heteroatom selected from O and S, preferably has 1 nitrogen heteroatom, k is selected from 0 or 1, #L4 represents the site of attachment to L4; L4 is selected from a bond or -NRc-, preferably L4 is selected from a bond.

In some embodiments, when L1 is selected from $B-NRb-C(=O)-#L2, any one of the following conditions is satisfied:
(1) L2 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from a bond or O;
preferably, L2 is selected from C1-3 alkylene, wherein, the C1-3 alkylene is optionally substituted by 1 substituent selected from C1-3 alkyl, C3-6 cycloalkyl; X1 is selected from a bond, L3 is selected from a bond, L4 is selected from a bond or O;
preferably, L2 is selected from C1-3 alkylene, wherein, the C1-3 alkylene is optionally substituted by 1 substituent selected from C1-3 alkyl, C3-6 saturated cycloalkyl; X1 is selected from a bond, L3 is selected from a bond, L4 is selected from a bond or O; (2) L2 is selected from C1-6 alkylene; X1 is selected from O; L3 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl,-O-C3-6 cycloalkyl, 4-10-membered heterocycloalkyl, -O-4-10-membered heterocycloalkyl; L4 is selected from -NRc-;
preferably, L2 is selected from C1-3 alkylene, X1 is selected from O; L3 is selected from C1-3 alkylene, wherein, the C1-3 alkylene is optionally substituted by 1 substituent selected from C1-3 alkyl; L4 is selected from -NRc-.

In some embodiments, when L1 is selected from -C(=O)-, any one of the following conditions is satisfied:
(1) L2 is selected from a bond; X1 is selected from a bond; L3 is selected from 4-10-membered heterocycloalkyl-(CH₂)ₖ-#L4 (preferably 4-10-membered saturated heterocycloalkyl-(CH₂)ₖ-#L4, the 4-10-membered saturated heterocycloalkyl has 1 or 2 nitrogen heteroatoms and 0 or 1 heteroatom selected from O and S, preferably has 1 nitrogen heteroatom), wherein, the 4-10-membered heterocycloalkyl-(CH₂)ₖ-#L4 (preferably 4-10-membered saturated heterocycloalkyl-(CH₂)ₖ-#L4) is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; k is selected from 0, 1, 2, 3; #L4 represents the site of attachment to L4, L4 is selected from -NRc-;
preferably, L3 is selected from 4-10-membered heterocycloalkyl-(CH₂)ₖ-#L4 (preferably 4-10-membered saturated heterocycloalkyl-(CH₂)ₖ-#L4, the 4-10-membered saturated heterocycloalkyl has 1 or 2 nitrogen heteroatoms and 0 or 1 heteroatom selected from O and S, preferably has 1 nitrogen heteroatom), wherein, the 4-10-membered heterocycloalkyl-(CH₂)ₖ-#L4 (preferably 4-10-membered saturated heterocycloalkyl-(CH₂)ₖ-#L4) is optionally substituted by 1 substituent selected from C1-3 alkyl;
preferably, L3 is selected from 4-6-membered monocyclic saturated heterocycloalkyl-(CH₂)ₖ-#L4, 7-9-membered saturated spiro heterocycloalkyl-(CH₂)ₖ-#L4, the 4-6-membered monocyclic saturated heterocycloalkyl-(CH₂)ₖ-#L4, 7-9-membered saturated spiro heterocycloalkyl-(CH₂)ₖ-#L4 are optionally substituted by 1 substituent selected from C1-3 alkyl, k is selected from 0, 1, or 2; wherein any of the heterocycloalkyl groups has 1 or 2 nitrogen heteroatoms and 0 or 1 heteroatom selected from O and S, preferably has 1 nitrogen heteroatom;
(2) L2 is selected from C1-6 alkylene; X1 is selected from O; L3 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; L4 is selected from -NRc-;
preferably, L2 is selected from C1-3 alkylene; X1 is selected from O; L3 is selected from C1-3 alkylene, wherein, the C1-3 alkylene is optionally substituted by 1 substituent selected from C1-3 alkyl; L4 is selected from -NRc-.

In some embodiments, when L1 is selected from -NRc-, L2 is selected from C1-6 alkylene; wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from -NRc-;
preferably, L2 is selected from C1-6 alkylene; wherein, the C1-6 alkylene is optionally substituted by 1 or more substituents selected from halogen, hydroxyl, oxo, C1-3 alkyl, C3-6 cycloalkyl, 4-7-membered heterocycloalkyl, -C(=O)-4-7-membered heterocycloalkyl,-C(=O)N(R_{L2})₂; the C3-6 cycloalkyl, 4-7-membered heterocycloalkyl, -C(=O)-4-7-membered heterocycloalkyl, as a substituent of C1-6 alkylene, are optionally substituted by 1 or more substituents selected from halogen, hydroxyl, cyano, C1-3 alkyl, C1-3 alkoxy, C1-3 haloalkyl, C1-3 haloalkoxy; each R_{L2} is independently selected from H, D, C1-3 alkyl, C3-6 cycloalkyl, 4-7-membered heterocycloalkyl, phenyl, 5-6-membered heteroaryl, and the C1-3 alkyl, C3-6 cycloalkyl, 4-7-membered heterocycloalkyl, phenyl, 5-6-membered heteroaryl are optionally substituted by a substituent selected from halogen, hydroxyl, cyano, C1-3 alkyl, C1-3 alkoxy; X1 is selected from a bond, L3 is selected from a bond, L4 is selected from -NRc-;
preferably, L2 is selected from C1-6 alkylene; wherein, the C1-6 alkylene is optionally substituted by 1 substituent selected from C1-3 alkyl.

In some embodiments, when L1 is selected from a bond, any one of the following conditions is satisfied:
(1) L2 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from a bond or -NRc-;
preferably, L2 is selected from C1-6 alkylene, wherein, the C1-6 alkylene is optionally substituted by 1 substituent selected from C1-3 alkyl;
(2) L2 is selected from C1-6 alkylene, X1 is selected from O, L3 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; L4 is selected from -NRc-;
preferably, L2 is selected from C1-3 alkylene, X1 is selected from O, L3 is selected from C1-3 alkylene, wherein, the C1-3 alkylene is optionally substituted by 1 substituent selected from C1-3 alkyl.

In some embodiments, when L1 is selected from 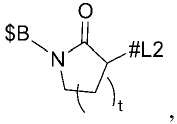 L2 is selected from a bond; X1 is selected from O or S; L3 is selected from a bond, C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl,-O-C3-6 cycloalkyl, 4-10-membered heterocycloalkyl, -O-4-10-membered heterocycloalkyl; and L4 is selected from a bond, $B represents the site of attachment to ring B, #L2 represents the site of attachment to L2; t, Ra, Rb, Rc are as defined in any of the above embodiments;
preferably, L2 is selected from a bond; X1 is selected from O, L3 is selected from a bond; L4 is selected from a bond.

In some embodiments, when L1 is selected from $B-C(=O)-NRa-#L2, X1 is selected from O, S, or -NRc-, and L3 and L4 both represent a single bond; or
when L1 is selected from $B-NRb-C(=O)-#L2, X1 represents a single bond, O, or S, L3 represents a single bond or substituted or unsubstituted C1-6 alkylene, L4 represents a single bond or -NRc-; or
when L1 is selected from a single bond, X1 is selected from O, S, or -NRc-, L3 represents substituted or unsubstituted C1-6 alkylene, L4 represents -NRc-; or
when L1 is selected from 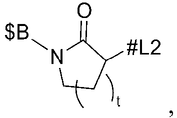 X1 is selected from O, S, or -NRc-, L3 represents a single bond or substituted or unsubstituted C1-6 alkylene, and L4 represents a single bond, $B represents the site of attachment to ring B, #L2 represents the site of attachment to L2;
wherein, t, Ra, Rb, and Rc are as defined above.

In some embodiments, when L1 is selected from $B-C(=O)-NRa-#L2, L2 is selected from: 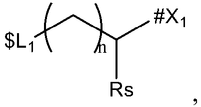 wherein $L1 represents the site of attachment to L1, #X1 represents the site of attachment to X1, n is an integer selected from 0, 1, 2, 3, 4, or 5, and wherein Rs is selected from H, substituted or unsubstituted C1-6 alkyl, substituted or unsubstituted C3-6 cycloalkyl, -C(=O)NRᵢᵢRᵢᵢᵢ, wherein Rᵢᵢ and Rᵢᵢᵢ may independently represent hydrogen, deuterium, C1-6 alkyl, C3-6 cycloalkyl, heterocycloalkyl , or aryl, or Rᵢᵢ and Rᵢᵢᵢ together with the atoms that they are attached to, form 4-10-membered heterocycloalkyl, the heterocycloalkyl is optionally substituted by 1-3 substituents selected from halogen, -OH, C1-6 alkyl, C1-6 alkoxy, C1-6 haloalkyl, C1-6 haloalkoxy,
wherein, Ra is as defined in any of the above embodiments; or
when L1 is selected from $B-C(=O)-NRa-#L2, L2 is selected from: 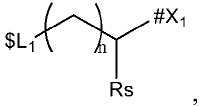 wherein $L1 represents the site of attachment to L1, #X1 represents the site of attachment to X1, n is an integer selected from 0, 1, 2, 3, 4, or 5, Rs is selected from H, D, halogen, hydroxyl, C1-6 alkyl, C1-6 alkoxy, C1-6 haloalkyl, C1-6 haloalkoxy, C3-10 cycloalkyl (preferably C3-10 saturated cycloalkyl), 4-10-membered heterocycloalkyl (preferably 4-10-membered saturated heterocycloalkyl, the saturated heterocycloalkyl has 0, 1 or 2 nitrogen heteroatoms and 0 or 1 heteroatom selected from O and S, preferably has 1 oxygen heteroatom), 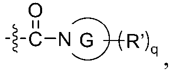 C(=O)N(R_{L2})₂; wherein, ring G is 4-10-membered nitrogen-containing saturated heterocycloalkyl (preferably 4-10-membered nitrogen-containing saturated heterocycloalkyl, the 4-10-membered nitrogen-containing saturated heterocycloalkyl has 1 or 2 nitrogen heteroatoms and 0 or 1 heteroatom selected from O and S, preferably has 1 nitrogen heteroatom and 0 or 1 oxygen heteroatom), R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, D, C1-6 alkyl, C3-10 cycloalkyl (preferably C3-10 saturated cycloalkyl), 4-10-membered heterocycloalkyl (preferably 4-10-membered saturated heterocycloalkyl, the saturated heterocycloalkyl has 0, 1, or 2 nitrogen heteroatoms and 0 or 1 heteroatom selected from O or S, preferably has 1 oxygen heteroatom), C6-10 aryl, 5-10-membered heteroaryl (preferably 5-10-membered heteroaryl, the heteroaryl has 0, 1, or 2 nitrogen heteroatoms and 0 or 1 heteroatom selected from O or S, preferably has 1 nitrogen heteroatom), and the C1-6 alkyl, C3-10 cycloalkyl (preferably C3-10 saturated cycloalkyl), 4-10-membered heterocycloalkyl (preferably 4-10-membered saturated heterocycloalkyl), C6-10 aryl, 5-10-membered heteroaryl are optionally substituted by a substituent selected from halogen, hydroxyl, cyano, C1-6 alkyl, C1-6 alkoxy;
preferably, Rs is selected from H, D, halogen, hydroxyl, C1-6 alkyl, C1-6 alkoxy, C1-6 haloalkyl, C1-6 haloalkoxy, C3-6 cycloalkyl (preferably C3-6 saturated cycloalkyl), 4-7-membered heterocycloalkyl (preferably 4-7-membered saturated heterocycloalkyl, the saturated heterocycloalkyl has 0, 1, or 2 nitrogen heteroatoms and 0 or 1 heteroatom selected from O and S, preferably has 1 oxygen heteroatom), 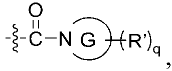 -C(=O)N(R_{L2})₂; wherein, ring G is 4-10-membered nitrogen-containing saturated heterocycloalkyl (preferably 4-10-membered nitrogen-containing saturated heterocycloalkyl, the 4-10-membered nitrogen-containing saturated heterocycloalkyl has 1 or 2 nitrogen heteroatoms and 0 or 1 heteroatom selected from O and S, preferably has 1 nitrogen heteroatom and 0 or 1 oxygen heteroatom), R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, D, C1-4 alkyl, C3-6 cycloalkyl (preferably C3-6-membered saturated cycloalkyl), 4-7-membered heterocycloalkyl (preferably 4-7-membered saturated heterocycloalkyl, the saturated heterocycloalkyl has 0, 1, or 2 nitrogen heteroatoms and 0 or 1 heteroatom selected from O or S, preferably has 1 oxygen heteroatom), phenyl, 5-6-membered heteroaryl (preferably 5-6-membered heteroaryl, the heteroaryl has 0, 1, or 2 nitrogen heteroatoms and 0 or 1 heteroatom selected from O or S, preferably has 1 nitrogen heteroatom), and the C1-4 alkyl, C3-6 cycloalkyl (preferably C3-6 saturated cycloalkyl), 4-7-membered heterocycloalkyl (4-7-membered saturated heterocycloalkyl), phenyl, 5-6-membered heteroaryl are optionally substituted by a substituent selected from halogen, hydroxyl, cyano, C1-3 alkyl, C1-3 alkoxy;
preferably, Rs is selected from H, C1-3 alkyl, C3-6 saturated cycloalkyl, 4-6-membered monocyclic saturated heterocycloalkyl, 7-membered saturated spiro heterocycloalkyl, 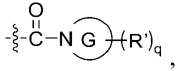 -C(=O)N(R_{L2})₂; wherein, ring G is 4-6-membered monocyclic nitrogen-containing saturated heterocycloalkyl, 7-membered nitrogen-containing saturated spiro heterocycloalkyl, R' is selected from halogen, cyano, C1-3 alkyl, C1-3 alkoxy, q is selected from 0, 1, or 2; each R_{L2} is independently selected from H, C1-4 alkyl, C3-6 saturated cycloalkyl, 4-6-membered monocyclic saturated heterocycloalkyl, 5-6-membered heteroaryl (preferably 6-membered heteroaryl), and the 4-6-membered monocyclic saturated heterocycloalkyl, 5-6-membered heteroaryl (preferably 6-membered heteroaryl) are optionally substituted by a substituent selected from C1-3 alkyl; wherein the 4-6-membered monocyclic saturated heterocycloalkyl and 7-membered saturated spiro heterocycloalkyl have 0, 1, or 2 nitrogen heteroatoms and 0 or 1 heteroatom selected from O and S, preferably have 1 oxygen heteroatom, the 4-6-membered monocyclic nitrogen-containing saturated heterocycloalkyl and 7-membered nitrogen-containing saturated spiro heterocycloalkyl have 1 or 2 nitrogen heteroatoms and 0 or 1 heteroatom selected from O and S, preferably have 1 nitrogen heteroatom and 0 or 1 oxygen heteroatom, the 5-6-membered heteroaryl (preferably 6-membered heteroaryl) has 1, 2, or 3 nitrogen heteroatoms and 0 or 1 heteroatom selected from O and S, preferably has 1 nitrogen heteroatom.

In some embodiments, when L1 is selected from $B-C(=O)-NRa-#L2, L2 is selected from: 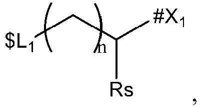 wherein $L1 represents the site of attachment to L1, #X1 represents the site of attachment to X1, n is an integer selected from 0, 1, 2, 3, 4, or 5, and wherein
Rs is selected from H, substituted or unsubstituted C1-6 alkyl, substituted or unsubstituted C1-6 cycloalkyl, -OC(=O)NRᵢᵢRᵢᵢᵢ, wherein Rᵢᵢ and Rᵢᵢᵢ may independently represent hydrogen, deuterium, C1-6 alkyl, C3-6 cycloalkyl, heterocycloalkyl, or aryl, or Rᵢᵢ and Rᵢᵢᵢ together with the atoms that they are attached to, form 4-10-membered heterocycloalkyl, the heterocycloalkyl is optionally substituted by 1-3 substituents selected from halogen, -OH, C1-6 alkyl, C1-6 alkoxy, C1-6 haloalkyl, C1-6 haloalkoxy,
wherein, Ra is as defined above.

In some embodiments, n is selected from 3, Rs is selected from the following substituted or unsubstituted groups: methyl, ethyl, propyl, cyclopropyl, cyclobutyl, cyclopentyl, cyclohexyl,

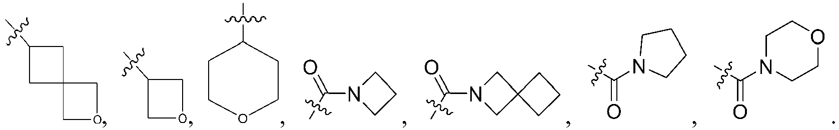

In some embodiments, n is selected from 3, Rs is selected from: methyl, cyclopropyl, cyclobutyl, cyclohexyl, 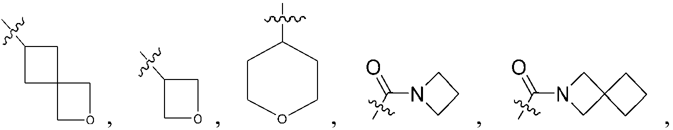 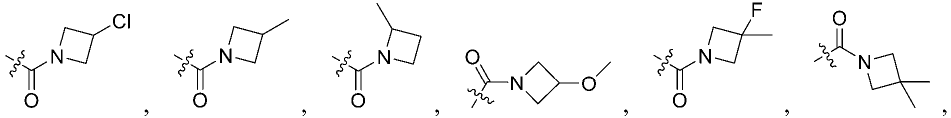 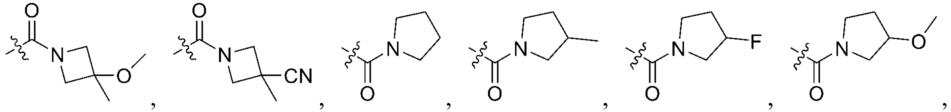 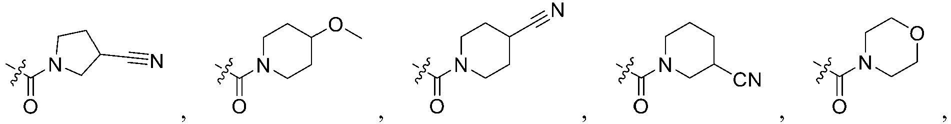 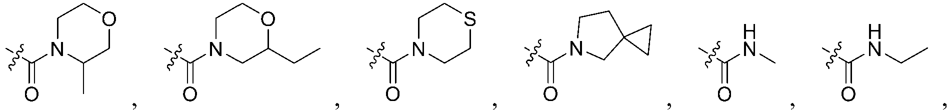 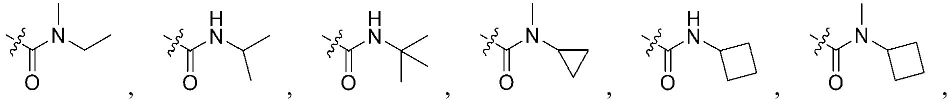 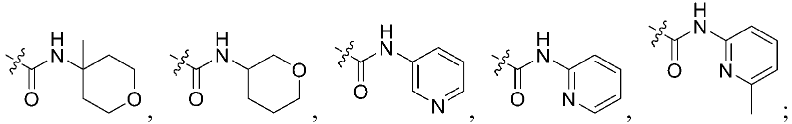 preferably, Rs is selected from methyl,

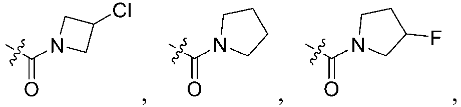

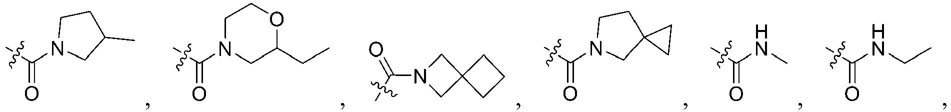

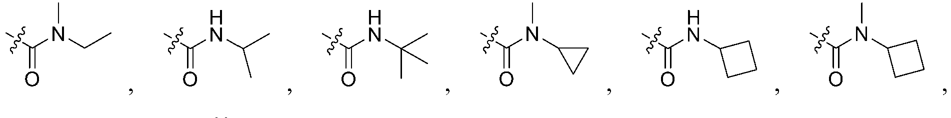

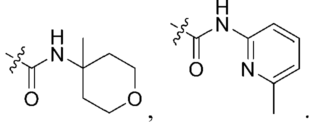

In some embodiments, when L1 is selected from $B-NRb-C(=O)-#L2, X1, L3, and L4 all represent a single bond, and L2 is selected from substituted or unsubstituted C1-4 alkylene, preferably substituted or unsubstituted methyl, ethyl or propyl; or
when L1 is selected from $B-NRb-C(=O)-#L2, X1 is selected from O or S, L2 and L3 are each independently selected from substituted or unsubstituted C1-4 alkylene, preferably substituted or unsubstituted methyl, ethyl, or propyl, and L4 is selected from -NRc-,
wherein, Rb and Rc are as defined above.

In some embodiments, when L1 is selected from a single bond, X1 is selected from O, S, or -NRc-, L2 and L3 are each independently selected from substituted or unsubstituted C1-4 alkylene, and L4 is selected from -NRc-, wherein Rb and Rc are as defined above.

In some embodiments, when L1 is selected from a single bond, X1 is selected from O or S, L2 and L3 are each independently selected from substituted or unsubstituted C1-4 alkylene, and L4 is selected from -NRc-, wherein Rb and Rc are as defined above.
preferably, wherein the carbon atom of L2 attached to ring B is substituted with =O to form a carbonyl.

In some embodiments, when L1 is selected from 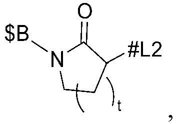 X1 is selected from O, L2, L3, and L4 all represent a single bond, and t is selected from 1; $B represents the site of attachment to ring B, #L2 represents the site of attachment to L2.

In some embodiments, L is selected from: 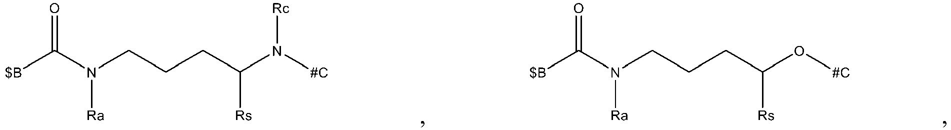 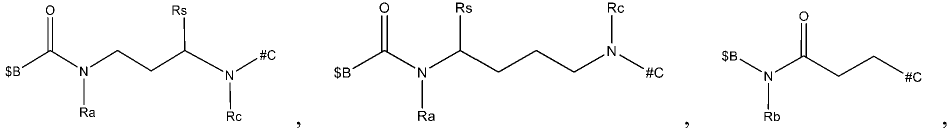 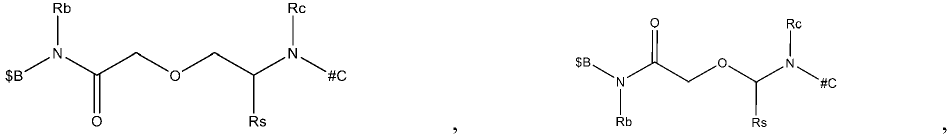 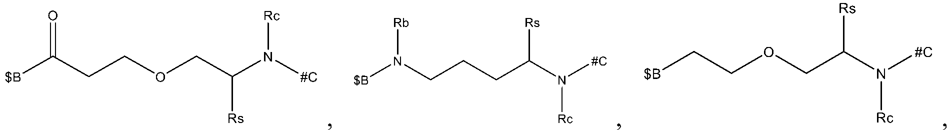 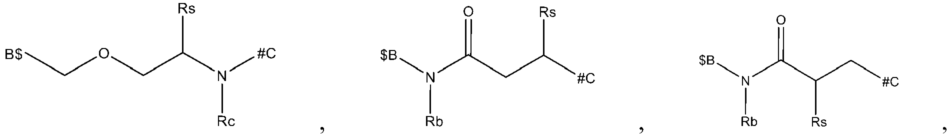 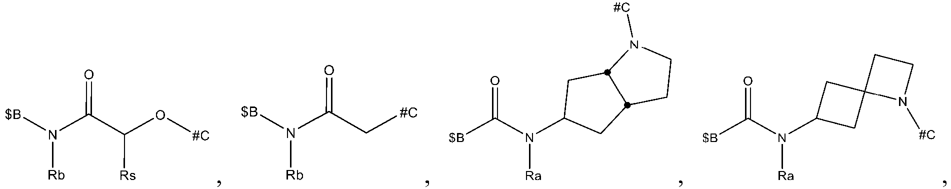 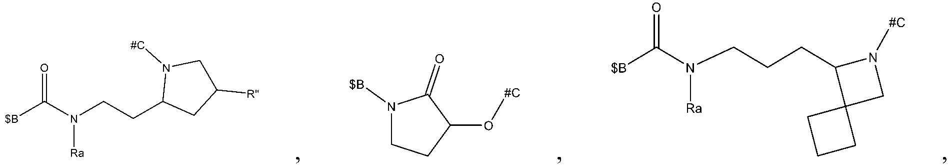 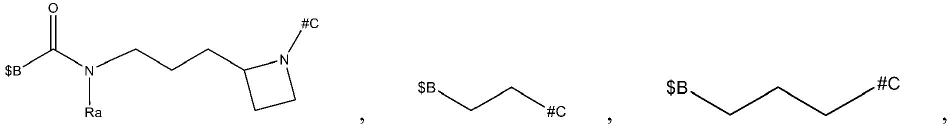 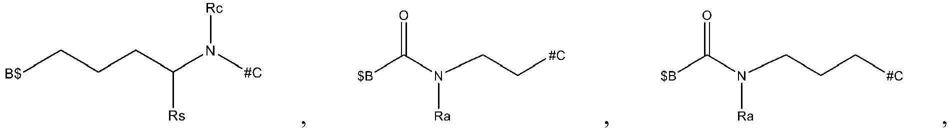 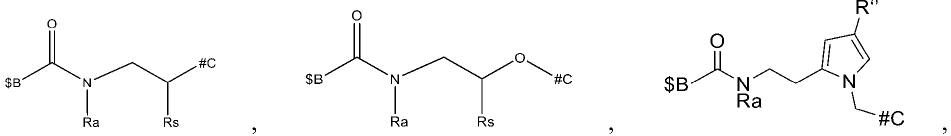 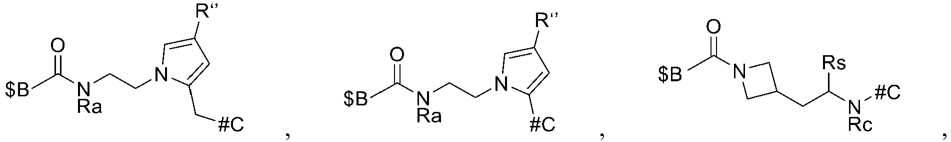 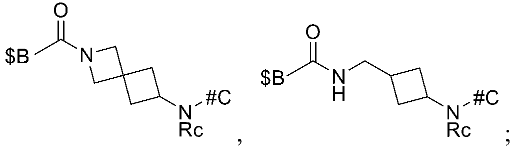
wherein, $B represents the site of attachment to ring B, #C represents the site of attachment to ring C, Ra, Rb, and Rc are as defined in any of the above embodiments, Rs is as defined in any of the above embodiments, R" is selected from C1-6 alkoxy, -O-C3-6 cycloalkyl, preferably, R" is selected from C1-6 alkoxy, -O-C3-6 saturated cycloalkyl.
preferably, Ra, Rb, and Rc are each independently selected from H or methyl;
preferably, Rs is selected from H, methyl, cyclopropyl, cyclobutyl, cyclohexyl,

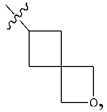

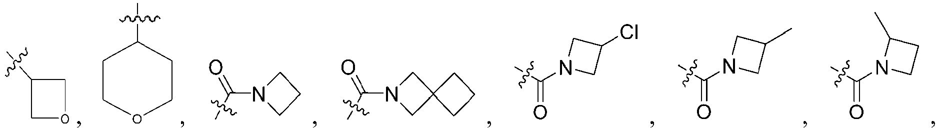

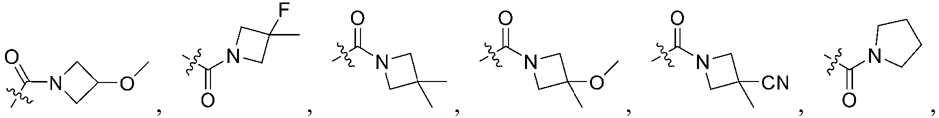

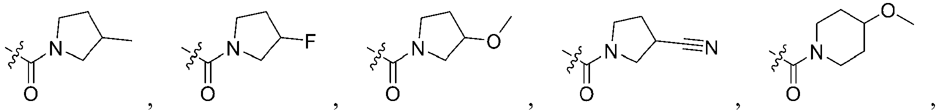

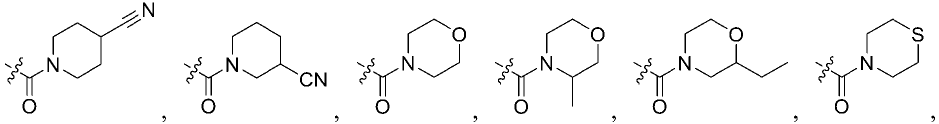

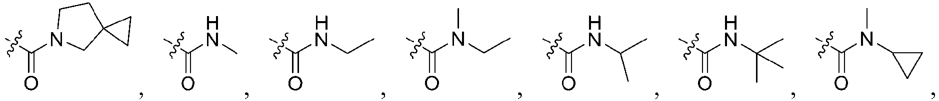

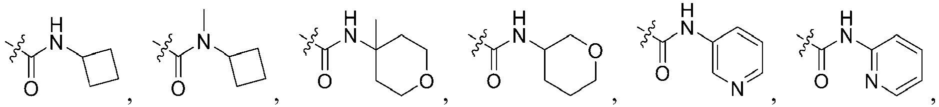

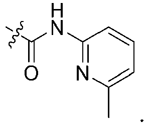

In some embodiments, L is selected from: 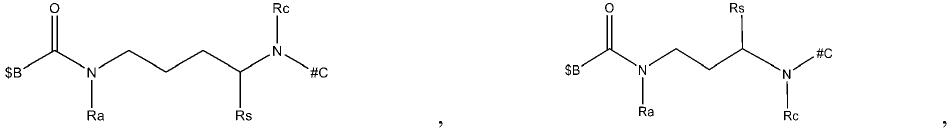 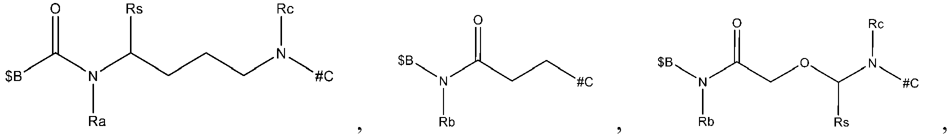 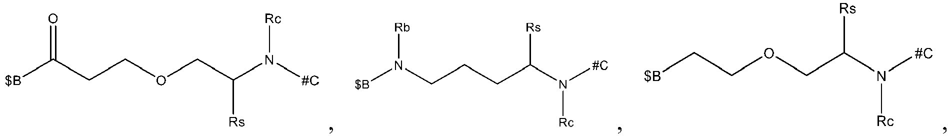 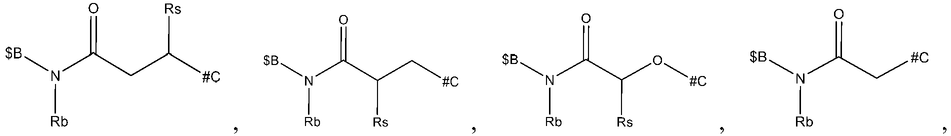 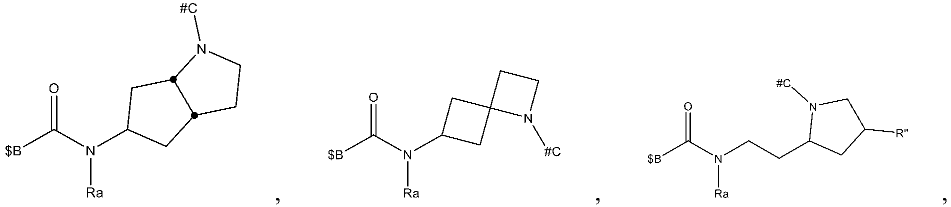 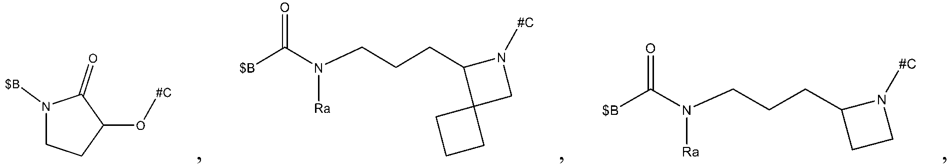 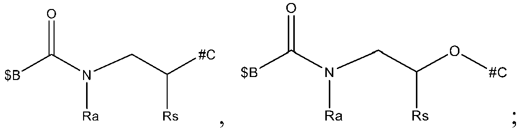
wherein, $B represents the site of attachment to ring B, #C represents the site of attachment to ring C, Ra, Rb, and Rc are as defined above, Rs is as defined above;
preferably, Ra, Rb, and Rc are each independently selected from H or methyl;
preferably, Rs is selected from H or the following substituted or unsubstituted groups: methyl, ethyl, propyl, cyclopropyl, cyclobutyl, cyclopentyl, cyclohexyl,

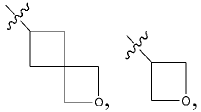

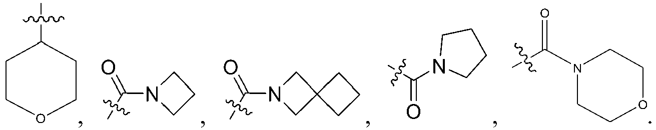

In some embodiments, L is selected from: 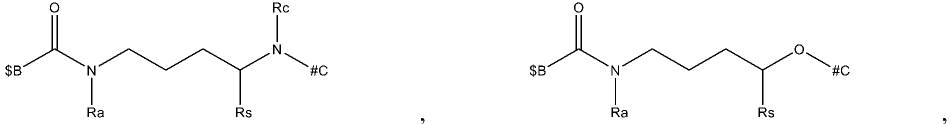 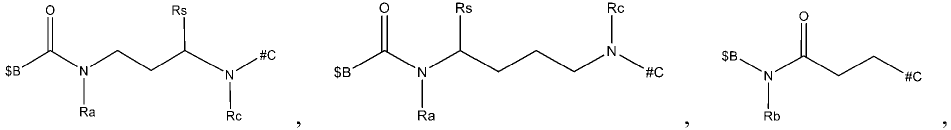 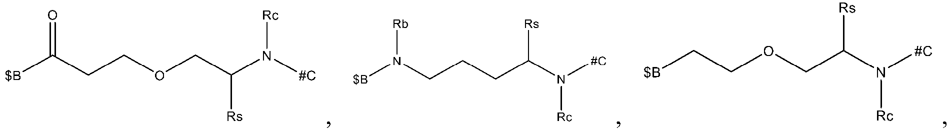 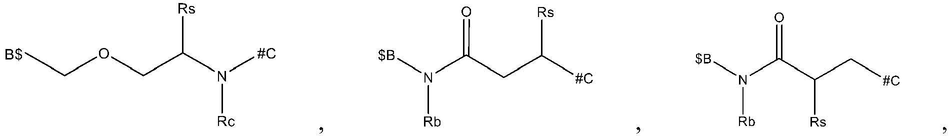 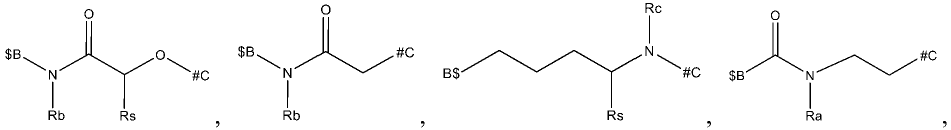 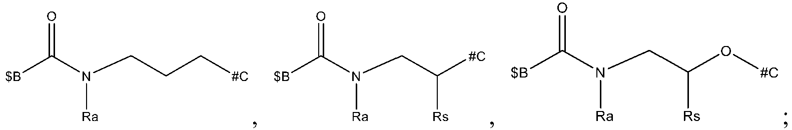
wherein, $B represents the site of attachment to ring B, #C represents the site of attachment to ring C, Ra, Rb, and Rc are as defined in any of the above embodiments, Rs is as defined in any of the above embodiments;
preferably, L is selected from: 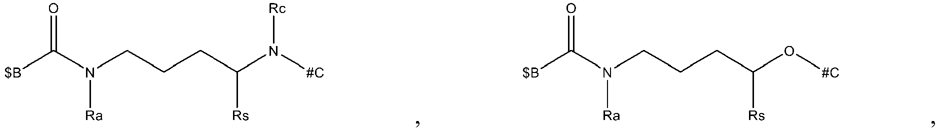 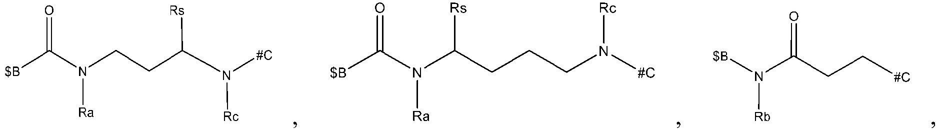 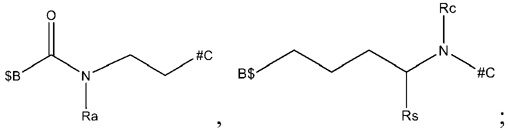
more preferably, L is selected from: 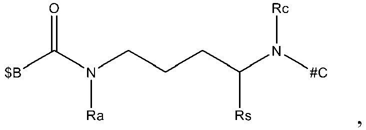 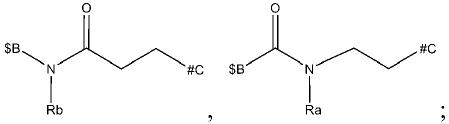
preferably, Ra, Rb, and Rc are each independently selected from H or methyl;
preferably, Rs is selected from H, D, halogen, hydroxyl, C1-6 alkyl, C1-6 alkoxy, C1-6 haloalkyl, C1-6 haloalkoxy, C3-6 cycloalkyl, 4-7-membered heterocycloalkyl, 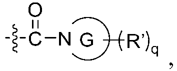 -C(=O)N(R_{L2})₂; wherein, ring G is 4-10-membered nitrogen-containing saturated heterocycloalkyl, R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, D, C1-4 alkyl, C3-6 cycloalkyl, 4-7-membered heterocycloalkyl, phenyl, 5-6-membered heteroaryl, and the C1-4 alkyl, C3-6 cycloalkyl, 4-7-membered heterocycloalkyl, phenyl, 5-6-membered heteroaryl are optionally substituted by a substituent selected from halogen, hydroxyl, cyano, C1-3 alkyl, C1-3 alkoxy;
preferably, Rs is selected from H, D, halogen, hydroxyl, C1-6 alkyl, C1-6 alkoxy, C1-6 haloalkyl, C1-6 haloalkoxy, C3-6 saturated cycloalkyl, 4-7-membered saturated heterocycloalkyl, 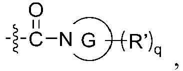 -C(=O)N(R_{L2})₂; wherein, ring G is 4-10-membered nitrogen-containing saturated heterocycloalkyl, R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, D, C1-4 alkyl, C3-6 saturated cycloalkyl, 4-7-membered saturated heterocycloalkyl, phenyl, 5-6-membered heteroaryl, and the C1-4 alkyl, C3-6 saturated cycloalkyl, 4-7-membered saturated heterocycloalkyl, phenyl, 5-6-membered heteroaryl are optionally substituted by a substituent selected from halogen, hydroxyl, cyano, C1-3 alkyl, C1-3 alkoxy; the 4-7-membered saturated heterocycloalkyl has 0, 1 or 2 nitrogen heteroatoms and 0 or 1 heteroatom selected from O and S, preferably has 1 oxygen atom, the 4-10-membered nitrogen-containing saturated heterocycloalkyl has 1 or 2 nitrogen heteroatoms and 0 or 1 heteroatom selected from O and S, preferably has 1 nitrogen heteroatom and 0 or 1 oxygen heteroatom, the 5-6-membered heteroaryl has 1, 2, or 3 nitrogen heteroatoms and 0 or 1 heteroatom selected from O and S, preferably has 1 nitrogen heteroatom;
more preferably, Rs is selected from C1-3 alkyl, C3-6 cycloalkyl (preferably C3-6 saturated cycloalkyl, for example cyclopropyl, cyclobutyl, cyclohexyl), 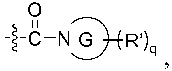 C(=O)N(R_{L2})₂; wherein, ring G is 4-10-membered nitrogen-containing saturated heterocycloalkyl (for example azetidinyl, azacyclopentyl, morpholinyl, 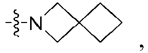 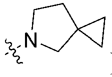 ), R' is selected from halogen (for example F, Cl), C1-3 alkyl (for example methyl, isopropyl), q is selected from 0, 1; each R_{L2} is independently selected from H, C1-3 alkyl (for example methyl, ethyl, isopropyl), C3-6 cycloalkyl (preferably C3-6 saturated cycloalkyl, for example cyclopropyl, cyclobutyl), 4-7-membered heterocycloalkyl (preferably 4-7-membered saturated heterocycloalkyl, for example oxanyl), 6-membered heteroaryl (for example pyridinyl), and the 4-7-membered heterocycloalkyl (preferably 4-7-membered saturated heterocycloalkyl), 6-membered heteroaryl are optionally substituted by a substituent selected from C1-3 alkyl; preferably, Rs is selected from H, methyl, ethyl, propyl, cyclopropyl, cyclobutyl, cyclopentyl, cyclohexyl.

In some embodiments, L is selected from: 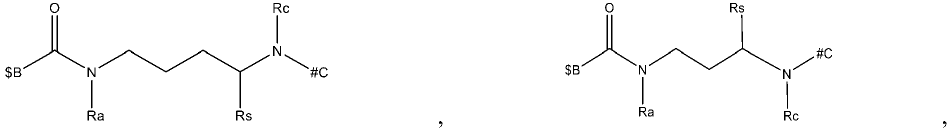 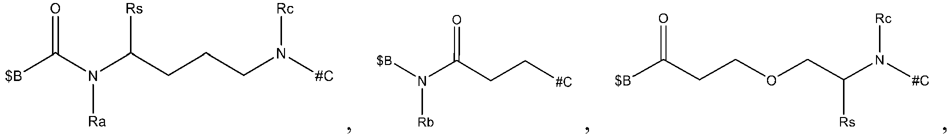 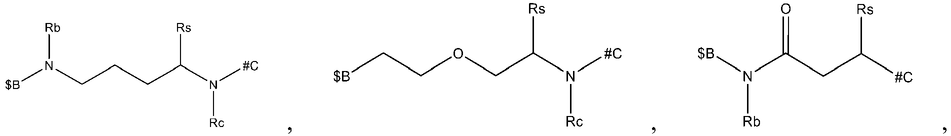 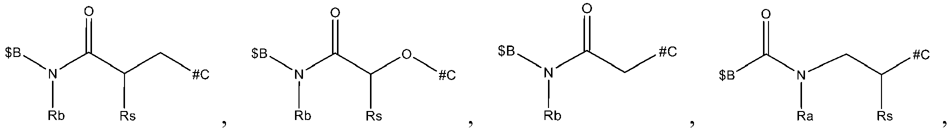 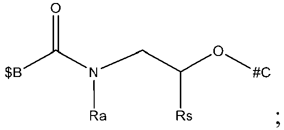
wherein, $B represents the site of attachment to ring B, #C represents the site of attachment to ring C, Ra, Rb, and Rc are as defined above, Rs is as defined above;
preferably, Ra, Rb, and Rc are each independently selected from H or methyl;
preferably, Rs is selected from H or the following substituted or unsubstituted groups: methyl, ethyl, propyl, cyclopropyl, cyclobutyl, cyclopentyl, cyclohexyl.

In some embodiments, L is selected from 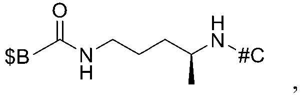 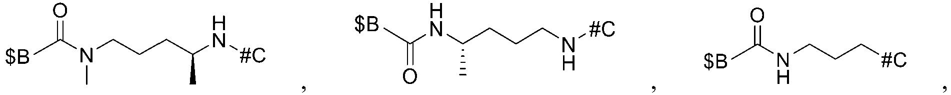 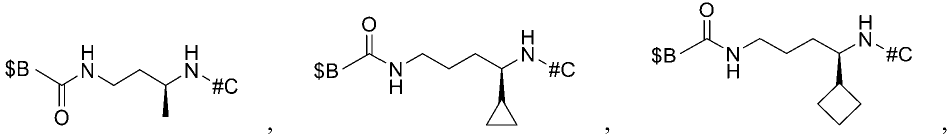 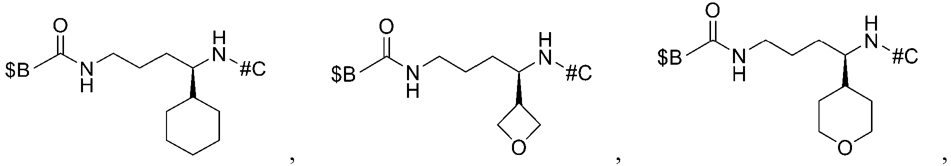 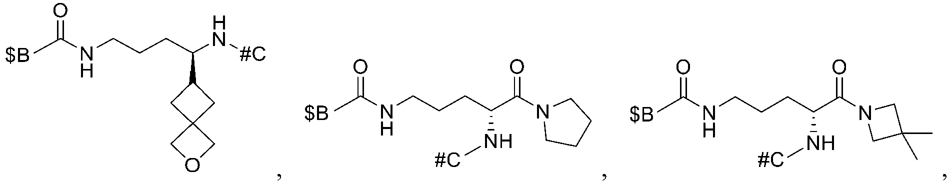 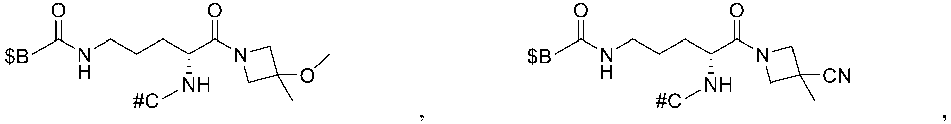 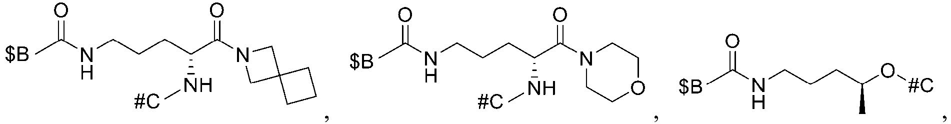 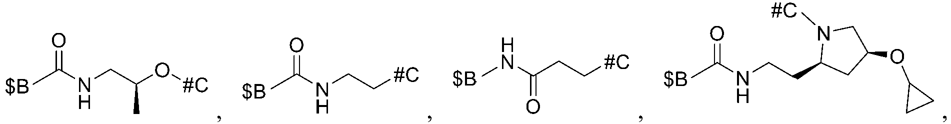 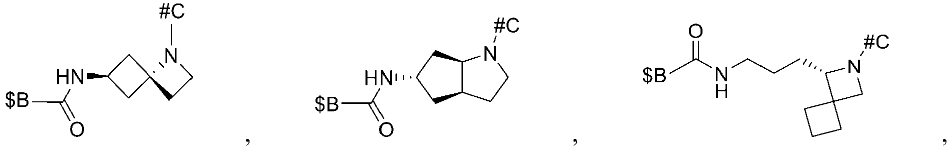 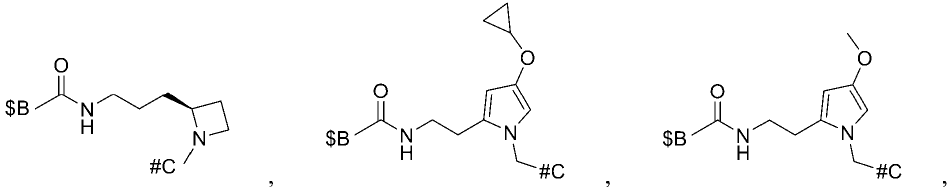 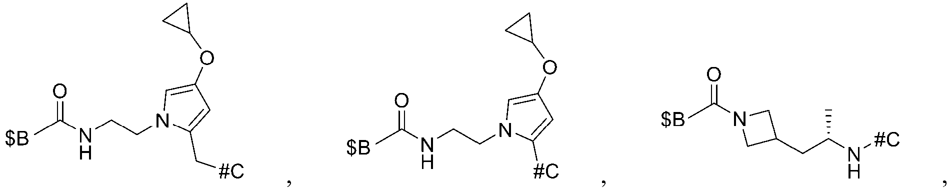 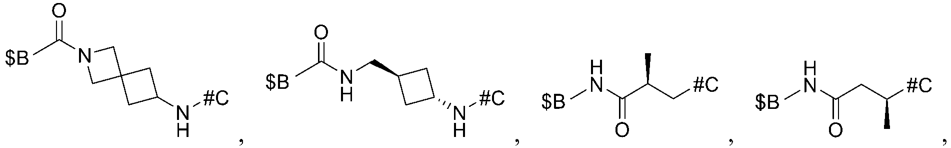 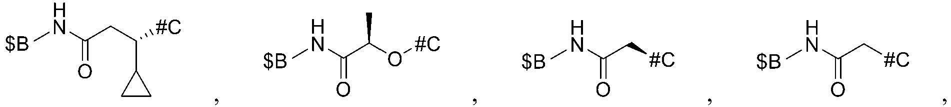 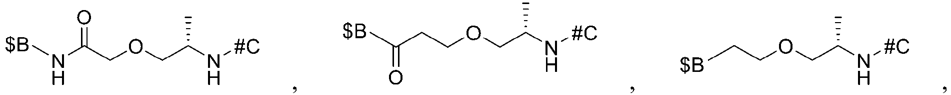 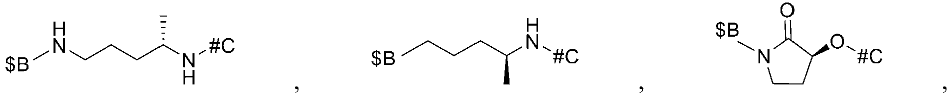 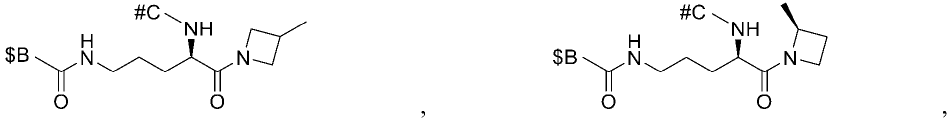 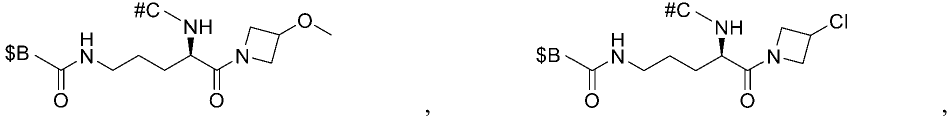 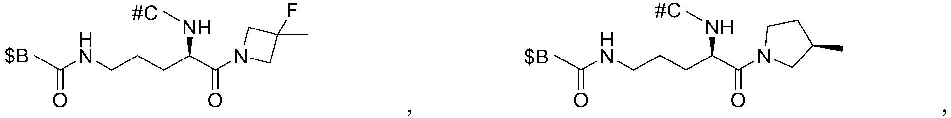 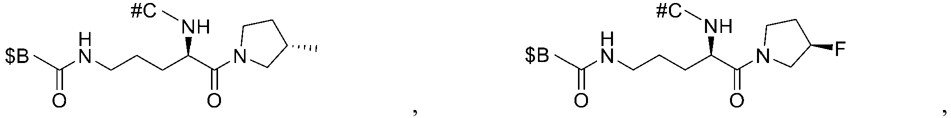 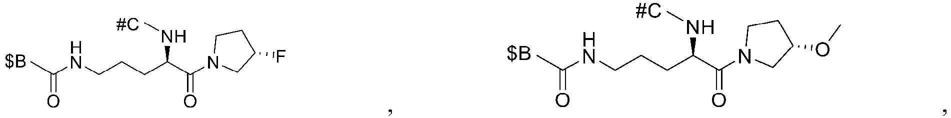 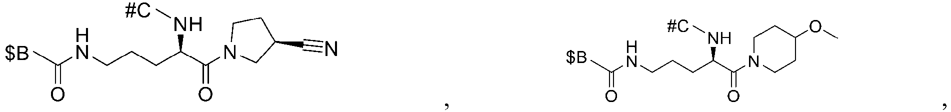 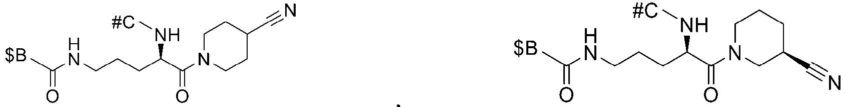 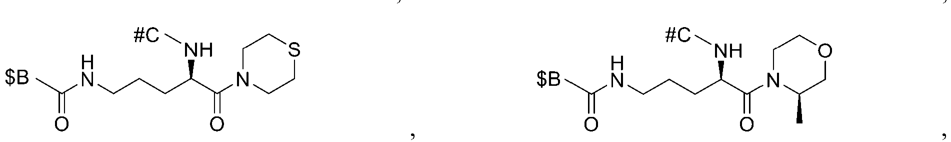 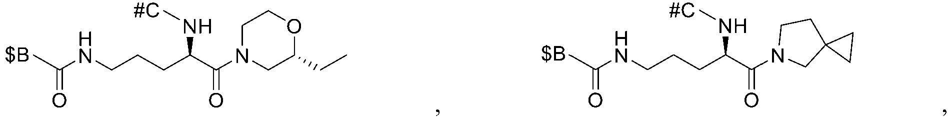 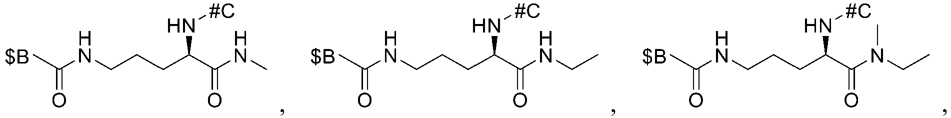 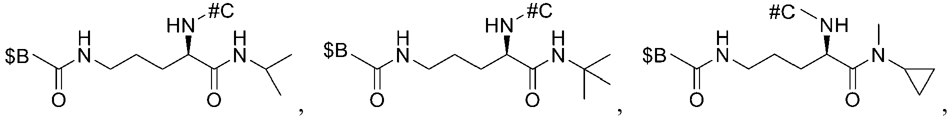 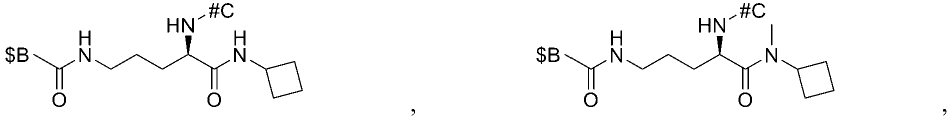 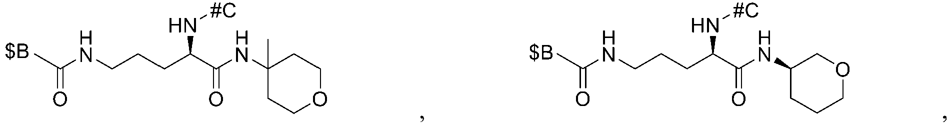 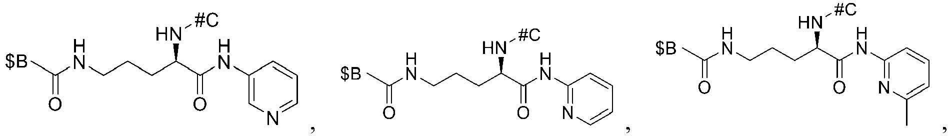 $B-CH₂-CH₂-#C, $B-CH₂-CH₂-CH₂-#C, 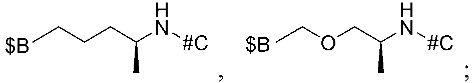 $B represents the site of attachment to ring B, #C represents the site of attachment to ring C; preferably, L is selected from 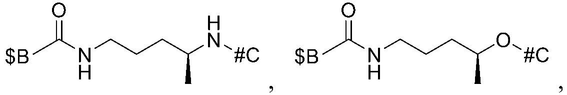 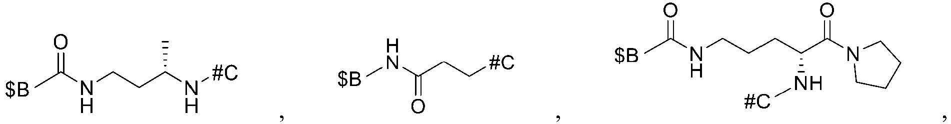 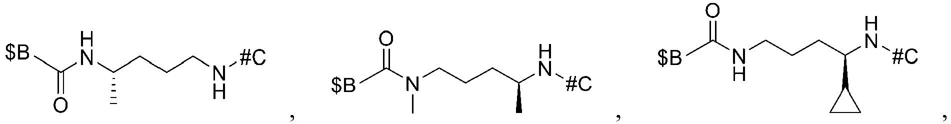 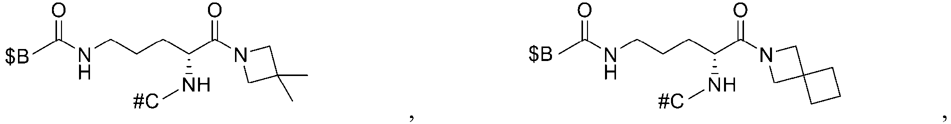 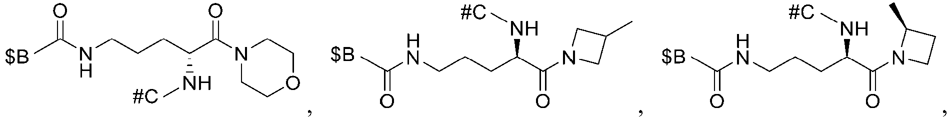 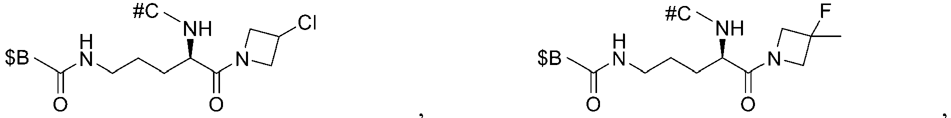 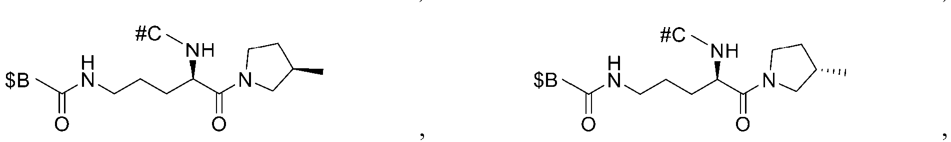 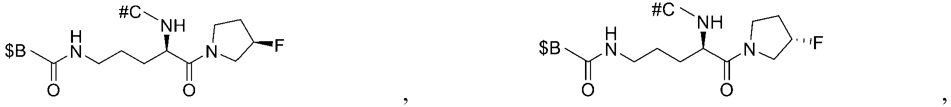 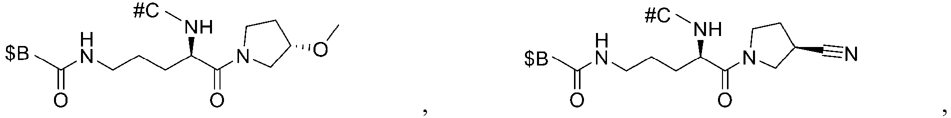 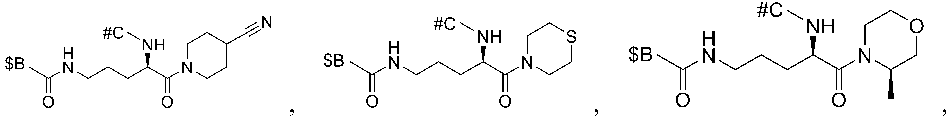 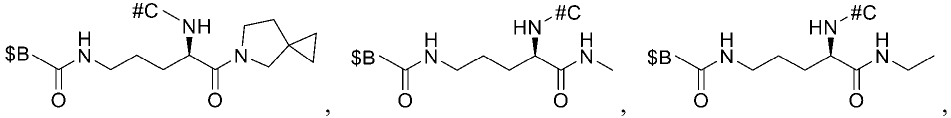 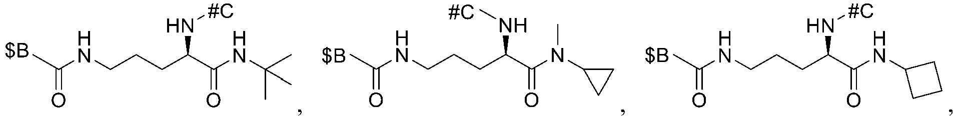 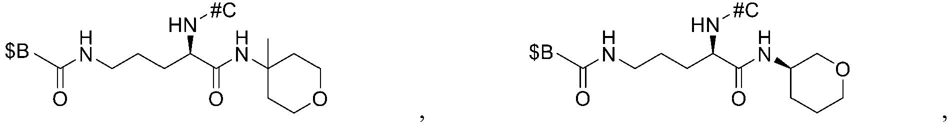 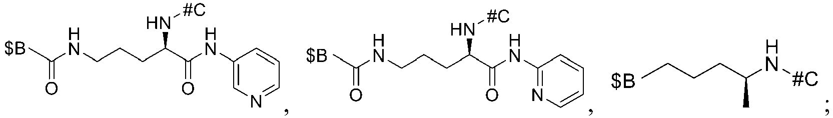 preferably, L is selected from 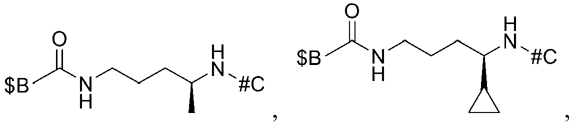 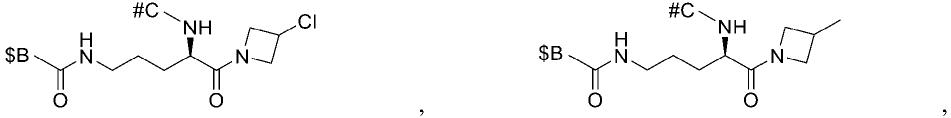 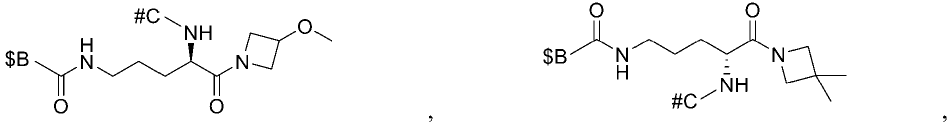 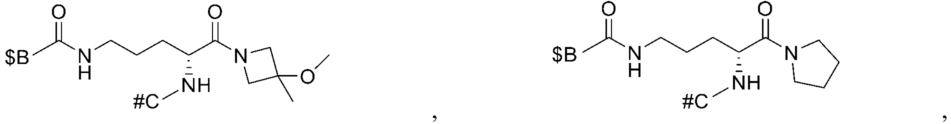 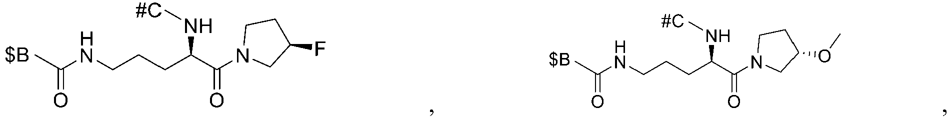 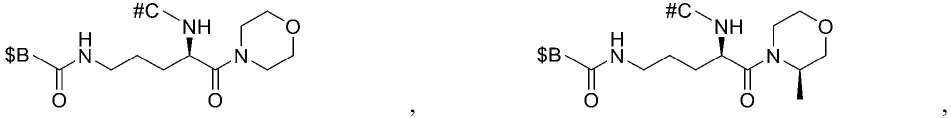 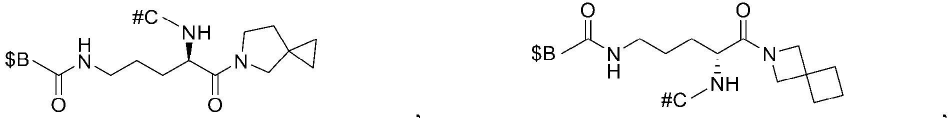 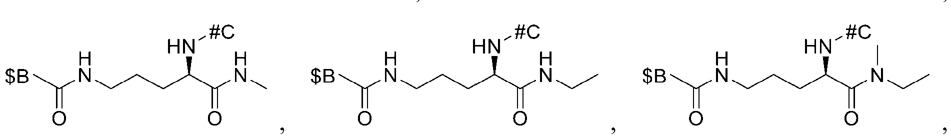 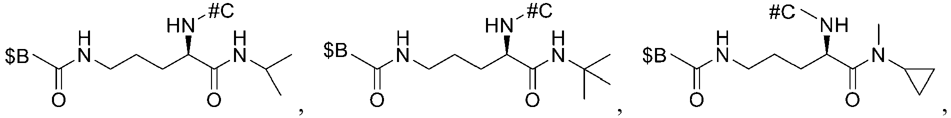 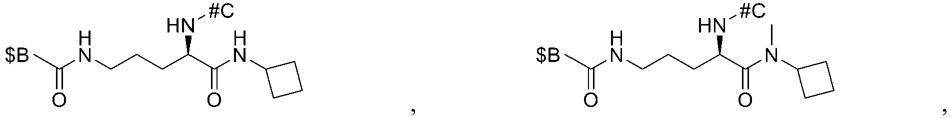 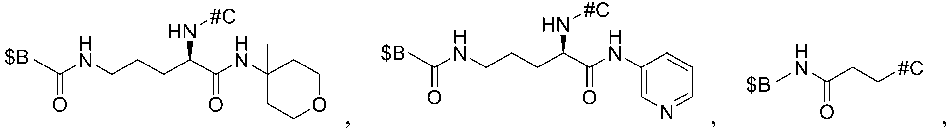 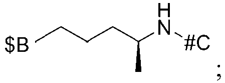 preferably, L is selected from

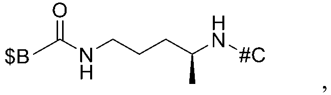

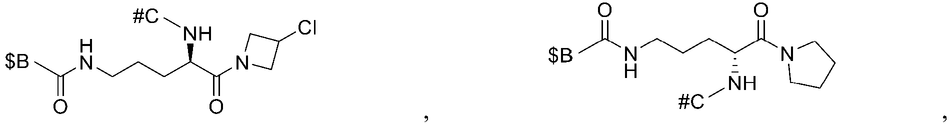

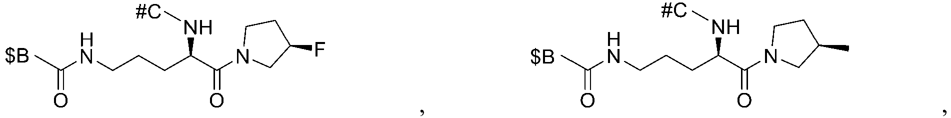

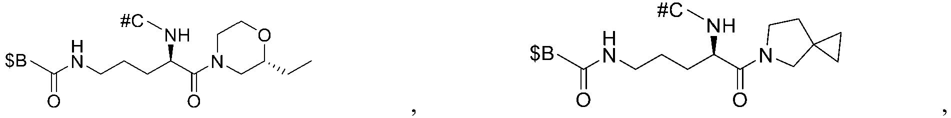

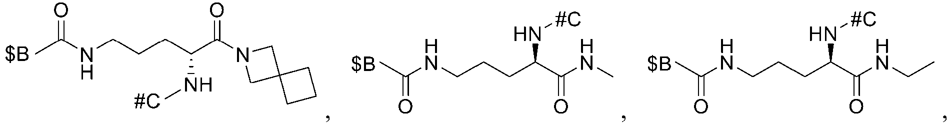

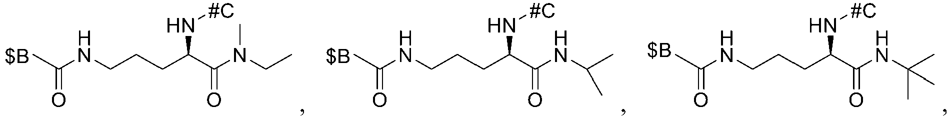

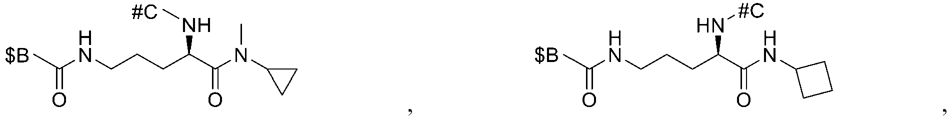

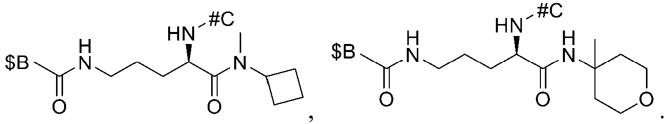

In some embodiments, L is selected from 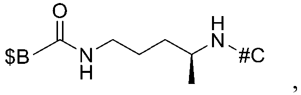 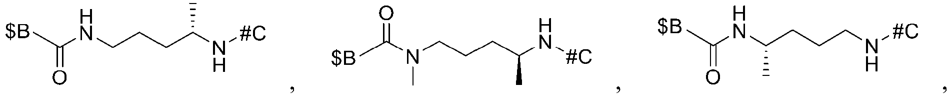 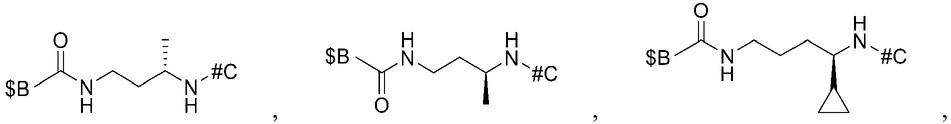 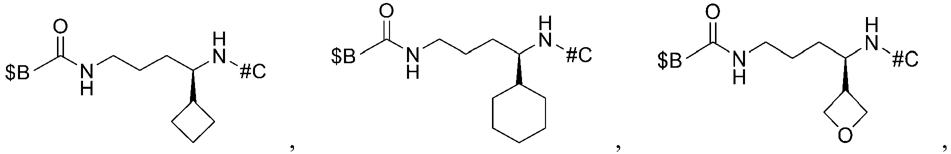 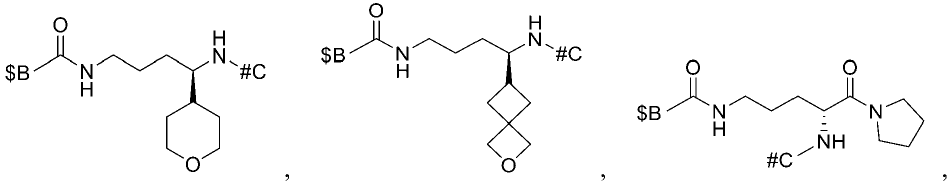 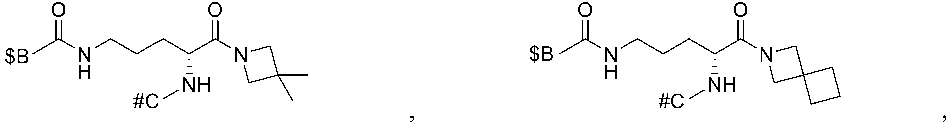 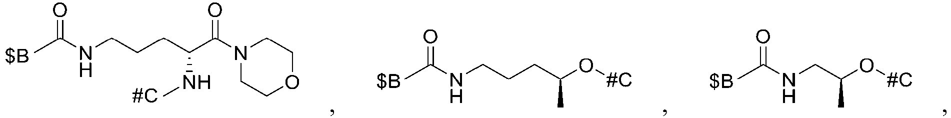 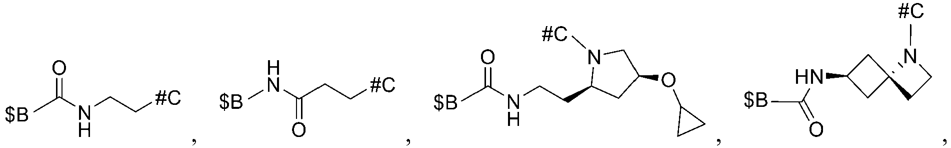 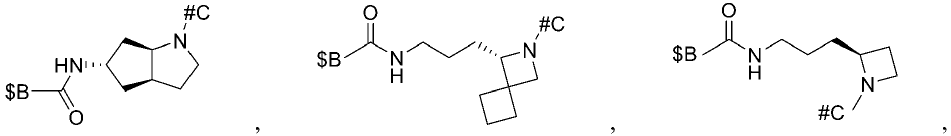 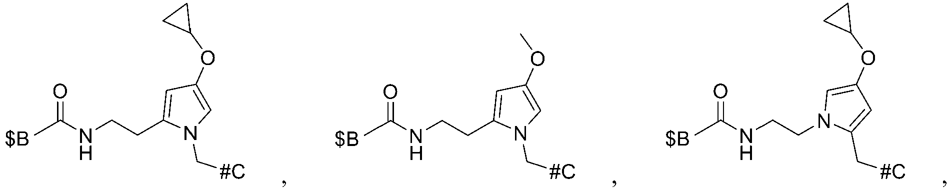 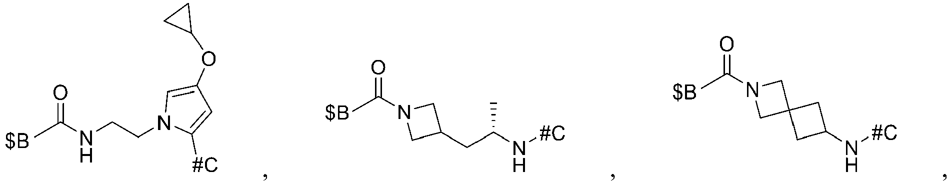 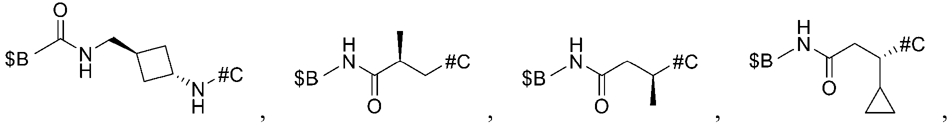 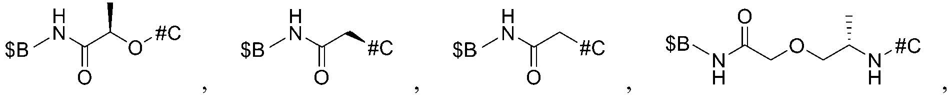 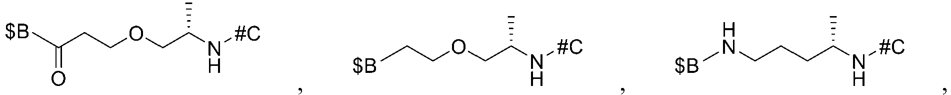 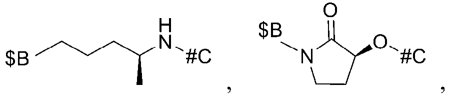 $B-CH₂-CH₂-#C; $B represents the site of attachment to ring B, #C represents the site of attachment to ring C.

In some embodiments, L is selected from

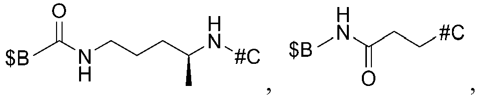

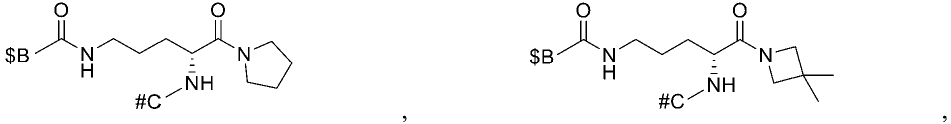

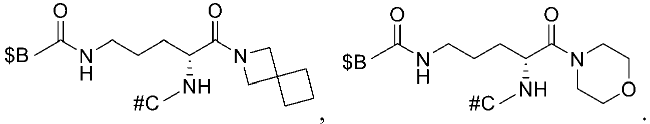

In some embodiments, ring C is selected from the following groups:

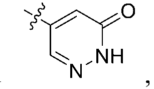

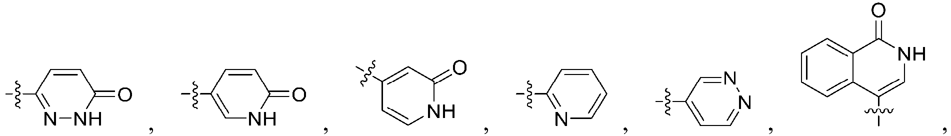

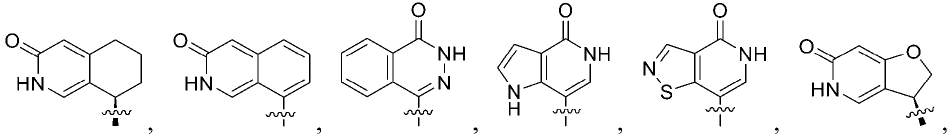

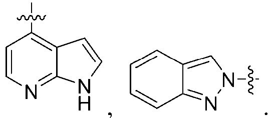

In some embodiments, ring C is selected from the following groups:

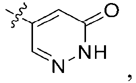

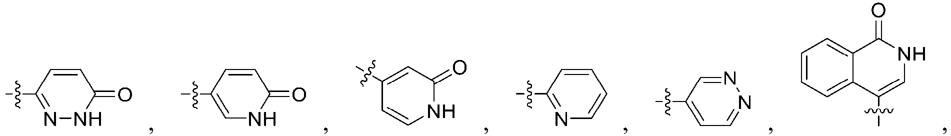

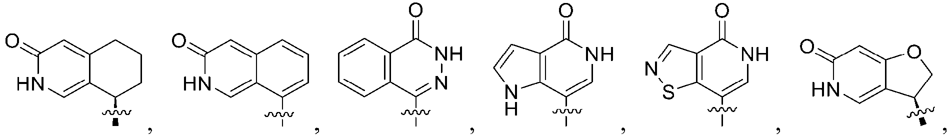

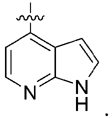

In some embodiments, ring C is selected from :

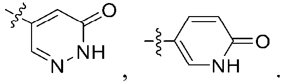

In some embodiments, ring C is

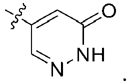

In some embodiments, 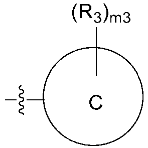 is selected from :

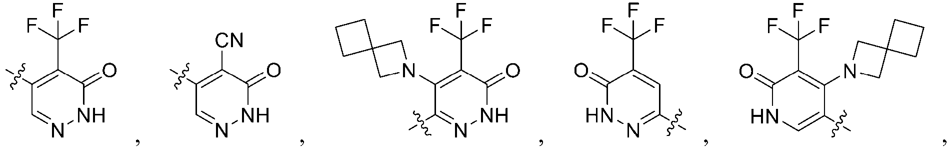

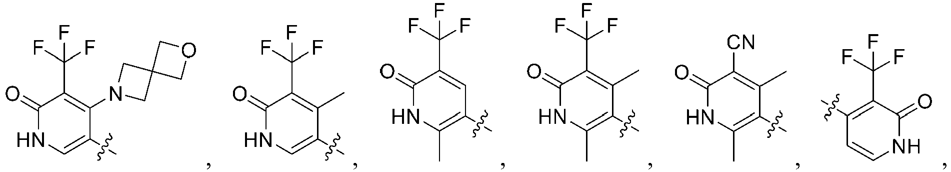

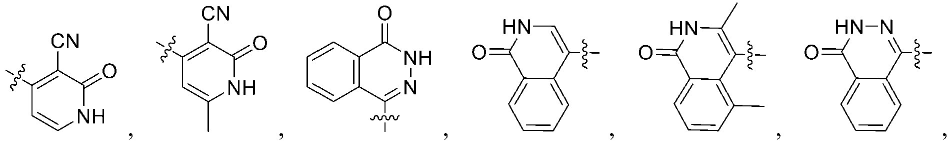

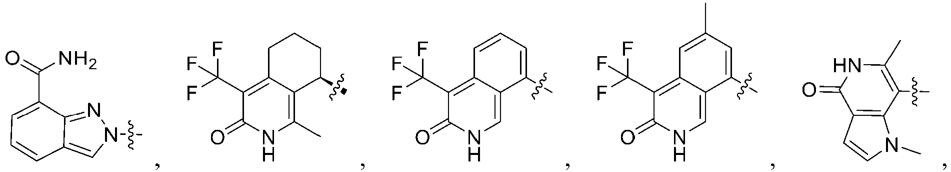

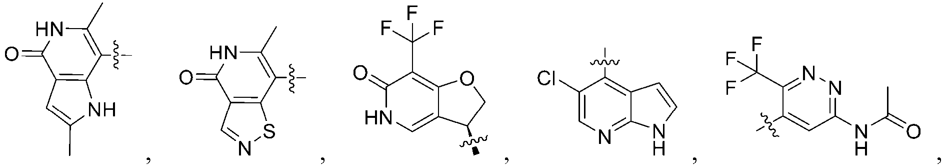

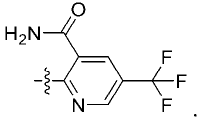

In some embodiments, 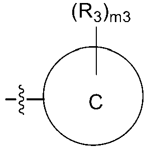 is selected from :

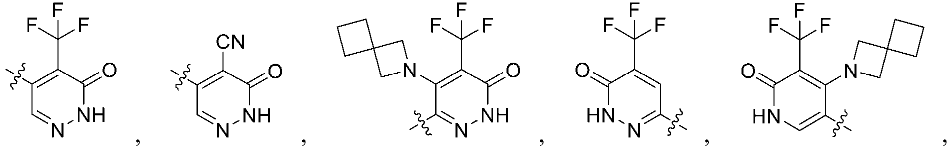

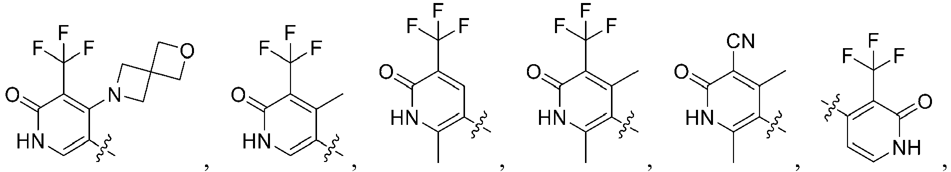

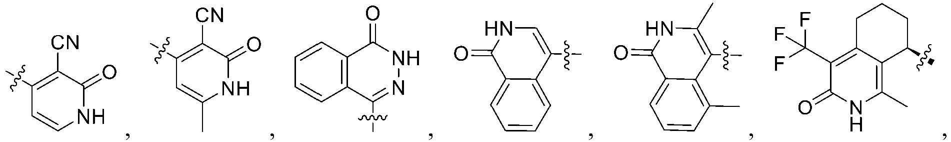

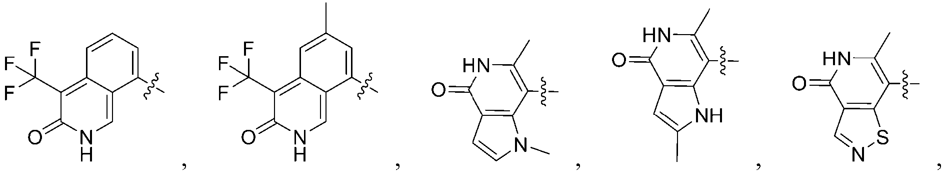

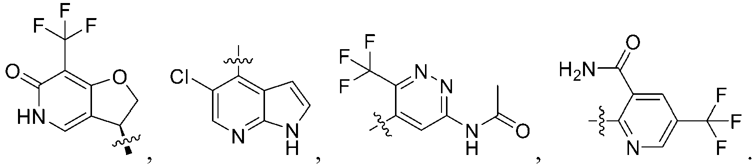

In some embodiments, 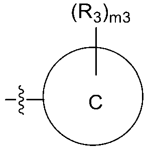 is selected from :

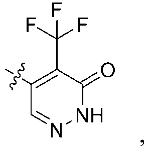

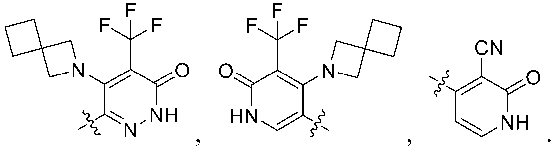

In some embodiments, 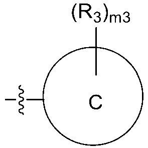 is selected from : 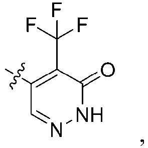 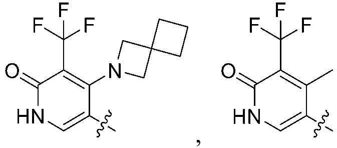 .In some embodiments, 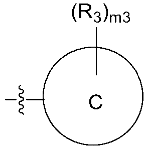 is selected from :

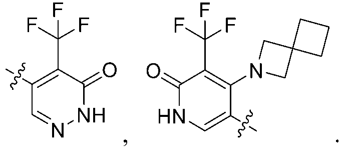

In some embodiments, 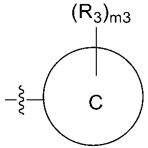 is selected from:

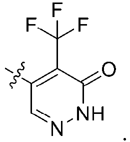

In some embodiments, R₃ is selected from halogen, -CN, C1-3 alkyl, C1-3 haloalkyl, - NReRf, -NReC(=O)Rf, -C(=O)-NReRf, wherein, Re and Rf are each independently selected from H, C1-3 alkyl, or Re and Rf together with the atoms that they are attached to, form 4-7-membered heterocycloalkyl (for example 7-membered nitrogen-containing spiro heterocycloalkyl);
preferably, R₃ is selected from halogen, -CN, C1-3 alkyl, C1-3 haloalkyl, -NReRf, - NReC(=O)Rf, -C(=O)-NReRf, wherein, Re and Rf are each independently selected from H, C1-3 alkyl, or Re and Rf together with the atoms that they are attached to, form 4-7-membered saturated heterocycloalkyl (for example 7-membered saturated nitrogen-containing spiro heterocycloalkyl); the 4-7-membered saturated heterocycloalkyl or 7-membered saturated nitrogen-containing spiro heterocycloalkyl has 1 or 2 nitrogen heteroatoms and 0 or 1 heteroatom selected from O and S, preferably has 1 nitrogen heteroatom and 0 or 1 oxygen heteroatom. preferably, R₃ is selected from halogen, -CN, C1-3 alkyl, C1-3 haloalkyl, - NReC(=O)Rf, -C(=O)-NReRf, wherein, Re and Rf are each independently selected from H, C1-3 alkyl, or Re and Rf together with the atoms that they are attached to, form 4-7-membered heterocycloalkyl; preferably, when R₃ is selected from -NReRf, the Re and Rf together with the atoms that they are attached to, form 4-7-membered saturated heterocycloalkyl, preferably Re and Rf together with the atoms that they are attached to, form 7-membered saturated spiro heterocycloalkyl; wherein any of the heterocycloalkyl groups has 1 or 2 nitrogen heteroatoms and 0 or 1 heteroatom selected from O and S, preferably has 1 nitrogen heteroatom and 0 or 1 oxygen heteroatom;
preferably, when R₃ is selected from -NReC(=O)Rf or -C(=O)-NReRf, the Re and Rf are each independently selected from H, C1-3 alkyl;
further preferably, R₃ is selected from Cl, methyl, -CF₃, -CN, -NHC(=O)CH₃, -C(=O)NH₂, 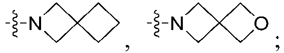
further preferably, R₃ is selected from -CF₃, -CN, 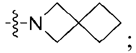
further preferably, R₃ is selected from -CF₃, 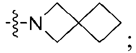
further preferably, R₃ is selected from -CF₃, methyl, 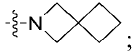
preferably, m3 is selected from 1, 2, 3; more preferably, m1 is selected from 1 or 2.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in any one of formulas I-1 to I-4, I-7 to I-10: 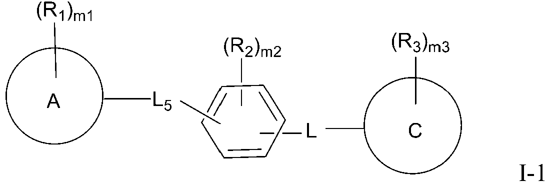 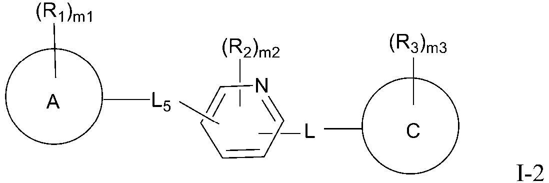 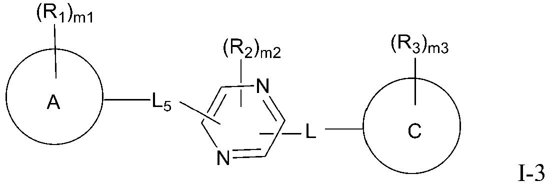 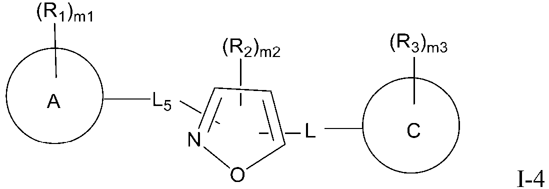 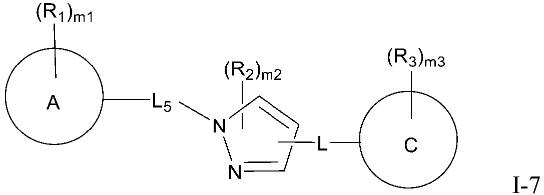 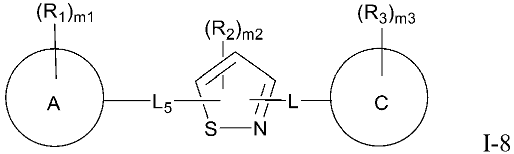 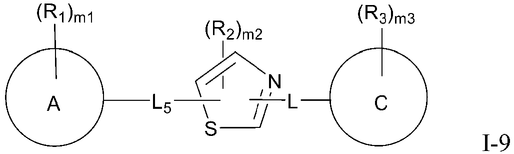 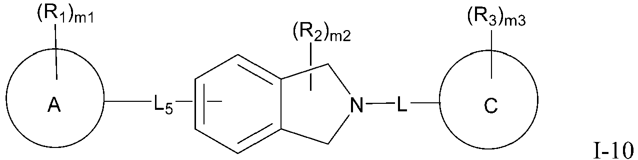 wherein, ring A, ring C, R₁, R₂, R₃, L, L₅, m1, m2, and m3 are as defined above.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in any one of formulas I-1 to I-4.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in formula I-5 or I-6: 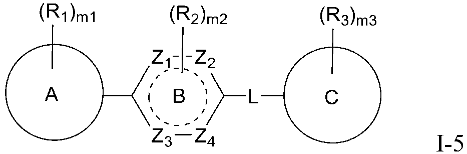 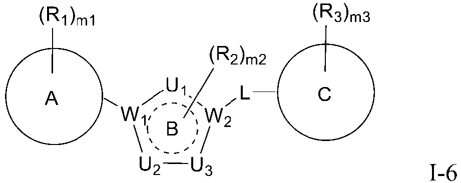 wherein,
in formula I-5, ring B is selected from phenyl or 6-membered heteroaryl, Z₁, Z₂, Z₃, Z₄ are each independently selected from N or CH, ring A, ring C, R₁, R₂, R₃, L, m1, m2, and m3 are as defined above;
preferably, at most two of Z₁, Z₂, Z₃, and Z₄ are N; preferably, ring B is selected from phenyl, pyrimidinyl, pyridinyl, pyrazinyl, pyridazinyl; preferably, ring B is selected from phenyl, pyridinyl, pyrazinyl; preferably, ring B is selected from the following groups 1) -5):
   1) Z₁, Z₂, Z₃, and Z₄ are all CH;
   2) Z₁ and Z₂ are both CH, Z₃ and Z₄ are both N;
   3) Z₁ and Z₄ are both CH, Z₂ and Z₃ are both N;
   4) only one of Z₁, Z₂, Z₃, and Z₄ is N;
   5) Z₁ and Z₃ are both CH, one of Z₂ and Z₄ is N and the other is CH;
in formula I-6, ring B is selected from 5-membered heteroaryl, U₁, U₂, U₃ are each independently selected from N, O, S, CH, and at most one of U₁, U₂, U₃ is S, at most one of U₁, U₂, U₃ is O, W₁ and W₂ are each independently selected from N, CH, ring A, ring C, R₁, R₂, R₃, L, m1, m2, and m3 are as defined above;
preferably, ring B is selected from the following groups 1) -5):
   1) Z₁, Z₂, Z₃, and Z₄ are all CH;
   2) Z₁ and Z₂ are both CH, Z₃ and Z₄ are both N;
   3) Z₃ and Z₄ are both CH, Z₁ and Z₂ are both N;
   4) only one of Z₁, Z₂, Z₃, and Z₄ is N;
   5) Z₁ and Z₃ are both CH, one of Z₂ and Z₄ is N and the other is CH;
in formula I-6, ring B is selected from 5-membered heteroaryl, U₁, U₂, and U₃ are each independently selected from N, O, S, CH, and at most one of U₁, U₂, U₃ is S, W₁ and W₂ are each independently selected from N, CH, ring A, ring C, R₁, R₂, R₃, L, m1, m2, and m3 are as defined above;
preferably, ring B contains 2 or 3 heteroatoms each independently selected from N, O, S;
preferably, ring B is selected from pyrazolyl, thiazolyl, isothiazolyl, isoxazolyl, triazolyl (for example 1,2,3-triazolyl), thiadiazolyl (for example 1,3,4-thiadiazolyl);
preferably, at least one of U₁, U₂, and U₃ is N; at most one of W₁ and W₂ is N;
preferably, at most two of U₁, U₂, and U₃ are heteroatoms;
preferably, U₁, U₂, and U₃ are each independently selected from N, O, CH;
preferably, U₁ and W₂ are CH; U₃ and W₁ are CH, N; U₂ is O, N;
preferably, ring B is selected from isoxazolyl, pyrazolyl;
preferably, ring B is selected from isoxazolyl;
preferably, U₁ is CH, U₂ is O, U₃ is N, W₁ is CH, W₂ is CH; or W₁ and U₂ are N, U₁, W₂, and U₃ are CH;
preferably, U₁ is CH, U₂ is O, U₃ is N, W₁ is CH, W₂ is CH.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in any one of formula II-1 or II-2: 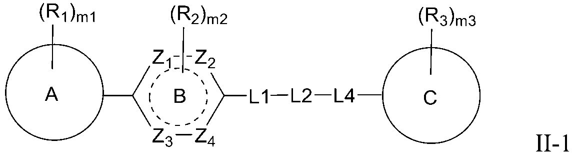 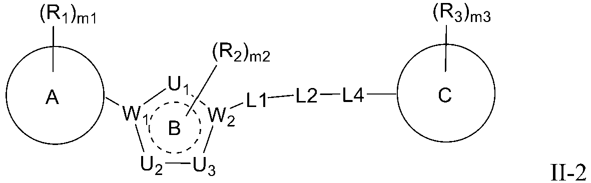
wherein, L1 is selected from $B-C(=O)-NRa-#L2, $B-NRb-C(=O)-#L2; Ra and Rb are as defined above;
L2 is selected from C1-6 alkylene, wherein, the C1-6 alkylene is optionally substituted by 1 or 2 substituents selected from oxo, C1-3 alkyl, C3-6 cycloalkyl, 4-7-membered nitrogen-containing saturated heterocycloalkyl, -C(=O)-4-7-membered nitrogen-containing saturated heterocycloalkyl, -C(=O)N(R_{L2})₂, the 4-7-membered nitrogen-containing saturated heterocycloalkyl, -C(=O)-4-7-membered nitrogen-containing saturated heterocycloalkyl, as a substituent of C1-6 alkylene, are optionally substituted by a substituent selected from halogen, cyano, C1-3 alkyl, C1-3 alkoxy, C1-3 haloalkyl, C1-3 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy;
preferably, L2 is selected from C1-6 alkylene, wherein, the C1-6 alkylene is optionally substituted by 1 or 2 substituents selected from oxo, C1-3 alkyl, C3-6 cycloalkyl, 4-7-membered nitrogen-containing saturated heterocycloalkyl, -C(=O)-4-7-membered nitrogen-containing saturated heterocycloalkyl, the 4-7-membered nitrogen-containing saturated heterocycloalkyl, -C(=O)-4-7-membered nitrogen-containing saturated heterocycloalkyl, as a substituent of C1-6 alkylene, are optionally substituted by a substituent selected from C1-3 alkyl;
preferably, L2 is selected from C2-5 alkylene; wherein, the C2-5 alkylene is optionally substituted by a substituent selected from C1-3 alkyl, C3-6 cycloalkyl (for example cyclopropyl, cyclobutyl, cyclohexyl), 4-7-membered heterocycloalkyl (for example oxetanyl, oxanyl, 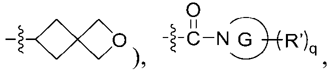 -C(=O)N(R_{L2})₂; wherein, ring G is 4-10-membered nitrogen-containing saturated heterocycloalkyl (for example azetidinyl, azacyclopentyl, morpholinyl, 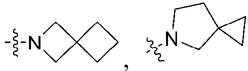 ), R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 5-6-membered heteroaryl (for example pyridinyl), and the C1-3 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy;
L4 is selected from a bond, O, -NRc-, wherein, Rc is selected from H, C1-3 alkyl.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in any one of formulas III-1 to III-6: 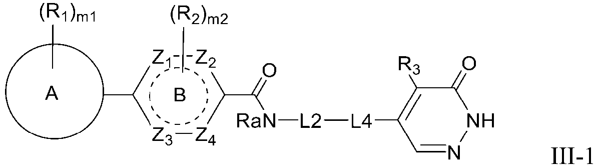 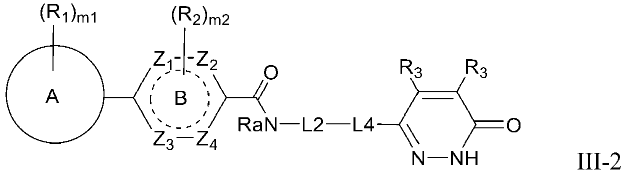 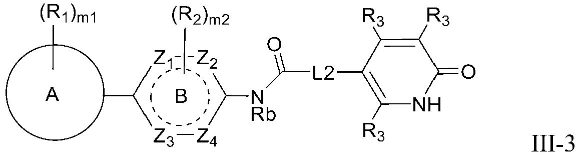 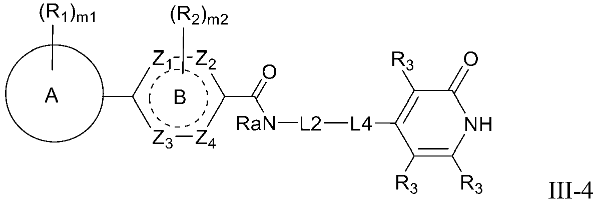 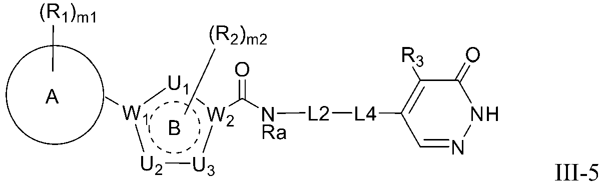 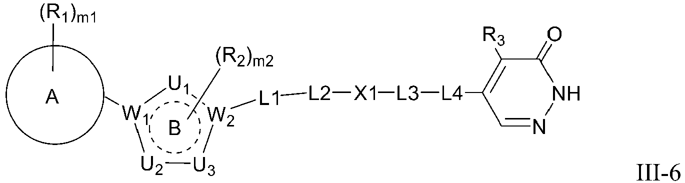 wherein, Z₁, Z₂, Z₃, Z₄, U₁, U₂, U₃, W₁, and W₂ are as defined above; L1, L2, L3, L4, Ra, and Rb are as defined above; ring A, R₁, R₂, R₃, m1, m2, and m3 are as defined above.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in any one of formulas IV-1 to IV-4: 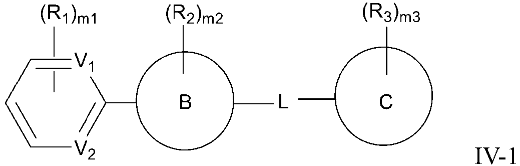 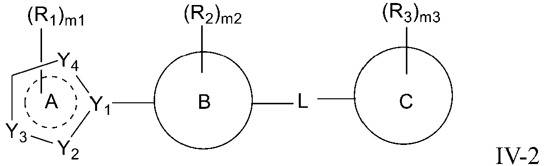 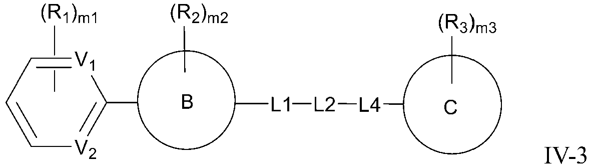 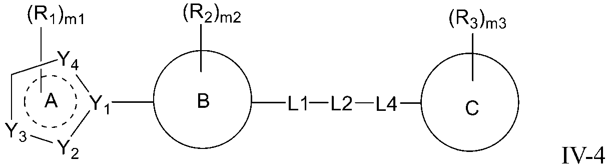
wherein, V₁ and V₂ are each independently selected from N, CH, Y₁ is N or CH, Y₂, Y₃, and Y₄ are each independently selected from N, CH, S, ring A is 5-membered heteroaromatic ring, ring B, ring C, L, R₁, R₂, R₃, m1, m2, and m3 are as defined above; L1, L2, and L4 are as defined above.
preferably, at least one of V₁ and V₂ is N; more preferably, V₁ and V₂ are both N;
preferably, only two of Y₁, Y₂, Y₃, and Y₄ are heteroatoms;
preferably, Y₂ is N, one of Y₁, Y₃, and Y₄ is a heteroatom;
preferably, Y₁ and Y₂ are N, Y₃ and Y₄ are CH; or Y₁ and Y₃ are CH, Y₂ and Y₄ are S and N, respectively; or Y₁ and Y₄ are CH, Y₂ is N, Y₃ is S.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in any one of formula V-1 to V-4: 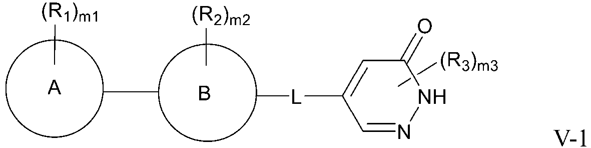 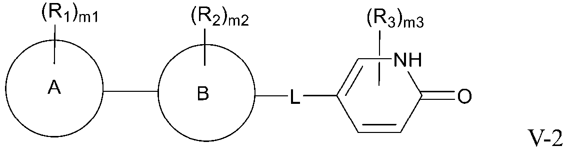 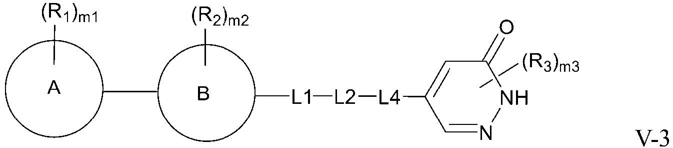 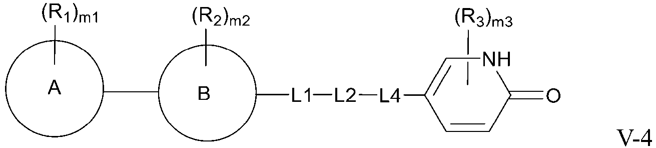 wherein, ring A, ring B, ring C, L, R₁, R₂, R₃, m1, m2, and m3 are as defined above; L1, L2, and L4 are as defined above.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in any one of formulas VI-1 to VI-6: 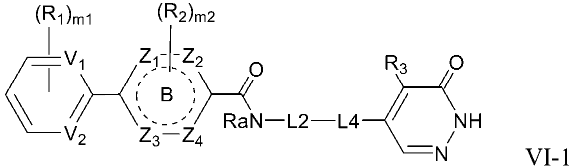 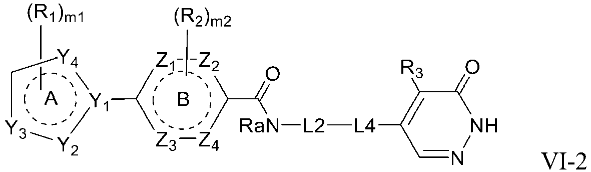 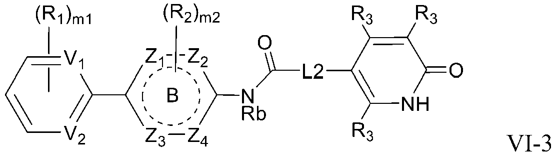 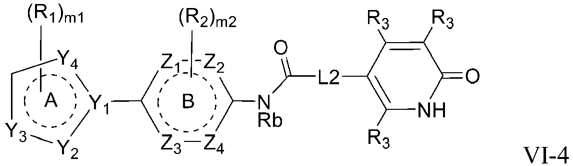 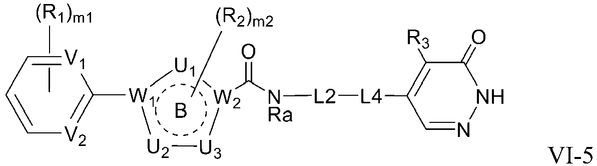 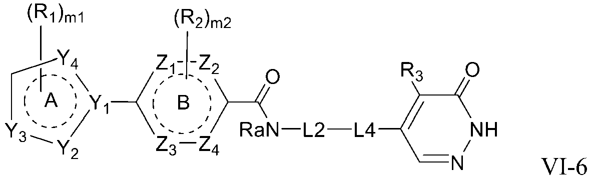 wherein, L1, L2, L3, L4, Ra, and Rb are as defined above; Z₁, Z₂, Z₃, Z₄, U₁, U₂, U₃, W₁, and W₂ are as defined above; ring A, V₁, V₂, Y₁, Y₂, Y₃, and Y₄ are as defined above.

In some embodiments, 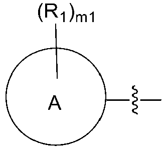 is selected from 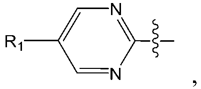 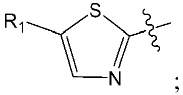 and/or 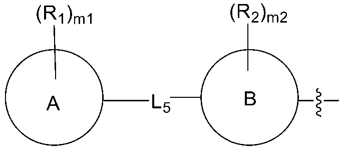 is selected from: 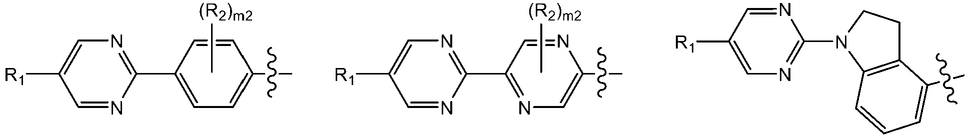 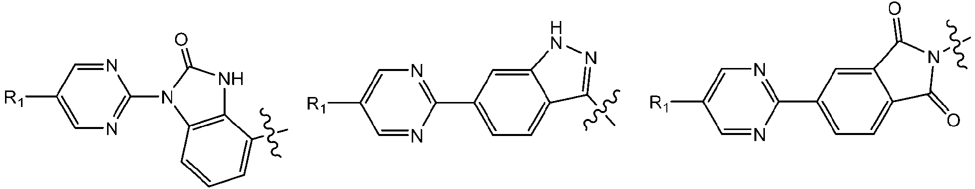 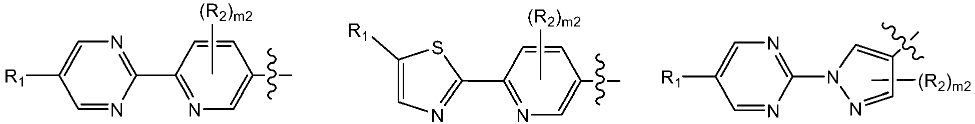 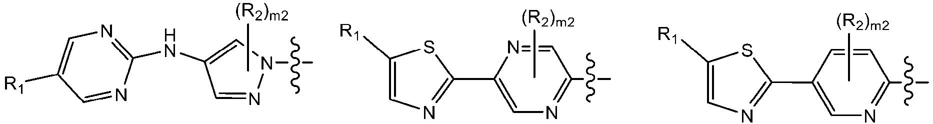 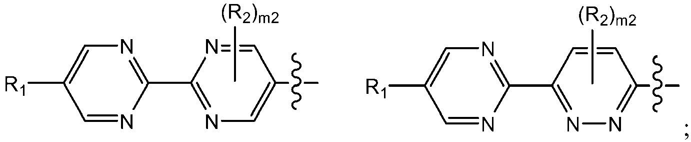 and/or 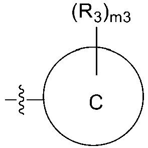 is selected from 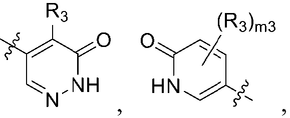
wherein, R₁ is selected from halogen, -OH, -CN, -NReRf, -CF₃, -CHF₂, substituted or unsubstituted C3-6 cycloalkyl, Re and Rf are each independently selected from H, substituted or unsubstituted C1-6 alkyl, substituted or unsubstituted C3-6 cycloalkyl, or Re and Rf together with the atoms that they are attached to, form 4-7-membered heterocycloalkyl;
each R₂ is independently selected from halogen, -OH, -CN, substituted or unsubstituted C1-6 alkyl, substituted or unsubstituted C1-6 alkoxy, substituted or unsubstituted C3-6 cycloalkyl;
each R₃ is independently selected from halogen, -OH, -CN, -NReRf, -CF₃, -CHF₂, substituted or unsubstituted C3-6 cycloalkyl, Re and Rf are each independently selected from H, substituted or unsubstituted C1-6 alkyl, substituted or unsubstituted C3-6 cycloalkyl, or Re and Rf together with the atoms that they are attached to, form 4-7-membered heterocycloalkyl; and/or
m2 and m3 are each independently selected from 0, 1, 2, or 3.

In some embodiments, ring A is selected from phenyl, pyrimidinyl, thiazolyl, isothiazolyl, imidazolyl, pyrazolyl, pyridinyl, pyridazinyl, oxazolyl, isoxazolyl, 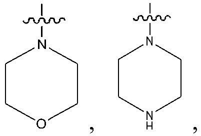 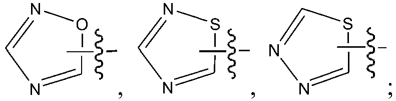
ring B is selected from phenyl, pyridinyl, pyrazolyl, pyrazinyl, oxazolyl, isoxazolyl, benzopyrazolyl, thiazolyl, isothiazole, pyridazinyl, pyrimidinyl, triazolyl, thiadiazole, indolinyl, benzimidazolyl, dihydrobenzimidazolyl, indolinyl, indazolyl, isoindolinyl, pyridopyridinyl, pyrrolopyrimidinyl;
ring C is selected from the following groups: 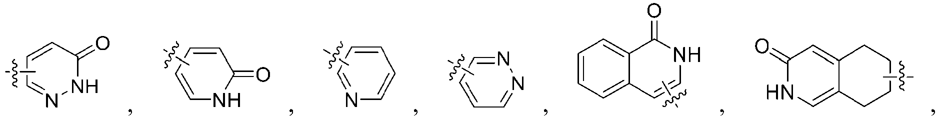 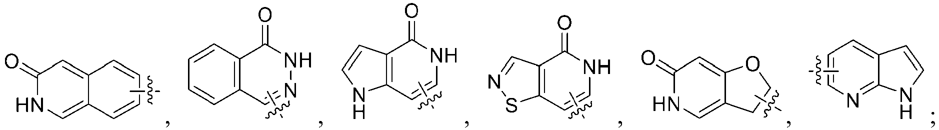 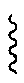 terminus is attached to L; L₅ is selected from a bond; and
L is selected from: 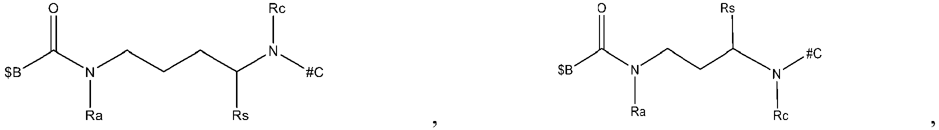 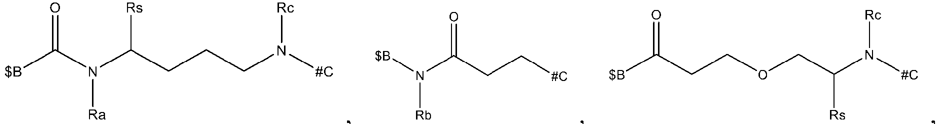 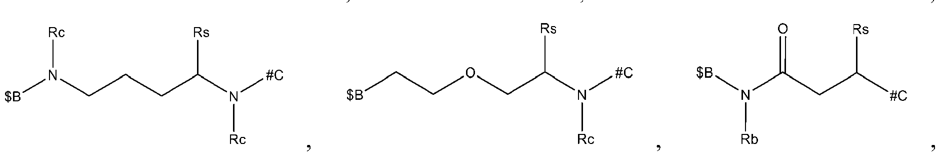 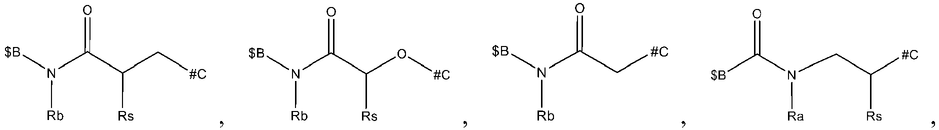 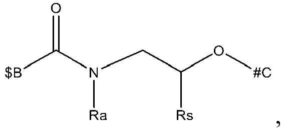
wherein,
$B represents the site of attachment to ring B, #C represents the site of attachment to ring C, Ra, Rb, and Rc are as defined above, Rs is as defined above;
preferably, Ra, Rb, and Rc are each independently selected from H or methyl;
preferably, Rs is selected from H, methyl, cyclopropyl, cyclobutyl, cyclopentyl, cyclohexyl, 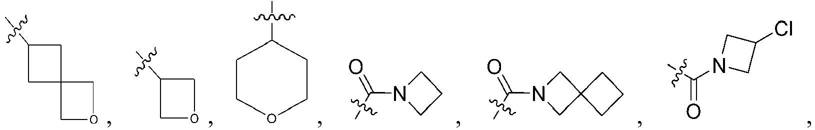 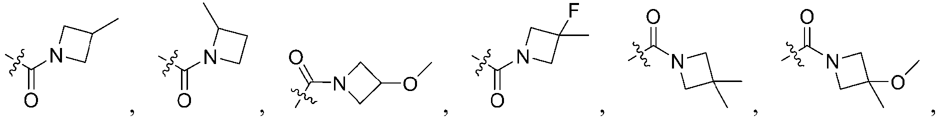 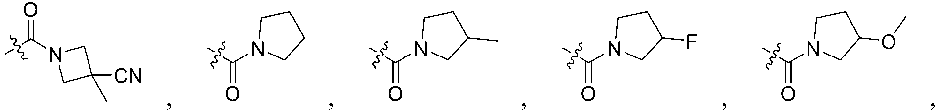 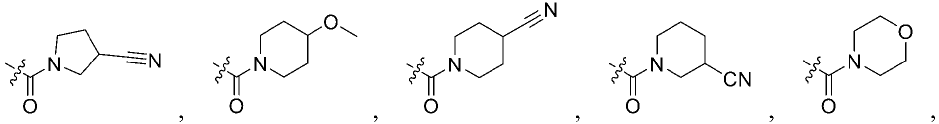 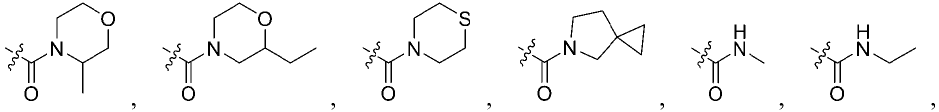 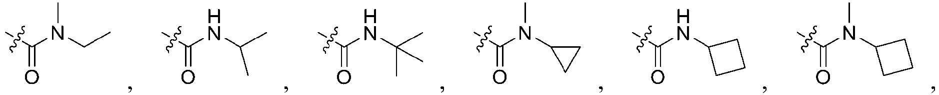 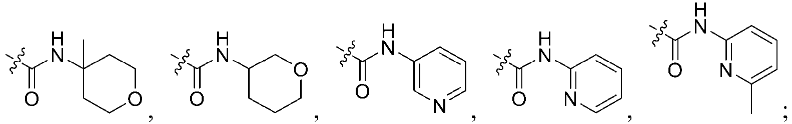
preferably, Rs is selected from H or the following substituted or unsubstituted groups: methyl, ethyl, propyl, cyclopropyl, cyclobutyl, cyclopentyl, cyclohexyl,

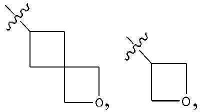

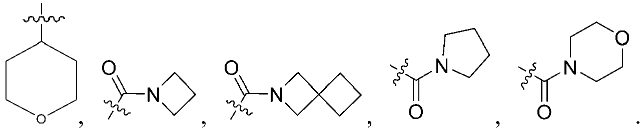

In some embodiments, ring A is selected from pyrimidinyl, thiazolyl, isothiazolyl, pyrazolyl, pyridinyl;
ring B is selected from: 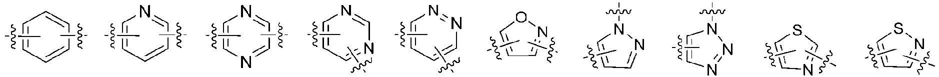 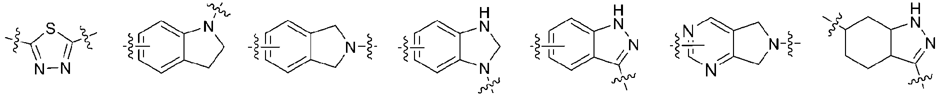 ; 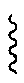 terminus is attached to L terminus or L₅;
ring C is selected from: 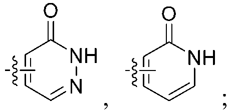
L₅ is a bond;
L is selected from: 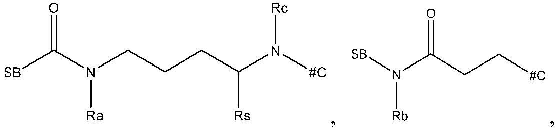 $B represents the site of attachment to ring B, #C represents the site of attachment to ring C;
Ra, Rb, and Rc are as defined above, Rs is as defined above;
preferably, Ra, Rb, and Rc are each independently selected from H or methyl;
preferably, Rs is selected from H, methyl, cyclopropyl, cyclobutyl, cyclohexyl, 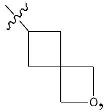 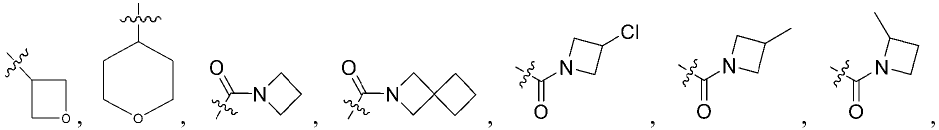 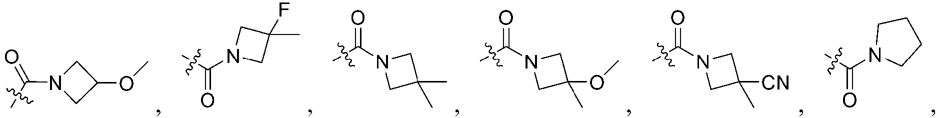 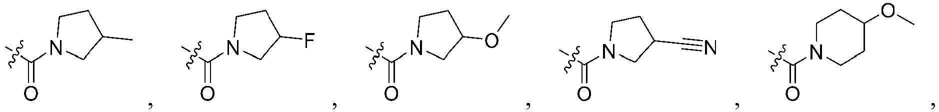 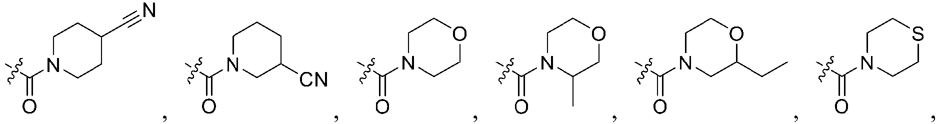 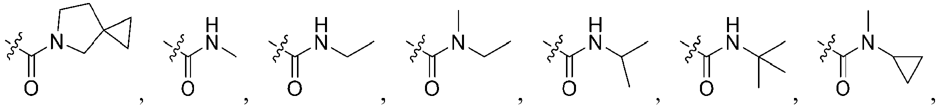 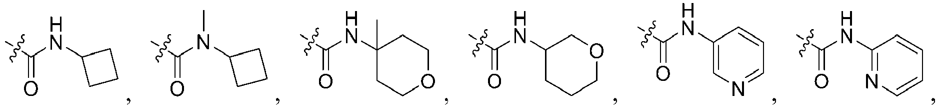 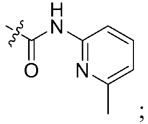
preferably, Rs is selected from H or the following substituted or unsubstituted groups: methyl, ethyl, propyl, cyclopropyl, cyclobutyl, cyclopentyl, cyclohexyl,

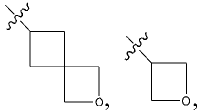

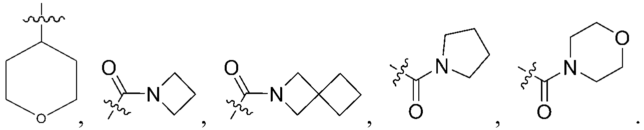

In some embodiments, m1 is selected from 1, 2, or 3, and each R₁ is independently selected from halogen, -OH, -CN, -NReRf, -C(=O)-NReRf, C1-6 alkyl, C2-6 alkynyl, C1-6 alkoxy, C1-6 haloalkyl, C1-6 haloalkoxy, C3-6 cycloalkyl, -C1-6 alkylene-C3-6 cycloalkyl, 5-6-membered heteroaryl, 4-7-membered heterocycloalkyl, wherein, the C3-6 cycloalkyl, -C1-6 alkylene-C3-6 cycloalkyl, 4-7-membered heterocycloalkyl, 5-6-membered heteroaryl are optionally substituted by 1 to 3 substituents selected from halogen, C1-3 alkyl; wherein, Re and Rf are each independently selected from H, C1-3 alkyl, or Re and Rf together with the atoms that they are attached to, form 4-7-membered heterocycloalkyl; preferably, each R₁ is independently selected from C1-3 haloalkyl (for example -CF₃), cyclopropyl, 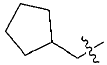 , -N(CH₃)₂; and/or
m2 is selected from 0 or 1, and each R₂ is independently selected from -OH, halogen, C1-3 alkyl, C1-3 haloalkyl, C1-3 alkoxy, C1-3 haloalkoxy, preferably -F, hydroxyl; and/or
m3 is selected from 1, 2, or 3, and each R₃ is independently selected from -CN, C1-3 alkyl, C1-3 haloalkyl (for example -CF₃), -NReRf, -C(=O)-NReRf; wherein, Re and Rf are each independently selected from H, C1-3 alkyl, or Re and Rf together with the atoms that they are attached to, form 4-7-membered heterocycloalkyl (for example 7-membered nitrogen-containing spiro heterocycloalkyl).

In some embodiments, m1 is selected from 1, 2, or 3, and each R₁ is independently selected from halogen, -OH, -CN, -NReRf, -C(=O)-NReRf, C1-6 alkyl, C1-6 alkoxy, C1-6 haloalkyl, C1-6 haloalkoxy, C3-6 cycloalkyl, 5-6-membered heteroaryl, 4-7-membered heterocycloalkyl, preferably C1-3 haloalkyl, cyclopropane; and/or
m2 is selected from 0 or 1, and each R₂ is independently selected from -OH, halogen, C1-3 alkyl, C1-3 haloalkyl, C1-3 alkoxy, C1-3 haloalkoxy, preferably -F, cyclopropane; and/or
m3 is selected from 1, 2, or 3, and each R₃ is independently selected from -CN, C1-3 alkyl, C1-3 haloalkyl, -C(=O)-NReRf, -NReC(=O)Rf;
wherein, Re and Rf are each independently selected from H, C1-6 alkyl, C1-6 haloalkyl, C3-6 cycloalkyl, 4-7-membered heterocycloalkyl, or Re and Rf together with the atoms that they are attached to, form 4-7-membered heterocycloalkyl, and the 4-7-membered heterocycloalkyl is optionally substituted by 1 or more substituents selected from halogen, hydroxyl, cyano, C1-6 alkyl, C1-6 haloalkyl, C1-6 alkoxy, C1-6 haloalkoxy;
preferably, Re is selected from H or C1-3 alkyl, and/or Rf is selected from H, C1-3 alkyl, C1-3 haloalkyl.

In some embodiments, at least one of the R₁ is selected from C1-6 haloalkyl, and/or at least one of the R₃ is selected from C1-6 haloalkyl;
preferably, at least one of the R₁ is selected from C1-3 haloalkyl, and/or at least one of the R₃ is selected from C1-3 haloalkyl;
preferably, at least one of the R₁ is trifluoromethyl, and/or at least one of the R₃ is trifluoromethyl.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the first aspect, has a structure as shown in formula VII-1 or VII-2: 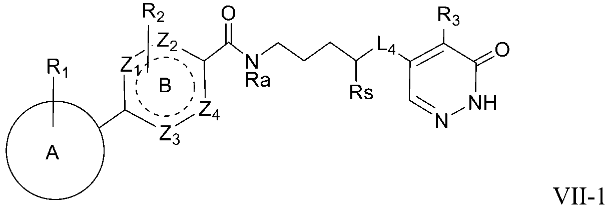 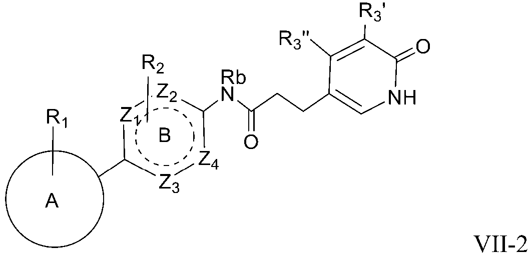 wherein, 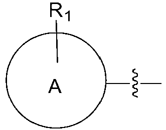 is selected from 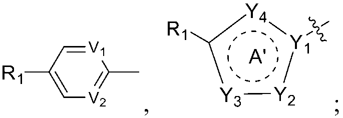
V₁ and V₂ are each independently selected from CH, N; preferably, at least one of V₁ and V₂ is N;
ring A' is 5-membered heteroaromatic ring, Yi is N or CH, Y₂, Y₃, and Y₄ are each independently selected from N, CH, S; preferably, Y₁ and Y₂ are N, Y₃ and Y₄ are CH; or Y₁ is CH, Y₂ is N, one of Y₃ and Y₄ is S and the other is CH; more preferably, Y₁ and Y₃ are CH, Y₂ is N, Y₄ is S;
R₁ is as defined in any of the above embodiments; preferably, R₁ is selected from C1-3 haloalkyl, -C1-3 alkylene-C3-6 cycloalkyl, -NReRf; Re and Rf are each independently selected from H, C1-3 alkyl; more preferably, R1 is -CF₃;
ring B is phenyl or 6-membered heteroaromatic ring, Z₁, Z₂, Z₃, and Z₄ are each independently selected from N or CH; preferably, ring B is phenyl, or only one of Z₁, Z₂, Z₃, and Z₄ is N, or Z₁ and Z₄ are both CH, Z₂ and Z₃ are both N;
R₂ is as defined in any of the above embodiments; preferably, R₂ is selected from H, halogen, hydroxyl, C1-3 alkyl; more preferably, R₂ is selected from H or F; more preferably, with the proviso that: when Z₁ and Z₄ are both CH, Z₂ and Z₃ are both N, then R₂ is H; ring B is phenyl, or when one of Z₂ or Z₄ is N, Z₁ and Z₃ are both CH, then R₂ is H or F, and F is at the ortho position relative to the -CONRa- in formula VII-1; more preferably, R₂ is H;
Ra, Rb are each independently selected from H or methyl; preferably, Ra is H; preferably, Rb is H;
Rs is as defined in any of the above embodiments, preferably, Rs is selected from C1-3 alkyl, C3-6 cycloalkyl (for example cyclopropyl, cyclobutyl, cyclohexyl), 4-7-membered heterocycloalkyl (for example oxetanyl, oxanyl, 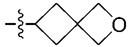 ), 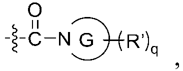 C(=O)N(R_{L2})₂; wherein, ring G is 4-10-membered nitrogen-containing saturated heterocycloalkyl (for example azetidinyl, azacyclopentyl, morpholinyl, 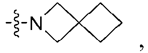 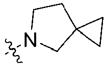 ), R' is selected from halogen (for example F, Cl), cyano, C1-3 alkyl (for example methyl, isopropyl), C1-3 haloalkyl, C1-3 alkoxy, C1-3 haloalkoxy, q is selected from 0, 1, or 2; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 5-6-membered heteroaryl (for example pyridinyl), and the C1-3 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy;
L4 is selected from NH or O, preferably NH;
R₃ is as defined in any of the above embodiments, preferably, R₃ is selected from C1-3 haloalkyl; more preferably, R₃ is selected from -CF₃;
R₃' and R₃" are each independently R₃; preferably, R₃' is selected from C1-3 haloalkyl, R₃" is selected from -NReRf, and Re and Rf together with the atoms that they are attached to, form 4-7-membered heterocycloalkyl (for example 7-membered nitrogen-containing spiro heterocycloalkyl); more preferably, R₃' is -CF₃, R₃" is

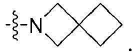

It should be noted that the compounds in the present embodiments have a better activity inhibitory effect against PARP7; the compounds in which ring A is a 6-membered heteroaromatic ring generally show a better selectivity for PARP7 compared to those in which ring A is a 5-membered heteroaromatic ring; the compounds in which Rs is -C(=O)N(R_{L2})₂ generally show a better selectivity for PARP7 compared to those in which Rs is 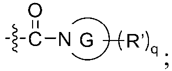 and for the compounds in which Rs is 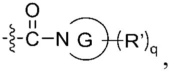 those with a larger number of ring atoms in ring G generally show a better selectivity for PARP7.

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the present invention is selected from the compounds listed in Table 1.

**Table 1 Exemplary compounds of the present invention**

| | | |
|---|---|---|
| compound 1 | compound 2 | compound |
| 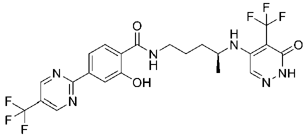 | 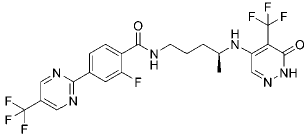 | 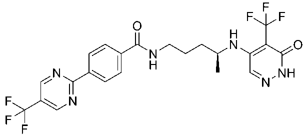 |
| compound 4 | compound 5 | compound 6 |
| 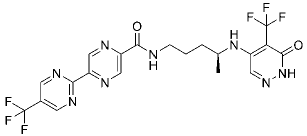 | 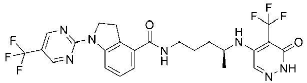 | 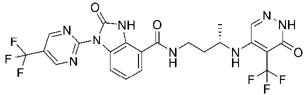 |
| compound 7 | compound 8 | compound 9 |
| 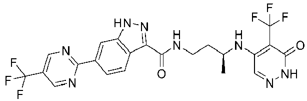 | 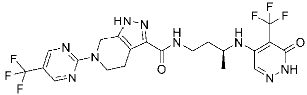 | 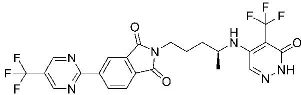 |
| compound 10 | compound 11 | compound 12 |
| 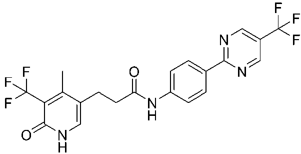 | 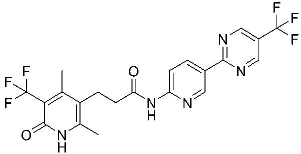 | 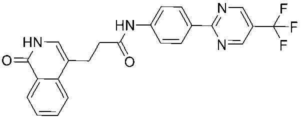 |
| compound 13 | compound 14 | compound 15 |
| 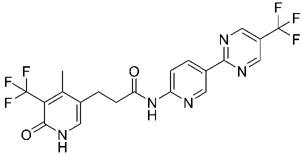 | 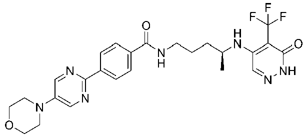 | 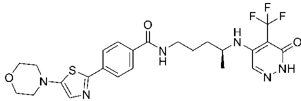 |
| compound 16 | compound 17 | compound 18 |
| 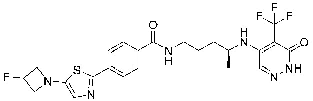 | 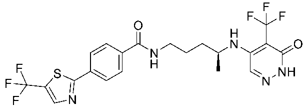 | 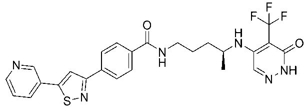 |
| compound 19 | compound 20 | compound 21 |
| 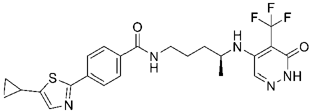 | 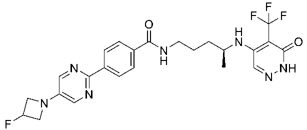 | 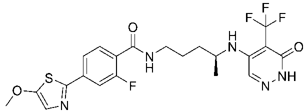 |
| compound 22 | compound 23 | compound 24 |
| 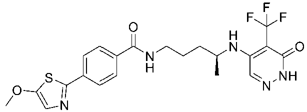 | 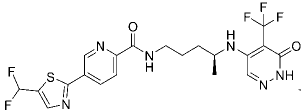 | 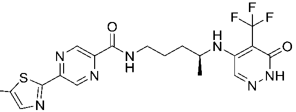 |
| compound 25 | compound 26 | compound 27 |
| 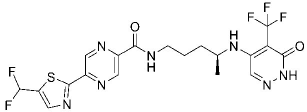 | 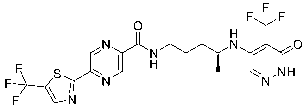 | 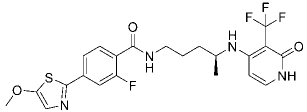 |
| compound 28 | compound 29 | compound 30 |
| 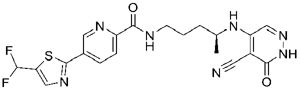 | 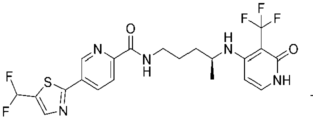 | 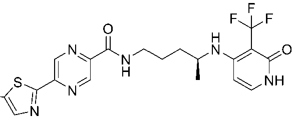 |
| compound 31 | compound 32 | compound 33 |
| 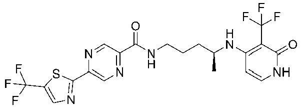 | 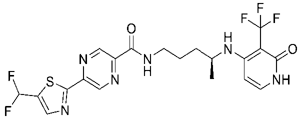 | 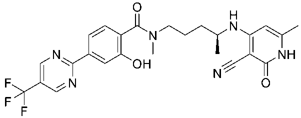 |
| compound 34 | compound 35 | compound 36 |
| 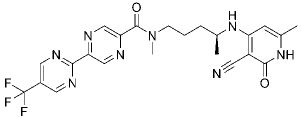 | 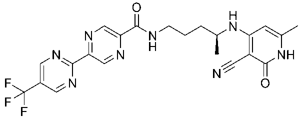 | 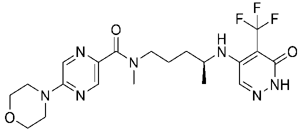 |
| compound 37 | compound 38 | compound 39 |
| 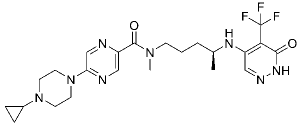 | 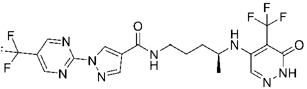 F | 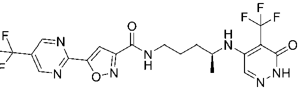 |
| compound 40 | compound 41 | compound 42 |
| 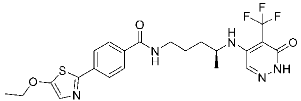 | 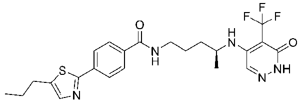 | 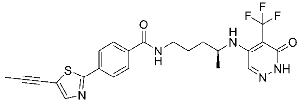 |
| compound 43 | compound 44 | compound 45 |
| 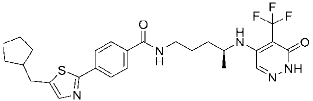 | 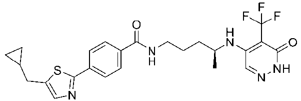 | 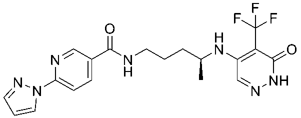 |
| compound 46 | compound 47 | compound 48 |
| 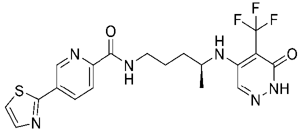 | 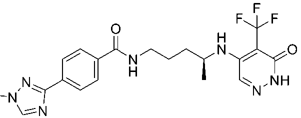 | 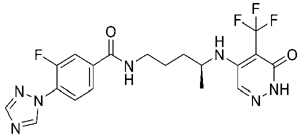 |
| compound 49 | compound 50 | compound 51 |
| 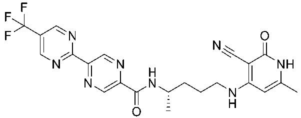 | 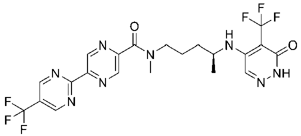 | 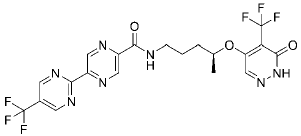 |
| compound 52 | compound 53 | compound 54 |
| 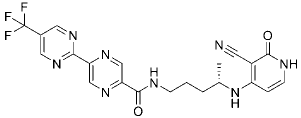 | 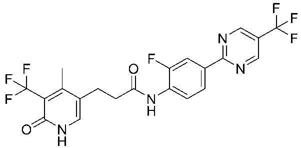 | 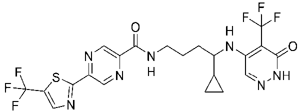 |
| compound 55 | compound 56 | compound 57 |
| 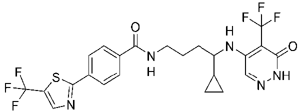 | 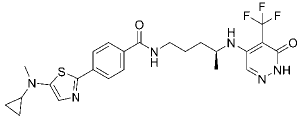 | 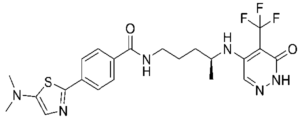 |
| compound 58 | compound 59 | compound 60 |
| 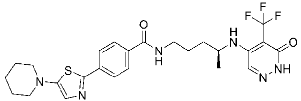 | 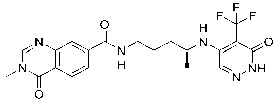 | 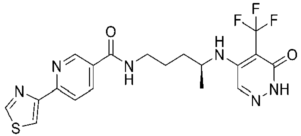 |
| compound 61 | compound 62 | compound 63 |
| 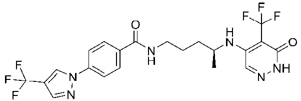 | 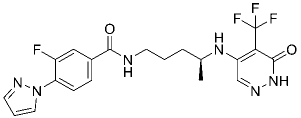 | 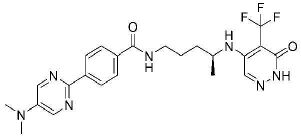 |
| compound 64 | compound 65 | compound 66 |
| 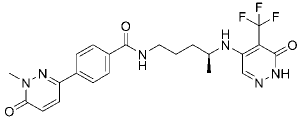 | 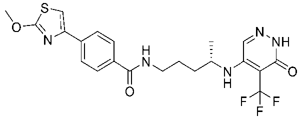 | 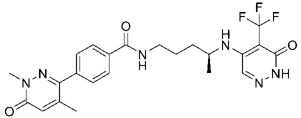 |
| compound 67 | compound 68 | compound 69 |
| 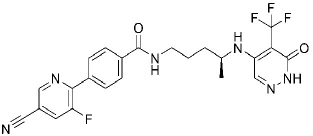 | 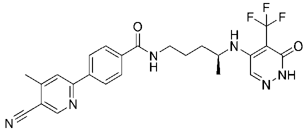 | 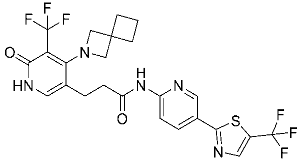 |
| compound 70 | compound 71 | compound 72 |
| 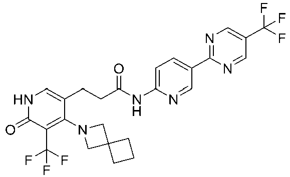 | 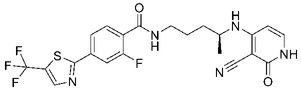 | 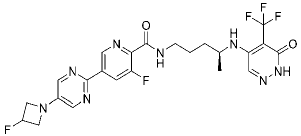 |
| compound 73 | compound 74 | compound 75 |
| 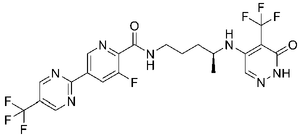 | 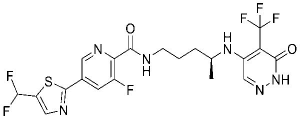 | 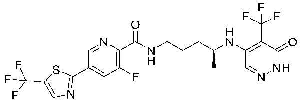 |
| compound 76 | compound 77 | compound 78 |
| 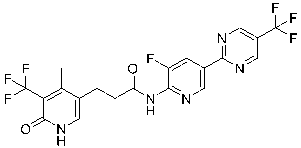 | 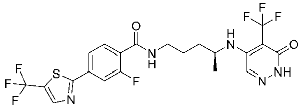 | 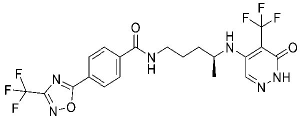 |
| compound 79 | compound 80 | compound 81 |
| 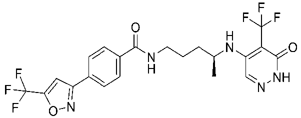 | 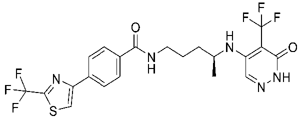 | 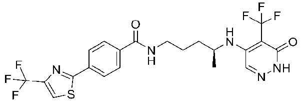 |
| compound 82 | compound 83 | compound 84 |
| 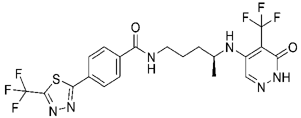 | 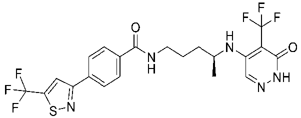 | 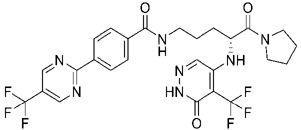 |
| compound 85 | compound 86 | compound 87 |
| 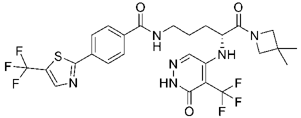 | 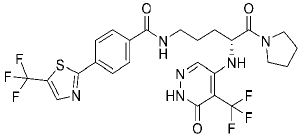 | 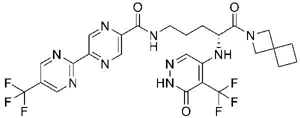 |
| compound 88 | compound 89 | compound 90 |
| 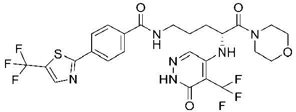 | 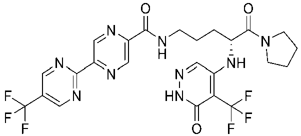 | 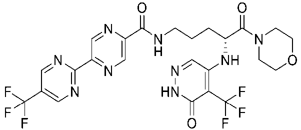 |
| compound 91 | compound 92 | compound 93 |
| 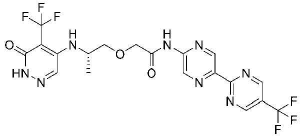 | 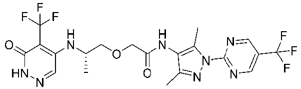 | 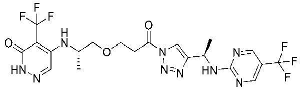 |
| compound 94 | compound 95 | compound 96 |
| 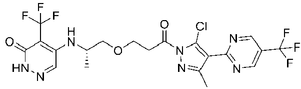 | 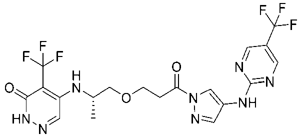 | 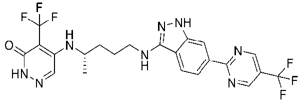 |
| compound 97 | compound 98 | compound 99 |
| 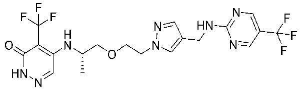 | 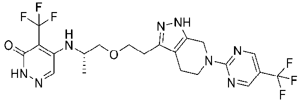 | 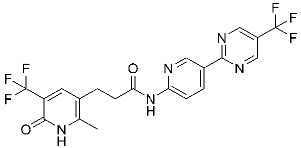 |
| compound 100 | compound 101 | compound 102 |
| 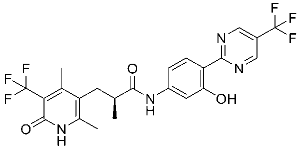 | 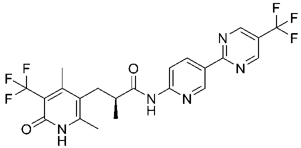 | 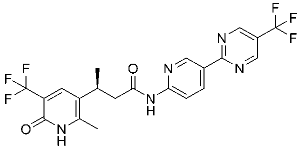 |
| compound 103 | compound 104 | compound 105 |
| 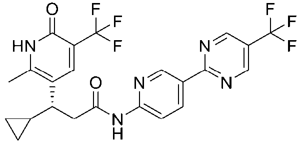 | 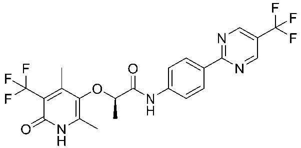 | 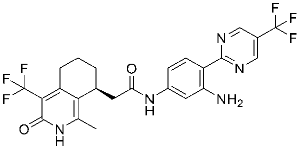 |
| compound 106 | compound 107 | compound 108 |
| 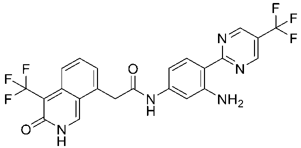 | 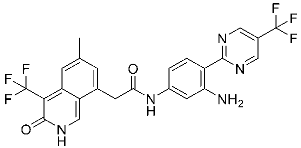 | 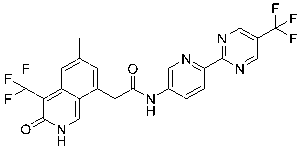 |
| compound 109 | compound 110 | compound 111 |
| 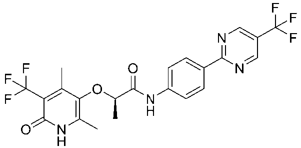 | 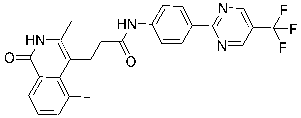 | 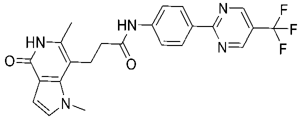 |
| compound 112 | compound 113 | compound 114 |
| 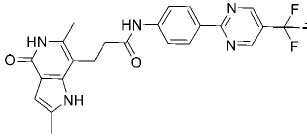 | 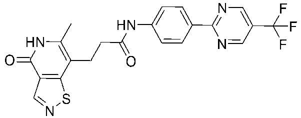 | 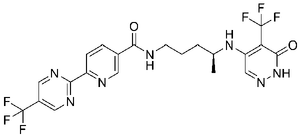 |
| compound 115 | compound 116 | compound 117 |
| 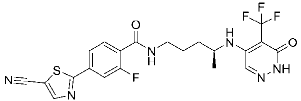 | 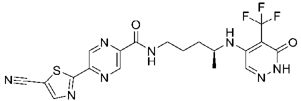 | 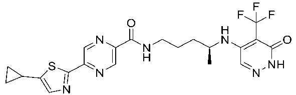 |
| compound 118 | compound 119 | compound 120 |
| 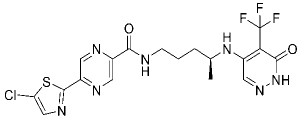 | 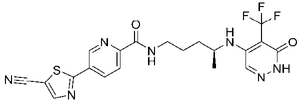 | 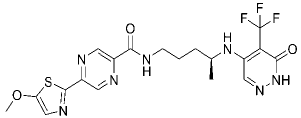 |
| compound 121 | compound 122 | compound 123 |
| 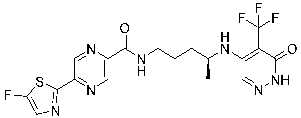 | 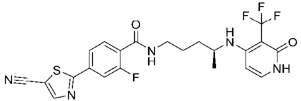 | 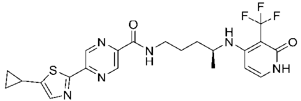 |
| compound 124 | compound 125 | compound 126 |
| 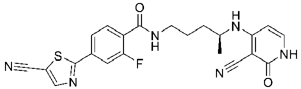 | 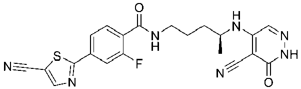 | 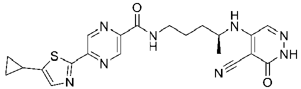 |
| compound 127 | compound 128 | compound 129 |
| 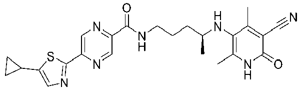 | 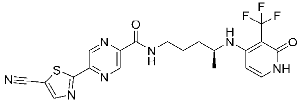 | 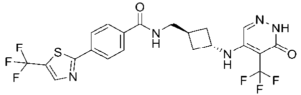 |
| compound 130 | compound 131 | compound 132 |
| 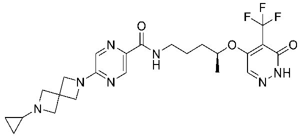 | 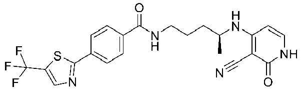 | 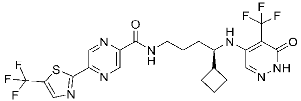 |
| compound 133 | compound 134 | compound 135 |
| 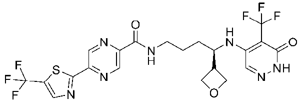 | 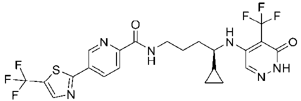 | 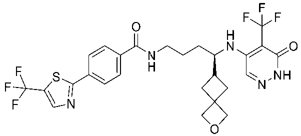 |
| compound 136 | compound 137 | compound 138 |
| 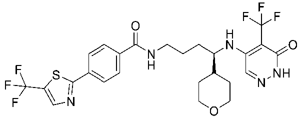 | 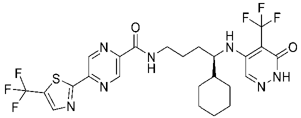 | 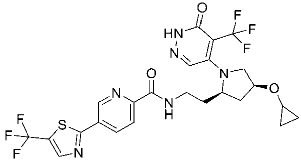 |
| compound 139 | compound 140 | compound 141 |
| 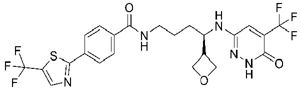 | 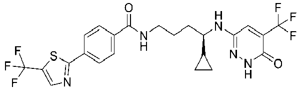 | 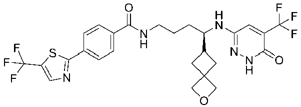 |
| compound 142 | compound 143 | compound 144 |
| 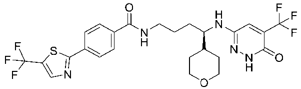 | 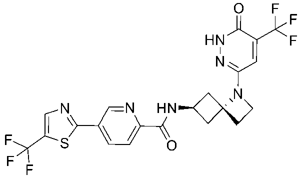 | 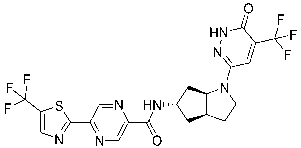 |
| compound 145 | compound 146 | compound 147 |
| 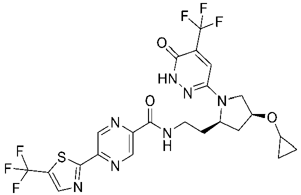 | 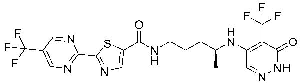 | 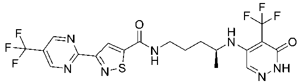 |
| compound 148 | compound 149 | compound 150 |
| 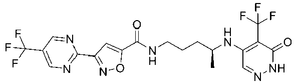 | 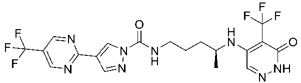 | 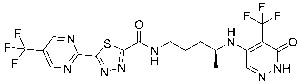 |
| compound 151 | compound 152 | compound 153 |
| 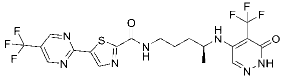 | 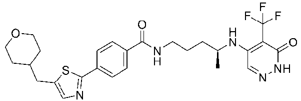 | 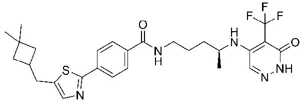 |
| compound 154 | compound 155 | compound 156 |
| 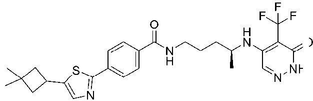 | 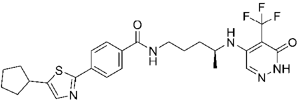 | 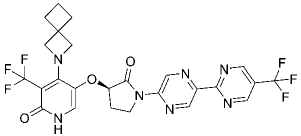 |
| compound 157 | compound 158 | compound 159 |
| 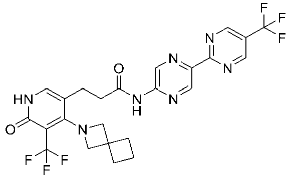 | 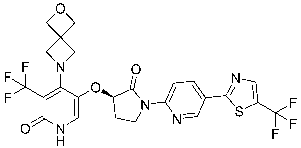 | 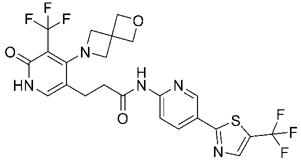 |
| compound 160 | compound 161 | compound 162 |
| 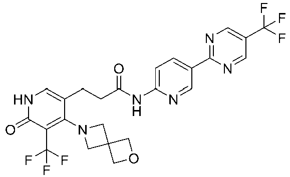 | 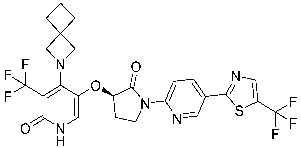 | 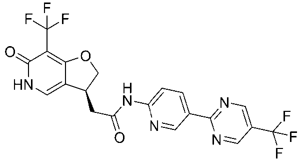 |
| compound 163 | compound 164 | compound 165 |
| 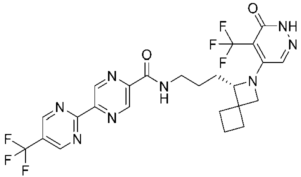 | 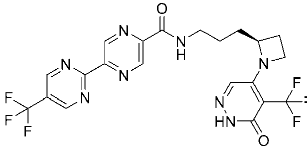 | 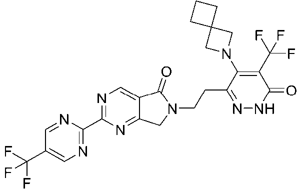 |
| compound 166 | compound 167 | compound 168 |
| 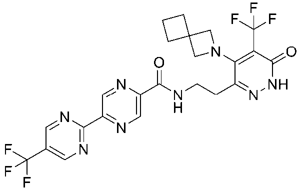 | 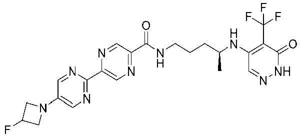 | 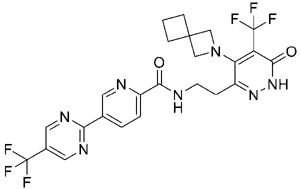 |
| compound 169 | compound 170 | compound 171 |
| 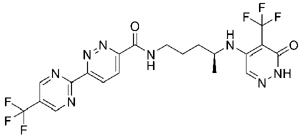 | 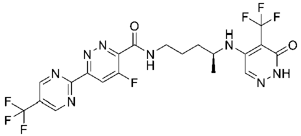 | 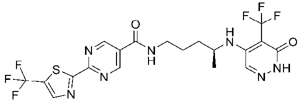 |
| compound 172 | compound 173 | compound 174 |
| 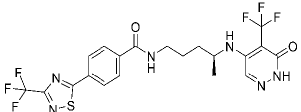 | 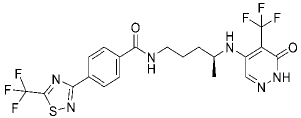 | 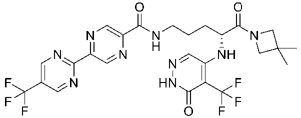 |
| compound 175 | compound 176 | compound 177 |
| 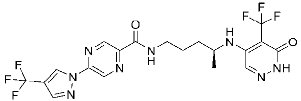 | 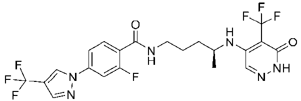 | 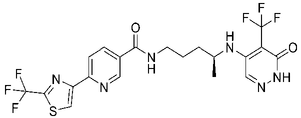 |
| compound 178 | compound 179 | compound 180 |
| 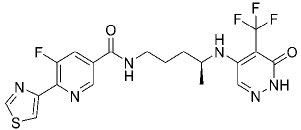 | 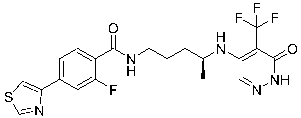 | 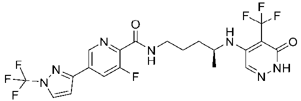 |
| compound 181 | compound 182 | compound 183 |
| 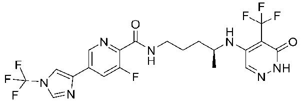 | 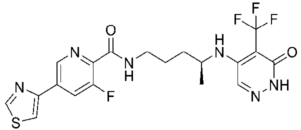 | 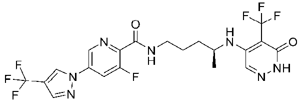 |
| compound 184 | compound 185 | compound 186 |
| 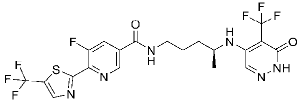 | 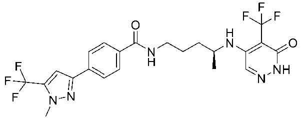 | 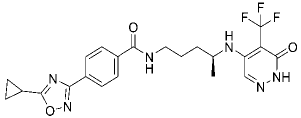 |
| compound 187 | compound 188 | compound 189 |
| 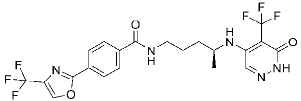 | 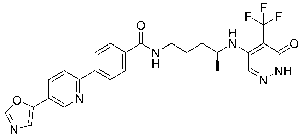 | 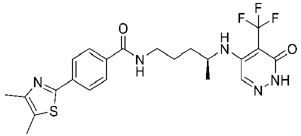 |
| compound 190 | compound 191 | compound 192 |
| 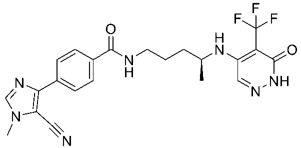 | 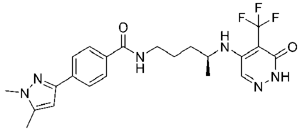 | 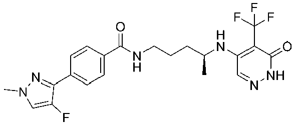 |
| compound 193 | compound 194 | compound 195 |
| 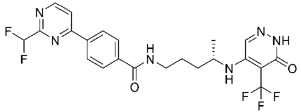 | 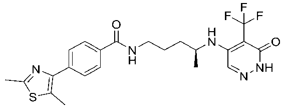 | 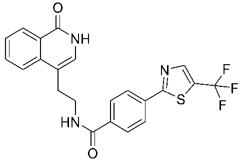 |
| compound 196 | compound 197 | compound 198 |
| 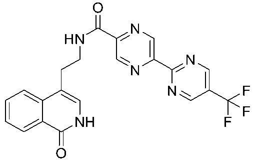 | 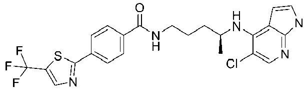 | 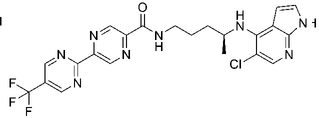 |
| compound 199 | compound 200 | compound 201 |
| 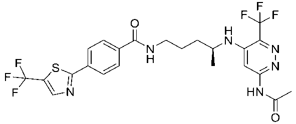 | 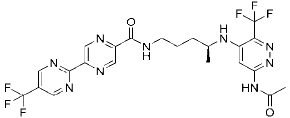 | 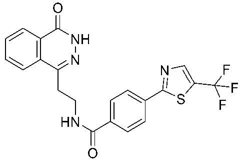 |
| compound 202 | compound 203 | compound 204 |
| 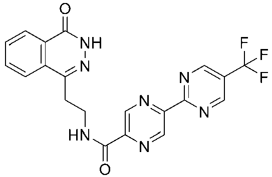 | 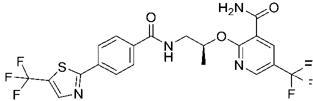 | 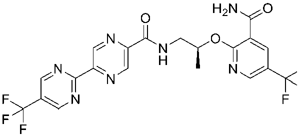 |
| compound 205 | compound 206 | compound 207 |
| 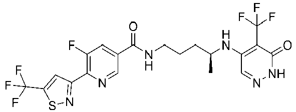 | 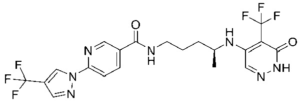 | 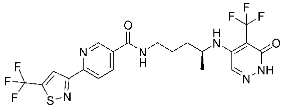 |
| compound 208 | compound 209 | compound 210 |
| 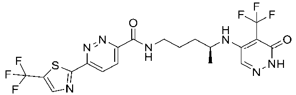 | 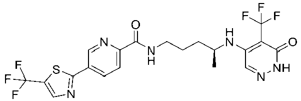 | 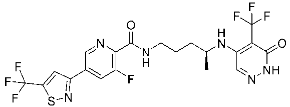 |
| compound 211 | compound 212 | compound 213 |
| 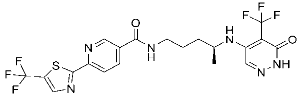 | 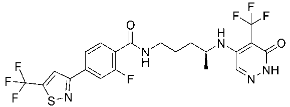 | 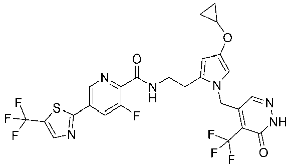 |
| compound 214 | compound 215 | compound 216 |
| 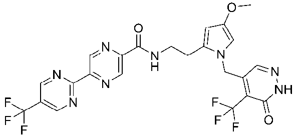 | 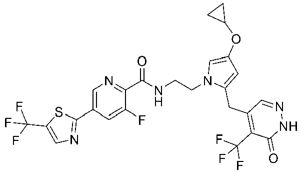 | 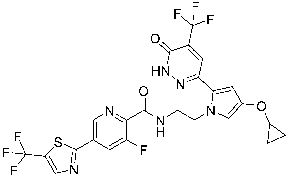 |
| compound 217 | compound 218 | compound 219 |
| 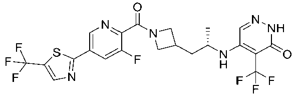 | 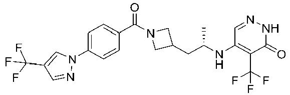 | 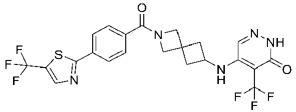 |
| compound 220 | compound 221 | compound 222 |
| 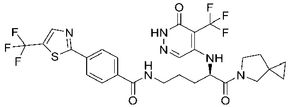 | 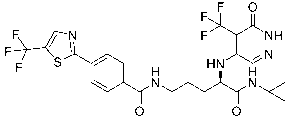 | 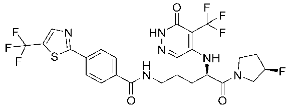 |
| compound 223 | compound 224 | compound 225 |
| 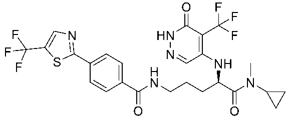 | 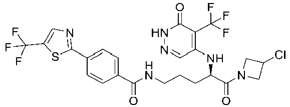 | 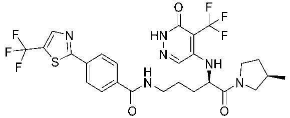 |
| compound 226 | compound 227 | compound 228 |
| 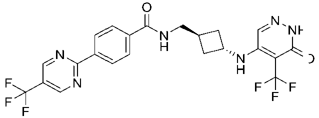 | 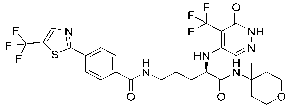 | 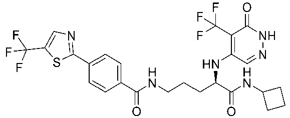 |
| compound 229 | compound 230 | compound 231 |
| 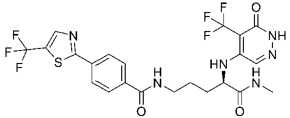 | 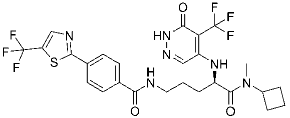 | 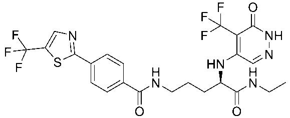 |
| compound 232 | compound 233 | compound 234 |
| 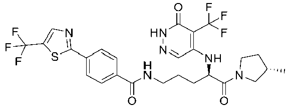 | 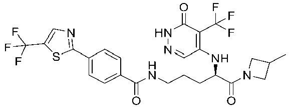 | 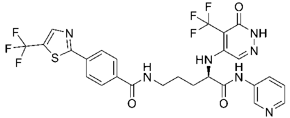 |
| compound 235 | compound 236 | compound 237 |
| 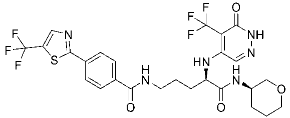 | 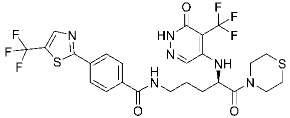 | 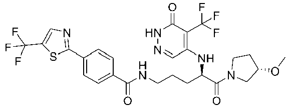 |
| compound 238 | compound 239 | compound 240 |
| 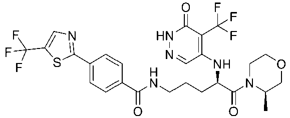 | 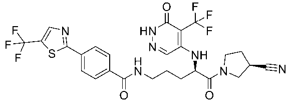 | 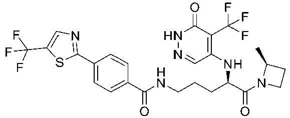 |
| compound 241 | compound 242 | compound 243 |
| 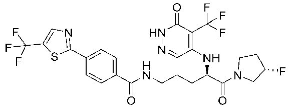 | 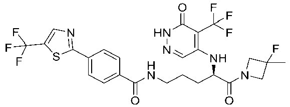 | 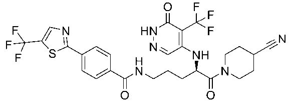 |
| compound 244 | compound 245 | compound 246 |
| 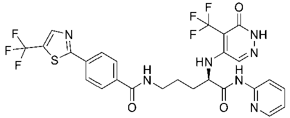 | 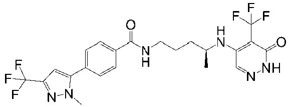 | 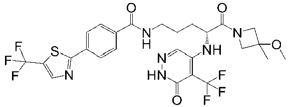 |
| compound 247 | compound 248 | compound 249 |
| 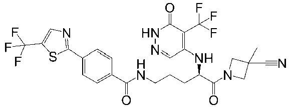 | 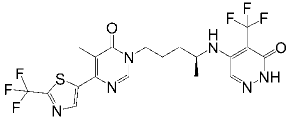 | 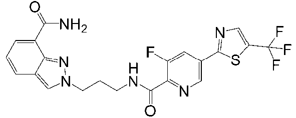 |
| compound 251 | compound 252 | compound 254 |
| 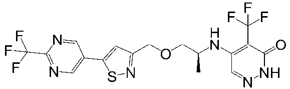 | 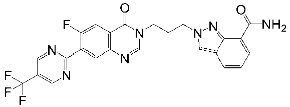 | 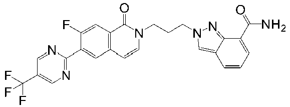 |
| compound 255 | compound 256 | compound 257 |
| 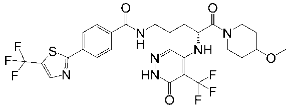 | 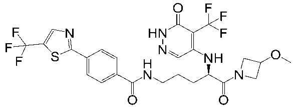 | 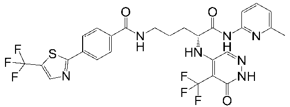 |
| compound 258 | compound 259 | compound 260 |
| 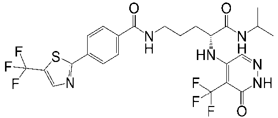 | 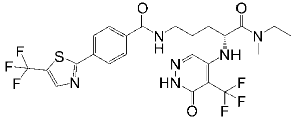 | 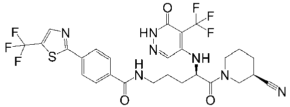 |
| compound 261 | compound 262 | |
| 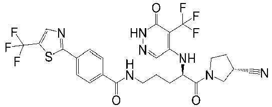 | 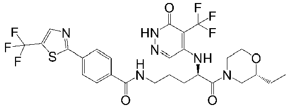 | |

In some embodiments, the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of the present invention is selected from compounds 1-219 listed in Table 1.

The present invention covers the compounds obtained by any combination of the various embodiments.

### Pharmaceutical compositions and use

In some embodiments, the present invention also provides a pharmaceutical composition, the pharmaceutical composition comprises a compound of formula I, or an enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof, and a pharmaceutically acceptable carrier.

Pharmaceutically acceptable carriers can be liquids, for example water and oils, including petroleum, animal oils, plant oils, or synthetic origin oils, for example peanut oil, soybean oil, mineral oil, sesame oil, etc. Pharmaceutical carriers can be brine, acacia senegal, gelatin, starch paste, talc, keratin, colloidal silica, urea, etc. In addition, an excipient, a stabilizer, a thickener, a lubricant, and a colorant can be used. When administered to a subject, the compound of formula I and the pharmaceutically acceptable carrier are preferably sterile. When the composition is administered intravenously, water is the preferred carrier. Saline solution and dextrose and glycerol aqueous solutions can also be used as liquid carriers, particularly for an injectable solution. Suitable pharmaceutical carriers also include excipients, for example starch, glucose, lactose, sucrose, gelatin, maltose, rice, flour, chalk, silica gel, sodium stearate, glyceryl monostearate, talc, sodium chloride, skimmed milk, glycerol, propylene glycol, water, ethanol, etc. If necessary, the compositions of the present invention may also contain small amounts of wetting agents or emulsifiers or pH buffers.

The compositions of the present invention may be in the form of solutions, suspensions, emulsions, tablets, pills, pellets, capsules, liquid-containing capsules, powders, sustained-release formulations, suppositories, aerosols, sprays, or any other suitable form for use. In one embodiment, the pharmaceutically acceptable carrier is a capsule. Other examples of suitable pharmaceutical carriers are described in E.W.Martin, "Remington's Pharmaceutical Sciences".

In some embodiments, appropriate combination drugs or excipients may also be selected according to actual needs to form a drug combination that has a combined pharmacodynamic effect or improves a certain aspect of the drug's performance (such as stability).

In some embodiments, the present invention also provides a pharmaceutical package or kit comprising one or more containers filled with one or more compounds of the present invention. Optionally, such a container is associated with a notification of the form prescribed by a government agency that manages and controls the manufacture, use, or sale of the medicine, indicating permission to use or sale it for human use. In certain preferred embodiments, for example, when administered to treat or prevent cancer, the kit may also contain one or more chemotherapeutic agents for treating cancer or tumor diseases, administered in combination with the compounds of the present invention. In certain preferred embodiments, for example when administered to treat or prevent a virus or autoimmune disease, the kit may contain one or more compounds of the present invention and one or more antiviral inhibitors or immunosuppressant.

In some embodiments, the compound of formula I, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof, can selectively inhibit the enzymatic activity of PARP7, and thus can be used to inhibit PARP7 in a subject in need thereof, or used to treat a PARP7-mediated disease; preferably, the PARP7-mediated disease is selected from cancer, immune diseases, inflammation or viral infections.

In some embodiments, the PARP7-mediated diseases to be treated or in which PARP7 is to be inhibited are hyperproliferative diseases, particularly cancer, including but not limited to breast cancer, central nervous system cancer, endometrial cancer, renal cancer, colorectal carcinoma, lung cancer, esophageal cancer, ovarian cancer, pancreatic cancer, prostate cancer, stomach cancer, head and neck cancer (upper aerodigestive tract cancer), urinary tract cancer, colon cancer, cancers with amplified PARP7 expression, etc. In some embodiments, the cancers treatable according to the present invention include hematopoietic malignancies, for example leukemia and lymphoma. Exemplary lymphomas include Hodgkin lymphoma or non-Hodgkin lymphoma, multiple myeloma, B-cell lymphoma (e.g., diffuse large B-cell lymphoma (DLBCL)), chronic lymphocytic lymphoma (CLL), T-cell lymphoma, hairy cell lymphoma, and Burkitt's lymphoma. Examples of leukemia include acute lymphocytic leukemia (ALL), acute myelogenous leukemia (AMIL), chronic lymphocytic leukemia (CLL), and chronic myelogenous leukemia (CML).

In some embodiments, the PARP7-mediated diseases to be treated or in which PARP7 is to be inhibited are autoimmune or inflammatory diseases, including but not limited to, rheumatoid arthritis, insulin-dependent diabetes mellitus, certain hemolytic anemias, rheumatic fever, thyroiditis, ulcerative colitis, myasthenia gravis, glomerulonephritis, allergic encephalomyelitis, continued damage to nerve and liver after viral hepatitis, multiple sclerosis, systemic lupus erythematosus, juvenile diabetes, autoimmune hemolytic anemia, psoriasis, idiopathic thrombocytopenic purpura, active chronic hepatitis, idiopathic leukopenia, primary biliary cirrhosis, thyrotoxicosis, dermatomyositis, discoid lupus erythematosus, psoriatic arthritis, regional enteritis, nephrotic syndrome, lupus nephritis, lupus hepatitis, Sjögren syndrome, Goodpasture syndrome, Wegener granulomatosis, scleroderma, sebaceous disease, uveitis and mumps mastitis, ulcerative colitis, Crohn's disease, multiple sclerosis, autoimmune liver disease, type I diabetes mellitus, bronchial asthma, systemic lupus erythematosus, rheumatoid arthritis, ankylosing spondylitis, juvenile idiopathic arthritis, psoriasis, polymyositis, and dermatomyositis.

In some embodiments, the PARP7-mediated diseases to be treated or in which PARP7 is to be inhibited are viral infections, including but not limited to, DNA viruses such as hepatitis B virus and hepatitis C virus; parvoviruses such as adeno-associated viruses and cytomegaloviruses; papillomaviruses such as papillomaviruses, polyomaviruses, and SV40; adenoviruses; herpesviruses such as herpes simplex virus type I (HSV-I), herpes simplex virus type II (HSV-II), and Epstein-Barr virus; poxviruses, such as variola virus (smallpox) virus and vaccinia virus; and RNA viruses such as human immunodeficiency virus type I (HIV-I), human immunodeficiency virus type II (HIV-II), human T-cell lymphotropic virus type I (HTLV-I), human T-cell lymphotropic virus type II (HTLV-II), influenza viruses, measles virus, rabies virus, Sendai virus, papillomaviruses such as polioviruses, coxsackieviruseses, rhinoviruses, reoviruses, togaviruses such as rubella virus (German measles) and Semliki Forest virus, dendritic virus, and hepatitis A virus.

### Examples

The following examples are provided to help understand the present invention. However, it should be understood that these examples are only used to illustrate the present invention and do not constitute any limitation. The actual scope of protection of the present invention is set forth in the claims. It should be understood that any modifications and changes can be made without departing from the spirit of the present invention. If a particular condition is not specified in the examples, it is carried out according to a conventional condition or the condition suggested by the manufacturer. The used reagents or instruments whose manufacturers are not specified are all commercially available conventional products.

The abbreviations and their corresponding names involved in the following examples are shown in Table 2.

**Table 2**

| Abbreviation | Name |
|---|---|
| AIBN | azobisisobutyronitrile |
| NMP | N-methylpyrrolidone |
| CDI | N,N'-carbonyldiimidazole |
| DCM | dichloromethane |
| DIBOC | Boc anhydride/di-tert-butyl dicarbonate |
| DIPEA | N,N-diisopropylethylamine |
| DMAP | 4-dimethylaminopyridine |
| DMF | N,N-dimethylformamide |
| DMSO | dimethyl sulfoxide |
| DPPF | 1,1-bis(diphenylphosphino)ferrocene |
| DPPA | diphenylphosphoryl azide |
| EA/EtOAc | ethyl acetate |
| HATU | O-(7-azabenzotriazole)-N,N,N',N'-tetramethyluronium hexafluorophosphate/ N,N,N',N'-tetramethyl-O-(7-azabenzotriazole-1-yl)uronium hexafluorophosphate/2-(7-azabenzotriazole)-N,N,N',N'-tetramethyluronium hexafluorophosphate |
| IPA | isopropanol |
| KTB | potassium tert-butoxide |
| LiOH | lithium hydroxide |
| MeCN | acetonitrile |
| MeOH | methanol |
| Pd/C | palladium on carbon |
| Pd(PPh₃)₄ | tetrakistriphenylphosphinepalladium/tetrakis(triphenylphosphine)palladium |
| PdCl₂(PPh₃)₂ | bis(triphenylphosphine)palladium dichloride |
| PE | petroleum ether |
| PMB | p-methoxybenzyl |
| HSiEt₃ | triethylsilane |
| TCFH | N,N,N',N'-tetramethylchloroformamidinium hexafluorophosphate |
| TFA | trifluoroacetic acid |
| TfOH | trifluoromethanesulfonic acid |
| THF | tetrahydrofuran |

NMR was determined using a Bruker Avance III 400 nuclear magnetic instrument, and the chemical shift (δ) is given in unit of 10⁻⁶ (ppm). The solvent was deuterated methanol (CD3OD), deuterated chloroform (CDCl₃), or hexadeuterated dimethyl sulfoxide (DMSO-d6), etc., with tetramethylsilane (TMS) as the internal standard.

MS was determined using an Agilent (ESI) mass spectrometer (Agilent 1260, Agilent 6125B).

High performance liquid chromatography (HPLC) determination conditions: Gilson highpressure liquid chromatograph (Gilson GX-281), C18 column (10 µM, 19 mm × 250 mm), ultraviolet detection bands of 220 and 254 nm, unless otherwise specified, the elution conditions being gradient elution of 5-95% acetonitrile (containing 0.05% v/v formic acid or ammonium bicarbonate) for 15 minutes.

Reversed-phase purification was performed using Biotage Isolera rapid purificatcion system.

Thin layer chromatography separation and purification were performed using a thin layer chromatography silica gel plate (an aluminum plate from Meck (20 cm × 20 cm × 1 mm) or GF 254 from Yantai).

Biotage Initiator + (400 W, RT 300°C) microwave reactor was used for a microwave reaction.

The reaction was routinely monitored using TLC or LCMS, the commonly used developing agent system included: DCM/methanol, n-hexane/EA, PE/EA. The volume ratio of the solvents was adjusted according to the different polarities of the compounds, or triethylamine or the like was added for adjustment.

The silica gel used in column chromatography is generally 100-200 mesh silica gel. The commonly used eluant system included: DCM/methanol, PE/EA. The volume ratio of the solvents was adjusted according to the different polarities of the compounds, and a small amount of triethylamine could also be added for adjustment.

The reagents and solvents and the like of the present invention were purchased from Aldrich Chemical Company, Energy, J&K Scientific, Shanghai Bide Pharmaceutical Technology Co., Ltd., PharmaBlock, and Shanghai Titan Technology Co., Ltd., among others.

### Synthesis examples

### Intermediate preparation example 1

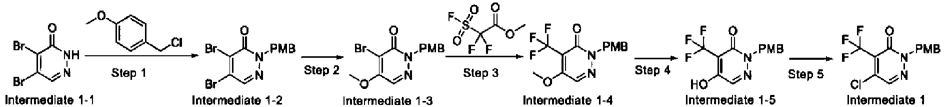

Step 1: 4,5-dibromopyridazin-3-one (50 g, 196.94 mmol Bide), potassium carbonate (40.83 g, 295.42 mmol), 4-methoxybenzylchloride (34.87 mL, 256.03 mmol, Titan) were dissolved in acetonitrile (500 mL). The reaction was carried out at 80°C for 16 hours. The reaction solution was filtered through celite and the filtrate was concentrated under reduced pressure. The residue was slurried for 1 hour by adding 300 mL methanol, filtered, and washed with methanol (50 mL×2). The resulting solid was blown dry under nitrogen at normal temperature to afford intermediate 1-2. MS m/z (ESI):375.0[M+1].

Step 2: Intermediate 1-2 (48 g, 128.33 mmol) was dissolved in methanol (480 mL), and then potassium hydroxide (28.80 g, 513.33 mmol) was added. The reaction was carried out at 20°C for 16 hours. The reaction solution was concentrated under reduced pressure. The residue was slurried for 1 hour by adding 300 mL water, filtered, and washed with water (100 mL×2). The resulting solid was dissolved with DCM (500 mL) and dried over anhydrous sodium sulfate. After filtering to remove the desiccant, the filtrate was concentrated under reduced pressure to afford intermediate 1-3. MS m/z (ESI):327.0[M+1].

Step 3: Intermediate 1-3 (39 g, 119.94 mmol), CuI (6.50 mL, 191.91 mmol), and methyl fluorosulfonyldifluoroacetate (51.12 mL, 401.80 mmol) were added to NMP (400 mL), followed by stirring at 110°C for another 4 hours under nitrogen protection. The reaction mixture was poured into EA (3000 mL)/water (1000 mL), followed by stirring. The two phases were separated. Then, the aqueous phase was extracted with EA (100 mL×2). The mixed organic phase was washed with saturated NaCl solution (30 mL ×4), dried over anhydrous sodium sulfate, eluted and filtered through a short silica gel column (100 g silica gel) with EA (100 mL×2). The filtrate was concentrated under reduced pressure to afford intermediate 1-4. MS m/z (ESI):315.2[M+1].

Step 4: Intermediate 1-4 (17 g, 54.10 mmol) and iodotrimethylsilane (13.92 mL, 97.37 mmol) were added to NMP (400 mL) for mixing, followed by stirring at 85°C for 3 hours. The mixture was diluted with water (400 mL), followed by extraction with EA (300 mL×3). The organic phases were combined, washed with saturated saline (200 mL×3), dried over anhydrous sodium sulfate, filtered, and concentrated to dryness. The intermediate 1-5 was afforded by elution with silica gel chromatography using DCM/MeOH = 5:1.

Step 5: Intermediate 1-5 (15 g, 49.96 mmol) was added to DMF (120 mL), followed by cooling to 0-5°C. 2-chloro-2-oxoacetyl chloride (25 mL, 291.52 mmol) was added dropwise. The resulting mixture was stirred at 20°C for another 1 hour. The reaction was carried out at 20°C for 6 hours. The reaction solution was added with 200 mL saturated sodium bicarbonate solution, followed by extraction with EA (150 mL×3). The organic phases were combined, and washed with half-saturated saline. The organic phases were dried over anhydrous sodium sulfate. After filtering to remove the desiccant, the filtrate was concentrated under reduced pressure to afford intermediate 1. MS m/z (ESI):341.0[M+Na].

### Intermediate preparation example 2

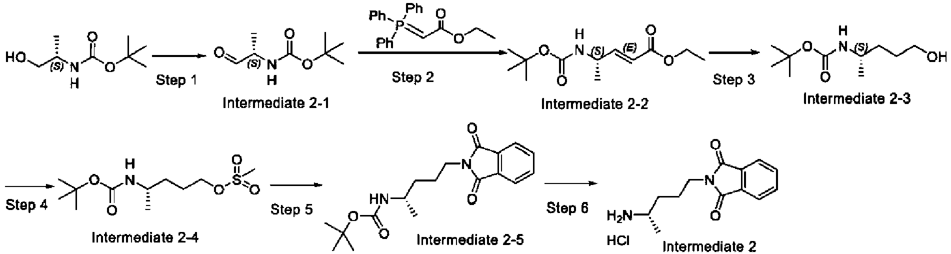

Step 1: Tert-butyl (S)-(1-hydroxylpropyl-2-yl)carbamate (100 g, 570.68 mmol, Bide) was dissolved in DCM (2000 mL), followed by reducing the temperature to 0°C. Dess-Martin oxidizing agent (290.46 g, 684.81 mmol) was added in batches. After the addition, the resulting mixture was allowed to warm to room temperature, and the reaction was carried out for 18 hours. 1000 mL methyl tert-butyl ether was added. The solid was filtered, followed by washing with methyl tert-butyl ether (200 mL×2). The filtrate was washed two times with sodium bicarbonate aqueous solution and saturated saline, respectively. The organic phases were dried over anhydrous sodium sulfate, and concentrated under reduced pressure to afford intermediate 2-1. 1H NMR (400 MHz, CDCl3) δ 9.55 (s, 1H), 5.11 (s, 1H), 4.37 - 4.06 (m, 1H), 1.44 (s, 9H), 1.38 - 1.23 (m, 3H).

Step 2: Intermediate 2-1 (70.2 g, 405.29 mmol) was dissolved in THF (700 mL). Ethoxyformylmethylenetriphenylphosphorane (141.19 g, 405.29 mmol) was added. The reaction was carried out at room temperature for 18 hours. The reaction solution was concentrated under reduced pressure. The residue was added with 500 mL DCM, filtered through a short silica gel column, and washed with DCM (100 mL ×2). The filtrates were combined and concentrated, and purified by silica gel column chromatography with an eluant system (DCM: MeOH=100/0->10/1) to afford intermediate 2-2. MS m/z (ESI):188.3[M-56+H].

Step 3: Intermediate 2-2 (20 g, 82.20 mmol) was dissolved in ethanol (400 mL). Sodium borohydride (18.66 g, 493.22 mmol) was added, followed by heating to 75°C and reacting for 8 hours. The resulting mixture was concentrated to dryness under reduced pressure, followed by adding DCM (500 mL) and filtering. The filtrate was concentrated under reduced pressure and purified by silica gel column chromatography with an eluant system (DCM: MeOH=100/0->10/1) to afford intermediate 2-3. MS m/z (ESI):148.3[M-56+H].

Step 4: Intermediate 2-3 (14.45 g, 71.08 mmol) and triethylamine (29.56 mL, 213.25 mmol) were dissolved in DCM (75 mL), followed by reducing the temperature to 0-5°C. Then, methylsulfonyl chloride (8.25 mL, 106.63 mmol) was added dropwise. The reaction was carried out at room temperature for 18 hours. The reaction solution was poured into ice water (150 mL), followed by extraction with DCM (100 mL ×3). The combined organic phase was washed with saturated sodium chloride solution (100 mL ×2), dried over anhydrous sodium sulfate, and filtered. The filtrate was concentrated under reduced pressure to afford intermediate 2-4. MS m/z (ESI): 226.0[M-56+H].

Step 5: Intermediate 2-4 (18.1 g, 64.33 mmol) was dissolved in N, N-dimethylformamide (200 mL) and then potassium phthalimide(15.9 g, 85.83 mmol) was added. The reaction was carried out at 50°C for 18 hours. The reaction solution was poured into water (300 mL), followed by extraction with EA (150 mL × 3). The combined organic phase was washed with saturated brine (100 mL × 2), dried over anhydrous sodium sulfate, and filtered. The filtrate was concentrated under reduced pressure and purified by silica gel chromatography with an eluant system (PE:EtOAc = 1/1) to afford intermediate 2-5. MS m/z (ESI): 233.1[M-100+H].

Step 6: Intermediate 2-5 (18.3 g, 55.05 mmol) was dissolved in 4 mol/L 1,4-dioxane, and the reaction was carried out at room temperature for 18 hours. The reaction solution was concentrated under reduced pressure to afford intermediate 2. MS m/z (ESI):233.1[M+1].

### Intermediate preparation example 3

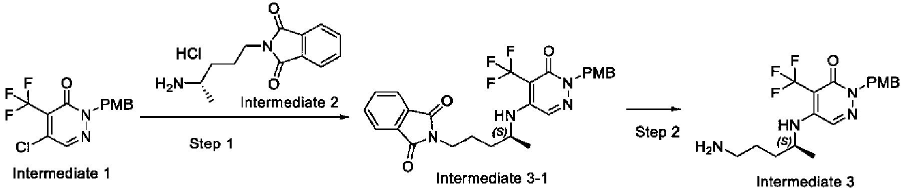

Step 1: Intermediate 1 (10.08 g, 31.64 mmol) was dissolved in acetonitrile (100 mL), and intermediate 2 (7 , 30.14 mmol) and triethylamine (12.53 mL, 90.42 mmol) were added. The reaction was carried out at 85°C for 3 hours. The reaction solution was concentrated under reduced pressure. The remaining solution was added with water (40 mL), followed by extraction with EA (20 mL×3). The organic phases were combined, and dried over anhydrous sodium sulfate. After filtering to remove the desiccant, the filtrate was concentrated under reduced pressure. The residue was purified by silica gel column chromatography with an eluant system (PE:EA=3/1) to afford intermediate 3-1. MS m/z (ESI): 515.2[M+1].

Step 2: Intermediate 3-1 (11.2 g, 21.77 mmol) was dissolved in ethanol (125 mL). Hydrazine hydrate (21.12 mL, 435.37 mmol) was added. The reaction was carried out at 20°C for 3 hours. The reaction solution was filtered, and the filtrate was concentrated under reduced pressure. Acetonitrile (100 mL) was added, followed by filtering under stirring. The filtrate was concentrated under reduced pressure, followed by repeating 4 times. The filtrate was concentrated under reduced pressure to afford intermediate 3. MS m/z (ESI): 385.2[M+1].

### Intermediate preparation example 4

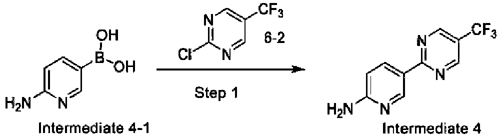

Intermediate 4-1 (500 mg, 3.63 mmol, Bide), compound 6-2 (661.68 mg, 3.63 mmol, Bide), and cesium carbonate (2362.21 mg, 7.25 mmol) were added to a solution of 1, 4-dioxane (6 mL) and water (1.5 mL). Bis[5-(diphenylphosphino)cyclopenta-1, 3-dienyl]-λ2-iron (II) DCM palladium chloride (592.07 mg, 0.73 mmol) was added. The mixture was purged with nitrogen 3 times. The reaction was carried out at 100°C for 18 hours. The resulting mixture was concentrated under reduced pressure and purified by silica gel chromatography (elution solution: PE: EA= 10: 1 to 1: 1) to afford intermediate 4. MS m/z (ESI):241.0[M+1].

### Intermediate preparation example 5

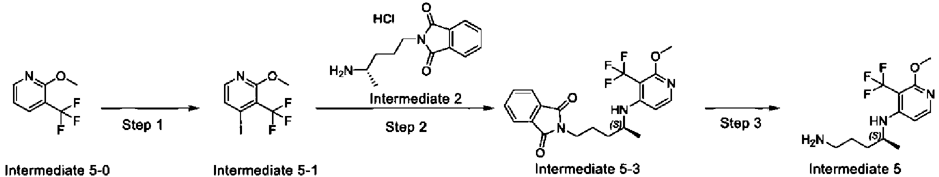

Step 1: At room temperature, diisopropylamine (22.40 mL, 141.14 mmol) was dissolved in THF (150 mL) under nitrogen protection. After the reaction temperature was lowered to - 78°C, a 2.6M n-butyl lithium solution (65.40 mL, 169.37 mmol) was added dropwise to the reaction solution, followed by stirring at this temperature for 10 minutes. After 10 minutes, intermediate 5-0 (25 g, 141.14 mmol) was added dropwise to the reaction solution. After reacting for two hours, a solution of elemental iodine (53.77 g, 211.7 mmol) in THF was added dropwise to the reaction solution at this temperature. The reaction was carried out at room temperature for 18 hours. After the reaction was completed, water (300 mL) and EA (300 mL) were added to the reaction solution. The organic phases were washed with saturated saline (300 mL×3), and dried over sodium sulfate. After filtering, the filtrate was concentrated under reduced pressure to afford the crude. The crude was purified by silica gel column (eluant: EA:PE= 0%-5%) to afford intermediate 5-1. ¹H NMR (400 MHz, DMSO-d6) *δ* 8.01 (d, *J= 5.2* Hz, 1H), 7.76 (d, *J=* 5.2 Hz, 1H), 3.95 (s, 3H).

Step 2: At room temperature, intermediate 5-1 (0.5 g, 1.65 mmol) and intermediate 2 (0.38 g, 1.65 mmol) were dissolved in 1,4-dioxane (6 mL). Cesium carbonate (1.075 g, 3.30 mmol), tris(dibezylideneacetone)dipalladium (0.151 g, 0.17 mmol), and 1, 1-binaphthyl-2, 2-bis(diphenylphosphine) (0.121 g, 0.17 mmol) were added to the reaction solution. The reaction was completed after reacting for 18 hours at 110°C. After the reaction was completed, the crude was afforded by concentration under reduced pressure. The crude was purified by silica gel column (eluant: EA:PE = 25%-33%) to afford intermediate 5-3. MS m/z (ESI): 408.1 [M+1].

Step 3: At room temperature, intermediate 5-3 (0.180 g, 0.44 mmol) was dissolved in ethanol (2 mL). Then, hydrazine hydrate (0.221 g, 4.42 mmol) was added, followed by stirring at 25°C for 18 hours until the reaction was completed. The reaction solution was concentrated under reduced pressure to afford the crude. The crude was slurried with acetonitrile, and then filtered. The filtrate was concentrated to afford intermediate 5. MS m/z (ESI): 278.1 [M+1].

### Intermediate preparation example 6

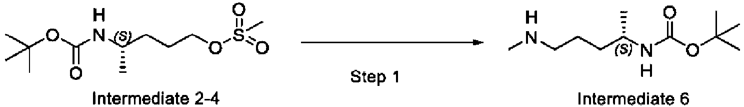

Intermediate 2-4 (1 g, 3.55 mmol) was added to a solution of 30-33 wt% methylamine in ethanol (10 mL). Under a nitrogen atmosphere, the reaction was carried out at 75°C for 16 hours. The reaction solution was concentrated under reduced pressure to afford intermediate 6. MS m/z (ESI):217.1[M+1].

### Intermediate preparation example 7

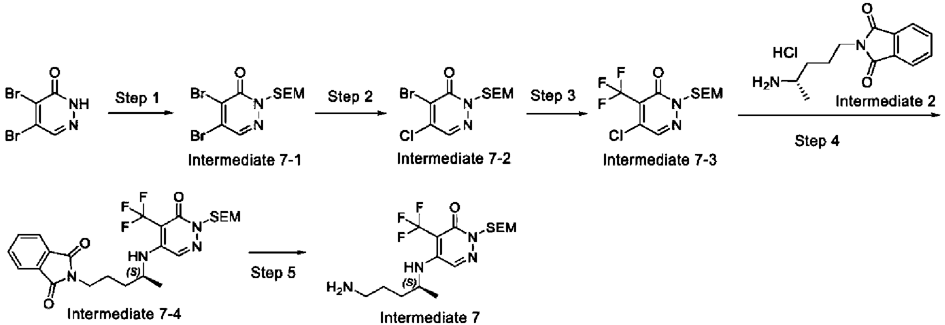

Step 1: 4,5-dibromopyridazin-3-one (25 g, 98.47 mmol, Bide), DIPEA (51.33 mL, 295.4 mmol) were dissolved in acetonitrile (500 mL). 2-(trimethylsilyl )ethoxymethyl chloride (18.26 g, 108.32 mmol) was added dropwise to the solution. The reaction was carried out at 70°C for 3 hours. After the reaction solution was filtered through celite, the filtrate was concentrated under reduced pressure and then eluted by silica gel chromatography with PE:EA = 10:1 to afford intermediate 7-1. MS m/z (ESI):357.0[M-27].

Step 2: Intermediate 7-1 (22 g, 57.27 mmol) was dissolved in NMP (66 mL) and then lithium chloride (2.42 g, 57.27 mmol) was added. The reaction was carried out at 95°C for 4 hours. The reaction solution was poured into 200 mL water, followed by stirring. Then, the aqueous phase was extracted with PE:EA=5:1 (500 mL×2). The mixed organic phase was washed with saturated NaCl solution (100 mL ×4), dried over anhydrous sodium sulfate and filtered. The filtrate was concentrated under reduced pressure to afford intermediate 7-2. MS m/z (ESI):311.0[M-27].

Step 3: Intermediate 7-2 (2 g, 5.89 mmol), CuI (110 mg, 0.59 mmol), and methyl 2,2-difluoro-2-(fluorodioxo-λ6-sulfanyl)acetate (2.25 mL, 17.66 mmol) were added to NMP (10 mL), followed by stirring at 110°C for another 4 hours under nitrogen protection. The reaction mixture was poured into water (20 mL), followed by stirring. The two phases were separated. Then, the aqueous phase was extracted with PE:EA=5:1 (50 mL×2). The mixed organic phase was washed with saturated NaCl solution (30 mL ×4), dried over anhydrous sodium sulfate, and eluted by silica gel chromatography with PE:DCM = 2:1 to afford intermediate 7-3. MS m/z (ESI):301.0[M-27]

Step 4: Intermediate 7-3 (10.0 g, 30.48 mmol) was dissolved in acetonitrile (100 mL), and intermediate 2 (7 g, 30.14 mmol) and triethylamine (12.53 mL, 90.42 mmol) were added. The reaction was carried out at 85°C for 3 hours. The reaction solution was concentrated under reduced pressure. The remaining solution was added with water (40 mL), followed by extraction with EA (20 mL×3). The organic phases were combined, and dried over anhydrous sodium sulfate. After filtering to remove the desiccant, the filtrate was concentrated under reduced pressure. The residue was purified by silica gel column chromatography with an eluant system (PE:EA=3/1) to afford intermediate 7-4. MS m/z (ESI): 497.0[M-27].

Step 5: Intermediate 7-4 (10.0 g, 25.3 mmol) was dissolved in ethanol (125 mL). Hydrazine hydrate (21.12 mL, 435.37 mmol) was added. The reaction was carried out at 20°C for 3 hours. The reaction solution was filtered, and the filtrate was concentrated under reduced pressure. Acetonitrile (100 mL) was added, followed by filtering under stirring. The filtrate was concentrated under reduced pressure, followed by repeating 4 times. The filtrate was concentrated under reduced pressure to afford intermediate 7. MS m/z (ESI): 367.2[M-27].

### Intermediate preparation example 8

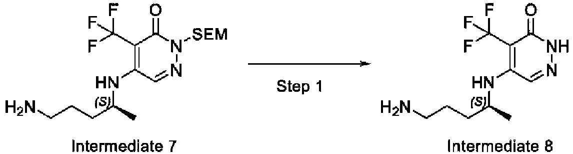

At room temperature, a solution of 4M hydrochloric acid in EA (10 mL) was added to a solution of intermediate 7 (3.0 g, 7.61 mmol) in EA (10 mL). The reaction solution was stirred at room temperature for 18 hours. After the reaction was completed, intermediate 8 was afforded by concentration under reduced pressure. MS m/z (ESI): 265.1 [M+1].

### Intermediate preparation example 9

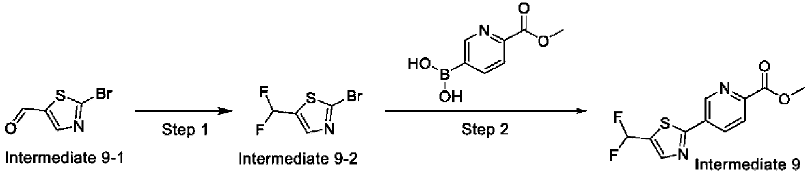

Step 1: At room temperature, intermediate 9-1 (10.00 g, 52.08 mmol, Bide) was dissolved in DCM (100 mL) under nitrogen protection. After the reaction temperature was lowered to - 0°C, bis(2-methoxyethyl)aminosulfur trifluoride (19.20 mL, 104.1 mmol) was added dropwise to the reaction solution, followed by stirring at this temperature for 30 minutes. After 30 minutes, the reaction solution was warmed to room temperature and reacted for two hours. The completion of the reaction was then monitored by TLC. After the reaction was completed, the reaction solution was concentrated under reduced pressure to afford the crude. The crude was purified by silica gel column (eluant: EA:PE= 0%~5%) to afford intermediate 9-2. 1H NMR (400 MHz, CDCl3) δ 7.75 (t, J = 2.2 Hz, 1H), 6.85 (t, J = 54.8 Hz, 1H).

Step 2: At room temperature, intermediate 9-2 (1.30 g, 6.08 mmol) and 6-(methoxycarbonyl)pyridin-3-ylboronic acid (1.00 g, 5.33 mmol, Bide) were dissolved in 1,4-dioxane (6 mL). Sodium carbonate (1.17 g, 11.0 mmol), palladium acetate (0.12 g, 0.55 mmol), and 4,5-bis(diphenylphosphino)-9,9-dimethylxanthene (0.32 g, 0.55 mmol) were added to the reaction solution. After reacting at 120°C for 18 hours, the reaction was completed. After the reaction was completed, the crude was afforded by concentration under reduced pressure. The crude was purified by silica gel column (eluant: EA:PE = 25%~33%) to afford intermediate 9. MS m/z (ESI): 271.0 [M+1].

### Intermediate preparation example 10

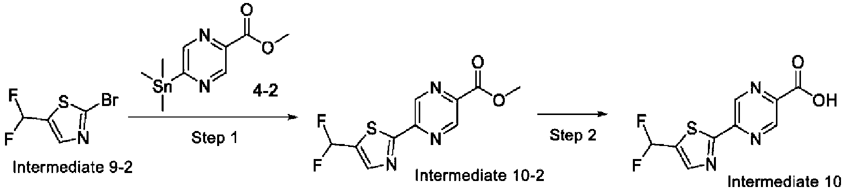

Step 1: At room temperature, intermediate 9-2 (300 mg, 1.40 mmol) and compound 4-2 (420 mg, 1.40 mmol) were dissolved in 1,4-dioxane (6 mL), and Pd(PPh₃)₄ (162 mg, 0.14 mmol) and cuprous iodide (26.7 mg, 0.14 mmol) were added to the reaction solution. After reacting at 120°C for 18 hours, the reaction was completed. After the reaction was completed, the crude was afforded by concentration under reduced pressure. The crude was purified by silica gel column (eluant: EA:PE = 25%~33%) to afford intermediate 10-2. MS m/z (ESI): 272.0 [M+1].

Step 2: Intermediate 10-2 (170 mg, 0.63 mmol) was dissolved in methanol (1 mL), THF (1 mL), and water (1 mL). LiOH (50.3 mg, 1.26 mmol) was added, followed by reacting at 25°C for 1 hour. The reaction solution was concentrated under reduced pressure. The residue was added with 50 mL water, followed by extraction with EA (5 mL×3). Aqueous phase was added with hydrochloric acid (1 M) for adjusting to pH=5, followed by extraction with DCM/methanol (10/1, 5 mL×3). The organic phases were combined. The organic phases were dried over anhydrous sodium sulfate. After filtering to remove the desiccant, the filtrate was concentrated under reduced pressure to afford intermediate 10. MS m/z (ESI):258.0[M+H]

### Intermediate preparation example 11

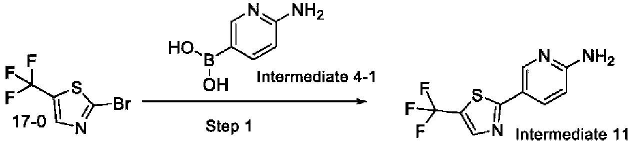

Intermediate 4-1 (300 mg, 2.18 mmol, Bide), compound 17-0 (504 mg, 2.18 mmol, Bide), and cesium carbonate (1.41 g, 4.35 mmol) were added to 1, 4-dioxane (6 mL) and water (1 mL). [1,1'-bis(diphenylphosphino)ferrocene]dichloropalladiumdichloromethane complex (355 mg, 0.44 mmol) was added. The mixture was purged with nitrogen three times. The reaction was carried out at 100°C for 18 hours. The reaction solution was concentrated under reduced pressure and purified by silica gel chromatography (elution solution: PE: EA = 2: 1-1: 1) to afford intermediate 11. MS m/z(ESI):246.0 [M+1].

### Intermediate preparation example 12

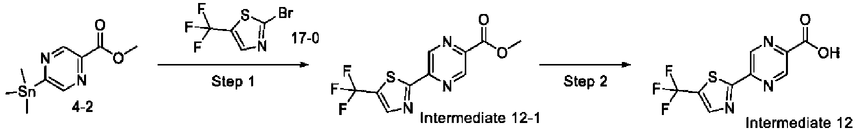

Step 1: Compound 4-2 (500 mg, 1.66 mmol), compound 17-0 (385 mg, 1.66 mmol, Bide) were dissolved in toluene (5 mL), and Pd(PPh₃)₄ (192 mg, 0.17 mmol) was added, followed by purging with nitrogen three times. The reaction was carried out at 100°C for 18 hours. The reaction solution was concentrated under reduced pressure and purified by silica gel column chromatography with an eluant system (PE: EA=10/1-5/1) to afford intermediate 12-1. MS m/z(ESI):290.0[M+1].

Step 2: Intermediate 12-1 (160 mg, 0.55 mmol) was dissolved in methanol (3 mL) and water (3 mL), and LiOH (40 mg, 1.55 mmol) was added. The reaction was carried out at room temperature for 18 hours. The reaction solution was concentrated under reduced pressure. The residue was added with 10 mL water, adjusted to pH=6 with 1M hydrochloric acid solution, filtered, and washed with water (5 mL). The filter cake was oven-dried to afford intermediate 12. MS m/z(ESI):276.0[M+1]

### Intermediate preparation example 13

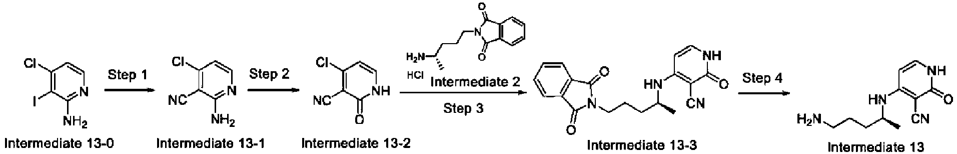

Step 1: Pd(PPh₃)₄ (2.04 g, 1.77 mmol) was added to a solution of 4-chloro-3-iodopyridin-2-ylamine (4.50 g, 17.68 mmol, Bide) and zinc cyanide (1.04 g, 8.84 mmol) in NMP (50 mL). The mixture was heated at 95°C and stirred for 24 hours under nitrogen protection. The reaction solution was poured into water (100 mL), followed by stirring for 5 minutes and filtering. The crude was washed with water (50 mL × 3), filtered, and dried to afford intermediate 13-1. MS m/z (ESI):154.0[M+H].

Step 2: At 0°C, sodium nitrite (1.24 g, 17.91 mmol) and copper sulfate pentahydrate (4.06 g, 16.28 mmol) were added sequentially to a solution of intermediate 13-1 (2.5 g, 16.28 mmol) in hydrochloric acid (30 mL, 6 M). The reaction solution was allowed to warm to room temperature naturally and stirred for 16 hours. The reaction mixture was poured into water (30 mL) and stirred, followed by extraction with EA (20 mL × 3). The organic phases were combined, and dried over anhydrous sodium sulfate. After filtering to remove the desiccant, the filtrate was concentrated under reduced pressure. The residue was purified by silica gel column chromatography with an eluant system (DCM:methanol=20: 1) to afford intermediate 13-2. MS m/z (ESI): 155.0[M+H].

Step 3: Intermediate 2 (413.31 mg, 1.78 mmol) and triethylamine (1.48 mL, 10.68 mmol) were added to a solution of intermediate 13-2 (550 mg, 3.56 mmol) in acetonitrile (20 mL). The mixture was heated under reflux at 85°C and stirred for 4 hours. The reaction solution was concentrated under reduced pressure, and eluted by silica gel chromatography with DCM:CH₃OH = 10:1 to afford intermediate 13-3. MS m/z (ESI):351.38[M+H].

Step 4: Intermediate 13-3 (425 mg, 1.21 mmol) and ethanol (15 mL) were added to 8 mL reactor. 20 equivalents of ammonia water was added, followed by stirring at 20°C for 3 hours under nitrogen protection. The resulting mixture was filtered and washed with MeCN (20 mL × 2). The filtrate was concentrated by rotary evaporator (35~40°C) to afford intermediate 13. MS m/z (ESI):221.28[M+H].

### Intermediate preparation example 14

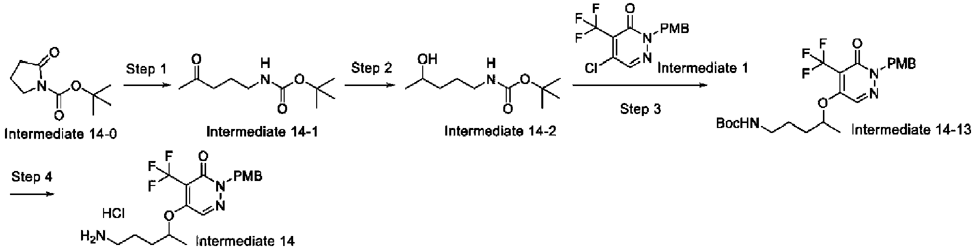

Step 1: Intermediate 14-0 (1.2 g, 6.48 mmol) and THF (25 mL) were added to 250 mL reactor. After cooling to -70°C, methylmagnesium bromide (3.0 mol/L ethyl ether solution) (3.24 mL, 9.72 mmol) was added dropwise. The resulting mixture was stirred at -70°C for another 2 hours. After concentration by rotary evaporator (35-40°C), the residue was added with saturated ammonium chloride aqueous solution (50 mL) and EA (20 mL). The resulting mixture was stirred for 15 minutes. The two phases were separated and the aqueous phase was back-extracted with EA (10 mL×2). The combined organic phase was washed with half-saturated saline solution and dried over anhydrous sodium sulfate. The filtered filtrate was concentrated by rotary evaporator (35-40°C) and purified by flash column chromatography (PE:EA=1/1) to afford intermediate 14-1. MS m/z (ESI):146.1[M-56].

Step 2: Intermediate 14-1 (900 mg, 4.47 mmol) and methanol (30 mL) were added to 100 mL reactor. Sodium borohydride (338.32 mg, 8.94 mmol) was added. The resulting mixture was stirred at 20°C for another 3 hours under nitrogen protection. The aqueous phase was back-extracted with EA (50 mL×2). The combined organic phase was washed with half-saturated saline solution and dried over anhydrous sodium sulfate. The filtered filtrate was concentrated by rotary evaporator (40-45°C) to afford intermediate 14-2. MS m/z (ESI):148.1[M-56].

Step 3: Intermediate 14-2 (685 mg, 3.37 mmol), intermediate 1 (1181.25 mg, 3.71 mmol), and DCM (30 mL) were added to 100 mL reactor. Sodium tert-butoxide (453.36 mg, 4.72 mmol) was added. The resulting mixture was stirred at 20°C for another 12 hours under nitrogen protection. The aqueous phase was extracted with DCM (40 mL×2). The combined organic phase was washed with half-saturated saline solution and dried over anhydrous sodium sulfate. The filtered filtrate was concentrated by rotary evaporator (35-40°C) and purified by flash column chromatography (PE:EA=1/1) to afford intermediate 14-3. MS m/z (ESI):430.2[M-56].

Step 4: At 25°C under nitrogen protection, intermediate 14-3 (300 mg, 0.62 mmol) and DCM (8 mL) were added to 50 mL reactor. A solution of hydrogen chloride in 1,4-dioxane (2 mL, 4 M) was added. The resulting mixture was stirred at 25°C for another 1 hour under nitrogen protection. Then, intermediate 14 was afforded by concentration under reduced pressure. MS m/z (ESI):386.4 [M+1].

### Intermediate preparation example 15

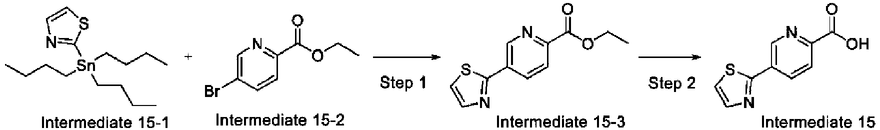

Step 1: Intermediate 15-1 (850 mg, 2.27 mmol, Bide), intermediate 15-2 (522.63 mg, 2.27 mmol, Bide), and bis(tri-tert-butylphosphine)palladium (116.10 mg, 0.23 mmol) were dissolved in 1,4-dioxane (3 mL) solution. Then, the resulting mixture was heated under reflux at 100°C and stirred for 16 hours under nitrogen protection. The reaction mixture was cooled to room temperature, and concentrated under reduced pressure. The residue was purified by silica gel column chromatography with an eluant system PE/EA to afford intermediate 15-3. MS m/z (ESI):235.0 [M-56]. ¹H NMR (400 MHz, DMSO-*d6*)*δ* 9.29 (d, J = 2.1 Hz, 1H), 8.51 (dd, J = 8.2, 2.3 Hz, 1H), 8.17 (d, J = 8.2 Hz, 1H), 8.09 (d, J = 3.2 Hz, 1H), 8.01 (d, J = 3.2 Hz, 1H), 4.38 (q, J = 7.1 Hz, 2H), 1.36 (t, J = 7.1 Hz, 3H).

Step 2: Intermediate 15-3 (230 mg, 0.98 mmol) and sodium hydroxide (196.35 mg, 4.91 mmol) were dissolved in methanol (4 mL) and water (0.8 mL) solution. The mixture was stirred at room temperature for 3 hours. 6M hydrochloric acid was added to reaction solution for adjusting pH to 3-4, followed by extraction with EA. The organic layer was dried over anhydrous sodium sulfate. The desiccant was filtered off. The filtrate was concentrated under reduced pressure to afford intermediate 15. MS m/z (ESI):207.0[M+H]. ¹H NMR (400 MHz, DMSO-*d6*)*δ* 13.43 (s, 1H), 9.27 (d, J = 1.8 Hz, 1H), 8.49 (dd, J = 8.2, 2.2 Hz, 1H), 8.12 (dd, J = 30.8, 5.7 Hz, 2H), 8.00 (d, J = 3.2 Hz, 1H).

### Example 1

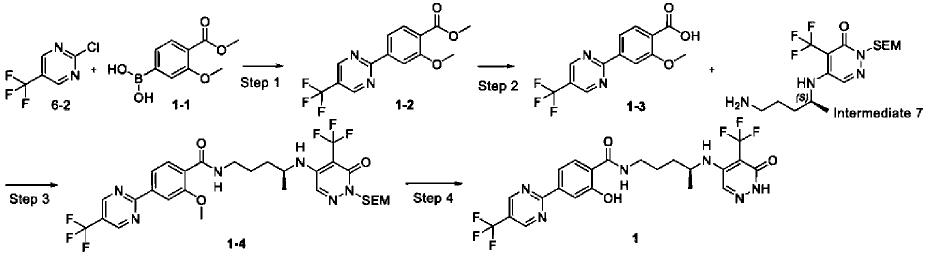

Step 1: Compound 1-1 (575 mg, 2.74 mmol, Bide), compound 6-2 (500 mg, 2.75 mmol), sodium carbonate (871 mg, 8.25 mmol), and 1,1-bis(diphenylphosphino)ferrocenedichloropalladium (80 mg, 0.11 mmol) were dissolved in a mixed solution of 6 mL 1,4-dioxane and 0.6 mL water. The reaction solution was heated at 90°C and reacted under stirring for 16 hours. 10 mL water was added to the reaction solution, followed by extraction with EA (20mL×2). The organic phases were concentrated to afford compound 1-2. MS m/z (ESI): 313.0[M+H].

Step 2: Compound 1-2 (650 mg, 2.08 mmol) was dissolved in methanol (4 mL) and water (3 mL). Sodium hydroxide (250 mg, 6.24 mmol) was added to the reaction solution. The reaction solution was reacted under stirring at 70°C for 3 hours. After the reaction was completed, the reaction solution was concentrated, added with dilute hydrochloric acid to make pH<7, followed by extraction with EA (20 mL×2). The organic phases were combined and washed with saturated saline. The organic phases were dried over anhydrous sodium sulfate. After filtering to remove the desiccant, the filtrate was concentrated under reduced pressure to afford compound 1-3. MS m/z (ESI): 299.0[M+H].

Step 3: Compound 1-3 (330 mg, 1.01 mmol) was dissolved in 5 mL DMF. HATU (383 mg, 1.01 mmol) and DIPEA (261 mg, 2.02 mmol) were added, followed by reacting under stirring at 25°C for 0.5 hours. Intermediate 7 (265 mg, 0.67 mmol) was added, followed by reacting under stirring at 25°C for 1 hour. The reaction solution was added with water (15 mL), followed by extraction with EA (10 mL × 2). The organic phases were washed with saturated saline (30 mL× 3), dried over anhydrous sodium sulfate, filtered and concentrated. The residue was purified by silica gel column chromatography with an eluant system (PE/EA=1/1) to afford compound 1-4. MS m/z (ESI): 647.2[M-27].

Step 4: Compound 1-4 (275 mg, 0.41 mmol) was dissolved in anhydrous DCM (10 mL). The temperature of the reaction solution was lowered to 0°C. 1 M boron tribromide (1.93 mL, 1.93 mmol) was slowly added to the reaction solution under a nitrogen condition, followed by reacting under stirring at 25°C for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 1. MS m/z (ESI): 531.1[M+H]. 1H NMR (400 MHz, DMSO) *δ* 12.63 (s, 1H), 12.44 (s, 1H), 9.39 (d, J = 0.9 Hz, 2H), 9.00 (s, 1H), 8.02 (d, J = 8.2 Hz, 1H), 7.98 - 7.91 (m, 3H), 4.00 (s, 1H), 1.73 - 1.65 (m, 1H), 1.58 (d, J = 5.0 Hz, 3H), 1.20 (d, J = 6.3 Hz, 3H).

### Example 2

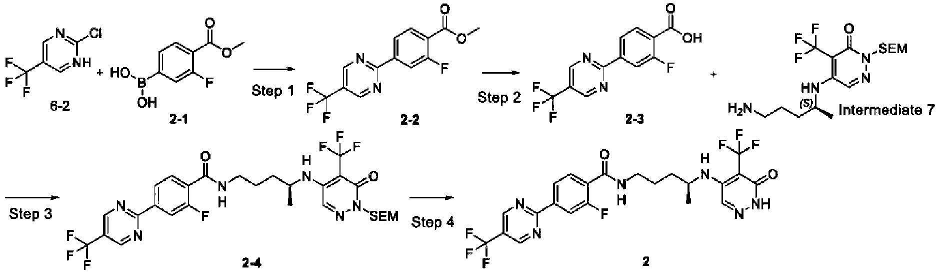

Step 1: Compound 2-1 (543 mg, 2.75 mmol, Bide), compound 6-2 (500 mg, 2.75 mmol), sodium carbonate (871 mg, 8.25 mmol), and 1,1-bis(diphenylphosphino)ferrocenedichloropalladium (80 mg, 0.11 mmol) were dissolved in a mixed solution of 6 mL 1,4-dioxane and 0.6 mL water. The reaction solution was heated at 90°C and reacted under stirring for 16 hours. 10 mL water was added to the reaction solution, followed by extraction with EA (20 mL×2). The organic phases were concentrated to afford compound 2-2. MS m/z (ESI): 301.0[M+H].

Step 2: Compound 2-2 (650 mg, 2.17 mmol) was dissolved in methanol (4 mL) and water (3 mL). Sodium hydroxide (260 mg, 6.50 mmol) was added to the reaction solution. The reaction solution was reacted under stirring at 70°C for 3 hours. After the reaction was completed, the reaction solution was concentrated, added with dilute hydrochloric acid to make pH<7, followed by extraction with EA (20 mL×2). The organic phases were combined and washed with saturated saline. The organic phases were dried over anhydrous sodium sulfate. After filtering to remove the desiccant, the filtrate was concentrated under reduced pressure to afford compound 2-3. MS m/z (ESI): 287.1[M+H].

Step 3: Compound 2-3 (112 mg, 0.39 mmol) was dissolved in 5 mL DMF. HATU (111 mg, 0.29 mmol) and DIPEA (76 mg, 0.59 mmol) were added, followed by reacting under stirring at 25°C for 0.5 hours. Intermediate 7 (79 mg, 0.20 mmol) was added, followed by reacting under stirring at 25°C for 16 hours. The reaction solution was added with water (15 mL), followed by extraction with EA (10 mL×2). The organic phases were washed with saturated saline (30 mL×3), dried over anhydrous sodium sulfate, filtered and concentrated. The residue was purified by silica gel column chromatography with an eluant system (PE/EA=1/1) to afford compound 2-4. MS m/z (ESI): 635.2[M-27].

Step 4: Compound 2-4 (156 mg, 0.24 mmol) was dissolved in a solution of 4M hydrochloric acid in 1,4-dioxane (10 mL). The reaction solution was reacted under stirring at 45°C for 1 hour. The reaction solution was blown dry with nitrogen, dissolved with methanol (4 mL), and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 2. MS m/z (ESI): 533.2[M+H]. 1H NMR (400 MHz, DMSO) δ 12.46 (s, 1H), 9.42 (s, 2H), 8.53 (t, J = 5.1 Hz, 1H), 8.33 (dd, J = 8.1, 1.5 Hz, 1H), 8.20 (dd, J = 11.3, 1.4 Hz, 1H), 7.94 (s, 1H), 7.79 (t, J = 7.7 Hz, 1H), 6.42 - 6.29 (m, 1H), 4.00 (s, 1H), 3.28 (d, J = 5.5 Hz, 2H), 1.68 (d, J = 7.5 Hz, 1H), 1.57 (d, J = 5.7 Hz, 3H), 1.20 (d, J = 6.3 Hz, 3H).

### Example 3

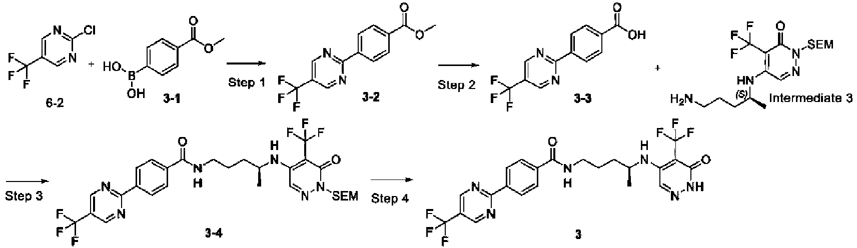

Step 1: Compound 3-1 (500 mg, 2.75 mmol, Bide), compound 6-2 (500 mg, 2.75 mmol), sodium carbonate (871 mg, 8.25 mmol), and 1,1-bis(diphenylphosphino)ferrocenedichloropalladium (80 mg, 0.11 mmol) were dissolved in a mixed solution of 6mL 1,4-dioxane and 0.6 mL water. The reaction solution was heated at 90°C and reacted under stirring for 16 hours. 10 mL water was added to the reaction solution, followed by extraction with EA (20mL×2). The organic phases were concentrated to afford compound 3-2. MS m/z (ESI): 283.0[M+H].

Step 2: Compound 3-2 (600 mg, 2.13 mmol) was dissolved in methanol (5 mL) and water (5 mL). Sodium hydroxide (256 mg, 6.39 mmol) was added to the reaction solution. The reaction solution was reacted under stirring at 70°C for 3 hours. After the reaction was completed, the reaction solution was concentrated, added with dilute hydrochloric acid to make pH<7, followed by extraction with EA (20 mL×2). The organic phases were combined and washed with saturated saline. The organic phases were dried over anhydrous sodium sulfate. After filtering to remove the desiccant, the filtrate was concentrated under reduced pressure to afford compound 3-3. MS m/z (ESI): 269.1[M+H].

Step 3: Compound 3-3 (104 mg, 0.39 mmol) was dissolved in 5 mL DMF. HATU (148 mg, 0.39 mmol), DIPEA (101 mg, 0.78 mmol) were added, followed by reacting under stirring at 25°C for 0.5 hours. Intermediate 7 (102 mg, 0.26 mmol) was added, followed by reacting under stirring at 25°C for 16 hours. The reaction solution was added with water (15 mL), followed by extraction with EA (10 mL×2). The organic phases were washed with saturated saline (30 mL×3), dried over anhydrous sodium sulfate, filtered and concentrated. The residue was purified by silica gel column chromatography with an eluant system (PE/EA=1/1) to afford compound 3-4. MS m/z (ESI): 617.2[M-27].

Step 4: Compound 3-4 (134 mg, 0.21 mmol) was dissolved in a solution of 4M hydrochloric acid in 1,4-dioxane (10 mL). The reaction solution was reacted under stirring at 45°C for 1 hour. The reaction solution was blown dry with nitrogen, dissolved with methanol (4 mL), and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 45%-65%, flow rate: 30 mL/min) to afford compound 3. MS m/z (ESI): 515.2[M+H]. 1H NMR (400 MHz, DMSO) δ 12.45 (s, 1H), 9.40 (s, 2H), 8.67 (t, J = 5.6 Hz, 1H), 8.52 (d, J = 8.5 Hz, 2H), 8.03 (d, J = 8.5 Hz, 2H), 7.94 (s, 1H), 6.35 (d, J = 4.9 Hz, 1H), 4.00 (s, 1H), 3.30 (d, J = 5.5 Hz, 2H), 1.68 (d, J = 7.9 Hz, 1H), 1.57 (s, 3H), 1.20 (d, J = 6.3 Hz, 3H).

### Example 4

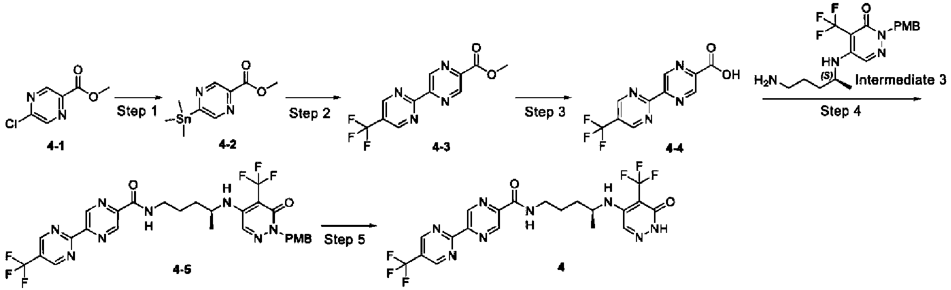

Step 1: Compound 4-1 (10.0 g, 58 mmol, Bide) was dissolved in toluene (200 mL). Hexamethylditin (24.7 g, 75.3 mmol, Bide) and Pd(PPh₃)₄ (3.35 g, 2.9 mmol, Bide) were added, followed by heating to 120°C and stirring for 2 hours under a nitrogen atmosphere. The reaction solution was filtered through celite and concentrated. The crude was purified by neutral alumina chromatographic column with an eluant system (PE: EtOAc=5/1) to afford compound 4-2. MS m/z (ESI):303.0 [M+1].

Step 2: Compound 4-2 (5.5 g, 18.3 mmol) was dissolved in toluene (100 mL). Pd(PPh₃)₄ (2.1 g, 1.83 mmol, Bide) and compound 6-2 (3.34 g, 18.3 mmol) were added, followed by heating to 100°C and stirring for 16 hours under a nitrogen atmosphere. The reaction solution was filtered to afford solid crude. The crude was purified by silica gel column chromatography with an eluant system (PE:EtOAc=1/1) to afford compound 4-3. MS m/z (ESI):285.1 [M+1].

Step 3: Compound 4-3 (1.4 g, 4.93 mmol) was dissolved in methanol (10 mL) and water (10 mL). LiOH (0.83 g, 19.7 mmol) was added, followed by reacting at 25°C for 1 hour. The reaction solution was concentrated under reduced pressure. The residue was added with 30 mL water, followed by extraction with EA (25 mL×3). Aqueous phase was added with hydrochloric acid (1 M) to pH=5, followed by extraction with DCM/methanol (10/1, 40 mL×3). The organic phases were combined. The organic phases were dried over anhydrous sodium sulfate. After filtering to remove the desiccant, the filtrate was concentrated under reduced pressure to afford compound 4-4. MS m/z (ESI):271.0[M+H].

Step 4: Compound 4-4 (1.22 g, 4.52 mmol) was dissolved in DMF (15 mL). Then, intermediate 3 (1.56 g, 4.06 mmol), TCFH (1.52 g, 5.42 mmol, Bide), and N- methylimidazole (1.11 g, 13.55 mmol, Bide) were added. The reaction was carried out at 20°C for 2 hours. The reaction solution was added with water (40 mL), followed by extraction with EA (30 mL×3). The organic phases were combined. The organic phases were washed with saturated saline (40 mL×3), and dried over anhydrous sodium sulfate. After filtering to remove the desiccant, the filtrate was concentrated under reduced pressure. The residue was purified by silica gel column chromatography with an eluant system (DCM: MeOH=20/1) to afford compound 4-5. MS m/z (ESI): 637.4[M+1].

Step 5: Compound 4-5 (1.80 g, 2.83 mmol) was dissolved in TFA (20 mL). Methanesulfonic acid (2 mL) was added. The reaction was carried out at 20°C for 1 hour. The reaction solution was added with methanol (10 mL), and concentrated under reduced pressure. The remaining solution was added with EA (20 mL), neutralized with saturated sodium bicarbonate solution to pH=8. The aqueous phase was extracted with EA (20 mL×3). The organic phases were combined, and dried over anhydrous sodium sulfate. After filtering to remove the desiccant, the filtrate was concentrated under reduced pressure. The residue was purified by silica gel column chromatography with an eluant system (DCM: MeOH=20/1) to afford the crude. The crude was purified by preparative to afford compound 4. MS m/z (ESI):517.2[M+1]. ¹H NMR (400 MHz, DMSO) δ 12.41 (s, 1H), 9.60 (d, J = 1.4 Hz, 1H), 9.52 (s, 2H), 9.37 (d, J = 1.4 Hz, 1H), 9.16 (t, J = 6.0 Hz, 1H), 7.92 (s, 1H), 6.31 (d, J = 4.9 Hz, 1H), 3.99 (d, J = 5.5 Hz, 1H), 3.35 (d, J = 6.3 Hz, 2H), 1.72 - 1.50 (m, 4H), 1.18 (d, J = 6.3 Hz, 3H).

### Example 5

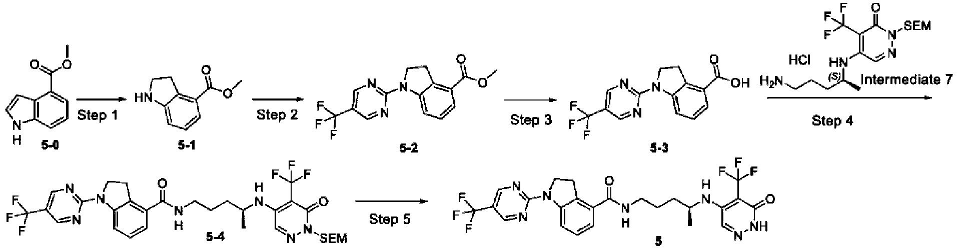

Step 1: Compound 5-0 (2.0 g, 11.42 mmol, Bide) was dissolved in 100 mL DCM. TFA (17.9 mL, 239.7 mmol) was added, and then triethylsilane (5.0 mL, 30.82 mmol) was added. The reaction was carried out at room temperature for 16h. The reaction solution was poured into 100 mL water, and adjusted to pH 9 with sodium hydroxide, followed by extraction with DCM (50 mL×2). The organic phases were combined. The organic phases were dried over anhydrous sodium sulfate. After filtering to remove the desiccant, the filtrate was concentrated under reduced pressure, to afford compound 5-1. MS m/z (ESI): 178.0[M+1].

Step 2: Compound 5-1 (600 mg, 3.38 mmol), DIPEA (0.88 mL, 5.08 mmol) were dissolved in 12 mL isopropanol. At room temperature, compound 6-2 (925 mg, 5.07 mmol) was added. The reaction system was reacted at 90°C for 3 hours. The reaction solution was concentrated under reduced pressure and purified by silica gel column chromatography with an eluant system (PE: EA=3/1) to afford compound 5-2. MS m/z (ESI): 324.1[M+1].

Step 3: Compound 5-2 (240 mg, 0.74 mmol) was added to methanol/water (3 mL/1 mL). Then, LiOH (71 mg, 2.96 mmol) was added. The reaction system was reacted at 70°C for 3 hours. After the reaction solution was concentrated under reduced pressure to remove methanol, 1M hydrochloric acid aqueous solution was added for adjusting pH to 5, followed by extraction with EA (20mL×2). The organic phases were combined. The organic phases were dried over anhydrous sodium sulfate. After filtering to remove the desiccant, the filtrate was concentrated under reduced pressure to afford compound 5-3. MS m/z (ESI): 310.0[M+1].

Step 4: Compound 5-3 (120 mg, 0.39 mmol) was dissolved in 2 mL DMF, and DIPEA (101 mg, 0.78 mmol) and HATU (148 mg, 0.39 mmol) were added sequentially. The reaction solution was reacted at room temperature for 0.5 hours. Intermediate 7 (102 mg, 0.26 mmol) was added. The reaction solution was reacted at room temperature for 16 hours. The reaction solution was added with 10 mL water, followed by extraction with EA (5 mL×2). The organic phases were combined, and washed with saturated saline (20 mL×3) . The organic phases were dried over anhydrous sodium sulfate. After filtering to remove the desiccant, the filtrate was concentrated under reduced pressure to afford compound 5-4. MS m/z (ESI): 658.2[M-27].

Step 5: Compound 5-4 (200 mg, 0.26 mmol) was dissolved in 4M hydrochloric acid in 1,4-dioxane (2 mL). The reaction was carried out at room temperature for 16 hours. The reaction solution was concentrated under reduced pressure and purified by high performance liquid chromatography (Waters-2545, chromatographic column: SharpSil-T C18, 30×150 mm,5 µm; mobile phase: water (containing 10 mmol/L ammonium bicarbonate) and acetonitrile, gradient ratio: acetonitrile 38%-45%, flow rate: 30 mL/min) to afford compound 5. MS m/z (ESI): 556.2[M+1]. ¹H NMR (400 MHz, DMSO) *δ* 8.97 (s, 2H), 8.48 (d, *J=* 7.9 Hz, 1H), 8.34 (t, *J* = 5.7 Hz, 1H), 7.93 (s, 1H), 7.32 (t, *J=* 7.8 Hz, 1H), 7.30 - 7.21 (m, 1H), 6.38 - 6.29 (m, 1H), 4.24 (t, *J* = 8.6 Hz, 2H), 3.99 (s, 1H), 3.38 (t, *J* = 8.6 Hz, 2H), 3.30 (s, 2H), 3.24 (d, *J* = 7.3 Hz, 1H), 1.75 - 1.62 (m, 1H), 1.54 (s, 3H), 1.20 (d, *J* = 6.3 Hz, 3H).

### Example 6

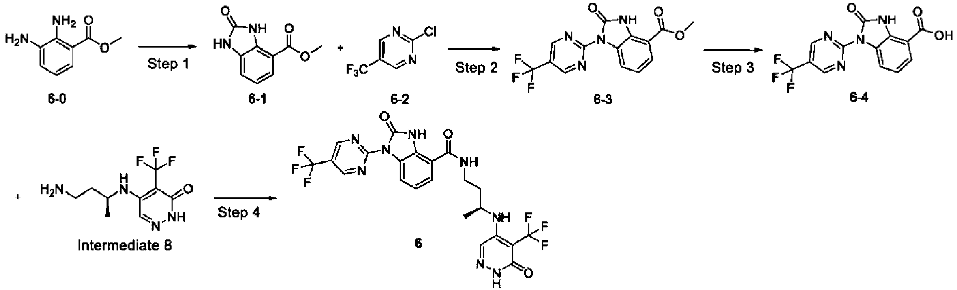

Step 1: Compound 6-0 (2500 mg, 15.04 mmol, Bide), N,N'-carbonyldiimidazole (2.25 mL, 18.05 mmol) were dissolved in 15 mL 1,2-dichloroethane. The reaction solution was reacted under stirring at room temperature for 16 hours. The reaction solution was concentrated. The residue was washed with 20 mL water, followed by filtering. The solid was collected and dried under reduced pressure to afford compound 6-1. MS m/z (ESI):193.0[M+1].

Step 2: Compound 6-1 (500 mg, 2.60 mmol) and compound 6-2 (712.38 mg, 3.90 mmol) were dissolved in DMF (15 mL). Cesium carbonate (2543.22 mg, 7.81 mmol) was added, followed by reacting under stirring at 90°C for 12 hours. The reaction solution was cooled to room temperature, and 10 mL water was added, followed by extraction with EA (10 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE/EA=1/1) to afford compound 6-3. MS m/z (ESI):339.0[M+1]. ¹H NMR (400 MHz, DMSO) δ 11.32 (s, 1H), 9.35 (s, 2H), 8.08 (d, J = 7.9 Hz, 1H), 7.64 (d, J = 8.0 Hz, 1H), 7.15 (t, J = 8.0 Hz, 1H), 3.85 (s, 3H).

Step 3: Compound 6-3 (250 mg, 0.74 mmol) was dissolved in 2 mL water, and then 10 mL hydrogen chloride solution was added. The reaction solution was reacted at 60°C for 4 hours. The reaction solution was concentrated under reduced pressure. The residue was purified by reversed-phase with an eluant system (acetonitrile /water=3/1) to afford compound 6-4. MS m/z (ESI):323.0[M-1].

Step 4: Compound 6-4 (30 mg, 0.09 mmol) and intermediate 8 (27.87 mg, 0.11 mmol) were dissolved in DMF (3 mL). Then, 1-hydroxylbenzotriazole (0.04 mL, 0.23 mmol) and DIPEA (0.04 mL, 0.23 mmol) were added. Finally, 1-(3-dimethylaminopropyl)-3-ethylcarbodiimide hydrochloride (35.58 mg, 0.19 mmol) was added. The reaction was carried out overnight at room temperature. The reaction solution was concentrated under reduced pressure and purified by high performance liquid chromatography (Waters-2545, chromatographic column: SharpSil-T C18, 30×150 mm,5 µm; mobile phase: water (containing 10 mmol/L TFA) and acetonitrile, gradient ratio: acetonitrile 30%-60%, flow rate: 30 mL/min) to afford compound 6. MS m/z (ESI):555.1[M-1]. ¹H NMR (400 MHz, DMSO) *δ* 12.45 (s, 1H), 10.94 (s, 1H), 9.41 (s, 2H), 8.61 (s, 1H), 8.02 (d, J = 8.0 Hz, 1H), 7.92 (s, 1H), 7.59 (d, J = 8.1 Hz, 1H), 7.16 (t, J = 8.0 Hz, 1H), 6.42 (s, 1H), 4.07 (s, 1H), 2.52 (s, 2H), 1.94 (dd, J = 13.9, 7.0 Hz, 1H), 1.83 (dd, J = 13.9, 6.1 Hz, 1H), 1.24 (d, J = 6.3 Hz, 3H).

### Example 7

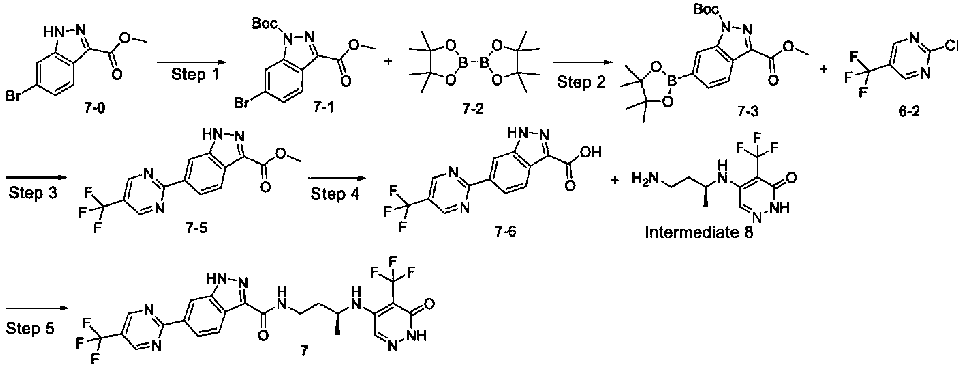

Step 1: Compound 7-0 (2.5 g, 9.8 mmol) was dissolved in 30 mL DCM. At room temperature, Boc anhydride (2.6 g, 11.9 mmol), triethylamine (2 g, 20 mmol), and DMAP (122 mg, 1 mmol) were added, followed by reacting under stirring for 16 hours while maintaining the temperature. The reaction solution was concentrated under reduced pressure. The residue was purified by silica gel column chromatography with an eluant system (PE/EA=5/1) to afford compound 7-1. MS m/z (ESI):298.9[M-56+1].

Step 2: 1,4-dioxane (20 mL) was added to compound 7-1 (1.3 g, 3.6 mmol), compound 7-2 (1.1 g, 4.4 mmol), potassium acetate (900 mg, 9.2 mmol), and 1,1-bis(diphenylphosphino) ferrocenedichloropalladium (II) (140 mg, 0.2 mmol), followed by purging with nitrogen 3 times, and reacting under stirring at 85°C for 16 hours. The reaction solution was cooled to room temperature, diluted with EA, and filtered. The filtrate was concentrated under reduced pressure. The residue was purified by silica gel column chromatography with an eluant system (DCM/methanol=50/1) to afford compound 7-3. MS m/z (ESI):347.1[M-56+1].

Step 3: Compound 7-3 (500 mg, 1.2 mmol) was added to a single-necked flask. Compound 6-2 (320 mg, 1.8 mmol), sodium carbonate (250 mg, 2.4 mmol), and bis(tri-tert-butylphosphine)palladium (75 mg, 146.1 µmol) were added, followed by purging with nitrogen 3 times. 1,4-dioxane (7 mL) and water (0.7 mL) were added, followed by reacting under stirring at 85°C for 5 hours. The reaction solution was cooled to room temperature, diluted with EA and filtered. The filtrate was concentrated under reduced pressure. The residue was purified by silica gel column chromatography with an eluant system (DCM/methanol=50/1) to afford compound 7-5. MS m/z (ESI):323.0[M+1].

Step 4: Compound 7-5 (150 mg, 465.8 µ mol) was dissolved in methanol (2.1 mL) and water (0.7 mL). Anhydrous LiOH (67.2 mg, 2.8 mol) was added, followed by reacting under stirring at 60°C for 3 hours. The reaction solution was concentrated under reduced pressure, and then 2M dilute hydrochloric acid was added for adjusting acidity-alkalinity to 5, followed by extraction with EA. The organic phases were dried over anhydrous sodium sulfate, and filtered. The filtrate was concentrated under reduced pressure to afford compound 7-6. MS m/z (ESI):309.0[M+1].

Step 5: Compound 7-6 (60 mg, 194.8 µmol) was dissolved in DMF (3 mL). At room temperature, DIPEA (82.4 mg, 638.8 µmol), 1-(3-dimethylaminopropyl)-3- ethylcarbodiimide hydrochloride (75 mg, 392.7 µmol), 1-hydroxylbenzotriazole (53 mg, 392.5 µmol), and intermediate 8 (30 mg, 120 µmol) were added. The reaction was carried out at room temperature for 4 hours. The reaction solution was concentrated under reduced pressure and purified by high performance liquid chromatography (Waters-2545, chromatographic column: SharpSil-T C18, 30×150 mm,5 µm ; mobile phase: water (containing 10 mmol/L ammonium bicarbonate) and acetonitrile, gradient ratio: acetonitrile 28%-41%, flow rate: 30 mL/min) to afford compound 7. MS m/z (ESI):541.2[M+1]. ¹H NMR (400 MHz, DMSO) δ 13.83 (s, 1H), 12.44 (s, 1H), 9.40 (s, 3H), 8.71 (s, 1H), 8.61 (t, *J=* 5.9 Hz, 1H), 8.33 (dt, *J* = 17.6, 8.6 Hz, 3H), 7.90 (s, 1H), 6.39 (s, 1H), 4.15 - 3.93 (m, 1H), 3.44 - 3.34 (m, 3H), 2.05 - 1.74 (m, 3H), 1.24 (d, *J=* 6.3 Hz, 5H).

### Example 8

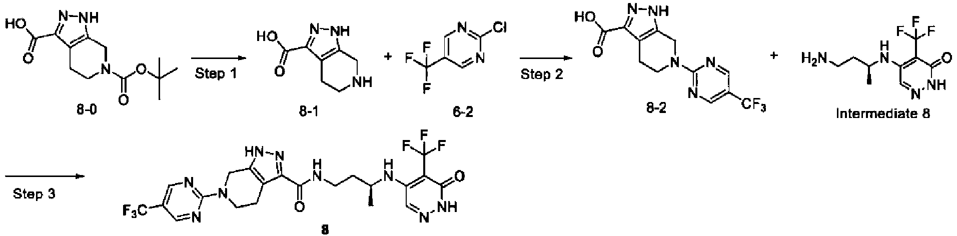

Step 1: Compound 8-0 (1 g, 3.7 mmol) was dissolved in 10 mL DCM. TFA (3 mL, Shanghai Bide) was added at room temperature, followed by reacting under stirring for 2 hours while maintaining the temperature. The reaction solution was directly concentrated under reduced pressure to afford compound 8-1. MS m/z (ESI):168.2[M+1].

Step 2: Compound 8-1 (640 mg, 3.8 mmol) was dissolved in 1,4-dioxane (10 mL), and compound 6-2 (800 mg, 4.2 mmol) and DIPEA (2.451 g, 19 mol) were added, followed by reacting under stirring at 85°C for 10 hours. The reaction solution was cooled to room temperature and concentrated under reduced pressure. The residue was purified by silica gel column chromatography with an eluant system (DCM/methanol=10/1) to afford compound 8-2. MS m/z (ESI):314.1[M+1].

Step 3: Compound 8-2 (50 mg, 159.7 µmol) was dissolved in DMF (3 mL). At room temperature, DIPEA (82.4 mg, 638.8 µmol), 1-(3-dimethylaminopropyl)-3- ethylcarbodiimide hydrochloride (61 mg, 319.4 µmol), 1-hydroxylbenzotriazole (43.1 mg, 319.4 µmol), and intermediate 8 (50 mg, 200 µmol) were added. The reaction was carried out at room temperature for 3 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 8. MS m/z (ESI):546.2[M+1]. ¹H NMR (400 MHz, DMSO) δ 13.03 (s, 1H), 12.42 (s, 1H), 8.77 (s, 2H), 8.13 (s, 1H), 7.85 (s, 1H), 6.35 (s, 1H), 4.95 (s, 2H), 4.09 (t, *J* = 5.5 Hz, 2H), 4.01 - 3.89 (m, 1H), 3.28 - 3.19 (m, 2H), 2.79 (s, 2H), 1.93 - 1.67 (m, 2H), 1.20 (d, *J* = 6.3 Hz, 3H).

### Example 9

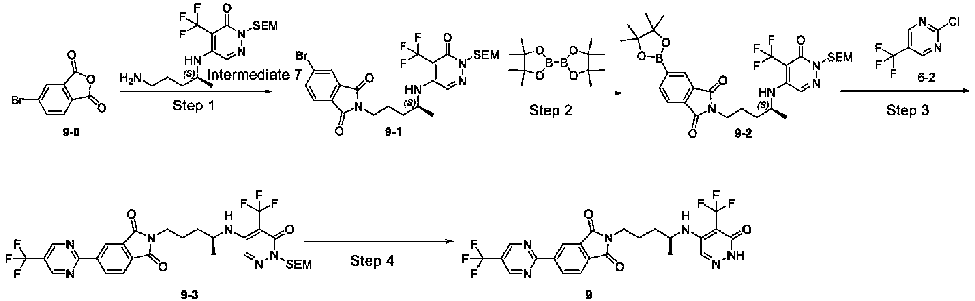

Step 1: Compound 9-0 (220 mg, 0.98 mmol, Bide), intermediate 7 (502 mg, 1.27 mmol) were dissolved in 20 mL ethanol. The reaction solution was reacted under stirring at room temperature for 16 hours. 10 mL acetic acid was added, followed by reacting under stirring at 90°C for 16 hours. The reaction solution was concentrated. The residue was purified by silica gel column chromatography with an eluant system (PE/EA=2/1) to afford compound 9-1. MS m/z (ESI): 575.0[M-28].

Step 2: Compound 9-1 (168 mg, 0.27 mmol) and bis(pinacolato)diboron (140 mg, 0.56 mmol) were dissolved in anhydrous 1,4-dioxane (3.5 mL). Potassium acetate (82 mg, 0.83 mmol), 2-dicyclohexylphosphino-2',4',6'-triisopropylbiphenyl (26 mg, 0.06 mmol), tris(dibezylideneacetone)dipalladium (25 mg, 0.03 mmol) were added sequentially, followed by reacting under stirring at 90°C for 4 hours under a nitrogen condition. After the reaction solution was cooled to room temperature, the reaction solution was concentrated. The residue was purified by silica gel column chromatography with an eluant system (PE/EA=5/1) to afford compound 9-2. MS m/z (ESI): 623.4[M-27].

Step 3: Compound 9-2 (123 mg, 0.19 mmol) was dissolved in a mixed solvent of 7.5 mL 1,4-dioxane and 1.5 mL water. Sodium carbonate (60 mg, 0.57 mmol), compound 6-2 (35 mg, 0.19 mmol), [1,1'-bis(diphenylphosphino) ferrocene]dichloropalladiumdichloromethane complex (15 mg, 2.02 mmol) were added, followed by reacting under stirring at 90°C for 16 hours under a nitrogen condition. The reaction solution was concentrated under reduced pressure. The residue was purified by silica gel column chromatography with an eluant system (PE/EA=10/1) to afford compound 9-3. MS m/z (ESI): 647.2[M-27].

Step 4: Compound 9-3 (31 mg, 0.05 mmol) was dissolved in a solution of 4M hydrochloric acid in 1,4-dioxane (4.5 mL), followed by reacting under stirring at 45°C for 16 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 9. MS m/z (ESI): 541.2[M+H]. ¹H NMR (400 MHz, DMSO) δ 12.41 (s, 1H), 8.87 (d, J = 7.9 Hz, 1H), 8.75 (s, 1H), 8.11 - 7.90 (m, 2H), 6.34 (s, 1H), 3.96 (s, 1H), 3.62 (t, J = 6.3 Hz, 2H), 1.66 (s, 4H), 1.52 (s, 1H), 1.16 (d, J = 6.3 Hz, 3H).

### Example 10

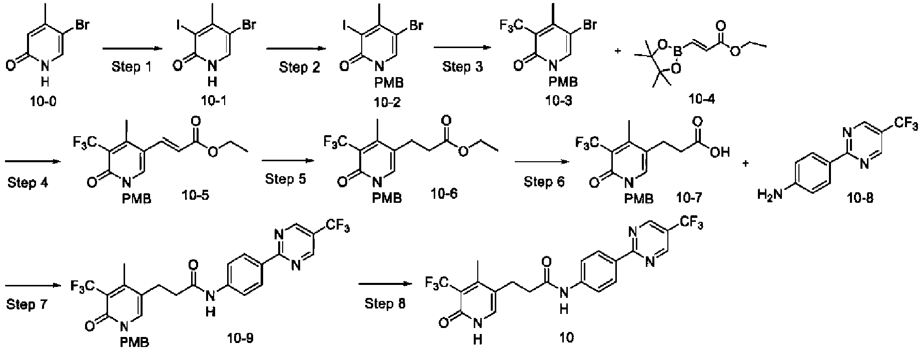

Step 1: Compound 10-0 (10 g, 53.19 mmol, Bide), N-iodosuccinimide (19.15 g, 85.10 mmol) were dissolved in 100 mL acetonitrile. The reaction solution was reacted under stirring at room temperature for 16 hours. The reaction solution was concentrated to afford compound 10-1. ¹H NMR (400 MHz, DMSO) δ 12.07 (s, 1H), 7.72 (s, 1H), 2.51 (s, 3H).

Step 2: Compound 10-1 (500 mg, 1.59 mmol) was dissolved in DMF (6 mL). Then, at 0°C, sodium hydride (38.23 mg, 1.59 mmol) was added. After half an hour, 4-methoxybenzylchloride (0.22 mL, 1.59 mmol) was added. The reaction solution was reacted under stirring at room temperature for 1 hour. After completion, the reaction solution was added with 10 mL water, followed by extraction with EA (20 mL×2). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE/EA=3/1) to afford compound 10-2. MS m/z (ESI):436.0[M+1].

Step 3: Compound 10-2 (320 mg, 0.74 mmol), methyl fluorosulfonyldifluoroacetate (141.63 mg, 0.74 mmol) were dissolved in 10 mL DMF. Then, cuprous iodide (28 mg, 0.15 mmol) was added. The reaction solution was reacted at 100°C for 6 hours. The reaction solution was cooled to room temperature, and 10 mL water was added, followed by extraction with EA (20 mL×2). The organic phases were combined, and washed with saturated saline. The organic phases were dried over anhydrous sodium sulfate. After filtering to remove the desiccant, the filtrate was concentrated under reduced pressure. The residue was purified by preparative silica gel plate with an eluant system (PE/EA=1/1) to afford compound 10-3. MS m/z (ESI):400.0[M+Na⁺].

Step 4: Compound 10-3 (180 mg, 0.48 mmol), compound 10-4 (216 mg, 0.96 mmol) were dissolved in a mixed solvent of 2 mL 1,4-dioxane and 0.2 mL water. Potassium carbonate (198 mg, 1.44 mmol) and [1,1'-bis(diphenylphosphino)ferrocene]dichloropalladium (II) (17.5 mg, 0.05 mmol) were added, followed by reacting at 85°C for 16 hours under a nitrogen condition. The reaction solution was cooled to room temperature and concentrated under reduced pressure. The residue was purified by silica gel column chromatography with an eluant system (PE/EA=2/1) to afford compound 10-5. MS m/z (ESI):396.1[M+H⁺].

Step 5: Compound 10-5 (152 mg, 0.74 mmol) was dissolved in 4 mL ethanol, and Pd/C (15 mg, 0.37 mmol) was added. The reaction solution was reacted at 25°C for16 hours. The reaction solution was filtered through celite. The filter cake was rinsed with ethanol (10 mL). The filtrate was concentrated under reduced pressure to afford compound 10-6. MS m/z (ESI): 398.1[M+H⁺].

Step 6: Compound 10-6 (121 mg, 0.30 mmol) was dissolved in a mixed solvent of 4 mL methanol and 2 mL water. LiOH (39 mg, 0.60 mmol) was added. The reaction solution was reacted at 25°C for 16 hours. The reaction solution was diluted by adding water (10 mL), followed by concentration under reduced pressure to remove methanol. 0.1M hydrochloric acid solution was added dropwise. The solution was adjusted to pH<7, followed by extraction with DCM/isopropanol (3/1) (8 mL×2). The organic phases were washed with saturated saline (20 mL×2), and dried over anhydrous sodium sulfate. After filtering to remove the desiccant, the filtrate was concentrated under reduced pressure. The residue was slurried by adding 10 mL PE, followed by filtering, to afford compound 10-7. MS m/z (ESI): 370.1[M+H⁺].

Step 7: Compound 10-7 (80 mg, 0.22 mmol) was dissolved in 3 mL acetonitrile, and N-methylimidazole (63 mg, 0.77 mmol) and TCFH (68 mg, 0.24 mmol) were added sequentially. The reaction solution was reacted at room temperature for 0.5 hours. Compound 10-8 (78 mg, 0.32 mmol) was added. The reaction solution was reacted at room temperature for 16 hours. The reaction solution was concentrated under reduced pressure. The residue was purified by silica gel column chromatography with an eluant system (PE/EA=1/1) to afford compound 10-9. MS m/z (ESI): 591.2[M+H⁺].

Step 8: Compound 10-9 (65 mg, 0.11 mmol) was dissolved in 3 mL TFA, and TfOH (34 mg, 0.23 mmol) was added. The reaction solution was reacted at 45°C for 16 hours. The reaction solution was concentrated under reduced pressure and purified by high performance liquid chromatography (Waters-2545, chromatographic column: SharpSil-T C18, 30×150 mm,5 µm; mobile phase: water (containing 10 mmol/L TFA) and acetonitrile, gradient ratio: acetonitrile 30%-60%, flow rate: 30 mL/min) to afford compound 10. MS m/z (ESI): 471.1[M+H⁺]. ¹H NMR (400 MHz, DMSO) δ 11.96 (s, 1H), 10.24 (s, 1H), 9.28 (s, 2H), 8.41 (d, J = 8.8 Hz, 2H), 7.78 (d, J = 8.8 Hz, 2H), 7.41 (s, 1H), 2.77 (t, J = 7.3 Hz, 2H), 2.67 (s, 1H), 2.58 (t, J = 7.6 Hz, 2H), 2.36 - 2.31 (m, 3H).

### Example 11

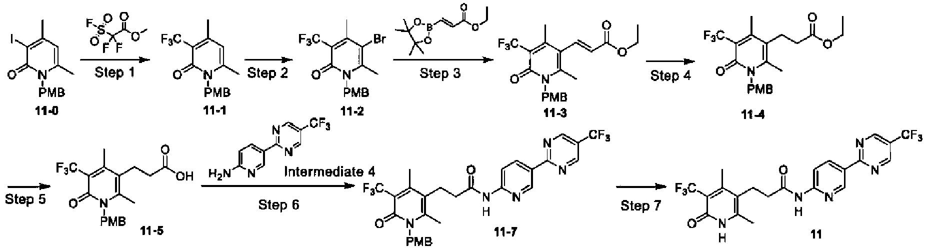

Step 1: At room temperature, methyl fluorosulfonyldifluoroacetate (5.46 g, 28.4 mmol) and cuprous iodide (2.32 g, 12.2 mmol) were added to a solution of compound 11-0 (3.00 g, 8.13 mmol) in NMP (40 mL). The reaction solution was reacted at 110°C for 18 hours. After the reaction was completed, the mixture was poured into water (100 mL), followed by extraction with EA (50 mL × 3). The organic phases were combined, washed with saturated saline (100 mL × 2), dried over anhydrous sodium sulfate, and filtered. The filtrate was concentrated under reduced pressure to afford the crude compound. The crude compound was purified by silica gel column (eluant: EA: PE = 25%-40%) to afford compound 11-1. MS m/z (ESI): 312.1 [M+1].

Step 2: At 25°C, N-bromosuccinimide (1.34 g, 7.52 mmol) was added to a solution of compound 11-1 (2.34 g, 7.52 mmol) in acetonitrile (30 mL). The reaction solution was stirred at room temperature for 2 hours. After the reaction was completed, the mixture was concentrated under reduced pressure to afford the crude compound. The crude compound was purified by silica gel column to afford compound 11-2. ¹H NMR (400 MHz, CDCl₃) δ 7.19 - 7.04 (m, 2H), 6.92 - 6.73 (m, 2H), 5.36 (s, 2H), 3.78 (d, *J=* 3.5 Hz, 3H), 2.60 (s, 3H), 2.54 (q, *J* = 2.3 Hz, 3H).

Step 3: At room temperature, 1,1-bis(diphenylphosphino)ferrocenedichloropalladium (0.62 g, 0.45 mmol) and potassium carbonate (2.55 g, 18.4 mmol) were added to a solution of compound 11-2 (2.40 g, 6.15 mmol) and ethyl (E)-3- (4,4,5,5-tetramethyl-1,3,2-dioxaborolan-2-yl)acrylate (2.78 g, 12.3 mmol) in 1, 4-dioxane (30 mL) and water (5 mL), followed by heating to 90°C and stirring for 2 h. After the reaction was completed, the crude product was afforded by concentration under reduced pressure. The crude product was purified by silica gel column (eluant: EA: PE = 10%-35%) to afford compound 11-3. MS m/z (ESI): 410.1 [M+1].

Step 4: At 25°C, palladium on carbon (200 mg, 10 wt%) was added to a solution of compound 11-3 (2.00 g, 4.89 mmol) in ethanol (30 mL), followed by stirring at room temperature for 2 hours under a hydrogen atmosphere. After the reaction was completed, the reaction solution was filtered, and the filtrate was concentrated under reduced pressure to afford compound 11-4. MS m/z (ESI): 412.2 [M+1].

Step 5: At 25°C, a solution of LiOH (85.6 mg, 3.57 mmol) in water (3 mL) was added to a solution of compound 11-4 (490 mg, 1.19 mmol) in THF (3 mL) and anhydrous methanol (3 mL). The reaction solution was stirred at room temperature for 2 hours. After the reaction was completed, the mixture was concentrated and adjusted to pH 4-5 with saturated sodium bicarbonate solution, followed by extraction with DCM (10 mL×3). The organic phases were combined, washed with saturated saline (10 mL×2), dried over anhydrous sodium sulfate, and filtered. The filtrate was concentrated under reduced pressure to afford compound 11-5. MS m/z (ESI): 384.1 [M+1].

Step 6: At room temperature, N-methylimidazole (99 mg, 0.77 mmol) and TCFH (117 mg, 0.31 mmol) were added to a solution of compound 11-5 (69 mg, 0.26 mmol) and intermediate 4 (60 mg, 0.26 mmol) in acetonitrile (2 mL). The reaction solution was stirred at room temperature for 18 h. After the reaction was completed, the crude product was afforded by concentration under reduced pressure. The crude product was purified by silica gel plate (eluant: EA: PE = 25%-60%) to afford compound 11-7. MS m/z (ESI): 606.4 [M+1].

Step 7: At room temperature, TfOH (0.20 mL, 2.25 mmol) was added to a solution of compound 11-7 (70 mg, 0.12 mmol) in TFA (2 mL). The reaction solution was stirred at 50°C for 3 hours. After the reaction was completed, the crude product was purified by preparative HPLC (chromatographic column: Waters-SunFire-C18-10 µm-19×250 mm; mobile phase: A: 0.1% formic acid/water, B: acetonitrile, gradient ratio: acetonitrile 40-70%, flow rate: 25 mL/min) to afford compound 11. MS m/z (ESI): 486.2 [M+1]. ¹H NMR (400 MHz, DMSO) δ 12.00 (s, 1H), 10.96 (s, 1H), 9.35 (s, 2H), 9.32 (d, *J* = 1.8 Hz, 1H), 8.74 (dd, *J* = 8.8, 2.3 Hz, 1H), 8.31 (d, *J* = 8.8 Hz, 1H), 2.82 - 2.72 (m, 2H), 2.61 - 2.53 (m, 2H), 2.34 - 2.31 (m, 3H), 2.30 (s, 3H).

### Example 12

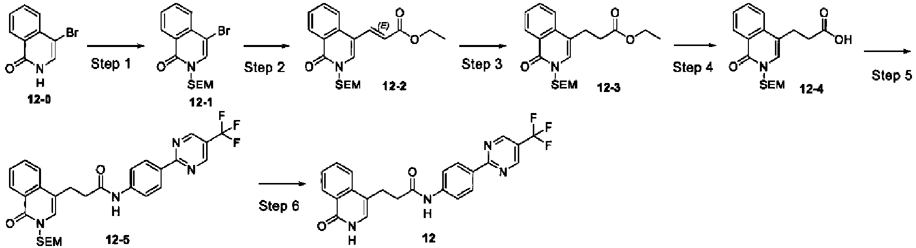

Step 1: Compound 12-0 (500 mg, 2.23 mmol) was dissolved in DMF (5 mL). At 0°C, sodium hydride (107 mg, 4.46 mmol) was added, followed by keeping the temperature for half an hour. Then, 2-(trimethylsilyl)ethoxymethyl chloride (371.8 mg, 2.23 mmol) was added dropwise. The reaction system was slowly warmed to room temperature, followed by reacting for 16 hours. The reaction solution was quenched by slowly adding dropwise water (0.1 mL), followed by adding saturated ammonium chloride solution (10 mL) and extraction with EA (20 mL×2). The organic phases were combined, and washed with saturated saline (20 mL× 3). The organic phases were dried over anhydrous sodium sulfate. After filtering to remove the desiccant, the resulting mixture was purified by silica gel column chromatography with an eluant system (PE: EA=1/1) to afford compound 12-1. MS m/z (ESI): 326.0[M-27].

Step 2: Compound 12-1 (300 mg, 0.85 mmol) was dissolved in 1,4-dioxane (5 mL). Potassium carbonate (352 mg, 2.55 mmol), 2-(ethoxycarbonyl)vinyl acetic acid dimethylbutanediol ester (288 mg, 1.275 mmol), dichloro di-tert-butyl-(4-dimethylaminophenyl)phosphine palladium (II) (60 mg, 0.085 mmol) and water (0.5 mL) were added. The reaction system was heated to 90°C under nitrogen protection and reacted for 12 hours. The reaction solution was concentrated under reduced pressure and purified by silica gel column chromatography with an eluant system (PE: EA=1/1) to afford compound 12-2. MS m/z (ESI): 376.2[M+1].

Step 3: Compound 12-2 (250 mg, 0.67 mmol) was dissolved in methanol (10 mL). Pd/C (10%, 50 mg) was added. The reaction was carried out at room temperature for 16 hours under a hydrogen atmosphere. The reaction solution was filtered through celite to remove Pd/C, and concentrated under reduced pressure to afford compound 12-3. MS m/z (ESI): 376.2[M+1].

Step 4: Compound 12-3 (250 mg, 0.67 mmol) was dissolved in ethanol (5 mL). LiOH (64 mg, 2.68 mmol) and water (5 mL) were added. The reaction was carried out at room temperature for 16 hours. After the reaction solution was concentrated under reduced pressure to remove methanol, 1M hydrochloric acid aqueous solution was added for adjusting pH to 5, followed by extraction with EA (20 mL×2). The organic phases were combined. The organic phases were dried over anhydrous sodium sulfate. After filtering to remove the desiccant, the filtrate was concentrated under reduced pressure to afford compound 12-4. MS m/z (ESI): 348.3[M+1].

Step 5: Compound 12-4 (80 mg, 0.23 mmol) was dissolved in 1 mL acetonitrile, and N-methylimidazole (0.06 mL, 0.69 mmol) and TCFH (64 mg, 0.23 mmol) were added sequentially. The reaction solution was reacted at room temperature for 0.5 hours. 4-[5-(trifluoromethyl)pyrimidin-2-yl]aniline (55 mg, 0.23 mmol) was added. The reaction solution was reacted at room temperature for 16 hours. The reaction solution was concentrated under reduced pressure and purified by silica gel column chromatography with an eluant system (DCM:MeOH=30/1) to afford compound 12-5. MS m/z (ESI): 569.4[M+1].

Step 6: Compound 12-5 (35 mg, 0.06 mmol) was dissolved in a solution of 4M hydrochloric acid in 1,4-dioxane (1 mL). The reaction was carried out at room temperature for 16 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 12. MS m/z (ESI): 439.1[M+1]. ¹H NMR (400 MHz, DMSO) δ 11.13 (s, 1H), 10.26 (s, 1H), 9.28 (s, 2H), 8.45 - 8.37 (m, 2H), 8.28 - 8.21 (m, 1H), 7.85 - 7.74 (m, 4H), 7.53 (ddd, J = 8.1, 6.5, 1.7 Hz, 1H), 7.02 (s, 1H), 3.02 (t, J = 7.5 Hz, 2H), 2.70 (t, J = 7.5 Hz, 2H).

### Example 13

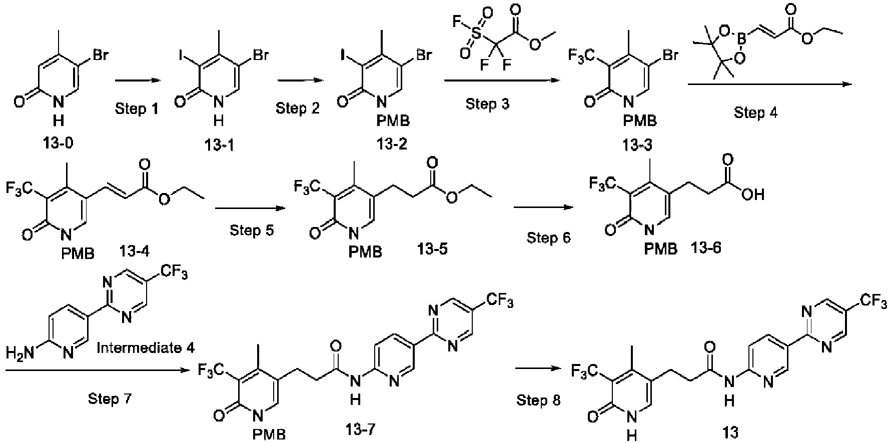

Step 1: Compound 13-0 (5 g, 26.59 mmol) was dissolved in acetonitrile (50 mL) and N-iodosuccinimide (17.95 g, 79.78 mmol) was added. The reaction was carried out at room temperature for 18 hours. The reaction solution was poured into water (50 mL), and EA (50 mL) was added. The mixture was filtered. The filter cake was washed with EA (20 mL). The filter cake was oven-dried to afford compound 13-1. MS m/z (ESI):313.8/315.8[M+1].

Step 2: Compound 13-1 (6.225 g, 19.83 mmol) and potassium carbonate (13.7 g, 99.15 mmol) were dissolved in DMF (50 mL). 4-methoxybenzylchloride (4.05 mL, 29.74 mmol) was added. The reaction was carried out at 50°C for 18 hours. The reaction solution was poured into ice water (100 mL), followed by extraction with EA (100 mL × 3). The combined organic phase was washed with brine (100 mL), and dried over anhydrous sodium sulfate. The filtrate was concentrated under reduced pressure to afford compound 13-2. MS m/z (ESI):433.9/435.9[M+1].

Step 3: Compound 13-2 (7.1 g, 16.39 mmol) and methyl fluorosulfonyldifluoroacetate (4.16 mL, 32.78 mmol) were dissolved in DMF (100 mL). Cuprous iodide (0.61 g, 3.28 mmol) was added. The reaction was carried out at 100°C for 18 hours. The reaction solution was poured into ice water (200 mL), followed by extraction with EA (100 mL × 3). The combined organic phase was washed with brine (100 mL), and dried over anhydrous sodium sulfate. The filtrate was concentrated under reduced pressure to afford the crude. The crude was purified by silica gel chromatography (elution liquid: PE: EtOAc = 2: 1 to 1: 1) to afford compound 13-3. MS m/z (ESI):375.2/377.2[M+1].

Step 4: Compound 13-3 (1.00 g, 2.66 mmol), 2-(ethoxycarbonyl)vinyl acetic acid dimethylbutanediol ester (1.20 g, 5.32 mmol, Bide), and potassium carbonate (1.10 g, 7.98 mmol) were added to a solution of 1, 4-dioxane (10 mL) and water (1 mL). Bis[5-(diphenylphosphino)cyclopentyl-1,3-dienyl]-λ2-iron (II) dichloromethane palladium chloride (97.26 mg, 0.13 mmol) was added. The mixture was purged with nitrogen 3 times, and the reaction was carried out at 100°C for 18 hours. The resulting mixture was concentrated under reduced pressure and purified by silica gel chromatography (elution solution: PE: EA = 10:1 to 2:1) to afford compound 13-4. MS m/z (ESI): 396.2[M+1].

Step 5: Compound 13-4 (715 mg, 1.81 mmol) was dissolved in methanol (10 mL). Palladium on carbon (75 mg, wt10%) was added. The mixture was purged with hydrogen 3 times. The reaction was carried out at room temperature for 18 hours. After the reaction was completed, the reaction solution was filtered and rinsed with methanol (10 mL×2). The filtrate was concentrated under reduced pressure to afford compound 13-5. MS m/z (ESI): 398.2.1[M+1].

Step 6: Compound 13-5 (680 mg, 1.71 mmol) was dissolved in methanol (5 mL) and water (5 mL). Then, LiOH (123 mg, 5.13 mmol) was added. The reaction was carried out at room temperature for 18 hours. The reaction solution was concentrated under reduced pressure. The residue was added with water (10 mL). The pH value was adjusted to 6 with 1M hydrochloric acid aqueous solution, followed by filtering, and washing with water (5 mL). The filter cake was oven-dried to afford compound 13-6. MS m/z (ESI):370.2[M+1].

Step 7: Compound 13-6 (123 mg, 0.33 mmol) was dissolved in acetonitrile (20 mL). Then, TCFH (140 mg, 0.5 mmol) and N-methylimidazole (79.66 µL, 1 mmol) were added sequentially. After stirring at room temperature for half an hour, intermediate 4 (80 mg, 0.33 mmol) was added. Under a nitrogen atmosphere, the reaction was carried out at room temperature for 16 hours. The reaction solution was concentrated under reduced pressure and purified by silica gel column chromatography with an eluant system (PE: EA=2:1 to 1: 1) to afford compound 13-7.

Step 8: Compound 13-7 (160 mg, 0.27 mmol) was dissolved in TFA (5 mL). TfOH (25 µL, 0.28 mmol) was added, and the reaction was carried out at 100°C for 18 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 13. MS m/z (ESI): 472.1[M+1]. ¹H NMR (400 MHz, DMSO) δ 11.98 (s, 1H), 10.92 (s, 1H), 9.34 (s, 2H), 9.32 (s, 1H), 8.73 (d, *J* = 9.8 Hz, 1H), 8.29 (d, *J =* 8.8 Hz, 1H), 7.41 (s, 1H), 2.84 - 2.72 (m, 2H), 2.70 - 2.61 (m, 2H), 2.32 (s, 3H).

### Example 14

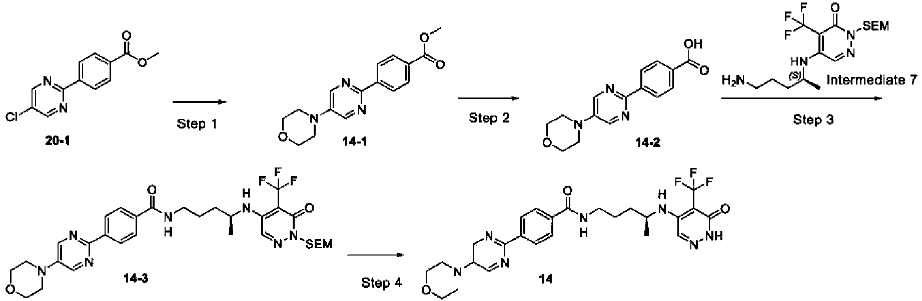

Step 1: Compound 20-1 (300 mg, 1.21 mmol) was dissolved in 5 mL anhydrous 1,4-dioxane. Morpholine (0.21 mL, 2.41 mmol), cesium carbonate (1.18 g, 3.62 mmol), 4,5-bis(diphenylphosphino)-9,9-dimethylxanthene (139 mg, 0.24 mmol), and tris(dibezylideneacetone)dipalladium (97 mg, 0.12 mmol) were added sequentially. The reaction was carried out at 100°C for 16 hours under nitrogen atmosphere protection. The reaction solution was concentrated under reduced pressure and purified by silica gel column chromatography with an eluant system (PE:EA=2/1) to afford compound 14-1. MS m/z (ESI): 300.1[M+1].

Step 2: Compound 14-1200 mg, 0.67 mmol) was dissolved in methanol (2 mL), and LiOH (64 mg, 2.6 mmol) and water (1 mL) were added. The reaction was carried out at 50°C for 3 hours. After the reaction solution was concentrated under reduced pressure to remove methanol, 1M hydrochloric acid aqueous solution was added for adjusting pH to 5, followed by extraction with EA (10 mL×2). The organic phases were combined. The organic phases were dried over anhydrous sodium sulfate. After filtering to remove the desiccant, the filtrate was concentrated under reduced pressure to afford compound 14-2. MS m/z (ESI): 286.1 [M+1].

Step 3: Compound 14-2 (120 mg, 0.42 mmol) was dissolved in 2 mL DMF, and DIPEA (163 mg, 1.26 mmol) and HATU (240 mg, 0.63 mmol) were added sequentially. The reaction solution was reacted at room temperature for 0.5 hours. Intermediate 7 (166 mg, 0.42 mmol) was added. The reaction solution was reacted at room temperature for 16 hours. The reaction solution was added with 10 mL water, followed by extraction with EA (5 mL×2). The organic phases were combined, and washed with saturated saline (20 mL× 3). The organic phases were dried over anhydrous sodium sulfate. After filtering to remove the desiccant, the filtrate was concentrated under reduced pressure to afford compound 14-3. MS m/z (ESI): 662.3[M+1].

Step 4: Compound 14-3 (100 mg) was dissolved in a solution of 4M hydrochloric acid in 1,4-dioxane (1 mL). The reaction was carried out at room temperature for 16 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 14. MS m/z (ESI): 532.2[M+1]. ¹H NMR (400 MHz, DMSO) δ 12.44 (s, 1H), 8.62 (s, 2H), 8.53 (t, J = 5.7 Hz, 1H), 8.33 (d, J = 8.2 Hz, 2H), 7.96 - 7.82 (m, 3H), 6.34 (s, 1H), 3.99 (s, 1H), 3.78 (t, J = 4.9 Hz, 4H), 3.32 - 3.25 (m, 6H), 1.55 (d, J = 6.4 Hz, 3H), 1.18 (dd, J = 10.6, 6.3 Hz, 3H).

### Example 15

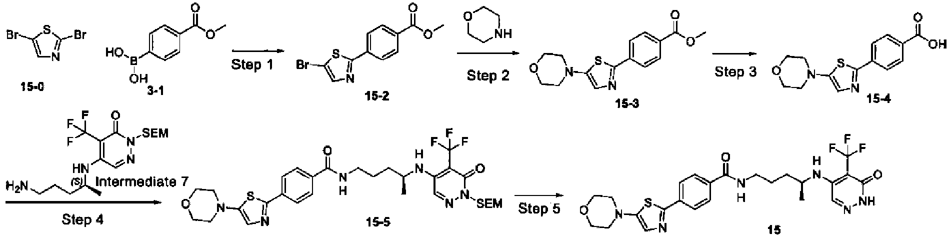

Step 1: Compound 15-0 (3.37 g, 13.89 mmol, Bide), compound 3-1 (5 g, 27.78 mmol) were dissolved in 12 mL anhydrous THF. Potassium phosphate (8.85 g, 41.67 mmol), 4,5-bis(diphenylphosphino)-9,9-dimethylxanthene (0.69 g, 0.40 mmol), and palladium acetate (0.16 mg, 0.40 mmol) were added sequentially. The reaction solution was reacted under stirring at 60°C for 24 hours under a nitrogen condition. The reaction solution was concentrated. The residue was purified by silica gel column chromatography with an eluant system (PE/EA=4/1) to afford compound 15-2. MS m/z (ESI): 297.9[M+H].

Step 2: Compound 15-2 (283 mg, 0.95 mmol) and morpholine (414 mg, 4.76 mmol) were dissolved in anhydrous 1,4-dioxane (15 mL). Cesium carbonate (642 mg, 1.97 mmol), 1,1'-binaphthyl-2,2'-bis(diphenylphosphine) (61 mg, 0.10 mmol), and palladium acetate (11 mg, 0.05 mmol) were added sequentially, followed by reacting under stirring at 100°C for 12 hours under a nitrogen condition. The reaction solution was cooled to room temperature, and concentrated under reduced pressure. The residue was purified by silica gel column chromatography with an eluant system (PE/EA=5/1) to afford compound 15-3. MS m/z (ESI): 305.0[M+H].

Step 3: Compound 15-3 (64 mg, 0.21 mmol) was dissolved in a mixed solvent of 2 mL methanol and 1 mL water. LiOH (18 mg, 0.42 mmol) was added, followed by reacting under stirring at 25°C for 16 hours. The reaction solution was diluted by adding water (5 mL), followed by concentration under reduced pressure to remove methanol. 0.1M hydrochloric acid solution was added dropwise. The solution was adjusted to pH<7, followed by extraction with DCM/IPA (3/1) (3 mL × 2). The organic phases were washed with saturated saline (10 mL× 2), dried over anhydrous sodium sulfate, filtered and concentrated to afford compound 15-4. MS m/z (ESI): 291.0[M+H].

Step 4: Compound 15-4 (67 mg, 0.23 mmol) was dissolved in 3 mL DMF, and DIPEA (0.11 mL, 0.69 mmol) and HATU (132 mg, 0.35 mmol) were added sequentially. The reaction solution was reacted at room temperature for 0.5 hours. Intermediate 7 (91 mg, 0.23 mmol) was added. The reaction solution was reacted at room temperature for 2 hours. The reaction solution was added with 10 mL water, followed by extraction with EA (6 mL×2). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE/EA=4/1) to afford compound 15-5. MS m/z (ESI): 667.2[M+H⁺].

Step 5: Compound 15-5 (70 mg, 0.10 mmol) was dissolved in 5 mL DCM, and 1 mL TFA was added, followed by reacting under stirring at 25°C for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 15. MS m/z (ESI): 537.2[M+H]. ¹H NMR (400 MHz, DMSO) δ 12.44 (s, 1H), 8.52 (d, J = 5.9 Hz, 1H), 7.95 - 7.78 (m, 5H), 7.19 (s, 1H), 6.34 (s, 1H), 3.98 (s, 1H), 3.75 (dd, J = 6.0, 3.8 Hz, 4H), 1.65 (d, J = 7.8 Hz, 0H), 1.54 (s, 4H), 1.17 (dd, J = 12.5, 6.3 Hz, 4H).

### Example 16

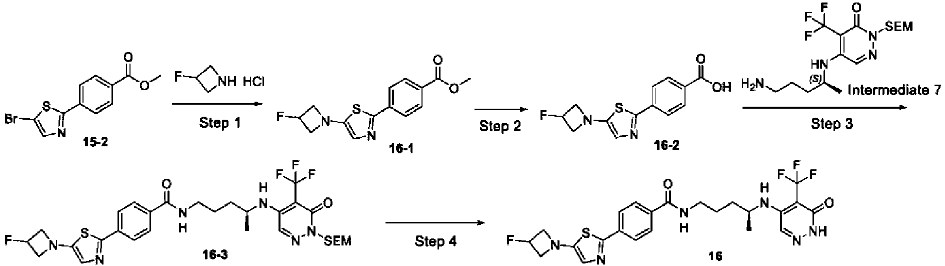

Step 1: Compound 15-2 (265 mg, 0.89 mmol) and 3-fluoroazetidine hydrochloride (2000 mg, 11.11 mmol) were dissolved in 6 mL anhydrous 1,4-dioxane. Cesium carbonate (869 mg, 2.67 mmol), 4,5-bis(diphenylphosphino)-9,9-dimethylxanthene (103 mg, 0.18 mmol), and tris(dibezylideneacetone)dipalladium (81 mg, 0.09 mmol) were added sequentially. The reaction solution was reacted under stirring at 100°C for 16 hours under a nitrogen condition. The reaction solution was concentrated under reduced pressure. The residue was purified by silica gel column chromatography with an eluant system (PE/EA=2/1) to afford compound 16-1. MS m/z (ESI): 293.0[M+H].

Step 2: Compound 16-1 (150 mg, 0.49 mmol) was dissolved in a mixed solvent of 5 mL methanol and 2.5 mL water. LiOH (41 mg, 0.98 mmol) was added, followed by reacting under stirring at 25°C for 16 hours. The reaction solution was diluted by adding water (10 mL), followed by concentration under reduced pressure to remove methanol. 0.1M hydrochloric acid solution was added dropwise. The solution was adjusted to pH<7, followed by extraction with DCM/isopropanol (3/1) (8 mL × 2). The organic phases were washed with saturated saline (20 mL× 2), dried over anhydrous sodium sulfate, filtered and concentrated to afford compound 16-2. MS m/z (ESI): 279.0[M+H].

Step 3: Compound 16-2 (130 mg, 0.47 mmol) was dissolved in 4 mL DMF, and DIPEA (181 mg, 1.40 mmol) and HATU (266 mg, 0.70 mmol) were added sequentially. The reaction solution was reacted at room temperature for 0.5 hours. Intermediate 7 (203 mg, 0.51 mmol) was added. The reaction solution was reacted at room temperature for 16 hours. The reaction solution was added with 10 mL water, followed by extraction with EA (5 mL×2). The organic phases were combined, and washed with saturated saline (20 mL× 3). The organic phases were dried over anhydrous sodium sulfate. After filtering to remove the desiccant, the filtrate was concentrated under reduced pressure. The residue was purified by silica gel plate chromatography with an eluant system (PE/EA=1/1) to afford compound 16-3. MS m/z (ESI): 655.2[M+H⁺].

Step 4: Compound 16-3 (105 mg, 0.16 mmol) was dissolved in 2.5 mL DCM, and 0.5 mL TFA was added, followed by reacting under stirring at 25°C for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10mmol/L TFA) and acetonitrile, gradient ratio: acetonitrile 30%-60%, flow rate: 30 mL/min) to afford compound 16. MS m/z (ESI): 525.1[M+H]. ¹H NMR (400 MHz, DMSO) δ 12.44 (s, 1H), 8.52 (t, J = 5.4 Hz, 1H), 7.94 - 7.78 (m, 5H), 6.96 (s, 1H), 6.33 (s, 1H), 4.25 (ddd, J = 21.0, 9.4, 5.7 Hz, 2H), 4.05 (dd, J = 24.8, 8.6 Hz, 2H), 3.99 - 3.90 (m, 1H), 3.27 (s, 2H), 1.54 (s, 4H), 1.17 (t, J = 6.4 Hz, 3H).

### Example 17

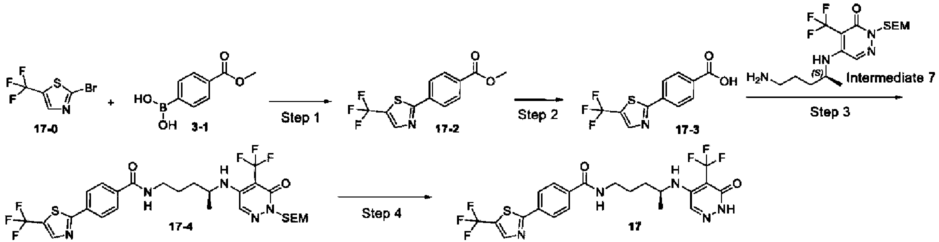

Step 1: Compound 17-0 (230 mg, 0.99 mmol) and compound 3-1 (196 mg, 1.09 mmol) were dissolved in 10 mL anhydrous THF. Potassium phosphate (631 mg, 2.97 mmol), 4,5-bis(diphenylphosphino)-9,9-dimethylxanthene (115 mg, 0.20 mmol), and palladium acetate (22 mg, 0.10 mmol) were added sequentially. The reaction solution was reacted under stirring at 60°C for 16 hours under a nitrogen condition. The reaction solution was concentrated under reduced pressure. The residue was purified by silica gel column chromatography with an eluant system (PE/EA=3/1) to afford compound 17-2. MS m/z (ESI): 288.0[M+H]. 1H NMR (400 MHz, DMSO) δ 8.64 (q, J = 1.3 Hz, 1H), 8.23 - 8.09 (m, 4H), 3.90 (d, J = 1.8 Hz, 3H).

Step 2: Compound 17-2 (215 mg, 0.75 mmol) was dissolved in a mixed solvent of 5 mL methanol and 2.5 mL water. LiOH (63 mg, 1.50 mmol) was added, followed by reacting under stirring at 25°C for 16 hours. The reaction solution was diluted by adding water (10 mL), followed by concentration under reduced pressure to remove methanol. 0.1M hydrochloric acid solution was added dropwise. The solution was adjusted to pH<7, followed by extraction with DCM/isopropanol (3/1) (8 mL × 2). The organic phases were washed with saturated saline (20 mL× 2), dried over anhydrous sodium sulfate, filtered and concentrated to afford compound 17-3. MS m/z (ESI): 274.0[M+H].

Step 3: Compound 17-3 (150 mg, 0.55 mmol) was dissolved in 2.5 mL DMF, and DIPEA (213 mg, 1.65 mmol) and HATU (313 mg, 0.82 mmol)were added sequentially. The reaction solution was reacted at room temperature for 0.5 hours. Intermediate 7 (217 mg, 0.55 mmol) was added. The reaction solution was reacted at room temperature for 16 hours. The reaction solution was added with 10 mL water, followed by extraction with EA (5 mL×2). The organic phases were combined, and washed with saturated saline (20 mL× 3). The organic phases were dried over anhydrous sodium sulfate. After filtering to remove the desiccant, the filtrate was concentrated under reduced pressure to afford compound 17-4. MS m/z (ESI): 650.2[M+H+].

Step 4: Compound 17-4 (346 mg, 0.53 mmol) was dissolved in a 3 mL solution of 4 M hydrochloric acid in 1,4-dioxane, followed by reacting under stirring at 40°C for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 17. MS m/z (ESI): 520.1[M+H]. ¹H NMR (400 MHz, DMSO) δ 12.43 (s, 1H), 8.66 (s, 0H), 8.60 (d, J = 1.4 Hz, 1H), 7.99 (d, J = 8.4 Hz, 1H), 7.93 (s, 1H), 3.98 (s, 1H), 3.29 (d, J = 6.0 Hz, 0H), 1.55 (s, 3H), 1.16 (s, 3H).

### Example 18

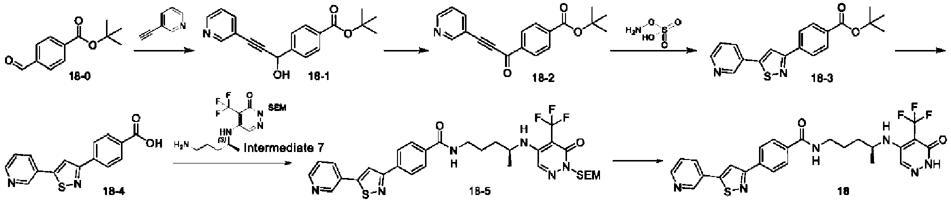

Step 1: Compound 3-ethynylpyridine (2.99 g, 29.02 mmol) was dissolved in 50 mL THF. Then, at -78°C, butyl lithium (2.5N, 11.96 mL, 29.89 mmol) was added dropwise, followed by stirring 2 h. A THF solution (50 mL) of compound 18-0 (4500 mg, 11.59 mmol) was added dropwise, followed by slowly warming to room temperature and reacting under stirring for 2 hours. The reaction solution was added with 10 mL saturated ammonium chloride for concentration, followed by adding EA and extraction with EA. After washing with saturated saline, drying and concentration, the residue was purified by column chromatography (PE/EA=1/1) to afford compound 18-1. MS m/z (ESI):310.2[M+1].

Step 2: Compound 18-1 (6200 mg, 20.0 mmol) and manganese dioxide (26.14 g, 300 mmol) were dissolved in chloroform (20 mL), followed by reacting under stirring at room temperature for 2 hours. The reaction solution was filtered and concentrated to afford compound 18-2. MS m/z (ESI):308.2[M+1].

Step 3: At 0°C, hydroxylamine sulfonic acid (965 mg, 9.76 mmol) was added to compound 18-2 (2500 mg, 8.13 mmol) in 1,4-dioxane (40 mL) and water (20 mL). The reaction was carried out at room temperature for 36 hours. Then, sodium bicarbonate (717 mg, 8.54 mmol) and sodium hydrosulfide (781 mg, 8.54 mmol) were added sequentially. The reaction was carried out at room temperature for 18 hours. The reaction solution was concentrated under reduced pressure, followed by adding EA and extraction with EA. After washing with saturated saline, drying and concentration, the residue was purified by column chromatography (PE/EA=1/2) to afford compound 18-3. MS m/z (ESI):339.2[M+1].

Step 4: Compound 18-3 (1.0 g, 2.95 mmol) was dissolved in TFA-DCM (20%, 20 mL), and the reaction was carried out at room temperature for 2 hours. The reaction solution was concentrated under reduced pressure to afford compound 18-4. MS m/z (ESI):283.0[M+1].

Step 5: Compound 18-4 (100 mg, 0.35 mmol) and intermediate 7 (167 mg, 0.43 mmol), HATU (161 mg, 0.43 mmol), and DIPEA (915 mg, 7.08 mmol) were dissolved in 3mL DMF. The reaction was carried out at room temperature for 2 hours. The reaction solution was concentrated. The residue was purified by silica gel column chromatography with an eluant system (methanol/DCM=1/20) to afford compound 18-5. MS m/z (ESI):659.2[M+1].

Step 6: Compound 18-5 (130 mg, 0.2 mmol) was dissolved in 5 mL of 4M hydrogen chloride in 1,4-dioxane. The reaction was carried out at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 2%-32%, flow rate: 30 mL/min) to afford compound 18. MS m/z (ESI):529.0[M+1]. ¹H NMR (400 MHz, DMSO) δ 12.46 (s, 1H), 9.09 (d, *J=* 1.8 Hz, 1H), 8.68 (dd, *J* = 4.8, 1.5 Hz, 1H), 8.64 - 8.56 (m, 2H), 8.31 - 8.22 (m, 1H), 8.18 (d, *J* = 8.4 Hz, 2H), 8.06 - 7.88 (m, 3H), 7.59 (dd, *J* = 7.9, 4.8 Hz, 1H), 6.37 (d, *J* = 4.6 Hz, 1H), 4.00 (s, 1H), 3.32 - 3.23 (m, 2H), 1.77 - 1.43 (m, 4H), 1.19 (d, *J* = 6.3 Hz, 3H).

### Example 19

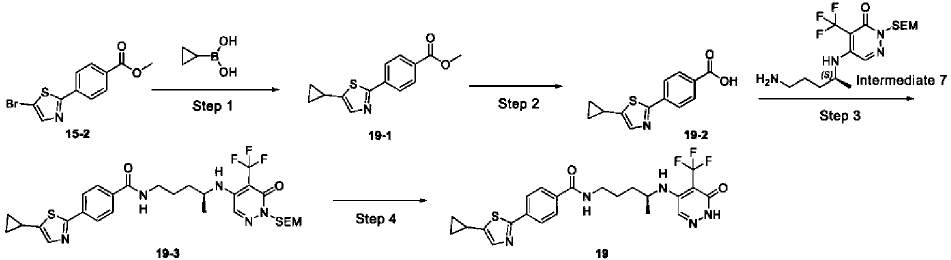

Step 1: Compound 15-2 (300 mg, 1.01 mmol) and cyclopropylboronic acid (173 mg, 2.01 mmol) were dissolved in 6 mL anhydrous 1,4-dioxane. Cesium carbonate (984 mg, 3.02 mmol) and 1,1-bis(diphenylphosphino)ferrocenedichloropalladium (74 mg, 0.10 mmol) were added. The reaction solution was reacted under stirring at 90°C for 16 hours under a nitrogen condition. The reaction solution was concentrated under reduced pressure. The residue was purified by silica gel column chromatography with an eluant system (PE/EA=5/1) to afford compound 19-1. MS m/z (ESI): 260.0[M+H].

Step 2: Compound 19-1 (102 mg, 0.39 mmol) was dissolved in a mixed solvent of 5 mL methanol and 2.5 mL water. LiOH (33 mg, 0.78 mmol) was added, followed by reacting under stirring at 25°C for 16 hours. The reaction solution was diluted by adding water (10 mL), and concentrated under reduced pressure. 0.1M hydrochloric acid solution was added dropwise. The solution was adjusted to pH<7, followed by extraction with DCM/isopropanol (3/1) (8 mL × 2). The organic phases were washed with saturated saline (20 mL× 2), dried over anhydrous sodium sulfate, filtered and concentrated to afford compound 19-2. MS m/z (ESI): 246.0[M+H].

Step 3: Compound 19-2 (97 mg, 0.40 mmol) was dissolved in 4 mL DMF, and DIPEA (155 mg, 1.20 mmol) and HATU (228 mg, 0.60 mmol) were added sequentially. The reaction solution was reacted at room temperature for 0.5 hours. Intermediate 7 (156 mg, 0.40 mmol) was added. The reaction solution was reacted at room temperature for 16 hours. The reaction solution was added with 10 mL water, followed by extraction with EA (5 mL×2). The organic phases were combined, and washed with saturated saline (20 mL× 3). The organic phases were dried over anhydrous sodium sulfate. After filtering to remove the desiccant, the filtrate was concentrated under reduced pressure to afford compound 19-3. MS m/z (ESI): 622.2[M+H⁺].

Step 4: Compound 19-3 (286 mg, 0.46 mmol) was dissolved in a 5 mL solution of 4 M hydrochloric acid in 1,4-dioxane, followed by reacting under stirring at 25°C for 3 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 19. MS m/z (ESI): 492.1[M+H]. ¹H NMR (400 MHz, MeOD) δ 8.90 (d, J = 6.0 Hz, 3H), 8.83 (d, J = 6.0 Hz, 2H), 7.65 (s, 1H), 4.00 (m, 1H), 3.45 (t, J = 6.4 Hz, 2H), 2.20 (m, 1H), 1.70 (m, J = 6.0 Hz, 4H), 1.27 (d, J = 2.4 Hz, 3H), 1.12 (m, 2H), 0.70 (m, 2H).

### Example 20

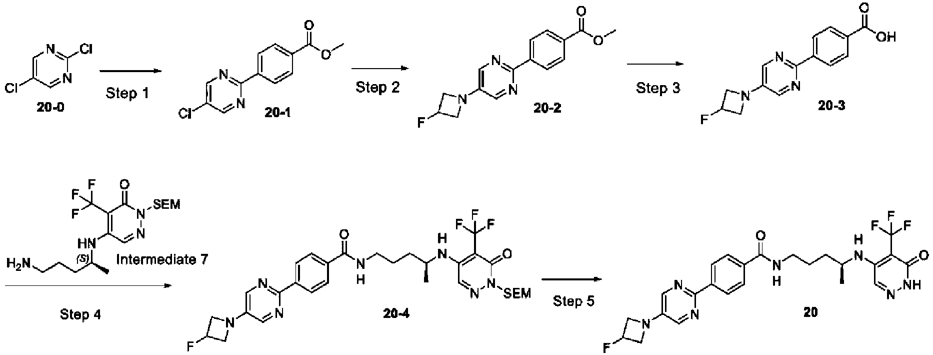

Step 1: Compound 20-0 (2.0 g, 13.43 mmol) was dissolved in 40 mL 1,4-dioxane. Compound 3-1 (2.42 g, 13.43 mmol), potassium carbonate (5.57 g, 40.29 mmol), [1,1'-bis(diphenylphosphino) ferrocene]dichloropalladiumdichloromethane complex (1.1 g, 1.34 mmol), and water (8 mL) were added sequentially. The reaction was carried out at 100°C for 16 hours under nitrogen atmosphere protection. The reaction solution was concentrated under reduced pressure and purified by silica gel column chromatography with an eluant system (PE:EA=10/1->3/1) to afford compound 20-1. MS m/z (ESI): 249.1[M+1].

Step 2: Compound 20-1 (250 mg, 1.01 mmol) was dissolved in 5 mL anhydrous 1,4-dioxane. 3-fluoroazetidine hydrochloride (112 mg, 1.01 mmol), cesium carbonate (982 mg, 3.03 mmol) , 4,5-bis(diphenylphosphino)-9,9-dimethylxanthene (116 mg, 0.2 mmol), and tris(dibezylideneacetone)dipalladium (81 mg, 0.1 mmol) were added sequentially. The reaction was carried out at 100°C for 16 hours under nitrogen atmosphere protection. The reaction solution was concentrated under reduced pressure and purified by silica gel column chromatography with an eluant system (PE:EA=10/1->2/1) to afford compound 20-2. MS m/z (ESI): 288.1[M+1].

Step 3: Compound 20-2 (200 mg, 0.7 mmol) was dissolved in methanol (2 mL), and LiOH (67 mg, 2.8 mmol) and water (1 mL) were added. The reaction was carried out at room temperature for 16 hours. After the reaction solution was concentrated under reduced pressure to remove methanol, 1M hydrochloric acid aqueous solution was added for adjusting pH to 5, followed by extraction with EA (10 mL×2). The organic phases were combined. The organic phases were dried over anhydrous sodium sulfate. After filtering to remove the desiccant, the filtrate was concentrated under reduced pressure to afford compound 20-3. MS m/z (ESI): 274.1[M+1].

Step 4: Compound 20-3 (100 mg, 0.37 mmol) was dissolved in 2 mL DMF, and DIPEA (142 mg, 1.1 mmol) and HATU (208 mg, 0.55 mmol) were added sequentially. The reaction solution was reacted at room temperature for 0.5 hours. Intermediate 7 (144 mg, 0.37 mmol) was added. The reaction solution was reacted at room temperature for 16 hours. The reaction solution was added with 10 mL water, followed by extraction with EA (5 mL×2). The organic phases were combined, and washed with saturated saline (20 mL× 3). The organic phases were dried over anhydrous sodium sulfate. After filtering to remove the desiccant, the filtrate was concentrated under reduced pressure to afford compound 20-4. MS m/z (ESI): 650.3[M+1].

Step 5: Compound 20-4 (100 mg) was dissolved in DCM (2 mL), TFA (1 mL) was added, and the reaction was carried out at room temperature for 16 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 20. MS m/z (ESI): 520.2[M+1]. ¹H NMR (400 MHz, DMSO) δ 12.42 (d, J = 12.4 Hz, 1H), 8.52 (t, J = 5.7 Hz, 1H), 8.30 (d, J = 8.5 Hz, 2H), 8.22 (s, 2H), 7.96 - 7.84 (m, 3H), 6.34 (s, 1H), 5.70 - 5.26 (m, 1H), 4.36 (ddd, J = 21.2, 10.0, 5.8 Hz, 2H), 4.18 - 4.05 (m, 2H), 3.99 (s, 1H), 1.66 (d, J = 8.2 Hz, 1H), 1.55 (s, 3H), 1.20 - 1.13 (m, 3H).

### Example 21

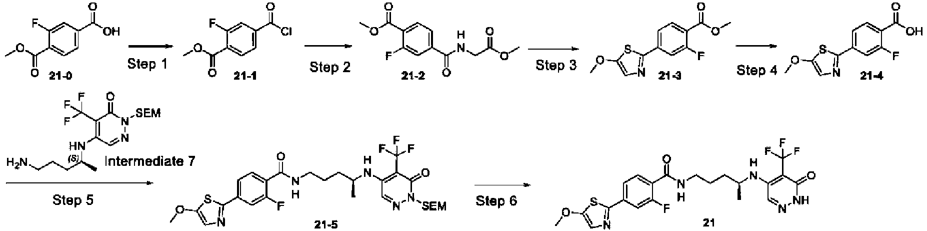

Step 1: Compound 3-fluoro-4-(methoxycarbonyl)benzoic acid (300 mg, 1.51 mmol) was dissolved in sulfoxide chloride (5 mL). The reaction was carried out at 85°C for 3 hours. The reaction solution was concentrated under reduced pressure to afford compound 21-1. MS m/z (ESI): 213.0[M-3].

Step 2: Compound 21-1 (325 mg, 1.50 mmol) was dissolved in DCM (5 mL). At 0°C, methyl glycinate hydrochloride (188 mg, 1.50 mmol) and triethylamine (0.62 mL, 4.5 mmol) were added. The reaction was carried out at 0°C for 2 hours. The reaction solution was added with water (10 mL), followed by extraction with DCM (10 mL×2). The organic phases were combined. The organic phases were dried over anhydrous sodium sulfate. After filtering to remove the desiccant, the filtrate was concentrated under reduced pressure, to afford compound 21-2. MS m/z (ESI): 270.2[M-3].

Step 3: Compound 21-2 (350 mg, 1.30 mmol) was dissolved in chloroform (10 mL). Phosphorus pentasulfide (578 mg, 2.60 mmol) and 4A type molecular sieve (300 mg) were added. The reaction was carried out at 85°C for 16 hours. The reaction solution was filtered to remove the molecular sieve. The filtrate was concentrated under reduced pressure, and purified by silica gel column chromatography with an eluant system (PE:EA=10/1->1/1) to afford compound 21-3. MS m/z (ESI): 268.1[M+1].

Step 4: Compound 21-3 (200 mg, 0.75 mmol) was dissolved in methanol (5 mL), and LiOH (72 mg, 3.0 mmol) and water (5 mL) were added. The reaction was carried out at room temperature for 16 hours. After the reaction solution was concentrated under reduced pressure to remove methanol, 1M hydrochloric acid aqueous solution was added for adjusting pH to 5, followed by extraction with EA (10 mL×2). The organic phases were combined. The organic phases were dried over anhydrous sodium sulfate. After filtering to remove the desiccant, the filtrate was concentrated under reduced pressure to afford compound 21-4. MS m/z (ESI): 254.0[M+1].

Step 5: Compound 21-4 (75 mg, 0.30 mmol) was dissolved in 2 mL DMF, and DIPEA (116 mg, 0.90 mmol) and HATU (171 mg, 0.45 mmol) were added sequentially. The reaction solution was reacted at room temperature for 0.5 hours. Intermediate 7 (117 mg, 0.30 mmol) was added. The reaction solution was reacted at room temperature for 16 hours. The reaction solution was added with 10 mL water, followed by extraction with EA (5 mL×2). The organic phases were combined, and washed with saturated saline (20 mL× 3). The organic phases were dried over anhydrous sodium sulfate. After filtering to remove the desiccant, the filtrate was concentrated under reduced pressure to afford compound 21-5 (150 mg). MS m/z (ESI): 630.2[M+1].

Step 6: Compound 21-5 (150 mg) was dissolved in a solution of 4M hydrochloric acid in 1,4-dioxane (2 mL). The reaction was carried out at room temperature for 16 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 21. MS m/z (ESI): 500.1[M+1]. ¹H NMR (400 MHz, DMSO) δ 8.40 (s, 1H), 7.93 (s, 1H), 7.66 (q, J = 3.4 Hz, 3H), 7.40 (s, 1H), 6.35 (s, 1H), 3.99 (s, 4H), 3.25 (d, J = 6.0 Hz, 2H), 1.67 (s, 1H), 1.53 (s, 3H), 1.19 (d, J = 6.3 Hz, 3H).

### Example 22

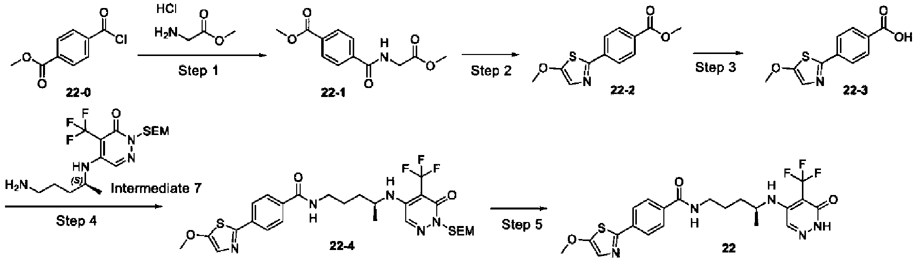

Step 1: Compound 22-0 (5 g, 25.18 mmol, Bide) was dissolved in 80 mL anhydrous DCM. Triethylamine (10.5 mL, 75.53 mmol) was added. The reaction solution was maintained at 0°C-10°C under a nitrogen condition. Methyl glycinate hydrochloride (3.36 g, 37.76 mmol) was dissolved in 10 mL anhydrous DCM, and added dropwise. The reaction solution was reacted under stirring at 25°C for 2 hours. The reaction solution was washed with water (20 mL× 2), dried over anhydrous sodium sulfate, and filtered. The filtrate was concentrated under reduced pressure to afford compound 22-1. MS m/z (ESI): 252.0[M+H].

Step 2: Compound 22-1 (2 g, 7.96 mmol) was dissolved in chloroform (18 mL). Phosphorus pentasulfide (3.54 g, 15.92 mmol) and 4A molecular sieve (4 g) were added. The reaction solution was purged with nitrogen and reacted under stirring at 60°C for 6 hours under a sealed condition. The reaction solution was cooled to room temperature, and filtered through celite. The filter cake was rinsed with DCM (20 mL× 2), and concentrated under reduced pressure to afford compound 22-2 (2.1 g). MS m/z (ESI): 250.0[M+H].

Step 3: Compound 022-2 (150 mg, 0.60 mmol) was dissolved in a mixed solvent of 5 mL methanol and 2.5 mL water. LiOH (51 mg, 1.2 mmol) was added, followed by reacting under stirring at 25°C for 16 hours. The reaction solution was diluted by adding water (8 mL), followed by concentration under reduced pressure to remove methanol. 0.1M hydrochloric acid solution was added dropwise. The solution was adjusted to pH<7, followed by extraction with DCM/isopropanol (3/1) (6 mL × 2). The organic phases were washed with saturated saline (15 mL× 2), dried over anhydrous sodium sulfate, filtered and concentrated to afford compound 22-3. MS m/z (ESI): 236.0[M+H].

Step 4: Compound 22-3 (73 mg, 0.31 mmol) was dissolved in 3 mL DMF, and DIPEA (120 mg, 0.93 mmol) and HATU (132 mg, 0.47 mmol) were added sequentially. The reaction solution was reacted at room temperature for 0.5 hours. Intermediate 7 (122 mg, 0.31 mmol) was added. The reaction solution was reacted at room temperature for 3 hours. The reaction solution was added with 10 mL water, followed by extraction with EA (6 mL×2). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE/EA=2/1) to afford compound 22-4. MS m/z (ESI): 612.2[M+H⁺].

Step 5: Compound 22-4 (152 mg, 0.25 mmol) was dissolved in a 5 mL solution of 4 M hydrochloric acid in 1,4-dioxane, followed by reacting under stirring at 45°C for 3 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 22. MS m/z (ESI): 482.1[M+H]. ¹H NMR (400 MHz, DMSO) δ 12.46 (s, 1H), 8.58 (t, J = 5.7 Hz, 1H), 7.96 - 7.82 (m, 5H), 7.37 (s, 1H), 6.36 (d, J = 7.9 Hz, 1H), 3.98 (s, 3H), 3.26 (d, J = 6.7 Hz, 2H), 1.65 (d, J = 8.2 Hz, 0H), 1.54 (s, 3H), 1.18 (d, J = 6.3 Hz, 3H).

### Example 23

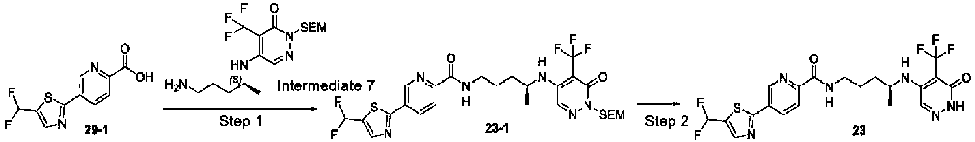

Step 1: Compound 29-1 (70 mg, 0.28 mmol) was dissolved in 3 mL DMF, and DIPEA (106 mg, 0.82 mmol) and HATU (156 mg, 0.41 mmol) were added sequentially. The reaction solution was reacted at room temperature for 0.5 hours. Intermediate 7 (108 mg, 0.28 mmol) was added. The reaction solution was reacted at room temperature for 2 hours. The reaction solution was added with 10 mL water, followed by extraction with EA (5 mL×2). The organic phases were combined, and washed with saturated saline (20 mL× 3). The organic phases were dried over anhydrous sodium sulfate. After filtering to remove the desiccant, the filtrate was concentrated under reduced pressure. The residue was purified by thin layer chromatography with a developing agent system (DCM/methanol=20/1) to afford compound 23-1. MS m/z (ESI): 633.2[M+H⁺].

Step 2: Compound 23-1 (30 mg, 0.05 mmol) was dissolved in a 2 mL solution of 4 M hydrochloric acid in 1,4-dioxane, followed by reacting under stirring at 45°C for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 23. MS m/z (ESI): 503.1[M+H]. ¹H NMR (400 MHz, DMSO) δ 9.21 (dd, J = 2.2, 0.8 Hz, 1H), 8.98 (t, J = 6.1 Hz, 1H), 8.55 (dd, J = 8.2, 2.3 Hz, 1H), 8.39 (t, J = 2.3 Hz, 1H), 8.16 (dd, J = 8.2, 0.9 Hz, 1H), 7.92 (s, 1H), 7.53 (t, J = 54.4 Hz, 1H), 6.33 (dd, J = 8.9, 3.7 Hz, 1H), 3.99 (s, 1H), 1.71 - 1.49 (m, 5H), 1.18 (d, J = 6.3 Hz, 4H).

### Example 24

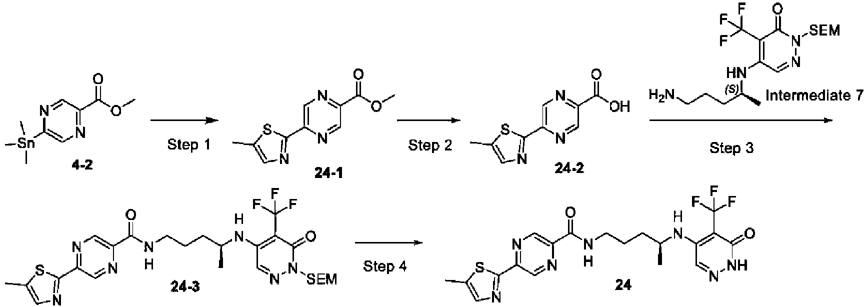

Step 1: Compound 4-2 (500 mg, 1.66 mmol), 2-bromo-5- methylthiazole (325 mg, 1.83 mmol), and PdCl₂(PPh₃)₂ were dissolved in 10 mL 1,4-dioxane, followed by stirring at 120°C for 24 hours under nitrogen protection. The filtrate was concentrated under reduced pressure. The residue was purified by silica gel column chromatography with an eluant system (PE/EA=2/1) to afford compound 24-1. MS m/z (ESI):236.0[M+1].

Step 2: Compound 24-1 (770 mg, 3.27 mmol) was dissolved in THF (3 mL). Methanol (3 mL), water (1 mL), and LiOH (391 mg, 16.36 mmol) were added, followed by reacting under stirring at room temperature for 3 hours. The reaction solution was filtered and concentrated under reduced pressure to afford compound 24-2. MS m/z (ESI):222.0[M+1].

Step 3: Compound 24-2 (50 mg, 0.23 mmol), compound intermediate 7 (89.16 mg, 0.23 mmol), HATU (85.94 mg, 0.23 mmol), and DIPEA (29.21 mg, 0.23 mmol) were dissolved in 1 mL DMF solution. The reaction was carried out at room temperature for 12 hours. The reaction solution was added with water, followed by extraction with EA. The organic phases were dried over anhydrous sodium sulfate. The filtrate was concentrated under reduced pressure and purified by silica gel column chromatography with an eluant system (DCM/methanol=20/1) to afford compound 24-3. MS m/z (ESI):598.2[M+1].

Step 4: Compound 24-3 (60 mg, 0.1 mmol) was dissolved in methanol (1 mL), and a 3 mL solution of 4M hydrochloric acid in 1,4-dioxane was added. The reaction was carried out at room temperature for 16 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 24. MS m/z (ESI):468.1[M+1]. ¹H NMR (400 MHz, DMSO) δ 12.43 (s, 1H), 9.27 (d, J = 1.4 Hz, 1H), 9.16 (d, J = 1.4 Hz, 1H), 9.07 (t, J = 6.0 Hz, 1H), 7.91 (d, J = 9.2 Hz, 1H), 7.85 (d, J = 1.1 Hz, 1H), 6.46 - 6.16 (m, 1H), 3.99 (s, 1H), 3.31 (s, 2H), 2.56 (s, 3H), 1.69 - 1.49 (m, 4H), 1.17 (d, J = 6.3 Hz, 3H).

### Example 25

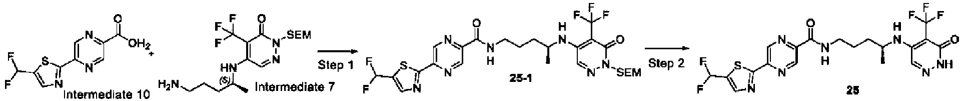

Step 1: Intermediate 10 (50 mg, 0.19 mmol) and intermediate 7 (84.36 mg, 0.21 mmol) were dissolved in DMF (1mL), and then HATU (88.70 mg, 0.23 mmol) and DIPEA (0.13 mL, 0.78 mmol) were added. The reaction was carried out at room temperature for 12 hours. After the reaction was completed, 5mL water was added for dilution, followed by extraction with 10mL EA and washing with saturated saline. The organic phases were dried over anhydrous sodium sulfate. After filtering to remove the desiccant, the filtrate was concentrated under reduced pressure. The residue was purified by preparative silica gel plate with an eluant system (DCM/methanol=20/1) to afford compound 25-1. MS m/z (ESI): 634.2[M+1].

Step 2: Compound 25-1 (53 mg, 0.08 mmol) was dissolved in 0.5 mL hydrochloric acid-1,4-dioxane solution. The reaction solution was reacted at room temperature for 2 hours. The reaction solution was concentrated under reduced pressure. The residue was purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 35%-50%, flow rate: 30 mL/min) to afford compound 25. MS m/z (ESI):504.2[M+1]. ¹H NMR (400 MHz, DMSO) δ 12.44 (s, 1H), 9.36 (d, J = 1.3 Hz, 1H), 9.23 (d, J = 1.4 Hz, 1H), 9.15 (t, J = 6.0 Hz, 1H), 8.47 (s, 1H), 7.93 (s, 1H), 7.56 (dd, J = 72.1, 36.8 Hz, 1H), 6.35 (s, 1H), 3.98 (d, J = 9.7 Hz, 1H), 3.32 (s, 2H), 1.60 (dt, J = 23.6, 12.4 Hz, 4H), 1.18 (t, J = 7.1 Hz, 3H).

### Example 26

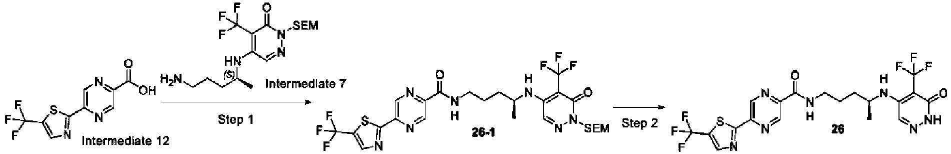

Step 1: Intermediate 12 (45 mg, 0.16 mmol) and intermediate 7 (77 mg, 0.20 mmol), HATU (74 mg, 0.2 mmol), and DIPEA (445 mg, 3.27 mmol) were dissolved in 3 mL DMF. The reaction was carried out at room temperature for 2 hours. The reaction solution was concentrated. The residue was purified by silica gel column chromatography with an eluant system (methanol/DCM=1/20) to afford compound 26-1. MS m/z (ESI):675.2[M+1].

Step 2: Compound 26-1 (40 mg, 0.06 mmol) was dissolved in 5 mL of 4M hydrogen chloride in 1,4-dioxane. The reaction was carried out at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 2%-32%, flow rate: 30 mL/min) to afford compound 26. MS m/z (ESI):522.2[M+1]. ¹H NMR (400 MHz, DMSO) δ 12.43 (s, 1H), 9.38 (d, *J =* 1.3 Hz, 1H), 9.25 (d, *J =* 1.4 Hz, 1H), 9.17 (t, *J =* 6.0 Hz, 1H), 8.78 (d, *J* = 1.0 Hz, 1H), 7.92 (s, 1H), 6.35 (s, 1H), 3.99 (s, 1H), 3.32 (s, 3H),1.60 (dt, *J =* 24.2, 12.6 Hz, 4H), 1.17 (d, *J* = 6.3 Hz, 3H).

### Example 27

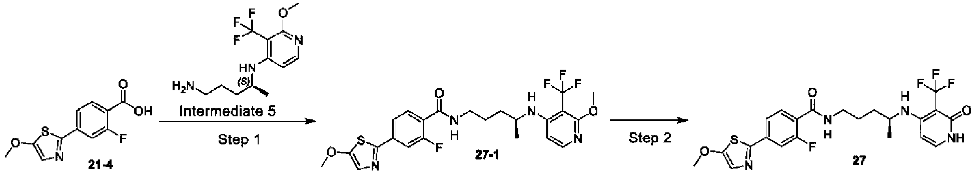

Step 1: At room temperature, intermediate 5 (0.05 g, 0.19 mmol) and compound 21-4 (0.047 g, 0.19 mmol) were dissolved in dimethylformamide (1 mL). HATU (0.105 g, 0.28 mmol) and DIPEA (0.09 mL, 0.56 mmol) were added to the reaction solution. After reacting at room temperature for 18 hours, the reaction was completed. After the reaction was completed, water (3 mL) and EA (3 mL) were added to the reaction solution. The organic phases were washed with saturated saline (3 mL×3), and dried over sodium sulfate. After filtering, the filtrate was concentrated under reduced pressure to afford the crude. The crude was purified by silica gel column (eluant: anhydrous methanol: DCM = 45%-55%) to afford compound 27-1. MS m/z (ESI): 513.1 [M+1].

Step 2: At room temperature, compound 27-1 (40 mg, 0.08 mmol) was dissolved in NMP (1 mL). Then, iodotrimethylsilane (31 mg, 0.16 mmol) was added, followed by stirring at 85°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 27%-95%, flow rate: 20 mL/min), to afford compound 27.MS m/z (ESI): 499.1 [M+1]. ¹H NMR (400 MHz, DMSO-d6) δ 10.88 (s, 1H), 8.45 - 8.33 (m, 1H), 7.68 - 7.63 (m, 3H), 7.40 (s, 1H), 7.31 - 7.19 (m, 1H), 5.95 - 5.91 (m, 1H), 5.82 - 5.75 (m, 1H), 3.99 (s, 3H), 3.77 - 3.65 (m, 1H), 3.26 - 3.21 (m, 2H), 1.65 - 1.48 (m, 4H), 1.15 (d, *J* = 6.2 Hz, 3H).

### Example 28

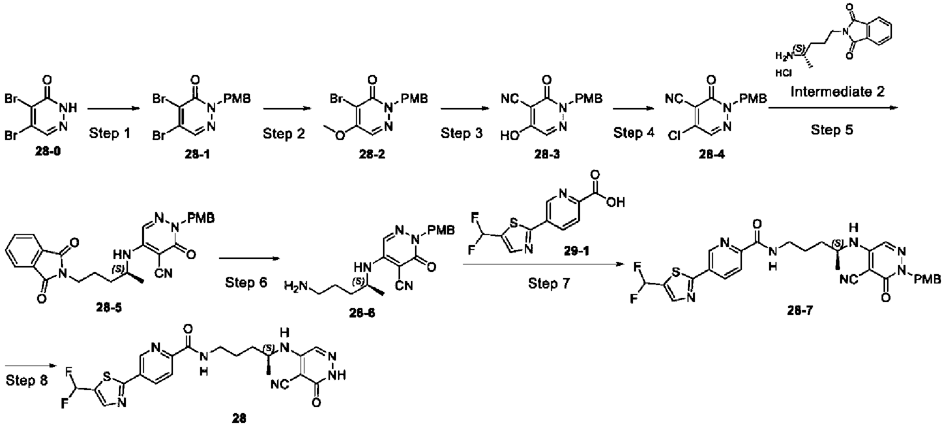

Step 1: Compound 28-0 (15 g, 59.08 mmol, Bide), potassium carbonate (12.25 g, 88.62 mmol), and 4-methoxybenzylchloride (10.46 mL, 76.81 mmol, Titan) were dissolved in acetonitrile (150 mL). The reaction was carried out at 80°C for 16 hours. The reaction solution was filtered through celite and the filtrate was concentrated under reduced pressure. The residue was slurried for 1 hour by adding 30 mL methanol, filtered, and washed with methanol (10 mL×2). The resulting solid was blown dry under nitrogen at normal temperature to afford compound 28-1. MS m/z (ESI):374.9[M+1]. ¹H NMR (400 MHz, CDCl3) δ 7.78 (s, 1H), 7.40 (d, J = 8.7 Hz, 2H), 6.88 - 6.82 (m, 2H), 5.25 (s, 2H), 3.78 (s, 3H).

Step 2: Compound 28-1 (6 g, 16.04 mmol) was dissolved in methanol (60 mL) and then potassium hydroxide (3.60 g, 64.17 mmol) was added. The reaction was carried out at 20°C for 16 hours. The reaction solution was concentrated under reduced pressure. The residue was slurried for 1 hour by adding 30mL water, filtered, and washed with water (10 mL×2). The resulting solid was dissolved with DCM (60 mL) and dried over anhydrous sodium sulfate. After filtering to remove the desiccant, the filtrate was concentrated under reduced pressure to afford compound 28-2. MS m/z (ESI):327.0[M+1]. ¹H NMR (400 MHz, CDCl3) δ 7.70 (s, 1H), 7.40 (d, J = 8.6 Hz, 2H), 6.84 (d, J = 8.6 Hz, 2H), 5.29 (s, 2H), 4.03 (s, 3H), 3.78 (s, 3H).

Step 3: Compound 28-2 (2 g, 6.15 mmol), zinc cyanide (1.09 g, 9.29 mmol), and Pd(PPh₃)₄ (0.71 g, 0.62 mmol, Bide) were dissolved in DMF (15 mL). Under a nitrogen atmosphere, the reaction was carried out at 140°C for 5 hours. The reaction solution was purified by reversed-phase C18 column chromatography (Waters-2545, chromatographic column: Santai C18 (SW-8201-040-IR), 40-63 µm, 60 Å, 55 g; mobile phase: water (containing 10 mmol/L ammonium bicarbonate) and acetonitrile, gradient ratio: acetonitrile 38%-45%, flow rate: 40 mL/min) to afford compound 28-3. MS m/z (ESI):258.1[M+1].

Step 4: Compound 28-3 (410 mg, 1.59 mmol) was dissolved in DMF (6 mL), followed by reducing the temperature to 0-5°C. Then, oxalyl chloride (273.35 µL, 3.19 mmol) was added dropwise. The reaction was carried out at 20°C for 6 hours. The reaction solution was added with 50 mL saturated sodium bicarbonate solution, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure to afford compound 28-4. MS m/z (ESI):298.0[M+Na].

Step 5: Intermediate 2 (360 mg, 1.34 mmol) was dissolved in acetonitrile (10 mL), triethylamine (742.75 µL, 5.36 mmol) was added, and then compound 28-4 (387.78 mg, 1.41 mmol) was added. The reaction was carried out at 20°C for 16 hours. The reaction solution was concentrated under reduced pressure. The residue was purified by silica gel column chromatography with an eluant system (PE: EA=10/1->1/2) to afford compound 28-5. MS m/z (ESI): 472.2[M+1].

Step 6: Compound 28-5 (260 mg, 0.55 mmol) was dissolved in ethanol (10 mL). Hydrazine hydrate (552.07 mg, 11.03 mmol) was added. The reaction was carried out at 20°C for 3 hours. After the reaction was completed, the reaction solution was filtered and rinsed with acetonitrile (20 mL × 2). The filtrate was concentrated under reduced pressure to afford compound 28-6. MS m/z (ESI): 342.1[M+1].

Step 7: Compound 29-1 (82.55 mg, 0.32 mmol) was dissolved in acetonitrile (20 mL). Then, TCFH (180.80 mg, 0.64 mmol) and N-methylimidazole (128.42 µL, 1.61 mmol) were added sequentially. After stirring at room temperature for 0.5 hours, compound 28-6 (110 mg, 0.32 mmol) was added. Under a nitrogen atmosphere, the reaction was carried out at room temperature for 16 hours. The reaction solution was concentrated under reduced pressure. The residue was added with 50 mL water, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (DCM: MeOH=DCM->10/1) to afford compound 28-7. MS m/z (ESI):580.2[M+1].

Step 8: Compound 28-7 (150 mg, 0.26 mmol) was dissolved in TFA (5 mL), TfOH (45.96 µL, 0.52 mmol) was added, and the reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 28. MS m/z (ESI): 460.2[M+1]. ¹H NMR (400 MHz, DMSO) δ 9.22 (d, J = 1.9 Hz, 1H), 9.01 (t, J = 6.0 Hz, 1H), 8.55 (dd, J = 8.2, 2.2 Hz, 1H), 8.40 (d, J = 2.2 Hz, 1H), 8.16 (d, J = 8.2 Hz, 1H), 7.52 (dd, J = 66.2, 42.6 Hz, 2H), 4.06 (s, 1H), 3.33 - 3.22 (m, 2H), 1.60 (s, 4H), 1.20 (d, J = 6.0 Hz, 3H).

### Example 29

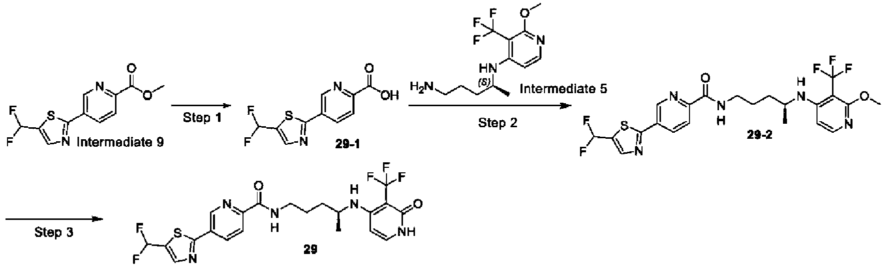

Step 1: At room temperature, intermediate 9 (0.17 g, 0.63 mmol) was dissolved in THF (1 mL), methanol (1 mL), and water (1 mL). Then, sodium hydroxide (0.05 g, 1.25 mmol) was added. The resulting mixture was stirred at room temperature for 2 hours until the reaction was completed. The reaction solution was concentrated under reduced pressure, followed by dissolving with water. The pH value was adjusted to 4-5 with 1M hydrochloric acid aqueous solution. The solid was precipitated and filtered. The filter cake was dried under reduced pressure to afford compound 29-1. MS m/z (ESI): 258.0 [M+1].

Step 2: At room temperature, compound 29-1 (0.025 g, 0.10 mmol) and intermediate 5 (0.040 g, 0.15 mmol) were dissolved in dimethylformamide (1 mL). HATU (0.054 g, 0.15 mmol) and DIPEA (0.05 mL, 0.29 mmol) were added to the reaction solution. After reacting at room temperature for 18 hours, the reaction was completed. After the reaction was completed, 3 mL water and 3 mL EA were added to the reaction solution. The organic phases were washed with saturated saline (3 mL×3), dried over sodium sulfate. After filtering, the filtrate was concentrated under reduced pressure to afford the crude. The crude was purified by silica gel column (eluant: EA:PE = 45%-55%) to afford compound 29-2. MS m/z (ESI): 516.0 [M+1].

Step 3: At room temperature, compound 29-2 (0.025 g, 0.05 mmol) was dissolved in NMP (1 mL). Then, iodotrimethylsilane (0.009 g, 0.05 mmol) was added, followed by stirring at 85°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 35%-95%, flow rate: 20 mL/min) to afford compound 29. MS m/z (ESI): 502.0 [M+1]. ¹H NMR (400 MHz, DMSO-*d6*) δ 10.87 (s, 1H), 9.22 (d, *J =* 1.6 Hz, 1H), 9.00 (t, *J* = 6.0 Hz, 1H), 8.55 (dd, *J* = 8.2, 2.2 Hz, 1H), 8.39 (s, 1H), 8.16 (d, *J =* 8.2 Hz, 1H), 7.53 (t, *J =* 54.4 Hz, 1H), 7.28 - 7.19 (m,1H), 5.92 (d, *J* = 7.8 Hz, 1H), 5.83 - 5.75 (m, 1H), 3.81 - 3.62 (m, 1H), 3.32 - 3.28 (m, 2H), 1.70 - 1.41 (m, 4H), 1.14 (d, *J* = 6.2 Hz, 3H).

### Example 30

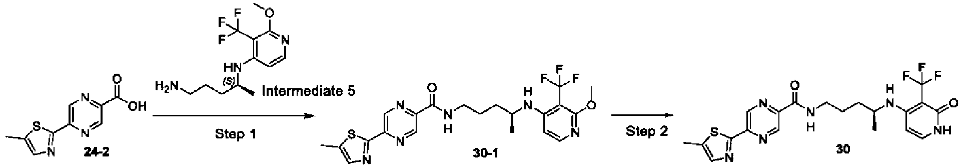

Step 1: At room temperature, intermediate 5 (0.125 g, 0.45 mmol) and compound 24-2 (0.1 g, 0.45 mmol) were dissolved in N,N-dimethylformamide (2 mL). HATU (0.257 g, 0.68 mmol) and DIPEA (0.22 mL, 1.36 mmol) were added to the reaction solution. After reacting at room temperature for 18 hours, the reaction was completed. After the reaction was completed, 3 mL water and 3 mL EA were added to the reaction solution. The organic phases were washed with saturated saline (3 mL×3), dried over sodium sulfate. After filtering, the filtrate was concentrated under reduced pressure to afford the crude. The crude was purified by silica gel column (eluant: EA: PE = 25%-33%) to afford compound 30-1. MS m/z (ESI): 482.1 [M+1].

Step 2: At room temperature, compound 30-1 (0.024 g, 0.05 mmol) was dissolved in NMP (1 mL). Then, iodotrimethylsilane (0.02 g, 0.10 mmol) was added, followed by stirring at 85°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 30%-95%, flow rate: 20 mL/min) to afford compound 30. MS m/z (ESI): 467.2 [M+1]. ¹H NMR (400 MHz, DMSO *d6*) δ 10.91 - 10.80 (m, 1H), 9.33 - 9.23 (m, 1H), 9.19 - 9.13 (m, 1H), 9.11 - 9.03 (m, 1H), 7.85 (d, 1H), 7.26 - 7.21 (m, 1H), 5.98 - 5.75 (m, 2H), 3.29 - 3.14 (m, 3H), 2.57 (s, 3H), 1.59 (s, 4H), 1.20 - 1.11 (m, 3H).

### Example 31

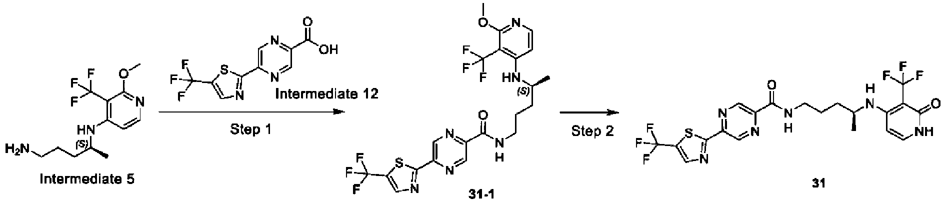

Step 1: At room temperature, intermediate 5 (0.04 g, 0.14 mmol) and compound intermediate 12 (0.047 g, 0.17 mmol) were dissolved in DMF (1 mL). HATU (0.082 g, 0.22 mmol) and DIPEA (0.07 mL, 0.43 mmol) were added to the reaction solution. After reacting at room temperature for 18 hours, the reaction was completed. After the reaction was completed, 3 mL water and 3 mL EA were added to the reaction solution. The organic phases were washed with saturated saline (3 mL×3), dried over sodium sulfate. After filtering, the filtrate was concentrated under reduced pressure to afford the crude. The crude was purified by silica gel column (eluant: EA:PE = 30%-35%) to afford compound 31-1. MS m/z (ESI): 535.2 [M+1].

Step 2: At room temperature, compound 31-1 (0.03 g, 0.06 mmol) was dissolved in NMP (1 mL). Then, iodotrimethylsilane (0.023 g, 0.11 mmol) was added, followed by stirring at 85°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 31. MS m/z (ESI): 521.1 [M+1]. ¹H NMR (400 MHz, DMSO-d6) δ 10.84 (s, 1H), 9.38 (d, *J =* 1.4 Hz, 1H), 9.25 (d, *J =* 1.4 Hz, 1H), 9.13 - 9.08 (m, 1H), 8.76 (s, 1H), 7.26 - 7.19 (m, 1H), 5.92 (d, *J =* 7.6 Hz, 1H), 5.79 - 5.70 (m, 1H), 3.78 - 3.65 (m, 1H), 3.36 - 3.31 (m, 2H), 1.64 - 1.50 (m, 4H), 1.15 (d, *J =* 6.2 Hz, 3H).

### Example 32

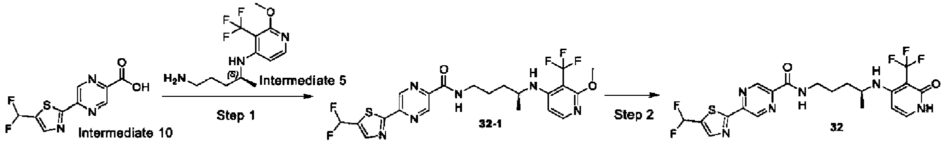

Step 1: At room temperature, intermediate 5 (0.036 g, 0.14 mmol) and intermediate 10 (0.040 g, 0.14 mmol) were dissolved in DMF (1 mL). HATU (0.082 g, 0.22 mmol) and DIPEA (0.056 g, 0.43 mmol) were added to the reaction solution. After reacting at room temperature for 18 hours, the reaction was completed. After the reaction was completed, 3 mL water and 3 mL EA were added to the reaction solution. The organic phases were washed with saturated saline (3 mL×3), dried over sodium sulfate. After filtering, the filtrate was concentrated under reduced pressure to afford the crude. The crude was purified by silica gel column (eluant: EA: PE = 25%-33%) to afford compound 32-1. MS m/z (ESI): 513.1 [M+1].

Step 2: At room temperature, compound 32-1 (0.07 g, 0.08 mmol) was dissolved in NMP (1 mL). Then, iodotrimethylsilane (0.031 g, 0.16 mmol) was added, followed by stirring at 85°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 30%-95%, flow rate: 20 mL/min) to afford compound 32. MS m/z (ESI): 503.1 [M+1]. ¹H NMR (400 MHz, DMSO-d6) *δ* 10.86 (s, 1H), 9.36 (d, *J =* 1.4 Hz, 1H), 9.24 (d, *J =* 1.4 Hz, 1H), 9.13 (t, *J* = 5.8 Hz, 1H), 8.47 (t, *J =* 2.0 Hz, 1H), 7.53 (t, *J* = 54.4 Hz, 1H), 7.24 (d, *J =* 6.8 Hz, 1H), 5.92 (d, *J =* 7*.*8 Hz, 1H), 5.83 - 5.75 (m, 1H), 3.78 - 3.66 (m, 1H), 3.32 - 3.28 (m, 2H), 1.62 - 1.49 (m, 4H), 1.14 (d, *J* = 6.2 Hz, 3H).

### Example 33

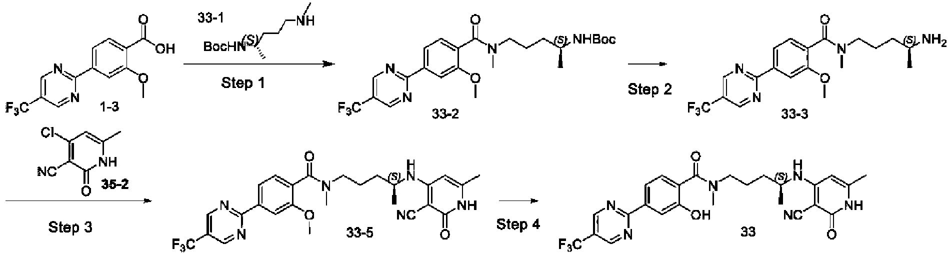

Step 1: Compound 1-3 (300 mg, 1 mmol) was added to N,N-dimethylformamide (3 mL). HATU (455.60 1.2 mmol) and DIPEA (387.2 mg, 1.2 mmol) were added sequentially. After stirring at room temperature for half an hour, compound 33-1 (216 mg, 1 mmol) was added. The reaction was carried out at room temperature for 16 hours. The reaction solution was added with water (10 mL), followed by extraction with EA (5 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=2:1 to 1:1) to afford compound 33-2. MS m/z (ESI):397.1[M-100+H].

Step 2: Compound 33-2 (380 mg, 0.77 mmol) was dissolved in 4M hydrochloric acid in EA (3 mL, 12 mmol) solution. The reaction was carried out at room temperature for 1 hour. The reaction solution was concentrated under reduced pressure to afford compound 33-3.

Step 3: Compound 33-3 (200 mg, 0.50 mmol) and compound 35-2 (85.0 mg, 0.50 mmol) were added to isopropanol (3 mL), and then DIPEA (0.25 mL, 1.5 mmol) was added. The reaction was carried out at 80°C for 18 hours. The reaction solution was concentrated under reduced pressure. The residue was purified by silica gel column chromatography with an eluant system (DCM: anhydrous methanol=100: 1 to 20: 1) to afford compound 33-5. MS m/z (ESI):529.4 [M+1].

Step 4: Compound 33-5 (106 mg, 0.2 mmol) was dissolved in DCM (2 mL), followed by purging with nitrogen three times, and reducing the temperature to -78°C. Boron tribromide (56.88 µL, 0.6 mmol) was added dropwise. After the dropwise addition was completed, the resulting mixture was allowed to warm to room temperature and reacted for 1 hour. After reducing the temperature to -78°C, 1 mL methanol was added dropwise. The resulting mixture was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 33. MS m/z (ESI): 515.2[M+1]. ¹H NMR (400 MHz, DMSO) δ 10.67 - 9.68 (m, 2H), 9.29 (s, 2H), 8.03 (d, *J* = 1.4 Hz, 1H), 7.94 (dd, *J* = 7.8, 1.5 Hz, 1H), 7.27 (d, *J =* 7. Hz, 1H), 6.43 - 6.23 (m, 1H), 5.73 (s, 1H), 3.92 - 3.65 (m, 1H), 3.56 - 3.20 (m, 2H), 2.89 (s, 3H), 2.08 (s, 3H), 1.81 - 1.31 (m, 4H), 1.16 (s, 3H).

### Example 34

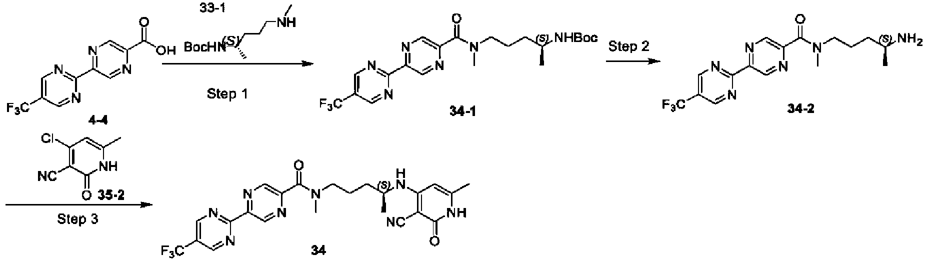

Step 1: At room temperature, DIPEA (251 mg, 1.94 mmol) and HATU (195 mg, 0.78 mmol) were added to a solution of compound 4-4 (175 mg, 0.65 mmol) and compound 33-1 (140 mg, 0.65 mmol) in DMF (4 mL). The reaction solution was stirred at room temperature for 18 h. After the reaction was completed, the mixture was poured into water (10 mL), followed by extraction with EA (5 mL×3). The organic phases were combined, washed with saturated saline (10 mL×2), dried over anhydrous sodium sulfate, filtered, and concentrated under reduced pressure to afford the crude compound. The crude compound was purified by silica gel column (eluant: EA: PE = 25%-50%) to afford compound 34-1. MS m/z (ESI): 369.1 [M+1-100].

Step 2: At room temperature, a 1,4-dioxane solution of hydrochloric acid (2 mL, 8.0 mmol) was added to a solution of compound 34-1 (200 mg, 0.43 mmol) in 1,4-dioxane (2 mL). The reaction solution was stirred at room temperature for 2 hours. After the reaction was completed, the reaction solution was concentrated under reduced pressure to afford compound 34-2. MS m/z (ESI): 369.1 [M+1].

Step 3: At room temperature, DIPEA (63 mg, 0.49 mmol) was added to a solution of compound 35-2 (28 mg, 0.16 mmol) and compound 34-2 (60 mg, 0.16 mmol) in isopropanol (1 mL), followed by heating to 80°C and stirring for 18 h. After the reaction was completed, the crude product was purified by preparative HPLC (chromatographic column: Waters-Xbridge-C18-10µm-19×250mm; mobile phase: A: 10 mM ammonium bicarbonate/water, B: acetonitrile, gradient ratio: acetonitrile 21-95%, flow rate: 25 mL/min) to afford compound 34. MS m/z (ESI): 501.2 [M+1]. ¹H NMR (400 MHz, DMSO) δ 11.26 - 10.73 (m, 1H), 9.65 - 9.45 (m, 3H), 9.03 (dd, *J* = 7.2, 1.4 Hz, 1H), 6.94 - 6.38 (m, 1H), 5.99 - 5.56 (m, 1H), 3.59 - 3.47 (m, 1H), 3.33 - 3.22 (m, 2H), 3.01 (d, *J* = 24.8 Hz, 3H), 2.07 (d, *J* = 21.2 Hz, 3H), 1.78 - 1.29 (m, 4H), 1.14 (dd, *J =* 38.4, 6.3 Hz, 3H).

### Example 35

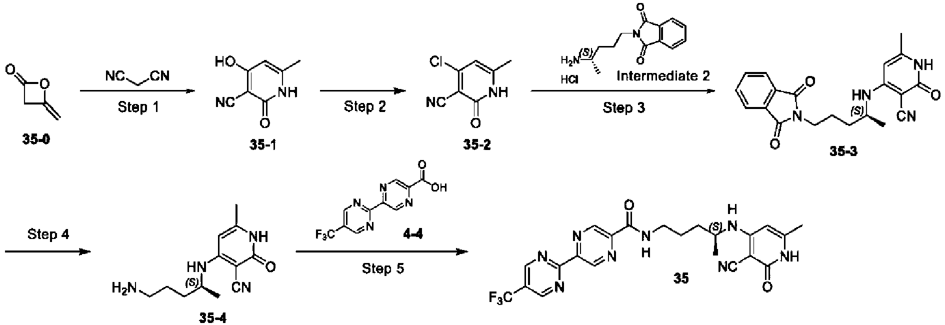

Step 1: At 0°C, sodium hydride (3.09 g, 77.32 mmol, 60% purity) was added to a solution of malononitrile (3.78 mL, 59.47 mmol, Bide) in THF (5 mL), followed by stirring for 1 hour. Then, compound 35-0 (5.0 g, 59.47 mmol, Bide) was added to the above reaction solution. The reaction was carried out at room temperature for another 2 hours. After reacting for 2 hours, the mixture was adjusted to a pH value of 5-6 using 4N hydrochloric acid (50 mL, 200 mmol). Subsequently, the mixture was concentrated under reduced pressure to remove THF, followed by the addition of further 4N hydrochloric acid (50 mL, 200 mmol). The mixture was heated to 100°C and stirred overnight. After the reaction was completed, the mixture was cooled and allowed to stand, and then filtered. The filter cake was dried under vacuum to afford compound 35-1. MS m/z (ESI): 151.1 [M+1]. ¹H NMR (400 MHz, DMSO) δ 12.38 (s, 1H), 11.70 (s, 1H), 5.82 (s, 1H), 2.17 (s, 3H).

Step 2: At 0°C, phosphorus oxitrichloride (3.91 g, 25.5 mmol) and phosphorus pentachloride (5.30 g, 25.5 mmol) were added to a solution of compound 35-1 (2.55 g, 12.7 mmol) in chloroform (30 mL). The reaction solution was heated to 75°C and stirred for 18 hours. After the reaction was completed, the mixture was poured into water (100 mL) and adjusted to neutral pH with saturated sodium bicarbonate solution, followed by extraction with DCM (100 mL × 3). The organic phases were combined, washed with saturated saline (100 mL × 2), dried over anhydrous sodium sulfate, filtered, and concentrated under reduced pressure to afford the crude compound. The crude compound was slurried with a mixed solution of DCM/methanol to afford compound 35-2. MS m/z (ESI): 169.0 [M+1]. ¹H NMR (400 MHz, DMSO) δ 12.94 (s, 1H), 6.53 (s, 1H), 2.29 (s, 3H).

Step 3: At room temperature, DIPEA (0.83 mL, 4.98 mmol) was added to a solution of intermediate 2 (232 mg, 1.00 mmol) and compound 35-2 (168 mg, 1.00 mmol) in isopropanol (3 mL), followed by heating to 80°C and stirring for 18 h. After the reaction was completed, the crude product was afforded by concentration under reduced pressure. The crude product was purified by silica gel column (eluant: EA: PE = 25%-75%) to afford compound 35-3. MS m/z (ESI): 365.1 [M+1].

Step 4: At 25°C, hydrazine hydrate (165 mg, 3.25 mmol) was added to a solution of compound 35-3 (200 mg, 0.33 mmol) in ethanol (3 mL), followed by stirring overnight at room temperature. After the reaction was completed, the crude compound was afforded by concentration under reduced pressure. The crude compound was slurried with acetonitrile solution (6 mL) to afford compound 35-4. MS m/z (ESI): 235.1 [M+1].

Step 5: At room temperature, DIPEA (99 mg, 0.77 mmol) and HATU (117 mg, 0.31 mmol) were added to a solution of compound 4-4 (69 mg, 0.26 mmol) and compound 35-4 (60 mg, 0.26 mmol) in DMF (2 mL). The reaction solution was stirred at room temperature for 18 hours. After the reaction was completed, the crude product was purified by preparative HPLC (chromatographic column: Waters-SunFire-C18-10µm-19×250mm; mobile phase: A: 0.1% formic acid/water, B: acetonitrile, gradient ratio: acetonitrile 0-32%, flow rate: 25 mL/min) to afford compound 35. MS m/z (ESI): 487.2 [M+1]. ¹H NMR (400 MHz, DMSO) δ 11.13 - 10.83 (m, 1H), 9.61 (d, *J* = 1.4 Hz, 1H), 9.54 (s, 2H), 9.37 (d, *J =* 1.4 Hz, 1H), 9.19 (t, *J =* 5.8 Hz, 1H), 6.73 - 6.44 (m, 1H), 6.02 - 5.60 (m, 1H), 3.90 - 3.61 (m, 1H), 3.39 - 3.33 (m, 2H), 2.08 (s, 3H), 1.73 - 1.44 (m, 4H), 1.15 (d, *J =* 6.4 Hz, 3H).

### Example 36

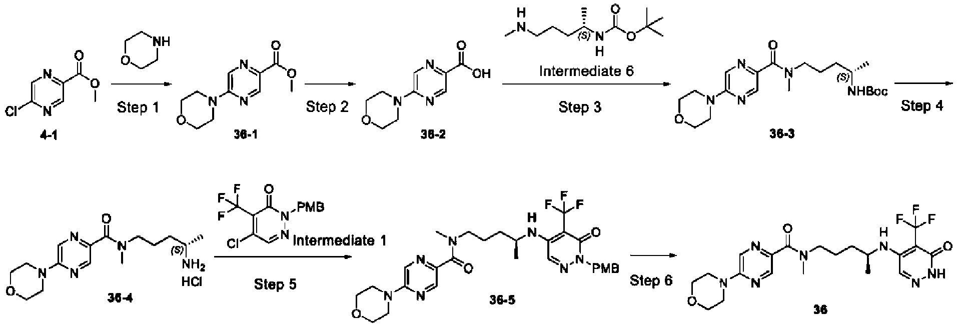

Step 1: Compound 4-1 (500 mg, 2.90 mmol), morpholine (252.42 mg, 2.90 mmol), and DIPEA were dissolved in 3 mL of 1,4-dioxane, followed by stirring at 60°C for 3 hours. The filtrate was concentrated under reduced pressure. The residue was purified by silica gel column chromatography with an eluant system (DCM/methanol=50/1) to afford compound 36-1. MS m/z (ESI):224.1[M+1].

Step 2: Compound 36-1 (900 mg, 4.03 mmol) was dissolved in methanol (4 mL) and water (2 mL). LiOH (338 mg, 8.06 mmol) was added, followed by reacting under stirring at room temperature for 2 hours. The reaction solution was filtered and concentrated under reduced pressure to afford compound 36-2. MS m/z (ESI):210.0[M+1].

Step 3: Compound 36-2 (500 mg, 2.39 mmol) was dissolved in DMF (15 mL), and then TCFH (1005.86 mg, 3.58 mmol) and N-methylimidazole (762.09 µL, 9.56 mmol) were added. The reaction was carried out at 20°C for 30 minutes. Then, intermediate 6 (155.10 mg, 0.72 mmol) was added. The reaction was carried out at 20°C for another 16 hours. The reaction solution was added with 80 mL water, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (DCM: MeOH=30/1) to afford compound 36-3. MS m/z (ESI):408.2[M+1].

Step 4: Compound 36-3 (230 mg, 0.56 mmol) was dissolved in a solution of 4 M hydrochloric acid in 1,4-dioxane (10 mL). The reaction was carried out at 20°C for 16 hours. The reaction solution was concentrated under reduced pressure to afford compound 36-4. MS m/z (ESI): 308.2[M+1].

Step 5: Compound 36-4 (200 mg, 0.65 mmol) was dissolved in acetonitrile (12 mL). Triethylamine (0.45 mL, 3.25 mmol) and intermediate 1 (207.34 mg, 0.65 mmol) were added. The reaction was carried out at 85°C for 3 hours. After the reaction was completed, the reaction solution was concentrated under reduced pressure. The residue was purified by silica gel column chromatography with an eluant system (DCM: MeOH=100/1->10/1) to afford compound 36-5. MS m/z (ESI): 590.3[M+1].

Step 6: Compound 36-5 (380 mg, 0.23 mmol) was dissolved in TFA (10 mL), TfOH (0.5 mL, 5.63 mmol) was added, and the reaction was carried out at room temperature for 1 hour. The reaction solution was concentrated under reduced pressure and purified by high performance liquid chromatography (Waters-2545, chromatographic column: SharpSil-T C18,30×150mm, 5 µm; mobile phase: water (containing 10 mmol/L ammonium bicarbonate) and acetonitrile, gradient ratio: acetonitrile 38%-45%, flow rate: 30 mL/min) to afford compound 36. MS m/z (ESI): 470.2[M+1]. ¹H NMR (400 MHz, DMSO) δ 12.44 (s, 1H), 8.34 (s, 1H), 8.24 (s, 0H), 8.14 (s, 0H), 7.88 (d, J = 37.4 Hz, 1H), 6.31 (d, J = 31.8 Hz, 1H), 3.71 (t, J = 4.8 Hz, 4H), 3.60 (s, 4H), 3.44 (s, 2H), 3.28 (s, 1H), 2.98 (d, J = 37.7 Hz, 3H), 1.73 - 1.28 (m, 4H), 1.21 - 1.11 (m, 3H).

### Example 37

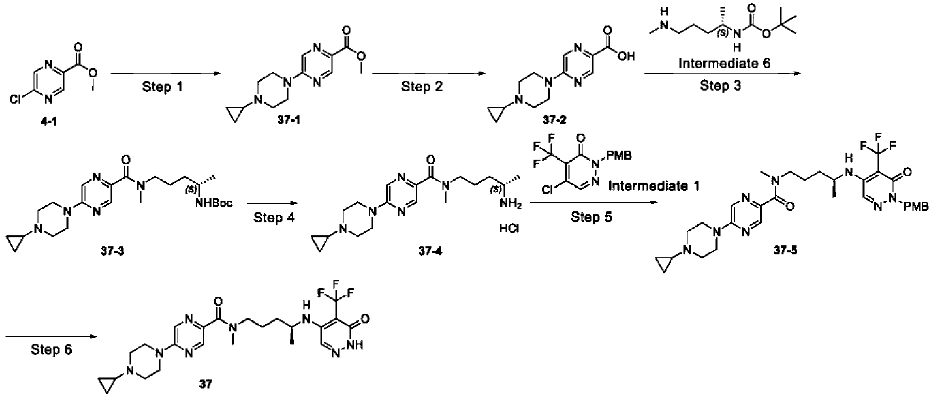

Step 1: 4-1 (683 mg, 3.96 mmol) was dissolved in 1,4-dioxane (10 mL). 1-cyclopropylpiperazine (500 mg, 3.96 mmol) and DIPEA (1.97 mL, 11.89 mmol) were added under nitrogen protection. The mixed solution was stirred at 100°C for 16 hours. The filtrate was concentrated under reduced pressure. The residue was purified by silica gel column chromatography eluant system (DCM: MeOH=10/1) to afford compound 37-1. MS m/z (ESI):263.1[M+1]. ¹H NMR (400 MHz, DMSO) δ 8.65 (d, J = 1.0 Hz, 1H), 8.37 (d, J = 1.0 Hz, 1H), 3.81 (s, 3H), 3.74 - 3.64 (m, 4H), 2.67 - 2.57 (m, 4H), 1.72 - 1.61 (m, 1H), 0.48 - 0.41 (m, 2H), 0.37 (d, J = 3.2 Hz, 2H).

Step 2: Compound 37-1 (800 mg, 3.05 mmol) was dissolved in methanol (8 mL) and water (4 mL). LiOH (73 mg, 3.05 mmol) was added, followed by reacting under stirring at room temperature for 16 hours. The reaction solution was filtered and concentrated under reduced pressure, to afford compound 37-2. MS m/z (ESI):249.1[M+1]. ¹H NMR (400 MHz, DMSO) δ 12.88 (s, 1H), 8.70 (d, J = 1.0 Hz, 1H), 8.47 (s, 1H), 4.63 (d, J = 12.7 Hz, 2H), 3.57 (t, J = 12.5 Hz, 6H), 2.84 (s, 1H), 1.22 (s, 2H), 0.80 (d, J = 6.0 Hz, 2H).

Step 3: Compound 37-2 (344.34 mg, 1.39 mmol) was dissolved in acetonitrile (10 mL), and then TCFH (648.53 mg, 2.31 mmol) and N-methylimidazole (474.47 mg, 5.78 mmol) were added. The reaction was carried out at 20°C for 0.5 hours. Intermediate 6 (250 mg, 1.16 mmol) was added. The reaction was carried out at 20°C for 16 hours. The reaction solution was added with 5 mL water, followed by extraction with EA (10 mL×3). The organic phases were combined, and dried over anhydrous sodium sulfate. After filtering to remove the desiccant, the filtrate was concentrated under reduced pressure. The residue was purified by silica gel plate chromatography with a developing agent system (DCM: MeOH=10/1) to afford compound 37-3. MS m/z (ESI): 447.4[M+1].

Step 4: Compound 37-3 (310 mg, 0.69 mmol) was dissolved in a 5 mL solution of 4M HCl in 1,4-dioxane. The reaction was carried out at 20°C for 2 hours. The reaction solution was directly concentrated under reduced pressure to afford compound 37-4. MS m/z (ESI): 347.2[M+1].

Step 5: Compound 37-4 (220 mg, 0.63 mmol) was dissolved in acetonitrile (5 mL). Intermediate 1 (222.58 mg, 0.70 mmol) and triethylamine (0.09 mL, 3.15 mmol) were added. The reaction was carried out at 85°C for 3 hours. The reaction solution was concentrated under reduced pressure. The remaining solution was added with water (10 mL), followed by extraction with EA (10 mL×3). The organic phases were combined, and dried over anhydrous sodium sulfate. After filtering to remove the desiccant, the filtrate was concentrated under reduced pressure. The residue was purified by silica gel column chromatography with an eluant system (DCM: MeOH=20/1) to afford compound 37-5. MS m/z (ESI): 629.4[M+1].

Step 6: Compound 37-5 (200 mg, 0.32 mmol) was dissolved in TFA (2 mL). Methanesulfonic acid (0.2 mL) was added. The reaction was carried out at 20°C for 1 hour. The reaction solution was added with methanol (10 mL), and concentrated under reduced pressure. The remaining solution was added with EA (20 mL), neutralized with saturated sodium bicarbonate solution to pH=8. The aqueous phase was extracted with EA (20 mL×3). The organic phases were combined, and dried over anhydrous sodium sulfate. After filtering to remove the desiccant, the filtrate was concentrated under reduced pressure. The residue was purified by preparative to afford compound 37. MS m/z (ESI):509.3[M+1]. ¹H NMR (400 MHz, DMSO) δ 12.44 (s, 1H), 8.32 (s, 1H), 8.19 (d, J = 4.0 Hz, 1H), 7.89 (t, J = 4.0 Hz, 1H), 6.35-6.28 (m, 1H), 3.99-3.81 (m, 1H), 3.59 (s, 4H), 3.44 (s, 2H), 3.04-2.93 (m, 3H), 2.64-2.61 (m, 4H),1.67-1.60 (m, 5H),1.18-1.15 (m, 3H),0.46-0.37 (m, 4H).

### Example 38

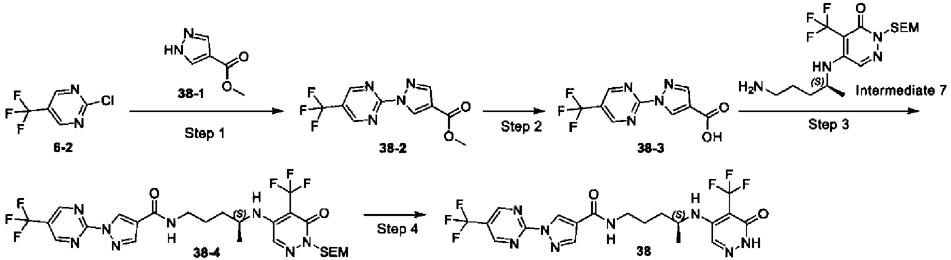

Step 1: At room temperature, compound 6-2 (0.5 g, 2.74 mmol), compound 38-1 (0.345 g, 2.74 mmol), and DIPEA (0.708 g, 5.48 mmol) were added to a 50 mL single-necked flask, and then N,N-dimethylformamide (7.0 mL) was added. After nitrogen protection, the temperature was raised to 50°C and the reaction was carried out for 4 hours. After the reaction was completed, 10 mL water and 10 mL EA were added to the reaction solution. The organic phases were washed with saturated saline (15 mL×3) and dried over sodium sulfate. After filtering, the filtrate was concentrated under reduced pressure to afford the crude. The crude was purified by silica gel chromatography (eluant: PE: EA = 15%-20%) to afford compound 38-2. MS m/z (ESI): 273.0 [M+1].

Step 2: At room temperature, compound 38-2 (0.46 g, 1.69 mmol) was added to a 25 mL single-necked flask, and then iodotrimethylsilane (6.0 mL) was added. The temperature was raised to 90°C and the reaction was carried out for 4 hours. After the reaction was completed, 10 mL water and 10 mL EA were added to the reaction solution. The organic phases were washed with saturated saline (15 mL×3) and dried over sodium sulfate. After filtering, the filtrate was concentrated under reduced pressure to afford compound 38-3. MS m/z (ESI): 259.0 [M+1].

Step 3: At room temperature, compound 38-3 (0.13 g, 0.51 mmol) and compound intermediate 7 (0.2 g, 0.51 mmol) were dissolved in DMF (2 mL). HATU (0.29 g, 0.76 mmol) and DIPEA (0.25 mL, 1.52 mmol) were added to the reaction solution. After reacting at room temperature for 18 hours, the reaction was completed. After the reaction was completed, 3 mL water and 3 mL EA were added to the reaction solution. The organic phases were washed with saturated saline (3 mL×3), dried over sodium sulfate. After filtering, the filtrate was concentrated under reduced pressure to afford the crude. The crude was purified by silica gel chromatography (eluant: EA: PE = 15%-20%) to afford compound 38-4. MS m/z (ESI): 633.2 [M-1].

Step 4: At room temperature, compound 38-4 (0.23 g, 0.36 mmol) was dissolved in a solution of 4M hydrochloric acid in 1,4-dioxane (3 mL), followed by stirring at room temperature for 2 hours until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 20%-95%, flow rate: 20 mL/min) to afford compound 38. MS m/z (ESI): 505.1 [M+1]. ¹H NMR (400 MHz, DMSO-d6) *δ* 12.43 (s, 1H), 9.37 (s, 2H), 9.23 (s, 1H), 8.49 - 8.42 (m, 1H), 8.23 (s, 1H), 7.93 (s, 1H), 6.38 - 6.30 (m, 1H), 4.06 - 3.92 (m, 1H), 3.27 - 3.19 (m, 2H), 1.72 - 1.63 (m, 1H), 1.53 (s, 3H), 1.19 (d, *J* = 6.4 Hz, 3H).

### Example 39

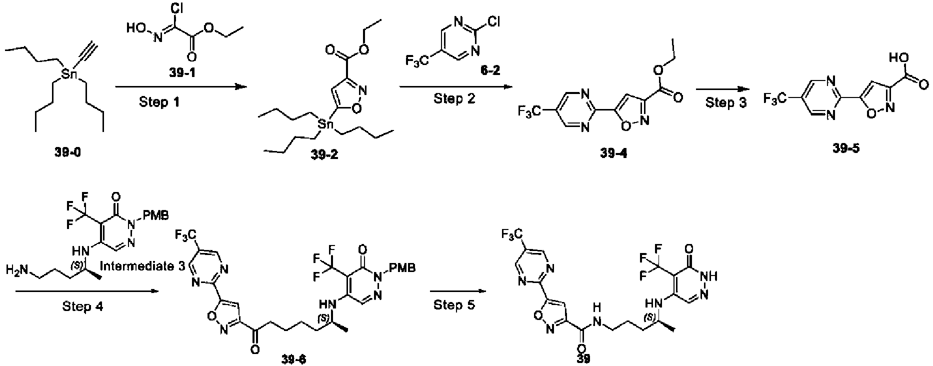

Step 1: At room temperature, compound 39-0 (2.086 g, 6.62 mmol) and compound 39-1 (1.0 g, 6.62 mmol) were dissolved in THF (15 mL). Triethylamine (4.6 mL, 33.1 mmol) was added to the reaction solution. After reacting at room temperature for 18 hours, the reaction was completed. After the reaction was completed, the crude was afforded by concentration under reduced pressure. The crude was purified by silica gel column (eluant: EA: PE = 5%-10%) to afford compound 39-2. ¹H NMR (400 MHz, DMSO-d6) δ 6.93 (s, 1H), 4.41 - 4.29 (m, 2H), 1.55 - 1.17 (m, 21H), 0.87 - 0.81 (m, 9H).

Step 2: At room temperature, compound 39-2 (0.708 g, 1.65 mmol) and compound 6-2 (0.3 g, 1.65 mmol) were dissolved in toluene (10 mL). Then, Pd(PPh₃)₄ (0.19 g, 0.165 mmol) was added, followed by stirring at 120°C for 18 hours until the reaction was completed. After the reaction was completed, the crude was afforded by concentration under reduced pressure. The crude was purified by silica gel column (eluant: EA: PE = 16%-20%) to afford compound 39-4. MS m/z (ESI): 288.1 [M+1].

Step 3: At room temperature, compound 39-4 (0.1 g, 0.17 mmol) was dissolved in THF (1 mL), methanol (1 mL), and water (1 mL). LiOH (0.013 g, 0.52 mmol) was added to the reaction solution. After reacting at room temperature for 2 hours, the reaction was completed. After the reaction was completed, the crude was afforded by concentration under reduced pressure. The crude was dissolved in water. The pH value was adjusted to 4-5 with 1M hydrochloric acid solution. After filtering, the filter cake was dried under reduced pressure to afford compound 39-5. MS m/z (ESI): 260.0 [M+1].

Step 4: At room temperature, intermediate 3 (0.1 g, 0.26 mmol) and compound 39-5 (0.067 g, 0.26 mmol) were dissolved in N,N-dimethylformamide (1 mL). HATU (0.148 g, 0.39 mmol) and DIPEA (0.135 mL, 0.78 mmol) were added to the reaction solution. After reacting at room temperature for 18 hours, the reaction was completed. After the reaction was completed, 3 mL water and 3 mL EA were added to the reaction solution. The organic phases were washed with saturated saline (3 mL×3), dried over sodium sulfate. After filtering, the filtrate was concentrated under reduced pressure to afford the crude. The crude was purified by silica gel column (eluant: EA: PE = 25%-33%) to afford compound 39-6. MS m/z (ESI): 626.2 [M+1].

Step 5: At room temperature, compound 39-6 (0.080 g, 0.13 mmol) was dissolved in TFA (1 mL). Then, TfOH (0.2 mL) was added, followed by stirring at 50°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 30%-95%, flow rate: 20 mL/min) to afford compound 39. MS m/z (ESI):506.1 [M+1]. ¹H NMR (400 MHz, DMSO-*d6*) *δ* 12.41 (s, 1H), 9.49 (s, 2H), 9.07 - 8.98 (m, 1H), 7.94 (s, 1H), 7.62 (s, 1H), 6.42 - 6.31 (m, 1H), 4.05 - 3.92 (m, 1H), 3.32 - 3.23 (m, 2H), 1.71 - 1.50 (m, 4H), 1.18 (d, *J=* 6.2 Hz, 3H).

### Example 40

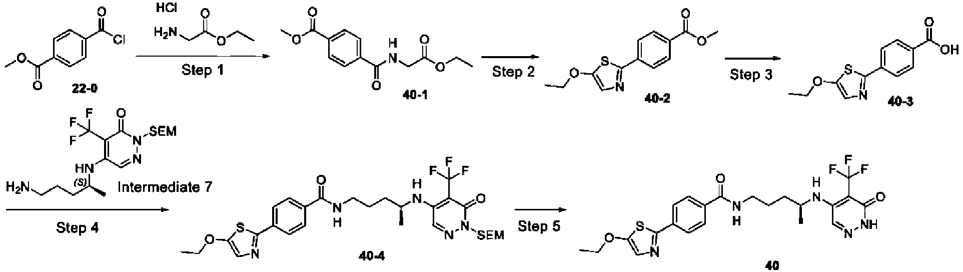

Step 1: Compound 22-0 (2 g, 10.07 mmol, Bide) was dissolved in 10 mL anhydrous DCM. Triethylamine (4.19 mL, 30.21 mmol) was added. The reaction solution was maintained at 0°C-10°C under a nitrogen condition. 2-aminoEA hydrochloride (1.56 g, 15.11 mmol) was dissolved in 5 mL anhydrous DCM, and added dropwise. The reaction solution was reacted under stirring at 25°C for 2 hours. The reaction solution was washed with water (20 mL× 2), dried over anhydrous sodium sulfate, and filtered. The filtrate was concentrated under reduced pressure to afford compound 40-1. MS m/z (ESI): 266.1[M+H].

Step 2: Compound 40-1 (900 mg, 3.58 mmol) was dissolved in chloroform (10 mL). Phosphorus pentasulfide (1.59 g, 7.16 mmol) and 4A molecular sieve (1.8 g) were added. The reaction solution was purged with nitrogen and reacted under stirring at 60°C for 6 hours under a sealed condition. The reaction solution was cooled to room temperature, and filtered through celite. The filter cake was rinsed with DCM (15 mL× 2), and concentrated under reduced pressure to afford compound 40-2. MS m/z (ESI): 264.0[M+H].

Step 3: Compound 40-2 (200 mg, 0.76 mmol) was dissolved in a mixed solvent of 5 mL methanol and 2.5 mL water. LiOH (64 mg, 1.52 mmol) was added, followed by reacting under stirring at 25°C for 16 hours. The reaction solution was diluted by adding water (8 mL), followed by concentration under reduced pressure to remove methanol. 0.1M hydrochloric acid solution was added dropwise. The solution was adjusted to pH<7, followed by extraction with DCM/isopropanol (3/1) (6 mL × 2). The organic phases were washed with saturated saline (15 mL× 2), dried over anhydrous sodium sulfate, filtered and concentrated to afford compound 40-3. MS m/z (ESI): 250.0[M+H].

Step 4: Compound 40-3 (82 mg, 0.33 mmol) was dissolved in 3 mL DMF, and DIPEA (128 mg, 0.99 mmol) and HATU (188 mg, 0.49 mmol) were added sequentially. The reaction solution was reacted at room temperature for 0.5 hours. Intermediate 7 (130 mg, 0.33 mmol) was added. The reaction solution was reacted at room temperature for 3 hours. The reaction solution was added with 10 mL water, followed by extraction with EA (6 mL×2). The organic phases were combined, and washed with saturated saline. The organic phases were dried over anhydrous sodium sulfate. After filtering to remove the desiccant, the filtrate was concentrated under reduced pressure to afford compound 40-4. MS m/z (ESI): 626.2[M+H⁺].

Step 5: Compound 40-4 (244 mg, 0.39 mmol) was dissolved in a 5 mL solution of 4 M hydrochloric acid in 1,4-dioxane, followed by reacting under stirring at 25°C for 16 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L TFA) and acetonitrile, gradient ratio: acetonitrile 30%-60%, flow rate: 30 mL/min) to afford compound 40. MS m/z (ESI): 496.1[M+H]. ¹H NMR (400 MHz, DMSO) δ 12.45 (s, 1H), 8.57 (t, J = 5.4 Hz, 1H), 7.98 - 7.81 (m, 5H), 7.36 (s, 1H), 6.36 (d, J = 5.5 Hz, 1H), 4.22 (q, J = 7.0 Hz, 2H), 3.98 (s, 1H), 3.27 (d, J = 5.2 Hz, 2H), 1.65 (d, J = 7.9 Hz, 1H), 1.54 (s, 3H), 1.38 (t, J = 7.0 Hz, 3H), 1.18 (d, J = 6.3 Hz, 3H).

### Example 41

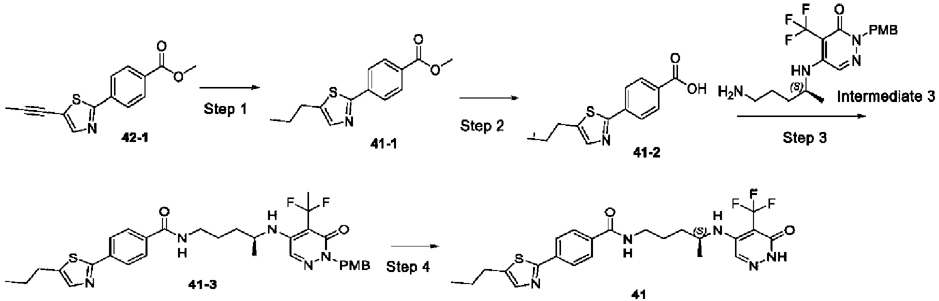

Step 1: Compound 42-1 (230 mg, 0.89 mmol) and 10% pd /C (123 mg, 1.16 mmol) were added with MeOH (2 mL). The resulting mixture was stirred at room temperature for another 16 hours under hydrogen protection. The reaction solution was filtered through celite, and rinsed with methanol (10 mL×1). The filtrate was concentrated under reduced pressure, to afford compound 41-0. MS m/z (ESI):262.0 [M+1].

Step 2: Compound 41-1 (200 mg, 0.78 mmol) and LiOH (73.86 mg, 1.76 mmol) were added to a mixed solution of MeOH (20 mL) and water (10 mL). The resulting mixture was stirred at room temperature for 16 hours. The reaction solution was added with water (10 mL), followed by extraction with EA (10 mL×3). The organic phases were combined. The organic phases were washed with saturated saline (20 mL×3), and dried over anhydrous sodium sulfate. After filtering to remove the desiccant, the filtrate was concentrated under reduced pressure to afford compound 41-2. MS m/z (ESI): 248.0[M+1].

Step 3: Compound 41-2 (50 mg, 0.20 mmol) was added to MeCN (4 mL). TCFH (56.73 mg, 0.20 mmol), N- methylimidazole (81.59 µL, 1.01 mmol), and intermediate 3 (77.72 mg, 0.20 mmol) were added. The reaction was carried out at room temperature for 16 hours. The reaction solution was added with water (10 mL), followed by extraction with EA (10 mL×3). The organic phases were combined. The organic phases were washed with saturated saline (20 mL×3), and dried over anhydrous sodium sulfate. After filtering to remove the desiccant, the filtrate was concentrated under reduced pressure. The residue was purified by silica gel column chromatography with an eluant (PE: EA=1/1) to afford compound 41-3. MS m/z (ESI): 614.4[M+1].

Step 4: Compound 41-3 (90.0 mg, 0.15 mmol) was dissolved in TFA (2 mL). TfOH (1.3 µL) was added. The reaction was carried out at room temperature for 1 hour. The reaction solution was added with methanol (10 mL), and concentrated under reduced pressure. The remaining solution was added with EA (20 mL), neutralized with saturated sodium bicarbonate solution to pH=8. The aqueous phase was extracted with EA (20 mL×3). The organic phases were combined, and dried over anhydrous sodium sulfate. After filtering to remove the desiccant, the filtrate was concentrated under reduced pressure. The residue was purified by C18 column chromatography (Waters-2545, chromatographic column: Santai C18 (SW-8201-040-IR), 40-63 µm, 60 Å, 55 g; mobile phase: water (containing 10 mmol/L ammonium bicarbonate) and acetonitrile, gradient ratio: acetonitrile 38%-45%, flow rate: 40 mL/min) to afford compound 41. MS m/z (ESI):494.2[M+1]. ¹H NMR (400 MHz, DMSO) δ 12.44 (s, 1H), 8.61 (s, 1H), 7.93 (d, J = 2.6 Hz, 5H), 7.68 (s, 1H), 6.34 (s, 1H), 3.97 (s, 1H), 3.25 (d, J = 5.4 Hz, 2H), 2.84 (t, J = 7.4 Hz, 2H), 1.66 (dd, J = 14.5, 7.3 Hz, 3H), 1.53 (s, 3H), 1.17 (d, J = 6.2 Hz, 3H), 0.94 (t, J = 7.3 Hz, 3H).

### Example 42

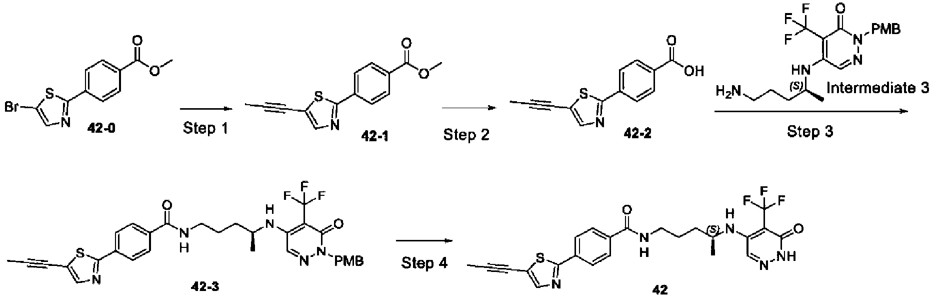

Step 1: Compound 42-0 (300 mg, 1.01 mmol, Bide), tributyl(prop-1-alkynyl)-λ4-stannane (306.63 µL, 1.01 mmol, Bide), and Pd(PPh₃)₄ (116.28 mg, 0.10 mmol) were added to DMF (20 mL). The resulting mixture was stirred at 120°C for 16 hours under nitrogen protection. The reaction solution was added with water (50 mL), followed by extraction with EA (10 mL×3). The organic phases were combined. The organic phases were washed with saturated saline (20 mL×3), and dried over anhydrous sodium sulfate. After filtering to remove the desiccant, the filtrate was concentrated under reduced pressure. The residue was purified by silica gel column chromatography with an eluant (PE: EA=5/1) to afford compound 42-1. MS m/z (ESI):258.0 [M+1].

Step 2: Compound 42-1 (200 mg, 0.78 mmol) and LiOH (65.23 g, 1.55 mmol) were added to a mixed solution of MeOH (20 mL) and water (10 mL), followed by stirring at room temperature for another 16 hours. The reaction solution was added with water (50 mL), followed by extraction with EA (10 mL×3). The organic phases were combined. The organic phases were washed with saturated saline (20 mL×3), and dried over anhydrous sodium sulfate. After filtering to remove the desiccant, the filtrate was concentrated under reduced pressure. The residue was purified by silica gel column chromatography with an eluant (DCM: MeOH=10/1) to afford compound 42-2. MS m/z (ESI): 244.0[M+1].

Step 3: Compound 42-2 (50 mg, 0.21 mmol) was added to MeCN (4 mL). TCFH (57.67 mg, 0.21 mmol), N- methylimidazole (81.92 µL, 1.03 mmol), and intermediate 3 (102.15 mg, 0.27 mmol) were added. The reaction was carried out at room temperature for 16 hours. The reaction solution was added with water (10mL), followed by extraction with EA (10 mL×3). The organic phases were combined. The organic phases were washed with saturated saline (20 mL×3), and dried over anhydrous sodium sulfate. After filtering to remove the desiccant, the filtrate was concentrated under reduced pressure. The residue was purified by silica gel column chromatography with an eluant (PE: EA=1/1) to afford compound 42-3. MS m/z (ESI): 610.4[M+1].

Step 4: Compound 42-3 (50 mg, 0.08 mmol) was added to TFA (2 mL), and trifluoromethanesulfonic acid (0.73 µL, 0.01 mmol) was added. The resulting mixture was stirred at room temperature for another 2 hours. The reaction solution was added with methanol (10 mL), and concentrated under reduced pressure. The remaining solution was added with EA (20 mL), neutralized with saturated sodium bicarbonate solution to pH=8. The aqueous phase was extracted with EA (20 mL×3). The organic phases were combined, and dried over anhydrous sodium sulfate. After filtering to remove the desiccant, the filtrate was concentrated under reduced pressure. The residue was purified by C18 column chromatography (Waters-2545, chromatographic column: Santai C18 (SW-8201-040-IR), 40-63 µm, 60 Å, 55 g; mobile phase: water (containing 10mmol/L ammonium bicarbonate) and acetonitrile, gradient ratio: acetonitrile 38%-45%, flow rate: 40 mL/min) to afford compound 42. MS m/z (ESI):490.2[M+1]. ¹H NMR (400 MHz, DMSO) δ 12.42 (s, 1H), 8.59 (s, 1H), 8.17 - 7.73 (m, 6H), 6.32 (s, 1H), 3.97 (s, 1H), 3.27 (s, 2H), 2.14 (s, 3H), 1.54 (s, 4H), 1.18 (d, J = 6.1 Hz, 3H).

### Example 43

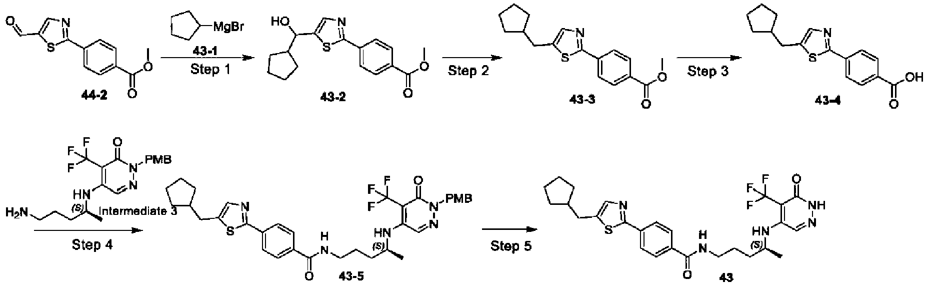

Step 1: At room temperature, compound 44-2 (0.5 g, 2.02 mmol) was dissolved in THF (5 mL). The temperature was reduced to -20°C. Compound 43-1 (3.03 mL, 3.04 mmol, Bide) was added dropwise to the reaction solution. After reacting at room temperature for 2 hours, the reaction was completed. After the reaction was completed, the crude was afforded by concentration under reduced pressure. The crude was purified by silica gel column (eluant: EA: PE = 25%-33%) to afford compound 43-2. MS m/z (ESI): 318.1 [M+1].

Step 2: At room temperature, compound 43-2 (0.170 g, 0.53 mmol) was dissolved in TFA (1 mL). Triethoxysilane (0.615 g, 5.3 mmol) was added to the reaction solution. After reacting at 100°C for 18 hours, the reaction was completed. After the reaction was completed, the crude was afforded by concentration under reduced pressure. The crude was purified by silica gel column (eluant: EA: PE = 90%-100%) to afford compound 43-3. MS m/z (ESI): 302.2 [M+1].

Step 3: At room temperature, compound 43-3 (0.180 g, 0.6 mmol) was dissolved in THF (2 mL), methanol (2 mL), and water (2 mL) in a 25 mL single-necked flask. LiOH (0.029 g, 1.2 mmol) was added to the reaction solution. After reacting at room temperature for 2 hours, the reaction was completed. After the reaction was completed, the crude was afforded by concentration under reduced pressure. The crude was dissolved in water. The pH value was adjusted to 4-5 with 1M hydrochloric acid solution. After filtering, the filter cake was dried under reduced pressure to afford compound 43-4. MS m/z (ESI): 302.2 [M+1].

Step 4: At room temperature, intermediate 3 (0.147 g, 0.38 mmol) and compound 43-4 (0.110 g, 0.38 mmol) were dissolved in acetonitrile (2 mL). TCFH (0.160 g, 0.57 mmol) and N-methylimidazole (0.091 mL, 1.14 mmol) were added to the reaction solution. After reacting at room temperature for 18 hours, the reaction was completed. After the reaction was completed, the crude was afforded by concentration under reduced pressure. The crude was purified by silica gel column (eluant: EA: PE = 90%-100%) to afford compound 43-5. MS m/z (ESI): 654.3 [M+1].

Step 5: At room temperature, compound 43-5 (0.250 g, 0.38 mmol) was dissolved in TFA (1 mL). Then, TfOH (0.1 mL) was added, followed by stirring at 25°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 50%-95%, flow rate: 20 mL/min) to afford compound 43. MS m/z (ESI):534.2[M+1]. ¹H NMR (400 MHz, DMSO-*d6) δ* 12.43 (s, 1H), 8.56 (t, *J =* 5.2 Hz, 1H), 7.99 - 7.89 (m, 5H), 7.68 (s, 1H),6.38-6.25 (m,1H),4.05- 3.90 (m, 1H), 3.29 - 3.24 (m, 2H), 2.87 (d, *J* = 7.4 Hz, 2H), 2.18 - 2.06 (m,1H),1.79-1.51 (m,10H),1.28-1.21 (m,2H),1.19 (d, *J =* 6.4 Hz, 3H).

### Example 44

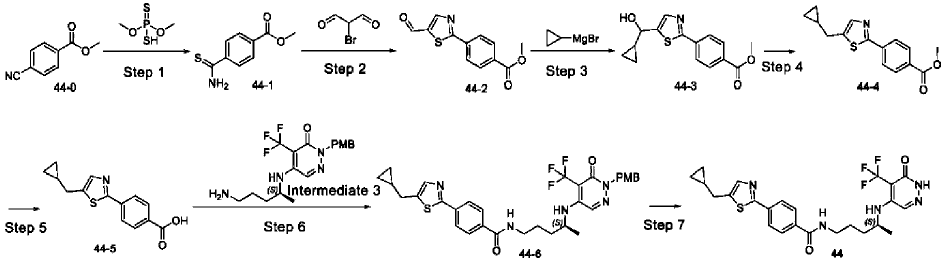

Step 1: Compound 44-0 (3 g, 18.62 mmol, Bide) was added to water (60 mL). Dimethylphosphorodithioate (4.57 mL, 37.24 mmol) was added. The reaction was carried out at 80°C for 18 hours. The reaction solution was filtered to afford the mixture. The filter cake was washed with water (30 mL). The resulting solid was blown dry under nitrogen at normal temperature to afford compound 44-1. MS m/z (ESI):196.0[M+1].

Step 2: Compound 44-1 (2.7 g, 13.97 mmol) and 2-bromomalondialdehyde were dissolved in THF (30 mL). Sodium bicarbonate (3.52 g, 41.92 mmol) was added. The reaction was carried out at 50°C for 6 hours. The reaction solution was concentrated under reduced pressure. The residue was added with a mixed solution of PE: EA=1:1 (50 mL) and water (50 mL), and slurried for 1 hour, followed by filtering, and washing with water (10 mL×2). The resulting solid was blown dry under nitrogen at normal temperature to afford compound 44-2. MS m/z (ESI):248.0[M+1].

Step 3: Compound 44-2 (500 mg, 2.02 mmol) was dissolved in THF (5 mL). After reducing the temperature to -78°C, 1M of cyclopropylmagnesium bromide solution (4.04 mL, 4.04 mmol) was added dropwise under a nitrogen atmosphere. After the dropwise addition was completed, the resulting mixture was allowed to warm to room temperature and reacted for 2 hours. The reaction solution was poured into a saturated ammonium chloride solution (10 mL) , followed by extraction with EA (30 mL×3). The combined organic phase was washed with saturated brine (20 mL), dried over anhydrous sodium sulfate, and filtered. The filtrate was concentrated under reduced pressure and purified by silica gel column chromatography with an eluant system (PE: EA=10: 1 to 3: 2) to afford compound 44-3. MS m/z (ESI):290.2[M+1].

Step 4: Compound 44-3 (370 mg, 1.28 mmol) was dissolved in TFA (2 mL) and DCM (2 mL) solution. Triethylsilane (2.07 mL, 12.79 mmol) was added to the reaction solution. After reacting at room temperature for 3 hours, the reaction was completed. The reaction solution was poured into water (20 mL), followed by extraction with EA (30 mL×3). The combined organic phase was washed with saturated brine (20 mL), dried over anhydrous sodium sulfate, and filtered. The filtrate was concentrated under reduced pressure and purified by silica gel column chromatography with an eluant system (PE: EA=10: 1 to 5: 1) to afford compound 44-4. MS m/z (ESI):274.2[M+H].

Step 5: Compound 44-4 (300 mg, 1.10 mmol) was dissolved in THF (3 mL) and water (3 mL). LiOH (80.0 mg, 3.29 mmol) was added to the reaction solution. After reacting at room temperature for 18 hours, the reaction was completed. After the reaction was completed, the crude was afforded by concentration under reduced pressure. The crude was dissolved in water (5 mL). The pH value was adjusted to 4-5 with 1M hydrochloric acid solution. After filtering, the filter cake was blown dry under nitrogen at normal temperature to afford compound 44-5. MS m/z (ESI): 260.0[M+1].

Step 6: Compound 44-5 (100 mg, 0.39 mmol) was dissolved in acetonitrile (2 mL). Then, TCFH (162.3 mg, 0.58 mmol) and N-methylimidazole (92.22 µL, 1.16 mmol) were added sequentially. After stirring at room temperature for half an hour, intermediate 3 (148.2 mg, 0.39 mmol) was added. Under a nitrogen atmosphere, the reaction was carried out at room temperature for 16 hours. The reaction solution was concentrated under reduced pressure. The residue was added with water (10 mL), followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1 to pure EA) to afford compound 44-6. MS m/z (ESI):626.4[M+1].

Step 7: Compound 44-6 (100 mg, 0.16 mmol) was dissolved in TFA (2 mL), TfOH (15 µL, 0.16 mmol) was added, and the reaction was carried out at 80°C for 1 hour. The reaction solution was concentrated under reduced pressure and purified by high performance liquid chromatography (Waters-2545, chromatographic column: SharpSil-T C18,30×150 mm, 5 µm; mobile phase: water (containing 10 mmol/L ammonium bicarbonate) and acetonitrile, gradient ratio: acetonitrile 38%-45%, flow rate: 30 mL/min) to afford compound 44. MS m/z (ESI): 506.2[M+1]. ¹H NMR (400 MHz, DMSO) δ 12.40 (s, 1H), 8.56 (t, *J =* 5.6 Hz, 1H), 8.05 - 7.84 (m, 5H), 7.72 (s, 1H), 6.47 - 6.19 (m, 1H), 4.10 - 3.88 (m, 1H), 3.27 (dd, *J* = 6.4, 5.8 Hz, 2H), 2.79 (d, *J* = 7.0 Hz, 2H), 1.76 - 1.48 (m, 4H), 1.19 (d, *J* = 6.4 Hz, 3H), 1.12 - 0.99 (m, 1H), 0.61 - 0.51 (m, 2H), 0.35 - 0.24 (m, 2H).

### Example 45

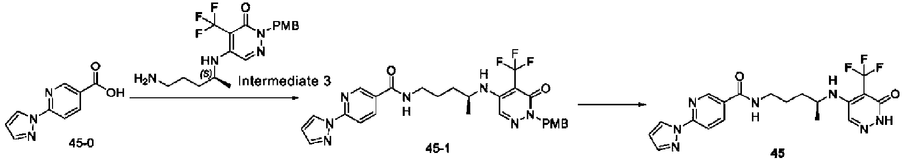

Step 1: Compound 45-0 (100 mg, 0.53 mol, Bide), TCFH (743 mg, 2.65 mmol), and N-methylimidazole (87 mg, 1.06 mmol) were dissolved in 3 mL DMF. At normal temperature, intermediate 3 (361 mg, 1.58 mmol) was added, followed by reacting under stirring for 12 hours while maintaining the temperature. The reaction solution was added with 30 mL water, followed by extraction with DCM (30 mL×3). The organic phases were combined, and washed with saturated saline solution. The organic phases were dried over anhydrous sodium sulfate. After filtering to remove the desiccant, the filtrate was concentrated under reduced pressure. The residue was purified by silica gel column chromatography with an eluant system (PE: EA=1:1) to afford compound 45-1. MS m/z (ESI):556.56[M+1].

Step 2: Compound 45-1 (400 mg, 0.14 mmol) was dissolved in TFA (5 mL). Several drops of TfOH were added until the reaction solution turned purple-red, followed by reacting under stirring at 25°C for 1 hour. The reaction solution was cooled to room temperature and concentrated under reduced pressure. The residue was added with saturated sodium bicarbonate solution to adjust pH=7. 30 mL water was added, followed by extraction with DCM (30 mL×3). The organic phases were combined, and washed with saturated saline solution. The organic phases were dried over anhydrous sodium sulfate. After filtering to remove the desiccant, the filtrate was concentrated under reduced pressure. The residue was purified by high performance liquid chromatography (Waters-2545, chromatographic column: SharpSil-T C18, 30×150mm, 5 µm; mobile phase: water (containing 10 mmol/L ammonium bicarbonate) and acetonitrile, gradient ratio: acetonitrile 38%-45%, flow rate: 30 mL/min) to afford compound 45. MS m/z (ESI):436.41[M+1]. ¹H NMR (400 MHz, DMSO) δ 8.88 (d, *J* = 1.6 Hz, 1H), 8.75 - 8.65 (m, 2H), 8.37 (dd, *J =* 8.6, 2.1 Hz, 1H), 8.00 (d, *J =* 8.6 Hz, 1H), 7.91 (d, *J =* 21.3 Hz, 2H), 6.62 (s, 1H), 6.37 (d, *J =* 4.5 Hz, 1H), 3.99 (s, 1H), 3.29 (d, *J =* 5.2 Hz, 2H), 1.76 - 1.66 (m, 1H), 1.56 (s, 3H), 1.19 (d, *J* = 6.3 Hz, 3H).

### Example 46:

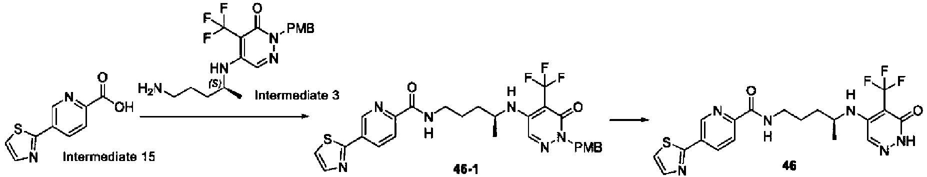

Step 1: Intermediate 15 (118 mg, 0.29 mol), HATU (148 mg, 0.39 mmol), and DIPEA (101 mg, 0.78mmol) were dissolved in 3 mL DMF. At normal temperature, intermediate 3 (53.6 mg, 0.26 mmol) was added, followed by reacting under stirring for 12 hours while maintaining the temperature. The reaction solution was added with 30 mL water, followed by extraction with DCM (30 mL×3). The organic phases were combined, and washed with saturated saline solution. The organic phases were dried over anhydrous sodium sulfate. After filtering to remove the desiccant, the filtrate was concentrated under reduced pressure. The residue was purified by silica gel column chromatography with an eluant system (PE: EA=2:1) to afford compound 46-1. MS m/z (ESI):573.6[M+1].

Step 2: Compound 46-1 (147 mg, 0.26 mmol) was dissolved in TFA (5 mL). Several drops of TfOH were added until the reaction solution turned purple-red, followed by reacting under stirring at 25°C for 1 hour. The reaction solution was cooled to room temperature and concentrated under reduced pressure. The residue was added with saturated sodium bicarbonate solution to adjust pH=7. 30 mL water was added, followed by extraction with DCM (30 mL×3). The organic phases were combined, and washed with saturated saline solution. The organic phases were dried over anhydrous sodium sulfate. After filtering to remove the desiccant, the filtrate was concentrated under reduced pressure. The residue was purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 46. MS m/z (ESI):436.41[M+1]. ¹H NMR (400 MHz, DMSO) δ 9.17 (s, 1H), 8.94 (s, 1H), 8.48 (d, *J* = 8.2 Hz, 1H), 8.14 (d, *J* = 8.1 Hz, 1H), 8.06 (d, *J* = 3.1 Hz, 1H), 7.97 (d, *J* = 3.1 Hz, 1H), 7.92 (s, 1H), 6.33 (s, 1H), 3.99 (s, 1H), 3.33 (s, 2H), 2.67 (s, 1H), 1.71 - 1.47 (m, 4H), 1.18 (d, *J* = 6.2 Hz, 3H).

### Example 47

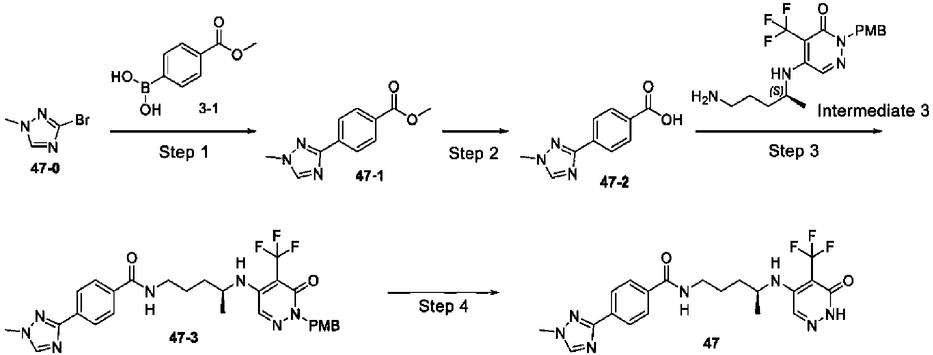

Step 1: Compound 47-0 (500 mg, 3.09 mmol), compound 3-1 (555.50 mg, 3.09 mmol), 1,1-bis(diphenylphosphino)ferrocenedichloropalladium (112.93 mg, 0.15 mmol), and sodium carbonate (981.45 mg, 9.26 mmol) were added to a mixed solution of 1,4-dioxane (10 mL) and water (0.2 mL), followed by stirring at 90°C for 3 hours under nitrogen protection. The reaction solution was added with water (10 mL), followed by extraction with EA (10 mL×3). The organic phases were combined. The organic phases were washed with saturated saline (20 mL×3), and dried over anhydrous sodium sulfate. After filtering to remove the desiccant, the filtrate was concentrated under reduced pressure. The residue was purified by silica gel column chromatography with an eluant (DCM: MeOH=10/1) to afford compound 47-1. MS m/z (ESI):218.2 [M+1].

Step 2: Compound 47-1 (340 mg, 1.57 mmol) and NaOH (313.03 mg, 7.83 mmol) were added to a mixed solution of methanol (15 mL) and water (3 mL), followed by stirring at normal temperature for 3 hours. pH was adjusted to 1 with hydrochloric acid (1M), followed by filtering through celite, and rinsing with acetonitrile (10 mL×3). The liquid was evaporated to dryness to afford compound 47-2 (560 mg). MS m/z (ESI): 195.0[M+1].

Step 3: Compound 47-2 (60.0 mg, 0.30 mmol) was dissolved in DMF (2 mL), and TCFH (165.70 mg, 0.59 mmol) and N-methylimidazole (0.12 mL, 1.48 mmol) were added. The reaction was carried out at 20°C for 0.5 hours. Intermediate 3 (102.15 mg, 0.27 mmol) was added, and the reaction was carried out at 20°C for 16 hours. The reaction solution was added with water (10 mL), followed by extraction with EA (10 mL×3). The organic phases were combined. The organic phases were washed with saturated saline (20 mL×3), and dried over anhydrous sodium sulfate. After filtering to remove the desiccant, the filtrate was concentrated under reduced pressure. The residue was purified by silica gel column chromatography with an eluant (DCM: MeOH=20/1) to afford compound 47-3. MS m/z (ESI): 570.4[M+1].

Step 4: Compound 47-3 (80.0 mg, 0.14 mmol) was dissolved in TFA (1 mL). Methanesulfonic acid (0.1 mL) was added. The reaction was carried out at 20°C for 1 hour. The reaction solution was added with methanol (10 mL), and concentrated under reduced pressure. The remaining solution was added with EA (20 mL), neutralized with saturated sodium bicarbonate solution to pH=8. The aqueous phase was extracted with EA (20 mL×3). The organic phases were combined, and dried over anhydrous sodium sulfate. After filtering to remove the desiccant, the filtrate was concentrated under reduced pressure. The residue was purified by preparative to afford compound 47. MS m/z (ESI):450.2[M+1]. ¹H NMR (400 MHz, DMSO) δ 12.40 (s, 1H), 8.52 (t, J = 5.5 Hz, 1H), 8.05 (d, J = 8.4 Hz, 2H), 7.91 (d, J = 8.6 Hz, 3H), 6.29 (s, 1H), 3.96 (s, 1H), 3.94 (s, 3H), 3.27 (d, J = 6.3 Hz, 2H), 1.70-1.51 (m, 4H), 1.19 (d, J = 6.3 Hz, 3H).

### Example 48

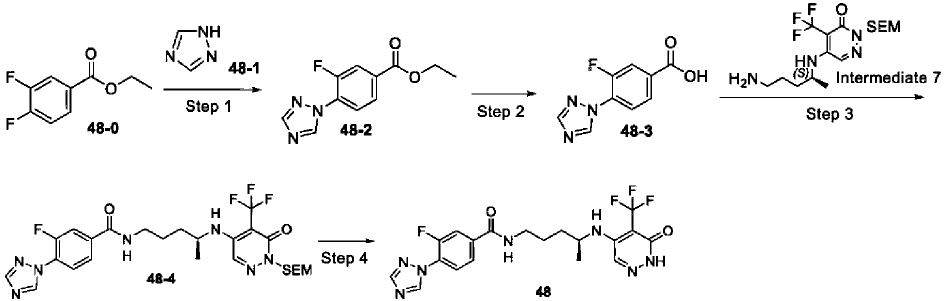

Step 1: Compound 48-0 (1 g, 5.37 mmol, Bide) was dissolved in N,N-dimethylformamide (5 mL) solution, and compound 48-1 (0.37 g, 5.37 mol, Bide) and potassium carbonate (1.48 g, 10.74 mmol) were added. The mixture was heated at 100°C for 30 minutes. After cooling to room temperature, water was added, followed by extraction with EA. The organic phases were dried over anhydrous sodium sulfate. After filtering to remove the desiccant, the filtrate was concentrated under reduced pressure. The residue was purified by silica gel column chromatography with an eluant system PE/EA to afford compound 48-2. MS m/z (ESI): 236.0 [M+1]. ¹H NMR (400 MHz, DMSO) δ 9.15 (d, J = 2.7 Hz, 1H), 8.38 (s, 1H), 8.05 - 7.96 (m, 3H), 4.37 (q, J = 7.1 Hz, 2H), 1.35 (t, J = 7.1 Hz, 3H).

Step 2: Compound 48-2 (300 mg, 1.28 mmol) was dissolved in methanol (5 mL) and water (1 mL), and sodium hydroxide (255.08 mg, 6.38 mmol) was added. The mixture was stirred at room temperature for 1h. The mixture was neutralized with 6M hydrochloric acid aqueous solution, concentrated, and then acidified with 6M HCl aqueous solution. The precipitate was filtered off, washed with a small amount of water, and dried by suction to afford compound 48-3. MS m/z (ESI): 208.0 [M+1]. ¹H NMR (400 MHz, DMSO) δ 13.60 (s, 1H), 9.14 (d, J = 2.6 Hz, 1H), 8.38 (s, 1H), 8.02 - 7.94 (m, 3H).

Step 3: Compound 48-3 (30 mg, 0.14 mmol), intermediate 7 (57.13 mg, 0.14 mmol), HATU (71.58 mg, 0.19 mmol), and DIPEA (0.05 mL, 0.29 mmol) were dissolved in DMF (2 mL) solution. The mixture was stirred at room temperature for 3 hours. The reaction mixture was diluted with EA, followed by adding water, extraction with EA, and washing with saturated saline. The organic phases were dried over anhydrous sodium sulfate. After filtering to remove the desiccant, the filtrate was concentrated under reduced pressure. The residue was purified by silica gel column chromatography with an eluant system DCM/methanol to afford compound 48-4. MS m/z (ESI): 584.3 [M+1].

Step 4: Compound 48-4 (46 mg, 0.08 mmol) was dissolved in a solution of 4.0 M hydrochloric acid in 1,4-dioxane (5 mL). The mixture was stirred at room temperature for 4 hours. The mixture was concentrated under reduced pressure and purified by high performance liquid chromatography to afford compound 48. MS m/z (ESI): 454.2 [M+1]. ¹H NMR (400 MHz, DMSO) δ 12.45 (s, 1H), 9.10 (d, J = 2.4 Hz, 1H), 8.72 (t, J = 5.5 Hz, 1H), 8.35 (s, 1H), 7.94 (t, J = 8.0 Hz, 3H), 7.89 - 7.85 (m, 1H), 6.35 (s, 1H), 3.98 (s, 1H), 3.28 (d, J = 6.3 Hz, 2H), 1.62 (d, J = 48.0 Hz, 4H), 1.18 (t, J = 6.8 Hz, 3H).

### Example 49

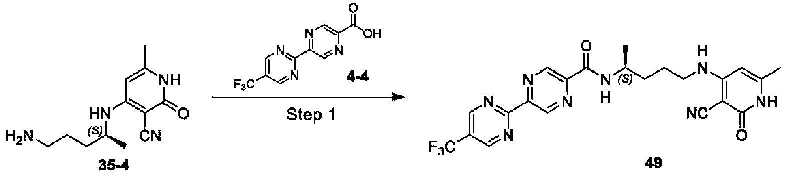

At room temperature, DIPEA (99 mg, 0.77 mmol) and HATU (117 mg, 0.31 mmol) were added to a solution of compound 4-4 (69 mg, 0.26 mmol) and compound 35-4 (60 mg, 0.26 mmol) in DMF (2 mL). The reaction solution was stirred at room temperature for 18 h. After the reaction was completed, the crude product was purified by preparative HPLC (chromatographic column: Waters-SunFire-C18-10µm-19×250mm; mobile phase: A: 0.1% formic acid/water, B: acetonitrile, gradient ratio: acetonitrile 0-32%, flow rate: 25 mL/min) to afford compound 49. MS m/z (ESI): 487.2 [M+1]. ¹H NMR (400 MHz, DMSO) δ 11.08 - 10.93 (m, 1H), 9.62 (d, *J =* 1.4 Hz, 1H), 9.54 (s, 2H), 9.38 (d, *J =* 1.4 Hz, 1H), 8.92 (d, *J =* 8.8 Hz, 1H), 7.38 - 6.94 (m, 1H), 5.76 (s, 1H), 4.28 - 3.95 (m, 1H), 3.30 - 3.10 (m, 2H), 2.07 (s, 3H), 1.72 - 1.45 (m, 4H), 1.21 (d, *J =* 6.6 Hz, 3H).

### Example 50

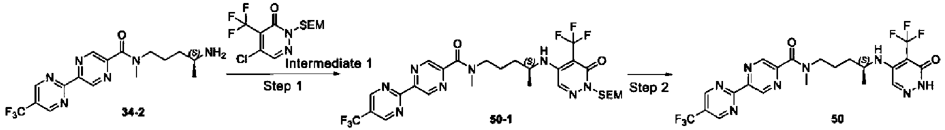

Step 1: At room temperature, DIPEA (59 mg, 0.46 mmol) was added to a solution of intermediate 1 (50 mg, 0.15 mmol) and compound 34-2 (56 mg, 0.15 mmol) in isopropanol (2 mL), followed by heating to 80°C and stirring for 18 h. After the reaction was completed, the mixture was concentrated under reduced pressure to afford the crude compound. The crude compound was purified by silica gel plate (eluant: EA: PE = 25%-75%) to afford compound 50-1. MS m/z (ESI): 369.1 [M+1-100].

Step 2: At room temperature, a solution of hydrochloric acid (1 mL, 4.0 mmol) in EA was added to a solution of compound 50-1 (30 mg, 0.04 mmol) in EA (1 mL). The reaction solution was stirred at room temperature for 18 hours. After the reaction was completed, the crude compound was afforded by concentration under reduced pressure. The crude compound was purified by preparative HPLC (chromatographic column: Waters-Xbridge-C18-10pum-19×250mm; mobile phase: A: 10 mM ammonium bicarbonate/water, B: acetonitrile, gradient ratio: acetonitrile 27-57%, flow rate: 20 mL/min) to afford compound 50. MS m/z (ESI): 531.1 [M+1]. ¹H NMR (400 MHz, DMSO) δ 12.43 (s, 1H), 9.60 - 9.46 (m, 3H), 9.03 (dd, *J* = 6.2, 1.3 Hz, 1H), 7.90 (d, *J =* 52.2 Hz, 1H), 6.50 - 6.22 (m, 1H), 4.10 - 3.79 (m, 1H), 3.53 (t, *J =* 6.6 Hz, 1H), 3.33 - 3.25 (m, 1H), 3.01 (d, *J =* 24.2 Hz, 3H), 1.78 - 1.32 (m, 4H), 1.17 (dd, *J* = 37.8, 6.3 Hz, 3H).

### Example 51

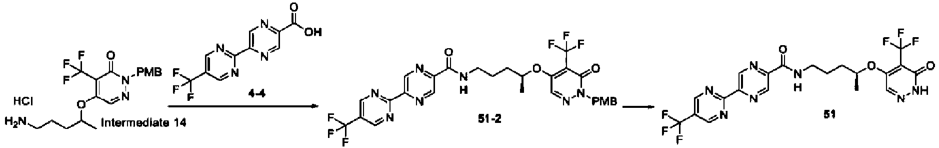

Step 1: Intermediate 14 (158 mg, 0.41 mol), TCFH (575 mg, 2.05 mmol, 5.0 eq), and N-methylimidazole (67 mg, 0.82 mmol) were dissolved in 4 mL DMF. At normal temperature, compound 4-4 (111 mg, 0.41 mmol) was added, followed by reacting under stirring for 12 hours while maintaining the temperature. The reaction solution was added with 30 mL water, followed by extraction three times with EA (30 mL×3). The organic phases were combined, and washed with saturated saline solution. The organic phases were dried over anhydrous sodium sulfate. After filtering to remove the desiccant, the filtrate was concentrated under reduced pressure. The residue was purified by silica gel column chromatography with an eluant system (PE: EA=1:1) to afford compound 51-2. MS m/z (ESI):638.54[M+1].

Step 2: Compound 51-2 (42 mg, 0.07 mmol) was dissolved in TFA (3 mL). Several drops of TfOH were added until the reaction solution turned purple-red, followed by reacting under stirring at 25°C for 1 hour. The reaction solution was cooled to room temperature and concentrated under reduced pressure. The residue was added with saturated sodium bicarbonate solution to adjust pH=7. 30 mL water was added, followed by extraction three times with DCM (20 mL×3). The organic phases were combined, and washed with saturated saline solution. The organic phases were dried over anhydrous sodium sulfate. After filtering to remove the desiccant, the filtrate was concentrated under reduced pressure. The residue was purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 51. MS m/z (ESI): 518.39[M+1]. ¹H NMR (400 MHz, DMSO) δ 13.29 - 12.92 (m, 1H), 9.60 (d, J = 1.4 Hz, 1H), 9.53 (s, 2H), 9.36 (d, J = 1.4 Hz, 1H), 9.18 (s, 1H), 8.27 (s, 1H), 5.03 (s, 1H), 3.37 (d, J = 6.3 Hz, 3H), 1.68 (dd, J = 13.2, 5.6 Hz, 4H), 1.30 (d, J = 6.0 Hz, 4H).

### Example 52

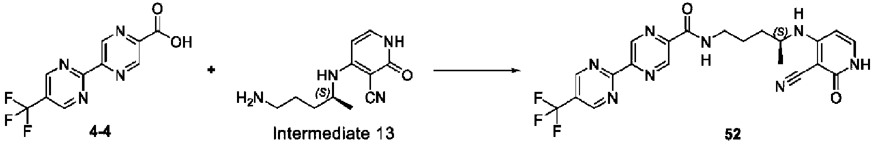

Compound 4-4 (95 mg, 0.43 mol), HATU (245.98 mg, 0.65 mmol, 1.5 eq), and DIPEA (167.22 mg, 1.29 mmol) were dissolved in 4 mL DMF. At normal temperature, compound intermediate 13 (116.52 mg, 0.43 mmol) was added. After reacting under stirring for 12 hours while maintaining the temperature. 30 mL water was added to the reaction solution, followed by extraction three times with EA (30 mL×3). The organic phases were combined, and washed with saturated saline solution. The organic phases were dried over anhydrous sodium sulfate. After filtering to remove the desiccant, the filtrate was concentrated under reduced pressure. The residue was purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 52. MS m/z (ESI):473.43[M+1]. ¹H NMR (400 MHz, DMSO) δ 10.98 (s, 1H), 9.60 (d, J = 1.3 Hz, 1H), 9.53 (s, 2H), 9.37 (d, J = 1.3 Hz, 1H), 9.15 (t, J = 6.0 Hz, 1H), 7.27 (s, 1H), 6.63 (s, 1H), 5.93 (s, 1H), 3.82 (s, 1H), 3.34 (d, J = 6.3 Hz, 2H), 1.59 (t, J = 27.1 Hz, 4H), 1.16 (d, J = 6.3 Hz, 3H).

### Example 53

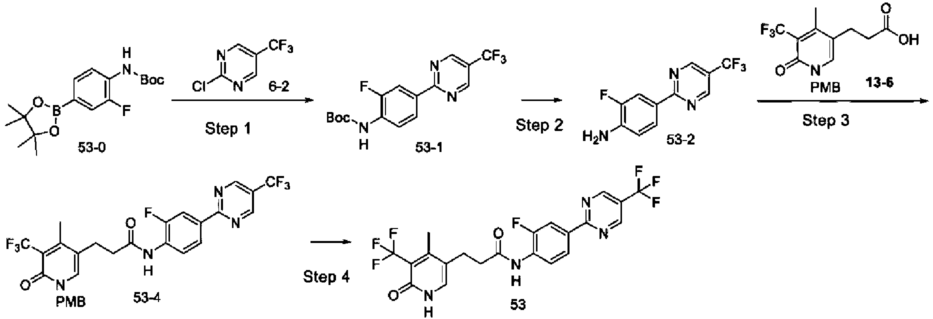

Step 1: Compound 53-0 (250 mg, 0.74 mmol, Bide), compound 6-2 (135.33 mg, 0.74 mmol), and cesium carbonate (483.13 mg, 1.48 mmol) were added to a solution of 1,4-dioxane (2 mL) and water (0.4 mL). Bis[5-(diphenylphosphino)cyclopentyl-1,3-dienyl]-λ2-iron(II) dichloromethane palladium chloride (121.1 mg, 0.15 mmol) was added. The mixture was purged with nitrogen three times, and the reaction was carried out at 100°C for 18 hours. The resulting mixture was concentrated under reduced pressure and purified by silica gel chromatography (elution solution: PE: EA = 3: 1 to 1: 1) to afford compound 53-1. MS m/z (ESI):358.2[M+1].

Step 2: Compound 53-1 (100 mg, 0.28 mmol) was dissolved in DCM (1 mL), and TFA (1 mL) was added. The reaction was carried out at room temperature for 1 hour. The reaction solution was concentrated under reduced pressure to afford compound 53-2.

Step 3: Compound 53-2 (72 mg, 0.28 mmol) was dissolved in acetonitrile (2 mL). Then, TCFH (157 mg, 0.56 mmol) and N-methylimidazole (89.27 µL, 1.12 mmol) were added sequentially. After stirring at room temperature for half an hour, compound 13-6 (103.4 mg, 0.28 mmol) was added. Under a nitrogen atmosphere, the reaction was carried out at room temperature for 16 hours. The reaction solution was concentrated under reduced pressure. The residue was added with water (10 mL), followed by extraction with EA (5 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=2:1 to 1:1) to afford compound 53-4. MS m/z (ESI):609.2 [M+1].

Step 4: Compound 53-4 (100 mg, 0.16 mmol) was dissolved in TFA (2 mL), TfOH (15 µL, 0.3 mmol) was added, and the reaction was carried out at 80°C for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 53. MS m/z (ESI): 489.1[M+1]. ¹H NMR (400 MHz, DMSO) δ 11.96 (s, 1H), 10.01 (s, 1H), 9.34 (s, 2H), 8.27 (d, *J =* 3.2 Hz, 2H), 8.19 (d, *J =* 12.4 Hz, 1H), 7.41 (s, 1H), 2.76 (t, *J =* 7.2 Hz, 2H), 2.68 (t, *J* = 6.8 Hz, 2H), 2.38 - 2.28 (m, 3H).

### Example 54

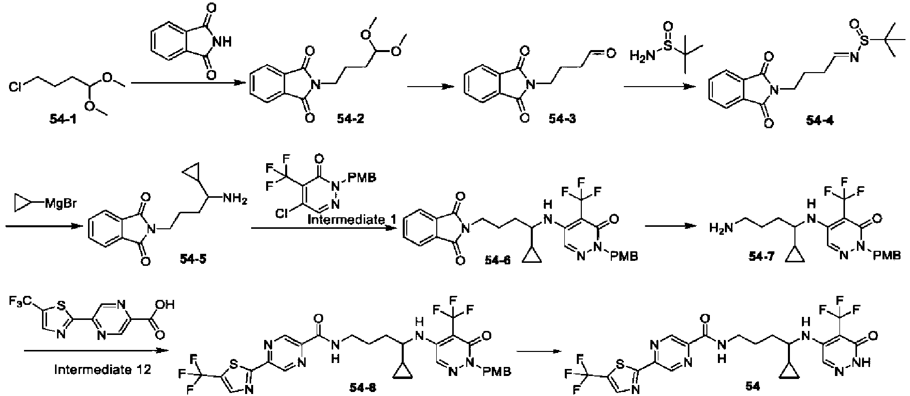

Step 1: Compound 54-1 (23.2 g, 0.153 mol, Bide) and phthalimide (22.4 g, 0.153 mol, Bide) were added to dimethyl sulfoxide (200 mL) solution. Potassium carbonate (21.1 g, 0.153 mol, Bide) and tetrabutylammonium bromide (4.92 g, 15.3 mmol, Bide) were added with stirring. The reaction solution was reacted at 80°C for 3 hours. The mixture was poured into water (500 mL), followed by extraction with EA (300 mL×3). The organic phases were combined, and washed with saturated saline. The organic phases were dried over anhydrous sodium sulfate. After filtering to remove the desiccant, the filtrate was concentrated under reduced pressure to afford compound 54-2. MS m/z(ESI): 286.0 [M+23].

Step 2: Compound 54-2 (38.0 g, 0.144 mol) was dissolved in THF (200 mL). Then, 4M hydrochloric acid (100 mL, 0.4 mol) was slowly added dropwise. After stirring at room temperature for 3 hours, saturated sodium bicarbonate solution was added for adjusting pH=7. The mixture was extracted with EA (200 mL×3). The organic phases were combined, and washed with saturated saline. The organic phases were dried over anhydrous sodium sulfate. After filtering to remove the desiccant, the filtrate was concentrated under reduced pressure to afford compound 54-3. MS m/z(ESI): 217.1 [M+1].

Step 3: Compound 54-3 (11.6 g, 53.4 mmol) was dissolved in THF (200 mL). Then, tert-butylsulfinamide (9.71 g, 80.1 mmol) and tetraethyl titanate (36.53 g, 180.2 mmol) were added. The reaction solution was reacted overnight at 65°C. After cooling, the reaction solution was poured into water (50 mL) and EA (500 mL). The mixed solid-liquid phase was filtered, the solid was washed 3-4 times with EA (200 mL), and the filtrates were combined and concentrated. The residue was purified by silica gel column chromatography with an eluant system (PE: EA=10:1-2:1) to afford compound 54-4. MS m/z(ESI): 321.1 [M+1].

Step 4: Compound 54-4 (6.20 g, 19.3 mmol) was dissolved in THF (100 mL). Then, at - 70°C, cyclopropylmagnesium bromide (29.0 mL, 29.0 mmol) was slowly added dropwise. The reaction solution was reacted at -70°C for half an hour, and allowed to warm to room temperature and reacted for another 2 hours. Then, 4M hydrochloric acid (10.0 mL, 40.0 mmol) was added to the reaction solution. The reaction solution was reacted at room temperature for 2 hours. After the reaction was completed, the reaction solution was concentrated. The residue was purified by reversed-phase preparative column (0.1M ammonium bicarbonate: acetonitrile =5-50%) to afford compound 54-5. MS m/z(ESI): 259.1 [M+1].

Step 5: Compound 54-5 (1.05 g, 4.08 mmol) was dissolved in IPA (20 mL). Then, intermediate 1 (1.30 g, 4.08 mmol) and DIPEA (2.64 g, 20.4 mmol) were added. The reaction solution was reacted overnight at 50°C. The reaction solution was concentrated. The residue was purified by silica gel column chromatography with an eluant system (PE: EA=10:1-3:1) to afford compound 54-6. MS m/z(ESI): 541.2 [M+1].

Step 6: Compound 54-6 (1.50 g, 2.78 mmol) was dissolved in ethanol (20 mL). Then, hydrazine hydrate (1.63 g, 27.7 mmol) was added. The reaction solution was reacted at room temperature for 3 hours. The reaction solution was concentrated. The residue was slurried with acetonitrile (20 mL) and purified to afford compound 54-7. MS m/z(ESI): 411.1 [M+1].

Step 7: At room temperature, 54-7 (246 mg, 0.60 mmol) and intermediate 12 (145 mg, 060 mmol) were dissolved in DMF (5 mL). HATU (273 mg, 0.72 mmol) and DIPEA (232 mg, 1.80 mmol) were added to the reaction solution. After reacting at room temperature for 18 hours, the reaction was completed. After the reaction was completed, water (3 mL) and EA (3 mL) were added to the reaction solution. The organic phases were washed with saturated saline (3 mL×3), and dried over sodium sulfate. After filtering, the filtrate was concentrated under reduced pressure to afford the crude. The crude was purified by silica gel chromatography (eluant: EA/PE = 0-33%) to afford compound 54-8. MS m/z(ESI): 668.2 [M+1].

Step 8: At room temperature, compound 54-8 (60 mg, 0.09 mmol) was dissolved in acetonitrile (1 mL) and water (1 mL). Then, cerium ammonium nitrate (148 mg, 0.27 mmol) was added, followed by stirring at 25°C for 18 hours until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 44%-**74**%, flow rate: 20 mL/min) to afford compound 54. MS m/z (ESI): 548.1 [M+1]. ¹H NMR (400 MHz, MeOD) δ 9.47 - 9.42 (m, 1H), 9.29 - 9.24 (m, 1H), 8.47 (s, 1H), 7.93 - 7.84 (m, 1H), 3.52 - 3.44 (m, 2H), 3.43 - 3.37 (m, 1H), 1.91 - 1.69 (m, 4H), 1.15 - 1.02 (m, 1H), 0.68 - 0.45 (m, 2H), 0.42 - 0.26 (m, 2H).

### Example 55

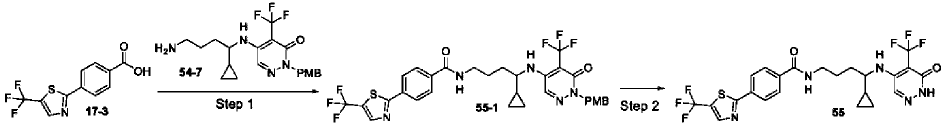

Step 1: At room temperature, compound 54-7 (0.090 g, 0.22 mmol) and compound 17-3 (0.060 g, 0.22 mmol) were dissolved in DMF (2 mL). HATU (0.125 g, 0.33 mmol) and DIPEA (0.116 mL, 0.66 mmol) were added to the reaction solution. After reacting at room temperature for 18 hours, the reaction was completed. After the reaction was completed, 3 mL water and 3 mL EA were added to the reaction solution. The organic phases were washed with saturated saline (3 mL×3), and dried over sodium sulfate. After filtering, the filtrate was concentrated under reduced pressure to afford the crude. The crude was purified by silica gel chromatography (eluant: EA/PE = 0-33%) to afford compound 55-1. MS m/z (ESI): 666.2 [M+1].

Step 2: At room temperature, compound 55-1 (0.030 g, 0.05 mmol) was dissolved in acetonitrile (1 mL) and water (1 mL). Then, cerium ammonium nitrate (0.027 g, 0.05 mmol) was added, followed by stirring at 25°C for 18 hours until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 55. MS m/z (ESI): 546.2 [M+1]. ¹H NMR (400 MHz, MeOD) *δ* 8.34 - 8.32 (m, 1H), 8.14 - 8.10 (m, 2H), 7.97 - 7.90 (m, 3H), 3.48 - 3.34 (m, 3H), 1.87 - 1.72 (m, 4H), 1.12 - 1.03 (m, 1H), 0.67 - 0.50 (m, 2H), 0.41 - 0.29 (m, 2H).

### Example 56

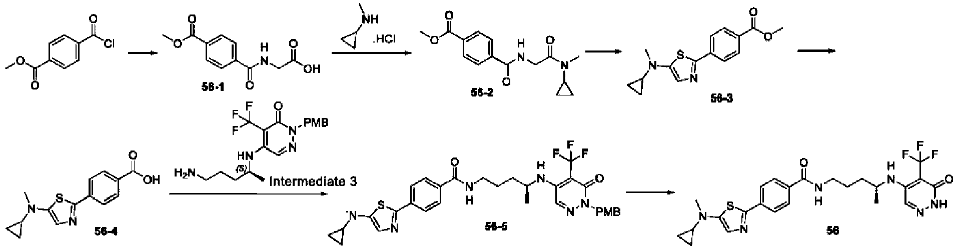

Step 1: Glycine (5 g, 5.21 mmol, 66.60 mmol) and NaHCO₃ (16.79g, 199.81 mmol) were dissolved in 250 mL water, and methyl 4-chlorocarbonylbenzoate (14.55 g, 73.26 mmol) was added. In a condition of nitrogen protection, the resulting mixture was reacted under stirring at room temperature for 5 hours. The reaction solution was concentrated. The residue was purified by silica gel column chromatography (DCM/methanol=20/1) to afford compound 56-1. MS m/z(ESI): 238.0 [M+1].

Step 2: Compound 56-1 (1.5 g, 6.32 mmol), compound N-methylcyclopropylamine hydrochloride (0.68 g, 6.32 mmol), HATU (3.60 g, 9.48 mmol), and DIEA (3.27 g, 25.29 mmol) were dissolved in 10 mL DMF, followed by reacting under stirring at 20°C for 4 hours. The reaction solution was extracted with water and EA system. The organic phases were collected. The organic phases was dried by spin and purified by silica gel column chromatography (DCM/methanol=30/1) to afford compound 56-2. MS m/z(ESI): 291.7 [M+1].

Step 3: Compound 56-2 (400 mg, 1.38 mmol) and Lawesson's reagent (668.70 mg, 1.65 mmol) were dissolved in toluene (4 mL), followed by reacting under stirring at 110°C for 3 hours. The reaction solution was concentrated and purified by silica gel column chromatography (DCM/EA=3/1) to afford compound 56-3. MS m/z(ESI): 289.7 [M+1].

Step 4: Compound 56-3 (330 mg, 1.14 mmol), LiOH (144.05 mg, 3.43 mmol), and methanol (3 mL), water (3 mL), THF (9 mL) were mixed, followed by stirring at room temperature for 16 hours. The pH of the reaction system was adjusted to 7 with 1M HCl, followed by extraction with EA. The organic phases were collected and concentrated to afford compound 56-4. MS m/z(ESI): 275.7 [M+1].

Step 5: Compound 56-4 (100 mg, 0.36 mg) and intermediate 3 (116.77 mg, 0.30 mmol), HOBt (61.57 mg, 0.46 mmol), EDCI (87.35 mL, 0.46 mmol), and DIEA (0.20 mL, 1.22 mmol) were dissolved in 5 mL DCM. The reaction was carried out at room temperature for 2 hours. The reaction solution was extracted with DCM and water. The organic phases were collected and concentrated. The concentrate was purified by silica gel column chromatography with an eluant system (PE/EA=0%-100%) to afford compound 56-5. MS m/z(ESI): 641.7 [M+1].

Step 6: Compound 56-5 (120 mg, 0.19 mmol) and TfOH (0.5 mL, 5.63 mmol) were dissolved in 6 mL TFA. The reaction was carried out at room temperature for 5 minutes. The reaction solution was quenched by adding methanol under stirring, concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile to afford compound 56. MS m/z(ESI): 521.7 [M+1]. ¹HNMR(400 MHz, DMSO) δ 12.43 (s, 1H), 8.50 (t, J = 5.4 Hz, 1H), 7.93 (s, 1H), 7.87 (d, J = 8.4 Hz, 2H), 7.81 (d, J = 8.5 Hz, 2H), 7.03 (s, 1H), 6.32 (d, J = 4.5 Hz, 1H), 3.98 (s, 1H), 3.29 - 3.24 (m,2H), 2.95 (s, 3H), 2.62 (td, J = 6.5, 3.4 Hz, 1H), 1.65 (d, J = 8.0 Hz, 1H), 1.55 (s, 3H), 1.19 (d, J = 6.3 Hz, 3H), 0.85 - 0.77 (m, 2H), 0.73 (d, J = 3.4 Hz, 2H).

### Example 57

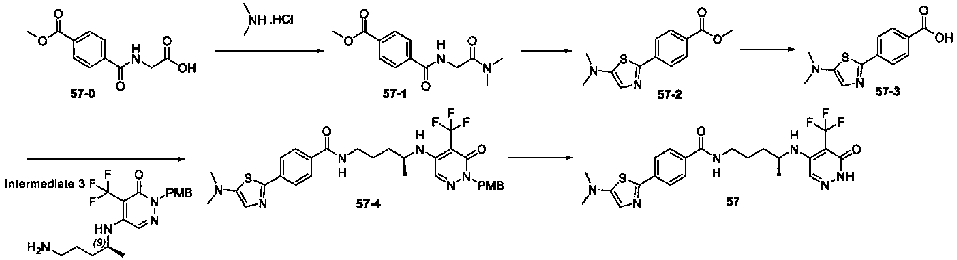

Step 1: Compound 57-0 (1 g, 4.22 mmol, ABClab), compound dimethylamine hydrochloride (0.69 g, 8.43 mmol), HOBt (0.85 g, 6.32 mmol), EDCI (1.21 mg, 6.32 mmol), and DIEA (3.27g, 25.29 mmol) were dissolved in 10 mL DCM, followed by reacting under stirring at 20°C for 4 hours. The reaction solution was extracted with water and DCM system. The organic phases were collected. The organic phases was dried by spin and purified by silica gel column chromatography (DCM/EA=3/1) to afford compound 57-1. MS m/z(ESI): 265.2 [M+1].

Step 2: Compound 57-1 (200 mg, 0.76 mmol) and phosphorus pentasulfide (336.39 mg, 1.51 mmol), molecular sieve(spherical) (200mg) were dissolved in chloroform (8 mL), followed by reacting under stirring at 60°C for 6 hours. The reaction solution was filtered and concentrated, and purified by silica gel column chromatography (DCM/EA=3/1) to afford compound 57-2. MS m/z(ESI): 263.7 [M+1].

Step 3: Compound 57-2 (200 mg, 0.76 mmol), LiOH (95.67mg, 2.28mmol), and methanol (3 mL), water (3 mL), THF (9 mL) were mixed, followed by stirring at room temperature for 16 hours. The pH of the reaction system was adjusted to 7 with 1M HCl, followed by extraction with EA. The organic phases were collected and concentrated to afford compound 57-3. MS m/z(ESI): 249.7 [M+1].

Step 4: Compound 57-3 (90 mg, 0.36 mg) and intermediate 3 (100 mg, 0.26 mmol), HATU (148.38 mg, 0.39 mmol), and DIEA (0.17mL, 1.04 mmol) were dissolved in 3 mL DMF, followed by reacting under stirring at room temperature for 1 hour. The reaction solution was extracted with EA and water. The organic phases were collected and concentrated. The concentrate was purified by silica gel column chromatography with an eluant system (DCM/EA=2/1) to afford compound 57-4. MS m/z(ESI): 615.7 [M+1].

Step 5: Compound 57-4 (100 mg, 0.16 mmol) and TfOH (0.5 mL, 5.63 mmol) were dissolved in 6 mL TFA. The reaction was carried out at room temperature for 2 minutes. The reaction solution was quenched by adding methanol under stirring, concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 57. MS m/z(ESI): 495.3 [M+1]. ¹H NMR (400 MHz, DMSO) δ 12.45 (s, 1H), 8.52 (t, J = 5.6 Hz, 1H), 7.94 (s, 1H), 7.86 (d, J = 8.5 Hz, 2H), 7.78 (d, J = 8.5 Hz, 2H), 6.92 (s, 1H), 6.36 (s,1H), 3.98 (s, 1H), 3.26 (d, J = 5.1 Hz, 2H), 2.96 (s, 6H), 1.70 - 1.44 (m, 4H), 1.18 (d, J = 6.3 Hz, 3H).

### Example 58

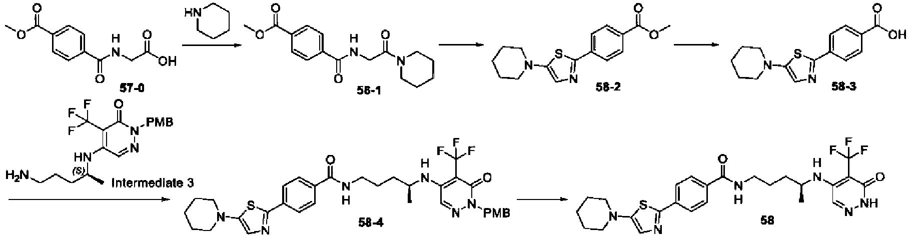

Step 1: Compound 57-0 (800.00 mg, 3.37 mmol) was dissolved in 10 mL DCM. EDC (1 g, 6.74 mmol), HOBT (1.42 g, 6.74 mmol), and DIEA (2.17 g, 15.9 mmol) were added. Then, after reacting under stirring at room temperature for 30 minutes, cyclohexylamine (430.76 mg, 5.06 mmol) was added, followed by reacting for another 2h. The reaction solution was concentrated. The residue was purified by alumina column chromatography (PE/EA=5/1) to afford compound 58-1. MS m/z(ESI): 305.2 [M+1].

Step 2: Compound 58-1 (450 mg, 2.10 mmol) was dissolved in 10 mL chloroform. Phosphorus pentasulfide (671.83 mg, 3.02 mmol) was added, followed by reacting under stirring at 60°C for 6 hours under nitrogen protection. The reaction solution was concentrated. The residue was purified by alumina column chromatography (PE/EA=1/1) to afford compound 58-2. MS m/z(ESI): 303.0 [M+1].

Step 3: Compound 58-2 (170 mg, 0.56 mmol) and LiOH (42.81 mg, 1.79 mmol) were dissolved in 3 mL methanol and 1 mL water. The reaction was carried out at room temperature for 16 hours. The reaction solution was concentrated under reduced pressure, neutralized with dilute hydrochloric acid, dried and concentrated to afford compound 58-3. MS m/z(ESI): 289.0 [M+1].

Step 4: Compound 58-3 (145 mg, 0.5 mmol) and intermediate 3 (88.29 mg, 0.23 mmol), HATU (145.56 mg, 0.38 mmol), and DIPEA (0.13 mL, 0.77 mmol) were dissolved in 6 mL DMF. The reaction was carried out at room temperature for 1 hour. The reaction solution was concentrated. The residue was purified by silica gel column chromatography with an eluant system (PE/EA=1/1) to afford compound 58-4. MS m/z(ESI): 655.2 [M+1].

Step 5: Compound 58-4 (120 mg, 0.18 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 58. MS m/z(ESI): 535.2 [M+1]. ¹H NMR (400 MHz, DMSO) δ 12.45 (s, 1H), 8.52 (t, J = 5.6 Hz, 1H), 7.94 (s, 1H), 7.87 (d, J = 8.5 Hz, 2H), 7.79 (d, J = 8.5 Hz, 2H), 7.11 (s, 1H), 6.36 (dd, J = 8.4, 3.3 Hz, 1H), 3.98 (s, 1H), 3.26 (d, J = 5.5 Hz, 2H), 1.65 (d, J = 5.2 Hz, 5H), 1.55 (d, J = 5.5 Hz, 5H), 1.18 (d, J = 6.3 Hz, 3H).

### Example 59

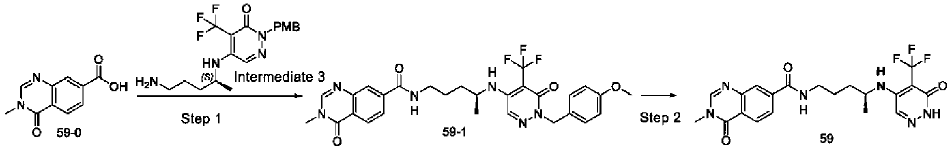

Step 1: Compound 59-0 (100 mg, 0.49 mmol) was dissolved in acetonitrile (2 mL). Then, TCFH (206.12 mg, 0.73 mmol) and N-methylimidazole (117.12 µL, 1.47 mmol) were added sequentially. After stirring at room temperature for 0.5 hours, intermediate 3 (188.26 mg, 0.49 mmol) was added. Under a nitrogen atmosphere, the reaction was carried out at room temperature for 16 hours. The reaction solution was concentrated under reduced pressure. The residue was added with 10 mL water, followed by extraction with EA(15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:3) to afford compound 59-1. MS m/z(ESI): 580.2 [M+1].

Step 2: Compound 59-1 (150 mg, 0.26 mmol) was dissolved in TFA (3 mL), and TfOH (15 µL, 0.15 mmol) was added. The reaction was carried out at 50°C for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 59. MS m/z(ESI): 451.1 [M+1]. ¹H NMR (400 MHz, DMSO) δ 12.42 (s, 1H), 8.77 (t, *J* = 5.4 Hz, 1H), 8.43 (s, 1H), 8.21 (d, *J* = 8.4 Hz, 1H), 8.15 - 8.07 (m, 1H), 7.99 - 7.89 (m, 2H), 6.43 - 6.22 (m, 1H), 4.07 - 3.90 (m, 1H), 3.51 (s, 3H), 3.30 - 3.26 (m, 2H), 1.76 - 1.49 (m, 4H), 1.19 (d, *J=* 6.4 Hz, 3H).

### Example 60

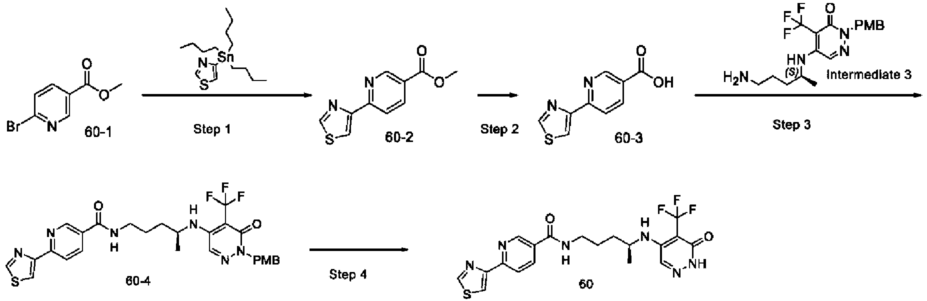

Step 1: Compound 60-1 (250 mg, 1.16 mmol, Bide) and 4-(tri-n-butylstannyl)thiazole (433.01 mg, 1.16 mmol) were dissolved in toluene (3 mL). Pd(PPh₃)₄ (66.87 mg, 0.06 mmol) was added, followed by purging with nitrogen three times. The reaction was carried out at 100°C for 18 hours. The reaction solution was concentrated under reduced pressure and purified by silica gel column chromatography with an eluant system (PE: EA=10:1-2:1) to afford compound 60-2. MS m/z(ESI): 221.0 [M+1].

Step 2: Compound 60-2 (250 mg, 1.14 mmol) was dissolved in methanol (3 mL) and water (3 mL), and LiOH (81.56 mg, 3.41 mmol) was added. The reaction was carried out at room temperature for 18 hours. The reaction solution was concentrated under reduced pressure. The residue was added with water (10 mL), adjusted to pH= 4-5 with 1M hydrochloric acid solution, filtered, and washed with water (5 mL). The filter cake was oven-dried to afford compound 60-3. MS m/z(ESI): 207.0 [M+1].

Step 3: Compound 60-3 (100 mg, 0.48 mmol) was dissolved in acetonitrile (2 mL). Then, TCFH (204.09 mg, 0.73 mmol) and N-methylimidazole (116 µL, 1.45 mmol) were added sequentially. After stirring at room temperature for 0.5 hours, intermediate 3 (186.4 mg, 0.48 mmol) was added. Under a nitrogen atmosphere, the reaction was carried out at room temperature for 16 hours. The reaction solution was concentrated under reduced pressure. The residue was added to water (10 mL), followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:3) to afford compound 60-4. MS m/z(ESI): 573.4 [M+1].

Step 4: Compound 60-4 (162 mg, 0.28 mmol) was dissolved in TFA (2 mL), TfOH (15 µL, 0.18 mmol) was added, and the reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 60. MS m/z(ESI): 453.1 [M+1]. ¹H NMR (400 MHz, DMSO) δ 12.43 (s, 1H), 9.26 (d, *J* = 2.0 Hz, 1H), 9.09 - 8.96 (m, 1H), 8.68 (t, *J =* 5.8 Hz, 1H), 8.45 (d, *J =* 2.0 Hz, 1H), 8.29 (dd, *J =* 8.2, 2.4 Hz, 1H), 8.22 - 8.12 (m, 1H), 7.93 (s, 1H), 6.46 - 6.19 (m, 1H), 3.99 (s, 1H), 3.30 - 3.24 (m, 2H), 1.81 - 1.46 (m, 4H), 1.19 (d, *J* = 6.4 Hz, 3H).

### Example 61

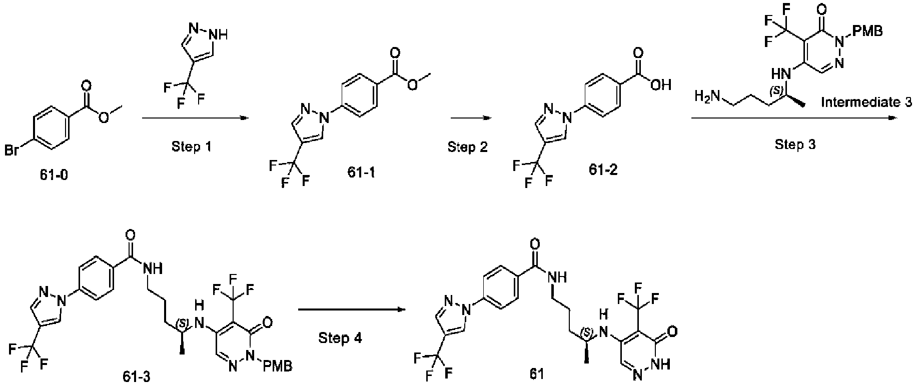

Step 1: Compound 61-0 (500 mg, 2.33 mmol, Bide), 4-(trifluoromethyl)-1H-pyrazole (317 mg, 2.33 mmol), L-proline (134 mg, 1.17 mmol), and potassium phosphate (741.85 mg, 3.49 mmol) were added to N,N-dimethylformamide (5 mL). Cuprous iodide (221.87 mg, 1.17 mmol) was added, followed by purging with nitrogen three times. The reaction was carried out at 80°C for 18 hours. The reaction solution was poured into water (20 mL), followed by extraction with EA (30 mL × 3). The combined organic phase was washed with brine (30 mL), dried over anhydrous sodium sulfate, and filtered. The filtrate was concentrated under reduced pressure and purified by silica gel chromatography (elution solution: PE: EA = 100: 1-10: 1) to afford compound 61-1. MS m/z(ESI): 271.0 [M+1].

Step 2: Compound 61-1 (235 mg, 0.87 mmol) was dissolved in methanol (3 mL) and water (3 mL), and LiOH (62.49 mg, 2.61 mmol) was added. The reaction was carried out at room temperature for 18 hours. The reaction solution was concentrated under reduced pressure. The residue was added with 10 mL water, adjusted to pH= 4-5 with 1M hydrochloric acid solution, filtered, and washed with water (5 mL). The filter cake was oven-dried to afford compound 61-2. MS m/z(ESI): 257.0 [M+1].

Step 3: Compound 61-2 (80 mg, 0.31 mmol) was dissolved in acetonitrile (2 mL). Then, TCFH (131.43 mg, 0.47 mmol) and N- methylimidazole (74.68 µL, 0.94 mmol) were added sequentially. After stirring at room temperature for 0.5 hours, intermediate 3 (120 mg, 0.31 mmol) was added. Under a nitrogen atmosphere, the reaction was carried out at room temperature for 16 hours. The reaction solution was concentrated under reduced pressure. The residue was added with 10 mL water, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:3) to afford compound 61-3. MS m/z(ESI): 623.4 [M+1].

Step 4: Compound 61-3 (127 mg, 0.20 mmol) was dissolved in TFA (2 mL), TfOH (15µL, 0.18 mmol) was added, and the reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 61. MS m/z(ESI): 503.1 [M+1]. ¹H NMR (400 MHz, DMSO) δ 12.45 (s, 1H), 9.30 (s, 1H), 8.59 (t, *J=* 5.6 Hz, 1H), 8.27 (s, 1H), 8.00 (s, 4H), 7.93 (s, 1H), 6.41 - 6.31 (m, 1H), 4.08 - 3.89 (m, 1H), 3.32 - 3.21 (m, 2H), 1.77 - 1.45 (m, 4H), 1.18 (d, *J =* 6.4 Hz, 3H).

### Example 62

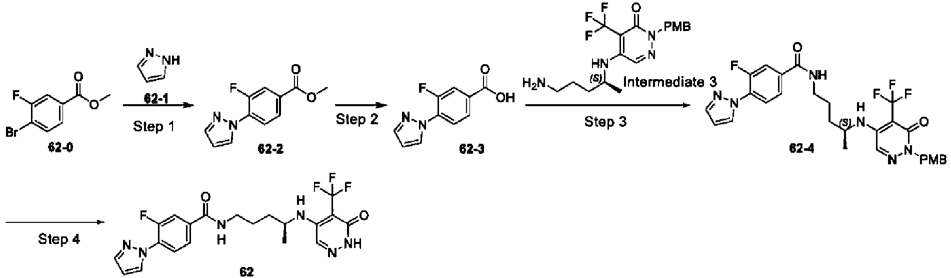

Step 1: At room temperature, compound 62-0 (0.8 g, 3.43 mmol) and compound 62-1 (0.23 g, 3.43 mmol) were dissolved in DMF (10 mL). Potassium phosphate (1.457 g, 6.87 mmol), L-proline (0.039 g, 0.34 mmol), and cuprous iodide (0.065 g, 0.34 mmol) were added to the reaction solution. After reacting at 100°C for 18 hours, the reaction was completed. After the reaction was completed, water (15 mL) and EA (15 mL) were added to the reaction solution. The organic phases were washed with saturated saline (15 mL×3), and dried over sodium sulfate. After filtering, the filtrate was concentrated under reduced pressure to afford the crude. The crude was purified by silica gel chromatography (eluant: EA/PE = 5%-10%) to afford compound 62-2. MS m/z (ESI): 221.1 [M+1].

Step 2: At room temperature, compound 62-2 (0.10 g, 0.45 mmol) was dissolved in THF (1 mL), methanol (1 mL), and water (1 mL) in a 25 mL single-necked flask. LiOH (0.022 g, 0.9 mmol) was added to the reaction solution. After reacting at room temperature for 2 hours, the reaction was completed. After the reaction was completed, the crude was afforded by concentration under reduced pressure. The crude was dissolved in water. The pH value was adjusted to 4-5 with 1M hydrochloric acid solution. After filtering, the filter cake was dried under reduced pressure to afford compound 62-3. MS m/z (ESI): 207.0 [M+1].

Step 3: At room temperature, intermediate 3 (0.140 g, 0.36 mmol) and compound 62-3 (0.075 g, 0.36 mmol) were dissolved in acetonitrile (2 mL). TCFH (0.151 g, 0.54 mmol) and N-methylimidazole (0.086 mL, 1.08 mmol) were added to the reaction solution. After reacting at room temperature for 18 hours, the reaction was completed. After the reaction was completed, the crude was afforded by concentration under reduced pressure. The crude was purified by silica gel chromatography (eluant: EA/PE = 5%-10%) to afford compound 62-4. MS m/z (ESI): 573.2 [M+1].

Step 4: At room temperature, compound 62-4 (0.145 g, 0.38 mmol) was dissolved in TFA (1 mL). Then, TfOH (0.1 mL) was added, followed by stirring at 25°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 55%-95%, flow rate: 20 mL/min) to afford compound 62. MS m/z (ESI): 453.2 [M+1]. ¹H NMR (400 MHz, DMSO-*d6*) *δ* 12.55 - 12.36 (m, 1H), 8.69 - 8.62 (m, 1H), 8.29 (t, *J* = 2.6 Hz, 1H), 7.97 - 7.81 (m, 5H), 6.62 (s, 1H), 6.42 - 6.31 (m, 1H), 4.05 - 3.90 (m, 1H), 3.30 - 3.22 (m, 2H), 1.73 - 1.49 (m, 4H), 1.19 (d, *J =* 6.2 Hz, 3H).

### Example 63

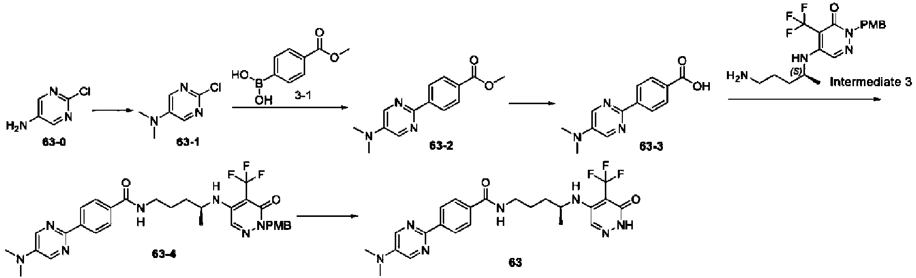

Step 1: Compound 63-0 (1 g, 7.72 mmol) was dissolved in 20 mL DMF. Firstly, potassium carbonate (5.33 g, 38.60 mmol) was added, and then iodomethane (3.29 g, 23.16 mmol) was added under a sealed condition. 50 mL water was added to the reaction solution, followed by extraction with 50 mL EA, and then washing three times with 50 mL water. The organic phases were dried over anhydrous sodium sulfate, and dried by spin to afford compound 63-1. MS m/z(ESI): 158.2 [M+1].

Step 2: Compound 63-1 (200 mg, 1.27 mmol), compound 3-1 (456.76 mg, 2.54 mmol), cesium carbonate (1.24 g, 3.81 mmol), and 1,1-bis(diphenylphosphino)ferrocenedichloropalladium (93.12 mg, 0.13 mmol) were dissolved in 10 mL 1,4-dioxane and 2 mL water, followed by reacting under stirring at 125°C for 0.5 hours in microwave under nitrogen protection. The reaction solution was concentrated. The residue was purified by alumina column chromatography (PE/EA=2/1) to afford compound 63-2. MS m/z(ESI): 258.3 [M+1].

### Step 3:

Compound 63-2 (92 mg, 0.38 mmol) and LiOH (42.81 mg, 1.79 mmol) were dissolved in 3 mL methanol and 1mL water. The reaction was carried out at room temperature for 16 hours. The reaction solution was concentrated under reduced pressure, neutralized with dilute hydrochloric acid, dried and concentrated to afford compound 63-3. MS m/z(ESI): 244.0 [M+1].

Step 4: Compound 63-3 (85 mg, 0.26 mmol) and intermediate 3 (107.45 mg, 0.28 mmol), HATU (74.46 mg, 0.51 mmol), and DIPEA (0.13 mL, 1.03 mmol) were dissolved in 6 mL DMF. The reaction was carried out at room temperature for 1 hour. The reaction solution was concentrated. The residue was purified by silica gel column chromatography with an eluant system (PE/EA=1/1) to afford compound 63-4. MS m/z(ESI): 610.2 [M+1].

Step 5: Compound 63-4 (94 mg, 0.15 mmol) was dissolved in 2 mL TFA, and 10 drops of TfOH were slowly added dropwise. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 63. MS m/z(ESI): 490.2 [M+1]. 1H NMR (400 MHz, DMSO) δ 12.45 (s, 1H), 8.52 (t, J = 5.6 Hz, 1H), 8.41 (s, 2H), 8.30 (d, J = 8.5 Hz, 2H), 7.97 - 7.84 (m, 3H), 6.36 (d, J = 4.2 Hz, 1H), 3.99 (s, 1H), 3.27 (d, J = 5.9 Hz, 2H), 3.03 (s, 6H), 1.66 (d, J = 7.7 Hz, 1H), 1.55 (s, 3H), 1.19 (d, J = 6.3 Hz, 3H).

### Example 64

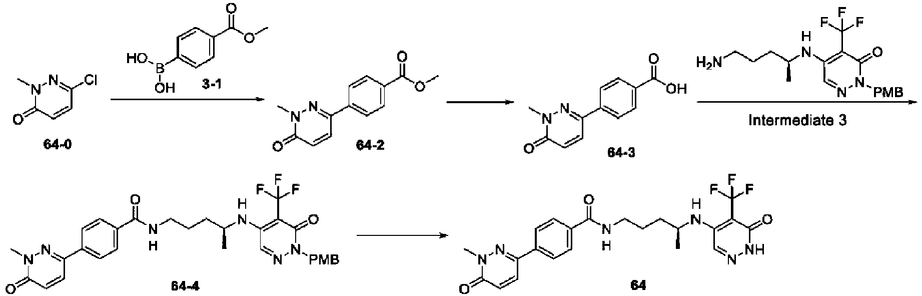

Step 1: Compound 64-0 (300 mg, 2.08 mmol), compound 3-1 (375 mg, 2.08 mmol), cesium carbonate (2.05 g, 6.24 mmol), and 1,1-bis(diphenylphosphino)ferrocenedichloropalladium (152 mg, 0.21 mmol) were dissolved in 3 mL 1,4-dioxane and 1 mL water, followed by reacting at 125°C for 0.5 hours in microwave. The reaction solution was concentrated. The residue was purified by silica gel column chromatography with an eluant system (PE/EA=3/1) to afford compound 64-2. MS m/z(ESI): 245.2 [M+1].

Step 2: Compound 64-2 (300 mg, 1.23 mmol) and LiOH (155 mg, 3.68 mmol) were dissolved in 3 mL methanol and 1 mL water. The reaction was carried out at room temperature for 3 hours. The reaction solution was concentrated under reduced pressure, neutralized with dilute hydrochloric acid, dried and concentrated to afford compound 64-3. MS m/z(ESI): 231.2 [M+1].

Step 3: Compound 64-3 (150 mg, 0.65 mmol) and intermediate 3 (375 mg, 0.98 mmol), HATU (371 mg, 0.98 mmol), and DIEA (252 mg, 1.95 mmol) were dissolved in 3 mL DMF. The reaction was carried out at room temperature for 0.5 hours. The reaction solution was concentrated. The residue was purified by silica gel column chromatography with an eluant system (methanol/DCM=1/20) to afford compound 64-4. MS m/z(ESI): 597.6 [M+1].

Step 4: Compound 64-4 (160 mg, 0.27 mmol) and TfOH (4 mg, 0.03 mmol) were dissolved in 2 mL TFA, and the reaction was carried out at room temperature for 0.5 hours. The reaction solution was concentrated under reduced pressure and purified by high performance liquid chromatography (Waters-2545, chromatographic column: Waters-CORTECS-C18-2.7µm-4.6×30mm; mobile phase: water (containing 10 mmol/L formic acid) and acetonitrile, gradient ratio: acetonitrile 5%-95%, flow rate: 30 mL/min) to afford compound 64. MS m/z(ESI):477.4[M+1]. 1H NMR (400 MHz, DMSO) δ 12.44 (s, 1H), 8.58 (t, J = 5.5 Hz, 1H), 8.12 (d, J = 9.8 Hz, 1H), 8.01 - 7.96 (m, 2H), 7.95 - 7.91 (m, 3H), 7.07 (d, J = 9.7 Hz, 1H), 6.41 - 6.31 (m, 1H), 4.06 - 3.90 (m, 1H), 3.76 (s, 3H), 3.33 - 3.22 (m, 2H), 1.71 - 1.62 (m, 1H), 1.60 - 1.49 (m, 3H), 1.18 (d, J = 6.3 Hz, 3H).

### Example 65:

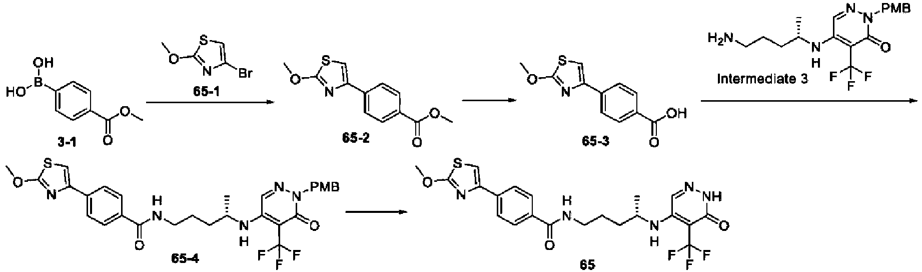

Step 1: Compound 3-1 (200 mg, 1.11 mmol), compound 65-1 (216.76 mg, 1.11 mmol), Pd(PPh3)4 (128.47 mg, 0.11 mmol), and cesium carbonate (724.12 mg, 2.22 mmol) were added sequentially to 1,4-dioxane : water solution (1 mL, 4:1). The mixture was reacted under stirring at 80°C for 1 hour under nitrogen protection. The reaction solution was concentrated to dryness. The residue was purified by flash (EA/PE=0 to 20%) to afford compound 65-2. MS m/z(ESI): 250.0 [M+1].

Step 2: 65-2 (180 mg, 0.72 mmol), water (2 mL), and LiOH (0.10 mL, 3.60 mmol) were added sequentially to methanol (1 mL). The system was reacted under stirring at 25°C for 1 hour. The reaction system was adjusted to pH 2-3 with 6M HCl, and filtered. The filter cake was concentrated to dryness to afford compound 65-3. MS m/z(ESI): 236.2 [M+1].

Step 3: Compound 65-3 (80 mg, 0.34 mmol) was dissolved in DMF (1 mL), and intermediate 3 (130.16 mg, 0.34 mmol), HATU (193.13 mg, 0.51 mmol), and DIPEA (87.36 mg, 0.68 mmol) were added sequentially. The system was reacted under stirring at 25°C for 1 hour. The reaction solution was concentrated to dryness. The residue was purified by flash (EA/PE=0 to 70%) to afford compound 65-4. MS m/z(ESI): 602.2 [M+1].

Step 4: 65-4 (100 mg, 0.17 mmol) and a catalytic amount of TfOH were added sequentially to TFA (1 mL). The mixture was reacted under stirring at 25°C for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 6%-40%, flow rate: 30 mL/min) to afford compound 65. MS m/z(ESI): 482.2 [M+1]. ¹H NMR (400 MHz, DMSO-*d*₆) δ 12.45 (s, 1H), 8.51 (t, *J =* 5.7 Hz, 1H), 8.04 - 7.82 (m, 5H), 7.62 (s, 1H), 6.94 - 6.05 (m, 1H), 4.11 (s, 3H), 3.98 (s, 1H), 3.29 - 3.17 (m, 2H), 1.78 - 1.43 (m, 4H), 1.18 (d, *J =* 6.3 Hz, 3H).

### Example 66

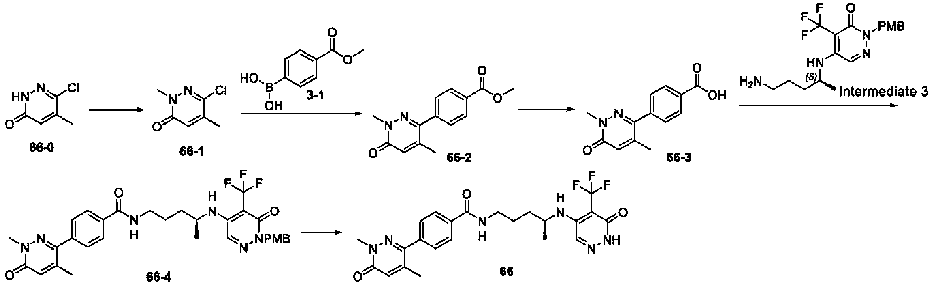

Step 1: Compound 66-0 (1 g, 6.92 mmol) was dissolved in 10 mL DMF. Potassium carbonate (1.91 g, 13.84 mmol) was added. Under sealed conditions, iodomethane (1.96 g, 13.84 mmol) was added. Then, the resulting mixture was reacted under stirring at room temperature for 4 hours. After the reaction was completed, the reaction product was extracted with distilled water and EA, to afford the organic layer. The organic layer was dried over anhydrous magnesium sulfate, filtered, and concentrated. The residue was purified by alumina column chromatography (PE/EA=5/1) to afford compound 66-1.

Step 2: Compound 66-1 (200 mg, 1.26 mmol), compound 3-1 (340.45 mg, 1.89 mmol), cesium carbonate (821.82 mg, 2.52 mmol), and 1,1-bis(diphenylphosphino)ferrocenedichloropalladium (95.03 mg, 0.13 mmol) were dissolved in 6 mL 1,4-dioxane:water (5:1), followed by reacting at 120°C for 0.5 hours in microwave under nitrogen protection. The reaction solution was concentrated. The residue was purified by alumina column chromatography (PE/EA=5/1) to afford compound 66-2. MS m/z(ESI): 259.0 [M+1].

Step 3: Compound 66-2 (230 mg, 0.89 mmol) and LiOH (99.69 mg, 4.17 mmol) were dissolved in 3 mL methanol and 1 mL water. The reaction was carried out at room temperature for 4 hours. The reaction solution was concentrated under reduced pressure, neutralized with dilute hydrochloric acid, dried and concentrated to afford compound 66-3. MS m/z(ESI): 245.0 [M+1].

Step 4: Compound 66-3 (190 mg, 0.78 mmol) and intermediate 3 (88.29 mg, 0.23 mmol), HATU (291.12 mg, 0.76 mmol), and DIPEA (0.26 mL, 1.54 mmol) were dissolved in 6 mL DMF. The reaction was carried out at room temperature for 1 hour. The reaction solution was concentrated. The residue was purified by silica gel column chromatography with an eluant system (PE/EA=1/1) to afford compound 66-4. MS m/z(ESI): 611.2 [M+1].

Step 5: Compound 66-4 (115 mg, 0.19 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 66. MS m/z(ESI): 491.2 [M+1]. 1H NMR (400 MHz, DMSO) δ 12.45 (s, 1H), 8.58 (t, J = 5.6 Hz, 1H), 7.99 - 7.87 (m, 3H), 7.58 (d, J = 8.3 Hz, 2H), 6.92 (s, 1H), 6.36 (d, J = 4.7 Hz, 1H), 6.36 (d, J = 4.7 Hz, 1H), 3.99 (s, 1H), 3.68 (s, 3H), 3.28 (d, J = 5.3 Hz, 2H), 2.13 (s, 3H), 1.66 (d, J = 7.6 Hz, 1H), 1.55 (s, 3H), 1.18 (t, J = 8.4 Hz, 3H).

### Example 67

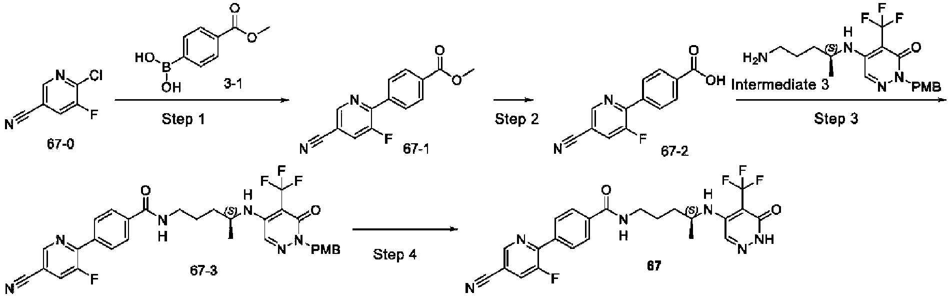

Step 1: Compound 67-0 (300 mg, 1.92 mmol, Bide), compound 3-1 (413.88 mg, 2.3 mmol), and cesium carbonate (1248.83 mg, 3.83 mmol) were added to THF (3 mL) and water (0.5 mL). [1,1'-bis(diphenylphosphino)ferrocene]dichloropalladiumdichloromethane complex (156.50 mg, 0.19 mmol) was added. The mixture was purged with nitrogen 3 times. The reaction was carried out at 85°C for 1 hour. The resulting mixture was concentrated under reduced pressure and purified by silica gel chromatography (elution solution: PE: EA = 5: 1-2: 1) to afford compound 67-1. MS m/z(ESI): 257.2 [M+1].

Step 2: Compound 67-1 (412 mg,1.61 mmol) was added to iodotrimethylsilane (5 mL), followed by reacting at 80°C for 18 hours. The reaction solution was poured into 20 mL water, followed by extraction with EA (10 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:3) to afford compound 67-2. MS m/z(ESI): 243.0 [M+1].

Step 3: Compound 67- 2 (100 mg, 0.41 mmol) was dissolved in acetonitrile (2 mL). Then, TCFH (173.76 mg, 0.62 mmol) and N-methylimidazole (98.74 µL, 1.24 mmol) were added sequentially. After stirring at room temperature for 0.5 hours, intermediate 3 (158.71 mg, 0.41 mmol) was added. Under a nitrogen atmosphere, the reaction was carried out at room temperature for 16 hours. The reaction solution was concentrated under reduced pressure. The residue was added with 10 mL water, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:5) to afford compound 67-3. MS m/z(ESI): 609.4 [M+1].

Step 4: Compound 67-3 (169 mg, 0.28 mmol) was dissolved in TFA (2 mL), TfOH (13.35 µL, 0.14 mmol) was added, and the reaction was carried out at room temperature for 18 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 35%-50%, flow rate: 30 mL/min) to afford compound 67. MS m/z(ESI): 489.2 [M+1]. ¹H NMR (400 MHz, DMSO) δ 12.44 (s, 1H), 9.04 (t, *J =* 1.4 Hz, 1H), 8.64 (t, *J =* 5.6 Hz, 1H), 8.58 (dd, *J =* 11.2, 1.6 Hz, 1H), 8.08 - 7.97 (m, 4H), 7.94 (s, 1H), 6.48 - 6.21 (m, 1H), 4.12 - 3.85 (m, 1H), 3.32 - 3.24 (m, 2H), 1.78 - 1.62 (m, 1H), 1.62 - 1.46 (m, 3H), 1.19 (d, *J =* 6.4 Hz, 3H).

### Example 68

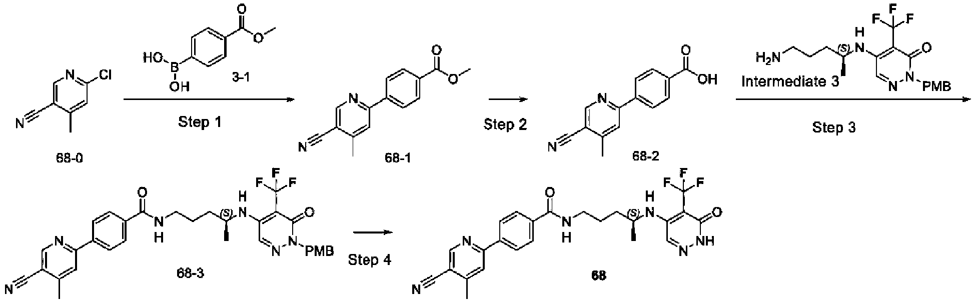

Step 1: Compound 68-0 (500 mg, 3.28 mmol, Bide), compound 3-1 (707.71 mg, 3.93 mmol), and cesium carbonate (2135.40 mg, 6.55 mmol) were added to 1,4-dioxane (5 mL) and water (1 mL). [1,1'-bis(diphenylphosphino)ferrocene]dichloropalladiumdichloromethane complex (267.61 mg, 0.33 mmol) was added. The mixture was purged with nitrogen 3 times. The reaction was carried out at 100°C for 18 hours. The resulting mixture was concentrated under reduced pressure and purified by silica gel chromatography (elution solution: PE: EA = 2: 1-1: 1) to afford compound 68-1. MS m/z(ESI): 253.0 [M+1].

Step 2: Compound 68-1 (300 mg,1.19 mmol) was added to methanol (5 mL) and water (5 mL), and LiOH (85.44 mg, 3.57 mmol) was added. The reaction was carried out at room temperature for 18 hours. The reaction solution was concentrated to remove methanol, adjusted to pH=7 with dilute hydrochloric acid, filtered, washed with water, and oven-dried to afford compound 68-2. MS m/z(ESI): 239.0 [M+1].

Step 3: Compound 68-2 (200 mg, 0.42 mmol) was dissolved in acetonitrile (2 mL). Then, TCFH (176.65 mg, 0.63 mmol) and N-methylimidazole (100.38 µL, 1.26 mmol) were added sequentially. After stirring at room temperature for 0.5 hours, intermediate 3 (161.34 mg, 0.42 mmol) was added. Under a nitrogen atmosphere, the reaction was carried out at room temperature for 16 hours. The reaction solution was concentrated under reduced pressure. The residue was added to water (10 mL), followed by extraction with EA(15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:5) to afford compound 68-3. MS m/z(ESI): 605.4 [M+1].

Step 4: Compound 68-3 (173 mg, 0.29 mmol) was dissolved in TFA (2 mL), TfOH (13.35 µL, 0.14 mmol) was added, and the reaction was carried out at room temperature for 18 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-50%, flow rate: 30 mL/min) to afford compound 68. MS m/z(ESI): 485.2 [M+1]. ¹H NMR (400 MHz, DMSO) δ 12.45 (s, 1H), 9.02 (s, 1H), 8.62 (t, *J =* 5.6 Hz, 1H), 8.26 (s, 1H), 8.24 (s, 2H), 7.98 (d, *J =* 8.6 Hz, 2H), 7.94 (s, 1H), 6.40 - 6.31 (m, 1H), 4.09 - 3.88 (m, 1H), 3.32 - 3.21 (m, 2H), 2.58 (s, 3H), 1.76 - 1.63 (m, 1H), 1.63 - 1.46 (m, 3H), 1.19 (d, *J =* 6.4 Hz, 3H).

### Example 69

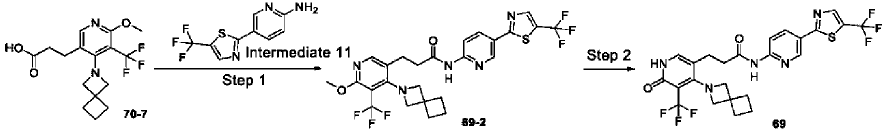

Step 1: At room temperature, N-methylimidazole (81 mg, 0.99 mmol) and TCFH (138 mg, 0.490 mmol) were added to a solution of compound 70-7 (85 mg, 0.25 mmol) and intermediate 11 (61 mg, 0.25 mmol) in acetonitrile (5 mL). The reaction solution was stirred at 60°C for 18 h. After the reaction was completed, the crude product was afforded by concentration under reduced pressure. The crude product was purified by silica gel plate (eluant: EA/PE = 25%-60%) to afford compound 69-2. MS m/z (ESI): 572.2 [M+1].

Step 2: At room temperature, compound 69-2 (50 mg, 0.09 mmol) was dissolved in NMP (1 mL). Then, iodotrimethylsilane (35 mg, 0.17 mmol) was added, followed by stirring at 85°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 48%-75%, flow rate: 20 mL/min) to afford compound 69. MS m/z (ESI): 558.1 [M+1]. ¹H NMR (400 MHz, DMSO) δ 10.88 (s, 1H), 10.77 (d, *J =* 5.4 Hz, 1H), 8.96 (d, *J* = 2.4 Hz, 1H), 8.56 (d, *J =* 1.0 Hz, 1H), 8.39 (dd, *J =* 8.8, 2.4 Hz, 1H), 8.25 (d, *J =* 8.8 Hz, 1H), 7.02 (d, *J =* 5.6 Hz, 1H), 4.36 - 4.15 (m, 4H), 2.79 - 2.68 (m, 2H), 2.59 - 2.53 (m, 2H), 2.25 - 2.07 (m, 4H), 1.82 - 1.60 (m, 2H).

### Example 70

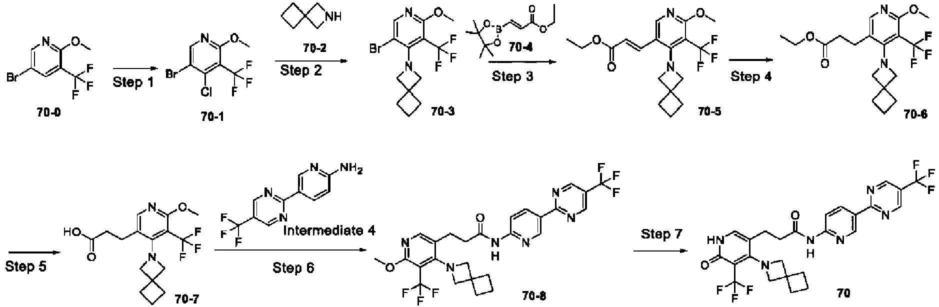

Step 1: Diisopropylamine (14.63 g, 140.6 mmol) was added to THF (500 mL) solution. At -80°C, n-butyl lithium (56.24 mL, 140.6 mmol) was added. The reaction solution was reacted at -80°C for half an hour. Compound 70-0 (24.0 g, 93.7 mmol) was slowly added to the above reaction solution at -78°C. The reaction solution was reacted for another 1 hour while the reaction temperature was maintained at -80°C. Then, hexachloroethane (33.29 g, 140.6 mmol) was added, followed by reacting another 1 hour. The resulting mixture was warmed to room temperature and reacted overnight. After the reaction was completed, the mixture was poured into ammonium chloride aqueous solution (500 mL), followed by extraction with EA (300 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=100:1-20:1) to afford compound 70-1. ¹H NMR (400 MHz, CDCl3) δ 8.44 (s, 1H), 4.01 (s, 3H).

Step 2: Compound 70-1 (1.00 g, 3.44 mol) was dissolved in isopropanol (10 mL). Then, compound 70-2 (0.33 g, 0.344 mol) and DIPEA (2.2 g, 17.2 mol) were added. The reaction solution was stirred overnight at 60°C. The reaction solution was concentrated to afford compound 70-3. MS m/z(ESI): 353.0 [M+1].

Step 3: At room temperature, 1,1-bis(diphenylphosphino)ferrocenedichloropalladium (196 mg, 0.270 mmol) and potassium carbonate (1.11 g, 8.03 mmol) were added to a solution of compound 70-3 (940 mg, 2.68 mmol) and compound 70-4 (1.21 g, 5.35 mmol) in 1,4-dioxane (10 mL) and water (2 mL), followed by heating to 90°C and stirring for 2 h. After the reaction was completed, the crude product was afforded by concentration under reduced pressure. The crude product was purified by silica gel column (eluant: EA/PE = 10%-35%) to afford compound 70-5. MS m/z (ESI): 371.1 [M+1].

Step 4: At 25°C, palladium on carbon (100 mg, 10 wt%) was added to a solution of compound 70-5 (540 mg, 1.46 mmol) in ethanol (15 mL), followed by stirring at room temperature for 2 hours under a hydrogen atmosphere. After the reaction was completed, the reaction solution was filtered, and the filtrate was concentrated under reduced pressure to afford compound 70-6. MS m/z (ESI): 373.1 [M+1].

Step 5: At 25°C, LiOH (162 mg, 3.87 mmol) and water (2 mL) solution were added to a solution of compound 70-6 (480 mg, 1.29 mmol) in THF (2 mL) and anhydrous methanol (2 mL). The reaction solution was stirred at room temperature for 2 hours. After the reaction was completed, the mixture was concentrated and adjusted to pH 4-5 with saturated sodium bicarbonate solution, followed by extraction with DCM (10 mL×3). The organic phases were combined, washed with saturated saline(10 mL×2), dried over anhydrous sodium sulfate, filtered, and concentrated under reduced pressure to afford compound 70-7. MS m/z (ESI): 345.1 [M+1].

Step 6: At room temperature, N-methylimidazole (191 mg, 2.32 mmol) and TCFH (326 mg, 1.16 mmol) were added to a solution of compound 70-7 (200 mg, 0.580 mmol) and intermediate 4 (195 mg, 0.810 mmol) in acetonitrile (5 mL). The reaction solution was stirred at 60°C for 18 h. After the reaction was completed, the crude product was afforded by concentration under reduced pressure. The crude product was purified by silica gel plate (eluant: EA/PE = 25%-60%) to afford compound 70-8. MS m/z (ESI): 567.2 [M+1].

Step 7: At room temperature, compound 70-8 (80 mg, 0.14 mmol) was dissolved in NMP (2 mL). Then, iodotrimethylsilane (56 mg, 0.28 mmol) was added, followed by stirring at 85°C for 1 hour until the reaction was completed. The crude product was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 48%-78%, flow rate: 20 mL/min) to afford compound 70. MS m/z (ESI): 277.2 [M/2+1]. ¹H NMR (400 MHz, DMSO) δ 10.89 (s, 1H), 10.77 (d, *J=* 6.0 Hz, 1H), 9.42 - 9.25 (m, 3H), 8.73 (dd, *J =* 8.8, 2.4 Hz, 1H), 8.29 (d, *J=* 8.8 Hz, 1H), 7.03 (d, *J* = 6.2 Hz, 1H), 4.37 - 4.15 (m, 4H), 2.76 - 2.70 (m, 2H), 2.59 - 2.56 (m, 2H), 2.22 - 2.08 (m, 4H), 1.81 - 1.68 (m, 2H).

### Example 71

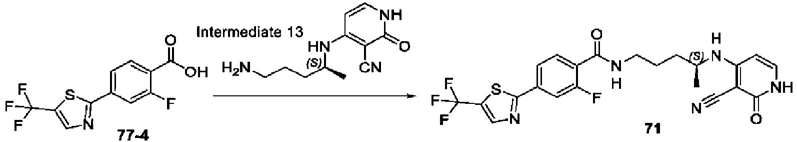

Compound 77-4 (85.93 mg, 0.30 mmol) was dissolved in DMF (3 mL), and intermediate 13 (65 mg, 0.30 mmol), HATU (168.30 mg, 0.44 mmol), and DIPEA (0.20 mL, 1.18 mmol) were added sequentially. The system was reacted under stirring at 25°C for 1 hour. After the reaction was completed, the resulting mixture was filtered and purified by high performance liquid chromatography (Waters-2545, chromatographic column: SharpSil-T C18, 30×150 mm, 5 µm; mobile phase: water (containing 10mmol/L ammonium bicarbonate) and acetonitrile, gradient ratio: acetonitrile 2%-32%, flow rate: 30 mL/min) to afford compound 71. MS m/z(ESI): 494.2 [M+1]. ¹H NMR (400 MHz, DMSO-*d*₆) δ 11.04 (s, 1H), 8.63 (d, *J =* 1.4 Hz, 1H), 8.37 (d, *J =* 8.4 Hz, 1H), 8.05 - 7.86 (m, 2H), 7.69 (t, *J =* 7.7 Hz, 1H), 7.32 (s, 2H), 5.91 (s, 1H), 4.17 - 3.88 (m, 1H), 3.27 (s, 2H), 1.74 - 1.37 (m, 4H), 1.15 (d, *J =* 6.6 Hz, 3H).

### Example 72

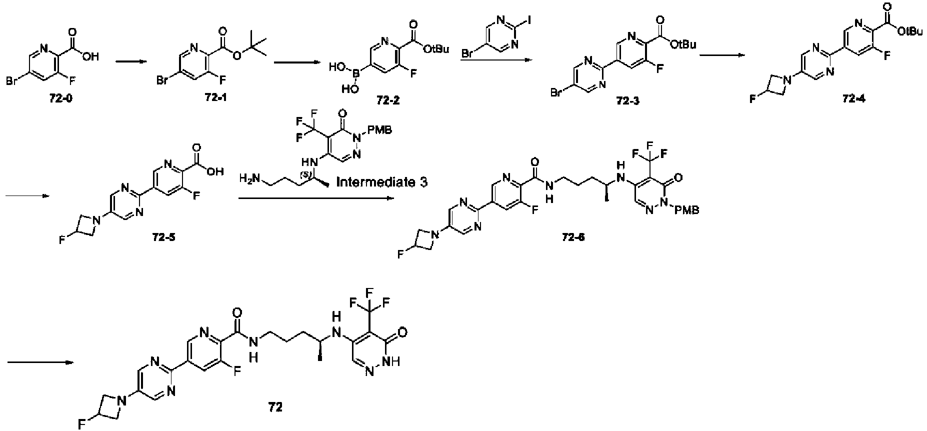

Step 1: Compound 72-0 (1 g, 4.55 mmol, Bide), di-tert-butyl dicarbonate (1.95 mL, 9.09 mmol, Titan), and DMAP (60 mg, 0.45 mmol, Titan) were dissolved in THF (15 mL). The reaction was carried out at 60°C for 3 hours. The reaction solution was directly concentrated under reduced pressure. The residue was purified by silica gel column chromatography with an eluant (PE: EA=5/1) to afford compound 72-1. MS m/z(ESI): 222.0 [M-56+1].

Step 2: 72-1 (1.1 g, 3.98 mmol) was dissolved in 1,4-dioxane (15 mL). Bis(pinacolato)diboron(2.02 g, 7.97 mmol, Titan), [1,1'-bis(diphenylphosphino)ferrocene]dichloropalladiumdichloromethane complex (330 mg, 0.40 mmol, Bide), and potassium acetate (980 mg, 9.96 mmol, Leyan) were added. The reaction was carried out at 110°C for 16 hours under nitrogen protection. The reaction solution was directly concentrated under reduced pressure. The residue was added with 10% sodium hydroxide solution (10 mL) and water (10 mL), followed by extraction with EA (10 mL×3). Aqueous phase was adjusted to pH=5 with HCl (1M), followed by extraction with EA (20 mL×3). The organic phases were combined, and dried over anhydrous sodium sulfate. After filtering to remove the desiccant, the filtrate was concentrated under reduced pressure to afford compound 72-2. MS m/z(ESI): 186.0 [M-56+1].

Step 3: 72-3 (450 mg, 1.87 mmol) was dissolved in 1,4-dioxane (2 mL) and water (0.4 mL). 5-bromo-2-iodopyrimidine (638.24 mg, 2.24 mmol, Bide), [1,1'-bis(diphenylphosphino)ferrocene]dichloropalladiumdichloromethane complex (152.47 mg, 0.19 mmol, Bide), and potassium carbonate (774.05 mg, 5.60 mmol, Leyan) were added. The reaction was carried out at 100°C for 16 hours under nitrogen protection. The reaction solution was directly concentrated under reduced pressure. The residue was added with water (10 mL), followed by extraction with EA (15 mL×3). The organic phases were combined, and dried over anhydrous sodium sulfate. After filtering to remove the desiccant, the filtrate was concentrated under reduced pressure. The residue was purified by silica gel column chromatography with an eluant (PE: EA=5/1) to afford compound 72-3. MS m/z(ESI): 299.0 [M-56+1].

Step 4: 72-3 (160 mg, 0.45 mmol) was dissolved in 1,4-dioxane (4 mL). 3-fluoroazetidine hydrochloride (101.34 mg, 0.91 mmol, Bide), tris(dibezylideneacetone)dipalladium (41.60 mg, 0.05 mmol, Bide), 4,5-bis(diphenylphosphino)-9,9-dimethylxanthene (52.57 mg, 0.09 mmol, Bide), and cesium carbonate (444.04 mg, 1.36 mmol, Leyan) were added. The reaction was carried out at 100°C for 16 hours under nitrogen protection. The reaction solution was directly concentrated under reduced pressure. The residue was purified by silica gel column chromatography with an eluant (PE: EA=1/1) to afford compound 72-4. MS m/z(ESI): 349.2 [M+1].

Step 5: Compound 72-4 (109 mg, 0.31 mmol) and TFA (0.5 mL, 6.71 mmol) were dissolved in DCM (5 mL), followed by reacting under stirring at room temperature for 1 hour. The reaction solution was concentrated to afford compound 72-5. MS m/z(ESI): 275.0 [M+1].

Step 6: Compound 72-5 (100 mg, 0.34 mmol) and intermediate 3 (88.29 mg, 0.23 mmol), HATU (145.56 mg, 0.38 mmol), and DIPEA (0.13 mL, 0.77 mmol) were dissolved in 6 mL DMF. The reaction was carried out at room temperature for 3 hours. The reaction solution was concentrated. The residue was purified by silica gel column chromatography with an eluant system (PE/EA=1/1) to afford compound 72-6. MS m/z(ESI): 658.4 [M+1].

Step 5: Compound 72-6 (92 mg, 0.14 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 72. MS m/z(ESI): 539.2 [M+1]. 1H NMR (400 MHz, DMSO) δ 12.41 (s, 1H), 9.22 (s, 1H), 8.76 (t, J = 5.9 Hz, 1H), 8.34 (d, J = 11.8 Hz, 1H), 8.25 (s, 2H), 7.92 (s, 1H), 6.31 (s, 1H), 5.77 - 5.21 (m, 1H), 4.51 - 4.27 (m, 2H), 4.16 (dd, J = 24.9, 9.3 Hz, 2H), 3.99 (s, 1H), 3.28 - 3.22 (m, 2H), 1.67 (s, 1H), 1.55 (s, 3H), 1.24 (s, 3H).

### Example 73

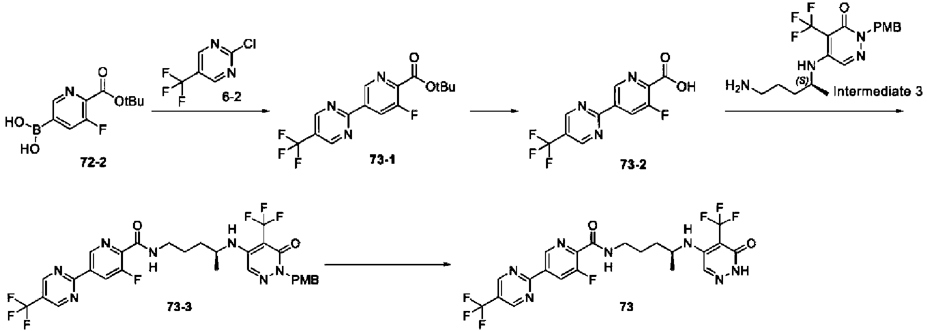

Step 1: Compound 72-2 (280.00 mg, 1.16 mmol) was dissolved in 5 mL THF. Palladium acetate (52.16 mg, 0.23 mmol), Xantphos (4,5-bis(diphenylphosphino)-9,9-dimethylxanthene) (268.90 mg, 0.46 mmol), potassium phosphate (739.77 mg, 3.49 mmol), and compound 6-2 (350 mg, 1.90 mmol) were added, followed by reacting under stirring at 60°C for 4 hours. The reaction solution was concentrated. The residue was purified by alumina column chromatography (PE/EA=5/1) to afford compound 73-1. MS m/z(ESI): 288.2 [M+1-56].

Step 2: Compound 73-1 (160 mg, 0.46 mmol) and TFA (0.5 mL, 6.71 mmol) were dissolved in DCM (5 mL), followed by reacting under stirring at room temperature for 2 hours. The reaction solution was concentrated to afford compound 73-2. MS m/z(ESI): 288.2 [M+1].

Step 3: Compound 73-2 (130 mg, 0.47 mmol) and intermediate 3 (88.29 mg, 0.23 mmol), HATU (145.56 mg, 0.38 mmol), and DIPEA (0.13 mL, 0.77 mmol) were dissolved in 6 mL DMF. The reaction was carried out at room temperature for 16 hours. The reaction solution was concentrated. The residue was purified by silica gel column chromatography with an eluant system (PE/EA=1/1) to afford compound 73-3. MS m/z(ESI): 654.3 [M+1].

Step 4: Compound 73-3 (100 mg, 0.15 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 73. MS m/z(ESI): 534.2 [M+1]. 1H NMR (400 MHz, DMSO) δ 12.44 (s, 1H), 9.48 (s, 2H), 9.39 (s, 1H), 8.92 (t, J = 5.9 Hz, 1H), 8.61 (dd, J = 11.2, 1.6 Hz, 1H), 7.94 (s, 1H), 6.36 (s, 1H), 4.00 (s, 1H), 3.28 (s, 2H), 1.67 (d, J = 7.8 Hz, 1H), 1.56 (d, J = 3.6 Hz, 3H), 1.23 (s, 3H).

### Example 74

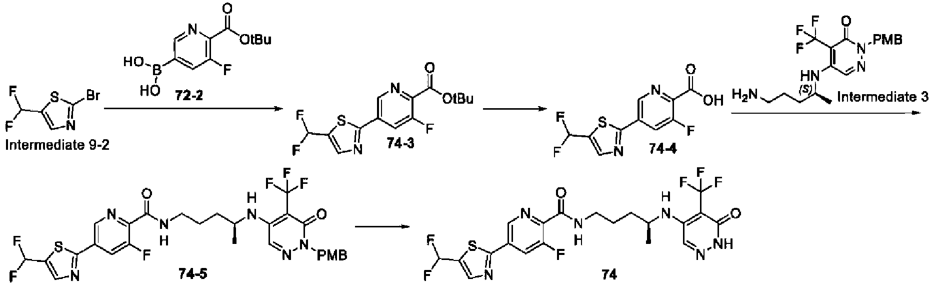

Step 1: Intermediate 9-2 (450 mg, 2.10 mmol), compound 72-2 (479 mg, 1.99 mmol), palladium acetate (47.15 mg, 0.21 mmol), 4,5-bis(diphenylphosphino)-9,9-dimethylxanthene (243.02 mg, 0.42 mmol), and potassium phosphate (1337.24 mg, 6.30 mmol) were dissolved in 10 mL THF, followed by reacting under stirring at 60°C for 4 hours under nitrogen protection. The reaction solution was concentrated. The residue was purified by alumina column chromatography (PE/EA=5/1) to afford compound 74-3. MS m/z(ESI): 275.0 [M+1-56].

Step 2: Compound 74-3 (100 mg, 0.30 mmol) and TFA (0.5 mL, 6.71 mmol) were dissolved in DCM (5 mL), followed by reacting under stirring at room temperature for 1 hour. The reaction solution was concentrated to afford compound 74-4. MS m/z(ESI): 275.0 [M+1].

Step 3: Compound 74-4 (95 mg, 0.26 mmol) and intermediate 3 (88.29 mg, 0.23 mmol), HATU (145.56 mg, 0.38 mmol), and DIPEA (0.13 mL, 0.77 mmol) were dissolved in 6 mL DMF. The reaction was carried out at room temperature for 3 hours. The reaction solution was concentrated. The residue was purified by silica gel column chromatography with an eluant system (PE/EA=1/1) to afford compound 74-5. MS m/z(ESI): 641.2 [M+1].

Step 4: Compound 74-5 (85 mg, 0.13 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 74. MS m/z(ESI): 521.0 [M+1]. 1H NMR (400 MHz, DMSO) δ 12.44 (s, 1H), 9.02 (s, 1H), 8.85 (t, J = 6.0 Hz, 1H), 8.42 - 8.37 (m, 2H), 7.93 (s, 1H), 7.22 (t, J = 54.3 Hz, 1H), 6.40 - 6.31 (m, 1H), 4.05 - 3.92 (m, 1H), 3.28 (d, J = 6.3 Hz, 2H), 1.71 - 1.49 (m, 4H), 1.18 (d, J = 6.3 Hz, 3H).

### Example 75

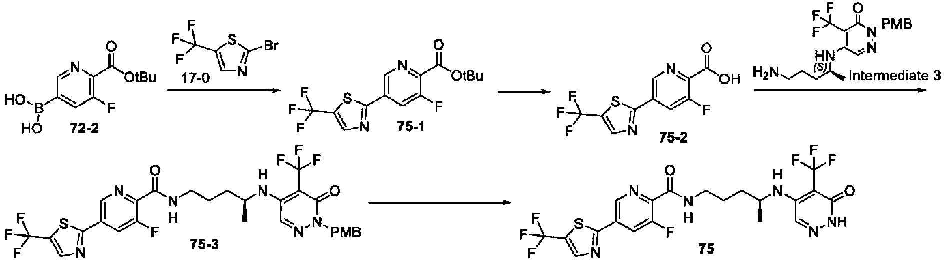

Step 1: Compound 72-2 (450 mg, 1.87 mmol), compound 17-0 (476 mg, 2.05 mmol), palladium acetate (41.98 mg, 0.19 mmol), 4,5-bis(diphenylphosphino)-9,9-dimethylxanthene (216.41 mg, 0.37 mmol), and potassium phosphate (1190.78 mg, 5.61 mmol) were dissolved in 10 mL THF, followed by reacting under stirring at 60°C for 4 hours under nitrogen protection. The reaction solution was concentrated. The residue was purified by alumina column chromatography (PE/EA=5/1) to afford compound 75-1. MS m/z(ESI): 293.0 [M+1-56].

Step 2: Compound 75-1 (100 mg, 0.29 mmol) and TFA (0.5 mL, 6.71 mmol) were dissolved in DCM (5 mL), followed by reacting under stirring at room temperature for 1 hour. The reaction solution was concentrated to afford compound 75-2 (100 mg). MS m/z(ESI): 293.0 [M+1].

Step 3: Compound 75-2 (90 mg, 0.23 mmol) and intermediate 3 (79.79 mg, 0.21 mmol), HATU (131.54 mg, 0.35 mmol), and DIPEA (0.11 mL, 0.69 mmol) were dissolved in 4 mL DMF. The reaction was carried out at room temperature for 3 hours. The reaction solution was concentrated. The residue was purified by silica gel column chromatography with an eluant system (PE/EA=1/1) to afford compound 75-3. MS m/z(ESI): 659.2 [M+1].

Step 4: Compound 75-3 (45 mg, 0.07 mmol) and TfOH (0.06 mL, 0.70 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 75. MS m/z(ESI): 539.0 [M+1]. 1H NMR (400 MHz, DMSO) δ 12.45 (s, 1H), 9.08 (s, 1H), 8.88 (t, J = 5.9 Hz, 1H), 8.72 (d, J = 1.0 Hz, 1H), 8.49 (dd, J = 10.9, 1.6 Hz, 1H), 7.93 (s, 1H), 6.36 (d, J = 4.8 Hz, 1H), 4.04 - 3.93 (m, 1H), 3.28 (dd, J = 12.4, 6.3 Hz, 2H), 1.71 - 1.51 (m, 4H), 1.18 (d, J = 6.3 Hz, 3H).

### Example 76

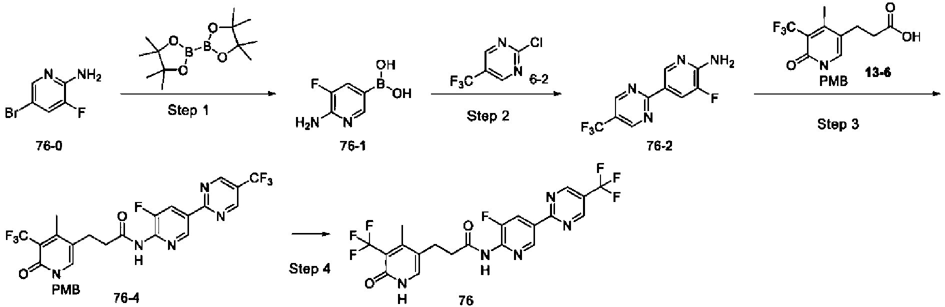

Step 1: Compound 76-0 (300 mg, 1.57 mmol, Bide), bis(pinacolato)diboron (478.63 mg, 1.88 mmol), and potassium acetate (462.44 mg, 4.71 mmol) were added to 1,4-dioxane (3 mL). Bis[5-(diphenylphosphino)cyclopentyl-1,3-dienyl]-λ2-iron(II)dichloromethane palladium chloride (115 mg, 0.16 mmol) was added. The mixture was purged with nitrogen 3 times, and the reaction was carried out at 100°C for 18 hours. Compound 76-1 was afforded without purification treatment. MS m/z(ESI): 157.0 [M+1].

Step 2: All compound 76-1 afforded in step 1, compound 6-2 (351.2 mg, 1.92 mmol), and cesium carbonate (1253.8 mg, 3.85 mmol) were added to 1, 4-dioxane (6 mL) and water (1.5 mL) solution. Bis[5-(diphenylphosphino)cyclopentyl-1, 3-dienyl]-λ2-iron(II) dichloromethane palladium chloride (314.25 mg, 0.38 mmol) was added. The mixture was purged with nitrogen 3 times, and the reaction was carried out at 100°C for 18 hours. The resulting mixture was concentrated under reduced pressure and purified by silica gel chromatography (elution solution: PE: EA = 5: 1-2: 1) to afford compound 76-2. MS m/z(ESI): 259.0 [M+1].

Step 3: Compound 76-2 (100 mg, 0.39 mmol) was dissolved in acetonitrile (2 mL). Then, TCFH (163 mg, 0.58 mmol) and N-methylimidazole (92.63 µL, 1.16 mmol) were added sequentially. After stirring at room temperature for 0.5 hours, compound 13-6 (143 mg, 0.39 mmol) was added. Under a nitrogen atmosphere, the reaction was carried out at room temperature for 16 hours. The reaction solution was concentrated under reduced pressure. The residue was added to water (10 mL), followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:5) to afford compound 76-4. MS m/z(ESI): 610.1 [M+1].

Step 4: Compound 76-4 (80 mg, 0.26 mmol) was dissolved in TFA (2 mL), and TfOH (15 µL, 0.3 mmol) was added. The reaction was carried out at 80°C for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 76. MS m/z(ESI): 490.1[M+1]. ¹H NMR (400 MHz, DMSO) δ 11.96 (s, 1H), 10.60 (s, 1H), 9.40 (s, 2H), 9.21 (d, *J =* 1.0 Hz, 1H), 8.51 (dd, *J =* 10.8, 1.8 Hz, 1H), 7.40 (s, 1H), 2.77 (t, *J =* 7.4 Hz, 2H), 2.66 (t, *J =* 7.4 Hz, 2H), 2.39 - 2.29 (m, 3H).

### Example 77

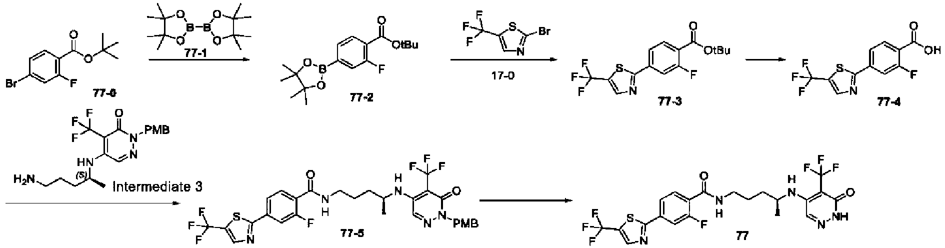

Step 1: Compound 77-0 (2g, 7.27 mmol), compound 77-1 (1.85 g, 7.27 mmol), 1,1-bis(diphenylphosphino)ferrocenedichloropalladium (0.53 g, 0.73 mmol), and potassium acetate (1.78 g, 18.18 mmol) were dissolved in 20 mL 1,4-dioxane. After reacting under stirring at 90°C for 8 hours under nitrogen protection, the reaction solution was dried by spin and purified by silica gel column chromatography (PE/DCM=4/1) to afford compound 77-2. MS m/z(ESI): 267.2 [M+1-56]

Step 2: Compound 77-2 (828.06 mg, 2.59 mmol), compound 17-0 (500 mg, 2.15 mmol), 1,1-bis(diphenylphosphino)ferrocenedichloropalladium (157.68 mg, 0.22 mmol), potassium phosphate (1143.54 mg, 5.39 mmol) were dissolved in 3 mL water and 15 mL THF. After reacting under stirring at 80°C for 5 hours under nitrogen protection, the reaction solution was dried by spin and purified by silica gel column chromatography (PE/EA=1/1) to afford compound 77-3. MS m/z(ESI): 348.7 [M+1]

Step 3: Compound 77-3 (260 mg, 0.75 mmol) was dissolved in 9 mL DCM. Compound TFA (2 mL, 26.84 mmol) was added. After reacting under stirring at 20°C for 12 hours under nitrogen protection, the reaction solution was dried by spin, followed by extraction with water and DCM. The organic phases were collected, dried over anhydrous sodium sulfate and concentrated to afford compound 77-4. MS m/z(ESI): 292.7 [M+1]

Step 4: Compound 77-4 (90 mg, 0.26 mg) and intermediate 3 (100 mg, 0.26 mmol), HATU (148.38 mg, 0.39 mmol), and DIEA (0.17 mL, 1.04 mmol) were dissolved in 3 mL DMF. The reaction was carried out at room temperature for 12 hours. The reaction solution was extracted with DCM and water. The organic phases were collected and concentrated. The concentrate was purified by silica gel column chromatography with an eluant system (DCM/EA=2/1) to afford compound 77-5 (84 mg). MS m/z(ESI): 658.7 [M+1].

Step 5: Compound 77-5 (84 mg, 0.14 mmol) and TfOH (0.5 mL, 5.63 mmol) were dissolved in 6 mL TFA. The reaction was carried out at room temperature for 10 minutes. The reaction solution was quenched by adding methanol under stirring, concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 77. MS m/z(ESI): 538.7 [M+1]. ¹HNMR (400 MHz, DMSO) δ 12.46 (s, 1H), 8.63 (d, J = 1.1 Hz, 1H), 8.52 (t, J = 5.2 Hz, 1H), 7.96 - 7.86 (m, 3H), 7.79 - 7.64 (m, 1H), 6.35 (dd, J = 8.3, 3.5 Hz, 1H), 3.99 (s, 1H), 3.26 (d, J = 5.8 Hz, 2H), 1.79 -1.62 (m, 1H), 1.55 (d, J = 5.2 Hz, 3H), 1.17 (dd, J = 18.4, 6.4 Hz, 3H).

### Example 78

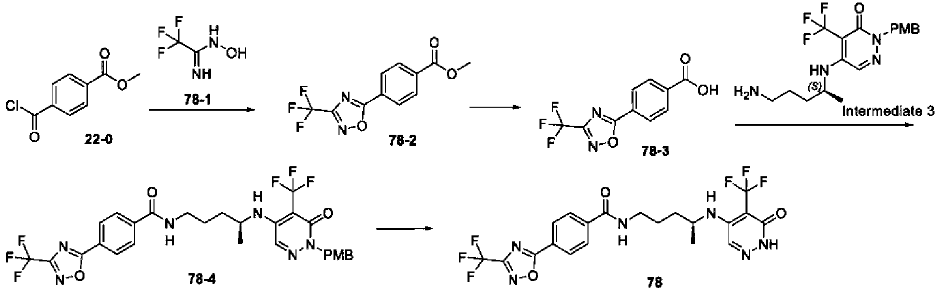

Step 1: Compound 22-0 (200 mg, 1.01 mmol) was added dropwise to compound 78-1 (128.96 mg, 1.01 mmol) and triethylamine (101.91 mg, 1.01 mmol) solution. At 0°C, 30 mL tert-butyl methyl ether was added. Then, the precipitated solid was washed with water to remove triethylamine hydrochloride, and dried by spin to afford compound 78-2. MS m/z(ESI): 273.0 [M+1].

Step 2: Compound 78-2 (430 mg, 1.58 mmol) and LiOH (99.69 mg, 4.17 mmol) were dissolved in 3 mL methanol and 1 mL water. The reaction was carried out at room temperature for 4 hours. The reaction solution was concentrated under reduced pressure, neutralized with dilute hydrochloric acid, dried and concentrated to afford compound 78-3. MS m/z(ESI): 259.0 [M+1].

Step 3: Compound 78-3 (110 mg, 0.4 mmol) and intermediate 3 (88.29 mg, 0.23 mmol), HATU (145.56 mg, 0.38 mmol), and DIPEA (0.13 mL, 0.77 mmol) were dissolved in 6 mL DMF. The reaction was carried out at room temperature for 3 hours. The reaction solution was concentrated. The residue was purified by silica gel column chromatography with an eluant system (PE/EA=1/1) to afford compound 78-4. MS m/z(ESI): 625.2 [M+1].

Step 4: Compound 78-4 (105 mg, 0.17 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 78. MS m/z(ESI): 505.2 [M+1]. 1H NMR (400 MHz, DMSO) δ 12.45 (s, 1H), 8.79 (t, J = 5.6 Hz, 1H), 8.28 (d, J = 8.4 Hz, 2H), 8.08 (d, J = 8.4 Hz, 2H), 7.93 (s, 1H), 6.36 (dd, J = 8.2, 3.2 Hz, 1H), 3.99 (s, 1H), 3.29 (d, J = 6.4 Hz, 2H), 1.78 - 1.62 (m, 1H), 1.56 (s, 3H), 1.19 (d, J = 6.3 Hz, 3H).

### Example 79

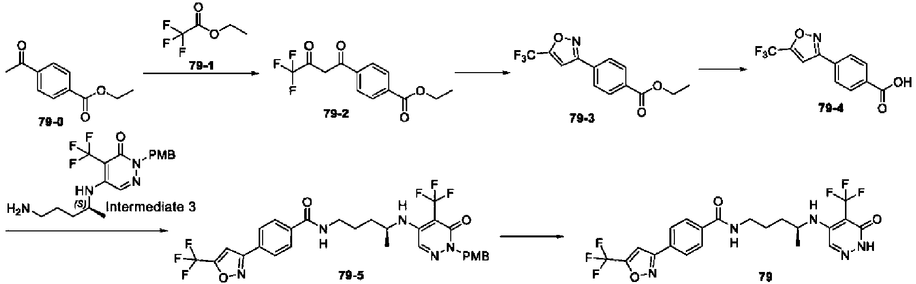

Step 1: Compound 79-1 (2.22 g, 15.61 mmol) was added to a solution of sodium ethoxide (1.06 g, 15.61 mmol) in 10 mL toluene, followed by stirring for 30 min. Then, compound 79-0 (1 g, 5.20 mmol) was added in batches. After reacting under stirring at 40°C for 3 hours, the reaction was carried out at room temperature for 16h. The solid was precipitated in an ice bath. The filtered solid was then partitioned between EtOAc (30 mL) and 5% of H₂SO₄ aqueous solution (10 mL). The organic layer was further washed with brine solution (2×50 mL), dried over MgSO₄, and filtered. The reaction solution was concentrated to afford compound 79-2. MS m/z(ESI): 289.0 [M+1]

Step 2: Compound 79-2 (220 mg, 0.76 mmol) in 2.5 mL glacial acetic acid was treated with hydroxylamine hydrochloride (63.65 mg, 0.9 mmol), followed by heating at 80-90°C for 16 hours. Then, the reaction was cooled and water was added to precipitate the solid. Then, the solid was dissolved in TFA (5 mL), followed by heating under reflux for 1 day. Then, the reaction was evaporated to afford compound 79-3.
MS m/z(ESI):286.2[M+1].

Step 3: Compound 79-3 (220 mg, 0.77 mmol) and LiOH (99.69 mg, 4.17 mmol) were dissolved in 3 mL methanol and 1 mL water. The reaction was carried out at room temperature for 4 hours. The reaction solution was concentrated under reduced pressure, neutralized with dilute hydrochloric acid, dried and concentrated to afford compound 79-4. MS m/z(ESI): 258.0 [M+1].

Step 4: Compound 79-4 (175 mg, 0.63 mmol) and intermediate 3 (88.29 mg, 0.23 mmol), HATU (145.56 mg, 0.38 mmol), and DIPEA (0.13 mL, 0.77 mmol) were dissolved in 6 mL DMF. The reaction was carried out at room temperature for 3 hours. The reaction solution was concentrated. The residue was purified by silica gel column chromatography with an eluant system (PE/EA=1/1) to afford compound 79-5. MS m/z(ESI):624.2[M+1].

Step 5: Compound 79-5 (85 mg, 0.13 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 79. MS m/z(ESI): 504.2 [M+1]. 1H NMR (400 MHz, DMSO) δ 12.45 (s, 1H), 8.66 (t, J = 5.6 Hz, 1H), 8.18 (s, 1H), 8.06 (d, J = 8.5 Hz, 2H), 8.00 (d, J = 8.5 Hz, 2H), 7.94 (s, 1H), 6.36 (dd, J = 8.6, 3.5 Hz, 1H), 3.99 (s, 1H), 3.28 (d, J = 6.4 Hz, 2H), 1.76 - 1.62 (m, 1H), 1.54 (d, J = 13.7 Hz, 3H), 1.19 (d, J = 6.3 Hz, 3H).

### Example 80

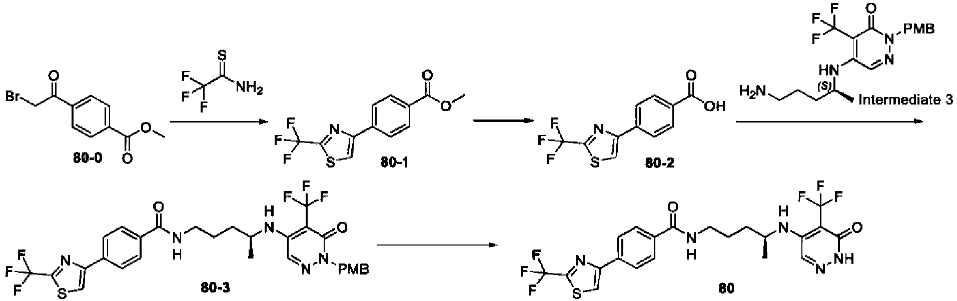

Step 1: Compound 80-0 (500 mg, 1.94 mmol) and trifluorothioacetamide (0.17 mL, 1.94 mmol) were dissolved in 4 mL toluene, followed by reacting under stirring at 100°C for 16 hours. The reaction solution was concentrated. The residue was purified by alumina column chromatography (PE/EA=1/1) to afford compound 80-1. MS m/z(ESI): 288.1 [M+1].

Step 2: Compound 80-1 (200 mg, 0.70 mmol) and LiOH (87.64 mg, 2.09 mmol) were dissolved in THF (12 mL), methanol (4 mL), and water (4 mL), followed by reacting under stirring at room temperature for 16 hours. The pH of the reaction solution was adjusted to 6 with 2M hydrochloric acid, followed by filtering to afford compound 80-2. MS m/z(ESI): 274.0 [M+1].

Step 3: Compound 80-2 (100 mg, 0.37 mmol) and intermediate 3 (126.62 mg, 0.33 mmol), HATU (139.16 mg, 0.37 mmol), and DIPEA (0.18 mL, 1.10 mmol) were dissolved in 3 mL DMF. The reaction was carried out at room temperature for 12 hours. The reaction solution was concentrated. The residue was purified by silica gel column chromatography with an eluant system (PE/EA=1/1) to afford compound 80-3. MS m/z(ESI): 640.2 [M+1].

Step 4: Compound 80-3 (140 mg, 0.22 mmol) and TfOH (0.10 mL, 1.09 mmol) were dissolved in 4 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 80. MS m/z(ESI): 520.2 [M+1]. 1H NMR (400 MHz, DMSO) δ 12.36 (s, 1H), 8.72 (s, 1H), 8.59 (t, J = 5.6 Hz, 1H), 8.09 (d, J = 8.4 Hz, 2H), 7.99 - 7.92 (m, 3H), 6.35 (d, J = 4.1 Hz, 1H), 3.99 (s, 1H), 3.28 (dd, J = 11.6, 5.8 Hz, 2H), 1.72 - 1.48 (m, 4H), 1.19 (d, J = 6.3 Hz, 3H).

### Example 81

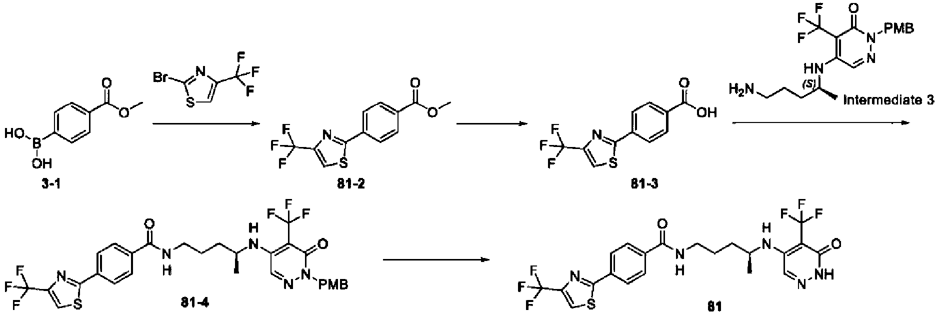

Step 1: Compound 3-1 (465.40 mg, 2.59 mmol), 2-bromo-4-trifluoromethylthiazole (500 mg, 2.15 mmol), [1,1'-bis(diphenylphosphino)ferrocene]dichloropalladiumdichloromethane complex (175.98 mg, 0.22 mmol), and sodium carbonate (456.81 mg, 4.31 mmol) were dissolved in 20 mL 1,4-dioxane and 4 mL water, followed by reacting under stirring at 100°C for 16 hours under nitrogen protection. The reaction solution was dried by spin and purified by silica gel column chromatography (PE/EA=10/1) to afford compound 81-2. MS m/z(ESI): 288.7 [M+1].

Step 2: Compound 81-2 (200 mg, 0.70 mmol), LiOH monohydrate (87.8mg, 2.09mmol), and methanol (3 mL), water (3 mL), THF (9 mL) were mixed, followed by stirring at room temperature for 16 hours. The pH of the reaction system was adjusted to 7 with 1M HCl, followed by extraction with DCM. The organic phases were collected and concentrated to afford compound 81-3. MS m/z(ESI): 274.7 [M+1].

Step 3: Compound 81-3 (71.08 mg, 0.26 mg) and intermediate 3 (100 mg, 0.26 mmol), HATU (148.38 mg, 0.39 mmol), and DIEA (0.17 mL, 1.04 mmol) were dissolved in 3 mL DMF. The reaction was carried out at room temperature for 1 hour. The reaction solution was extracted with DCM and water. The organic phases were collected and concentrated. The concentrate was purified by silica gel column chromatography with an eluant system (DCM/EA=2/1) to afford compound 81-4. MS m/z(ESI):640.7[M+1].

Step 4: Compound 81-4 (88 mg, 0.14 mmol) and TfOH (0.5 mL, 5.63 mmol) were dissolved in 6 mL TFA. The reaction was carried out at room temperature for 1 hour. The reaction solution was quenched by adding methanol under stirring, concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 81. MS m/z(ESI): 520.7 [M+1]. ¹HNMR(400 MHz, DMSO) δ 12.45 (s, 1H), 8.67 (dd, J = 14.0, 8.3 Hz, 2H), 8.09 (d, J = 8.5 Hz, 2H), 7.98 (d, J = 8.5 Hz, 2H), 7.94 (s, 1H), 6.45 - 6.26 (m, 1H), 3.99 (s, 1H), 3.28 (d, J = 5.5 Hz, 2H), 1.73 - 1.50(m, 4H), 1.19 (d, J = 6.3 Hz, 3H).

### Example 82

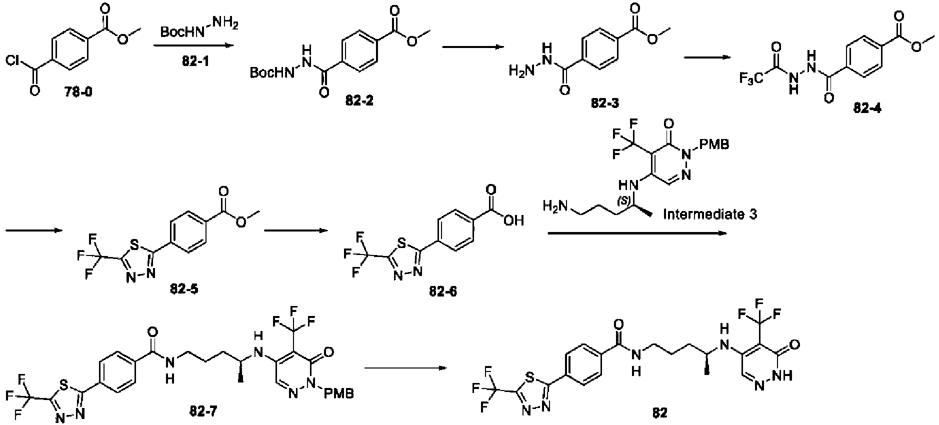

Step 1: Compound 78-0 (2 g, 10.07 mmol) was dissolved in 50 mL DCM, and triethylamine (2.04 g, 20.14 mmol) and compound 82-1 (2.00 g, 15.11 mmol) were added. Then, after reacting under stirring at room temperature for 30 minutes, 100 mL water was added to the reaction solution. The organic phases were dried by spin to afford compound 82-2. MS m/z(ESI): 195.0 [M+1-100].

Step 2: Compound 82-2 (2.5 g, 8.49 mmol) was dissolved in 30 mL DCM, and 6 mL TFA was added. Then, the resulting mixture was reacted under stirring at room temperature for 2 hours. The reaction solution was concentrated to afford compound 82-3. MS m/z(ESI): 195.0 [M+1].

Step 3: Compound 82-3 (1.7 g, 8.75 mmol) was dissolved in 50 mL THF. Trifluoroacetic anhydride (1.27 g, 13.13 mmol) was slowly added dropwise. After the reaction has cooled down to room temperature, the resulting mixture was directly concentrated to afford compound 82-4. MS m/z(ESI): 291.2 [M+1].

Step 4: Compound 82-4 (1 g, 3.45 mmol) and Lawesson's reagent (2.78 g, 6.9 mmol) were dissolved in 15 mL toluene. The reaction was carried out at 120°C for 2 hours in a nitrogen environment. The reaction solution was concentrated. The residue was purified by silica gel column chromatography with an eluant system (PE/EA=5/1) to afford compound 82-5. MS m/z(ESI): 289.2 [M+1].

Step 5: Compound 82-5 (160.00 mg, 0.55 mmol) and LiOH (66.46 mg, 2.78 mmol) were dissolved in 3 mL methanol and 1 mL water. The reaction was carried out at room temperature for 4 hours. The reaction solution was concentrated under reduced pressure, neutralized with dilute hydrochloric acid, and concentrated to afford compound 82-6. MS m/z(ESI): 275.1 [M+1].

Step 6: Compound 82-6 (145 mg, 0.53 mmol) and intermediate 3 (81.31 mg, 0.21 mmol), HATU (406.04 mg, 0.38 mmol), and DIPEA (0.45 mL, 2.65 mmol) were dissolved in 6 mL DMF. The reaction was carried out at room temperature for 1 hour. The reaction solution was concentrated. The residue was purified by silica gel column chromatography with an eluant system (PE/EA=1/1) to afford compound 82-7. MS m/z(ESI): 641.2 [M+1].

Step 7: Compound 82-7 (85 mg, 0.13 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 82. MS m/z(ESI): 521.2 [M+1]. 1H NMR (400 MHz, DMSO) δ 12.45 (s, 1H), 8.73 (t, J = 5.5 Hz, 1H), 8.20 (d, J = 8.5 Hz, 2H), 8.03 (d, J = 8.5 Hz, 2H), 7.94 (s, 1H), 6.36 (dd, J = 8.4, 3.5 Hz, 1H), 3.99 (s, 1H), 3.32 - 3.22 (m, 2H), 1.67 (d, J = 8.0 Hz, 1H), 1.56 (s, 3H), 1.19 (d, J = 6.3 Hz, 3H).

### Example 83

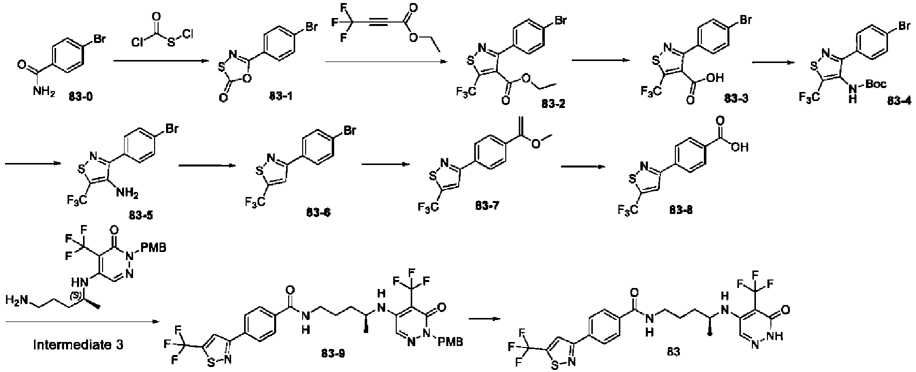

Step 1: 83-0 (6.5 g, 32.49 mmol) was dissolved in 100 mL toluene. Then, chloro(chlorosulfonyl)methanone (8.51 g, 64.99 mmol) was added, followed by reacting under stirring at 100°C for 16 hours in a nitrogen environment. The reaction solution was concentrated. The residue was purified by alumina column chromatography (PE/EA=20/1) to afford compound 83-1.

Step 2: Compound 83-1 (3 g, 11.62 mmol) and ethyl 4,4,4-trifluoro-2-butynoate (2.90 g, 17.44 mmol) were dissolved in 50 mL 2,6-dichlorobenzene, followed by reacting under stirring at 150°C for 16 hours under nitrogen protection. The reaction solution was concentrated. The residue was purified by alumina column chromatography (PE/EA=50/1) to afford compound 83-2. MS m/z(ESI): 380.0 [M+1].

Step 3: Compound 83-2 (2.5 g, 6.58 mmol) and LiOH (787.36 mg, 32.88 mmol) were dissolved in 20 mL THF and 10 mL water, followed by reacting under stirring at room temperature for 1 hour. The resulting mixture was acidified to pH=2 with 1N hydrochloric acid, and concentrated. The residue was purified by silica gel column chromatography with an eluant system (PE/EA=10/1) to afford compound 83-3. MS m/z(ESI): 352.0 [M+1].

Step 4: Compound 83-3 (2.3 g, 6.53 mmol), triethylamine (1.98 g, 19.60 mmol), and DPPA (1.79 g, 6.53 mmol) were dissolved in 20 mL toluene and 20 mL t-butyl alcohol, followed by reacting under stirring at 100°C for 16 hours under nitrogen protection. The reaction solution was concentrated. The residue was purified by alumina column chromatography (PE/EA=50/1) to afford compound 83-4. MS m/z(ESI): 423.1 [M+1].

Step 5: Compound 83-4 (2.2 g, 5.20 mmol) was dissolved in 20 mL DCM. 5mL TFA was added, followed by reacting under stirring at room temperature for 2 hours. The reaction solution was concentrated. The residue was neutralized with sodium bicarbonate aqueous solution, followed by extraction two times with 50 mL EA, drying and concentration. The residue was purified by silica gel column chromatography with an eluant system (PE/EA=5/1) to afford compound 83-5. MS m/z(ESI): 323.1 [M+1].

Step 6: Compound 83-5 (1.6 g, 4.95 mmol) was dissolved in 10 mL THF and 2 mL DMSO. Tert-butyl nitrite (1.53 g, 14.85 mmol) was added, followed by reacting under stirring at room temperature for 16 hours. The reaction solution was diluted with 30 mL water, followed by extraction three times with 30 mL EA, removing water and concentration. The residue was purified by silica gel column chromatography with an eluant system (PE/EA=10/1) to afford compound 83-6. MS m/z(ESI): 308.1 [M+1].

Step 7: Compound 83-6 (180 mg, 0.58 mmol), triethylamine (295.58 mg, 2.92 mmol), and [1,1'-bis(diphenylphosphino)ferrocene]dichloropalladiumdichloromethane complex (42 mg, 0.06 mmol) were dissolved in 10 mL methanol, followed by reacting under stirring at 80°C for 16 hours in a carbon monoxide environment. The reaction solution was concentrated. The residue was purified by silica gel column chromatography with an eluant system (PE/EA=3/1) to afford compound 83-7. MS m/z(ESI): 288.1 [M+1].

Step 8: Compound 83-7 (110 mg, 0.38 mmol) was dissolved in 3 mL methanol and 1 mL water. Then, LiOH (45.85 mg, 1.91 mmol) was added, followed by reacting under stirring at room temperature for 2 hours. The reaction solution was concentrated under reduced pressure, neutralized with dilute hydrochloric acid, dried and concentrated to afford compound 83-8. MS m/z(ESI): 274.0 [M+1].

Step 9: Compound 83-8 (95 mg, 0.35 mmol) and intermediate 3 (88.29 mg, 0.23 mmol), HATU (145.56 mg, 0.38 mmol), and DIPEA (0.13 mL, 0.77 mmol) were dissolved in 6 mL DMF. The reaction was carried out at room temperature for 3 hours. The reaction solution was concentrated. The residue was purified by silica gel column chromatography with an eluant system (PE/EA=1/1) to afford compound 83-9. MS m/z(ESI): 640.2 [M+1].

Step 10: Compound 83-9 (85 mg, 0.11 mmol) and TfOH (0.12 mL, 1.30 mmol) were dissolved in 2 mL TFA. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, to afford compound 83 (16.2 mg). MS m/z(ESI): 520.2 [M+1]. 1H NMR (400 MHz, DMSO) δ 12.45 (s, 1H), 8.79 (s, 1H), 8.63 (t, J = 5.6 Hz, 1H), 8.20 (d, J = 8.3 Hz, 2H), 8.00 - 7.88 (m, 3H), 6.36 (d, J = 4.9 Hz, 1H), 3.99 (s, 1H), 3.28 (d, J = 5.5 Hz, 2H), 1.67 (d, J = 8.0 Hz, 1H), 1.56 (s, 3H), 1.19 (d, J = 6.3 Hz, 3H).

### Example 84

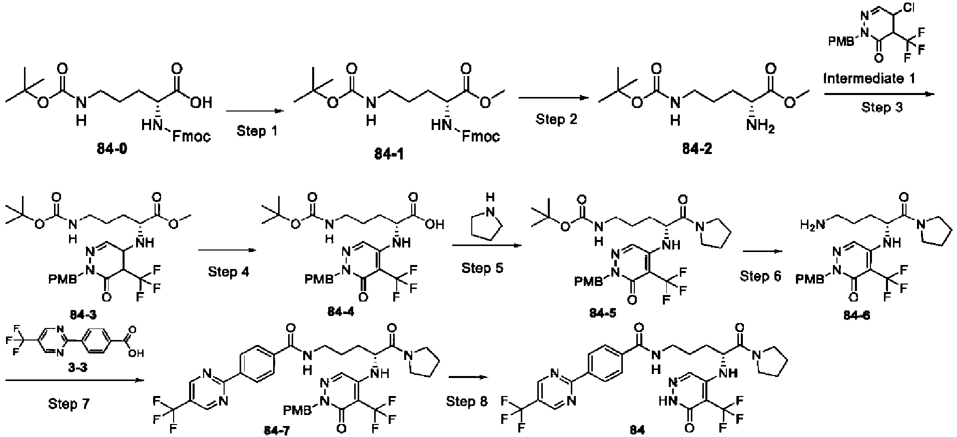

Step 1: 84-0 (2.0 g, 4.4 mmol) was dissolved in anhydrous methanol (10 mL) and toluene (20 mL). Then, trimethylsilyldiazomethane (6.6 mL, 13.2 mmol) was slowly added. Under a nitrogen atmosphere, the reaction was carried out at 80°C for 16 hours. The reaction solution was concentrated under reduced pressure. The residue was purified by silica gel column chromatography with an eluant system (PE: EA=10:1-1:1) to afford compound 84-1. MS m/z(ESI): 369.2 [M+1-100].

Step 2: 84-1 (1.50 g, 3.20 mmol) was dissolved in DCM (20 mL), and then diethylamine (2.0 mL, 19.4 mmol) was slowly added. Under a nitrogen atmosphere, the reaction was carried out at room temperature for 16 hours. The reaction solution was concentrated under reduced pressure. The residue was adjusted to pH=4-5 with 4M hydrochloric acid, and then concentrated under reduced pressure. The remaining solid was slurried with a mixed solvent of (PE/EA=10:1, 20 mL) to afford compound 84-2. MS m/z(ESI): 247.1[M+1-100].

Step 3: 84-2 (1.0 g, 4.06 mmol) was dissolved in isopropanol (20 mL). Then intermediate 1 (1.29 g, 4.06 mmol) and DIPEA (2.62 g, 20.3 mmol) were added sequentially. Under a nitrogen atmosphere, the reaction was carried out at 60°C for 16 hours. The reaction solution was concentrated under reduced pressure. The residue was purified by silica gel column chromatography with an eluant system (PE: EA=10:1-1:1) to afford compound 84-3. MS m/z(ESI): 529.2 [M+1].

Step 4: At 25°C, LiOH (290 mg, 6.81 mmol) and water (8 mL) solution were added to a solution of compound 84-3 (1.20 g, 2.27 mmol) in THF (8 mL) and anhydrous methanol (8 mL). The reaction solution was stirred at room temperature for 2 hours. After the reaction was completed, the mixture was concentrated and adjusted to pH 4-5 with saturated sodium bicarbonate solution, followed by extraction with DCM (10 mL×3). The organic phases were combined, washed with saturated saline (10 mL×2), dried over anhydrous sodium sulfate, and filtered. The filtrate was concentrated under reduced pressure to afford compound 84-4. MS m/z (ESI): 515.2 [M+1]

Step 5: 84-4 (300 mg, 0.58 mmol) was dissolved in acetonitrile (3 mL). Then, TCFH (331.27 mg, 0.87 mmol) and N-methylimidazole (288.73 µL, 1.74 mmol) were added sequentially. After stirring at room temperature for 0.5 hours, compound tetrahydropyrrole (82.50 mg, 1.16 mmol) was added. Under a nitrogen atmosphere, the reaction was carried out at room temperature for 16 hours. The reaction solution was concentrated under reduced pressure. The residue was added with 10 mL water, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:3) to afford compound 84-5. MS m/z(ESI): 568.4 [M+1].

Step 6: Compound 84-5 (60 mg, 0.11 mmol,) was added to a solution of 4M hydrochloric acid in 1, 4-dioxane (1 mL). The reaction was carried out at room temperature for 1 hour. Compound 84-6 was afforded by concentration under reduced pressure. MS m/z(ESI): 468.4 [M+1].

Step 7: Compound 3-3 (34.42 mg, 0.13 mmol) was dissolved in acetonitrile (1 mL). Then, TCFH (45.01 mg, 0.16 mmol) and N-methylimidazole (25.58 µL, 0.32 mmol) were added sequentially. After stirring at room temperature for 0.5 hours, compound 84-6 (49.42 mg, 0.11 mmol) was added. Under a nitrogen atmosphere, the reaction was carried out at room temperature for 16 hours. The reaction solution was concentrated under reduced pressure. The residue was added with 10 mL water, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:3) to afford compound 84-7. MS m/z(ESI): 718.4 [M+1].

Step 8: Compound 84-7 (50 mg, 0.07 mmol) was dissolved in TFA (1 mL), TfOH (3.1 µL, 0.035 mmol) was added, and the reaction was carried out at room temperature for 18 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 84. MS m/z(ESI): 598.2 [M+1]. ¹H NMR (400 MHz, DMSO) δ 12.60 (s, 1H), 9.39 (s, 2H), 8.65 (t, *J =* 5.8 Hz, 1H), 8.51 (d, *J =* 8.6 Hz, 2H), 8.07 - 7.89 (m, 3H), 6.92 (dd, *J =* 11.2, 4.8 Hz, 1H), 4.92 - 4.85 (m, 1H), 3.71 (dd, *J =* 12.6, 7.4 Hz, 1H), 3.44 - 3.35 (m, 2H), 3.26 (dd, *J =* 14.2, 8.2 Hz, 3H), 1.82 (dt, *J =* 29.2, 10.8 Hz, 6H), 1.63 - 1.38 (m, 2H).

### Example 85

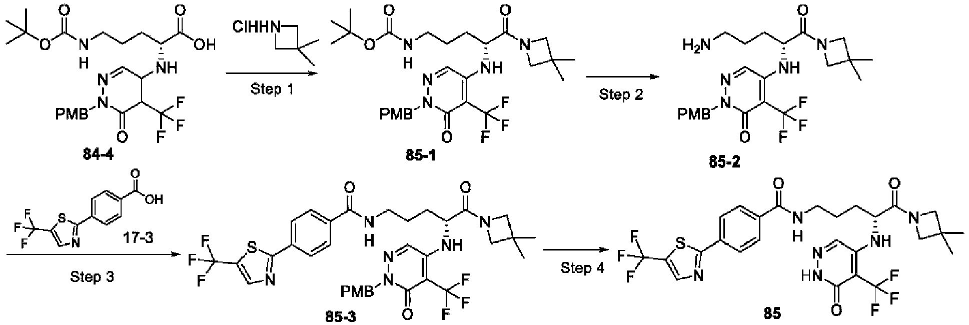

Step 1: Compound 84-4 (210 mg, 0.41 mmol) was dissolved in DMF (3 mL). Then, HATU (309.19 mg, 0.81 mmol) and DIPEA (0.34 mL, 2.03 mmol) were added sequentially. After stirring at room temperature for 5 minutes, compound 3,3-dimethylazetidine hydrochloride (49.44 mg, 0.41 mmol) was added. The reaction was carried out at room temperature for 2 hours. The reaction solution was added with 10 mL water, followed by extraction with EA (10 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:10) to afford compound 85-1. MS m/z(ESI): 526.4 [M+1].

Step 2: Compound 85-1 (60 mg, 0.11 mmol) was added to a solution of 4M hydrochloric acid in 1, 4-dioxane (1 mL). The reaction was carried out at room temperature for 1 hour. Compound 85-2 was afforded by concentration under reduced pressure. MS m/z(ESI): 482.4 [M+1].

Step 3: Compound 17-3 (22.7 mg, 0.08 mmol) was dissolved in DMF (2 mL). Then, HATU (63.17 mg, 0.17 mmol) and DIPEA (0.04 mL, 0.25 mmol) were added sequentially. After stirring at room temperature for 5 minutes, compound 85-2 (50 mg, 0.08 mmol) was added. The reaction was carried out at room temperature for 2 hours. The reaction solution was added with 2 mL water, followed by extraction with EA (3 mL×3). The organic phases were combined, and washed with saturated saline. The organic phases were dried over anhydrous sodium sulfate. After filtering to remove the desiccant, the filtrate was concentrated under reduced pressure. The residue was purified by preparative silica gel plate to afford compound 85-3. MS m/z(ESI): 737.4 [M+1].

Step 4: Compound 85-3 (30 mg, 0.04 mmol) was dissolved in TFA (1.5 mL), TfOH (0.2 mL) was added, and the reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 85. MS m/z(ESI): 617.3 [M+1]. ¹H NMR (400 MHz, DMSO-d6) δ 12.61 (s, 1H), 8.67 (m, 1H), 8.61 - 8.58 (m, 1H), 8.13 - 8.08 (m, 2H), 7.99 - 7.94 (m, 2H), 7.91 (s, 1H), 6.78 - 6.71 (m, 1H), 4.69 (d, *J=* 6.4 Hz, 1H), 4.01 (d, *J =* 8.4 Hz, 1H), 3.79 (d, *J =* 8.4 Hz, 1H), 3.63 (d, *J =* 9.4 Hz, 1H), 3.56 (d, *J =* 9.4 Hz, 1H), 3.27 (q, *J =* 6.6 Hz, 2H), 1.85 - 1.66 (m, 2H), 1.57 - 1.41 (m, 2H), 1.20 (d, *J* = 5.2 Hz, 6H).

### Example 86

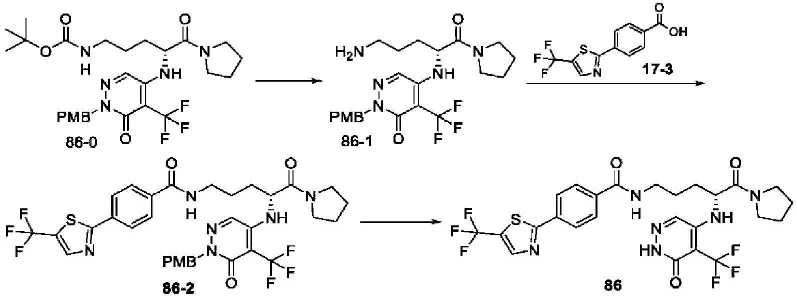

Step 1: Compound 86-0 (110 mg, 0.19 mmol) was added to a 1, 4-dioxane (1 mL) solution of 4M hydrochloric acid. The reaction was carried out at room temperature for 1 hour. Compound 86-1 was afforded by concentration under reduced pressure. MS m/z(ESI): 468.4 [M+1].

Step 2: Compound 17-3 (35.07 mg, 0.13 mmol) was dissolved in acetonitrile (1 mL). Then, TCFH (45.01 mg, 0.16 mmol) and N-methylimidazole (25.58 µL, 0.32 mmol) were added sequentially. After stirring at room temperature for 0.5 hours, compound 86-1 (50 mg, 0.11 mmol) was added. Under a nitrogen atmosphere, the reaction was carried out at room temperature for 16 hours. The reaction solution was concentrated under reduced pressure. The residue was added with 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:3) to afford compound 86-2. MS m/z(ESI): 723.4 [M+1].

Step 3: Compound 86-2 (76 mg, 0.11 mmol) was dissolved in TFA (1 mL), TfOH (5 µL, 0.055 mmol) was added, and the reaction was carried out at room temperature for 18 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-48%, flow rate: 30 mL/min) to afford compound 86. MS m/z(ESI): 603.2 [M+1]. ¹H NMR (400 MHz, DMSO) δ 12.59 (s, 1H), 8.65 (t, *J =* 5.4 Hz, 1H), 8.61 (d, *J =* 1.2 Hz, 1H), 8.11 (d, *J =* 8.4 Hz, 2H), 7.99 - 7.93 (m, 3H), 6.97 - 6.90 (m, 1H), 4.93 - 4.83 (m, 1H), 3.76 - 3.66 (m, 1H), 3.43 - 3.36 (m, 2H), 3.32 - 3.22 (m, 3H), 1.98 - 1.67 (m, 6H), 1.61 - 1.38 (m, 2H).

### Example 87

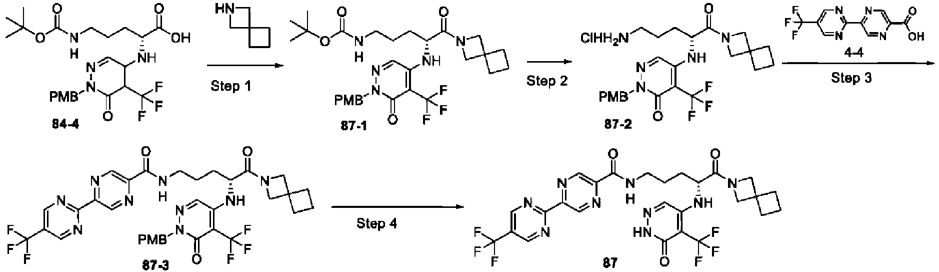

Step 1: Compound 84-4 (100 mg, 0.19 mmol) was dissolved in DMF (3 mL). Then, HATU (147.23 mg, 0.39 mmol) and DIPEA (0.19 mL, 1.16 mmol) were added sequentially. After stirring at room temperature for 5 minutes, compound 2-azaspiro[3.3]heptane (18.81 mg, 0.19 mmol) was added. The reaction was carried out at room temperature for 2 hours. The reaction solution was added with 10 mL water, followed by extraction with EA (5 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:10) to afford compound 87-1. MS m/z(ESI): 538.2 [M+1].

Step 2: Compound 87-1 (60 mg, 0.10 mmol) was added to a solution of 4M hydrochloric acid in 1, 4-dioxane (2 mL). The reaction was carried out at room temperature for 0.5 hours. Compound 87-2 was afforded by concentration under reduced pressure. MS m/z(ESI): 494.4 [M+1].

Step 3: Compound 4-4 (27.37 mg, 0.10 mmol) was dissolved in DMF (1 mL). Then, HATU (77.04 mg, 0.20 mmol) and DIPEA (0.05 mL, 0.30 mmol) were added sequentially. After stirring at room temperature for 5 minutes, compound 87-2 (50 mg, 0.10 mmol) was added. The reaction was carried out at room temperature for 2 hours. The reaction solution was added with 3 mL water, followed by extraction with EA (3 mL×3). The organic phases were combined, and washed with saturated saline. The organic phases were dried over anhydrous sodium sulfate. After filtering to remove the desiccant, the filtrate was concentrated under reduced pressure. The residue was purified by preparative silica gel plate to afford compound 87-3. MS m/z(ESI): 746.4 [M+1].

Step 4: Compound 87-3 (40 mg, 0.05 mmol) was dissolved in TFA (2.0 mL), TfOH (0.2 mL) was added, and the reaction was carried out at room temperature for 0.5 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 87. MS m/z(ESI): 626.4 [M+1]. ¹H NMR (400 MHz, DMSO-d6) δ 12.58 (s, 1H), 9.59 (s, 1H), 9.53 (s, 2H), 9.35 (s, 1H), 9.18 (s, 1H), 7.88 (s, 1H), 6.70 (t, *J =* 6.0 Hz, 1H), 4.66 (d, *J=* 6.4Hz,1H), 4.31 (d, *J* = 9.0 Hz, 1H), 4.09 (d, *J =* 9.0 Hz, 1H), 3.96 - 3.81 (m, 2H), 2.10 (q, *J =* 7.2 Hz, 4H), 1.83 - 1.64 (m, 4H), 1.61 - 1.43 (m, 2H).

### Example 88

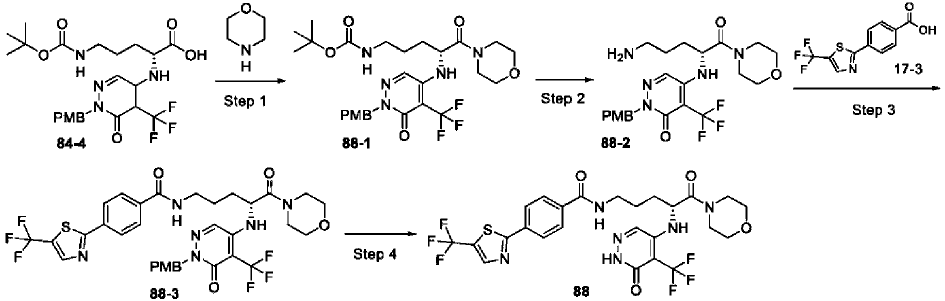

Step 1: 84-4 (200 mg, 0.39 mmol) was dissolved in DMF (3 mL). Then, HATU (294.46 mg, 0.77 mmol) and DIPEA (0.19 mL, 1.16 mmol) were added sequentially. After stirring at room temperature for 5 minutes, compound morpholine (0.03 mL, 0.39 mmol) was added. The reaction was carried out at room temperature for 2 hours. The reaction solution was added with 50 mL water, followed by extraction with EA (50 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:10) to afford compound 88-1. MS m/z(ESI): 528.4 [M+1].

Step 2: Compound 88-1(200 mg, 0.34 mmol) was added to a solution of 4M hydrochloric acid in 1, 4-dioxane (3 mL). The reaction was carried out at room temperature for 0.5 hours. Compound 88-2 was afforded by concentration under reduced pressure. MS m/z(ESI): 484.2 [M+1].

Step 3: Compound 17-3 (22.60 mg, 0.08 mmol) was dissolved in DMF (1 mL). Then, HATU (62.92 mg, 0.17 mmol) and DIPEA (0.04 mL, 0.25 mmol) were added sequentially. After stirring at room temperature for 5 minutes, compound 88-2 (40 mg, 0.08 mmol) was added. The reaction was carried out at room temperature for 2 hours. The reaction solution was added with 5 mL water, followed by extraction with EA (5 mL×3). The organic phases were combined, and washed with saturated saline. The organic phases were dried over anhydrous sodium sulfate. After filtering to remove the desiccant, the filtrate was concentrated under reduced pressure. The residue was purified by preparative silica gel plate to afford compound 88-3. MS m/z(ESI): 739.4 [M+1].

Step 4: Compound 88-3 (40 mg, 0.05 mmol) was dissolved in TFA (2.0 mL), TfOH (0.2 mL) was added, and the reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 88. MS m/z(ESI): 619.2 [M+1]. ¹H NMR (400 MHz, DMSO-d6) δ 12.60 (s, 1H), 8.66 (t, *J =* 5.6 Hz, 1H), 8.60 (d, *J =* 1.4 Hz, 1H), 8.15 - 8.07 (m, 2H), 8.01 (s, 1H), 7.99 - 7.91 (m, 2H), 7.03 -6.94 (m, 1H), 5.12 (d, *J* = 6.2 Hz, 1H), 3.70 - 3.51 (m, 9H), 3.25 (q, *J =* 6.6 Hz, 2H), 1.85 - 1.64 (m, 2H), 1.58 - 1.35 (m, 2H).

### Example 89

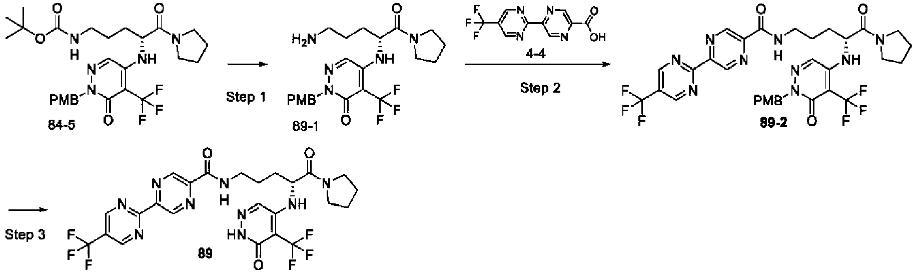

Step 1: Compound 84-5 (110 mg, 0.19 mmol) was added to a solution of 4M hydrochloric acid in 1, 4-dioxane (1 mL). The reaction was carried out at room temperature for 1 hour. Compound 89-1 was afforded by concentration under reduced pressure. MS m/z(ESI): 468.4 [M+1].

Step 2: Compound 4-4 (34.67 mg, 0.13 mmol) was dissolved in acetonitrile (1 mL). Then, TCFH (45.01 mg, 0.16 mmol) and N-methylimidazole (25.58 µL, 0.32 mmol) were added sequentially. After stirring at room temperature for 0.5 hours, compound 89-1 (50 mg, 0.11 mmol) was added. Under a nitrogen atmosphere, the reaction was carried out at room temperature for 16 hours. The reaction solution was concentrated under reduced pressure. The residue was added with 5 mL water, followed by extraction with EA (5 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1-1:2) to afford compound 89-2. MS m/z(ESI): 720.4 [M+1].

Step 3: Compound 89-2 (76 mg, 0.11 mmol) was dissolved in TFA (1 mL), TfOH (5 µL, 0.055 mmol) was added, and the reaction was carried out at room temperature for 18 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-48%, flow rate: 30 mL/min) to afford compound 89. MS m/z(ESI): 600.2 [M+1]. ¹H NMR (400 MHz, DMSO) δ 9.59 (d, *J =* 1.4 Hz, 1H), 9.54 (s, 2H), 9.35 (d, *J =* 1.4 Hz, 1H), 9.18 (t, *J =* 5.8 Hz, 1H), 7.97 (s, 1H), 6.94 - 6.87 (m, 1H), 4.92 - 4.83 (m, 1H), 3.77 - 3.65 (m, 1H), 3.43 - 3.36 (m, 2H), 3.33 - 3.27 (m, 3H), 1.94 - 1.70 (m, 6H), 1.64 - 1.42 (m, 2H).

### Example 90

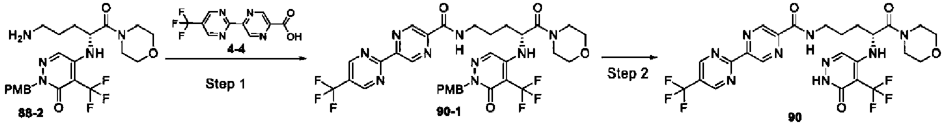

Step 1: Compound 4-4 (33.53 mg, 0.12 mmol) was dissolved in DMF (1 mL). Then, HATU (94.37 mg, 0.25 mmol) and DIPEA (0.06 mL, 0.37 mmol) were added sequentially. After stirring at room temperature for 5 minutes, compound 88-2 (60 mg, 0.12 mmol) was added. The reaction was carried out at room temperature for 2 hours. The reaction solution was added with 5 mL water, followed by extraction with EA (3 mL×3). The organic phases were combined, and washed with saturated saline. The organic phases were dried over anhydrous sodium sulfate. After filtering to remove the desiccant, the filtrate was concentrated under reduced pressure. The residue was purified by preparative silica gel plate to afford compound 90-1. MS m/z(ESI): 736.4 [M+1].

Step 2: Compound 90-1 (40 mg, 0.05 mmol) was dissolved in TFA (1.5 mL), TfOH (0.2 mL) was added, and the reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 90. MS m/z(ESI): 616.4 [M+1]. ¹H NMR (400 MHz, DMSO-d6) δ 12.57 (s, 1H), 9.59 (d, *J =* 1.4 Hz, 1H), 9.53 (s, 2H), 9.35 (d, *J =* 1.4 Hz, 1H), 9.19 (t, *J =* 6.0 Hz, 1H), 8.00 (s, 1H), 7.00 - 6.93 (m, 1H), 5.15 - 5.01 (m, 1H), 3.61 - 3.52 (m, 7H), 3.49 - 3.41 (m, 2H), 3.31 - 3.28 (m, 1H), 1.83 - 1.65 (m, 2H), 1.58 - 1.40 (m, 2H).

### Example 129

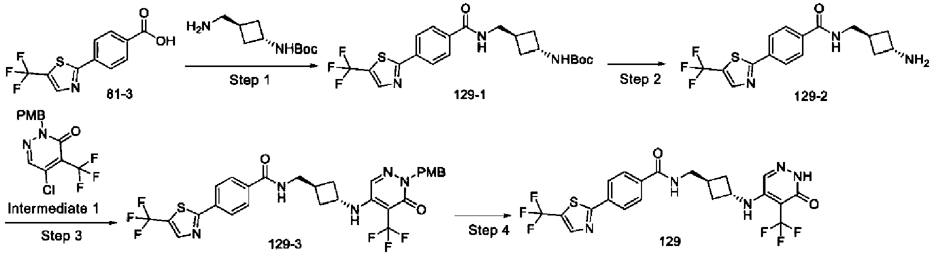

Step 1: Compound 81-3 (400 mg, 1.46 mmol), tert-butyl ((1r, 3r)-3-(aminomethyl)cyclobutyl)carbamate (291 mg, 1.46 mmol), HATU (834 mg, 2.20 mmol), and DIPEA (0.46 mL, 2.80 mmol) were added sequentially to DMF (4 mL). The system was reacted under stirring at room temperature for 2 hours, followed by adding EA (100 mL). The resulting mixture was washed with water (50 mL×3) and saturated saline (50 mL), respectively, dried over anhydrous sodium sulfate, and filtered. The filtrate was concentrated to dryness, and purified by silica gel column chromatography (EA/PE=0 to 50%) to afford compound 129-1. MS m/z(ESI):456.2 [M+1].

Step 2: Compound 129-1 (260 mg, 0.57 mmol) was added to DCM (3 mL). TFA (1 mL) was added. Then, the reaction was carried out at room temperature for 2 hours. The reaction solution was directly concentrated to dryness to afford compound 129-2. MS m/z(ESI):356.1 [M+1].

Step 3: Compound 129-2 (280 mg, 0.79 mmol) and intermediate 1 (157 mg, 0.79 mmol) were added sequentially to acetonitrile (5 mL). The system was heated to 100°C, followed by reacting under stirring for 18 hours. The reaction solution was concentrated to dryness, and purified by silica gel column chromatography (EA/PE=0 to 50%) to afford compound 129-3. MS m/z(ESI):638.2 [M+1].

Step 4: Compound 129-3 (100 mg, 0.16 mmol) was added to TFA (5 mL). Then, TfOH (0.5 mL) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 129. MS m/z(ESI):518.2 [M+1]. ¹H NMR (400 MHz, DMSO-*d*₆) δ 12.58 (s, 1H), 8.62 (d, *J =* 6.2 Hz, 1H), 8.60 (s, 1H), 8.12 - 8.09 (m, 2H), 8.03 (d, *J =* 15.1 Hz, 2H), 7.97 - 7.92 (m, 1H), 6.84 (s, 1H), 4.38 - 4.36 (m, 1H), 2.54 - 2.49 (m, 1H), 2.49-2.46 (m, 2H), 2.23 - 2.21 (m, 4H).

### Example 138

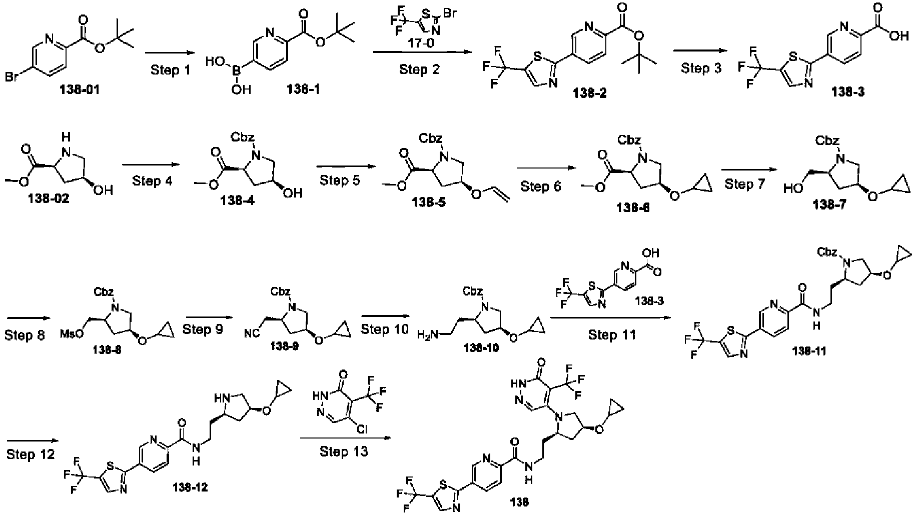

Step 1: Compound 138-01 (950 mg, 3.68 mmol) and 4,4,5,5-tetramethyl-2-(4,4,5,5-tetramethyl-1,3,2-dioxacyclopenten-2-yl)-1,3,2-dioxacyclopentane (1869.67 mg, 7.36 mmol) were dissolved in a solution of 1,4-dioxane (10 mL) and DMSO (2 mL). Bis[5-(diphenylphosphino)cyclopenta-1,3-dienyl]-λ2-iron (II) palladium chloride (269.15 mg, 0.37 mmol) and potassium acetate (1082.05 mg, 11.04 mmol) were added. The mixture was stirred at 90°C for 18 hours. The mixture was quenched with cold water and then concentrated to remove THF. The aqueous phase was extracted with EA (100 mL×3). The organic phases were then washed successively with saturated sodium bicarbonate (20 mL) and saturated saline (20 mL), and finally dried over anhydrous sodium sulfate. The resulting mixture was purified by silica gel chromatography (elution solution: PE: EA = 10: 1 to 1: 1) to afford compound 138-1 MS m/z(ESI):224.1 [M+1].

Step 2: Compound 17-0 (500 mg, 2.15 mmol) was dissolved in THF (20 mL). Compound 138-1 (0.56 mL, 2.37 mmol), palladium acetate (48.38 mg, 0.22 mmol), 4,5-bis(diphenylphosphino)-9,9-dimethylxanthene (249.39 mg, 0.43 mmol), and potassium phosphate tribasic (914.83mg, 4.31mmol) were added. The resulting mixture was stirred at 60°C for 18 h under a nitrogen atmosphere. After the reaction was completed, the mixture was poured into water (10 mL), followed by extraction with EA (10 mL×3). The combined organic phase was washed with brine (10 mL×3), and dried over anhydrous sodium sulfate. The residue was purified by silica gel column chromatography with an eluant system (EA/PE=1: 100 to 1: 5) to afford compound 138-2. MS m/z(ESI):289.0 [M+1].

Step 3: Compound 138-2 (240 mg, 0.73 mmol) was dissolved in DCM: TFA = 3: 1 (4 mL) solution. The resulting mixture was stirred at room temperature for 5 hours. After the reaction was completed, the resulting mixture was concentrated to afford compound 138-3, which was used directly in subsequent reactions. MS m/z(ESI):275.1 [M+1].

Step 4: At 0°C, methyl (2S, 4S)-4-hydroxyltetrahydropyrrole-2-carboxylate (13 g, 71.82 mmol) was dissolved in a solution of 1,4-dioxane : water = 1: 1 (4 mL). Sodium bicarbonate (12.07 g, 143.65 mmol) was added. The resulting mixture was stirred at 0°C for 0.5 hours. Then, benzyl chloroformate (12.25 mL, 86.19 mmol) was added to the mixture, followed by stirring continuously at 25°C for 6 hours. After the reaction was completed, the mixture was poured into water (10 mL), followed by extraction with EA (10 mL×3). The combined organic phase was washed with brine (10 mL×3), and dried over anhydrous sodium sulfate. The residue was purified by silica gel column chromatography with an eluant system (EA/PE=1/100 to 1/1) to afford compound 138-4. MS m/z(ESI):280.4 [M+1].

Step 5: In a condition of nitrogen protection, palladium acetate (1.13 g, 5.01 mmol) and 1,10-phenanthroline (0.84 mL, 5.51 mmol) were dissolved in 1,2-dichloroethane (20 mL), followed by stirring at room temperature for 10 min. Then, ethoxyethylene (60 mL, 624.05 mmol) was added thereto. The reaction mixture was stirred at 25°C for 20 min. Compound 138-4 (14 g, 50.13 mmol) was dissolved in 1,2-dichloroethane (20 mL). The above reaction mixture was added, followed by stirring at 80°C for 18 hours. The resulting mixture was concentrated and then diluted with water (10 mL), followed by extraction with EA (30 mL×3). The organic phases were combined, washed with brine (10 mL), dried over anhydrous sodium sulfate, filtered and concentrated, and purified by silica gel column chromatography with an eluant system (EA/PE=1/100-1/1) to afford compound 138-5. MS m/z(ESI):306.0 [M+1].

Step 6: At 0°C, under a nitrogen atmosphere, bis(ethane)zinc (63.87 mL, 63.87 mmol) was dissolved in 1,2-dichloroethane (20 mL) solution. A solution of diiodomethane (5.15 mL, 63.87mmol) in 1,2-dichloroethane (20 mL) was added dropwise. The reaction mixture was stirred at 0°C for 30 minutes. Then, at 0°C, a solution of compound 138-5 (6.5 g, 21.29 mmol) in 1,2-dichloroethane (20 mL) was added dropwise. The reaction mixture was heated to 80°C and stirred for 18 hours to afford the suspension. The suspension was concentrated under reduced pressure. The residue was added with 30 mL water, followed by extraction with EA (20 mL×3). The organic phases were combined, and washed with saturated saline solution. The organic phases were dried over anhydrous sodium sulfate. After filtering to remove the desiccant, the filtrate was concentrated under reduced pressure. The residue was purified by silica gel column chromatography with an eluant system (EA/PE=1/100-1/3) to afford compound 138-6. MS m/z(ESI):320.1 [M+1].

Step 7: Compound 138-6 (3600 mg, 11.27 mmol) was dissolved in THF (30 mL) and ethanol (30 mL), and calcium chloride (1251.03 mg, 11.27 mmol) and sodium borohydride (0.83 mL, 22.55 mmol) were slowly added at 0°C. The reaction mixture was heated to 250°C and stirred for 1 hour to afford the suspension. The suspension was concentrated under reduced pressure. The residue was added with 30 mL water, followed by extraction with EA (20 mL×3). The organic phases were combined, and washed with saturated saline solution. The organic phases were dried over anhydrous sodium sulfate. After filtering to remove the desiccant, the filtrate was concentrated under reduced pressure. The residue was purified by silica gel column chromatography with an eluant system (EA/PE=1/100-1/1) to afford compound 138-7. MS m/z(ESI): 292.2 [M+1].

Step 8: Methylsulfonyl chloride (1.12 mL, 14.42 mmol) and triethylamine (4.00 mL, 28.83 mmol) were added to a solution of compound 138-7 (2800 mg, 9.61 mmol) in DCM (10 mL). The resulting mixture was stirred at 25°C for 2 hours. The mixture was concentrated to dryness under reduced pressure to afford compound 138-8. MS m/z(ESI): 370.2 [M+1].

Step 9: Compound 138-8 (3550 mg, 9.61 mmol) was dissolved in DMF (15 mL). Trimethylsilyl cyanide (3.62 mL, 28.83 mmol) and potassium carbonate (3984.06 mg, 28.83 mmol) were added. The resulting mixture was stirred at 80°C for 50 hours. The mixture was poured into EtOAc (50 mL), followed by washing with water (50 mL×3). The combined organic phase was washed with brine (50 mL), dried over anhydrous sodium sulfate, filtered, and concentrated to dryness under reduced pressure, to afford a crude product. The crude product was purified by silica gel chromatography (PE/EA =100/1- 10/1) to afford compound 138-9. MS m/z(ESI): 301.2 [M+1].

Step 10: At 0°C, borane (19.98 mL, 19.98 mmol) was added to a solution of compound 138-9 (1000 mg, 3.33 mmol) in THF (10 mL). The resulting mixture was stirred at 25°C for 16 hours in a nitrogen environment. The reaction solution was concentrated under reduced pressure. The residue was added with 10 mL water, followed by extraction with EA (15 mL×3). The organic phases were combined, and washed with saturated saline. The organic phases were dried over anhydrous sodium sulfate. The filtrate was concentrated under reduced pressure, and purified by preparative high performance liquid chromatography, to afford compound 138-10. MS m/z(ESI): 305.2[M+1].

Step 11: Compound 138-3 (273 mg, 1. 00 mmol) was dissolved in DMF (3 mL). Compound 138-10 (330 mg, 1.08 mmol), HATU (613.30 mg, 1.61 mmol), and DIPEA (0.94 mL, 5.40 mmol) were added. The resulting mixture was stirred at 25°C for 2 hours. The reaction solution was concentrated under reduced pressure. The residue was added with 10 mL water, followed by extraction with EA (15 mL × 3). The organic phases were combined, and washed with saturated saline. The organic phases were dried over anhydrous sodium sulfate. After filtering to remove the desiccant, the filtrate was concentrated under reduced pressure. The residue was purified by silica gel chromatography (PE/EA = 100/1-5/1) to afford compound 138-11. MS m/z(ESI): 561.2 [M+1].

Step 12: Compound 138-11 (300 mg, 0.54 mmol) was dissolved in TFA (6 mL), followed by reacting under stirring at 25°C for 48 hours. The reaction solution was concentrated under reduced pressure. The residue was purified by silica gel chromatography (DCM/methanol =100/1-7/1) to afford compound 138-12. MS m/z(ESI): 427.2 [M+1].

Step 13: Compound 138-12 (40 mg, 0.09 mmol) was dissolved in acetonitrile (5 mL). 5-chloro-4-(trifluoromethyl)-2H,3H-1,2-diazin-3-one (18.62 mg, 0.09 mmol) and DIPEA (24.20 mg, 0.19 mmol) were added. Then, the resulting mixture was stirred at 60°C for 2 hours. The reaction solution was concentrated under reduced pressure and purified by high performance liquid chromatography (Waters-SunFire-C18-10µm-19×250mm; mobile phase: water (containing 10 mmol/L TFA) and acetonitrile, gradient ratio: acetonitrile 47%-95%, flow rate: 25 mL/min) to afford compound 138. MS m/z(ESI): 589.2 [M+1]. ¹H NMR (400 MHz, CDCl₃) δ 10.69 (s, 1H), 9.13 (d, *J =* 1.6 Hz, 1H), 8.45 - 8.35 (m, 1H), 8.30 (d, *J* = 8.2 Hz, 1H), 8.25 - 8.13 (m, 2H), 7.73 (s, 1H), 4.38 - 4.22 (m, 1H), 4.18 - 4.04 (m, 1H), 3.75 - 3.58 (m, 3H), 3.55 - 3.42 (m, 1H), 3.37 - 3.27 (m, 1H), 2.74 - 2.59 (m, 1H), 2.09 - 1.75 (m, 3H), 0.66 - 0.48 (m, 4H).

### Example 151

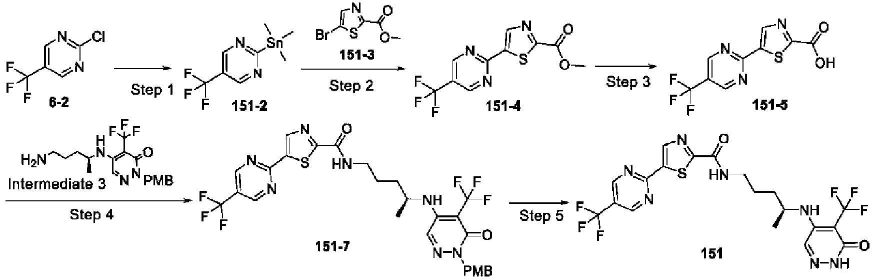

Step 1: Hexamethylditin (2.73 mL, 13.15 mmol) and Pd(PPh₃)₄ (1.27 g, 1.10 mmol) were added to a solution of compound 6-2 (2 g, 10.9 mmol) in toluene (20 mL). The mixture was reacted under stirring at 100°C for 2 hours under a nitrogen atmosphere. The mixture was concentrated to afford the crude product. The crude product was purified by neutral alumina column chromatography (elution solution: PE: EA=500:1 to 3:1) to afford compound 151-2. MS m/z(ESI): 311.0[M+1].

Step 2: Compound 151-2 (500 mg, 1.61 mmol), bis(tri-tert-butylphosphine)palladium (82 mg, 0.16 mmol), and cesium fluoride (488 mg, 3.21 mmol) were added to a solution of compound 151-3 (400 mg, 1.80 mmol) in1,4-dioxane (15 mL). Under a nitrogen atmosphere, the mixture was reacted under stirring at 100°C for 18 hours. The mixture was concentrated to afford the crude product. The crude product was purified by silica gel chromatography (elution solution: PE: EA= 10: 1 to 1: 1) to afford compound 151-4. MS m/z(ESI): 290.0[M+1].

Step 3: LiOH monohydrate (50 mg, 1.19 mmol) was added to a solution of compound 151-4 (120 mg, 0.41 mmol) in THF (3 mL) and water (1 mL). The mixture was stirred at 25°C for 2 hours. The mixture was concentrated and diluted with water (5 mL). Then, the mixture was adjusted to pH = 7 with 2M hydrochloric acid aqueous solution, to form a suspension. The filter cake was filtered and concentrated to afford compound 151-5. MS m/z(ESI): 276.0[M+1].

Step 4: Intermediate 3 (130.70 mg, 0.34 mmol), HATU (160 mg, 0.42 mmol), and DIPEA (130 mg, 1.01 mmol) were added to a solution of compound 151-5 (94 mg, 0.34 mmol) in DMF (3 mL). The mixture was reacted under stirring at 25°C for 18 hours under a nitrogen atmosphere. The mixture was poured into water (20 mL), followed by extraction with EA (30 mL×3). The combined organic phase was washed with brine (10 mL), dried over anhydrous sodium sulfate, filtered, and concentrated under reduced pressure to afford the crude product. The crude product was purified by silica gel chromatography (elution solution: DCM: methanol = 100: 1 to 20: 1) to afford compound 151-7. MS m/z(ESI): 641.8[M+1].

Step 5: TfOH (0.1 mL) was added to a solution of compound 151-7 (100 mg, 0.16 mmol) in TFA (2.5 mL). The mixture was stirred at 25°C for 18 hours. The reaction solution was concentrated to afford the reaction solution of crude product, and the reaction solution of crude product was concentrated under reduced pressure and purified by high performance liquid chromatography (Gilson_306_1741, chromatographic column: Waters-SunFire-C18-10µm-19×250mm; mobile phase: water (containing 0.1% formic acid) and acetonitrile, gradient ratio: acetonitrile 42%-95%, flow rate: 25 mL/min) to afford compound 151. MS m/z(ESI): 522.2[M+1]. ¹H NMR (400 MHz, DMSO) δ 12.43 (s, 1H), 9.35 (s, 2H), 9.11 (t, *J =* 6.0 Hz, 1H), 8.80 (s, 1H), 7.92 (s, 1H), 6.40 - 6.23 (m, 1H), 4.07 - 3.89 (m, 1H), 3.31 - 3.27 (m, 2H), 1.73 - 1.63 (m, 1H), 1.62 - 1.46 (m, 3H), 1.18 (d, *J =* 6.4 Hz, 3H).

### Example 155

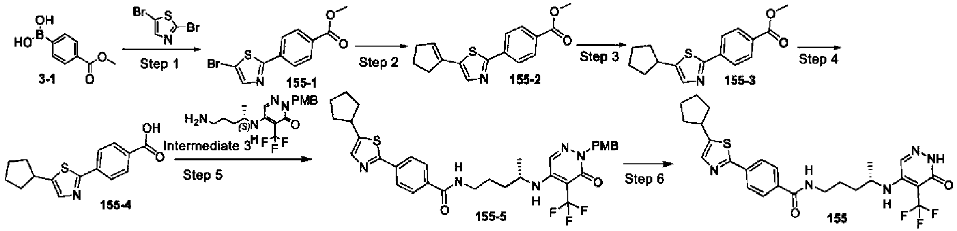

Step 1: Compound 3-1 (1.00 g, 5.56 mmol) and 2,5-dibromothiazole (1.34 g, 5.56 mmol) were dissolved in 1,4-dioxane (10 mL). Then, palladium acetate (251 mg, 1.11 mmol), 4,5-bis(diphenylphosphino)-9,9-dimethylxanthene (643 mg, 1.11 mmol), and potassium phosphate tribasic (3.53 g, 16.6 mmol) were added sequentially. The reaction was carried out at 100°C for 16 hours in a nitrogen environment. The reaction solution was concentrated under reduced pressure. The residue was purified by silica gel column chromatography with an eluant system (PE:EA=10:1-5: 1) to afford compound 155-1. MS m/z(ESI):297.9[M+1].

Step 2: Compound 155-1 (250 mg, 0.84 mmol) and cyclopenten-1-ylboronic acid (187 mg, 1.68 mmol) were dissolved in 1,4-dioxane (4.00 mL) and water (1.00 mL). Then, [1,1'-bis(diphenylphosphino)ferrocene]dichloropalladiumdichloromethane complex (122 mg, 0.17 mmol) and potassium carbonate (347 mg, 2.52 mmol) were added sequentially. The reaction was carried out at 100°C for 16 hours in a nitrogen environment. The reaction solution was concentrated under reduced pressure. The residue was purified by silica gel column chromatography with an eluant system (PE:EA=100:1-10: 1) to afford compound 155-2. MS m/z(ESI):286.0[M+1].

Step 3: Compound 155-2 (200 mg, 0.70 mmol) was dissolved in methanol (5.00 mL), and Pd/C (10%) (74.5 mg, 0.70 mmol) was added. The reaction was carried out at room temperature for 18 hours in a hydrogen environment. The reaction solution was filtered and the filter cake was washed with EA (5 mL×3). The filtrate was concentrated under reduced pressure to afford compound 155-1. MS m/z(ESI):288.0[M+1].

Step 4: Compound 155-3 (140 mg, 0.49 mmol) was dissolved in THF (3 mL), methanol (1 mL), and water (1 mL). LiOH (61.3 mg, 1.46 mmol) was added. The reaction was carried out at room temperature for 2 hours. The reaction mixture was adjusted to pH = 6 with HCl (1M). The mixture was poured into water (20 mL), followed by extraction with EA (30 mL×3). The combined organic phase was washed with brine (10 mL), dried over anhydrous sodium sulfate, filtered, and concentrated to dryness under reduced pressure, to afford compound 155-4. MS m/z(ESI):274.0[M+1].

Step 5: Compound 155-4 (80.0 mg, 0.29 mmol) was dissolved in DMF (2.00 mL). Then, HATU (222 mg, 0.59 mmol) and DIPEA (0.15 mL, 0.88 mmol) were added sequentially. After stirring at room temperature for 5 minutes, intermediate 3 (112 mg, 0.29 mmol) was added. The reaction was carried out at room temperature for 2 hours. The reaction solution was added with 20 mL water, followed by extraction with EA (30 mL×3). The organic phases were combined, and washed with saturated saline. The organic phases were dried over anhydrous sodium sulfate, and filtered. After removing the desiccant, the filtrate was concentrated under reduced pressure. The residue was purified by preparative silica gel plate (PE:EA=1:1) to afford compound 155-5. MS m/z(ESI):640.4[M+1].

Step 6: Compound 155-5 (100 mg, 0.16 mmol) was dissolved in TFA (1.00 mL), and TfOH (0.20 mL) was added. The reaction was carried out at room temperature for 1 hour. The reaction solution was concentrated under reduced pressure and purified by preparative high performance liquid chromatography (Phenomenex Gemini 150 mm×25 mm×10 µm column, elution solution:30%-60% (v/v) acetonitrile and water, 0.025% formic acid) to afford compound 155. MS m/z(ESI): 520.3 [M+1]. ¹H NMR (400 MHz, DMSO-d6) δ 12.43 (s, 1H), 8.57 (t, J = 5.6 Hz, 1H), 7.99 - 7.86 (m, 5H), 7.70 (s, 1H), 6.38 - 6.28 (m, 1H), 4.04 - 3.92 (m, 1H), 3.32 - 3.22 (m, 3H), 2.19 - 2.07 (m, 2H), 1.82 - 1.72 (m, 2H), 1.72 - 1.49 (m, 8H), 1.18 (d, J = 6.4 Hz, 3H).

### Example 164

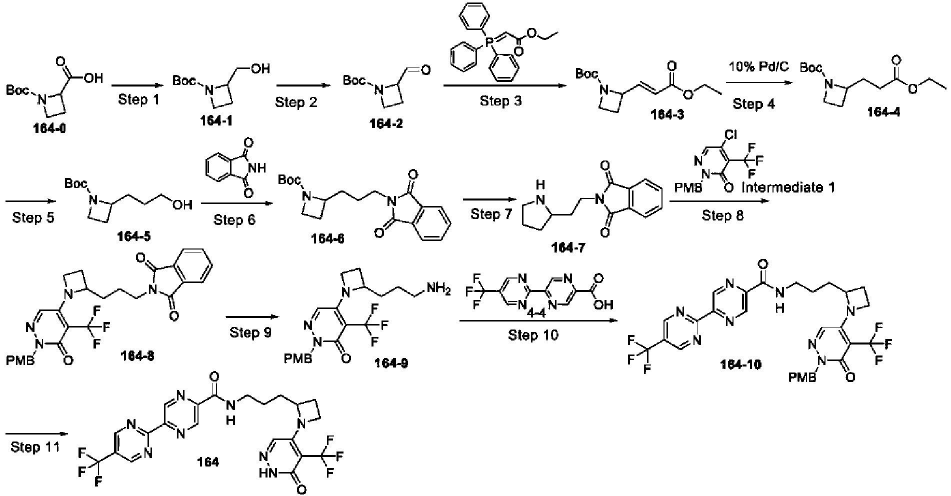

Step 1: At 0°C under a nitrogen atmosphere, a solution of borane (50 mmol) in THF(50 mL) was added to a solution of compound 164-0 (2 g, 9.94 mmol) in THF (10 mL). The mixture was reacted under stirring at 25°C for 18 hours under a nitrogen atmosphere. The mixture was quenched with cold water and then concentrated to remove THF. The aqueous phase was extracted with EA (100 mL×3). Then, the organic phases were washed with saturated sodium bicarbonate solution (20 mL) and saturated saline (20 mL), and finally dried over anhydrous sodium sulfate, filtered and concentrated to afford compound 164-1. MS m/z(ESI):132.1 [M-56+H].

Step 2: At -78°C, under a nitrogen atmosphere, DMSO (0.95 mL, 13.35 mmol) was slowly added dropwise to a solution of oxalyl chloride (0.69 mL, 8.01 mmol) in DCM (25 mL). The solution was stirred for 30 minutes. Compound 164-1 (1.0 g, 5.34 mmol) was added dropwise to the solution. The mixture was stirred at -78°C for 30 minutes under a nitrogen atmosphere. Then, triethylamine (3.70 mL, 26.70 mmol) was slowly added dropwise to the reaction solution. Then, the temperature was raised to 25°C, and stirring was continued for 30 minutes. After the reaction was completed, the mixture was poured into water (10 mL), followed by extraction with EA (10 mL×3). The combined organic phase was washed with brine (10 mL×3), dried over anhydrous sodium sulfate, filtered and concentrated to afford compound 164-2. MS m/z(ESI):130.1 [M-56+H].

Step 3: (triphenyl-λ5-phosphanylidene) EA (1.88 g, 5.40 mmol) was added to a solution of compound 164-2 (1 g, 5.40 mmol) in THF (20 mL). The mixture was stirred at 25°C for 18 hours under a nitrogen atmosphere. After the reaction was completed, the crude product was afforded by concentration. The crude product was purified by silica gel chromatography (elution solution: PE: EA = 100: 1 to 2: 1) to afford compound 164-3. MS m/z(ESI):200.0 [M-56+H].

Step 4: Pd/C (10%) (20 mg, 0.20 mmol) was added to a solution of compound 164-3 (870 mg, 3.41 mmol) in ethanol (10 mL). The mixture was stirred at 25°C for 2 hours under a hydrogen atmosphere. Compound 164-4 was afforded by filtering and concentration. MS m/z(ESI):280.2 [M+Na].

Step 5: Sodium borohydride (2.38 g, 63.00 mmol) was added to a solution of compound 164-4 (810 mg, 3.15 mmol) in methanol (10 mL). The mixture was stirred at 25°C for 48 hours. The mixture was concentrated and diluted with water (10 mL), and then extracted with EA (30 mL×3). The combined organic phase was washed with brine (10mL), dried over anhydrous sodium sulfate, filtered and concentrated, to afford compound 164-5. MS m/z(ESI):160.2 [M-56+H].

Step 6: At 0°C, 2H-isoindol-1,3-dione (180 mg, 1.22 mmol) was dissolved in THF (2 mL) solution. Compound 164-5 (500 mg, 1.39 mmol), triphenylphosphine (479.99 mg, 1.83 mmol), and diisopropyl azodicarboxylate (369.66 mg, 1.83mmol) were added, followed by stirring for 2 hours under a nitrogen atmosphere. The reaction solution was concentrated under reduced pressure. The residue was added with 10 mL water, followed by extraction with EA (15 mL×3). The organic phases were combined, and washed with saturated sodium bicarbonate aqueous solution. The organic phases were dried over anhydrous sodium sulfate. After filtering to remove the desiccant, the filtrate was concentrated under reduced pressure. The residue was purified by silica gel column chromatography with an eluant system (EA/PE=1/100-1/3) to afford compound 164-6. MS m/z(ESI):367.2 [M+23].

Step 7: Compound 164-6 (304 mg, 0.88 mmol) was dissolved in a mixed solution of DCM: TFA=5:1 (3 mL). The reactant was stirred at 25°C for 3 hours. The reaction solution was concentrated under reduced pressure to afford compound 164-7, which was used directly in the next step. MS m/z(ESI): 245.5 [M+1].

Step 8: At room temperature, compound 164-7 (215 mg, 0.88 mmol) was dissolved in isopropanol (5 mL) solution. Intermediate 1 (420.71 mg, 1.32 mmol) and DIPEA (0.77 mL, 4.40 mmol) were added. Then, the resulting mixture was stirred at 60°C for 16 hours. After completion, the reaction mixture was cooled to room temperature and concentrated under reduced pressure. The residue was purified by silica gel column chromatography with an eluant system (EA/PE=1/100-1/2) to afford compound 164-8. MS m/z(ESI): 527.2 [M+1].

Step 9: Compound 164-8 (304 mg, 0.58 mmol) was dissolved in ethanol (10 mL). Hydrazine hydrate (721.72 mg, 11.55 mmol) was added to the mixture. The resulting mixture was stirred at 80°C for 1 hour. The mixture was filtered, and the filtered solid was washed with ethanol (3 mL×2). The filtrate was concentrated to dryness under reduced pressure to afford the crude product. Then, the crude product was added to acetonitrile (10 mL), the mixture was filtered, and the filtered solid was washed with ethanol. The filtrate was concentrated to dryness under reduced pressure to afford compound 164-9, which was used directly in the next step. MS m/z(ESI): 397.2 [M+1].

Step 10: Compound 164-9 (30 mg, 0.11 mmol) was dissolved in DMF (4 mL) solution. Compound 4-4 (52.82 mg, 0.13 mmol), HATU (63.29 mg, 0.17 mmol), and DIPEA (42.97 mg, 0.33 mmol) were added, followed by reacting under stirring at 25°C for 3 hours. The reaction solution was concentrated under reduced pressure. The residue was added with 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure to afford compound 164-10. MS m/z(ESI): 649.4 [M+1].

Step 11: Compound 164-10 (80 mg, 0.10 mmol) was dissolved in TFA (3 mL) solution. TfOH (0.04 mL, 0.50 mmol) was added, followed by reacting under stirring at 25°C for 2 hours. The reaction solution was concentrated under reduced pressure and purified by high performance liquid chromatography (Waters-SunFire-C18-10 µm-19×250 mm; mobile phase: water (containing 10 mmol/L formic acid) and acetonitrile, gradient ratio: acetonitrile 36%-95%, flow rate: 25 mL/min) to afford compound 164. MS m/z(ESI): 529.2 [M+1]. ¹H NMR (400 MHz, CDCl₃) δ 10.60 (s, 1H), 9.73 - 9.65 (m, 1H), 9.63 - 9.56 (m, 1H), 9.22 (s, 2H), 8.04 - 7.86 (m, 1H), 7.64 (s, 1H), 5.74 - 5.51 (m, 3H), 3.63 - 3.57 (m, 2H), 3.44 - 3.37 (m, 2H), 2.49 - 2.39 (m, 4H).

### Example 166

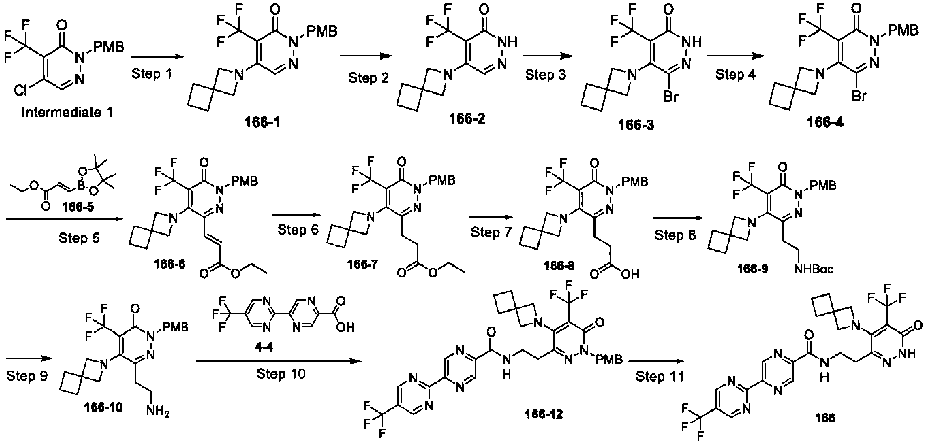

Step 1: At room temperature, intermediate 1 (636 mg, 2.00 mmol) was dissolved in isopropanol (10 mL). 2-azaspiro[3.3]heptane (194 mg, 2.00 mmol) and DIPEA (0.99 mL, 5.99 mmol) were added to the reaction solution. The reaction was carried out at 80°C for 18 hours. After the reaction was completed, the resulting mixture was concentrated under reduced pressure and dried. The crude was purified by silica gel column (eluant: EA/PE=5%-20%) to afford compound 166-1. MS m/z (ESI): 380.1[M+1].

Step 2: At room temperature, compound 166-1 (2 g, 5.28 mmol) was dissolved in TFA (20 mL), TfOH (1.88 mL, 21.08 mmol) was added to the reaction solution, and the reaction was carried out at room temperature for 4 hours. After the reaction was completed, the resulting mixture was concentrated under reduced pressure and dried. The residue was adjusted to pH 8-9 with sodium bicarbonate saturated solution, followed by extraction with DCM (50 mL×3). The organic phases were combined, dried over anhydrous sodium sulfate, filtered and then concentrated under reduced pressure to afford compound 166-2. MS m/z (ESI): 260.0 [M+1].

Step 3: At room temperature, compound 166-2 (1.5 g, 5.21 mmol) and sodium acetate trihydrate (1.06 g, 7.81 mmol) were dissolved in acetonitrile (10 mL). Then, liquid bromine (1.25 g, 7.81 mmol) was added, followed by stirring at 80°C for 2 hours. After the reaction was completed, the crude was afforded by concentration under reduced pressure. The crude was purified by silica gel column (eluant: EA: PE = 16%-25%) to afford compound 166-3. MS m/z (ESI): 338.0, 340.0 [M+1].

Step 4: At room temperature, compound 166-3 (1.5 g, 4.44 mmol) was dissolved in DMF (20 mL), and potassium carbonate (1.84 g, 13.31 mmol) and 4-methoxybenzylchloride (906.19 µL, 6.65 mmol) were added. The reaction was carried out at room temperature for 18 hours. After the reaction was completed, the reaction solution was poured into water (30 mL), followed by extraction with EA (50 mL×3). The organic phases were combined, washed with saturated saline (30 mL), dried over anhydrous sodium sulfate, filtered and then concentrated under reduced pressure to afford the crude. The crude was purified by silica gel column (eluant: EA: PE = 10%-20%) to afford compound 166-4. MS m/z (ESI): 458.2, 460.2 [M+1].

Step 5: Under nitrogen protection, compound 166-4 (1.10 g, 2.77 mmol) and compound 166-5 (0.81 g, 3.60 mmol) were dissolved in 1,4-dioxane (15 mL) and water (3 mL). Potassium carbonate (1.00 g, 7.2 mmol) and DPPFdichloropalladium (0.18 g, 0.24 mmol) were added, followed by stirring at 80°C for 2 hours. After the reaction was completed, the reaction solution was poured into water (30 mL), followed by extraction with EA (50 mL×3). The organic phases were combined, washed with saturated saline (30 mL), dried over anhydrous sodium sulfate, filtered and then concentrated under reduced pressure to afford the crude. The crude was purified by silica gel column (eluant: EA: PE = 15%-20%) to afford compound 166-6. MS m/z (ESI): 478.2 [M+1].

Step 6: Under hydrogen protection, compound 166-6 (0.9 g, 2.77 mmol) was dissolved in THF (15 mL). Pd/C (10%) (200.59 mg, 0.19 mmol) was added. The reaction solution was stirred at 25°C for 3 hours after purging with hydrogen three times. After the reaction was completed, the reaction solution was filtered. The filter cake was washed with EA (50 mL). The filtrate was concentrated under reduced pressure to afford compound 166-7. MS m/z (ESI): 480.2 [M+1].

Step 7: At room temperature, compound 166-7 (779 mg, 1.46 mmol) was dissolved in methanol (5 mL), THF (5 mL), and water (5 mL). Potassium hydroxide (184.06 mg, 4.39 mmol) was added. The reaction solution was stirred at 25°C for 2 hours. After the reaction was completed, the reaction solution was concentrated under reduced pressure and dried. The residue was adjusted to pH 3-4 with hydrochloric acid aqueous solution (1M), followed by extraction with DCM (20 mL×3). The organic phases were combined, dried over anhydrous sodium sulfate, filtered and then concentrated under reduced pressure to afford compound 166-8. MS m/z (ESI): 452.2 [M+1].

Step 8: Compound 166-8 (300 mg, 0.66 mmol) was dissolved in t-butyl alcohol (5 mL). DPPA (144.00 µL, 0.66 mmol) and triethylamine (92.00 µL, 0.66 mmol) were added, followed by stirring at 100°C for 2 hours. After the reaction was completed, the reaction solution was poured into water (20 mL), followed by extraction with EA (20 mL×3). The organic phases were combined, washed with saturated saline (20 mL), dried over anhydrous sodium sulfate, filtered and then concentrated under reduced pressure to afford compound 166-9. MS m/z (ESI): 523.4 [M+1].

Step 9: At room temperature, compound 166-9 (120 mg, 0.23 mmol) was dissolved in DCM (5 mL). TFA (1.49 mL, 20.02 mmol) was added, followed by stirring at 25°C for 1 hour. After the reaction was completed, the reaction solution was concentrated under reduced pressure to afford compound 166-10. MS m/z (ESI): 395.2 [M+1].

Step 10: At room temperature, compound 166-10 (50 mg, 0.12 mmol) was dissolved in DMF (2 mL). Compound 4-4 (25.94 mg, 0.10 mmol), HATU (68.44 mg, 0.18 mmol), and DIPEA (59.65 µL, 0.36 mmol) were added, followed by stirring at 25°C for 18 hours. After the reaction was completed, the reaction solution was poured into water (30 mL), followed by extraction with EA (50 mL×3). The organic phases were combined, washed with saturated saline (30 mL), dried over anhydrous sodium sulfate, filtered and then concentrated under reduced pressure to afford compound 166-12. MS m/z (ESI): 675.4 [M+1].

Step 11: At room temperature, compound 166-12 (50 mg, 0.07 mmol) was dissolved in TFA (1 mL). Then, TfOH (0.2 mL) was added, followed by stirring at 25°C for 0.5 hours to afford the crude product. Then, the crude product was purified by preparative HPLC (chromatographic column: Waters-Xbridge-C18-10µm-19×250 mm; mobile phase: A: 10 mM ammonium bicarbonate/water B: acetonitrile, gradient ratio: acetonitrile 36%-66%, flow rate: 20 mL/min) to afford compound 166. MS m/z (ESI):555.2 [M+1]. ¹H NMR (400 MHz, DMSO-*d6*) *δ* 10.73 (s, 1H), 9.72 (d, *J* = 1.2 Hz, 1H), 9.58 (d, *J =* 1.2 Hz, 1H), 9.22 (d, *J* = 0.9 Hz, 2H), 8.35 - 8.19 (m, *J* = 6.2 Hz, 1H), 4.41 (s, 4H), 3.86 (m, *J =* 6.4 Hz, 2H), 2.98 (m, *J =* 6.4 Hz, 2H), 2.22 (t, *J =* 7.6 Hz, 4H), 1.90 - 1.89 (m, 2H).

### Example 174

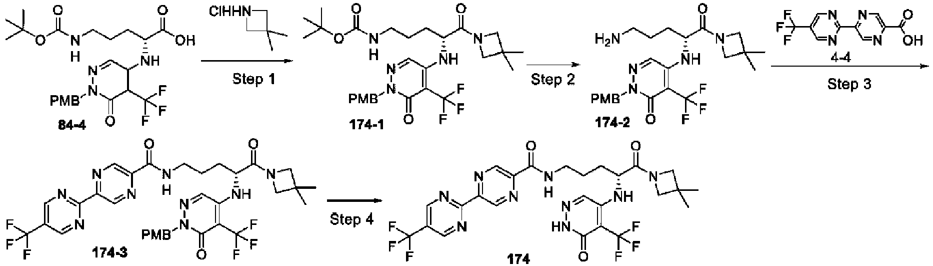

Step 1: Compound 84-4 (190 mg, 0.37 mmol) was dissolved in DMF (5 mL). Then, TCFH (279 mg, 0.74 mmol) and DIPEA (0.30 mL, 1.84 mmol) were added sequentially. After stirring at room temperature for 5 minutes, 3,3-dimethylazetidine hydrochloride (44.7 mg, 0.37 mmol) was added. The reaction was carried out at room temperature for 2 hours. The reaction solution was added with 50 mL water, followed by extraction with EA (50 mL× 3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=10:1-1/1) to afford compound 174-1. MS m/z(ESI):582.4[M+1].

Step 2: Compound 174-1 (60 mg, 0.10 mmol) was added to a solution of 4 M hydrochloric acid in 1, 4-dioxane (1.50 ml). The reaction was carried out at room temperature for 1 hour. Compound 174-2 was afforded by concentration under reduced pressure. MS m/z(ESI):482.4[M+1].

Step 3: Compound 4-4 (18.5 mg, 0.07 mmol) was dissolved in DMF (1.00 mL). Then, TCFH (52.1 mg, 0.14 mmol) and DIPEA (0.03 mL, 0.21 mmol) were added sequentially. After stirring at room temperature for 5 minutes, compound 174-2 (33.0 mg, 0.07 mmol) was added. The reaction was carried out at room temperature for 1 hour. The reaction solution was added with 20 mL water, followed by extraction with EA (30 mL×3). The organic phases were combined, and washed with saturated saline. The organic phases were dried over anhydrous sodium sulfate. After filtering to remove the desiccant, the filtrate was concentrated under reduced pressure. The residue was purified by preparative silica gel plate (PE:EA=1:1) to afford compound 174-3. MS m/z(ESI):734.4[M+1].

Step 4: Compound 174-3 (20 mg, 0.03 mmol) was dissolved in TFA (1.50 mL), and TfOH (0.20 mL) was added. The reaction was carried out at room temperature for 1 hour. The reaction solution was concentrated under reduced pressure and purified by preparative high performance liquid chromatography (Phenomenex Gemini 150 mm×25 mm×10 µm column, elution solution:30%-60% (v/v) acetonitrile and water, 0.025% formic acid) to afford compound 174. MS m/z(ESI): 614.4 [M+1]. ¹H NMR (400 MHz, DMSO-d6) δ 12.56 (s, 1H), 9.59 (d, J = 1.4 Hz, 1H), 9.53 (s, 2H), 9.35 (d, J = 1.4 Hz, 1H), 9.17 (t, J = 6.0 Hz, 1H), 8.45 (s, 1H), 7.89 (s, 1H), 6.77 - 6.68 (m, 1H), 4.72 - 4.64 (m, 1H), 4.01 (d, J = 8.4 Hz, 1H), 3.79 (d, J = 8.4 Hz, 1H), 3.62 (d, J = 9.4 Hz, 1H), 3.56 (d, J = 9.4 Hz, 1H), 1.85 - 1.45 (m, 5H), 1.19 (d, J = 5.8 Hz, 6H).

### Example 175

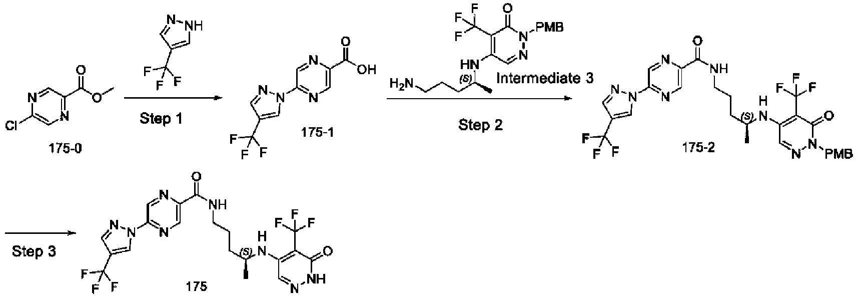

Step 1: Compound 175-0 (300 mg, 1.74 mmol, Bide), 4-(trifluoromethyl)-1H-pyrazole (260.22 mg, 1.91 mmol), and cesium carbonate (736.34 mg, 2.26 mmol) were added to N,N-dimethylformamide (6 mL). Cuprous iodide (165.54 mg, 0.87 mmol) was added, followed by purging with nitrogen three times. The reaction was carried out at 120°C for 18 hours. The reaction solution was filtered and purified by high performance liquid chromatography (Waters-2545, chromatographic column: SharpSil-T C18, 30×150 mm, 5 µm; mobile phase: water (containing 0.5% TFA) and acetonitrile, gradient ratio: acetonitrile 50%-80%, flow rate: 30 mL/min) to afford compound 175-1. MS m/z(ESI):259.0[M+1].

Step 2: Compound 175-1 (70 mg, 0.27 mmol) was dissolved in acetonitrile (2 mL). Then, TCFH (114.12 mg, 0.41 mmol) and N-methylimidazole (64.85 µL, 0.81 mmol) were added sequentially. After stirring at room temperature for 0.5 hours, intermediate 3 (104.23 mg, 0.27 mmol) was added. Under a nitrogen atmosphere, the reaction was carried out at room temperature for 16 hours. The reaction solution was concentrated under reduced pressure. The residue was added with 10 mL water, followed by extraction with EA (15 mL×3). The organic phases were combined, and washed with half-saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:1 to 0:1) to afford compound 175-2. MS m/z(ESI):625.4[M+1].

Step 3: Compound 175-2 (52 mg, 0.08 mmol) was dissolved in TFA (1 mL), TfOH (3.7 µL, 0.04 mmol) was added, and the reaction was carried out at room temperature for 18 hours. The reaction solution was concentrated under reduced pressure and purified by high performance liquid chromatography (Waters-2545, chromatographic column: SharpSil-T C18, 30×150 mm, 5 µm; mobile phase: water (containing 10mmol/L ammonium bicarbonate) and acetonitrile, gradient ratio: acetonitrile 38%-45%, flow rate: 30 mL/min) to afford compound 175. MS m/z(ESI): 505.1[M+1]. ¹H NMR (400 MHz, DMSO-d6) δ 12.43 (s, 1H), 9.36 (s, 1H), 9.25 (d, *J =* 1.4 Hz, 1H), 9.10 - 9.03 (m, 2H), 8.47 (s, 1H), 7.92 (s, 1H), 6.34 (dd, *J* = 8.6, 3.6 Hz, 1H), 3.99 (dt, *J =* 13.8, 7.0 Hz, 1H), 1.71 - 1.63 (m, 1H), 1.62 - 1.49 (m, 3H), 1.18 (d, *J* = 6.4 Hz, 3H).

### Example 176

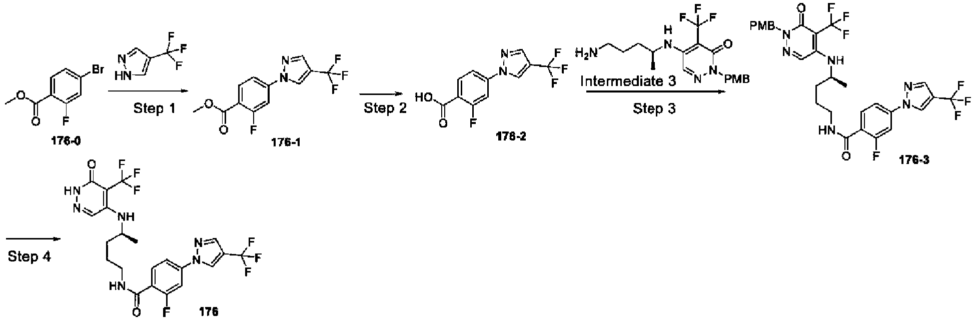

Step 1: Compound 176-0 (350 mg, 1.50 mmol, Bide) was dissolved in 1,4-dioxane (10.0 mL). Then, 4-(trifluoromethyl)-1H-pyrazole (265 mg, 1.95 mmol), cuprous iodide (57.2 mg, 0.30 mmol), potassium carbonate (57.2 mg, 0.30 mmol), and trans-(1R,2R)-N,N'-dimethyl1,2-cyclohexanediamine (415 mg, 3.00 mmol) were added sequentially. The reaction was carried out at 110°C for 18 hours in a nitrogen environment. The reaction solution was concentrated under reduced pressure. The residue was purified by silica gel column chromatography with an eluant system (PE: EA=10:1 to 1:1) to afford compound 176-1. MS m/z(ESI):289.0[M+1].

Step 2: Compound 176-1 (360 mg, 1.25 mmol) was dissolved in THF (9 mL), methanol (3 mL), and water (3 mL). LiOH (157 mg, 3.75 mmol) was added. The reaction was carried out at room temperature for 2 hours. The reaction mixture was adjusted to pH = 6 with HCl (1M). The mixture was poured into water (10 mL), followed by extraction with EA (10 mL×3). The combined organic phase was washed with brine (50 mL), dried over anhydrous Na₂SO₄, filtered, and concentrated to dryness under reduced pressure, to afford compound 176-2. MS m/z(ESI):275.0[M+1].

Step 3: Compound 176-2 (70.0 mg, 0.26 mmol) was dissolved in DMF (2.00 mL), and then TCFH (194 mg, 0.51 mmol) and DIPEA (0.13 mL, 0.77 mmol) were added sequentially. After stirring at room temperature for 5 minutes, intermediate 3 (70.0 mg, 0.26 mmol) was added. The reaction was carried out at room temperature for 2 hours. The reaction solution was added with 50 mL water, followed by extraction with EA (50 mL×3). The organic phases were combined, and washed with half-saturated saline. The organic phases were dried over anhydrous sodium sulfate. After filtering to remove the desiccant, the filtrate was concentrated under reduced pressure. The residue was purified by preparative silica gel plate (PE:EA=1:1) to afford compound 176-3. MS m/z(ESI):641.4[M+1].

Step 4: Compound 176-3 (90.0 mg, 0.14 mmol) was dissolved in TFA (1.50 mL), and TfOH (0.20 mL) was added. The reaction was carried out at room temperature for 1 hour. The reaction solution was concentrated under reduced pressure and purified by preparative high performance liquid chromatography (Phenomenex Gemini 150 mm×25 mm×10 µm column, elution solution:30%-60% (v/v) acetonitrile and water, 0.025% formic acid) to afford compound 176. MS m/z(ESI): 521.2 [M+1]. ¹H NMR (400 MHz, DMSO-*d6*) δ 12.44 (s, 1H), 9.31 (s, 1H), 8.41 (t, J = 4.8 Hz, 1H), 8.29 (s, 1H), 7.95 - 7.81 (m, 3H), 7.76 (t, J = 8.2 Hz, 1H), 6.37 - 6.27 (m, 1H), 3.98 (s, 1H), 3.26 (q, J = 6.6 Hz, 2H), 1.74 - 1.48 (m, 4H), 1.19 (d, J = 6.4 Hz, 3H).

### Example 177

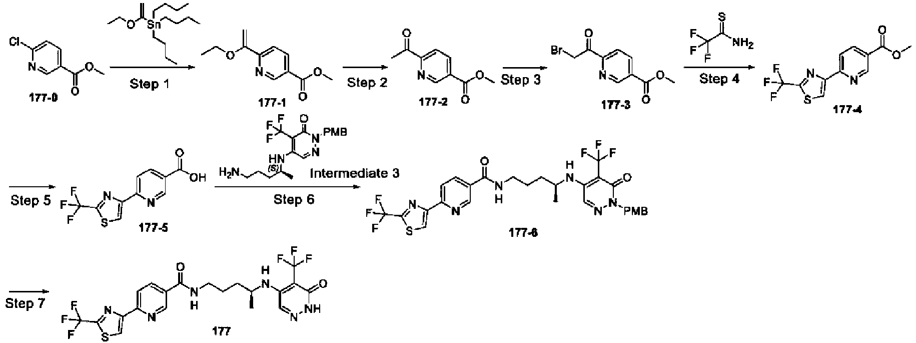

Step 1: Compound 177-0 (100 mg, 0.58 mmol, Bide) was dissolved in toluene (10 mL). Then, tributyl (1-ethoxyvinyl) -λ4-stannane (0.24 mL, 0.72 mmol) and PdCl₂(PPh₃)₂ (20.45 mg, 0.03 mmol) were added. The resulting mixture was stirred at 100°C for 6 hours under a nitrogen atmosphere. The reaction solution was concentrated under reduced pressure to afford compound 177-1. MS m/z(ESI): 208.0 [M+1].

Step 2: Compound 177-1 (1200 mg, 5.79 mmol) was dissolved in 10 mL methanol solution. 5 mL concentrated hydrochloric acid was added. Then, the mixture was stirred at 25°C for 16 hours. The reaction solution was concentrated under reduced pressure. The residue was added with 10 mL water, followed by extraction with EA (15 mL×3). The organic phases were combined, and washed with saturated sodium bicarbonate aqueous solution. The organic phases were dried over anhydrous sodium sulfate. After filtering to remove the desiccant, the filtrate was concentrated under reduced pressure. The residue was purified by silica gel column chromatography with an eluant system (PE: EA=1:100 to 1:3) to afford compound 177-2. MS m/z(ESI): 180.0 [M+1].

Step 3: Compound 177-2 (99 mg, 0.55 mmol) was dissolved in 30% EA solution of hydrogen bromide (0.3 mL). Liquid bromine (0.03 mL, 0.59 mmol) was added dropwise to the mixture. The resulting mixture was stirred at 25°C for 16 hours under a nitrogen atmosphere. The reaction solution was concentrated under reduced pressure. The residue was added with 10 mL water, followed by extraction with EA (15 mL×3). The organic phases were combined, and washed with saturated sodium bicarbonate aqueous solution. The organic phases were dried over anhydrous sodium sulfate. After filtering to remove the desiccant, the filtrate was concentrated under reduced pressure to afford compound 177-3. MS m/z(ESI): 259.0 [M+1].

Step 4: At room temperature, compound 177-3 (720 mg, 2.79 mmol) was dissolved in toluene (8 mL) solution. 1-amino-2,2,2-trifluoroethane-1-thione (0.97 mL, 11.16 mmol) was added. The reaction mixture was heated at 100°C for 16 hours in a sealed tube. After completion, the reaction mixture was cooled to room temperature and concentrated under reduced pressure. The residue was purified by silica gel column chromatography with an eluant system (PE: EA=1:100 to 1:3) to afford compound 177-4. MS m/z(ESI): 289.4 [M+1].

Step 5: Compound 177-4 (100 mg, 0.35 mmol) was dissolved in a solution of methanol/water=3/1 (4 mL). LiOH (50 mg, 2.09 mmol) was added to the mixture. The resulting mixture was stirred at 25°C for 16 hours under a nitrogen condition. The pH value of the reaction mixture was adjusted to weakly acidic with dilute hydrochloric acid, followed by extraction with EA and concentration under reduced pressure, to afford compound 177-5. MS m/z(ESI): 275.0 [M+1].

Step 6: Compound 177-5 (90 mg, 0.33 mmol) was dissolved in DMF (4 mL). Intermediate 3 (126.16 mg, 0.33 mmol), HATU (187.08 mg, 0.49 mmol), and DIPEA (127.01 mg, 0.98 mmol) were added to the mixture. The resulting mixture was stirred at 25°C for 2 hours. The reaction solution was concentrated under reduced pressure. The residue was added with 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:100 to 1:3) to afford compound 177-6. MS m/z(ESI): 641.4 [M+1].

Step 7: Compound 177-6 (280 mg, 0.44 mmol) was dissolved in TFA (3 mL) solution, and TfOH (0.04 mL, 0.44 mmol) was added. The resulting mixture was stirred at room temperature for 1 hour. The reaction solution was concentrated under reduced pressure and purified by high performance liquid chromatography (Waters-SunFire-C18-10µm-19×250mm; mobile phase: water (containing 10 mmol/L formic acid) and acetonitrile, gradient ratio: acetonitrile 36%-95%, flow rate: 25 mL/min) to afford compound 177. MS m/z(ESI): 521.2 [M+1]. ¹H NMR (400 MHz, DMSO) δ 12.44 (s, 1H), 9.10 - 9.01 (m, 1H), 8.81 (s, 1H), 8.76 (t, *J* = 5.6 Hz, 1H), 8.36 - 8.30 (m, 1H), 8.17 (d, *J =* 8.2 Hz, 1H), 7.93 (s, 1H), 6.45 - 6.21 (m, 1H), 3.99 (s, 1H), 3.33 - 3.27 (m, 2H), 1.75 - 1.48 (m, 4H), 1.19 (d, *J* = 6.2 Hz, 3H).

### Example 178

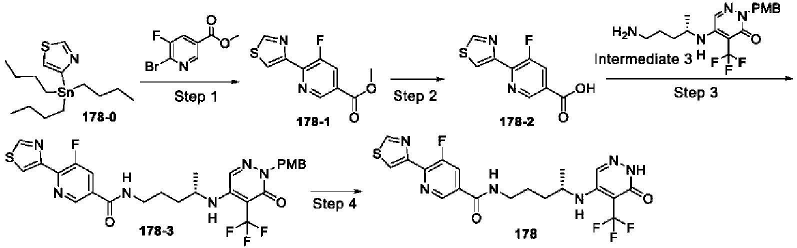

Step 1: 4-(tributyl-λ4-stannanyl)-1,3-thiazole (380 mg, 1.02 mmol) and Pd(PPh₃)₄ (49.35 mg, 0.04 mmol) were added to a mixed solution of compound 178-0 (200 mg, 0.85 mmol) and toluene (5 mL). The resulting mixture was stirred at 100°C for 18 hours under nitrogen. The reaction solution was concentrated under reduced pressure. The residue was added with 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (EA/PE=1/100-1/5) to afford compound 178-1. MS m/z(ESI): 239.0 [M+1].

Step 2: Compound 178-1 (100 mg, 0.42 mmol) was dissolved in a mixed solution of methanol:water = 3: 1 (5 mL). LiOH (0.04 mL, 1.26 mmol) was added. The resulting mixture was stirred at 25°C for 16 hours. The reaction solution was concentrated under reduced pressure to afford compound 178-2, which was used directly in the next step. MS m/z(ESI): 225.1 [M+1].

Step 3: Compound 178-2 (94 mg, 0.42 mmol) was dissolved in DMF (3 mL) solution. Intermediate 3 (161.16 mg, 0.42 mmol), HATU (239.40 mg, 0.63 mmol), and DIPEA (0.22 mL, 1.26 mmol) were added. The resulting mixture was stirred at 60°C for 5 hours. The reaction solution was concentrated under reduced pressure. The residue was added to 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (EA/PE=1/100-1/3) to afford compound 178-3. MS m/z(ESI): 591.2 [M+1].

Step 4: Compound 178-3 (320 mg, 0.54 mmol) was dissolved in TFA (3 mL), and TfOH (0.24 mL, 2.71 mmol) was added. The resulting mixture was stirred at room temperature for 18 hours. The reaction solution was concentrated under reduced pressure and purified by high performance liquid chromatography (Waters-SunFire-C18-10µm-19×250mm; mobile phase: water (containing 10 mmol/L formic acid) and acetonitrile, gradient ratio: acetonitrile 23%-95%, flow rate: 25 mL/min) to afford compound 178. MS m/z(ESI): 471.1 [M+1]. ¹H NMR (400 MHz, DMSO) δ 12.43 (s, 1H), 9.28 (d, *J =* 2.0 Hz, 1H), 8.92 (t, *J =* 1.6 Hz, 1H), 8.78 (t, *J* = 5.6 Hz, 1H), 8.46 - 8.39 (m, 1H), 8.17 (dd, *J* = 11.8, 1.7 Hz, 1H), 7.94 (s, 1H), 6.42 - 6.26 (m, 1H), 4.07 - 3.90 (m, 1H), 3.34 - 3.27 (m, 2H), 1.75 - 1.50 (m, 4H), 1.19 (d, *J* = 6.4 Hz, 3H).

### Example 179

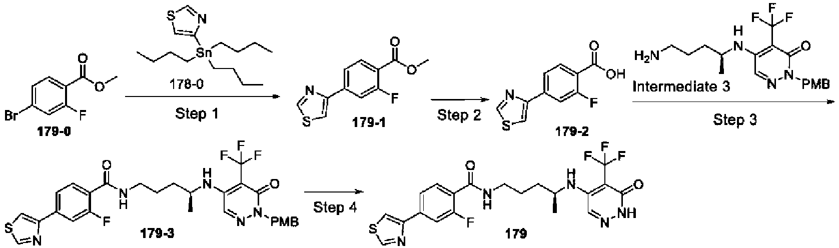

Step 1: Compound 179-0 (300 mg, 1.29 mmol) was dissolved in toluene (3 mL). Then, compound 178-0 (724.02 mg, 1.94 mmol) and Pd(PPh₃)₄ (74.54 mg, 0.06 mmol) were added sequentially. The reaction was carried out at 90°C for 18 hours in a nitrogen environment. The reaction solution was concentrated under reduced pressure. The residue was purified by silica gel column chromatography with an eluant system (PE:EA=1:1-1/10) to afford compound 179-1. MS m/z(ESI):238.0[M+1].

Step 2: Compound 179-1 (260 mg, 1.10 mmol) was dissolved in THF (9 mL), methanol (3 mL), and water (3 mL). LiOH (137.95 mg, 3.29 mmol) was added. The reaction was carried out at room temperature for 2 hours. The reaction mixture was adjusted to pH = 6 with HCl (1 M). The mixture was poured into water (50 mL), followed by extraction with EA (50 mL×3). The combined organic phase was washed with brine (50 mL), dried over anhydrous sodium sulfate, filtered, and concentrated to dryness under reduced pressure, to afford compound 179-2. MS m/z(ESI):224.0[M+1].

Step 3: Compound 179-2 (70 mg, 0.31 mmol) was dissolved in DMF (1.5 mL). Then, HATU (238.48 mg, 0.63 mmol) and DIPEA (0.16 mL, 0.94 mmol) were added sequentially. After stirring at room temperature for 5 minutes, intermediate 3 (120.54 mg, 0.31 mmol) was added. The reaction was carried out at room temperature for 2 hours. The reaction solution was added to 50 mL water, followed by extraction with EA (50 mL×3). The organic phases were combined, and washed with saturated saline. The organic phases were dried over anhydrous sodium sulfate. After filtering to remove the desiccant, the filtrate was concentrated under reduced pressure. The residue was purified by preparative silica gel plate (PE:EA=1:1) to afford compound 179-3. MS m/z(ESI):590.4[M+1].

Step 4: Compound 179-3 (70 mg, 0.04 mmol) was dissolved in TFA (1.5 mL), TfOH (0.2 mL) was added, and the reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 179. MS m/z(ESI): 470.2 [M+1]. ¹H NMR (400 MHz, DMSO-d6) δ 12.45 (s, 1H), 9.24 (d, J = 2.0 Hz, 1H), 8.40 (d, J = 2.0 Hz, 1H), 8.37 - 8.31 (m, 1H), 7.93 (s, 1H), 7.92 - 7.84 (m, 2H), 7.67 (t, J = 7.8 Hz, 1H), 6.37 - 6.29 (m, 1H), 4.03 - 3.93 (m, 1H), 3.29 - 3.21 (m, 2H), 1.72 - 1.50 (m, 4H), 1.19 (d, J = 6. Hz, 3H).

### Example 182

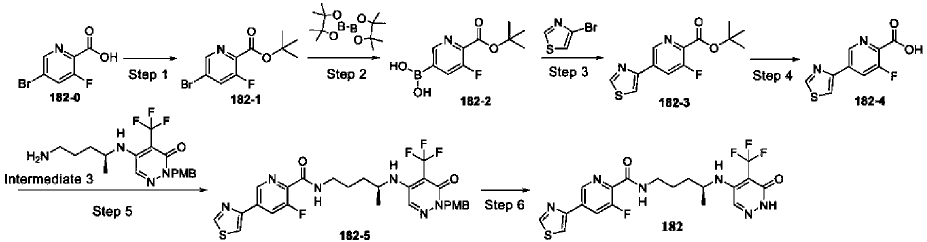

Step 1: Compound 182-0 (2 g, 9.09 mmol) was dissolved in THF (20 mL). DMAP (0.56 g, 4.55 mmol) and Boc anhydride (3.89 mL, 18.18 mmol) were added sequentially, followed by reacting under stirring at 80°C for 16 hours. The reaction solution was directly concentrated under reduced pressure. The residue was purified by silica gel column chromatography (PE:EA=1:1 to 0:1) to afford compound 182-1. MS m/z(ESI):276.0 [M+1].

Step 2: Compound 182-1 (500 mg, 1.81 mmol) was dissolved in 1,4-dioxane (5 mL). Bis(pinacolato)diboron (0.94 mL, 3.62 mmol), potassium acetate (355.27 mg, 3.62 mmol), and 1,1-bis(diphenylphosphino)ferrocenedichloropalladium (1991.04 mg, 2.72 mmol) were added. The resulting mixture was reacted under stirring at room temperature for 2 hours. The reaction solution was directly concentrated to dryness. The residue was purified by silica gel column chromatography (PE:EA=1:1 to 0:1) to afford compound 182-2. MS m/z(ESI):242.0 [M+1].

Step 3: Compound 182-2 (480 mg, 1.99 mmol) was dissolved in 1,4-dioxane (5 mL). 4-bromothiazole (328.00 mg, 1.99 mmol) and PdCl₂(PPh₃)₂ (2096.70 mg, 2.99 mmol), and water (1 mL) were added sequentially. The reaction was carried out at 120°C for 24 hours. The mixture was dried by spin. The residue was purified by silica gel column chromatography (PE: EA=1:1 to 0:1) to afford compound 182-3. MS m/z(ESI): 281.0[M+1].

Step 4: Compound 182-3 (380 mg, 1.36 mmol) was added to a mixed solution of solvent TFA (5 mL) and DCM (5 mL), followed by reacting under stirring at room temperature for 2 hours. The mixture was dried by spin to afford compound 182-4. MS m/z(ESI): 225.0[M+1].

Step 5: Compound 182-4 (150 mg, 0.67 mmol) was dissolved in DMF (5 mL). Intermediate 3 (257 mg, 0.67 mmol), DIPEA (0.11 mL, 0.67 mmol), and HATU (382.14 mg, 1.01 mmol) were added sequentially, followed by reacting under stirring at room temperature for 16 hours. Water (20 mL) was added to the system, followed by filtering. The filter cake was concentrated to afford compound 182-5. MS m/z(ESI): 591.2[M+1].

Step 6: Compound 182-5 (120 mg, mmol) was added to TFA (5 mL). Then, TfOH (10 mL, 112.61 mmol) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 182. MS m/z(ESI): 471.2[M+1]. ¹H NMR (400 MHz, DMSO-*d*₆) δ 12.43 (s, 1H), 9.31 (d, *J* = 1.8 Hz, 1H), 9.08 (t, *J* = 1.6 Hz, 1H), 8.76 (t, *J* = 6.0 Hz, 1H), 8.59 (d, *J* = 1.9 Hz, 1H), 8.35 (dd, *J* = 11.8, 1.7 Hz, 1H), 7.93 (s, 1H), 6.34 (s, 1H), 3.96 (d, *J* = 22.6 Hz, 1H), 3.27 (d, *J* = 6.3 Hz, 2H), 1.62 (d, *J* = 53.0 Hz, 4H), 1.18 (d, *J* = 6.3 Hz, 3H).

### Example 183

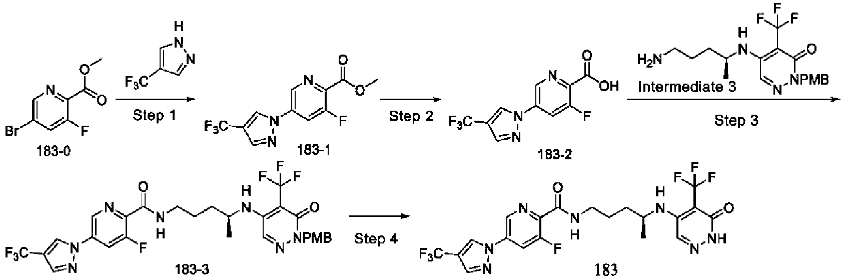

Step 1: At room temperature, compound 183-0 (500 mg, 2.89 mmol) and 4-(trifluoromethyl)-1H-pyrazole (250 mg, 1.44 mmol) was dissolved in DMSO (6 mL), and potassium carbonate (500 mg, 3.62 mmol) was added to the reaction solution. After the system was reacted at 80°C for 16 hours, the reaction was completed. After the reaction was completed, 30 mL water and 30 mL EA were added to the reaction solution. The organic phases were washed with saturated saline (3 mL×3), and dried over sodium sulfate. After filtering, the filtrate was concentrated under reduced pressure to afford the crude. The crude was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 183-1. MS m/z (ESI): 290.0 [M+1].

Step 2: Compound 183-1 (140 mg, 0.48 mmol) was added to methanol (5 mL) and water (1 mL), and LiOH (0.13 mL, 4.84 mmol) was added. After reacting at room temperature for two hours, the reaction was completed. 1N dilute hydrochloric acid was added for adjusting pH to 7. Methanol was evaporated off, followed by extraction three times with EA. The organic phases were dried by spin to afford compound 183-2. MS m/z (ESI): 276.2 [M+1].

Step 3: Compound 183-2 (135 mg, 0.49 mmol), o-(7-azabenzotriazol-1-yl)uronium hexafluorophosphate (386.93 mg, 1.02 mmol) and DIPEA (0.42 mL, 2.54 mmol) were added to DMF (5 mL), followed by stirring at room temperature for 0.5 hours. Then, intermediate 3 (136.91 mg, 0.36 mmol) was added, followed by reacting for 16 hours. Under an ice bath, 15 mL of water was added to the reaction solution to precipitate the product, followed by filtering. Then, the solid was washed with clean water to afford compound 183-3. MS m/z (ESI): 642.2[M+1].

Step 4: Compound 183-3 (120 mg, 0.19 mmol) was added to TFA (5 mL). TfOH (0.08 mL, 0.94 mmol) was slowly added dropwise. The reaction was carried out at room temperature for 1 hour. 10 mL methanol was added, followed by drying by spin. The product was dissolved in 4 mL dimethyl sulfoxide, and purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 183. MS m/z (ESI): 522.2 [M+1]. ¹H NMR (400 MHz, DMSO-*d*₆) δ 12.43 (s, 1H), 9.41 (t, *J* = 1.0 Hz, 1H), 9.20 - 9.00 (m, 1H), 8.80 (t, *J* = 5.9 Hz, 1H), 8.51 - 8.34 (m, 2H), 7.93 (s, 1H), 6.34 (dd, *J* = 8.8, 3.9 Hz, 1H), 3.99 (s, 1H), 3.31 - 3.21 (m, 2H), 1.73 - 1.61 (m, 1H), 1.60 - 1.39 (m, 3H), 1.18 (d, *J* = 6.3 Hz, 3H).

### Example 184

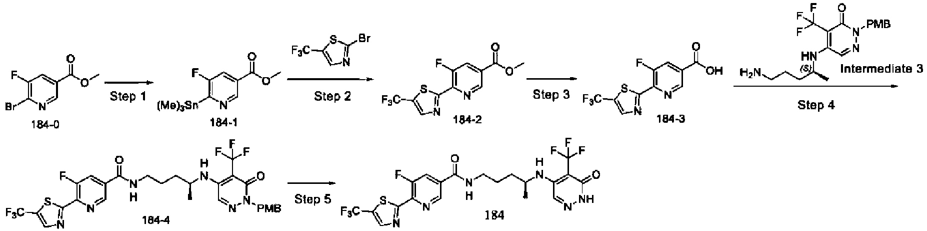

Step 1: Compound 184-0 (500 mg, 2.14 mmol) was dissolved in 1,4-dioxane (5 mL). Pd(PPh₃)₄ (246.99 mg, 0.21 mmol) and hexamethylditin (1400.01 mg, 4.27 mmol) were added sequentially. The system was heated to 100 °C and reacted under stirring for 4 hours. The reaction solution was concentrated to dryness and purified by alumina column chromatography with an eluant system (PE: EA=100: 1 to 10/1) to afford compound 184-1. MS m/z (ESI): 320.0 [M+1].

Step 2: Compound 184-1 (260.07 mg, 1.10 mmol) and 2-bromo-5-trifluoromethylthiazole (256.20 mg, 1.10 mmol) was dissolved in DMF (3 mL). Pd(PPh₃)₄ (129.90 mg, 0.10 mmol) was added. The system was heated to 100°C and reacted under stirring for 4 hours. The reaction solution was concentrated to dryness and purified by silica gel column chromatography with an eluant system (PE: EA=100: 1 to 10/1) to afford compound 184-2. MS m/z (ESI): 307.0 [M+1].

Step 3: Compound 184-2 (160 mg, 0.52 mmol) was dissolved in methanol (4 mL). LiOH (0.01 mL, 0.52 mmol) and water (1 mL) were added sequentially. The system was reacted under stirring at 30°C for 3 hours. The pH of the system was adjusted to 2-3, followed by filtering. The filter cake was concentrated to dryness under reduced pressure under vacuum to afford compound 184-3. MS m/z (ESI): 293.0 [M+1].

Step 4: Compound 184-3 (45 mg, 0.15 mmol), intermediate 3 (59.20 mg, 0.15 mmol), HATU (87.83 mg, 0.23 mmol), and DIPEA (59.60 mg, 0.46 mmol) were added sequentially to DMF (2 mL). The system was reacted under stirring at 25°C for 2 hours. Under an ice bath, 15 mL of water was added to the reaction solution to precipitate the product, followed by filtering. Then, the solid was washed with clean water to afford compound 184-4. MS m/z (ESI): 659.2 [M+1].

Step 5: Compound 184-4 (29 mg, 0.04 mmol) and TfOH (0.01 mL, 0.06 mmol) were added sequentially to TFA (2 mL). The system was reacted under stirring at room temperature for 2 hours. The reaction solution was concentrated to dryness. The crude was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 184. MS m/z (ESI): 539.2 [M+1]. ¹H NMR (400 MHz, Methanol-*d*₄) δ 8.89 (t, *J =* 1.4 Hz, 1H), 8.42 (d, *J =* 1.4 Hz, 1H), 8.17 (dd, *J =* 10.9, 1.7 Hz, 1H), 7.94 (s, 1H), 4.01 (d, *J* = 6.4 Hz, 1H), 3.57 - 3.35 (m, 2H), 1.71 (td, *J* = 8.3, 5.5 Hz, 4H), 1.30 (d, *J* = 6.4 Hz, 3H).

### Example 185

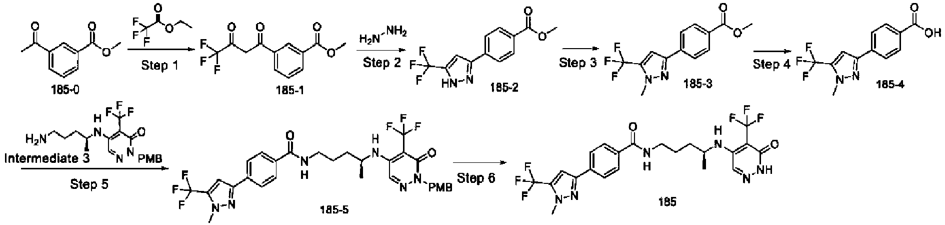

Step 1: Compound 185-0 (5500 mg, 30.87 mmol), ethyl 2,2,2-trifluoroacetate (5.53 mL, 46.30 mmol), and sodium methoxide (5000.28 mg, 92.60 mmol) were added sequentially to methanol (55 mL), followed by reacting under stirring at room temperature for 1 hour. The reaction solution was diluted with EA (300 mL) and then washed with water (100 mL×3) and saturated saline (100 mL), respectively. The organic phase was dried over anhydrous sodium sulfate, and filtered. The filtrate was concentrated to dryness. The crude was purified by silica gel column chromatography with an eluant system (PE: EA=10/1->3/1) to afford compound 185-1. MS m/z (ESI): 275.0 [M+1].

Step 2: Compound 185-1 (5.3 g, 19.3 mmol) and hydrazine hydrate (6.17 g, 193 mmol) were added to methanol (10 mL), followed by heating to 50°C and reacting under stirring for 2 hours. The reaction solution was diluted with EA (200 mL) and then washed with water (100 mL×3) and saturated saline (100 mL), respectively. The organic phase was dried over anhydrous sodium sulfate and filtered. The filtrate was concentrated to dryness. The crude was purified by silica gel column chromatography with an eluant system (PE: EA=10:1 to 3: 1) to afford compound 185-2. MS m/z (ESI): 271.0 [M+1].

Step 3: Compound 185-2 (5000 mg, 18.50 mmol), dimethyl sulfate (1.93 mL, 20.35 mmol), and potassium carbonate (7671.81 mg, 55.51 mmol) were added sequentially to acetonitrile (50 mL). The system was reacted under stirring at room temperature for 2 hours. EA (200 mL) was added to the system for dilution, followed by washing with water (100 mL×3) and saturated saline (100 mL), respectively. The organic phase was dried over anhydrous sodium sulfate and filtered. The filtrate was concentrated to dryness. The crude was purified by silica gel column chromatography with an eluant system (DCM: methanol=100: to 10: 1) to afford compound 185-3. MS m/z (ESI): 285.0 [M+1].

Step 4: Compound 185-3 (120 mg, 0.42 mmol), LiOH (0.04 mL, 1.27 mmol), and water (2 mL) were added sequentially to methanol (2 mL). The system was reacted under stirring at room temperature for two hours. 1N dilute hydrochloric acid was added for adjusting pH to 7. Methanol was evaporated off, followed by extraction three times with EA. The organic phases were dried by spin to afford compound 185-4. MS m/z (ESI): 271.0 [M+1].

Step 5: Compound 185-4 (80 mg, 0.30 mmol), intermediate 3 (113.81 mg, 0.30 mmol), HATU (168.86 mg, 0.44 mmol), and DIPEA (0.15 mL, 0.89 mmol) were added to DMF (2 mL). The system was reacted under stirring at 25°C for 2 hours. Under an ice bath, 15 mL of water was added to the reaction solution to precipitate the product, followed by filtering. Then, the solid was washed with clean water to afford compound 185-5. MS m/z (ESI): 637.2 [M+1].

Step 6: Compound 185-5 (80 mg, 0.13 mmol) and TfOH (0.01 mL, 0.06 mmol) were added sequentially to TFA (2 mL). The system was reacted under stirring at room temperature for 2 hours. The reaction solution was concentrated to dryness. The crude was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 185. MS m/z (ESI): 517.2 [M+1]. ¹H NMR (400 MHz, CD₃OD) δ 7.93-7.90 (m, 3H), 7.86-7.84 (m, 2H), 7.21 (s, 1H), 4.05 (s, 3H), 4.02 - 3.97 (m, 1H), 3.99 (s, 1H), 3.43 - 3.04 (m, 2H), 1.71 - 1.50 (m, 4H), 1.17 (t, *J* = 8.0 Hz, 3H).

### Example 186

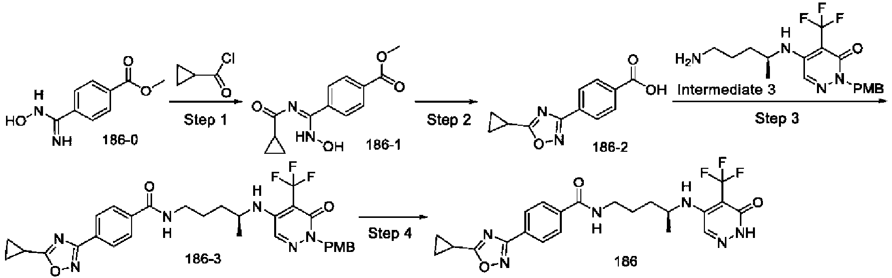

Step 1: Compound 186-0 (500 mg, 2.57 mmol), cyclopropylformyl chloride (0.47 mL, 5.15 mmol), and sodium bicarbonate (648.93 mg, 7.72 mmol) were added sequentially to DCM (10 mL), followed by reacting under stirring at room temperature for 2 hours. EA (300 mL) was added for dilution, followed by washing with water (100 mL×3) and saturated saline (100 mL), respectively. The resulting mixture was dried over anhydrous sodium sulfate and filtered. The filtrate was concentrated to dryness, and purified by silica gel column chromatography (DCM/methanol=20/1 to 10/1) to afford compound 186-1. MS m/z(ESI): 263.1[M+1].

Step 2: Compound 186-1 (400 mg, 1.53 mmol), sodium hydroxide (183.02 mg, 4.58 mmol), and water (1 mL) were added sequentially to DMSO (2 mL), followed by reacting under stirring at room temperature for 2 hours. EA (300 mL) was added for dilution, followed by washing with water (100 mL×3) and saturated saline (100 mL) respectively. The resulting mixture was dried over anhydrous sodium sulfate and filtered. The filtrate was concentrated to dryness, and purified by silica gel column chromatography (DCM/methanol=20/1 to 10/1) to afford compound 186-2. MS m/z(ESI): 231.0[M+1].

Step 3: Compound 186-2 (200 mg, 0.87 mmol), intermediate 3 (367.34 mg, 0.96 mmol), HATU (495.49 mg, 1.30 mmol), and DIPEA (0.43 mL, 2.61 mmol) were added sequentially to DMF (4 mL), followed by reacting under stirring at room temperature for 16 hours. Water (20 mL) was added, followed by filtering. The filter cake was concentrated to afford compound 186-3. MS m/z(ESI): 597.2[M+1].

Step 4: Compound 186-3 (120 mg, 0.20 mmol) was added to TFA (5 mL), and then TfOH (0.5 mL) was added. The system was reacted under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 186. MS m/z(ESI): 477.2[M+1]. ¹H NMR (400 MHz, DMSO-*d*₆) δ 12.45 (s, 1H), 8.65 (t, *J* = 5.7 Hz, 1H), 8.17 - 7.75 (m, 5H), 6.43 - 6.13 (m, 1H), 4.03 - 3.91 (m, 1H), 3.28 (d, *J* = 6.1 Hz, 2H), 2.47 - 2.38 (m, 1H), 1.78 - 1.47 (m, 4H), 1.35 - 1.27 (m, 2H), 1.23 - 1.12 (m, 5H).

### Example 188

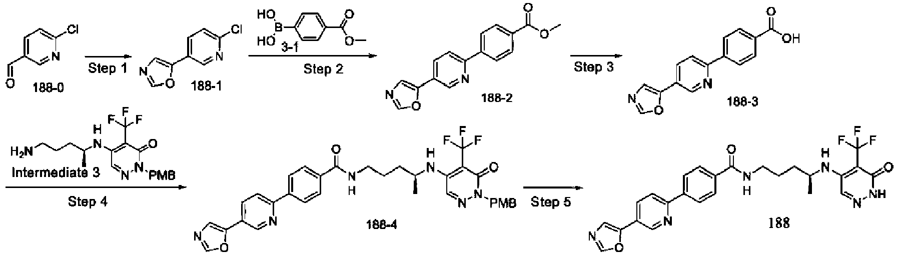

Step 1: Compound 188-0 (1 g, 7.06 mmol), p-toluenesulfonylmethyl isocyanide (1.38 g, 7.06 mmol), and potassium carbonate (0.98 g, 7.06 mmol) were added sequentially to methanol, followed by reacting under stirring at 80°C for 1 hour. The reaction solution was directly concentrated to dryness. The residue was purified by silica gel column chromatography (PE:EA=1:1 to 0:1) to afford compound 188-1. MS m/z(ESI):181.0 [M+1].

Step 2: Compound 188-1 (840 mg, 4.65 mmol), compound 3-1 (837.12 mg, 4.65 mmol), DPPFdichloropalladium (20.26 mg, 0.03 mmol), water(1 mL), and potassium carbonate (1285.65 mg, 9.30 mmol) were added sequentially to 1,4-dioxane (10 mL), followed by heating to 90°C and reacting under stirring for 3 hours under nitrogen protection. The reaction solution was directly concentrated to dryness. The residue was purified by silica gel column chromatography (EA:PE=0:1 to 1:1) to afford compound 188-2. MS m/z(ESI):281.0 [M+1].

Step 3: Compound 188-2 (310 mg, 1.11 mmol), LiOH (93 mg, 0.22 mmol), and water (1 mL) were added sequentially to methanol (2 mL), followed by reacting under stirring at 25°C for 1 hour. pH was adjusted to 2-3 with 6M hydrochloric acid, followed by filtering. The filter cake was concentrated to afford compound 188-3. MS m/z(ESI):267.0 [M+1].

Step 4: Compound 188-3 (120 mg, 0.45 mmol), intermediate 3 (173.24 mg, 0.45 mmol), HATU (171.11 mg, 0.45 mmol), and DIPEA (116.10 mg, 0.90 mmol) were added sequentially to DMF (4 mL), followed by reacting under stirring at room temperature for 2 hours. Water (20 mL) was added to the system, followed by filtering. The filter cake was concentrated to afford compound 188-4. MS m/z(ESI): 633.2[M+1].

Step 5: Compound 188-4 (130 mg, 0.21 mmol) was added to TFA (5 mL). Then, TfOH (0.5 mL) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 188. MS m/z(ESI): 513.2[M+1]. ¹H NMR (400 MHz, DMSO-*d*₆) δ 12.44 (s, 1H), 9.08 (d, *J* = 2.2 Hz, 1H), 8.58 (d, *J* = 2.6 Hz, 2H), 8.38 - 8.10 (m, 4H), 8.06 - 7.80 (m, 4H), 6.55 - 6.21 (m, 1H), 3.99 (s, 1H), 3.29 (d, *J =* 7.0 Hz, 2H), 1.62 (d, *J =* 48.0 Hz, 4H), 1.19 (d, *J* = 6.4 Hz, 3H).

### Example 189

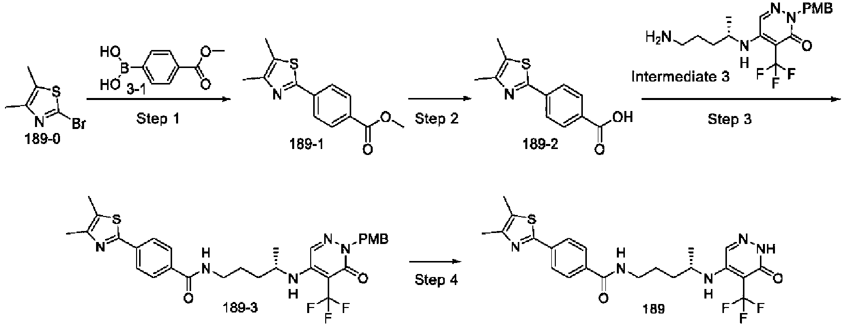

Step 1: Compound 189-0 (230.00 mg, 1.19 mmol), water (1 mL), compound 3-1 (210 mg, 1.17 mmol), Pd(PPh₃)₄ (12.85 mg, 0.01 mmol), and potassium carbonate (322.52 mg, 2.33 mmol) were added sequentially to 1,4-dioxane (4 mL). The resulting mixture was purged with nitrogen 3 times, heated to 80°C, and reacted under stirring for 3 hours. The reaction solution was directly concentrated to dryness, and purified by silica gel column chromatography (EA/PE=0 to 30%) to afford compound 189-1. MS m/z(ESI):248.0 [M+1].

Step 2: Compound 189-1 (54408.20 mg, 220 mmol), LiOH (18.42 mL, 1100.00 mmol), and water (1 mL) were added sequentially to methanol (2 mL), followed by reacting under stirring at room temperature for 2 hours. pH was adjusted to 2-3 with 6M hydrochloric acid, followed by filtering. The filter cake was concentrated to afford compound 189-2. MS m/z(ESI):234.0 [M+1].

Step 3: Compound 189-2 (100 mg, 0.43 mmol), intermediate 3 (173.24 mg, 0.45 mmol), HATU (244.34 mg, 0.64 mmol), and DIPEA (166.20 mg, 1.29 mmol) were added sequentially to DMF (4 mL), followed by reacting under stirring at room temperature for 2 hours. Water (20 mL) was added, followed by filtering. The filter cake was concentrated to afford compound 189-3. MS m/z(ESI):600.2 [M+1].

Step 4: Compound 189-3 (100 mg, 0.17 mmol) was added to TFA (5 mL). Then, TfOH (0.5 mL) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 189. MS m/z(ESI):480.2 [M+1]. ¹H NMR (400 MHz, DMSO-*d*₆) δ 12.44 (s, 1H), 8.57 (t, *J* = 5.7 Hz, 1H), 7.92 (d, *J* = 10.4 Hz, 5H), 6.34 (d, *J* = 6.7 Hz, 1H), 3.98 (s, 1H), 3.27 (d, *J* = 6.6 Hz, 2H), 2.40 (s, 3H), 2.33 (s, 3H), 1.73 - 1.46 (m, 4H), 1.18 (d, *J =* 6.3 Hz, 3H).

### Example 191

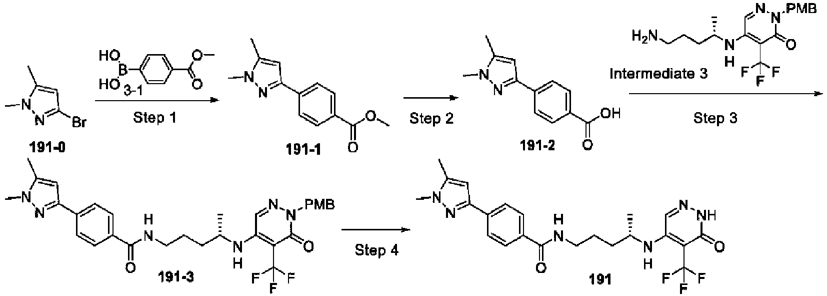

Step 1: Compound 191-0 (0.19 mL, 1.71 mmol) was dissolved in 1, 4-dioxane (8 mL). Compound 3-1 (161 mg, 2.56 mmol), DPPFdichloropalladium (125.29 mg, 0.17 mmol), and potassium carbonate (473.75 mg, 3.43 mmol) were added. The resulting mixture was stirred at 100°C for 16 hours under a nitrogen atmosphere. The reaction solution was concentrated under reduced pressure. The residue was added with 10 mL water, followed by extraction with EA (15 mL×3). The organic phases were combined, and washed with saturated saline. The organic phases were dried over anhydrous sodium sulfate and filtered. After removing the desiccant, the filtrate was concentrated under reduced pressure. The residue was purified by silica gel column chromatography with an eluant system (EA/PE=1/100-1/5) to afford compound 191-1. MS m/z(ESI):231.0[M+1].

Step 2: Compound 191-1 (100 mg, 0.43 mmol) was dissolved in a solution of methanol: water=3:1 (10 mL). LiOH (0.04 mL, 1.29 mmol) was added to the mixture. The resulting mixture was stirred at 25°C for 16 hours. The reaction solution was concentrated under reduced pressure to afford compound 191-2, which was used directly in the next step. MS m/z(ESI): 217.2 [M+1].

Step 3: Compound 191-2 (74 mg, 0.34 mmol), 4-(5-cyano-4- methylpyridin-2-yl)benzoic acid (200 mg, 0.42 mmol), TCFH (144.03 mg, 0.51 mmol), and 1-methylimidazole (91.13 mg, 1.11 mmol) were dissolved in acetonitrile (8 mL) solution, followed by stirring at room temperature for 10 minutes. Intermediate 3 (131.55 mg, 0.34 mmol) was added, followed by stirring at room temperature for 18 hours. The reaction solution was concentrated under reduced pressure. The residue was added to 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (EA/PE=1/100-1/3) to afford compound 191-3. MS m/z(ESI):583.4 [M+1].

Step 4: Compound 191-3 (45 mg, 0.08 mmol) was dissolved in TFA (3 mL) solution, and TfOH (0.1 mL, 0.04 mmol) was added. The resulting mixture was stirred at room temperature for 18 hours. The reaction solution was concentrated under reduced pressure and purified by high performance liquid chromatography (Waters-SunFire-C18-10µm-19×250 mm; mobile phase: water (containing 10 mmol/L formic acid) and acetonitrile, gradient ratio: acetonitrile 25%-95%, flow rate: 25 mL/min) to afford compound 191. MS m/z(ESI): 463.1[M+1]. ¹H NMR (400 MHz, DMSO) δ 12.45 (s, 1H), 8.45 (t, *J* = 5.6 Hz, 1H), 7.93 (s, 1H), 7.81 (q, *J* = 8.4 Hz, 4H), 6.55 (s, 1H), 6.35 (d, *J* = 5.2 Hz, 1H), 3.98 (s, 1H), 3.77 (s, 3H), 3.26 (d, *J* = 5.6 Hz, 2H), 2.29 (s, 3H), 1.70 - 1.47 (m, 4H), 1.18 (d, *J* = 6.4 Hz, 3H).

### Example 192

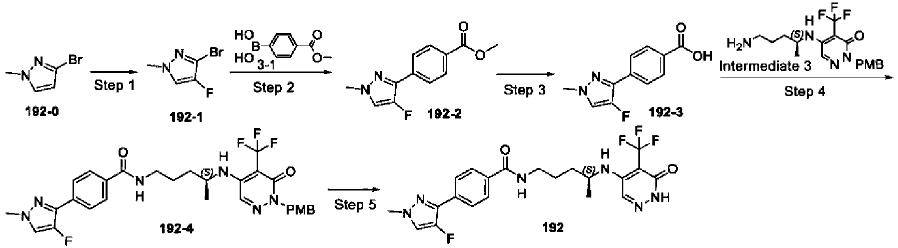

Step 1: Compound 192-0 (5.00 g, 31.06 mmol) was added to acetonitrile (75 mL). 1-chloromethyl-4-fluoro-1,4-diazoniabicyclo[2.2.2]octane bis(tetrafluoroborate)(16.50 g, 46.58 mmol) was added, followed by reacting at 25°C for 4 days. The reaction solution was added with saturated sodium bicarbonate solution (100 mL), followed by extraction with EA (150 mL). The organic phases were combined and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=1:0 to 6:1) to afford compound 192-1. MS m/z(ESI):179.0[M+1].

Step 2: Compound 192-1 (100 mg, 0.56 mmol), compound 3-1 (150.82 mg, 0.84 mmol), potassium carbonate (231.63 mg, 1.68 mmol), and DPPFdichloropalladium (40.88 mg, 0.06 mmol) were added to 1,4-dioxane (1.5 mL) and water (0.3mL), followed by purging with nitrogen three times. The reaction was carried out at 80°C for 1 hour. The reaction solution was concentrated under reduced pressure. The residue was purified by silica gel column chromatography with an eluant system (PE: EA=1:0 to 2:1) to afford compound 192-2. MS m/z(ESI):235.0[M+1].

Step 3: Compound 192-2 (55.0 mg, 0.23 mmol) was dissolved in THF (0.5 mL), methanol (0.5 mL), and water (0.5 mL). LiOH (29.56 mg, 0.70 mmol) was added. The reaction was carried out at room temperature for 2 hours. The reaction mixture was adjusted to pH = 4 with HCl (1M), and then poured into water (10 mL), followed by extraction with EA (10 mL×3). The combined organic phase was washed with brine (10 mL), dried over anhydrous sodium sulfate, filtered, and concentrated to dryness under reduced pressure, to afford compound 192-3. MS m/z(ESI):221.0[M+1].

Step 4: Intermediate 3 (38.41 mg, 0.10 mmol) was dissolved in acetonitrile (1 mL). Then, compound 192-3 (22 mg, 0.10 mmol), TCFH (42.05 mg, 0.15 mmol), and N-methylimidazole (23.89 µL, 0.30 mmol) were added sequentially, and the reaction was carried out at room temperature for 2 hours. The reaction solution was concentrated under reduced pressure. The residue was purified by silica gel column chromatography with an eluant system (PE: EA=1:0 to 0:1) to afford compound 192-4. MS m/z(ESI):587.2[M+1].

Step 5: Compound 192-4 (32 mg, 0.05 mmol) was dissolved in TFA (1.2 mL), 3 drops of TfOH was added, and the reaction was carried out at room temperature for 5 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-95%, flow rate: 30 mL/min) to afford compound 192. MS m/z(ESI): 467.2[M+1]. ¹1H NMR (400 MHz, DMSO-d6) δ 12.44 (s, 1H), 8.50 (t, J = 5.6 Hz, 1H), 7.99 (d, J = 4.6 Hz, 1H), 7.96 - 7.88 (m, 3H), 7.79 (d, J = 8.2 Hz, 2H), 6.34 (dd, J = 8.4, 4.0 Hz, 1H), 4.04 - 3.92 (m, 1H), 3.84 (s, 3H), 3.30 - 3.22 (m, 2H), 1.75 - 1.61 (m, 1H), 1.60 - 1.46 (m, 3H), 1.18 (d, J = 6.2 Hz, 3H).

### Example 205

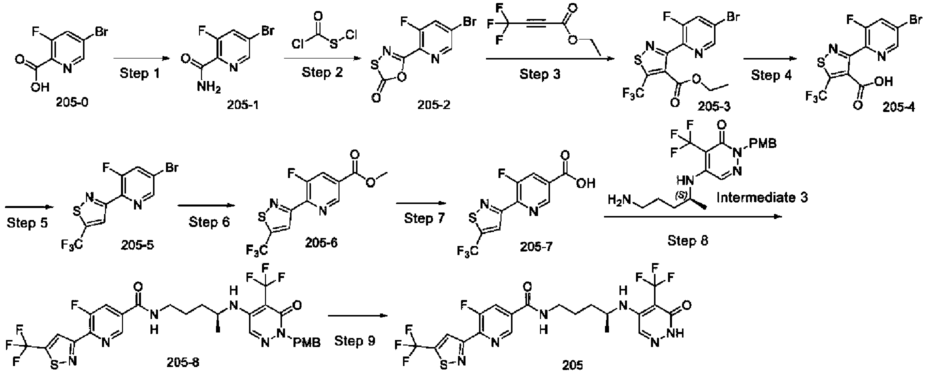

Step 1: Compound 205-0 (9 g, 40.91 mmol), ammonium chloride (4.32 mL, 122.73 mmol), HATU (23.38 g, 61.36 mmol), and DIPEA (15.83 g, 122.73 mmol) were added sequentially to THF (100 mL). The system was reacted under stirring at 25°C for 2 hours. The reaction solution was concentrated to dryness, dissolved by adding EA (100 mL), and washed with water (100 mL×3) and saturated saline (100 mL), respectively. The organic phase is separated and concentrated to dryness. The residue was purified by silica gel column chromatography with an eluant system (PE: EA=100: 1 to 2/1) to afford compound 205-1. MS m/z (ESI): 219.0 [M+1].

Step 2: Compound 205-1 (4 g, 18.26 mmol) was dissolved in toluene (40 mL). Chlorothiocarbonyl chloride (3.03 mL, 36.53 mmol) was added. The system was heated to 110 °C and reacted under stirring for 16 hours. The reaction solution was concentrated to dryness to afford compound 205-2. MS m/z (ESI): 277.0 [M+1].

Step 3: Compound 205-2 (4 g, 14.43 mmol) was dissolved in 2,6-dichlorobenzene (16 mL), and then ethyl 4,4,4-trifluorobut-2-ynoate (2.36 mL, 23.10 mmol) was added. The system was heated to 150°C and reacted under stirring for 16 hours. The reaction solution was concentrated to dryness and purified by silica gel column chromatography with an eluant system (PE: EA=100: 1 to 2/1) to afford compound 205-3. MS m/z (ESI): 399.0 [M+1].

Step 4: Compound 205-3 (2.8 g, 7.01 mmol), LiOH (0.58 mL, 21.04 mmol), and water (20 mL) were added sequentially to methanol (30 mL). The system was reacted under stirring at room temperature for 2 hours. The reaction solution was cooled to 0°C, added with 6M hydrochloric acid for adjusting pH to 5-6, and filtered. The filter cake was concentrated to dryness to afford compound 205-4. MS m/z (ESI): 371.0 [M+1].

Step 5: Compound 205-4 (1.0 g, 2.7 mmol) was dissolved in dimethyl sulfoxide (10 mL). Cuprous oxide (1.16 g, 8.10 mmol) was added. The system was heated to 150°C and reacted under stirring for 16 hours. The reaction solution was cooled to room temperature. EA (500 mL) and water (500 mL) were added. The organic phase was separated and concentrated to dryness, and purified by silica gel column chromatography with an eluant system (PE: EA=100: 0 to 10: 1) to afford compound 205-5. MS m/z (ESI): 327.0 [M+1].

Step 6: Compound 205-5 (600 mg, 1.83 mmol) was dissolved in ethanol (2 mL). Pd(PPh₃)₄ (211.87 mg, 0.18 mmol) and triethylamine (555.81 mg, 5.50 mmol) were added sequentially. The system was heated to 100°C and reacted under stirring for 6 hours in a carbon monoxide environment. The reaction solution was concentrated to dryness and purified by silica gel column chromatography with an eluant system (PE: EA=100: 1 to 5: 1) to afford compound 205-6. MS m/z (ESI): 307.0 [M+1].

Step 7: Compound 205-6 (220 mg, 0.69 mmol) was dissolved in methanol (4 mL). LiOH (0.05 mL, 2.75 mmol) and water (1mL) were added sequentially. The system was reacted under stirring at 30°C for 3 hours. The pH of the system was adjusted to 5-6, followed by filtering. The filter cake was concentrated to dryness under reduced pressure under vacuum to afford compound 205-7. MS m/z (ESI): 293.0 [M+1].

Step 8: Compound 205-7 (90 mg, 0.31 mmol), intermediate 3 (118.39 mg, 0.31 mmol), HATU (175.56 mg, 0.46 mmol), and DIPEA (79.46 mg, 0.62 mmol) were added sequentially to DMF (3 mL). The system was reacted under stirring at 25°C for 2 hours. Under an ice bath, 15 mL of water was added to the reaction solution to precipitate the product, followed by filtering. Then, the solid was washed with clean water to afford compound 205-8. MS m/z (ESI): 659.2 [M+1].

Step 9: Compound 205-8 (60 mg, 0.09 mmol) and TfOH (0.01 mL, 0.06 mmol) were added sequentially to TFA (2 mL). The system was reacted under stirring at room temperature for 2 hours. The reaction solution was concentrated to dryness. The crude was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 205. MS m/z (ESI): 539.2 [M+1]. ¹H NMR (400 MHz, DMSO-*d*₆) δ 12.44 (s, 1H), 8.97 (d, *J* = 1.6 Hz, 1H), 8.93 - 8.73 (m, 1H), 8.59 (s, 1H), 8.44 - 8.19 (m, 1H), 7.90 (d, *J* = 27.3 Hz, 1H), 6.37 - 6.05 (m, 1H), 3.99 (s, 1H), 3.34 - 3.25 (m, 2H), 1.64 (d, *J* = 56.9 Hz, 4H), 1.19 (dd, *J* = 6.5, 2.8 Hz, 3H).

### Example 206

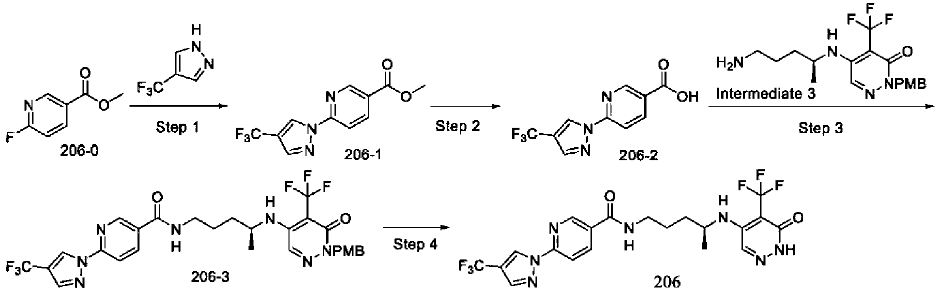

Step 1: Compound 206-0 (300 mg, 1.93 mmol), 4- (trifluoromethyl)-1H-pyrazole (341.91 mg, 2.51 mmol), and potassium carbonate (400.89 mg, 2.90 mmol) were added sequentially to solvent DMF (5 mL). The reaction was carried out at room temperature for 2 hours. EA (50 mL) and water (50 mL) were added. The organic phase is separated and concentrated to dryness, and purified by silica gel column chromatography with an eluant system (PE: EA=100: 1 to 10: 1) to afford compound 206-1. MS m/z (ESI): 272.1 [M+1].

Step 2: Compound 206-1 (330 mg, 1.22 mmol) was placed into a reaction flask. LiOH (0.05 mL, 1.83 mmol) and methanol (0.07 mL, 1.83 mmol) were added, and then solvent water (5 mL) was added. The reaction was carried out at room temperature for 2 hours. pH was adjusted to 5-6 by adding an acid, followed by filtering. The filter cake was concentrated to dryness to afford compound 206-2. MS m/z (ESI): 258.1 [M+1].

Step 3: Compound 206-2 (140 mg, 0.54 mmol), intermediate 3 (209.25 mg, 0.54 mmol), HATU (351.12 mg, 0.92 mmol), and DIPEA (238.38 mg, 1.86 mmol) were added sequentially to DMF (3 mL). The system was reacted under stirring at 25°C for 2 hours. Under an ice bath, 15 mL of water was added to the reaction solution to precipitate the product, followed by filtering. Then, the solid was washed with clean water to afford compound 206-3. MS m/z (ESI): 624.2 [M+1].

Step 4: Compound 206-3 (100 mg) was placed into a reaction flask. Trifluoromethylsulfonic acid (0.5 mL) was added, and then solvent TFA (3 mL) was added. The reaction was carried out at room temperature for 2 h. The reaction solution was concentrated to dryness and purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 206. MS m/z (ESI): 504.2 [M+1]. ¹H NMR (400 MHz, DMSO-*d*₆) δ 12.44 (s, 1H), 9.927 (s, 1H), 8.929 (s, 1H), 8.771 (s, 1H), 8.45 - 8.35 (m, 1H), 8.052-8.074 (m, 1H)7.93 (s, 1H), 6.35 (s, 1H), 3.99 (s, 1H), 3.34 - 3.22 (m, 2H), 1.565 - 1.183 (m, 4H), 1.19 (dd, *J* = 6.5, 4.2 Hz, 3H).

### Example 207

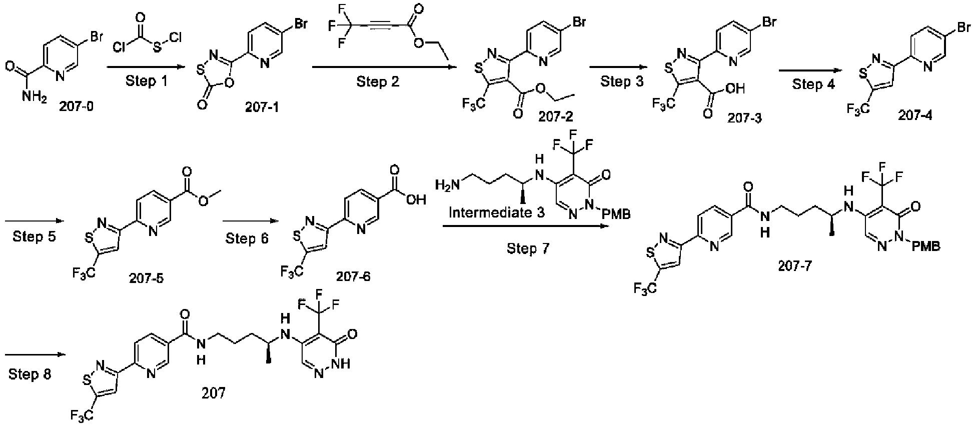

Step 1: Compound 207-0 (5 g, 24.87 mmol) was dissolved in toluene (40 mL). Chlorothiocarbonyl chloride (4.12 mL, 49.75 mmol) was added. The system was heated to 110°C and reacted under stirring for 16 hours. The reaction solution was concentrated to dryness to afford compound 207-1. MS m/z (ESI): 259.0 [M+1].

Step 2: Compound 207-1 (6 g, 23.16 mmol) was dissolved in 2,6-dichlorobenzene (40 mL), and then ethyl 4,4,4-trifluorobut-2-ynoate (2.37 mL, 23.16 mmol) was added. The system was heated to 150°C and reacted under stirring for 16 hours. The reaction solution was concentrated to dryness and purified by silica gel column chromatography with an eluant system (PE: EA=100: 1 to 2: 1) to afford compound 207-2. MS m/z (ESI): 381.0 [M+1].

Step 3: Compound 207-2 (4.5 g, 11.81 mmol), LiOH (1.49 g, 35.42 mmol), and water (20 mL) were added sequentially to methanol (30 mL). The system was reacted under stirring at room temperature for 2 hours. The reaction solution was cooled to 0°C, added with 6M hydrochloric acid for adjusting pH to 5-6, and filtered. The filter cake was concentrated to dryness to afford compound 207-3. MS m/z (ESI): 353.0 [M+1].

Step 4: Compound 207-3 (1.0 g, 2.7 mmol) was dissolved in dimethyl sulfoxide (10 mL). Cuprous oxide (3.57 g, 25.49 mmol) was added. The system was heated to 150°C and reacted under stirring for 16 hours. The reaction solution was cooled to room temperature. EA (50 mL) and water (50 mL) were added. The organic phase was separated and concentrated to dryness, and purified by silica gel column chromatography with an eluant system (PE: EA=100: 1 to 10: 1) to afford compound 207-4. MS m/z (ESI): 309.0 [M+1].

Step 5: Compound 207-4 (0.9 g, 2.91 mmol) was dissolved in methanol (10 mL). [1,1'-bis(diphenylphosphino)ferrocene]dichloropalladium (0.21 g, 0.29 mmol) and triethylamine (3 mL) were added sequentially. The system was heated to 100°C and reacted under stirring for 6 hours in a carbon monoxide environment. The reaction solution was concentrated to dryness and purified by silica gel column chromatography with an eluant system (PE: EA=100: 1 to 5: 1) to afford compound 207-5. MS m/z (ESI): 289.0 [M+1].

Step 6: Compound 207-5 (270 mg, 0.94 mmol) was dissolved in methanol (4 mL), and LiOH (0.08 mL, 2.81 mmol) and water (1 mL) were added sequentially. The system was reacted under stirring at 30°C for 3 hours. The pH of the system was adjusted to 5-6, followed by filtering. The filter cake was concentrated to dryness under reduced pressure under vacuum to afford compound 207-6. MS m/z (ESI): 275.0 [M+1].

Step 7: Compound 207-6 (100 mg, 0.36 mmol), intermediate 3 (140.18 mg, 0.36 mmol), HATU (208.41 mg, 0.55 mmol), and DIPEA (94.09 mg, 0.73 mmol) were added to DMF (2 mL). The system was reacted under stirring at 25°C for 2 hours. Under an ice bath, 15 mL of water was added to the reaction solution to precipitate the product, followed by filtering. Then, the solid was washed with clean water to afford compound 207-7. MS m/z (ESI): 641.2 [M+1].

Step 8: Compound 207-7 (105 mg, 0.16 mmol) and TfOH (0.5 mL) were added sequentially to TFA (2 mL). The system was reacted under stirring at room temperature for 2 hours. The reaction solution was concentrated to dryness. The crude was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 207. MS m/z (ESI): 521.2 [M+1]. ¹H NMR (400 MHz, DMSO-*d*₆) δ 12.44 (s, 1H), 9.33 - 8.98 (m, 1H), 8.80 (t, *J* = 5.7 Hz, 1H), 8.65 (d, *J=* 1.2 Hz, 1H), 8.42 - 8.22 (m, 2H), 7.93 (s, 1H), 6.35 (s, 1H), 3.99 (s, 1H), 3.34 - 3.22 (m, 2H), 1.63 (dt, *J* = 45.3, 7.6 Hz, 4H), 1.19 (dd, *J* = 6.5, 4.2 Hz, 3H).

### Example 209

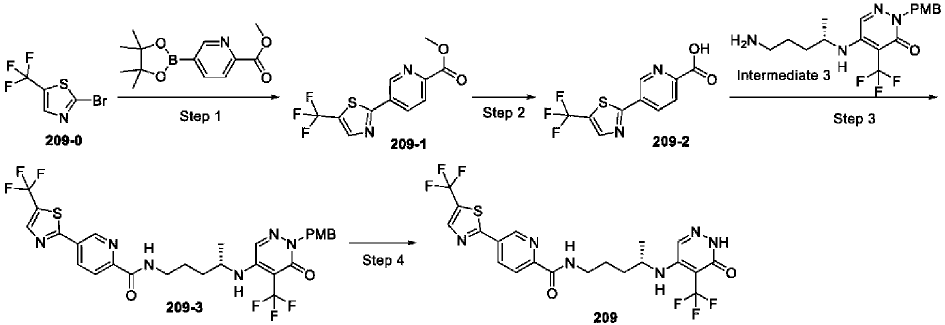

Step 1: Compound 209-0 (200 mg, 0.86 mmol, Bide) was dissolved in THF (8 mL). Then, methyl 5-(4,4,5,5-tetramethyl-1,3,2-dioxaborolan-2-yl)pyridin-2-carboxylate (272 mg, 1.03 mmol), palladium acetate (19.3 mg, 0.09 mmol), 4,5-bis(diphenylphosphino)-9,9-dimethylxanthene (99.7 mg, 0.17 mmol), and potassium phosphate (365 mg, 1.72 mmol) were added sequentially. The reaction was carried out at 60°C for 18 hours in a nitrogen environment. The reaction solution was concentrated under reduced pressure. The residue was purified by silica gel column chromatography with an eluant system (PE: EA=1:1 to 1:10) to afford compound 209-1. MS m/z(ESI):289.0[M+1].

Step 2: Compound 209-1 (220 mg, 0.76 mmol) was dissolved in THF (3 mL), methanol (1mL), and water (1 mL). LiOH (96.0 mg, 2.29 mmol) was added. The reaction was carried out at room temperature for 2 hours. The reaction mixture was adjusted to pH = 6 with HCl (1M). The mixture was poured into water (10 mL), followed by extraction with EA (10 mL×3). The combined organic phase was washed with brine (30 mL), dried over anhydrous sodium sulfate, filtered, and concentrated to dryness under reduced pressure, to afford compound 209-2. MS m/z(ESI):275.0[M+1].

Step 3: Compound 209-2 (70 mg, 0.26 mmol) was dissolved in DMF (4 mL). Then, HATU (194 mg, 0.51 mmol) and DIPEA (0.13 mL, 0.77 mmol) were added sequentially. After stirring at room temperature for 1 minutes, intermediate 3 (98.1 mg, 0.26 mmol) was added. The reaction was carried out at room temperature for 2 hours. The reaction solution was added with 50 mL water, followed by extraction with EA (50 mL×3). The organic phases were combined, and washed with half-saturated saline. The organic phases were dried over anhydrous sodium sulfate. After filtering to remove the desiccant, the filtrate was concentrated under reduced pressure. The residue was purified by preparative silica gel plate (PE: EA=1: 1) to afford compound 209-3. MS m/z(ESI):641.4[M+1].

Step 4: Compound 209-3 (90 mg, 0.14 mmol) was dissolved in TFA (2.00 mL), and TfOH (0.20 mL) was added. The reaction was carried out at room temperature for 1 hour. The reaction solution was concentrated under reduced pressure and purified by preparative high performance liquid chromatography (Phenomenex Gemini 150 mm×25 mm×10µm column, elution solution:30%-60% (v/v) acetonitrile and water, 0.025% formic acid) to afford compound 209. MS m/z(ESI): 521.2 [M+1]. ¹H NMR (400 MHz, DMSO-d6) δ 12.42 (s, 1H), 9.23 (d, J = 2.2 Hz, 1H), 9.00 (t, J = 6.2 Hz, 1H), 8.73 - 8.65 (m, 1H), 8.58 (dd, J = 8.2, 2.4 Hz, 1H), 8.17 (d, J =8.2 Hz, 1H), 7.91 (s, 1H), 6.39 - 6.27 (m, 1H), 4.06 - 3.91 (m, 1H), 3.35 - 3.27 (m, 2H), 1.73 - 1.46 (m, 4H), 1.17 (d, J = 6.4 Hz, 3H).

### Example 211

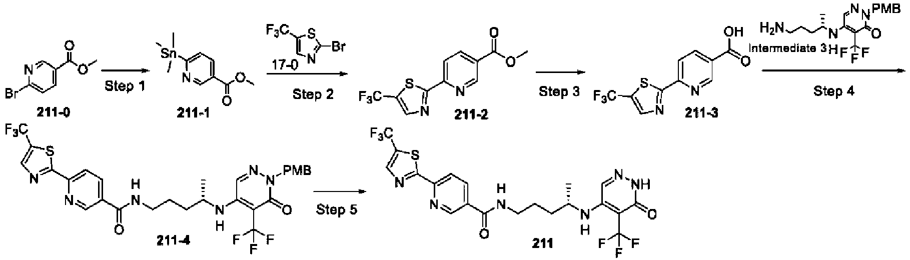

Step 1: Compound 211-0 (1000 mg, 4.63 mmol) and trimethyl(trimethyl-λ4-stannanyl)-λ4-stannane (1.06 mL, 5.09 mmol) were dissolved in toluene (3 mL) solution. Pd(PPh₃)₄ (320.79 mg, 0.28 mmol) was added. The mixture was degassed and purged 3 times with nitrogen, followed by stirring at 120°C for 2 hours under nitrogen. The reaction solution was concentrated under reduced pressure. The residue was added with 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (EA/PE=1/100-1/3) to afford compound 211-1. MS m/z(ESI): 300.4 [M+1].

Step 2: Compound 211-1 (300 mg, 1.00 mmol) and compound 17-0 (0.24 mL, 2.00 mmol) were dissolved in toluene (5 mL) solution. Pd(PPh₃)₄ (115.52 mg, 0.10 mmol) was added. The mixture was degassed and purged 3 times with nitrogen, followed by stirring at 120°C for 16 hours under nitrogen. The reaction solution was concentrated under reduced pressure. The residue was added to 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (EA/PE=1/100-1/3) to afford compound 211-2. MS m/z(ESI): 289.0 [M+1].

Step 3: Compound 211-2 (150 mg, 0.52 mmol) was dissolved in methanol:water=3:1 (10 mL). LiOH (0.10 mL, 6.26 mmol) was added to the mixture. The resulting mixture was stirred at room temperature of 25°C for 16 hours. The reaction solution was concentrated under reduced pressure to afford compound 211-3, which was used directly in the next step. MS m/z(ESI): 275.0 [M+1].

Step 4: Compound 211-3 (115 mg, 0.42 mmol) was dissolved in DMF (3 mL). Intermediate 3 (161.21 mg, 0.42 mmol), HATU (239.04 mg, 0.63 mmol), and DIPEA (0.22 mL, 1.26 mmol) were added. The resulting mixture was stirred at 25°C for 2 hours. The reaction solution was concentrated under reduced pressure. The residue was added to 10 mL water, followed by extraction with EA (15 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (EA/PE=1/100-1/3) to afford compound 211-4. MS m/z(ESI): 641.2 [M+1].

Step 5: Compound 211-4 (260 mg, 0.41 mmol) was dissolved in TFA (3 mL) solution, and TfOH (0.01 mL, 0.03 mmol) was added. The resulting mixture was stirred at room temperature for 1 hour. The reaction solution was concentrated under reduced pressure and purified by high performance liquid chromatography (Waters-SunFire-C18-10µm-19×250mm; mobile phase: water (containing 10 mmol/L formic acid) and acetonitrile, gradient ratio: acetonitrile 25%-95%, flow rate: 25 mL/min) to afford compound 211. MS m/z(ESI): 521.2 [M+1]. ¹H NMR (400 MHz, DMSO) δ 12.44 (s, 1H), 9.06(d, *J* = 1.4 Hz, 1H), 8.82(t, *J =* 5.6 Hz, 1H), 8.66 (d, *J* = 1.2 Hz, 1H), 8.40 (dd, *J* = 8.2 Hz, 2.2 Hz, 1H), 8.29 (d, *J =* 8.2 Hz, 1H),7.93 (s, 1H), 6.41-6.29 (m, 1H), 4.08-3.89 (m, 1H),3.32-3.26 (m, 2H), 1.79-1.63(m, 1H),1.62-1.48 (m, 3H) 1.19 (d, *J* = 6.2 Hz, 3H).

### Example 212

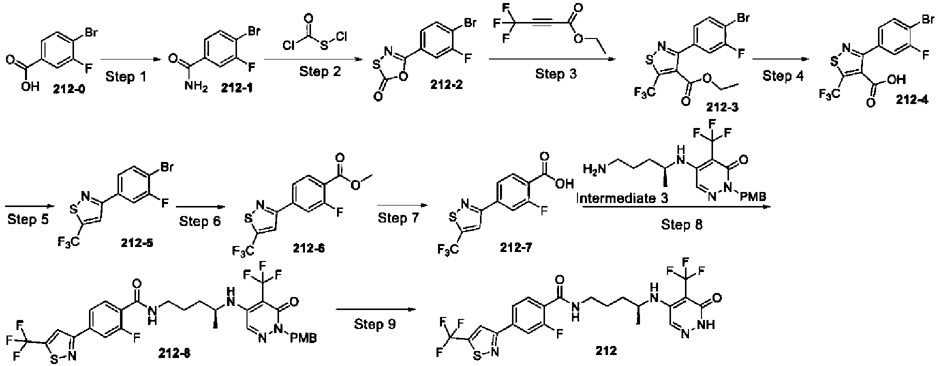

Step 1: At room temperature, compound 212-0 (5 g, 22.83 mmol), ammonium chloride (1.22 g, 22.83 mmol), and HATU (11.29 g, 29.68 mmol) were dissolved in DMF (100 mL). DIPEA (9.46 mL, 57.08 mmol) was added, followed by stirring at 25°C for 18 hours. After the reaction was completed, the reaction solution was poured into water (500 mL), followed by extraction with DCM (250 mL×3). The organic phases were combined, washed with saturated saline (200 mL), dried over anhydrous sodium sulfate, filtered and then concentrated under reduced pressure to afford the residue. The residue was purified by silica gel column (EA/PE=0%~100%) to afford the crude. The crude was slurried with PE/DCM (10: 1, 200 mL) to afford compound 212-1. MS m/z (ESI): 218.0 [M+1].

Step 2: At room temperature, compound 212-1 (4.76 g, 21.83 mmol) was dissolved in toluene (80 mL). Chlorocarbonylsulfenyl chloride (2.17 mL, 26.20 mmol) was added, followed by stirring at 110°C for 18 hours under a nitrogen atmosphere. After the reaction was completed, the reaction solution was concentrated under reduced pressure to afford the residue. The residue was purified by silica gel column (EA/PE=0%~30%) to afford compound 212-2. MS m/z (ESI): 276.0 [M+1].

Step 3: At room temperature, compound 212-2 (2.50 g, 9.06 mmol) was dissolved in 1,3-dichlorobenzene (40 mL). Ethyl 4,4,4-trifluoro-2-butynoate (1.39 mL, 13.58 mmol) was added, followed by stirring at 145°C for 18 hours under a nitrogen atmosphere. After the reaction was completed, the reaction solution was concentrated under reduced pressure to afford the residue. The residue was purified by silica gel column (EA/PE=0%~30%) to afford compound 212-3. MS m/z (ESI): 398.0 [M+1].

Step 4: At room temperature, compound 212-3 (3.20 g, 8.04 mmol) and LiOH monohydrate (675 mg, 24.12 mmol) were dissolved in THF (30 mL). Methanol (30 mL) and water (30 mL) were added, followed by stirring at 25°C for 1 hour. After the reaction was completed, 1M hydrochloric acid was added to the reaction solution for adjusting pH to 4, followed by extraction with EA (50 mL×3). The organic phases were combined, washed with saturated saline (30 mL), dried over anhydrous sodium sulfate, filtered and then concentrated under reduced pressure to afford the crude. The crude was purified by silica gel column (methanol: DCM = 0%~10%) to afford compound 212-4. MS m/z (ESI): 370.0 [M+1].

Step 5: At room temperature, compound 212-4 (1.41 g, 3.81 mmol) and cuprous oxide (2.21 g, 15.24 mmol) were dissolved in DMSO (28 mL), followed by stirring at 145°C for 6 hours under a nitrogen atmosphere. After the reaction was completed, the reaction solution was filtered. The filter cake was washed with EA (10 mL×3). Ammonia water (20 mL) and water (200 mL) were added to the filtrate, followed by extraction with EA (100 mL×3). The organic phases were combined, washed with saturated saline (100 mL), dried over anhydrous sodium sulfate, filtered and then concentrated under reduced pressure to afford the crude. The crude was purified by silica gel column (EA/PE=0%~20%) to afford compound 212-5. MS m/z (ESI): 326.0 [M+1].

Step 6: Compound 212-5 (900 mg, 2.76 mmol) was dissolved in methanol (10 mL). At room temperature, the reaction solution was added with triethylamine (837.8 mg, 8.28 mmol) and [1,1'-bis(diphenylphosphino)ferrocene]dichloropalladium (201.94 mg, 0.28 mmol). The reaction solution was reacted at 80°C for 18 hours under a carbon monoxide atmosphere. The reaction solution was filtered, and then the solid was washed with EA (20 mL×3), followed by drying by spin under reduced pressure to afford the crude. The crude was purified by silica gel column chromatography (PE/EA=100: 1 to 1: 1) to afford compound 212-6. MS m/z(ESI):306.01 [M+1].

Step 7: Compound 212-6 (400 mg, 1.31 mmol) was dissolved in methanol (4 mL), THF (4 mL), and water (4 mL). At room temperature, LiOH monohydrate (164.95 mg, 3.93 mmol) was added. The reaction solution was reacted at room temperature for 1 hour. The reaction solution was concentrated to dryness to afford compound 212-7. MS m/z(ESI):292.0 [M+1].

Step 8: At room temperature, compound 212-7 (270 mg, 0.93 mmol) was dissolved in acetonitrile (5 mL). Intermediate 3 (356.39 mg, 0.93 mmol), TCFH (521.88 mg, 1.86 mmol), and N-methylimidazole (296.55 µL, 3.72 mmol) were added, followed by stirring at 25°C for 18 hours. After the reaction was completed, the reaction solution was poured into water (10 mL), followed by extraction with EA (15 mL×3). The organic phases were combined, washed with saturated saline (20 mL), dried over anhydrous sodium sulfate, filtered and then concentrated under reduced pressure to afford the crude. The crude was purified by silica gel column (EA/PE=50%~60%) to afford compound 212-8. MS m/z (ESI): 658.2 [M+1].

Step 9: At room temperature, compound 212-8 (250 mg, 0.38 mmol) was dissolved in TFA (5 mL). Then, TfOH (135.03 µL, 1.52 mmol) was added, followed by stirring at 25°C for 3 hours until the reaction was completed. The reaction solution was concentrated to dryness under reduced pressure to afford the crude product. The crude product was purified by preparative HPLC (chromatographic column: Waters-SunFire-C18-10µm-19×250mm; mobile phase: A: 0.1% formic acid/water B: acetonitrile, gradient ratio: acetonitrile 54%~84%, flow rate: 20 mL/min) to afford compound 212. MS m/z (ESI):538.1 [M+1].¹H NMR (400 MHz, DMSO-d6) *δ* 12.45 (s, 1H), 8.81 (d, *J* = 1.2 Hz, 1H), 8.47 - 8.41 (m, 1H), 8.05 - 7.99 (m, 2H), 7.93 (s, 1H), 7.72 (t, *J* = 7.6 Hz, 1H), 6.37 - 6.29 (m, 1H), 4.05 - 3.92 (m, 1H), 3.31 - 3.21 (m, 2H), 1.72 - 1.47 (m, 4H), 1.19 (d, *J* = 6.4 Hz, 3H).

### Example 218

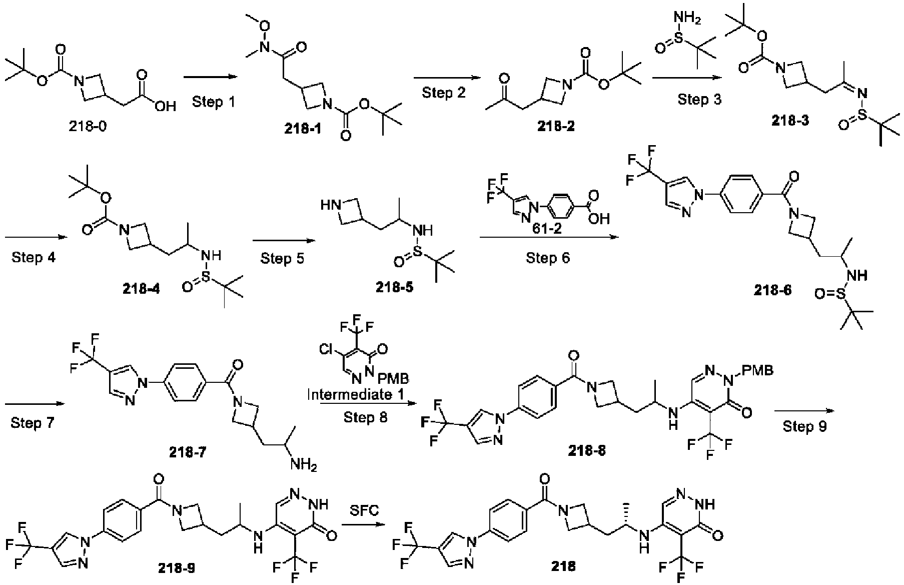

Step 1: At 0°C under a nitrogen atmosphere, dimethylhydroxylamine hydrochloride (2.27 g, 23.20 mmol), DIPEA (9.62 mL, 58.1 mmol), and HATU (11.5 g, 30.2 mmol) were added to a solution of compound 218-0 (5.00 g, 23.2 mmol, Bide) in DCM (50 mL). The mixture was reacted under stirring at 25°C for 18 hours under a nitrogen atmosphere. The mixture was quenched with water (5 mL) and then concentrated to remove DCM. Water (100 mL) was added, followed by extraction with EA (100 mL×3). The organic phases were washed with saturated saline (200 mL), dried over anhydrous sodium sulfate, filtered and concentrated to afford the crude. The crude product was purified by silica gel column (THF: PE=0-60%) to afford compound 218-1. MS m/z(ESI):259.2 [M+1].

Step 2: At -78°C, under a nitrogen atmosphere, methylmagnesium bromide (12.9 mL, 38.7 mmol) was slowly added dropwise to a solution of compound 218-1 (5.00 g, 19.4 mmol) in THF (55 mL). The solution was stirred at -78°C for 1 hour, followed by heating to 25°C and reacting for 2 hours. After the reaction was completed, the mixture was poured into water (50 mL), followed by extraction with EA (50 mL×3). The combined organic phase was washed with brine (100 mL×2), dried over anhydrous sodium sulfate, filtered and concentrated to afford the crude. The crude product was purified by silica gel column (THF: PE=0-30%) to afford compound 218-2. MS m/z(ESI):214.1 [M+1].

Step 3: At 25°C, under a nitrogen atmosphere, tetraethyl titanate (5.89 mL, 28.1 mmol) was added dropwise to a solution of compound 218-2 (2.00 g, 9.38 mmol) and tert-butylsulfinamide (1.70 g, 14.1 mmol) in THF (20 mL). The solution was reacted at 70°C for 18 hours. After the reaction was completed, the mixture was poured into water (50 mL), followed by filtering. The filtrate was extracted with EA (50 mL×3). The combined organic phase was washed with brine (100 mL×2), dried over anhydrous sodium sulfate, filtered and concentrated to afford compound 218-3. MS m/z(ESI):317.1 [M+1].

Step 4: At -78°C, under a nitrogen atmosphere, 1M of a solution of lithium Triethylborohydride in THF (22.1 mL) was slowly added dropwise to a solution of compound 218-3 (3.50 g, 11.1 mmol) in THF (50 mL). The solution was reacted at -78°C for 2 hours. After the reaction was completed, the mixture was poured into water (50 mL), followed by extraction with EA (50 mL×3). The combined organic phase was washed with brine (100 mL×2), dried over anhydrous sodium sulfate, filtered and concentrated to afford the crude. The crude product was purified by silica gel column (methanol: DCM=0-10%) to afford compound 218-4. MS m/z (ESI): 319.2 [M+1].

Step 5: compound 218-4 (1 g, 3.14 mmol) was dissolved in a solution of 4M hydrochloric acid in 1,4-dioxane (6 mL). The reaction was carried out at room temperature for 1 hour. The reaction solution was concentrated under reduced pressure to afford compound 218-5. MS m/z(ESI):219.2 [M+1].

Step 6: Compound 218-5 (762 mg, 1.10 mmol) was dissolved in DMF (5.00 mL). Then, HATU (2.26 g, 5.95 mmol) and DIPEA (2.47 mL, 14.8 mmol) were added sequentially. After stirring at room temperature for 1 minute, compound 61-2 (650 mg, 2.98 mmol) was added. The reaction was carried out at room temperature for 2 hours. The reaction solution was added to 50 mL water, followed by extraction with EA (50 mL×3). The organic phases were combined, and washed with saturated saline. The organic phases were dried over anhydrous sodium sulfate and filtered. The filtrate was concentrated under reduced pressure. The residue was purified by silica gel column chromatography with an eluant system (PE/EA=10:1-1:1) to afford compound 218-6. MS m/z(ESI):457.2[M+1].

Step 7: Compound 218-6 (180 mg, 0.39 mmol) was dissolved in a solution of 4M hydrochloric acid in 1,4-dioxane (3 mL). The reaction was carried out at room temperature for 0.5 hours. The reaction solution was concentrated under reduced pressure to afford compound 218-7. MS m/z(ESI): 353.2[M+1].

Step 8: Compound 218-7 (140 mg, 0.40 mmol) was dissolved in isopropanol (3 mL), and then intermediate 1 (126 mg, 0.40 mmol) and DIPEA (0.33 mL, 1.99 mmol) were added sequentially. The reaction was carried out at 60°C for 18 hours. The reaction solution was concentrated under reduced pressure. The residue was purified by preparative silica gel plate (PE/EA=1:1) to afford compound 218-8. MS m/z(ESI): 635.4 [M+1].

Step 9: Compound 218-8 (76.0 mg, 0.12 mmol) was dissolved in TFA (1.00 mL). TfOH (0.2 mL) was added. The reaction was carried out at room temperature for 1 hour. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford 218-7 (45.0 mg), followed by chiral resolution (instrument: Waters UPC2 analytical SFC (SFC-H); column: ChiralPak AD, 50×4.6 mm I.D., 3 µm; mobile phase: A for CO₂ and B for Ethanol (0.05%DEA); gradient: B 40% ; flow rate: 3 mL/min; pressure: 100 bar; column temperature: 35°C; wavelength: 220 nm) to afford compound 218. MS m/z(ESI): 515.4 [M+1]. 1H NMR (400 MHz, DMSO-d6) δ 12.46 (s, 1H), 9.29 (s, 1H), 8.26 (s, 1H), 8.01 - 7.94 (m, 2H), 7.92 (d, J = 9.0 Hz, 1H), 7.81 - 7.74 (m, 2H), 6.37 (s, 1H), 4.40 (q,J = 7.6 Hz, 1H), 4.18 - 3.96 (m, 3H), 3.82 - 3.70 (m, 1H), 2.75 - 2.62 (m, 1H), 2.09 - 1.95 (m, 1H), 1.90 - 1.77 (m, 1H), 1.17 (d, J = 6.4 Hz, 3H).

### Example 219

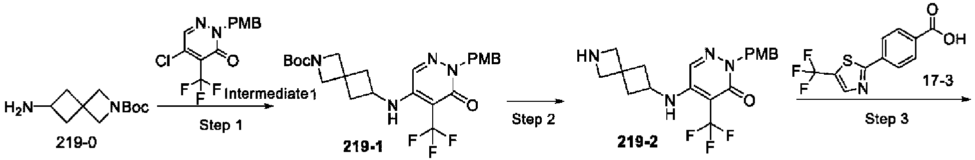

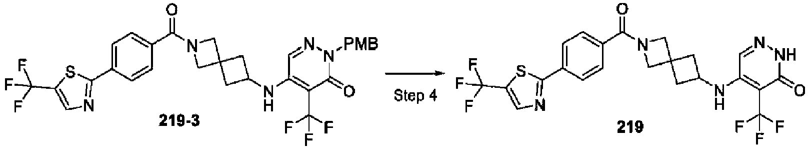

Step 1: At room temperature, compound 219-0 (200 mg, 0.94 mmol) and intermediate 1 (300.23 mg, 0.94 mmol) were dissolved in isopropanol (5 mL). DIPEA (0.47 mL, 2.83 mmol) was added, followed by stirring at 80°C for 18 hours. After the reaction was completed, the reaction solution was poured into water (150 mL), followed by extraction with EA (20 mL×3). The organic phases were combined, washed with saturated saline (30 mL), dried over anhydrous sodium sulfate, filtered and then concentrated under reduced pressure to afford the crude. The crude was purified by silica gel column (eluant: EA: PE = 50%~70%) to afford compound 219-1. MS m/z (ESI): 495.4 [M+1].

Step 2: At room temperature, compound 219-1 (330 mg, 0.67 mmol) was dissolved in DCM (5 mL). TFA (1.49 mL, 20.02 mmol) was added, followed by stirring at 25°C for 1 hour. After the reaction was completed, the reaction solution was concentrated under reduced pressure to afford compound 219-2. MS m/z (ESI): 395.2 [M+1].

Step 3: At room temperature, compound 219-2 (50 mg, 0.13 mmol) was dissolved in acetonitrile (1 mL). Compound 17-3 (34.64 mg, 0.13 mmol), TCFH (71.14 mg, 0.25 mmol), and N-methylimidazole (80.85 µL, 1.01 mmol) were added, followed by stirring at 25°C for 18 hours. After the reaction was completed, the reaction solution was poured into water (10 mL), followed by extraction with EA (15 mL×3). The organic phases were combined, washed with saturated saline (20 mL), dried over anhydrous sodium sulfate, filtered and then concentrated under reduced pressure to afford compound 219-3. MS m/z (ESI): 650.4 [M+1].

Step 4: At room temperature, compound 219-3 (40 mg, 0.06 mmol) was dissolved in TFA (1 mL). Then, TfOH (273.39 µL, 3.08 mmol) was added, followed by stirring at 25°C for 1 hour. After concentration to dryness, the crude was purified by preparative HPLC (chromatographic column: Waters-SunFire-C18-10µm-19×250mm; mobile phase: A: 0.1% formic acid/water B: acetonitrile, gradient ratio: acetonitrile 48%~78%, flow rate: 20 mL/min) to afford compound 219. MS m/z (ESI):530.2 [M+1]. ¹H NMR (400 MHz, DMSO-*d6*) *δ* 12.54 (s, 1H), 8.60 (s, 1H), 8.13 - 8.06 (m, 2H), 7.82 - 7.70 (m, 3H), 6.85 (s, 1H), 4.45 - 4.00 (m, 5H), 2.69 - 2.60 (m, 2H), 2.40 - 2.29 (m, 2H).

### Example 226

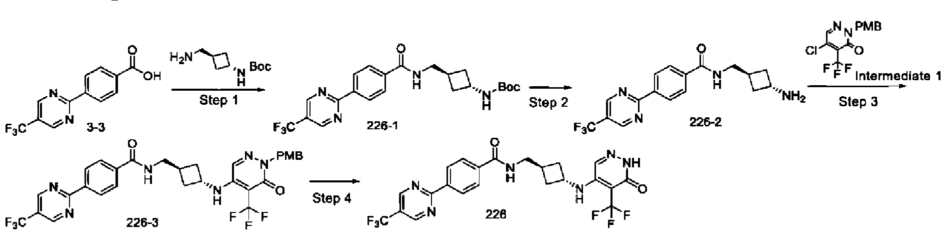

Step 1: Compound 3-3 (250 mg, 0.93 mmol), tert-butyl ((1r,3r)-3-(aminomethyl)cyclobutyl)carbamate (205.36 mg, 1.03 mmol), HATU (531.66 mg, 1.40 mmol), and DIPEA (0.46 mL, 2.80 mmol) were added sequentially to DMF (4 mL), followed by reacting under stirring at room temperature for 2 hours. The resulting mixture was extracted with EA (100 mL), washed with water (50 mL×3) and saturated saline (50 mL), respectively, dried over anhydrous sodium sulfate, and filtered. The filtrate was concentrated to dryness, and purified by silica gel column chromatography (EA/PE=0 to 50%) to afford compound 226-1. MS m/z(ESI): 451.2[M+1].

Step 2: Compound 226-1 (220 mg, 0.49 mmol) was dissolved in 1,4-dioxane (1 mL). A solution of 4M hydrochloric acid in 1,4-dioxane (4 mL) was added, followed by reacting under stirring at room temperature for 2 hours. The reaction solution was directly concentrated to dryness to afford compound 226-2. MS m/z(ESI): 351.2[M+1].

Step 3: Compound 226-2 (180 mg, 0.51 mmol), intermediate 1 (327.46 mg, 1.03 mmol), HATU (244.34 mg, 0.64 mmol), and DIPEA (0.34 mL, 2.06 mmol) were added sequentially to DMF (4 mL), followed by reacting under stirring at room temperature for 2 hours. Water (20 mL) was added, followed by filtering. The filter cake was concentrated to afford compound 226-3. MS m/z(ESI):633.2 [M+1].

Step 4: Compound 226-3 (190 mg, 0.30 mmol) was added to TFA (5 mL). Then, TfOH (0.5 mL) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 226. MS m/z(ESI): 513.3[M+1]. ¹H NMR (400 MHz, DMSO-*d*₆) δ 12.54 (s, 1H), 9.39 (d, *J* = 1.0 Hz, 2H), 8.71 (t, *J* = 5.6 Hz, 1H), 8.59 - 8.45 (m, 2H), 8.12 - 7.88 (m, 2H), 7.69 (s, 1H), 6.85 (q, *J* = 3.2 Hz, 1H), 4.39 (q, *J* = 7.2 Hz, 1H), 3.46 (dd, *J* = 7.6, 5.7 Hz, 2H), 2.24 (t, *J* = 7.0 Hz, 4H).

### Example 231

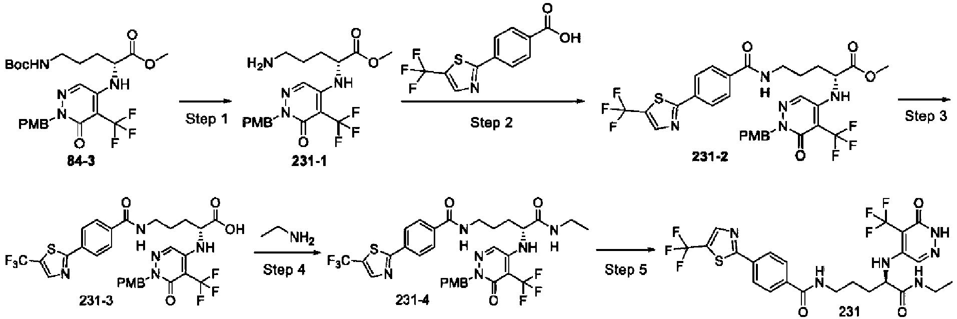

Step 1: Compound 84-3 (4.5 g, 8.51 mmol) was added to a solution of hydrochloric acid (80.0 mmol, 4 M) in 1,4-dioxane (20 mL). The reaction was carried out at room temperature for 1 hour. Compound 231-1 was afforded by concentration under reduced pressure. MS m/z(ESI):429.2[M+1].

Step 2: 4-(5-(trifluoromethyl)thiazol-2-yl)benzoic acid (2.30 g, 8.42 mmol) was dissolved in DMF (30 mL). Then, HATU (6.40 mg, 16.84 mmol) and DIPEA (6.97 mL, 42.09 mmol) were added sequentially. After stirring at room temperature for 5 minutes, compound 231-1 (4.33 g, 10.1 mmol) was added. The reaction was carried out at room temperature for 2 hours. The reaction solution was diluted with water (50 mL), followed by extraction with EA (50 mL×3). The organic phases were combined, and washed with saturated saline. The organic phases were dried over anhydrous sodium sulfate. After filtering to remove the desiccant, the filtrate was concentrated under reduced pressure. The residue was purified by silica gel column chromatography with an eluant system (PE: EA=10: 1 to 1: 1) to afford compound 231-2. MS m/z(ESI): 684.4[M+1].

Step 3: Compound 231-2 (5.0 g, 7.31 mmol) was dissolved in a mixed solvent of methanol (15 mL), THF (20 mL), and water (15 mL). LiOH (0.92 g, 21.94 mmol) was added. The reaction was carried out at room temperature for 1 hour. The reaction solution was concentrated under reduced pressure. The residue was added with water (40 mL), adjusted to pH=6 with hydrochloric acid solution (1M), and filtered under reduced pressure. The filter cake was washed with water(10 mL), and oven-dried to afford compound 231-3. MS m/z(ESI):669.8 [M+1].

Step 4: Ethylamine (450 mg, 5.0 mmol), compound 231-3 (335 mg, 0.50 mmol), 2- (7-azabenzotriazol-1-yl) -N, N, N', N'-tetramethyluronium hexafluorophosphate (296.86 mg, 0.78 mmol), and DIPEA (0.19 mL, 1.17 mmol) were reacted under stirring at 25°C for 2 hours. Under an ice bath, 15 mL of water was added to the reaction solution to precipitate the product, followed by filtering. Then, the solid was washed with clean water to afford compound 231-4. MS m/z (ESI): 697.2 [M+1].

Step 5: Compound 231-4 (190 mg, 0.27 mmol) was added to TFA (2 mL), and TfOH (0.01 mL, 0.16 mmol) was added dropwise. The reaction was carried out at room temperature for two hours. Methanol (1 mL) was added, followed by drying by spin. The crude was purified by preparative HPLC (chromatographic column: Waters-Xbridge-C18-10µm-19×250mm; mobile phase: A: 10 mM ammonium bicarbonate/water B: acetonitrile, gradient ratio: acetonitrile 43%-95%, flow rate: 20 mL/min) to afford compound 231. MS m/z (ESI): 577.2 [M+1]. ¹H NMR (400 MHz, DMSO-*d*₆) δ 12.62 (s, 1H), 8.66 (t, *J* = 5.6 Hz, 1H), 8.60 (d, *J* = 1.3 Hz, 1H), 8.26 (t, *J =* 5.6 Hz, 1H), 8.15 - 8.08 (m, 2H), 8.00 - 7.93 (m, 2H), 7.69 (s, 1H), 6.66 (d, *J* = 7.6 Hz, 1H), 4.39 (d, *J =* 6.6 Hz, 1H), 3.27 (t, *J =* 6.4 Hz, 2H), 3.13 (p, *J =* 6.7 Hz, 2H), 1.83 (dd, *J* = 10.2, 5.6 Hz, 2H), 1.50 (t, *J* = 8.0 Hz, 2H), 1.03 (t, *J =* 7.2 Hz, 3H).

The preparation methods of the respective compounds in the table below can be referred to example 231, except that the ethylamine in the step 4 is replaced with the respective raw materials in the table below. That is, the preparation routes for preparing compound 231 in example 231 can be referred to, and the ethylamine in the step 4 is replaced to afford a compound similar to compound 231.

| Example | Structure | Raw material | ¹H NMR & MS |
|---|---|---|---|
| 220 | 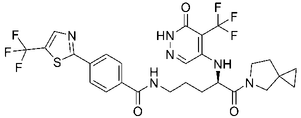 | 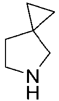 | ¹H NMR (400 MHz, DMSO-*d*₆) δ 12.59 (s, 1H), 8.67 - 8.57 (m, 2H), 8.11 (d, *J* = 8.1 Hz, 2H), 7.96 - 7.93 (m, 3H), 6.85 (s, 1H), 5.52 (s, 1H),4.86 (s, 1H), 4.48 - 4.01 (m, 3H), 3.27 (s, 2H),2.08 - 2.06 (m, 2H), 1.51 - 1.48 (m, 2H), 1.23 - 1.01 (m,6H). |
| | | | MS m/z (ESI): 629.0 [M+1] |
| 221 | 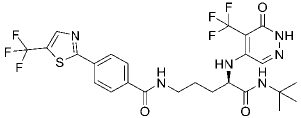 | 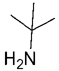 | ¹H NMR (400 MHz, DMSO-*d*₆) δ 12.63 (s, 1H), 8.67 (t, *J =* 5.6 Hz, 1H), 8.60 (q, *J* = 1.3 Hz, 1H), 8.15 - 8.07 (m, 2H), 8.00 - 7.93 (m, 2H), 7.88 (s, 1H), 7.61 (s, 1H), 6.62 (dd, *J =* 7.5, 4.1 Hz, 1H), 4.35 (d, *J* = 6.6 Hz, 1H), 3.28 (q, *J =* 6.5 Hz, 2H), 1.81 (dt, *J* = 14.0, 7.3 Hz, 2H), 1.49 (q, *J* = 7.4 Hz, 2H), 1.26 (s, 9H). |
| | | | MS m/z (ESI): 605.2 [M+1] |
| 222 | 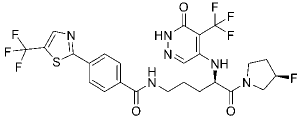 | 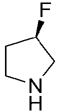 | ¹H NMR (400 MHz, DMSO-*d*₆) δ 12.59 (d, *J* = 5.1 Hz, 1H), 8.63 (d, *J* = 22.5 Hz, 2H), 8.10 (dd, *J* = 8.4, 2.7 Hz, 2H), 8.02 - 7.91 (m, 3H), 6.92 - 6.77 (m, 1H), 5.38 (dd, *J* = 52.9, 36.5 Hz, 1H), 5.02 - 4.76 (m, 1H), 3.75 - 3.65 (m, 4H), 3.26 (d, *J* = 6.5 Hz, 2H), 2.27 - 2.00 (m, 2H), 1.91 - 1.73 (m, 2H), 1.52 (d, *J =* 31.7 Hz, 2H). |
| | | | MS m/z (ESI): 621.2 [M+1] |
| 223 | 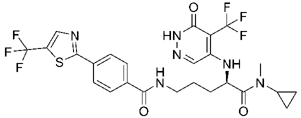 | 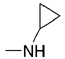 | ¹H NMR (400 MHz, DMSO-*d*₆) δ 12.58 (d, *J* = 13.7 Hz, 1H), 8.65 (s, 1H), 8.60 (t, *J =* 1.3 Hz, 1H), 8.14 - 8.07 (m, 2H), 7.96 (d, *J =* 7.9 Hz, 3H), 6.93 (d, *J* = 56.1 Hz, 1H), 5.11 (d, *J =* 66.8 Hz, 1H), 3.28 (d, *J* = 6.2 Hz, 2H), 2.99 (s, 1H), 2.85 (s, 3H), 1.88 (d, *J* = 7.2 Hz, 2H), 1.52 (d, *J* = 19.9 Hz, 2H), 1.09 - 0.61 (m, 4H). |
| | | | MS m/z (ESI): 603.2 [M+1] |
| 224 | 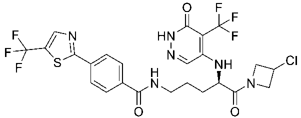 | 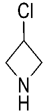 | ¹H NMR (400 MHz, DMSO-*d*₆) δ 12.62 (s, 1H), 8.69 - 8.57 (m, 2H), 8.11 (d, *J* = 8.4 Hz, 2H), 7.96 (dd, *J =* 8.3, 3.7 Hz, 2H), 7.90 (d, *J =* 8.8 Hz, 1H), 6.67 (s, 1H), 4.94 - 4.66 (m, 3H), 4.48 (ddd, *J* = 20.1, 11.0, 7.1 Hz, 2H), 3.97 (ddd, *J* = 24.1, 10.9, 4.0 Hz, 1H), 3.28 (t, *J* = 6.6 Hz, 2H), 1.83 - 1.65 (m, 2H), 1.62 - 1.43 (m, 2H). |
| | | | MS m/z (ESI): 623.2 [M+1] |
| 225 | 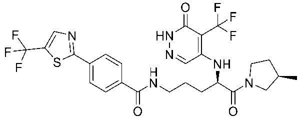 | 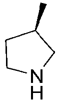 | ¹H NMR (400 MHz, DMSO-*d*₆) δ 12.57 (s, 1H), 8.67 - 8.57 (m, 2H), 8.11 (d, *J* = 8.1 Hz, 2H), 8.00 - 7.92 (m, 3H), 6.93 (d, *J* = 4.8 Hz, 1H), 4.90 (s, 1H), 3.94 - 3.76 (m, 1H), 3.36 - 3.18 (m, 3H), 2.95 - 2.77 (m, 1H), 2.31 - 1.93 (m, 2H), 1.78 (d, *J* = 16.8 Hz, 2H), 1.56 - 1.29 (m, 3H), 1.03 - 0.94 (m, 3H). |
| | | | MS m/z (ESI): 617.0 [M+1] |
| 227 | 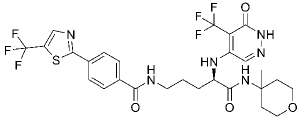 | 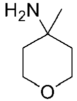 | ¹H NMR (400 MHz, DMSO-*d*₆) δ 12.65 (s, 1H), 8.70 (t, *J* = 5.7 Hz, 1H), 8.60 (d, *J* = 1.4 Hz, 1H), 8.15 - 8.07 (m, 2H), 8.01 - 7.94 (m, 2H), 7.83 (s, 1H), 7.65 (s, 1H), 6.61 (dd, *J =* 7.7, 4.1 Hz, 1H), 4.42 (d, *J* = 6.8 Hz, 1H), 3.58 (d, *J* = 11.4 Hz, 2H), 3.31 (t, *J* = 6.4 Hz, 4H), 2.00 (dd, *J* = 36.3, 13.7 Hz, 4H), 1.58 - 1.42 (m, 4H), 1.28 (s, 3H). |
| | | | MS m/z (ESI): 647.0 [M+1] |
| 228 | 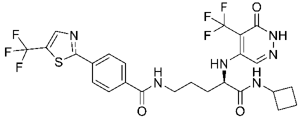 | 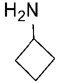 | ¹H NMR (400 MHz, DMSO-*d*₆) δ 12.62 (s, 1H), 8.67 (t, *J =* 5.7 Hz, 1H), 8.60 (q, *J* = 1.3 Hz, 1H), 8.50 (d, *J =* 7.3 Hz, 1H), 8.15 - 8.07 (m, 2H), 8.00 - 7.93 (m, 2H), 7.67 (s, 1H), 6.64 (dd, *J* = 7.3, 4.3 Hz, 1H), 4.36 (d, *J =* 6.6 Hz, 1H), 4.18 (q, *J* = 8.0 Hz, 1H), 3.28 (q, *J* = 6.5 Hz, 2H), 2.16 (dq, *J =* 7.4, 4.0 Hz, 2H), 1.95 - 1.79 (m, 4H), 1.70 - 1.57 (m, 2H), 1.49 (t, *J* = 8.1 Hz, 2H). |
| | | | MS m/z (ESI): 603.2 [M+1] |
| 229 | 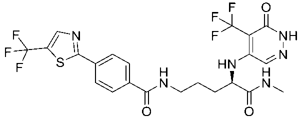 | 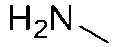 | ¹H NMR (400 MHz, DMSO-*d*₆) δ 12.62 (s, 1H), 8.66 (t, *J =* 5.6 Hz, 1H), 8.60 (t, *J* = 1.3 Hz, 1H), 8.18 (q, *J* = 4.5 Hz, 1H), 8.15 - 8.08 (m, 2H), 8.01 - 7.94 (m, 2H), 7.70 (s, 1H), 6.66 (dd, *J* = 7.5, 4.2 Hz, 1H), 4.41 (q, *J* = 6.5 Hz, 1H), 3.27 (q, *J* = 6.5 Hz, 2H), 2.65 (d, *J* = 4.5 Hz, 3H), 1.87 - 1.80 (m, 2H), 1.49 (q, *J* = 7.6 Hz, 2H). |
| | | | MS m/z (ESI): 563.2 [M+1] |
| 230 | 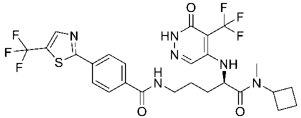 | 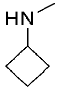 | ¹H NMR (400 MHz, DMSO-*d*₆) δ 12.43 (s, 1H), 9.30 (d, *J =* 1.4 Hz, 1H), 8.59 (t, *J* = 5.6 Hz, 1H), 8.27 (s, 1H), 8.00 (s, 4H), 7.94 (s, 1H), 6.36 (dd, *J* = 8.9, 3.8 Hz, 1H), 3.99 (s, 1H), 3.27 (s, 2H), 1.87 - 1.34 (m, 4H), 1.19 (d, *J =* 6.3 Hz, 3H). |
| | | | MS m/z (ESI): 617.3 [M+1] |
| 232 | 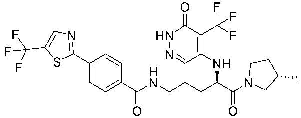 | 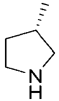 | ¹H NMR (400 MHz, DMSO-*d*₆) δ 12.57 (s, 1H), 8.62 (d, *J* = 15.1 Hz, 2H), 8.14 - 8.07 (m, 2H), 7.99 - 7.91 (m, 3H), 6.89 (s, 1H), 4.86 (d, *J* = 24.1 Hz, 1H), 3.58 (d, *J* = 24.1 Hz, 3H), 3.24 (dd, *J* = 14.0, 7.4 Hz, 3H), 2.33 - 2.16 (m, 1H), 2.04 - 1.88 (m, 1H), 1.79 (d, *J =* 5.1 Hz, 2H), 1.56 - 1.35 (m, 3H), 1.03 - 0.94 (m, 3H). |
| | | | MS m/z (ESI): 617.2 [M+1] |
| 233 | 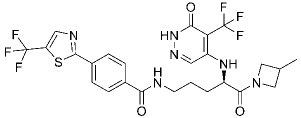 | 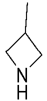 | ¹H NMR (400 MHz, DMSO-*d*₆) δ 12.59 (s, 1H), 8.70 - 8.57 (m, 2H), 8.14 - 8.08 (m, 2H), 8.00 - 7.89 (m, 3H), 6.74 (t, *J* = 6.2 Hz, 1H), 4.68 (s, 1H), 4.34 (dt, *J =* 74.3, 8.5 Hz, 1H), 4.03 (dt, *J* = 25.0, 9.0 Hz, 1H), 3.79 (ddd, *J* = 86.8, 8.6, 5.4 Hz, 1H), 3.31 - 3.23 (m, 2H), 2.69 (s, 1H), 2.54 (s, 1H), 1.82 - 1.66 (m, 2H), 1.48 (dd, *J =* 11.5, 5.7 Hz, 2H), 1.16 (dd, *J* = 8.7, 6.9 Hz, 3H). |
| | | | MS m/z (ESI): 603.2 [M+1] |
| 234 | 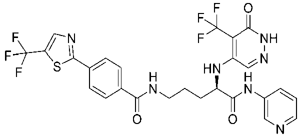 | 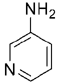 | ¹H NMR (400 MHz, DMSO-*d*₆) δ 12.69 (s, 1H), 11.24 (s, 1H), 9.07 (d, *J =* 2.5 Hz, 1H), 8.73 (t, *J* = 5.7 Hz, 1H), 8.60 (d, *J* = 1.4 Hz, 1H), 8.55 - 8.49 (m, 1H), 8.39 (d, *J =* 8.7 Hz, 1H), 8.13 - 8.07 (m, 2H), 8.01 - 7.95 (m, 2H), 7.79 (d, *J* = 7.2 Hz, 2H), 6.71 (dd, *J =* 8.1, 4.2 Hz, 1H), 4.78 (d, *J* = 6.9 Hz, 1H), 3.34 (t, *J* = 6.6 Hz, 2H), 2.06 - 1.99 (m, 2H), 1.63 (d, *J =* 7.7 Hz, 2H). |
| | | | MS m/z (ESI): 625.8 [M+1] |
| 235 | 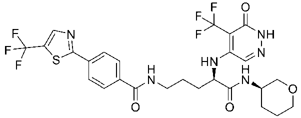 | 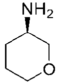 | ¹H NMR (400 MHz, DMSO-*d*₆) δ 12.64 (s, 1H), 8.66 (t, *J =* 4.8 Hz, 1H), 8.60 (d, *J* = 1.4 Hz, 1H), 8.31 - 8.22 (m, 1H), 8.15 - 8.08 (m, 2H), 8.00 - 7.94 (m, 2H), 7.66 (d, *J =* 7.0 Hz, 1H), 6.67 - 6.60 (m, 1H), 4.44 (d, *J* = 7.0 Hz, 1H), 3.73 - 3.60 (m, 3H), 3.35 (s, 2H), 3.28 (d, *J* = 6.5 Hz, 2H), 3.16 (ddd, *J* = 28.7, 10.9, 7.5 Hz, 1H), 1.94 - 1.74 (m, 3H), 1.67 (s, 1H), 1.50 (d, *J* = 8.7 Hz, 4H). |
| | | | MS m/z (ESI): 633.2 [M+1] |
| 236 | 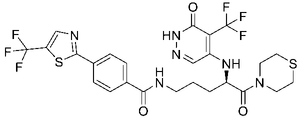 | 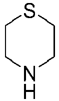 | ¹H NMR (400 MHz, DMSO-*d*₆) δ 12.57 (s, 1H), 8.85 - 8.39 (m, 2H), 8.14 - 8.07 (m, 2H), 8.02 (s, 1H), 7.95 (d, *J* = 8.3 Hz, 2H), 7.02 (t, *J* = 5.9 Hz, 1H), 5.07 (d, *J* = 35.2 Hz, 1H), 3.95 - 3.64 (m, 4H), 2.74 - 2.59 (m, 2H), 2.45 - 2.02 (m, 2H), 1.89 - 1.16 (m, 6H). |
| | | | MS m/z (ESI): 635.2 [M+1] |
| 237 | 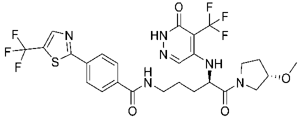 | 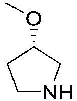 | ¹H NMR (400 MHz, DMSO-*d*₆) δ 12.57 (s, 1H), 8.67 - 8.57 (m, 2H), 8.11 (d, *J* = 8.1 Hz, 2H), 8.00 - 7.92 (m, 3H), 6.93 (d, *J* = 4.8 Hz, 1H), 4.90 (s, 1H), 4.01 - 3.91 (m, 2H), 3.91 - 3.88 (m, 2H),3.27 - 3.21 (m, 5H), 2.54 - 2.50 (m, 1H), 1.90 - 1.79 (m,4H), 1.53 - 1.43 (m, 2H). MS m/z (ESI): 632.9 [M+1] |
| 238 | 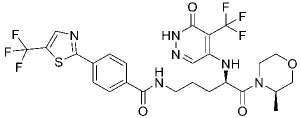 | 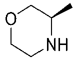 | ¹H NMR (400 MHz, DMSO-*d*₆) δ 12.58 (s, 1H), 8.67 (s, 1H), 8.60 (q, *J* = 1.3 Hz, 1H), 8.14 - 8.03 (m, 2H), 8.02 - 7.91 (m, 3H), 7.04 (d, *J* = 40.6 Hz, 1H), 5.10 (d, *J* = 84.5 Hz, 1H), 4.33 (t, *J* = 35.2 Hz, 1H), 4.17 - 3.71 (m, 2H), 3.63 (d, *J=* 12.0 Hz, 1H), 3.49 - 3.17 (m, 5H), 1.74 (d, *J* = 48.2 Hz, 2H), 1.59 - 1.30 (m, 2H), 1.30 - 1.10 (m, 3H). |
| | | | MS m/z (ESI): 633.2 [M+1] |
| 239 | 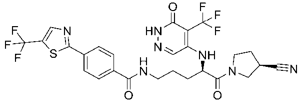 | 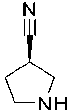 | ¹H NMR (400 MHz, DMSO-*d*₆) δ 12.60 (d, *J* = 3.5 Hz, 1H), 8.66 - 8.57 (m, 2H), 8.10 (dd, *J=* 8.4, 1.8 Hz, 2H), 7.95 (ddd, *J* = 8.5, 4.3, 2.2 Hz, 3H), 6.82 (d, *J* = 6.9 Hz, 1H), 4.90 (s, 1H), 4.17 - 3.85 (m, 1H), 3.77 - 3.47 (m, 4H), 3.30 - 3.23 (m, 2H), 2.38 - 2.00 (m, 2H), 1.80 (s, 2H), 1.62 - 1.38 (m, 2H). |
| | | | MS m/z (ESI): 628.2 [M+1] |
| 240 | 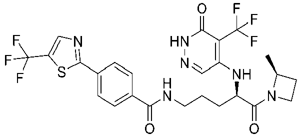 | 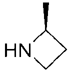 | ¹H NMR (400 MHz, DMSO-*d*₆) δ 12.57 (s, 1H), 8.67 - 8.57 (m, 2H), 8.00 - 7.86 (m, 3H), 4.77 (s, 1H), 3.50 - 3.40 (m, 3H), 2.99 (s, 2H),2.10 - 1.96 (m, 4H), 1.73 (s, 2H), 1.34 - 1.30 (m,3H). |
| | | | MS m/z (ESI): 603.2 [M+1] |
| 241 | 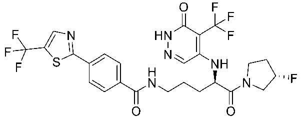 | 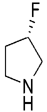 | ¹H NMR (400 MHz, DMSO-*d*₆) δ 12.59 (d, *J* = 5.2 Hz, 1H), 8.64 (dt, *J=* 10.3, 5.4 Hz, 1H), 8.60 (q, *J* = 1.2 Hz, 1H), 8.10 (dq, *J* = 8.6, 1.7 Hz, 2H), 8.02 - 7.93 (m, 3H), 6.88 (d, *J* = 10.0 Hz, 1H), 5.38 (dd, *J* = 53.0, 25.1 Hz, 1H), 4.96 (s, 1H), 4.21 - 3.98 (m, 1H), 3.75 - 3.65 (m, 1H), 3.60 - 3.42 (m, 2H), 3.27 (d, *J* = 5.9 Hz, 2H), 2.28 - 1.96 (m, 2H), 1.90 - 1.70 (m, 2H), 1.62 - 1.37 (m, 2H). |
| | | | MS m/z (ESI): 621.2 [M+1] |
| 242 | 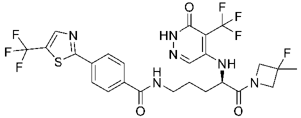 | 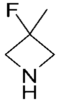 | ¹H NMR (400 MHz, DMSO-*d*₆) δ 12.61 (s, 1H), 8.69 - 8.58 (m, 2H), 8.14 - 8.07 (m, 2H), 7.99 - 7.87 (m, 3H), 6.70 (s, 1H), 4.73 (s, 1H), 4.61 - 4.39 (m, 1H), 4.30 (d, *J* = 20.5 Hz, 1H), 4.09 - 3.90 (m, 2H), 3.28 (s, 2H), 1.81 (s, 2H), 1.58 (d, *J* = 22.1 Hz, 5H). |
| | | | MS m/z (ESI): 621.2 [M+1] |
| 243 | 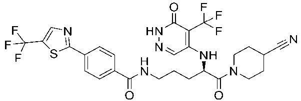 | 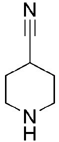 | ¹H NMR (400 MHz, DMSO-*d*₆) δ 12.57 (s, 1H), 8.70 - 8.57 (m, 2H), 8.10 (dd, *J* = 8.5, 1.5 Hz, 2H), 8.02 (d, *J* = 3.6 Hz, 1H), 7.95 (dd, *J* = 10.4, 8.2 Hz, 2H), 7.00 (s, 1H), 5.12 (s, 1H), 3.99 - 3.71 (m, 2H), 3.32 - 3.02 (m, 4H), 2.54 (s, 1H), 1.74 (dd, *J =* 75.1, 24.7 Hz, 6H), 1.44 (d, *J =* 50.4 Hz, 2H). |
| | | | MS m/z (ESI): 642.2 [M+1] |
| 244 | 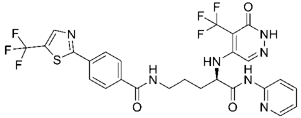 | 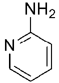 | ¹H NMR (400 MHz, DMSO-*d*₆) δ 12.70 (s, 1H), 10.90 (s, 1H), 8.69 (t, *J* = 5.7 Hz, 1H), 8.60 (d, *J* = 1.3 Hz, 1H), 8.35 (dd, *J* = 5.4, 1.8 Hz, 1H), 8.14 - 8.07 (m, 2H), 8.05 (d, *J* = 8.4 Hz, 1H), 8.00 - 7.94 (m, 2H), 7.83 (td, *J* = 8.4, 7.9, 2.0 Hz, 1H), 7.71 (s, 1H), 7.17 (ddd, *J* = 7.5, 4.9, 1.1 Hz, 1H), 6.71 (dd, *J* = 7.9, 4.1 Hz, 1H), 4.72 (d, *J* = 7.2 Hz, 1H), 3.33 (t, *J* = 6.5 Hz, 2H), 2.02 - 1.94 (m, 2H), 1.68 - 1.51 (m, 2H). |
| | | | MS m/z (ESI): 626.2 [M+1] |
| 246 | 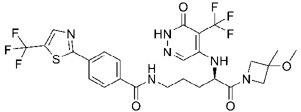 | 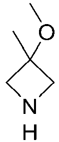 | ¹H NMR (400 MHz, DMSO-*d*₆) δ 12.60 (s, 1H), 8.68 - 8.62 (m, 1H), 8.60 (d, *J* = 1.4 Hz, 1H), 8.11 (dd, *J =* 8.3, 1.5 Hz, 2H), 7.96 (dd, *J =* 8.4, 1.6 Hz, 2H), 7.89 (d, *J* = 10.2 Hz, 1H), 6.72 (s, 1H), 4.73 (d, *J =* 6.4 Hz, 1H), 4.23 (dd, *J* = 50.4, 9.2 Hz, 1H), 4.07 - 3.77 (m, 3H), 3.27 (q, *J* = 6.6 Hz, 2H), 3.15 (s, 3H), 1.78 (t, *J =* 8.7 Hz, 2H), 1.49 (s, 2H), 1.39 (d, *J =* 5.1 Hz, 3H). |
| | | | MS m/z (ESI):633.2 [M+1] |
| 247 | 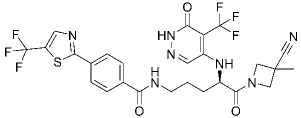 | 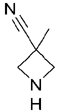 | ¹H NMR (400 MHz, DMSO-*d*₆) δ 12.61 (s, 1H), 8.67 - 8.62 (m, 1H), 8.60 (d, *J =* 1.4 Hz, 1H), 8.11 (d, *J* = 8.3 Hz, 2H), 8.00 - 7.89 (m, 3H), 6.67 (s, 1H), 4.74 (d, *J =* 9.2 Hz, 2H), 4.36 - 4.05 (m, 2H), 3.89 (dd, *J* = 24.4, 9.9 Hz, 1H), 3.28 (d, *J* = 7.1 Hz, 2H), 1.88 - 1.67 (m, 2H), 1.62 (d, *J* = 1.3 Hz, 3H), 1.47 (dt, *J =* 18.0, 5.5 Hz, 2H). |
| | | | MS m/z (ESI):628.2 [M+1] |
| 255 | 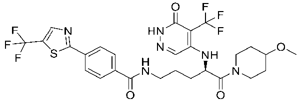 | 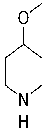 | ¹H NMR (400 MHz, DMSO-d6) δ 12.56 (s, 1H), 8.82 - 8.44 (m, 2H), 8.26 - 7.77 (m, 5H), 7.04 (s, 1H), 5.48 - 5.02 (m, 1H), 3.77 (s, 2H), 3.22 (d, J = 8.4 Hz, 5H),2.73 - 2.43 (m, 3H), 2.00 (q, J = 7.0, 6.5 Hz, 2H), 1.87 - 1.64 (m, 4H), 1.48 (dd, J = 13.5, 6.7 Hz, 2H). |
| | | | MS m/z (ESI):647.2 [M+1] |
| 256 | 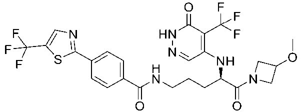 | 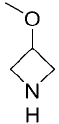 | ¹H NMR (400 MHz, DMSO-*d*₆) δ 12.60 (s, 1H), 8.72 - 8.58 (m, 2H), 8.14 - 8.08 (m, 2H), 7.96 (d, *J =* 8.3 Hz, 2H), 7.89 (d, *J* = 11.7 Hz, 1H), 6.70 (s, 1H), 4.72 (d, *J* = 6.4 Hz, 1H), 4.58 - 3.90 (m, 4H), 3.73 (t, *J =* 14.5 Hz, 1H), 3.27 (s, 2H), 3.20 (d, *J* = 2.5 Hz, 3H), 1.75 (d, *J* = 20.8 Hz, 2H), 1.49 (s, 2H). |
| | | | MS m/z (ESI):619.2 [M+1] |
| 257 | 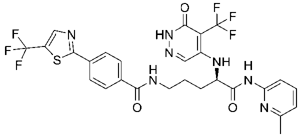 | 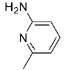 | ¹H NMR (400 MHz, DMSO-*d*₆) δ 12.70 (s, 1H), 10.79 (s, 1H), 8.71 (t, *J* = 5.7 Hz, 1H), 8.60 (d, *J =* 1.4 Hz, 1H), 8.16 - 8.07 (m, 2H), 8.09 - 7.96 (m, 2H), 7.87 (d, *J* = 8.2 Hz, 1H), 7.77 - 7.67 (m, 2H), 7.01 (d, *J* = 7.5 Hz, 1H), 6.71 (dd, *J = 8.2,* 4.1 Hz, 1H), 4.71 (d, *J =* 7.3 Hz, 1H), 3.33 (s, 2H), 2.41 (s, 3H), 1.97 (s, 2H), 1.62 - 1.56 (m, 2H). |
| | | | MS m/z (ESI):640.2 [M+1] |
| 258 | 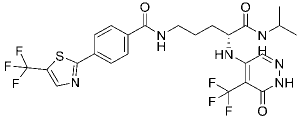 | 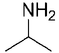 | ¹H NMR (400 MHz, DMSO-*d*₆) δ 12.62 (s, 1H), 8.67 (t, *J =* 5.6 Hz, 1H), 8.60 (q, *J* = 1.3 Hz, 1H), 8.17 - 8.08 (m, 3H), 8.00 - 7.93 (m, 2H), 7.66 (s, 1H), 6.66 (dd, *J =* 7.3, 4.5 Hz, 1H), 4.38 - 4.32 (m, 1H), 3.87 (dq, *J =* 13.6, 6.8 Hz, 1H), 3.28 (q, *J* = 6.6 Hz, 2H), 1.82 (dd, *J* = 10.8, 5.7 Hz, 2H), 1.50 (t, *J* = 8.1 Hz, 2H), 1.07 (dd, *J* = 8.8, 6.6 Hz, 6H). |
| | | | MS m/z (ESI):591.2 [M+1] |
| 259 | 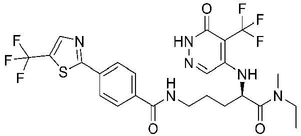 | 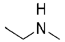 | ¹H NMR (400 MHz, DMSO-*d*₆) δ 12.57 (d, *J* = 2.5 Hz, 1H), 8.65 (t, *J* = 5.7 Hz, 1H), 8.60 (q, *J* = 1.2 Hz, 1H), 8.10 (d, *J =* 8.3 Hz, 2H), 8.04 (d, *J* = 19.3 Hz, 1H), 7.94 (dd, *J =* 8.5, 1.6 Hz, 2H), 7.00 (t, *J* = 5.9 Hz, 1H), 5.06 (s, 1H), 3.51 - 3.19 (m, 4H), 3.05 (s, 2H), 2.85 (s, 1H), 1.78 (dd, *J* = 10.7, 6.0 Hz, 2H), 1.57 - 1.46 (m, 1H), 1.42 (s, 1H), 1.11 (t, *J =* 7.0 Hz, 1H), 1.01 (t, *J =* 7.1 Hz, 2H). |
| | | | MS m/z (ESI):591.2 [M+1] |
| 260 | 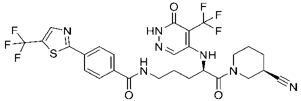 | 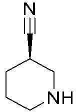 | ¹H NMR (400 MHz, DMSO-*d*₆) δ 12.58 (s, 1H), 8.62 (d, *J =* 6.2 Hz, 1H), 8.60 (s, 1H), 8.12 - 8.09 (m, 2H), 8.03 (d, *J =* 15.1 Hz, 1H), 7.97 - 7.92 (m, 2H), 6.96 (s, 1H), 5.18 (dd, *J* = 4.6, 1.5 Hz, 1H), 4.09 - 3.81 (m, 2H), 3.26 - 3.05 (m, 4H), 2.52 (s, 1H), 1.98 - 1.72 (m, 4H), 1.70 - 1.60 (m, 2H), 1.55 - 1.47 (m, 2H). |
| | | | MS m/z (ESI):642.2 [M+1] |
| 261 | 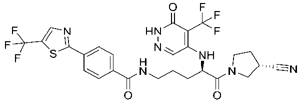 | 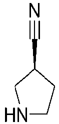 | ¹H NMR (400 MHz, DMSO-*d*₆) δ 12.59 (s, 1H), 8.68 - 8.54 (m, 2H), 8.10 (d, *J =* 7.3 Hz, 2H), 7.99 - 7.95 (m, 2H), 7.95 - 7.92 (m, 1H), 6.82 (d, *J* = 4.2 Hz, 1H), 5.06 - 4.68 (m, 1H), 4.27 - 3.89 (m, 2H), 3.45 (dd, *J =* 13.9, 6.8 Hz, 2H), 3.31 - 3.22 (m, 2H), 2.55 (d, *J =* 3.5 Hz, 1H), 2.25 - 1.99 (m, 2H), 1.91 - 1.69 (m, 2H), 1.62 - 1.40 (m, 2H). |
| | | | MS m/z (ESI):628.2 [M+1] |
| 262 | 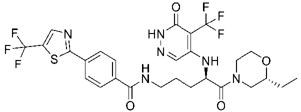 | 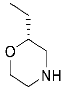 | ¹H NMR (400 MHz, DMSO-*d*₆) δ 12.58 (s, 1H), 8.66 (d, *J =* 28.9 Hz, 1H), 8.60 (d, *J =* 1.2 Hz, 1H), 8.11 (d, *J =* 8.1 Hz, 2H), 8.09 - 7.97 (m, 1H), 8.01 - 7.91 (m, 2H), 7.00 (s, 1H), 5.12 (d, *J* = 33.4 Hz, 1H), 4.16 (s, 1H), 3.97 (d, *J* = 13.3 Hz, 1H), 3.85 (d, *J =* 11.3 Hz, 1H), 3.32 - 3.16 (m, 4H), 3.12 (d, *J =* 24.5 Hz, 1H), 2.79 (dd, *J* = 20.6, 9.8 Hz, 1H), 1.80 (s, 2H), 1.51 (s, 1H), 1.41 (dt, *J =* 13.5, 7.0 Hz, 2H), 1.38 - 1.30 (m, 1H), 0.91 - 0.76 (m, 3H). |
| | | | MS m/z (ESI):647.2 [M+1] |

### Example 245

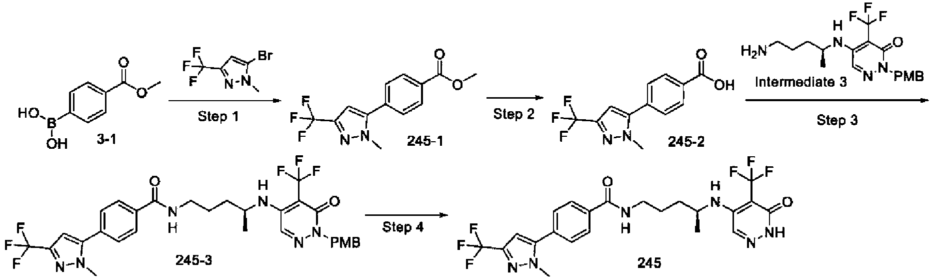

Step 1: Compound 3-1 (900 mg, 5 mmol), 5-bromo-1-methyl-3-(trifluoromethyl)-1H-pyrazole (1145 mg, 5 mmol), DPPFdichloropalladium dichloromethane complex (453.76 mg, 0.56 mmol), water (2 mL), and cesium carbonate (2715.62 mg, 8.33 mmol) were added sequentially to 1,4-dioxane (10 mL). The resulting mixture was purged with nitrogen 3 times, heated to 80°C and reacted under stirring for 3 hours. The reaction solution was directly concentrated to dryness, and purified by silica gel column chromatography (EA/PE=0 to 30%) to afford compound 245-1. MS m/z(ESI): 285.0 [M+1].

Step 2: Compound 245-1 (65 mg, 0.23 mmol), LiOH (0.02 mL, 0.69 mmol), and water (1 mL) were added sequentially to methanol (2 mL), followed by reacting under stirring at 50°C for 2 hours. The reaction solution was adjusted to pH 4-5 and filtered. The filter cake was concentrated to dryness to afford compound 245-2. MS m/z(ESI): 271.0[M+1].

Step 3: Compound 245-2 (55 mg, 0.20 mmol), intermediate 3 (78.24 mg, 0.20 mmol), HATU (116.09 mg, 0.31 mmol), and DIPEA (0.1 mL) were added sequentially to DMF (4 mL), followed by reacting under stirring at room temperature for 2 hours. The resulting mixture was extracted with EA (100 mL), washed with water (50 mL×3) and saturated saline (50 mL), respectively, dried over anhydrous sodium sulfate, and filtered. The filtrate was concentrated to dryness, and purified by silica gel column chromatography (EA/PE=0 to 50%) to afford compound 245-3. MS m/z(ESI): 637.2[M+1].

Step 4: Compound 245-3 (45 mg, 0.07 mmol) was added to TFA (3 mL). Then, TfOH (0.3 mL) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 245. MS m/z(ESI): 517.2[M+1]. ¹H NMR (400 MHz, Methanol-*d*₄) δ 7.95 (d, *J =* 8.6 Hz, 3H), 7.64 (d, *J =* 8.4 Hz, 2H), 6.76 (s, 1H), 4.06 - 3.90 (m, 4H), 3.47 - 3.41 (m, 2H), 1.78 - 1.61 (m, 4H), 1.29 (d, *J =* 6.3 Hz, 3H).

### Example 248

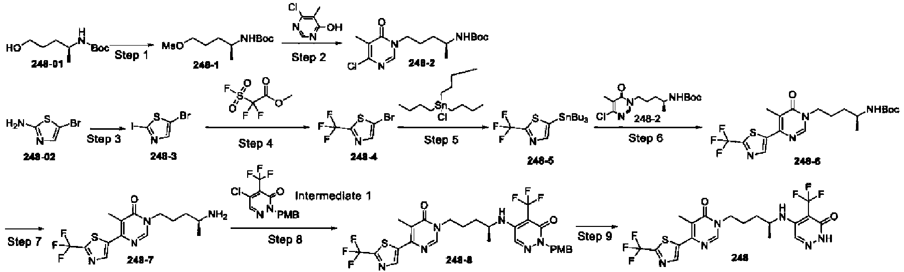

Step 1: Compound 248-01 (4 g, 19.68 mmol), triethylamine (5.96 g, 59.03 mmol), and methylsulfonyl chloride (1.83 mL, 23.61 mmol) were added sequentially to DCM (40 mL), followed by reacting under stirring at room temperature for 1 hour. DCM (100 mL) and water (100 mL) were added to the system. The organic phase is separated and concentrated to dryness to afford compound 248-1.

Step 2: Compound 248-1 (2.21 g, 7.87 mmol) and 4-chloro-5-methyl-6-hydroxylpyrimidine (1.14 g, 7.87 mmol, Bide) were dissolved in DMF (10 mL). Potassium carbonate (1.09 g, 7.87 mmol) was added. The reaction was carried out at 60°C for 18 hours. The reaction solution was added to water (10 mL), followed by extraction with EA (10 mL×3). The organic phases were combined, and washed with saturated saline (10 mL). The organic phases were dried over anhydrous sodium sulfate and filtered. The filtrate was concentrated under reduced pressure. The residue was purified by silica gel column chromatography with an eluant system (PE/EA=100/1-10/1) to afford compound 248-2.

Step 3: Compound 248-02 (5 g, 27.9 mmol) was dissolved in THF (50 mL). Isoamyl nitrite (5.64 mL) and diiodomethane (2.7 mL) were added. The reaction was carried out at room temperature for 2 hours. The reaction solution was concentrated under reduced pressure. The residue was purified by silica gel column chromatography with an eluant system (PE/EA=100/1) to afford compound 248-3. MS m/z(ESI):290.0[M+1].

Step 4: Compound 248-3 (1 g, 3.45 mmol) was dissolved in DMF (5 mL). Then, cuprous iodide (0.99 g, 5.17 mmol) and methyl fluorosulfonyldifluoroacetate (0.66 mL, 5.21 mmol) were added sequentially. The reaction was carried out at 70°C for 18 hours. The reaction solution was added to 10 mL water, followed by extraction with n-hexane (20 mL). The organic phases were combined, and washed with saturated saline. The organic phases were dried over anhydrous sodium sulfate and filtered. The filtrate was concentrated under reduced pressure to afford compound 248-4. MS m/z(ESI):232.0[M+1].

Step 5: Compound 248-4 (500 mg, 2.15 mmol) and tributyltin chloride (0.88 mL, 3.23 mmol) were dissolved in THF (10 mL). At -78°C, n-butyl lithium (207 mg, 3.23 mmol) was added to the reaction solution. The reaction was carried out at -78°C for 1 hour, followed by warming to room temperature and reacting for 18 hours. The reaction solution was added to 50 mL water, followed by extraction with EA (50 mL×3). The organic phases were combined and washed with saturated saline. The organic phases were dried over anhydrous sodium sulfate and filtered. The filtrate was concentrated under reduced pressure. The residue was purified by silica gel column chromatography with an eluant system (PE/EA=100/1) to afford compound 248-5.

Step 6: Compound 248-2 (65 mg, 0.2 mmol) and compound 248-5 (174 mg, 0.39 mmol) were dissolved in toluene (3 mL). Pd(PPh₃)₄ (22.7 mg, 0.02 mmol) was added. The reaction was carried out at 120°C for 18 hours. The reaction solution was concentrated under reduced pressure. The residue was purified by silica gel column chromatography with an eluant system (PE: EA=10:1 to 1/1) to afford compound 248-6. MS m/z(ESI): 347.2 [M-100+1].

Step 7: Compound 248-6 (40 mg, 0.09 mmol) was dissolved in a solution of 4M hydrochloric acid in 1,4-dioxane(1 mL). The reaction was carried out at room temperature for 1 hour. The reaction solution was concentrated under reduced pressure to afford compound 248-7. MS m/z(ESI):347.0[M+1].

Step 8: Compound 248-7 (35 mg, 0.10 mmol) was dissolved in isopropanol (2 mL), and then intermediate 1 (32.2 mg, 0.10 mmol) and DIPEA (0.08 mL, 0.51 mmol) were added sequentially. The reaction was carried out at 60°C for 18 hours. The reaction solution was concentrated under reduced pressure. The residue was purified by preparative silica gel plate (PE: EA=1:1) to afford compound 248-8. MS m/z(ESI): 629.4 [M+1].

Step 9: Compound 248-8 (30 mg, 0.05 mmol) was dissolved in TFA (1.00 mL). TfOH (0.2 mL) was added. The reaction was carried out at room temperature for 1 hour. The reaction solution was concentrated under reduced pressure, and the reaction solution was adjusted pH=8 with saturated sodium bicarbonate solution. Then, the mixed solution was concentrated under reduced pressure to afford the crude. The crude was purified by preparative silica gel plate (PE/EA=1:1) to afford compound 248. MS m/z(ESI): 509.1 [M+1]. 1H NMR (400 MHz, DMSO-d6) δ 12.44 (s, 1H), 8.57 (s, 1H), 8.47 (s, 1H), 7.90 (s, 1H), 6.39 - 6.30 (m, 1H), 4.03 - 3.87 (m, 3H), 2.31 (s, 3H), 1.79 - 1.58 (m,3H), 1.57 - 1.42 (m, 1H), 1.16 (d, J = 6.3 Hz, 3H).

### Example 249

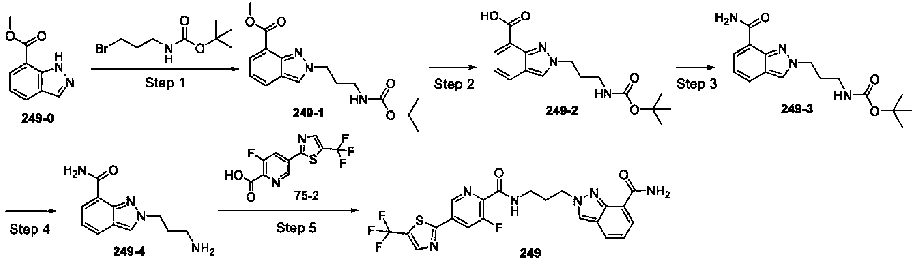

Step 1: Compound 249-0 (1 g, 5.68 mmol) was dissolved in DMF (10 mL). At 0°C, NaH (0.34 g, 8.52 mmol) was added. After stirring for 30 minutes, N-Boc-3-aminopropyl bromide (1.62 g, 6.82 mmol) was added, followed by warming to room temperature and reacting for 18 hours. The reaction solution was added to 100 mL water, followed by extraction with EA (100 mL×3). The organic phases were combined, washed with saturated saline, dried over anhydrous sodium sulfate, and filtered. The filtrate was concentrated under reduced pressure. The residue was purified by silica gel column chromatography with an eluant system (PE: EA=10:1-1:1) to afford compound 249-1. MS m/z(ESI):334.2[M+1].

Step 2: Compound 249-1 (360 mg, 1.08 mmol) was dissolved in THF (3 mL), methanol (1 mL), and water (1 mL). LiOH (135 mg, 3.24 mmol) was added. The reaction was carried out at room temperature for 1 hour. The reaction mixture was adjusted to pH = 6 with HCl (1 M). The mixture was poured into water (50 mL), followed by extraction with EA (50 mL×3). The combined organic phase was washed with brine (50 mL), dried over anhydrous sodium sulfate, filtered, and concentrated to dryness under reduced pressure, to afford compound 249-2. MS m/z(ESI):320.2 [M+1].

Step 3: Compound 249-2 (360 mg, 1.13 mmol) was dissolved in DMF (5 mL). Then, TCFH (857 mg, 2.25 mmol) and DIPEA (0.56 mL, 3.38 mmol) were added sequentially. After stirring at room temperature for 1 minute, ammonium chloride (60.4 mg, 1.13 mmol) was added. The reaction was carried out at room temperature for 1 hour. The reaction solution was added to 50 mL water, followed by extraction with EA (50 mL×3). The organic phases were combined, washed with saturated saline, dried over anhydrous sodium sulfate, and filtered. The filtrate was concentrated under reduced pressure. The residue was purified by silica gel column chromatography with an eluant system (PE: EA=100:1-10:1) to afford compound 249-3. MS m/z (ESI): 319.2 [M+1].

Step 4: Compound 249-3 (100 mg, 0.31 mmol) was dissolved in a solution of 4M hydrochloric acid in 1,4-dioxane (2 mL). The reaction was carried out at room temperature for 1 hour. The reaction solution was concentrated under reduced pressure to afford compound 249-4. MS m/z(ESI): 219.2 [M+1].

Step 5: Compound 75-2 (50 mg, 0.17 mmol) was dissolved in DMF (1 mL). Then, TCFH (130 mg, 0.34 mmol) and DIPEA (0.14 mL, 0.86 mmol) were added sequentially. After stirring at room temperature for 1 minute, compound 249-4 (44.8 mg, 0.21 mmol) was added. The reaction was carried out at room temperature for 1 hour. The reaction solution was added to 20 mL water, followed by extraction with EA (30 mL×3). The organic phases were combined, washed with saturated saline, dried over anhydrous sodium sulfate, and filtered. The filtrate was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 249. MS m/z(ESI): 493.0 [M+1]. ¹H NMR (400 MHz, DMSO-*d*₆) δ 9.04 (s, 1H), 8.91 (t, J = 6.0 Hz, 1H), 8.72 (s, 1H), 8.66 (s, 1H), 8.56 (s, 1H), 8.50 - 8.43 (m, 1H), 7.99 - 7.91 (m, 2H), 7.81 (s, 1H), 7.21 - 7.13 (m, 1H), 4.59 (t, J = 6.8 Hz, 2H), 3.40 - 3.34 (m, 2H), 2.32 - 2.20 (m, 2H).

### Example 250

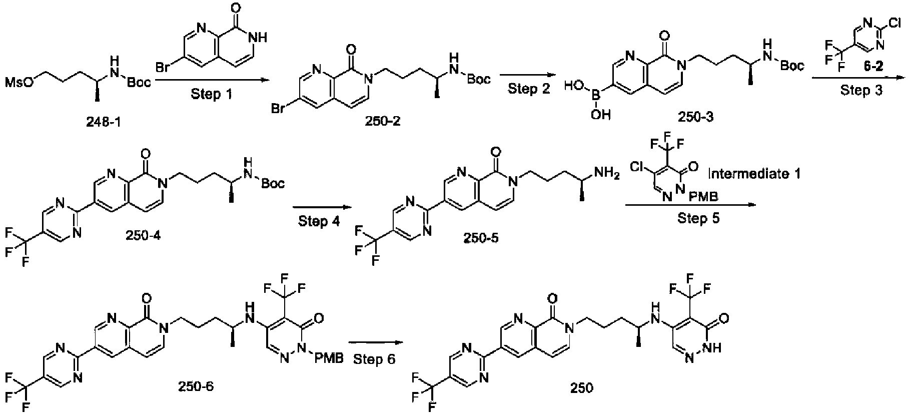

Step 1: Compound 248-1 (1 g, 3.55 mmol), 3-bromo-7,8-dihydropyrido[3,4-b]pyridin-8-one (0.8 g, 3.55 mmol), and cesium carbonate (1.16 g, 3.55 mmol) were added sequentially to acetonitrile (20 mL), followed by reacting under stirring at 80°C for 16 hours. EA(100 mL) and water (100 mL) were added to the reaction solution. The organic phase is separated and concentrated to dryness to afford compound 250-2. MS m/z(ESI): 410.2[M+1].

Step 2: Compound 250-2 (800 mg, 1.94 mmol), potassium acetate (191.35 mg, 1.95 mmol), and Pd(PPh₃)₄ (112.66 mg, 0.10 mmol) were added sequentially to 1,4-dioxane (15 mL). The resulting mixture was purged with nitrogen 3 times, heated to 90°C, and reacted under stirring for 16 hours. EA (100 mL) and water (100 mL) were added to the reaction solution. The organic phase was separated and concentrated to dryness. The residue was purified by silica gel column chromatography (EA/PE=0 to 50%) to afford compound 250-3. MS m/z(ESI):376.2 [M+1].

Step 3: Compound 250-3 (780 mg, 1.7 mmol), compound 6-2 (310 mg, 1.7 mmol), DPPFdichloropalladium dichloromethane complex (453.76 mg, 0.56 mmol), water (2 mL), and cesium carbonate (2715.62 mg, 8.33 mmol) were added sequentially to 1,4-dioxane (10 mL). The resulting mixture was purged with nitrogen 3 times, heated to 80°C and reacted under stirring for 3 hours. The reaction solution was directly concentrated to dryness, and purified by silica gel column chromatography (EA/PE=0 to 30%) to afford compound 250-4. MS m/z(ESI): 478.2[M+1].

Step 4: Compound 250-4 (162 mg, 0.34 mmol) was added to a solution of 4M hydrochloric acid in EA (1 mL), followed by reacting under stirring at room temperature for 1 hour. The reaction solution was directly concentrated to dryness to afford compound 250-5. MS m/z(ESI):378.2 [M+1].

Step 5: Compound 250-5 (100 mg, 0.26 mmol), intermediate 1 (84.45 mg, 0.26 mmol), and DIPEA (102.55 mg, 0.79 mmol) were added sequentially to acetonitrile (2 mL). The reaction solution was directly concentrated to dryness, and purified by silica gel column chromatography (EA/PE=0 to 40%) to afford compound 250-6. MS m/z(ESI):660.2 [M+1].

Step 6: Compound 250-6 (150 mg, 0.23 mmol) was added to TFA (3mL), and then TfOH (0.3 mL) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 250. MS m/z(ESI): 540.2[M+1]. ¹H NMR (400 MHz, Methanol-*d*₄) δ 9.65 (s, 1H), 9.15 (s, 2H), 9.04 (s, 1H), 7.74 (s, 1H), 7.43 (d, *J =* 7.3 Hz, 1H), 6.70 (d, *J =* 7.1 Hz, 1H), 4.01 (t, *J =* 7.0 Hz, 2H), 3.92 - 3.68 (m, 1H), 1.93 - 1.69 (m, 2H), 1.67 - 1.44 (m, 2H), 1.12 (d, *J =* 6.3 Hz, 3H).

### Example 251

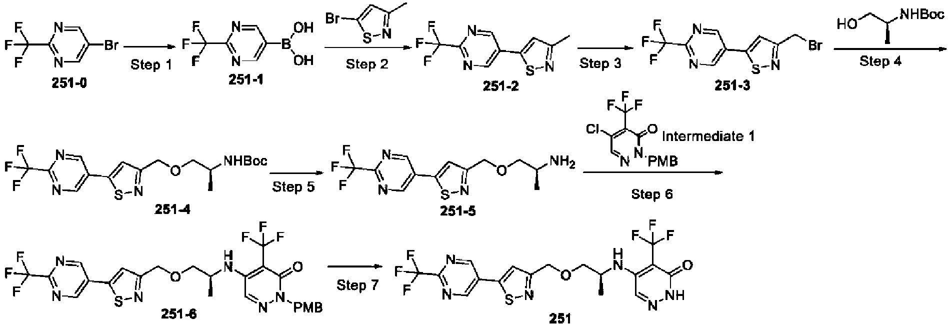

Step 1: Compound 251-0 (1 g, 4.41 mmol) was dissolved in 1,4-dioxane (10 mL). Bis(pinacolato)diboron (2238.08 mg, 8.81 mmol) and DPPFdichloropalladium (161.03 mg, 0.22 mmol), potassium acetate(864.75 mg, 8.81 mmol) were added. The resulting mixture was stirred at 90°C for 16 hours under nitrogen. The reaction solution was concentrated under reduced pressure. The residue was added to 10 mL water, followed by extraction with EA (15 mL×3). The organic phases were combined and washed with saturated saline. The organic phases were dried over anhydrous sodium sulfate and filtered. The filtrate was concentrated under reduced pressure. The residue was purified by silica gel column chromatography with an eluant system (EA/PE=1/100-5/1) to afford compound 251-1. MS m/z(ESI): 193.0 [M+1].

Step 2: Compound 251-1 (670 mg, 3.49 mmol) was dissolved in 1,4-dioxane : water=4:1 (10 mL) solution. 5-bromo-3-methyl-1,2-thiazole (745.97 mg, 4.19 mmol), DPPFdichloropalladium (255.57 mg, 0.35 mmol), potassium carbonate (965.02 mg, 6.98 mmol) were added. The resulting mixture was stirred at 100°C for 2 hours under a nitrogen atmosphere. The reaction solution was concentrated under reduced pressure. The residue was added to 10 mL water, followed by extraction with EA (15 mL×3). The organic phases were combined, and washed with saturated saline. The organic phases were dried over anhydrous sodium sulfate and filtered. The filtrate was concentrated under reduced pressure. The residue was purified by silica gel column chromatography with an eluant system (EA/PE=1/100-1/5) to afford compound 251-2. MS m/z(ESI): 246.0 [M+1].

Step 3: Compound 251-2 (400 mg, 1.63 mmol) was dissolved in carbon tetrachloride (10 mL) solution. N-bromosuccinimide (348.13 mg, 1.96 mmol) and AIBN (53.53 mg, 0.33 mmol) were added. The resulting mixture was stirred at 90°C for 20 hours. The reaction solution was concentrated under reduced pressure. The residue was added to 10 mL water, followed by extraction with EA (15 mL×3). The organic phases were combined, and washed with saturated saline. The organic phases were dried over anhydrous sodium sulfate and filtered. The filtrate was concentrated under reduced pressure. The residue was purified by silica gel chromatography (PE/EA=100/1-3/1) to afford compound 251-3. MS m/z(ESI): 324.0 [M+1].

Step 4: Compound 251-3 (123.26 mg, 0.70 mmol) was dissolved in THF (8 mL) solution. Sodium hydride (46.90 mg, 1.17 mmol, 60%) was added, followed by stirring at 0°C for 40 minutes. Then, tert-butyl (S)-(1-hydroxylpropan-2-yl)carbamate (190 mg, 0.59 mmol) was added dropwise to the mixture, followed by heating to room temperature, and stirring for 2 hours under nitrogen. The reaction solution was concentrated under reduced pressure. The residue was added to 10 mL water, followed by extraction with EA (15 mL×3). The organic phases were combined, and washed with saturated saline. The organic phases were dried over anhydrous sodium sulfate and filtered. The filtrate was concentrated under reduced pressure. The residue was purified by silica gel column chromatography with an eluant system (EA/PE=1/100-1/3) to afford compound 251-4. MS m/z(ESI): 441.2 [M+23].

Step 5: Compound 251-4 (132 mg, 0.32 mmol) was dissolved in a solution of DCM:TFA=3:1 (4 mL), followed by stirring at 25°C for 1 hour. Compound 251-5 was afforded by concentration under reduced pressure. MS m/z(ESI): 319.5 [M+1].

Step 6: Intermediate 1 (148.19 mg, 0.47 mmol) and DIPEA (202.63 mg, 1.57 mmol) were added to a solution of compound 251-5 (100 mg, 0. 31mmol) in isopropanol (5 mL), followed by stirring at 60°C for 4 hours. The mixture was concentrated under reduced pressure to afford compound 251-6, which was used directly in the next step. MS m/z(ESI): 599.4 [M-1].

Step 7: Compound 251-6 (188 mg, 0.31mmol) was dissolved in TFA (2 mL). TfOH (0.14 mL, 1.57 mmol) was added. Then, the resulting mixture was stirred at 25°C for 4 hours. The reaction solution was concentrated under reduced pressure and purified by high performance liquid chromatography (Waters-SunFire-C18-10µm-19×250 mm; mobile phase: water (containing 10 mmol/L formic acid) and acetonitrile, gradient ratio: acetonitrile 38%-95%, flow rate: 25 mL/min) to afford compound 251. MS m/z(ESI): 481.2 [M+1]. ¹H NMR (400 MHz, DMSO) δ 12.44 (s, 1H), 9.43 (s, 2H), 7.97 (d, *J =* 11.2 Hz, 2H), 6.38 (M, 1H), 4.70 (s, 2H), 4.32 - 4.17 (m, 1H), 3.62 (d, *J=* 6.0 Hz, 2H), 1.20 (d, *J =* 6.8 Hz, 3H).

### Example 252

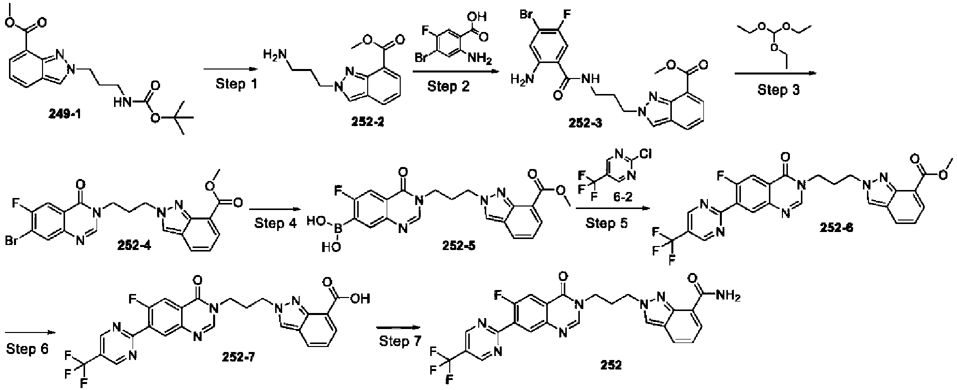

Step 1: Compound 249-1 (2.10 g, 6.30 mmol) was dissolved in a solution of 4M hydrochloric acid in 1,4-dioxane (15 mL). The reaction was carried out at room temperature for 1 hour. The reaction solution was concentrated under reduced pressure to afford compound 252-2. MS m/z(ESI):234.2[M+1].

Step 2: Compound 252-2 (650 mg, 2.78 mmol) was dissolved in DMF (10.0 mL). Then, HATU (2.11 g, 5.56 mmol) and DIPEA (2.30 mL, 13.8 mmol) were added sequentially. After stirring at room temperature for 1 minutes, 2-amino-4-bromo-5-fluorobenzoic acid (647 mg, 2.78 mmol, Bide) was added. The reaction was carried out at room temperature for 1 hour. The reaction solution was added to 100 mL water, followed by extraction with EA (100 mL×3). The organic phases were combined, and washed with saturated saline. The organic phases were dried over anhydrous sodium sulfate and filtered. The filtrate was concentrated under reduced pressure. The residue was purified by silica gel column chromatography with an eluant system (PE/EA=10:1-1:1) to afford compound 252-3. MS m/z(ESI):449.2[M+1].

Step 3: Compound 252-3 (1 g, 2.23 mmol) was dissolved in triethyl orthoformate (20 mL), followed by reacting at 140°C for 2 hours. The reaction solution was concentrated under reduced pressure. The residue was purified by silica gel column chromatography with an eluant system (PE:EA=10:1-1/1) to afford compound 252-4. MS m/z(ESI):459.2 [M+1].

Step 4: Compound 252-4 (560 mg, 1.22 mmol) was dissolved in 1,4-dioxane (10.0 mL). Then, bis(pinacolato)diboron(371 mg, 1.46 mmol), potassium acetate (239 mg, 2.44 mmol), and DPPFdichloropalladium dichloromethane complex (89.2 mg, 0.12 mmol) were added sequentially, and the reaction was carried out at 100°C for 18 hours in a nitrogen environment. The reaction solution was concentrated under reduced pressure. The residue was purified by silica gel column chromatography with an eluant system (PE/EA=10:1-1/1) to afford compound 252-5. MS m/z(ESI):425.2[M+1].

Step 5: Compound 252-5 (150 mg, 0.35 mmol) and compound 6-2 (64.5 mg, 0.35 mmol) were dissolved in 1,4-dioxane (2 mL) and water (0.5 mL). Then, DPPFdichloropalladium dichloromethane complex (25.8 mg, 0.04 mmol) and sodium carbonate (74.9 mg, 0.71 mmol) were added sequentially, and the reaction was carried out at 100°C for 18 hours. The reaction solution was concentrated under reduced pressure. The residue was purified by silica gel column chromatography with an eluant system (PE/EA=10:1-1:1) to afford compound 252-6. MS m/z(ESI): 527.2 [M+1].

Step 6: Compound 252-6 (70 mg, 0.13 mmol) was dissolved in THF (3 mL), methanol (1 mL), and water (1 mL). LiOH (16.7 mg, 0.40 mmol) was added. The reaction was carried out at room temperature for 1 hour. The reaction mixture was adjusted to pH = 6 with HCl (1M). The mixture was poured into water (50 mL) , followed by extraction with EA (50 mL×3). The combined organic phase was washed with brine (50 mL), dried over anhydrous sodium sulfate, filtered, and concentrated to dryness under reduced pressure, to afford compound 252-7. MS m/z(ESI):512.9 [M+1].

Step 7: Compound 252-7 (60.0 mg, 0.12 mmol) was dissolved in DMF (1 mL). Then, TCFH (89 mg, 0.23 mmol) and DIPEA (0.06 mL, 0.35 mmol) were added sequentially. After stirring at room temperature for 1 minutes, ammonium chloride (6.26 mg, 0.12 mmol) was added. The reaction was carried out at room temperature for 1 hour. The reaction solution was added to 20 mL water, followed by extraction with EA (30 mL×3). The organic phases were combined, washed with saturated saline, dried over anhydrous sodium sulfate, and filtered. The filtrate was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 30%-40%, flow rate: 30 mL/min) to afford compound 252. MS m/z(ESI):512.1 [M+1]. ¹H NMR (400 MHz, DMSO-d6) δ 9.49 (s, 2H), 8.62 (s, 1H), 8.52 - 8.48 (m, 1H), 8.40 (s, 1H), 8.25 (d, J = 6.8 Hz, 1H), 7.96 - 7.91 (m, 2H), 7.90 - 7.88 (m, 1H), 7.81 - 7.76 (m, 1H), 7.16 - 7.10 (m, 1H), 4.64 (t, J = 6.8 Hz, 2H), 4.11 (t, J = 6.8 Hz, 2H), 2.55 - 2.51 (m, 2H).

### Example 253

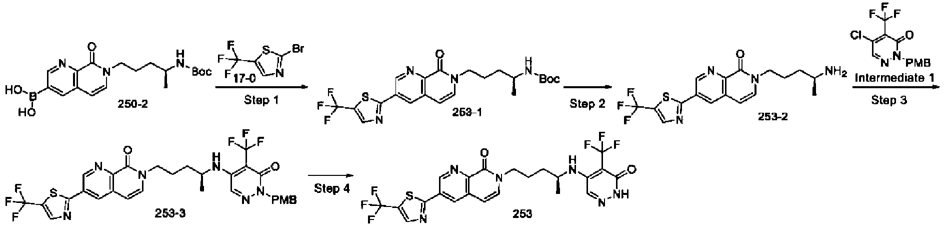

Step 1: Compound 250-2 (400 mg, 0.87 mmol), compound 17-0 (223.20 mg, 0.96 mmol), Pd(PPh₃)₄ (50.53 mg, 0.04 mmol), potassium carbonate (362.59 mg, 2.62 mmol), and water (3 mL) were added sequentially to 1,4-dioxane (10 mL), followed by stirring at 80°C for 8 hours.EA (100 mL) and water (100 mL) were added to the reaction solution. The organic phase is separated and concentrated to dryness. The residue was purified by silica gel column chromatography (EA/PE=0 to 50%) to afford compound 253-1. MS m/z(ESI):483.2 [M+1].

Step 2: Compound 253-1 (330 mg, 0.68 mmol) was added to a solution of 4M hydrochloric acid in 1,4-dioxane (3 mL), followed by reacting under stirring at room temperature for 2 hours. The reaction solution was directly concentrated to dryness to afford compound 253-2. MS m/z(ESI): 383.2 [M+1].

Step 3: Compound 253-2 (280 mg, 0.73 mmol), intermediate 1 (280 mg, 0.88 mmol), DIPEA (283.91 mg, 2.2 mmol) were added sequentially to acetonitrile (5 mL), followed by reacting at 90°C for 2 hours in microwave. EA (300 mL) was added for dilution, followed by washing with water (100 mL×3) and saturated saline (100 mL), respectively, drying over anhydrous sodium sulfate, and filtering. The filtrate was concentrated to dryness, and purified by silica gel column chromatography (EA/PE=0 to 50%) to afford compound 253-3. MS m/z(ESI):665.2 [M+1].

Step 4: Compound 253-3 (400 mg, 0.6 mmol) was added to TFA (3 mL). Then, TfOH (0.3 mL) was added, followed by reacting under stirring at room temperature for 2 hours. The reaction solution was concentrated under reduced pressure and purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 253. MS m/z(ESI):545.2 [M+1]. ¹H NMR (400 MHz, CDCl₃-*d*₄) δ 10.73 (s, 1H), 9.47 (s, 1H), 8.50 (s, 1H), 8.35 (s, 1H), 7.74 (s, 1H), 7.43 (d, *J* = 7.3 Hz, 1H), 6.70 (d, *J* = 7.1 Hz, 1H),5.40 (s, 1H),4.01 (t, *J =* 7.0 Hz, 2H), 3.92 - 3.68 (m, 1H), 1.93 - 1.69 (m, 2H), 1.67 - 1.44 (m, 2H), 1.12 (d, *J =* 6.3 Hz, 3H).

### Example 254

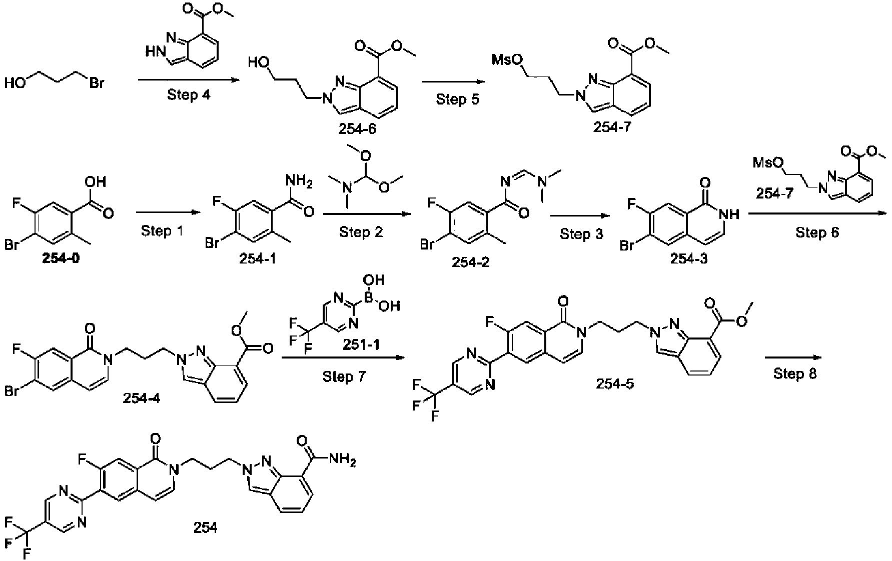

Step 1: Compound 254-0 (10 g, 42.9 mmol) was added to THF (100 mL). N,N-carbonyldiimidazole (6.95g, 42.9 mmol) was added in batches, followed by stirring at room temperature for two hours. Ammonia water was added. The reaction was carried out overnight at room temperature. EA (100 mL) was added to the reaction solution, followed by washing with water (100 mL×3). The organic phase is separated and concentrated to dryness to afford compound 254-1. MS m/z(ESI): 232.0[M+1].

Step 2: Compound 254-1 (9.8 g, 42.2 mmol) and N,N-dimethylformamidedimethyl acetal (19.60 mL, 146.40 mmol) were added to THF (100 mL), followed by heating to 60°C and reacting for 16 hours. The reaction solution was directly dried by spin to afford compound 254-2. MS m/z(ESI):287.0 [M+1].

Step 3: Compound 254-2 (11 g, 38.3 mmol) was added to DMF (100 mL). KTB (4.29 g, 38.3 mmol) was slowly added, followed by reacting at 80°C for 2 hours. EA (500 mL) was added to the reaction solution, followed by washing with water (300 mL×3). The organic phase is separated and concentrated to dryness. The residue was purified by silica gel column chromatography (EA/PE=0 to 100%) to afford compound 254-3. MS m/z(ESI): 242.0[M+1].

Step 4: Methyl 2H-indazole-7-carboxylate (8 g,45.4mmol), cesium carbonate (44.38 g, 136.22 mmol), and 3-bromopropan-1-ol (12.62 g, 90.82 mmol) were added sequentially to DMF (150 mL). The reaction was carried out at 70°C for 16 h. Water (300 mL) and EA (300 mL) were added for extraction, followed by removing water. The organic phases were dried by spin. The residue was purified by silica gel column chromatography (EA/PE=0 to 100%) to afford compound 245-6. MS m/z(ESI): 235.2[M+1].

Step 5: Compound 245-6 (1 g, 4.27 mmol) was added to DCM (15 mL). Triethylamine (2.37 mL, 17.08 mmol) was added. After reducing the temperature to 0°C, methylsulfonyl chloride(0.73 g, 6.4 mmol) was slowly added dropwise, followed by reacting for 1 hour under an ice bath. Water (50 mL) and DCM (100 mL) were added. The organic phase was separated and concentrated to dryness to afford compound 254-7.

Step 6: Compound 254-3 (500 mg, 2.07 mmol), compound 254-7 (1290.39 mg, 4.13 mmol), and cesium carbonate (2019.13 mg, 6.20 mmol) were added to acetonitrile (20 mL). The reaction was carried out at 70°C for 16 hours. The reaction solution was poured into water (10 mL), followed by extraction with EA (20 mL×3), and removing water. The organic phases were dried by spin. The residue was purified by silica gel column chromatography (PE/EA=0 to 100%) to afford compound 254-4. MS m/z(ESI):458.0 [M+1].

Step 7: Compound 254-4 (230 mg, 0.50 mmol), compound 251-1 (192 mg, 1 mmol), DPPFdichloropalladium dichloromethane complex (45.37 mg, 0.05 mmol), water (1 mL), and potassium carbonate (21 mg, 0.15 mmol) were added sequentially to 1,4-dioxane (4 mL). The resulting mixture was purged with nitrogen 3 times, heated to 100°C and reacted under stirring for 3 hours. Water (30 mL) and EA (30 mL) were added. The organic phase was separated and concentrated to dryness. The residue was purified by silica gel column chromatography (EA/PE=0 to 30%) to afford compound 254-5. MS m/z(ESI):526.2 [M+1].

Step 8: Compound 254-5 (100 mg, 0.19 mmol) was added to an ammonia methanol solution (10 mL), followed by heating to 80°C, sealing and reacting for 48 hours. The reaction solution was cooled to room temperature, and concentrated to dryness under reduced pressure under vacuum. The residue was purified by high performance liquid chromatography (Waters-2545, chromatographic column: SharpSil-T C18, 30×150 mm, 5 µm; mobile phase: water (containing 10 mmol/L ammonium bicarbonate) and acetonitrile, gradient ratio: acetonitrile 38%-45%, flow rate: 30 mL/min) to afford compound 254. MS m/z(ESI):511.2[M+1]. ¹H NMR (400 MHz, DMSO-*d*₆) δ 9.38 (d, *J =* 1.4 Hz, 2H), 8.64 (s, 1H), 8.49 (d, *J =* 3.2 Hz, 1H), 8.06 (d, *J =* 7.3 Hz, 1H), 8.00 (d, *J =* 11.0 Hz, 1H), 7.95 - 7.88 (m, 2H), 7.78 (d, *J =* 3.1 Hz, 1H), 7.52 (d, *J* = 7.4 Hz, 1H), 7.16 (dd, *J =* 8.3, 7.0 Hz, 1H), 6.64 (d, *J =* 7.4 Hz, 1H), 4.60 (t, *J =* 6.8 Hz, 2H), 4.12 (t, *J =* 6.9 Hz, 2H), 2.49 - 2.39 (m, 2H).

It should be noted that other compounds in the present application can be prepared by the preparation methods of the above examples (if necessary, appropriate modifications can be made, for example the replacement of a raw material).

### Biological test

### Test example 1: Experiment on the inhibition of the compound on PARP7 protein activity

### 1. Test purpose

In this experiment, the inhibitory effect of a compound on the ADP-ribosylation function of PARP7 was detected. The inhibitory effect of the compound on the PARP7 target was evaluated based on IC₅₀.

### 2. Experimental method

Coating of reaction plates: Histone H1 (Active Motif, 81126) was diluted with PBS (Cell Signaling Technology, 9808S) to 2 µg/mL. 25 µL/well was added to each well of a 384-well reaction plate (Thermo, 460372), followed by incubation at 37°C for 2 h, and then washing 5 times with PBST (Cell Signaling Technology, 9809S) solution. 75 µL of Blocking buffer (Thermo Scientific^{™}, 37535) was added to each well, followed by incubation at room temperature for 1 h, then washing 5 times with PBST. Residual solution was removed by a clean tissue.

Compound formulation: Using an ECHO (Beckman, ECHO550), the test compound was diluted to a 10 mM solution with DMSO. Then, the 10 mM solution was serially diluted in 3-fold gradient to obtain the test compound with 10 concentrations (10 mM, 3.33mM, 1.11 mM, 3.70 µM, 1.23 µM, 41.1 pM, 13.7 pM, 4.57 pM, 1.52 pM, 0.51 pM). 25 nL of the compound was transferred to each well of a 384-well reaction plate for each concentration, with duplicate wells per concentration. Min control wells (reaction systems without protein) and Max control wells (reaction systems without compound) were added with 25 nL DMSO, respectively.

Ribosylation reaction: A 6.25 nM of PARP7 solution was formulated using 1× Assay buffer (BPS, 80602). 10 µL was added to each well of the compound test wells and Max control wells, and 10 µL of 1× Assay buffer (BPS, 80602) was added to each well of the Min control wells, followed by incubation at room temperature for 15 min. 1.67 µM of Biotin-Labeled NAD⁺ (BPS, 80610) solution was formulated using 1× Assay buffer, and 15 µL was added to each well. After centrifugation, the reaction was initiated and incubated at room temperature for 2 h. After the reaction was completed, the reaction plate was washed with a PBST solution for 5 times. Residual solution was removed by a clean tissue.

Detection: Streptavidin-HRP (Abcam, ab7403) was diluted to 0.2 µg/mL with a Blocking Buffer. 25 µL was added to each well, followed by incubation at room temperature for 30 min, and washing 5 times using a PBST solution. 50 µL of a mixed solution of ELISA ECL Substrate A and ELISA ECL Substrate B (Seracare, 5430-0040) was added to each well, and after 5 min, the chemiluminescence value was read using EnSight (Perkin Elmer, EnSight).

### 3. Data analysis

The inhibition ratio of a compound on PARP7 activity was calculated using the following formula:$Inhibition ratio = \left(Signal_max-Signal_sample\right) / \left(Signal_max-Signal_min\right) \times 100 %$ wherein: Signal_sample is the average signal value of the sample well; Signal_min is the average signal value of the Min control well; and Signal_max is the average signal value of the Max control well.

The dose-effect curve was fitted using the log(inhibitor) vs. response -Variable slope of the analysis software GraphPad Prism 5, thereby affording the IC₅₀ value of each compound for PARP7 enzyme activity. Some exemplary results are shown in table 3 below.

**Table 3**

| Compound number | PARP7 activity inhibition IC₅₀ | Compound number | PARP7 activity inhibition IC₅₀ | Compound number | PARP7 activity inhibition IC₅₀ |
|---|---|---|---|---|---|
| 1 | A | 31 | A | 61 | A |
| 2 | A | 32 | A | 62 | D |
| 3 | A | 33 | D | 63 | A |
| 4 | A | 34 | D | 64 | C |
| 5 | A | 35 | C | 65 | D |
| 6 | C | 36 | D | 66 | D |
| 7 | | 37 | E | 67 | A |
| 8 | | 38 | A | 68 | A |
| 9 | A | 39 | A | 69 | A |
| 10 | A | 40 | A | 70 | B |
| 11 | C | 41 | A | 71 | E |
| 12 | B | 42 | A | 72 | A |
| 13 | A | 43 | A | 73 | A |
| 14 | A | 44 | A | 74 | A |
| 15 | B | 45 | B | 75 | A |
| 16 | B | 46 | C | 76 | B |
| 17 | A | 47 | B | 77 | A |
| 18 | A | 48 | C | 78 | B |
| 19 | A | 49 | D | 79 | B |
| 20 | A | 50 | A | 80 | C |
| 21 | A | 51 | A | 81 | D |
| 22 | A | 52 | C | 82 | B |
| 23 | A | 53 | C | 83 | |
| 24 | | 54 | B | 84 | A |
| 25 | A | 55 | A | 85 | A |
| 26 | A | 56 | A | 86 | A |
| 27 | A | 57 | B | 87 | A |
| 28 | D | 58 | B | 88 | A |
| 29 | A | 59 | D | 89 | A |
| 30 | B | 60 | A | 90 | A |
| 151 | B | 188 | A | 230 | B |
| 155 | A | 189 | A | 231 | A |
| 164 | E | 192 | B | 232 | A |
| 166 | B | 205 | A | 233 | A |
| 174 | B | 206 | A | 235 | A |
| 175 | A | 207 | A | 237 | A |
| 176 | A | 209 | A | 238 | A |
| 177 | A | 211 | A | 239 | A |
| 178 | A | 212 | A | 241 | A |
| 179 | A | 221 | A | 242 | B |
| 182 | B | 222 | A | 243 | A |
| 183 | A | 223 | B | 244 | A |
| 184 | A | 224 | A | 245 | C |
| 185 | A | 228 | B | 246 | A |
| 186 | A | 229 | A | 247 | B |

| | | | | | |
|---|---|---|---|---|---|
| Note: A represents IC₅₀<3 nM, B represents 3 nM≤IC₅₀<10 nM, C represents 10 nM≤IC₅₀<30 nM, D represents 30 nM≤IC₅₀<100 nM, and E represents 100 nM≤IC₅₀. | | | | | |

As shown in table 3, at least some compounds of the present invention have a better activity inhibitory effect against PARP7.

### Test example 2: Experiment of inhibition on NCI-H1373 cell proliferation

### 1. Test purpose

The inhibitory effect of the compound on PARP7 was evaluated by testing the inhibitory effect of the compound on NCI-H1373 cell proliferation.

### 2. Experimental method

NCI-H1373 cells (ATCC, CRL-5866) were cultured in a complete medium, namely, a RPMI-1640 medium (Gibco, 22400089) containing 10% fetal bovine serum (Corning, 35-081-CV).

Compound preparation: Using an ECHO (Beckman, ECHO650), the test compound was diluted to a 10 mM solution with DMSO. Then, the 10 mM solution was serially diluted in 3-fold gradient to obtain the test compound with 10 concentrations (10 mM, 3.33mM, 1.11 mM, 3.70 µM, 1.23 µM, 41.1 pM, 13.7 pM, 4.57 pM, 1.52 pM, 0.51 pM). 50 nL of the dilution solution of the compound was transferred to each well of the reaction plate for each concentration, with duplicate wells per concentration. Negative control wells and positive control wells were added with 50 nL DMSO (Sigma, D2650), respectively.

In "Day 0" detection plate, NCI-H1373 cells were seeded at a density of 600 cells/well (50 µL) in a complete medium into the Day 0 test wells of a 384-well detection plate (Grenier, 781091). Then, 25 µL of Cell-Titer-Glo reagent (Promega, G7573) was added to each well, followed by centrifugation at 1000 rpm at room temperature for 1 minute and incubation at room temperature under shaded conditions for 10 min. Chemiluminescence signals were collected using an Envision (Perkin Elmer, EnVision 2105) to obtain the average value of the Day 0 test values.

In "compound detection plate", NCI-H1373 cells were seeded at a density of 600 cells/well (50 µL) in a complete medium into the negative control wells and the compound detection wells of a 384-well detection plate (Grenier, 781091), and 50 µL of cell culture medium was added to the positive control wells, followed by centrifugation at 1000 rpm at room temperature for 1 minute and placing in a cell incubator at 37°C, 5% CO2 for incubation for 6 days. After 6 days, 25 µL of Cell-Titer-Glo reagent (Promega, G7573) was added to each well of the compound detection plate, followed by centrifugation at 1000 rpm at room temperature for 1 minute and incubation at room temperature under shaded conditions for 10 min. Chemiluminescence signals were collected using an Envision (Perkin Elmer, EnVision 2105). The test values for each compound well, negative control well, and positive control well were obtained.

### 3. Data analysis

The proliferation inhibition ratio formula is as follows: 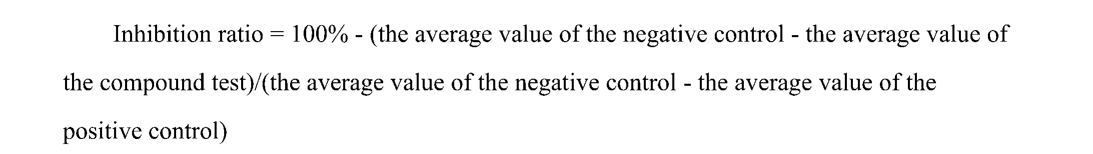 Inhibition ratio = 100% - (the average value of the negative control - the average value of the compound test)/(the average value of the negative control - the average value of the positive control)

The average value of the negative control = the average value of the Day 6 test values of each negative control well minus the average value of the Day 0 test values

The average value of the positive control = the average value of the Day 6 test values of each positive control well

The average value of the compound test = (the Day 6 test value of compound test well 1 + the Day 6 test value of compound test well 2)/2 - the average value of the Day 0 test values

The dose-effect curve was fitted using the log(inhibitor) vs. response -Variable slope of Graphpad Prism8, thereby affording the IC₅₀ valueof each compound for cell activity. Some exemplary results are shown in table 4 below.

**Table 4**

| Compound number | NCI-H1373 cell proliferation inhibition IC₅₀ | Compound number | NCI-H1373 cell proliferation inhibition IC₅₀ | Compound number | NCI-H1373 cell proliferation inhibition IC₅₀ |
|---|---|---|---|---|---|
| 1 | C | 57 | C | 192 | D |
| 2 | B | 58 | D | 205 | B |
| 3 | B | 59 | D | 206 | c |
| 4 | B | 60 | B | 207 | C |
| 5 | D | 61 | A | 209 | A |
| 6 | D | 62 | D | 211 | c |
| 7 | D | 63 | A | 212 | A |
| 8 | D | 64 | D | 220 | A |
| 9 | D | 65 | D | 221 | A |
| 10 | C | 66 | D | 222 | A |
| 13 | D | 67 | B | 223 | A |
| 14 | D | 68 | B | 224 | A |
| 15 | D | 69 | D | 225 | A |
| 16 | D | 70 | D | 226 | |
| 17 | A | 72 | B | 227 | A |
| 18 | B | 73 | B | 228 | A |
| 19 | B | 74 | D | 229 | A |
| 20 | C | 75 | A | 230 | A |
| 21 | C | 76 | D | 231 | A |
| 22 | C | 77 | A | 232 | B |
| 23 | B | 78 | D | 233 | B |
| 24 | D | 79 | D | 234 | B |
| 25 | C | 80 | D | 235 | B |
| 26 | C | 81 | D | 236 | B |
| 27 | D | 82 | D | 237 | B |
| 28 | D | 83 | A | 238 | B |
| 29 | D | 84 | B | 239 | B |
| 30 | D | 85 | B | 240 | D |
| 31 | D | 86 | A | 241 | C |
| 32 | D | 87 | A | 242 | c |
| 34 | D | 88 | B | 243 | D |
| 35 | D | 89 | B | 244 | D |
| 38 | D | 90 | D | 245 | D |
| 39 | B | 151 | D | 246 | C |
| 40 | D | 155 | C | 247 | D |
| 41 | B | 164 | D | 248 | D |
| 42 | D | 166 | D | 249 | D |
| 43 | A | 174 | D | 250 | D |
| 44 | C | 175 | B | 251 | D |
| 45 | D | 176 | A | 252 | D |
| 46 | D | 177 | C | 253 | C |
| 47 | D | 178 | D | 255 | B |
| 48 | D | 179 | D | 256 | B |
| 50 | D | 182 | D | 257 | A |
| 51 | A | 183 | B | 258 | A |
| 52 | D | 184 | B | 259 | A |
| 53 | D | 185 | B | 260 | c |
| 54 | D | 186 | C | 261 | B |
| 55 | C | 188 | D | 262 | A |
| 56 | B | 189 | C | | |

| | | | | | |
|---|---|---|---|---|---|
| Note: A represents <300 nM, B represents 300 nM≤IC₅₀<1000 nM, C represents 1000 nM≤IC₅₀<3000 nM, and D represents 3000 nM≤IC₅₀. | | | | | |

As shown in table 4, at least some compounds of the present invention have a better activity inhibitory effect against the proliferation of NCI-H1373 cells.

### Test example 3: PARP7 TR-FRET binding inhibition experiment

### 1. Test purpose

The ability of the compound to substitute the probe RBN011147 (Wigle et al., 2020, Cell Chemical Biology 27, 877-887) was tested by a TR-FRET method, and IC₅₀ was calculated to evaluate the binding ability of the compound to PARP7.

### 2. Experimental method

### 1, Compound formulation:

Using an ECHO (Beckman, ECHO550), the test compound was diluted to a 10 mM solution with DMSO. Then, the solution was serially diluted in 3-fold gradient to obtain the test compound with 11 concentrations (10 mM, 3.33mM, 1.11 mM, 3.70 µM, 1.23 µM, 41.1 pM, 13.7 pM, 4.57 pM, 1.52 pM, 0.51 pM, 0.17 pM). 200 nL was transferred to each well of the corresponding 384-well reaction plate for each concentration, with duplicate wells per concentration. PC control wells (reaction systems without protein) and NC control wells (reaction systems without compound) were added with 200 nL DMSO, respectively.

### 2. Binding reaction

1×assay buffer (20 mM HEPES pH 8.0, 100 mM NaCl, 0.1% BSA, 2 mM DTT and 0.002% Tween 20) was formulated for diluting the PARP7 protein and the probe RBN011147;
50 nM of PARP7 (BPS, 80527) and 40 nM of Biotin-labeled RBN011147 were formulated by using 1×assay buffer, respectively;
and according to the experimental plate map, 5 µL of 50 nM PARP7 and 5 µL of 40 nM RBN011147 were transfered sequentially to the wells of a 384-well detection plate,
followed by incubation at 25°C for 30 min.

### 3. TR-FRET signal detection

Eu-streptavidin (Cisbio, 610SAKLA) and anti-FLAG M2-XL665 (Cisbio, 61FG2XLB) were diluted in 1:100 using PPI Europium detection buffer (Cisbio, 61DB9RDF);
10 µL of Eu-straptavidin and anti-FLAG M2-XL665 mixture was added to each well, and the final concentrations of Eu-straptavidin and anti-FLAG M2-XL665 in the reaction system were 3 nM and 13.4 nM, respectively, followed by centrifugation at 1000 rpm for 1 min;
and incubation at 25°C for 1 h;
the TR-FRET signals were detected by Envision.

### 3. Data analysis

The inhibition ratio of a compound on PARP7 binding was calculated using the following formula:$Inhibition ratio = \left(Signal_max-Signal_sample\right) / \left(Signal_max-Signal_min\right) \times 100 %$ wherein: Ave_sample is the average signal value of the sample well; Ave.PC is the average signal value of the PC control well; and Ave.NC is the average signal value of the NC control well.

The dose-effect curve was fitted using the log(inhibitor) vs. response -Variable slope of the analysis software GraphPad Prism 5, the IC₅₀ valuewas calculated, and some exemplary results are shown in table 5.

**Table 5**

| Compound number | PARP7 binding inhibition IC₅₀ | Compound number | PARP7 binding inhibition IC₅₀ | Compound number | PARP7 binding inhibition IC₅₀ |
|---|---|---|---|---|---|
| 1 | A | 11 | D | 21 | A |
| 2 | A | 12 | B | 22 | B |
| 3 | A | 13 | C | 23 | B |
| 4 | A | 14 | A | 24 | B |
| 5 | B | 15 | B | 25 | A |
| 6 | C | 16 | B | 26 | A |
| 7 | C | 17 | A | 27 | B |
| 8 | B | 18 | A | 28 | D |
| 9 | A | 19 | A | 29 | B |
| 10 | A | 20 | A | 30 | C |
| 31 | B | 41 | B | 51 | C |
| 32 | B | 42 | C | 52 | C |
| 33 | D | 43 | A | 53 | C |
| 34 | D | 44 | A | 54 | B |
| 35 | C | 45 | B | 55 | A |
| 36 | D | 46 | B | 56 | A |
| 37 | D | 47 | B | 57 | A |
| 38 | A | 48 | C | 58 | A |
| 39 | A | 49 | D | 59 | C |
| 40 | B | 50 | B | 60 | A |
| 61 | A | 71 | D | 81 | C |
| 62 | C | 72 | A | 82 | C |
| 63 | A | 73 | A | 83 | A |
| 64 | D | 74 | A | 84 | B |
| 65 | C | 75 | A | 85 | A |
| 66 | D | 76 | B | 86 | A |
| 67 | A | 77 | A | 87 | B |
| 68 | A | 78 | B | 88 | A |
| 69 | A | 79 | A | 89 | A |
| 70 | A | 80 | C | 90 | C |
| 129 | D | 178 | B | 205 | A |
| 138 | D | 179 | B | 206 | A |
| 151 | C | 182 | C | 207 | B |
| 155 | B | 183 | A | 209 | B |
| 164 | D | 184 | A | 211 | A |
| 166 | D | 185 | C | 212 | A |
| 174 | B | 186 | B | 218 | D |
| 175 | B | 188 | B | 219 | D |
| 176 | A | 189 | C | 220 | A |
| 177 | A | 192 | C | 221 | A |
| 222 | A | 232 | B | 242 | B |
| 223 | A | 233 | A | 243 | A |
| 224 | A | 234 | A | 244 | C |
| 225 | A | 235 | A | 245 | D |
| 226 | D | 236 | B | 246 | A |
| 227 | A | 237 | A | 247 | B |
| 228 | A | 238 | A | 248 | C |
| 229 | B | 239 | B | 249 | C |
| 230 | C | 240 | B | 250 | B |
| 231 | A | 241 | B | 251 | D |
| 252 | D | 256 | A | 260 | B |
| 253 | A | 257 | A | 261 | C |
| 254 | D | 258 | A | 262 | B |
| 255 | A | 259 | A | | |

| | | | | | |
|---|---|---|---|---|---|
| Note: A represents IC₅₀<10 nM, B represents 10 nM≤IC₅₀<30 nM, C represents 30 nM≤IC₅₀<100 nM, and D represents 100 nM≤IC₅₀. | | | | | |

As shown in table 5, at least some compounds of the present invention are able to effectively inhibit the binding of PARP7 to RBN011147.

### Test example 4: Test on the inhibition of the compound on PARP1, PARP2, PARP7 in vitro enzyme activity

### 1. Test purpose

The inhibitory effect of the compound on the ADP-ribosylation function of PARP1, PARP2, and PARP7 was detected by Chemiluminescence assay, thereby evaluating the selectivity of the compound for PARP7.

### 2. Experimental method

Following the method in test example 1, the IC₅₀ value of the inhibition of each compound on PARP7 activity were detected and obtained. Reference was made to the method in test example 1, except that the 6.25 nM of PARP7 solution in the ribosylation reaction step was replaced with a 5 nM of PARP1 solution or a 5 nM of PARP2. Similar to the 6.25 nM of PARP7 solution, the 5 nM of PARP1 solution and 5 nM of PARP2 solution were also formulated and obtained by using 1× Assay buffer.

### 3. Data analysis

The inhibition ratio of a compound on PARP 1/2/7 activity was calculated using the following formula:$Inhibition ratio = \left(Signal_max-Signal_sample\right) / \left(Signal_max-Signal_min\right) \times 100 %$ wherein: Signal_sample is the average signal value of the sample well; Signal_min is the average signal value of the Min control well; and Signal_max is the average signal value of the Max control well. 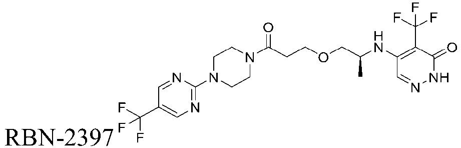 was used as a positive control.

The dose-effect curve was fitted using the log(inhibitor) vs. response -Variable slope of the analysis software GraphPad Prism 5, thereby affording the IC₅₀ value of each compound for PARP1/2/7 enzyme activity. Some exemplary results are shown in table 6.

**Table 6**

| Compound number | PARP1 activity inhibition IC₅₀ | PARP2 activity inhibition IC₅₀ | PARP7 activity inhibition IC₅₀ | PARP1 activity inhibition IC₅₀/ PARP7 activity inhibition IC₅₀ | PARP2 activity inhibition IC₅₀/ PARP7 activity inhibition IC₅₀ |
|---|---|---|---|---|---|
| 4 | D | B | A | ++ | ++ |
| 10 | B | B | A | ++ | + |
| 17 | C | A | A | ++ | + |
| 69 | E | E | A | ++ | ++ |
| 70 | E | E | B | ++ | ++ |
| 84 | E | C | A | ++ | ++ |
| 85 | D | B | A | ++ | + |
| 86 | C | B | A | ++ | + |
| 87 | D | C | A | ++ | ++ |
| 88 | D | B | A | ++ | ++ |
| 89 | D | C | A | ++ | ++ |
| 90 | D | D | A | ++ | ++ |
| 166 | E | E | B | ++ | ++ |
| 174 | D | B | B | ++ | + |
| 221 | E | B | A | ++ | + |
| 222 | C | A | A | ++ | + |
| 223 | B | D | B | ++ | ++ |
| 224 | B | C | A | ++ | ++ |
| 228 | E | B | B | ++ | + |
| 229 | D | C | A | ++ | ++ |
| 230 | D | B | B | ++ | + |
| 231 | C | B | A | ++ | ++ |
| 232 | B | B | A | ++ | + |
| 233 | B | A | A | ++ | + |
| 235 | B | B | A | ++ | ++ |
| 237 | C | B | A | ++ | + |
| 238 | D | C | A | ++ | ++ |
| 239 | C | E | A | ++ | ++ |
| 241 | C | B | A | ++ | + |
| 242 | D | B | B | ++ | + |
| 243 | B | B | A | ++ | + |
| 244 | B | D | A | ++ | ++ |
| 246 | C | B | A | ++ | + |
| 247 | C | D | B | ++ | ++ |

| | | | | | |
|---|---|---|---|---|---|
| Note: A represents IC₅₀<3 nM, B represents 3 nM≤IC₅₀<10 nM, C represents 10 nM≤IC₅₀<30 nM, D represents 30 nM≤IC₅₀<100 nM, and E represents 100 nM≤IC₅₀; ++ represents >3, + represents ≤3. | | | | | |

As shown in table 6, at least some compounds of the present invention have a significant selectivity for PARP7 relative to PARP1 and/or PARP2.

The preferred embodiments of the present invention have been described in detail above. However, the present invention is not limited to the specific details in the above embodiments. Within the scope of the technical concept of the present invention, various simple modifications can be made to the technical solution of the present invention, and these simple modifications all fall within the protection scope of the present invention.

It should be further noted that the various specific technical features described in the above detailed description can be combined in any suitable manner without contradiction. To avoid unnecessary repetition, the present invention will not further describe all possible combinations.

In addition, various different embodiments of the present invention may be arbitrarily combined with each other, as long as such combinations do not depart from the spirit of the present invention, and they shall also be regarded as the content disclosed in the present invention.

## Claims

1. A compound of formula I, or an enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof: 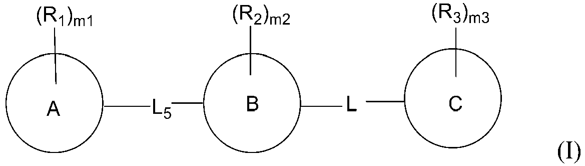 wherein,
ring A is selected from C6-10 aryl, 5-10-membered heteroaryl, 4-10-membered heterocycloalkyl;
ring B is selected from C6-10 aryl, 5-10-membered heteroaryl, 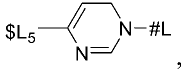 5-6-membered heterocycloalkyl-fused-5-6-membered heterocycloalkyl, C6-10 aryl-fused-5-10-membered heteroaryl, C6-10 aryl-fused-5-6-membered heterocycloalkyl, C6-10 aryl-fused-5-6-membered cycloalkyl, 5-6-membered heteroaryl-fused-5-6-membered heterocycloalkyl, 5-6-membered heteroaryl-fused-5-6-membered cycloalkyl; $L₅ represents the site of attachment to L₅, #L represents the site of attachment to L;
ring C is selected from: 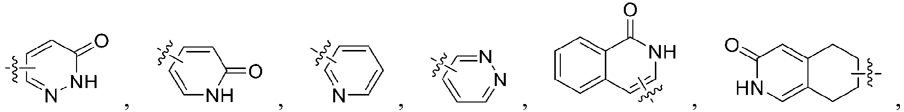 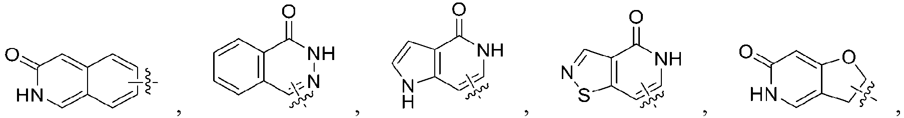 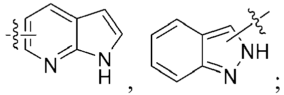 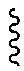 terminus is attached to L;
L is a linking group between ring B and ring C, and the number of main chain atoms of the linking group is between 2-7, preferably between 4-7;
L₅ is selected from a bond, $B-(CH₂)s-O-#A, $B-O-(CH₂)s-#A, $B-(CH₂)s-NRd-#A, $B-NRd-(CH₂)s-#A, wherein, s is 0, 1, or 2, the $B-(CH₂)s-O-#A, $B-O-(CH₂)s-#A, $B-(CH₂)s-NRd-#A, $B-NRd-(CH₂)s-#A are optionally substituted by one or more substituents selected from halogen, hydroxyl, C1-6 alkyl, C1-6 alkoxy, C1-6 haloalkyl, C1-6 haloalkoxy, $B represents the site of attachment to ring B, #A represents the site of attachment to ring A;
preferably, L₅ is selected from a bond, $B-(CH₂)s-NRd-#A, wherein, the $B-(CH₂)s-NRd-#A is optionally substituted by one or more substituents selected from halogen, hydroxyl, C1-3 alkyl, C1-3 alkoxy, C1-3 haloalkyl, C1-3 haloalkoxy;
further preferably, L₅ is selected from a bond, $B-(CH₂)s-NRd-#A, wherein, the $B-(CH₂)s-NRd-#A is optionally substituted by one or more C1-3 alkyl;
more preferably, L₅ is selected from a bond;
each R₁, R₂, and R₃ is independently selected from H, D, halogen, hydroxyl, -CN, -NReRf, -NReC(=O)Rf, -C(=O)-NReRf, C1-6 alkyl, C2-6 alkenyl, C2-6 alkynyl, C1-6 alkoxy, C1-6 haloalkyl, C1-6 haloalkoxy, C3-6 cycloalkyl, -C1-6 alkylene-C3-6 cycloalkyl, 5-6-membered heteroaryl, -C1-6 alkylene-5-6-membered heteroaryl, 4-7-membered heterocycloalkyl, -C1-6 alkylene-4-7-membered heterocycloalkyl, wherein, the C3-6 cycloalkyl, -C1-6 alkylene-C3-6 cycloalkyl, 5-6-membered heteroaryl, -C1-6 alkylene-5-6-membered heteroaryl, 4-7-membered heterocycloalkyl, -C1-6 alkylene-4-7-membered heterocycloalkyl are optionally substituted by 1 or more substituents selected from halogen, hydroxyl, C1-6 alkyl, C1-6 alkoxy, C1-6 haloalkyl, C1-6 haloalkoxy; optionally, two R₁ or R₂ attached to the same carbon atom form C=O with that carbon atom;
Rd is selected from H, C1-6 alkyl, C1-6 haloalkyl;
Re and Rf are each independently selected from H, C1-6 alkyl, C1-6 haloalkyl, C3-6 cycloalkyl, 4-7-membered heterocycloalkyl, or Re and Rf together with the atoms that they are attached to, form 4-7-membered heterocycloalkyl, and the 4-7-membered heterocycloalkyl is optionally substituted by 1 or more substituents selected from halogen, hydroxyl, cyano, C1-6 alkyl, C1-6 haloalkyl, C1-6 alkoxy, C1-6 haloalkoxy;
m1, m2, and m3 each independently represent an integer of 0-4.

2. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 1, wherein:
ring A is selected from 5-6-membered heteroaryl, 6-10-membered heterocycloalkyl;
preferably, ring A is selected from 5-6-membered heteroaryl, 6-membered heterocycloalkyl, 7-10-membered spiro heterocycloalkyl;
further preferably, ring A is selected from 5-6-membered heteroaryl;
further preferably, ring A is selected from pyrimidinyl, pyridinyl, 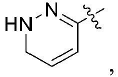 piperazinyl, morpholinyl, 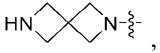 thiazolyl, isothiazolyl, imidazolyl, pyrazolyl, triazolyl (for example 1,2,4-triazolyl, 1,3,4-triazolyl), oxazolyl, isoxazolyl, 1,2,4-oxadiazole, 1,3,4-thiadiazole, 1,2,4-thiadiazole; 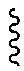 terminus is attached to L₅;
further preferably, ring A is selected from pyrimidinyl, thiazolyl, isothiazolyl, pyrazolyl, pyridinyl; further preferably, ring A is selected from pyrimidinyl (for example 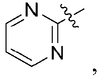 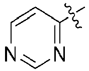 ), pyridinyl (for example 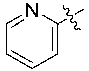 ), 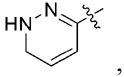 piperazinyl (for example 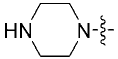 ), morpholinyl (for example 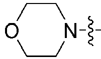 ), 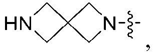 thiazolyl (for example 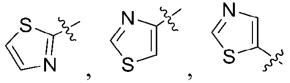 ), isothiazolyl (for example 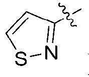 ), imidazolyl (for example 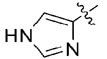 ), pyrazolyl (for example 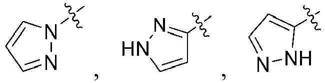 ), 1,2,4-triazolyl (for example 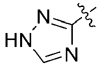 ), 1,3,4-triazolyl (for example 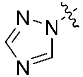 ), oxazolyl (for example 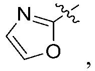 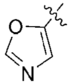 ), isoxazolyl (for example 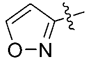 ), 1,2,4-oxadiazole (for example 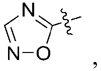 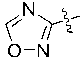 ), 1,3,4-thiadiazole ( 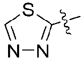 ), 1,2,4-thiadiazole (for example 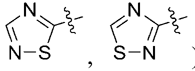 ); terminus is attached to L₅;
more preferably, ring A is selected from 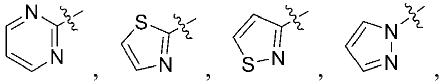 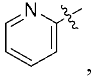 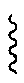 terminus is attached to L₅.

3. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 1 or 2, wherein:
each R₁ is independently selected from halogen, hydroxyl, -CN, C1-6 alkyl, C2-6 alkynyl, C1-6 alkoxy, C1-6 haloalkyl, C3-6 cycloalkyl, -C1-6 alkylene-C3-6 cycloalkyl, 4-7-membered heterocycloalkyl, -C1-6 alkylene-4-7-membered heterocycloalkyl, 5-6-membered heteroaryl, - NReRf, -C(=O)-NReRf, wherein, the C3-6 cycloalkyl, -C1-6 alkylene-C3-6 cycloalkyl, 4-7-membered heterocycloalkyl, -C1-6 alkylene-4-7-membered heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-6 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 substituents selected from halogen, C1-3 alkyl;
preferably, each R₁ is independently selected from halogen, hydroxyl, -CN, C1-6 alkyl, C2-6 alkynyl, C1-6 alkoxy, C1-6 haloalkyl, C1-6 haloalkoxy, C3-6 cycloalkyl, -C1-6 alkylene-C3-6 cycloalkyl, 4-7-membered heterocycloalkyl, 5-6-membered heteroaryl, -NReRf; wherein, the C3-6 cycloalkyl, -C1-6 alkylene-C3-6 cycloalkyl, 4-7-membered heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-6 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 substituents selected from halogen, C1-3 alkyl;
preferably, each R₁ is independently selected from halogen, hydroxyl, -CN, C1-3 alkyl, C2-6 alkynyl, C1-3 alkoxy, C1-3 haloalkyl, C3-6 cycloalkyl, -C1-3 alkylene-C3-6 cycloalkyl, 4-7-membered heterocycloalkyl, 5-6-membered heteroaryl, -NReRf; wherein, the C3-6 cycloalkyl, -C1-3 alkylene-C3-6 cycloalkyl, 4-7-membered heterocycloalkyl, 5-6-membered heteroaryl are optionally substituted by 1 to 3 substituents selected from halogen, C1-3 alkyl; optionally, two R₁ attached to the same carbon atom form C=O with that carbon atom; Re and Rf are each independently selected from H, C1-3 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 halogens;
preferably, each R₁ is independently selected from halogen, hydroxyl, -CN, C1-3 alkyl, C2-6 alkynyl, C1-3 alkoxy, C1-3 haloalkyl, C3-6 cycloalkyl, -C1-3 alkylene-C3-6 cycloalkyl, 4-7-membered heterocycloalkyl, -C1-3 alkylene-4-7-membered heterocycloalkyl, 5-6-membered heteroaryl;
preferably, each R₁ is independently selected from F, Cl, -CN, methyl, -CH₂CH₂CH₃, 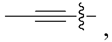 -CHF₂, -CF₃, methoxy, ethoxy, cyclopropyl, 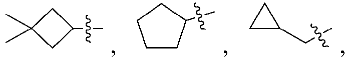 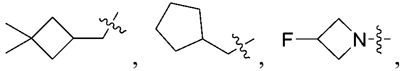 piperidinyl (for example 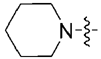 ), morpholinyl (for example 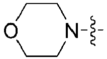 ), 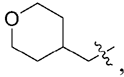 oxazolyl (for example 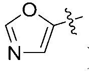 ), pyridinyl (for example 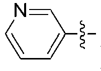 ), -N(CH₃)₂, 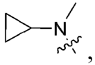 optionally, two R₁ attached to the same carbon atom form C=O with that carbon atom; 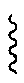 terminus is attached to ring A;
preferably, each R₁ is independently selected from halogen, -CN, C1-3 alkyl, C1-3 alkoxy, C1-3 haloalkyl, C3-6 cycloalkyl, -C1-3 alkylene-C3-6 cycloalkyl, pyridinyl (for example 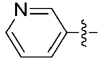 ), -NReRf; Re and Rf are each independently selected from C1-3 alkyl, C3-6 cycloalkyl, or Re and Rf together with the atoms that they are attached to, form 4-7-membered heterocycloalkyl, and the 4-7-membered heterocycloalkyl is optionally substituted by 1 to 3 substituents selected from halogen, C1-3 alkyl; 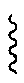 terminus is attached to ring A;
preferably, R₁ is selected from F, -CN, methyl, -CHF₂, -CF₃, methoxy, cyclopropyl, 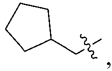 pyridinyl (for example 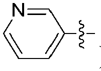 ), -N(CH₃)₂, 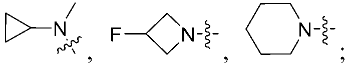 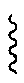 terminus is attached to ring A;
more preferably, R₁ is selected from -CF₃, 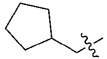 , -N(CH₃)₂; 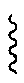 terminus is attached to ring A;
preferably, m1 is 0, 1, or 2;
more preferably, m1 is 0 or 1;
most preferably, m1 is 1.

4. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 3, wherein:
the 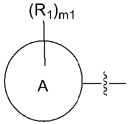 is selected from 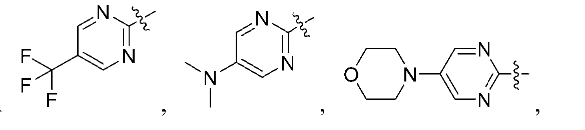 , 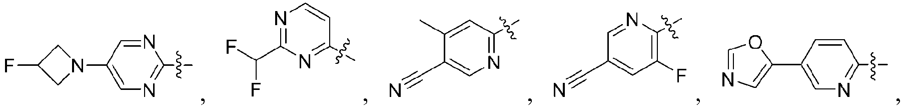 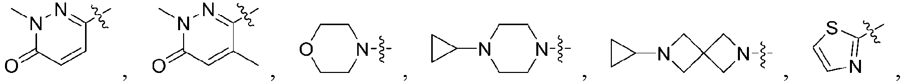 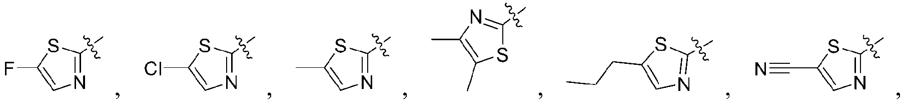 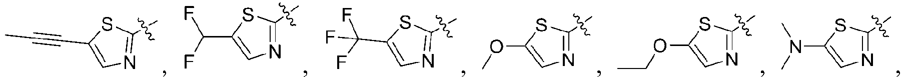 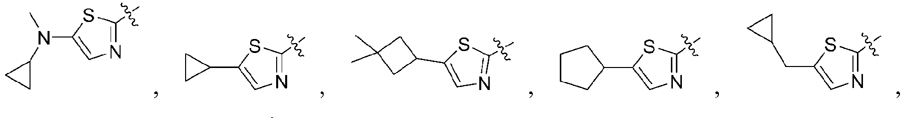 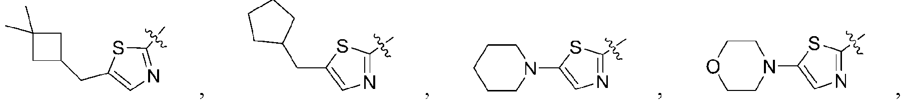 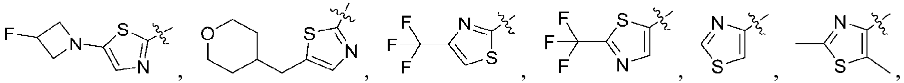 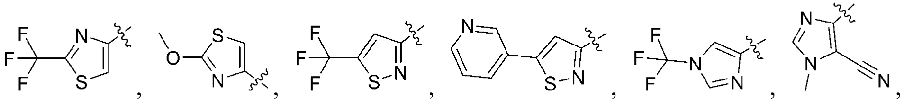 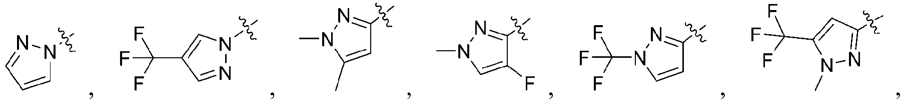 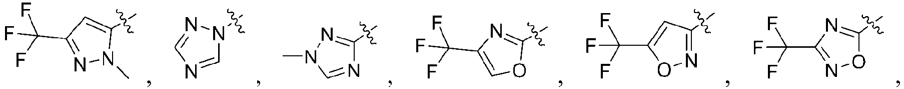 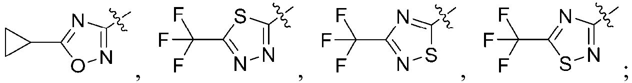 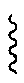 terminus is attached to L₅;
preferably, 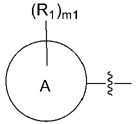 is selected from 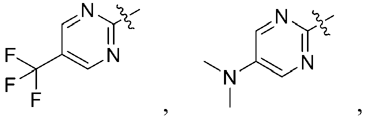 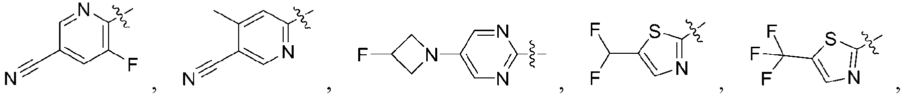 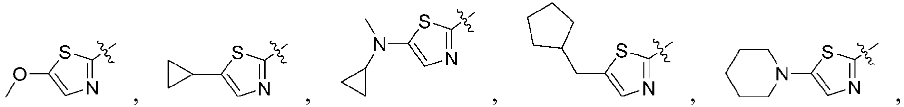 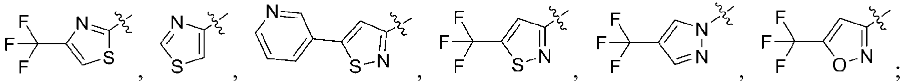 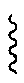 terminus is attached to L₅;
more preferably, 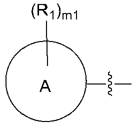 is selected from 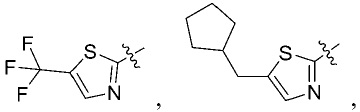 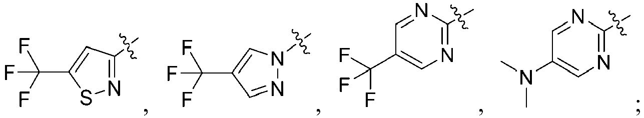 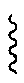 terminus is attached to L₅;
more preferably, 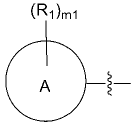 is selected from 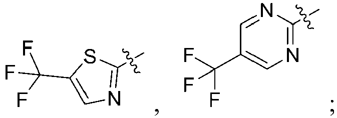 terminus is attached to L₅.

5. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 4, wherein: with the proviso that:
1) 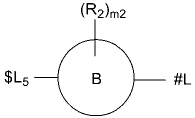 is not group E, the group E is selected from: 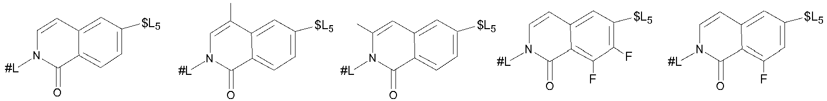 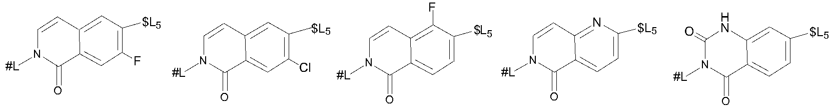 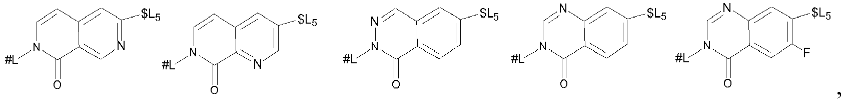 wherein, $L₅ represents the point of attachment to L₅, #L represents the point of attachment to L; or
2) 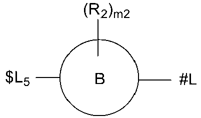 is not group E, the group E is selected from: 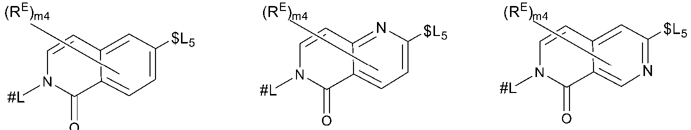 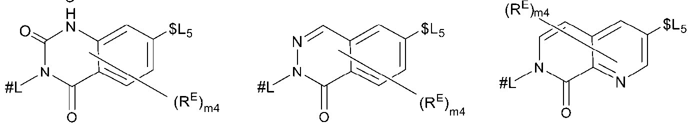 wherein, R^{E} is selected from C1-6 alkyl (preferably methyl), halogen (preferably fluorine, chlorine), m4 is selected from an integer of 0-5, $L₅ represents the point of attachment to L₅, #L represents the point of attachment to L; or
3) 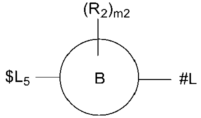 is not group E, the group E is selected from: 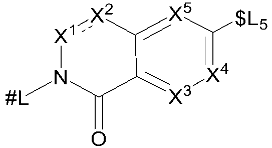
X¹ is selected from N, C=O, C-R^{F1}, C-(R^{F1})₂;
X² is selected from N, N-R^{F2}, C-R^{F3}, C-(R^{F3})₂;
X³ is selected from N, C-R^{F4};
X⁴ is selected from N, C-R^{F4};
X⁵ is selected from N, C-R^{F4};
wherein R^{F1}, R^{F3}, and R^{F4} are each independently selected from H, halogen, CH₃, CH₂F, CHF₂, CF₃, CH₂CF₃, OCH₃, OCF₃, OCHF₂, NO₂, CN, O-R^{F5}, C(O)-R^{F5}, C(O)-N(R^{F6})(R^{F7}), N(R^{F6})(R^{F7}), N(R^{F6})C(O)-R^{F5}, N(R^{F6})C(O)O-R^{F5}, N(R^{F6})S(O)₂(R^{F5}), -N(R^{F6})C(O)-N(R^{F7})(R^{F7}), S(O)₂R^{F5}, -SF₅, S(O)₂N(R^{F6})(R^{F7}), S(O)(NH)R^{F6}, S(O)(NR^{F6})NR^{F7}, C1-9 alkyl, C2-9 alkenyl, C2-9 alkynyl, C3-15 cycloalkyl, C6-10 aryl, 5-12-membered heteroaryl, or 4-12-membered heterocycloalkyl, wherein any alkyl, alkenyl, alkynyl, cycloalkyl, aryl, heteroaryl, or heterocycloalkyl is optionally substituted by one or more R^{F5};
R^{F2}, R^{F6}, R^{F7} are each independently selected from H, C1-9 alkyl, C2-9 alkenyl, C2-9 alkynyl, C3-15 cycloalkyl, C6-10 aryl, 5-12-membered heteroaryl, or 4-12-membered heterocycloalkyl, wherein any alkyl, alkenyl, alkynyl, cycloalkyl, aryl, heteroaryl, or heterocycloalkyl is optionally substituted by one or more R^{F5};
R^{F5} is independently selected from: H, C=O, halogen, -NO₂, -CN, C1-9 alkyl, C2-6 alkenyl, C2-6 alkynyl, C3-15 cycloalkyl, C1-8 haloalkyl, C6-10 aryl, 5-12-membered heteroaryl, 4-12-membered heterocycloalkyl, -OH, -O(C1-9 alkyl), -O(C2-6 alkenyl), -O(C2-6 alkynyl), -O(C3-15 cycloalkyl), -O(C1-8 haloalkyl), -O(C6-10 aryl), -O(5-12-membered heteroaryl), -O(4-12-membered heterocycloalkyl), -NH₂, -NH(C1-9 alkyl), -NH(C2-6 alkenyl), -NH(C2-6 alkynyl), -NH(C3-15 cycloalkyl), -NH(C1-8 haloalkyl), -NH(C6-10 aryl), -NH(5-12-membered heteroaryl), -NH(4-12-membered heterocycloalkyl), -N(C1-9 alkyl)₂, -N(C3-15 cycloalkyl)₂, - N(C2-6 alkenyl)₂, -N(C2-6 alkynyl)₂, -N(C3-15 cycloalkyl)₂, -N(C1-8 haloalkyl)₂, -N(C6-10 aryl)₂, -N(5-12-membered heteroaryl)₂, -N(4-12-membered heterocycloalkyl)₂, -N(C1-9 alkyl)(C3-15 cycloalkyl), -N(C1-9 alkyl)(C2-6 alkenyl), -N(C1-9 alkyl)(C2-6 alkynyl), -N(C1-9 alkyl)(C3-15 cycloalkyl), -N(C1-9 alkyl)(C1-8 haloalkyl), -N(C1-9 alkyl)(C6-10 aryl), - N(C1-9 alkyl)(5-12-membered heteroaryl), -N(C1-9 alkyl)(4-12-membered heterocycloalkyl), -C(O)(C1-9 alkyl), -C(O)(C2-6 alkenyl), -C(O)(C2-6 alkynyl), -C(O)(C3-15 cycloalkyl), - C(O)(C1-8 haloalkyl), -C(O)(C6-10 aryl), -C(O)(5-12-membered heteroaryl), -C(O)(4-12-membered heterocycloalkyl), -C(O)O(C1-9 alkyl), -C(O)O(C2-6 alkenyl), -C(O)O(C2-6 alkynyl), -C(O)O(C3-15 cycloalkyl), -C(O)O(C1-8 haloalkyl), -C(O)O(C6-10 aryl), -C(O)O(5-12-membered heteroaryl), -C(O)O(4-12-membered heterocycloalkyl), -C(O)NH₂, - C(O)NH(C1-9 alkyl), -C(O)NH(C2-6 alkenyl), -C(O)NH(C2-6 alkynyl), -C(O)NH(C3-15 cycloalkyl), -C(O)NH(C1-8 haloalkyl), -C(O)NH(C6-10 aryl), -C(O)NH(5-12-membered heteroaryl), -C(O)NH(4-12-membered heterocycloalkyl), -C(O)N(C1-9 alkyl)₂, -C(O)N(C3-15 cycloalkyl)₂, -C(O)N(C2-6 alkenyl)₂, -C(O)N(C2-6 alkynyl)₂, -C(O)N(C3-15 cycloalkyl)₂, - C(O)N(C1-8 haloalkyl)₂, -C(O)N(C6-10 aryl)₂, -C(O)N(5-12-membered heteroaryl)₂, - C(O)N(4-12-membered heterocycloalkyl)₂, -NHC(O)(C1-9 alkyl), -NHC(O)(C2-6 alkenyl), - NHC(O)(C2-6 alkynyl), -NHC(O)(C3-15 cycloalkyl), -NHC(O)(C1-8 haloalkyl), - NHC(O)(C6-10 aryl), -NHC(O)(5-12-membered heteroaryl), -NHC(O)(4-12-membered heterocycloalkyl), -NHC(O)O(C1-9 alkyl), -NHC(O)O(C2-6 alkenyl), -NHC(O)O(C2-6 alkynyl), -NHC(O)O(C3-15 cycloalkyl), -NHC(O)O(C1-8 haloalkyl), -NHC(O)O(C6-10 aryl), -NHC(O)O(5-12-membered heteroaryl), -NHC(O)O(4-12-membered heterocycloalkyl), - NHC(O)NH(C1-9 alkyl), -NHC(O)NH(C2-6 alkenyl), -NHC(O)NH(C2-6 alkynyl), - NHC(O)NH(C3-15 cycloalkyl), -NHC(O)NH(C1-8 haloalkyl), -NHC(O)NH(C6-10 aryl), - NHC(O)NH(5-12-membered heteroaryl), -NHC(O)NH(4-12-membered heterocycloalkyl), - SH, -S(C1-9 alkyl), -S(C2-6 alkenyl), -S(C2-6 alkynyl), -S(C3-15 cycloalkyl), -S(C1-8 haloalkyl), -S(C6-10 aryl), -S(5-12-membered heteroaryl), -S(4-12-membered heterocycloalkyl), -NHS(O)(C1-9 alkyl), -N(C1-9 alkyl)(S(O)(C1-9 alkyl), -S(O)N(C1-9 alkyl)₂, -S(O)(C1-9 alkyl), -S(O)(NH)(C1-9 alkyl), -S(O)(NH)(C3-9 cycloalkyl), -S(O)(NC1-9 alkyl)(C1-9 alkyl), -S(O)(NH)(C6-10 aryl), -S(O)(NH)(5-12-membered heteroaryl), - S(O)(C2-6 alkenyl), -S(O)(C2-6 alkynyl), -S(O)(C3-15 cycloalkyl), -S(O)(C1-8 haloalkyl), - S(O)(C6-10 aryl), -S(O)(5-12-membered heteroaryl), -S(O)(4-12-membered heterocycloalkyl), -S(O)₂(C1-9 alkyl), -S(O)₂(C2-6 alkenyl), -S(O)₂(C2-6 alkynyl), -S(O)₂(C3-15 cycloalkyl), - S(O)₂(C1-8 haloalkyl), -S(O)₂(C6-10 aryl), -S(O)₂(5-12-membered heteroaryl), -S(O)₂(4-12-membered heterocycloalkyl), -S(O)₂NH(C1-9 alkyl), or -S(O)₂N(C1-9 alkyl)₂; wherein any alkyl, cycloalkyl, aryl, heteroaryl, or heterocycloalkyl is optionally substituted by one or more of the following: halogen, C1-9 alkyl, C1-8 haloalkyl, -OH, -NH₂, -NH(C1-9 alkyl), -NH(C3-15 cycloalkyl), -NH(C1-8 haloalkyl), -NH(C6-10 aryl), -NH(5-12-membered heteroaryl), - NH(4-12-membered heterocycloalkyl), -N(C1-9 alkyl)₂, -N(C3-15 cycloalkyl)₂, -NHC(O)(C3-15 cycloalkyl), -NHC(O)(C1-8 haloalkyl), -NHC(O)(C6-10 aryl), -NHC(O)(5-12-membered heteroaryl), -NHC(O)(4-12-membered heterocycloalkyl), -NHC(O)O(C1-9 alkyl), - NHC(O)O(C2-6 alkynyl), -NHC(O)O(C3-15 cycloalkyl), -NHC(O)O(C1-8 haloalkyl), - NHC(O)O(C6-10 aryl), -NHC(O)O(5-12-membered heteroaryl), -NHC(O)O(4-12-membered heterocycloalkyl), -NHC(O)NH(C1-9 alkyl), -S(O)(NH)(C1-9 alkyl), S(O)₂(C1-9 alkyl), - S(O)₂(C3-15 cycloalkyl), -S(O)₂(C1-8 haloalkyl), -S(O)₂(C6-10 aryl), -S(O)₂(5-12-membered heteroaryl), -S(O)₂(4-12-membered heterocycloalkyl), -S(O)₂NH(C1-9 alkyl), -S(O)₂N(C1-9 alkyl)₂, -O(C3-15 cycloalkyl), -O(C1-8 haloalkyl), -O(C6-10 aryl), -O(5-12-membered heteroaryl), -O(4-12-membered heterocycloalkyl), or -O(C1-9 alkyl); wherein, $L₅ represents the point of attachment to L₅, #L represents the point of attachment to L.

6. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 4, wherein:
ring B is selected from phenyl, 5-6-membered heteroaryl, 5-6-membered heterocycloalkyl-fused-5-6-membered heterocycloalkyl, benzo-fused-5-6-membered heteroaryl, benzo-fused-5-6-membered heterocycloalkyl, 5-6-membered heteroaryl-fused-5-6-membered heterocycloalkyl; preferably, ring B is selected from phenyl, pyridinyl, pyrazolyl, pyrazinyl, oxazolyl, isoxazolyl, benzopyrazolyl, thiazolyl, isothiazolyl, pyridazinyl, pyrimidinyl, triazolyl, thiadiazolyl, indolinyl, benzimidazolyl, dihydrobenzimidazolyl, indazolyl, isoindolinyl, pyridopyridinyl, pyrrolopyrimidinyl;
preferably, ring B is selected from phenyl, pyrimidinyl, pyridinyl, pyrazinyl, pyridazinyl, pyrazolyl, thiazolyl, isothiazolyl, isoxazolyl, triazolyl (for example 1,2,3-triazolyl), thiadiazolyl (for example 1,3,4-thiadiazolyl), 5-membered nitrogen-containing heterocycloalkyl-fused-phenyl, phenyl-fused-pyrazolyl, phenyl-fused-5-membered nitrogen-containing heterocycloalkyl (for example phenyl-fused-tetrahydropyrrole), pyrimidinyl-fused-5-membered nitrogen-containing heterocycloalkyl, 6-membered nitrogen-containing heterocycloalkyl-fused-5-membered nitrogen-containing heterocycloalkyl, 6-membered nitrogen-containing heterocycloalkyl-fused-pyrazolyl;
preferably, ring B is selected from phenyl, 5-6-membered heteroaryl, benzo-fused-5-6-membered heteroaryl;
preferably, ring B is selected from phenyl, 5-6-membered heteroaryl;
preferably, ring B is selected from phenyl, 6-membered heteroaryl;
preferably, ring B is selected from phenyl, pyrimidinyl, pyridinyl, pyrazinyl, pyridazinyl, pyrazolyl, thiazolyl, isothiazolyl, isoxazolyl, triazolyl (for example 1,2,3-triazolyl), thiadiazolyl (for example 1,3,4-thiadiazolyl);
preferably, ring B is selected from phenyl, pyridinyl, pyrazinyl, isoxazolyl;
preferably, ring B is selected from phenyl, pyridinyl, pyrazinyl;
preferably, ring B is selected from the following groups: 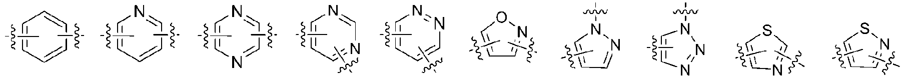 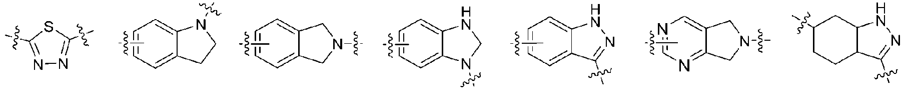 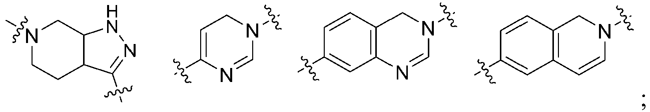 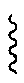 terminus is attached to L terminus or L₅;
preferably, ring B is selected from 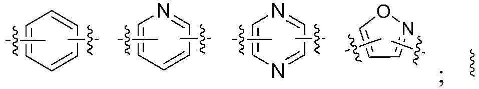 terminus is attached to L terminus or L₅;
preferably, ring B is selected from 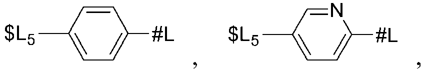 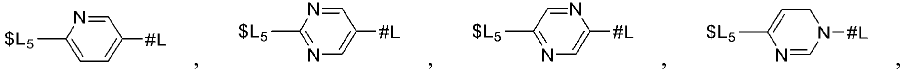 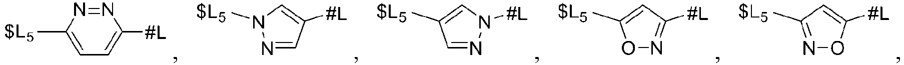 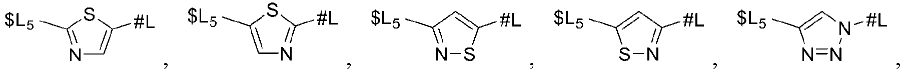 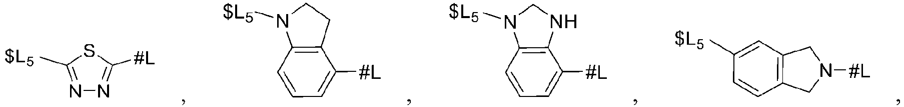 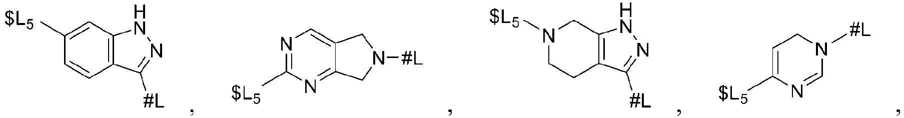 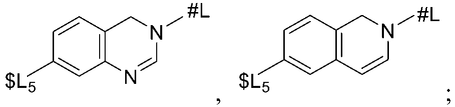 $L₅ represents the site of attachment to L₅, #L represents the site of attachment to L;
preferably, ring B is selected from 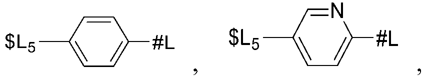 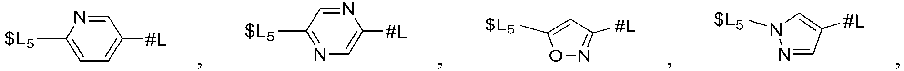 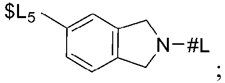 $L₅ represents the site of attachment to L₅, #L represents the site of attachment to L;
more preferably, ring B is selected from 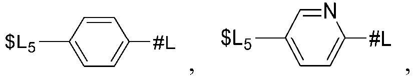 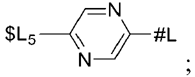 $L₅ represents the site of attachment to L₅, #L represents the site of attachment to L.

7. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 6, wherein:
R₂ is selected from halogen, hydroxyl, C1-3 alkyl, C1-3 haloalkyl, C1-3 alkoxy, C1-3 haloalkoxy, C3-4 cycloalkyl, -C1-2 alkylene-C3-4 cycloalkyl, -NReRf; Re and Rf are each independently selected from H and C1-3 alkyl; optionally, two R₂ attached to the same carbon atom form C=O with that carbon atom;
preferably, R₂ is selected from F, Cl, hydroxyl, amino, methyl; optionally, two R₂ attached to the same carbon atom form C=O with that carbon atom;
preferably, R₂ is selected from F or hydroxyl; more preferably, R₂ is F;
preferably, m2 is an integer of 0-2;
preferably, m2 is 0 or 1;
preferably, m2 is 0.

8. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 7, wherein:
the 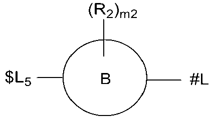 is selected from 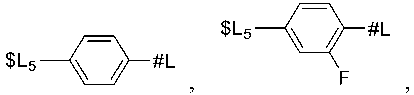 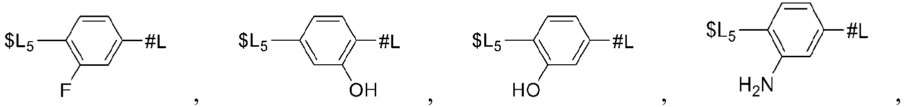 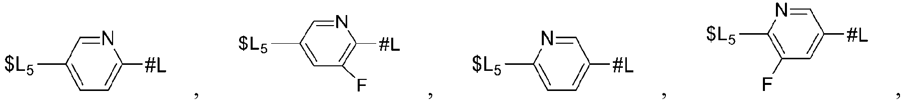 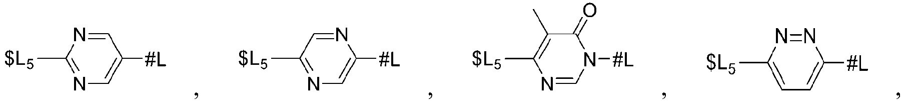 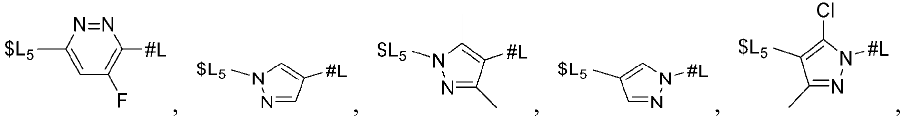 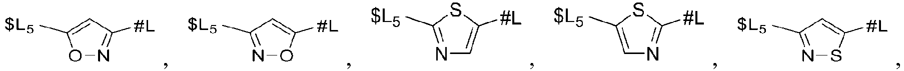 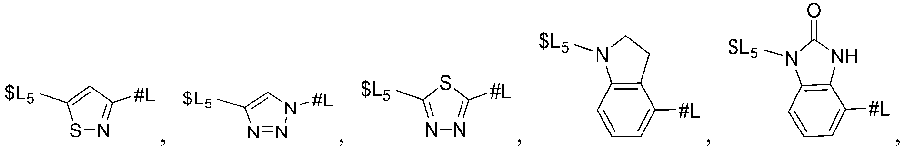 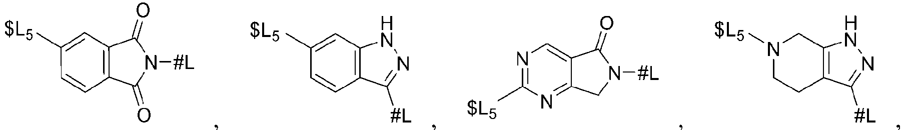 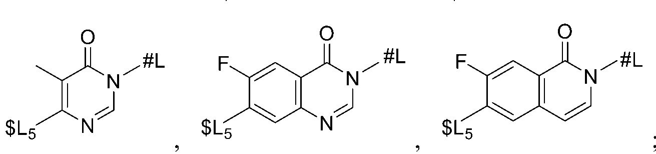 $L₅ represents the site of attachment to L₅, #L represents the site of attachment to L;
preferably, 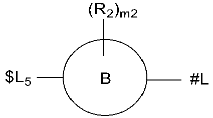 is selected from 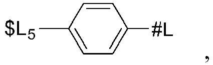 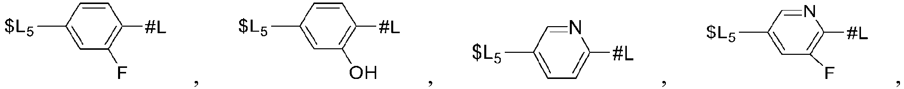 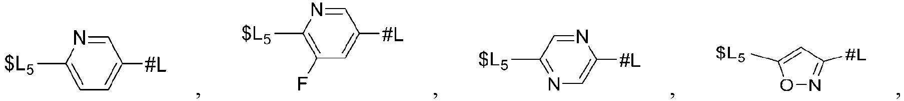 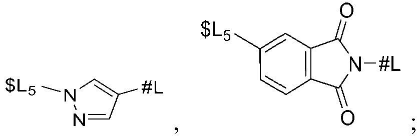 $L₅ represents the site of attachment to L₅, #L represents the site of attachment to L;
more preferably, 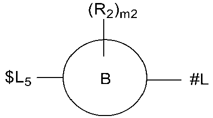 is selected from 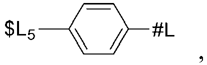 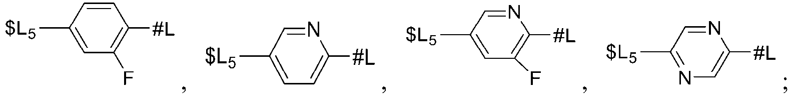 $L₅ represents the site of attachment to L₅, #L represents the site of attachment to L;
more preferably, 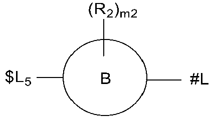 is selected from 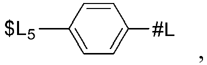 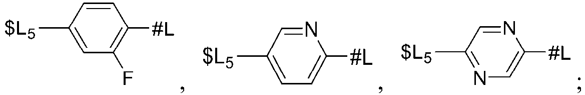 $L₅ represents the site of attachment to L₅, #L represents the site of attachment to L.

9. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 8, wherein:
L is the linking group represented by formula L: $B-L1-L2-X1-L3-L4-#C (L) ;
wherein, $B represents the site of attachment to ring B, #C represents the site of attachment to ring C;
L1 is selected from a bond, -C(=O)-, $B-C(=O)-NRa-#L2, -NRb-, $B-NRb-C(=O)-#L2, 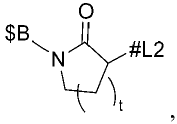 t is 1, 2, or 3, the 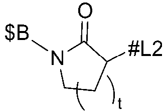 is optionally substituted by a substituent selected from halogen, hydroxyl, cyano, C1-6 alkyl, C1-6 alkoxy, C1-6 haloalkyl, C1-6 haloalkoxy;
Ra and Rb are each independently selected from H, D, C1-6 alkyl, C1-6 haloalkyl, C3-6 cycloalkyl, 4-7-membered heterocycloalkyl; $B represents the site of attachment to ring B, #L2 represents the site of attachment to L2;
preferably, Ra and Rb are each independently selected from H, C1-6 alkyl;
preferably, Ra and Rb are each independently selected from H, C1-3 alkyl;
preferably, L1 is selected from a bond, -C(=O)-, $B-C(=O)NH-#L2, $B-C(=O)N(CH₃)-#L2, $B-NH(C=O)-#L2, -NH-, 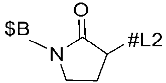
(for example 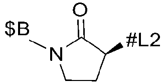
);
further preferably, L1 is selected from -C(=O)-, $B-C(=O)NH-#L2, $B-NH(C=O)-#L2;
more preferably, L1 is selected from $B-C(=O)NH-#L2, $B-NH(C=O)-#L2;
L2 is selected from a bond, C1-6 alkylene; wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy;
preferably, L2 is selected from a bond, C1-6 alkylene; wherein, the C1-6 alkylene is optionally substituted by 1 to 3 substituents selected from halogen, oxo, C1-3 alkyl, C3-6 cycloalkyl, 4-7-membered heterocycloalkyl, -C(=O)-4-7-membered heterocycloalkyl, - C(=O)N(R_{L2})₂; the C3-6 cycloalkyl, 4-7-membered heterocycloalkyl, -C(=O)-4-7-membered heterocycloalkyl, as a substituent of C1-6 alkylene, are optionally substituted by 1 or more substituents selected from halogen, cyano, C1-3 alkyl, C1-3 alkoxy, C1-3 haloalkyl, C1-3 haloalkoxy; each R_{L2} is independently selected from H, D, C1-6 alkyl, C3-6 cycloalkyl, 4-7-membered heterocycloalkyl, phenyl, 5-6-membered heteroaryl, and the C1-6 alkyl, C3-6 cycloalkyl, 4-7-membered heterocycloalkyl, phenyl, 5-6-membered heteroaryl are optionally substituted by a substituent selected from halogen, hydroxyl, cyano, C1-3 alkyl, C1-3 alkoxy;
further preferably, L2 is selected from a bond, C2-5 alkylene; wherein, the C2-5 alkylene is optionally substituted by 1 or 2 substituents selected from oxo, C1-3 alkyl, C3-6 cycloalkyl, 4-7-membered nitrogen-containing saturated heterocycloalkyl, -C(=O)-4-7-membered nitrogen-containing saturated heterocycloalkyl, -C(=O)N(R_{L2})₂, the 4-7-membered nitrogen-containing saturated heterocycloalkyl, -C(=O)-4-7-membered nitrogen-containing saturated heterocycloalkyl, as a substituent of C2-5 alkylene, are optionally substituted by a substituent selected from halogen, cyano, C1-3 alkyl; each R_{L2} is independently selected from H, C1-4 alkyl, C3-6 cycloalkyl, 4-7-membered heterocycloalkyl, 5-6-membered heteroaryl, and the C1-4 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy;
further preferably, L2 is selected from a bond, C2-5 alkylene; wherein, the C2-5 alkylene is optionally substituted by a substituent selected from C1-3 alkyl, C3-6 cycloalkyl (for example cyclopropyl, cyclobutyl, cyclohexyl), 4-7-membered heterocycloalkyl (for example oxetanyl, oxanyl, 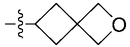 ) , 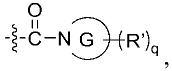 -C(=O)N(R_{L2})₂; wherein, ring G is 4-10-membered nitrogen-containing saturated heterocycloalkyl, R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 5-6-membered heteroaryl (for example pyridinyl), and the C1-3 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy;
more preferably, L2 is selected from C2-5 alkylene, the C2-5 alkylene is optionally substituted by a substituent selected from C1-3 alkyl, cyclopropyl, 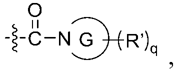 C(=O)N(R_{L2})₂; wherein, ring G is 4-10-membered nitrogen-containing saturated heterocycloalkyl (for example azetidinyl, azacyclopentyl, morpholinyl, 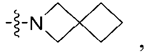 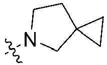 ), R' is selected from halogen (for example F, Cl), C1-3 alkyl (for example methyl, isopropyl), q is selected from 0, 1; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 6-membered heteroaryl (for example pyridinyl), and the 4-7-membered heterocycloalkyl, 6-membered heteroaryl are optionally substituted by a substituent selected from C1-3 alkyl;
X1 is selected from a bond, O, S;
preferably, X1 is selected from a bond, O;
further preferably, X1 is selected from a bond;
L3 is selected from a bond, C1-6 alkylene, C3-10 cycloalkyl-(CH₂)ₖ-#L4, 4-10-membered heterocycloalkyl-(CH₂)ₖ-#L4, 5-6-membered heteroaryl-(CH₂)ₖ-#L4; wherein, the C1-6 alkylene, C3-10 cycloalkyl-(CH₂)ₖ-#L4, 4-10-membered heterocycloalkyl-(CH₂)ₖ-#L4, 5-6-membered heteroaryl-(CH₂)ₖ-#L4 are optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; k is selected from 0, 1, 2, 3; #L4 represents the site of attachment to L4;
preferably, L3 is selected from a bond, C1-3 alkylene, C3-6 cycloalkyl-(CH₂)ₖ-#L4, 4-6-membered monocyclic heterocycloalkyl-(CH₂)ₖ-#L4, 6-10-membered spiro heterocycloalkyl-(CH₂)ₖ-#L4, 6-10-membered bridged heterocycloalkyl-(CH₂)ₖ-#L4, 5-6-membered heteroaryl-(CH₂)ₖ-#L4; wherein, the C1-3 alkylene, C3-6 cycloalkyl-(CH₂)ₖ-#L4, 4-6-membered monocyclic heterocycloalkyl-(CH₂)ₖ-#L4, 6-10-membered spiro heterocycloalkyl-(CH₂)ₖ-#L4, 6-10-membered bridged heterocycloalkyl-(CH₂)ₖ-#L4, or 5-6-membered heteroaryl-(CH₂)ₖ-#L4 are optionally substituted by a substituent selected from halogen, hydroxyl, C1-3 alkyl, C1-3 haloalkyl, C1-3 alkoxy, C1-3 haloalkoxy, -O-C3-6 cycloalkyl;
further preferably, L3 is selected from a bond, C1-3 alkylene, 4-6-membered monocyclic heterocycloalkyl-(CH₂)ₖ-#L4, 6-10-membered spiro heterocycloalkyl-(CH₂)ₖ-#L4, pyrrolyl-(CH₂)ₖ-#L4; wherein, the C1-3 alkylene, 4-6-membered monocyclic heterocycloalkyl-(CH₂)ₖ-#L4, 6-10-membered spiro heterocycloalkyl-(CH₂)ₖ-#L4, pyrrolyl-(CH₂)ₖ-#L4 are optionally substituted by a substituent selected from C1-3 alkyl, C1-3 alkoxy, -O-C3-6 cycloalkyl;
more preferably, L3 is selected from a bond, C1-3 alkylene;
L4 is selected from a bond, O, S, -NRc-; Rc is selected from H, C1-6 alkyl, C1-6 haloalkyl, C3-6 cycloalkyl, 4-7-membered heterocycloalkyl;
preferably, L4 is selected from a bond, O, -NRc-; Rc is selected from H, C1-3 alkyl;
further preferably, L4 is selected from -NH-.

10. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 9, wherein:
when L1 is selected from $B-C(=O)-NRa-#L2, any one of the following conditions is satisfied:
(1) L2 is selected from C1-6 alkylene; wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from a bond, O, -NRc-, Rc is selected from H, C1-3 alkyl;
preferably, L2 is selected from C2-6 alkylene; wherein, the C2-6 alkylene is optionally substituted by a substituent of C1-3 alkyl, C3-6 cycloalkyl, 4-7-membered heterocycloalkyl, 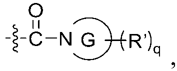 -C(=O)N(R_{L2})₂; wherein, ring G is 4-10-membered nitrogen-containing saturated heterocycloalkyl, R' is selected from halogen, hydroxyl, cyano, C1-3 alkyl, C1-3 haloalkyl, C1-3 alkoxy, C1-3 haloalkoxy, q is selected from 0, 1, or 2; each R_{L2} is independently selected from H, methyl, ethyl, isopropyl, cyclopropyl, cyclobutyl, oxanyl, pyridinyl, and the oxanyl is optionally substituted by a substituent selected from C1-3 alkyl;
(2) L2 is selected from a bond, C1-6 alkylene; X1 is selected from a bond; L3 is selected from C3-10 cycloalkyl-(CH₂)ₖ-#L4, 4-10-membered heterocycloalkyl-(CH₂)ₖ-#L4, 5-6-membered heteroaryl-(CH₂)ₖ-#L4, wherein, the C3-10 cycloalkyl-(CH₂)ₖ-#L4, 4-10-membered heterocycloalkyl-(CH₂)ₖ-#L4, 5-6-membered heteroaryl-(CH₂)ₖ-#L4 are optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; k is selected from 0, 1, 2, 3; #L4 represents the site of attachment to L4; L4 is selected from a bond or -NRc-;
preferably, L2 is selected from a bond, C1-3 alkylene; X1 is selected from a bond; L3 is selected from C3-6 cycloalkyl, 4-10-membered heterocycloalkyl, 5-6-membered heteroaryl-(CH₂)ₖ-#L4, wherein, the C3-6 cycloalkyl, 4-10-membered heterocycloalkyl, 5-6-membered heteroaryl-(CH₂)ₖ-#L4 are optionally substituted by 1 or more substituents selected from C1-3 alkyl, C1-3 alkoxy, -O-C3-6 cycloalkyl; k is selected from 0, 1, 2; #L4 represents the site of attachment to L4; L4 is selected from a bond or -NRc-; when L1 is selected from $B-NRb-C(=O)-#L2, any one of the following conditions is satisfied:
(1) L2 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from a bond or O;
preferably, L2 is selected from C1-3 alkylene, wherein, the C1-3 alkylene is optionally substituted by 1 substituent selected from C1-3 alkyl, C3-6 cycloalkyl; X1 is selected from a bond, L3 is selected from a bond, L4 is selected from a bond or O;
(2) L2 is selected from C1-6 alkylene; X1 is selected from O; L3 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; L4 is selected from -NRc-;
preferably, L2 is selected from C1-3 alkylene, X1 is selected from O; L3 is selected from C1-3 alkylene, wherein, the C1-3 alkylene is optionally substituted by 1 substituent selected from C1-3 alkyl; L4 is selected from -NRc-;
when L1 is selected from -C(=O)-, any one of the following conditions is satisfied:
(1) L2 is selected from a bond; X1 is selected from a bond; L3 is selected from 4-10-membered heterocycloalkyl-(CH₂)ₖ-#L4, wherein, the 4-10-membered heterocycloalkyl-(CH₂)ₖ-#L4 is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, - O-C3-6 cycloalkyl, 4-10-membered heterocycloalkyl, -O-4-10-membered heterocycloalkyl; k is selected from 0, 1, 2, 3; #L4 represents the site of attachment to L4, L4 is selected from - NRc-;
preferably, L3 is selected from 4-10-membered heterocycloalkyl-(CH₂)ₖ-#L4, wherein, the 4-10-membered heterocycloalkyl-(CH₂)ₖ-#L4 is optionally substituted by 1 substituent selected from C1-3 alkyl;
(2) L2 is selected from C1-6 alkylene; X1 is selected from O; L3 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; L4 is selected from -NRc-;
preferably, L2 is selected from C1-3 alkylene; X1 is selected from O; L3 is selected from C1-3 alkylene, wherein, the C1-3 alkylene is optionally substituted by 1 substituent selected from C1-3 alkyl; L4 is selected from -NRc-;
when L1 is selected from -NRc-, L2 is selected from C1-6 alkylene; wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from -NRc-;
preferably, L2 is selected from C1-6 alkylene; wherein, the C1-6 alkylene is optionally substituted by 1 or more substituents selected from halogen, hydroxyl, oxo, C1-3 alkyl, C3-6 cycloalkyl, 4-7-membered heterocycloalkyl, -C(=O)-4-7-membered heterocycloalkyl, - C(=O)N(R_{L2})₂; the C3-6 cycloalkyl, 4-7-membered heterocycloalkyl, -C(=O)-4-7-membered heterocycloalkyl, as a substituent of C1-6 alkylene, are optionally substituted by 1 or more substituents selected from halogen, hydroxyl, cyano, C1-3 alkyl, C1-3 alkoxy, C1-3 haloalkyl, C1-3 haloalkoxy; each R_{L2} is independently selected from H, D, C1-3 alkyl, C3-6 cycloalkyl, 4-7-membered heterocycloalkyl, phenyl, 5-6-membered heteroaryl, and the C1-3 alkyl, C3-6 cycloalkyl, 4-7-membered heterocycloalkyl, phenyl, 5-6-membered heteroaryl are optionally substituted by a substituent selected from halogen, hydroxyl, cyano, C1-3 alkyl, C1-3 alkoxy; X1 is selected from a bond, L3 is selected from a bond, L4 is selected from -NRc-;
when L1 is selected from a bond, any one of the following conditions is satisfied:
(1) L2 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-10 cycloalkyl, -O-C3-10 cycloalkyl, 4-10-membered heterocycloalkyl, -O-4-10-membered heterocycloalkyl, -C(=O)-4-10-membered heterocycloalkyl, -C(=O)N(R_{L2})₂; the C3-10 cycloalkyl, -O-C3-10 cycloalkyl, 4-10-membered heterocycloalkyl, -C(=O)-4-10-membered heterocycloalkyl, as a substituent of C1-6 alkylene, are optionally substituted by 1 or more substituents selected from halogen, hydroxyl, cyano, C1-6 alkyl, C1-6 alkoxy, C1-6 haloalkyl, C1-6 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy; X1 is selected from a bond, L3 is selected from a bond, L4 is selected from a bond or -NRc-;
preferably, L2 is selected from C1-6 alkylene, wherein, the C1-6 alkylene is optionally substituted by 1 substituent selected from C1-3 alkyl;
(2) L2 is selected from C1-6 alkylene, X1 is selected from O, L3 is selected from C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; L4 is selected from -NRc-;
preferably, L2 is selected from C1-3 alkylene, X1 is selected from O, L3 is selected from C1-3 alkylene, wherein, the C1-3 alkylene is optionally substituted by 1 substituent selected from C1-3 alkyl;
when L1 is selected from 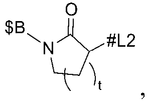
L2 is selected from a bond; X1 is selected from O, S; L3 is selected from a bond, C1-6 alkylene, wherein, the C1-6 alkylene is optionally substituted by 1 or more substituents selected from D, halogen, hydroxyl, oxo, C1-6 alkyl, C1-6 alkoxy, C1-6 haloalkyl, C1-6 haloalkoxy, C3-6 cycloalkyl, -O-C3-6 cycloalkyl, 4-10-membered heterocycloalkyl, -O-4-10-membered heterocycloalkyl; and L4 is selected from a bond, $B represents the site of attachment to ring B, #L2 represents the site of attachment to L2;
preferably, L2 is selected from a bond; X1 is selected from O, L3 is selected from a bond; L4 is selected from a bond;
wherein, t, Ra, Rb, and Rc are as defined in claim 9.

11. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 9, wherein:
when L1 is selected from $B-C(=O)-NRa-#L2, L2 is selected from: 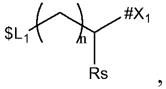 wherein $L1 represents the site of attachment to L1, #X1 represents the site of attachment to X1, n is an integer selected from 0, 1, 2, 3, 4, or 5, and wherein Rs is selected from H, substituted or unsubstituted C1-6 alkyl, substituted or unsubstituted C3-6 cycloalkyl, -C(=O)NRᵢᵢRᵢᵢᵢ, wherein Rᵢᵢ and Rᵢᵢᵢ may independently represent hydrogen, deuterium, C1-6 alkyl, C3-6 cycloalkyl, heterocycloalkyl, or aryl, or Rᵢᵢ and Rᵢᵢᵢ together with the atoms that they are attached to, form 4-10-membered heterocycloalkyl, the heterocycloalkyl is optionally substituted by 1-3 substituents selected from halogen, -OH, C1-6 alkyl, C1-6 alkoxy, C1-6 haloalkyl, C1-6 haloalkoxy,
wherein, Ra is as defined in claim 9; or
when L1 is selected from $B-C(=O)-NRa-#L2, L2 is selected from: 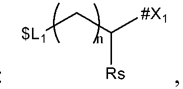 wherein $L1 represents the site of attachment to L1, #X1 represents the site of attachment to X1, n is an integer selected from 0, 1, 2, 3, 4, or 5, Rs is selected from H, D, halogen, hydroxyl, C1-6 alkyl, C1-6 alkoxy, C1-6 haloalkyl, C1-6 haloalkoxy, C3-10 cycloalkyl, 4-10-membered heterocycloalkyl, 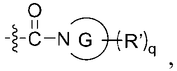 -C(=O)N(R_{L2})₂; wherein, ring G is 4-10-membered nitrogen-containing saturated heterocycloalkyl, R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy;
preferably, Rs is selected from H, D, halogen, hydroxyl, C1-6 alkyl, C1-6 alkoxy, C1-6 haloalkyl, C1-6 haloalkoxy, C3-6 cycloalkyl, 4-7-membered heterocycloalkyl, 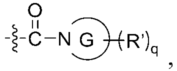 -C(=O)N(R_{L2})₂; wherein, ring G is 4-10-membered nitrogen-containing saturated heterocycloalkyl, R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, D, C1-4 alkyl, C3-6 cycloalkyl, 4-7-membered heterocycloalkyl, phenyl, 5-6-membered heteroaryl, and the C1-4 alkyl, C3-6 cycloalkyl, 4-7-membered heterocycloalkyl, phenyl, 5-6-membered heteroaryl are optionally substituted by a substituent selected from halogen, hydroxyl, cyano, C1-3 alkyl, C1-3 alkoxy.

12. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 11, wherein:
n is selected from 3, Rs is selected from: methyl, cyclopropyl, cyclobutyl, cyclohexyl, 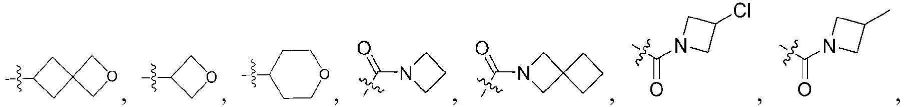 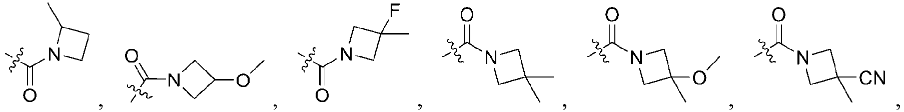 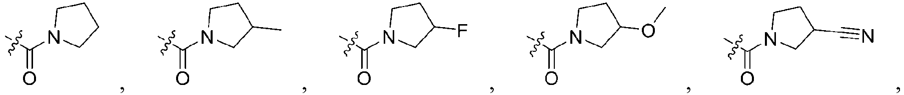 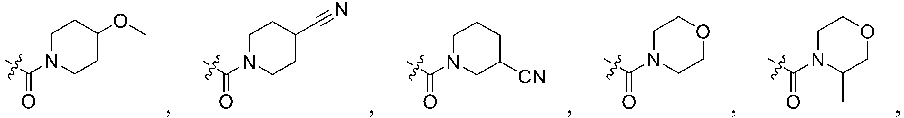 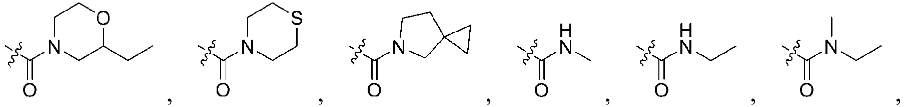 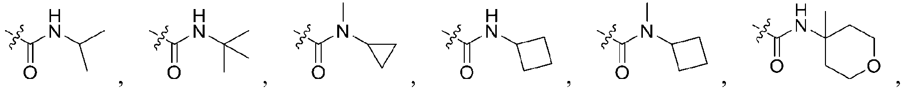 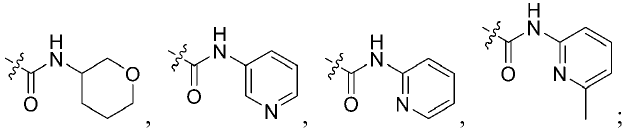
preferably, Rs is selected from methyl,

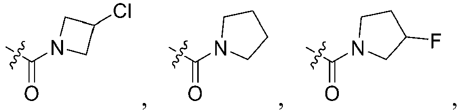

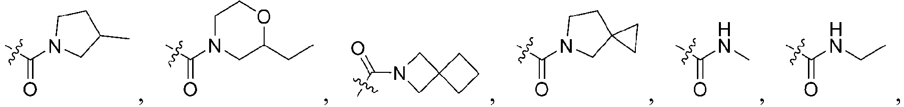

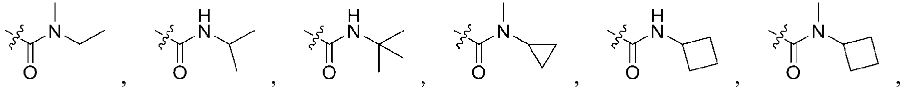

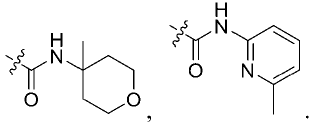

13. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 9, wherein:
when L1 is selected from $B-NRb-C(=O)-#L2, X1, L3, and L4 all represent a single bond, and L2 is selected from substituted or unsubstituted C1-4 alkylene, preferably substituted or unsubstituted methyl, ethyl, or propyl; or
when L1 is selected from $B-NRb-C(=O)-#L2, X1 is selected from O or S, L2 and L3 are each independently selected from substituted or unsubstituted C1-4 alkylene, preferably substituted or unsubstituted methyl, ethyl, or propyl, and L4 is selected from -NRc-,
wherein, Rb and Rc are as defined in claim 9.

14. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 9, wherein:
when L1 is selected from a single bond, X1 is selected from O, S, L2 and L3 are each independently selected from substituted or unsubstituted C1-4 alkylene, and L4 is selected from -NRc-, wherein Rb and Rc are as defined in claim 9,
preferably, wherein the carbon atom of L2 attached to ring B is substituted with =O to form a carbonyl.

15. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 9, wherein:
when L1 is selected from 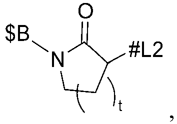 X1 is selected from O, L2, L3, and L4 all represent a single bond, and t is selected from 1; $B represents the site of attachment to ring B, #L2 represents the site of attachment to L2.

16. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 9, wherein:
L is selected from: 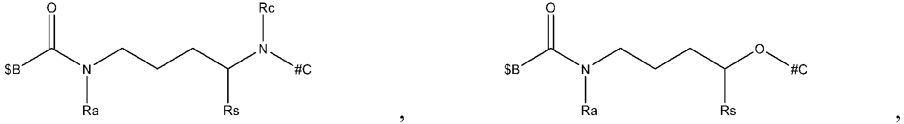 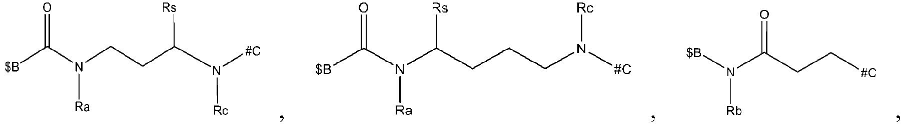 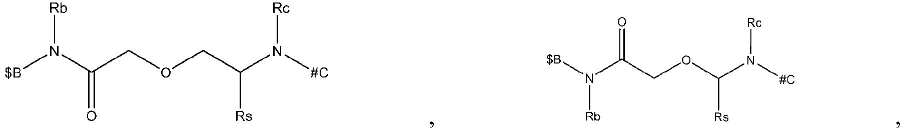 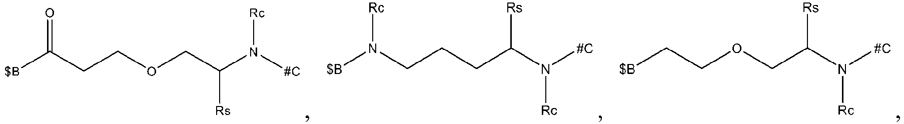 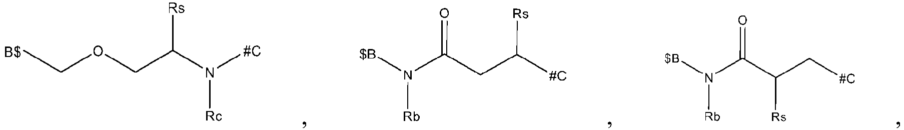 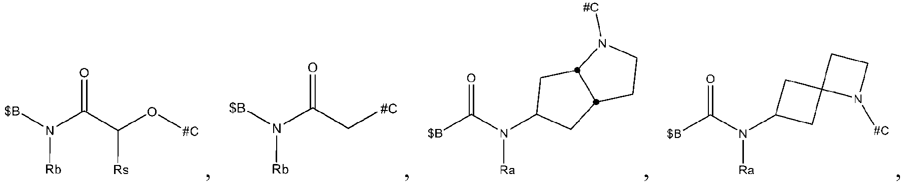 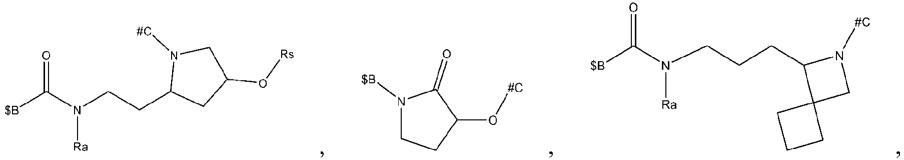 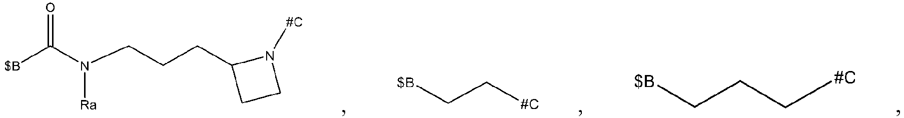 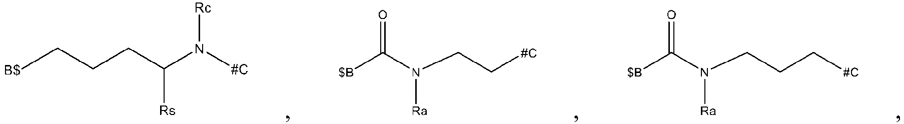 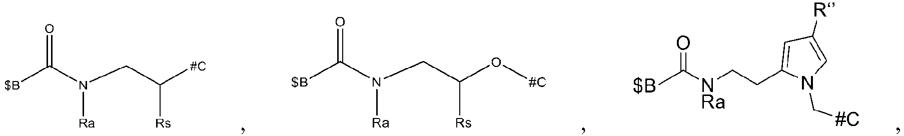 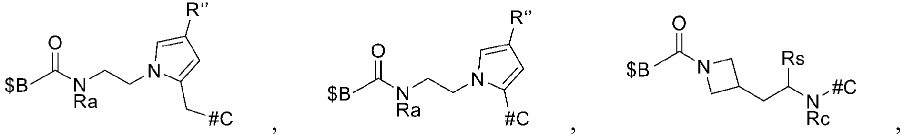 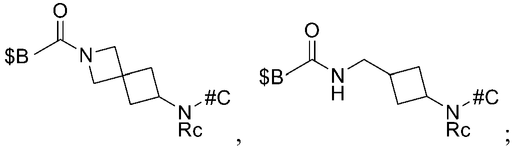
wherein, $B represents the site of attachment to ring B, #C represents the site of attachment to ring C, Ra, Rb, and Rc are as defined in claim 9, Rs is as defined in claim 11, R" is selected from C1-6 alkoxy, -O-C3-6 cycloalkyl;
preferably, Ra, Rb, and Rc are each independently selected from H or methyl;
preferably, Rs is selected from H, methyl, cyclopropyl, cyclobutyl, cyclohexyl,

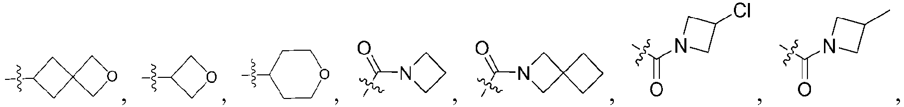

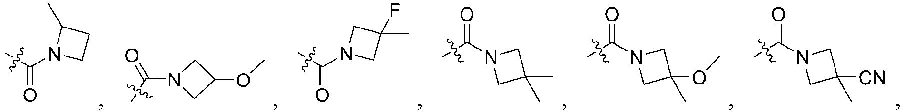

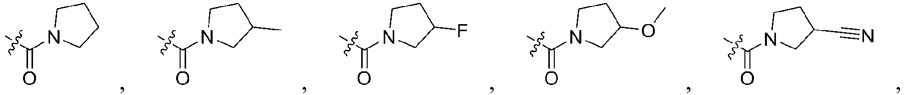

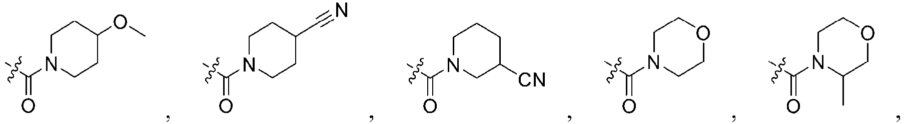

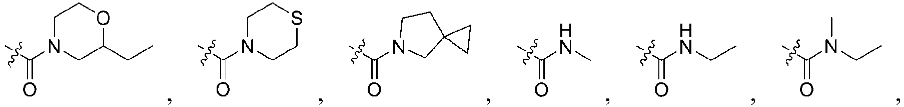

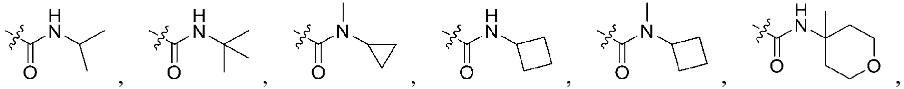

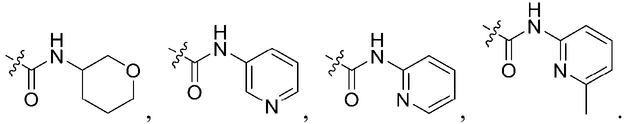

17. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 9, wherein:
L is selected from: 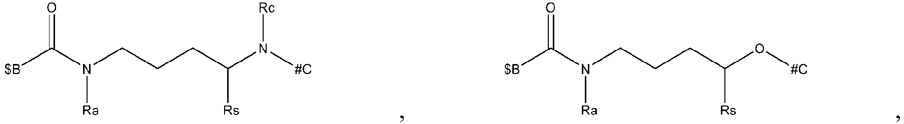 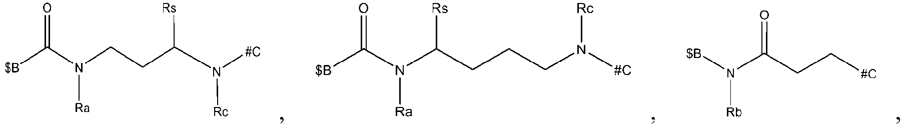 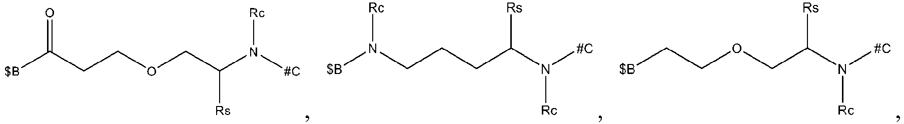 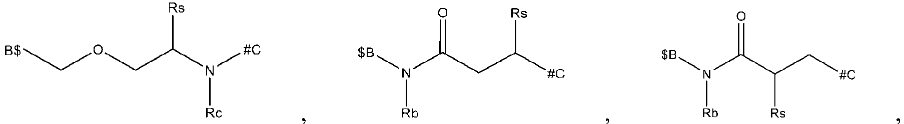 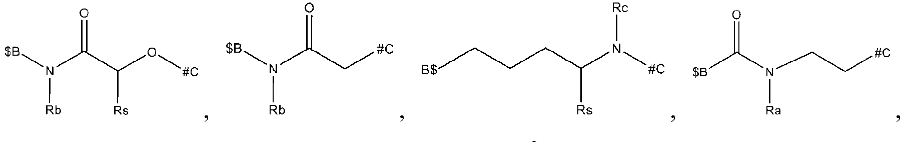 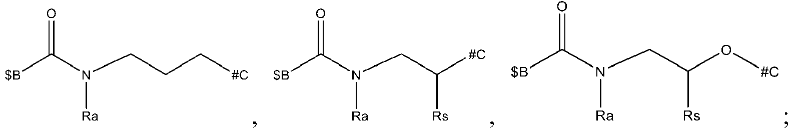
wherein, $B represents the site of attachment to ring B, #C represents the site of attachment to ring C, Ra, Rb, and Rc are as defined in claim 9, Rs is as defined in claim 11;
preferably, L is selected from: 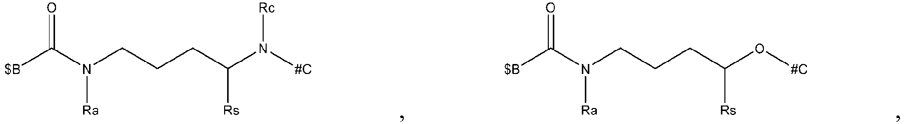 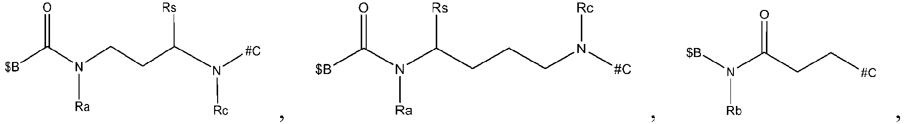 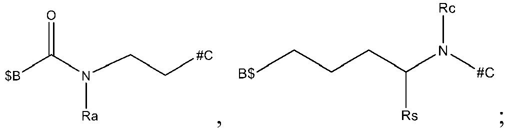
more preferably, L is selected from: 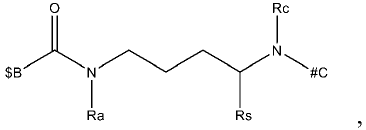 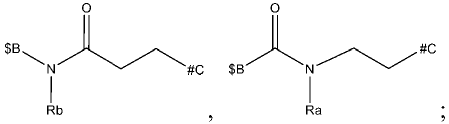
preferably, Ra, Rb, and Rc are each independently selected from H or methyl;
preferably, Rs is selected from H, D, halogen, hydroxyl, C1-6 alkyl, C1-6 alkoxy, C1-6 haloalkyl, C1-6 haloalkoxy, C3-6 cycloalkyl, 4-7-membered heterocycloalkyl, 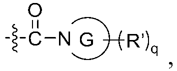 -C(=O)N(R_{L2})₂; wherein, ring G is 4-10-membered nitrogen-containing saturated heterocycloalkyl, R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, D, C1-4 alkyl, C3-6 cycloalkyl, 4-7-membered heterocycloalkyl, phenyl, 5-6-membered heteroaryl, and the C1-4 alkyl, C3-6 cycloalkyl, 4-7-membered heterocycloalkyl, phenyl, 5-6-membered heteroaryl are optionally substituted by a substituent selected from halogen, hydroxyl, cyano, C1-3 alkyl, C1-3 alkoxy;
more preferably, Rs is selected from C1-3 alkyl, C3-6 cycloalkyl (for example cyclopropyl, cyclobutyl, cyclohexyl), 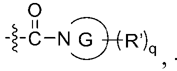 -C(=O)N(R_{L2})₂; wherein, ring G is 4-10-membered nitrogen-containing saturated heterocycloalkyl (for example azetidinyl, azacyclopentyl, morpholinyl, 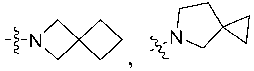 ), R' is selected from halogen (for example F, Cl), C1-3 alkyl (for example methyl, isopropyl), q is selected from 0, 1; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 6-membered heteroaryl (for example pyridinyl), and the 4-7-membered heterocycloalkyl, 6-membered heteroaryl are optionally substituted by a substituent selected from C1-3 alkyl;
preferably, Rs is selected from H or the following substituted or unsubstituted groups: methyl, ethyl, propyl, cyclopropyl, cyclobutyl, cyclopentyl, cyclohexyl.

18. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 9, wherein:
L is selected from 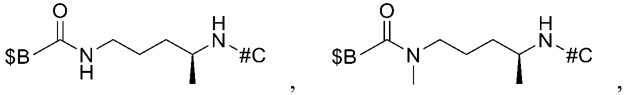 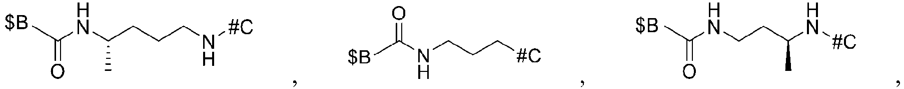 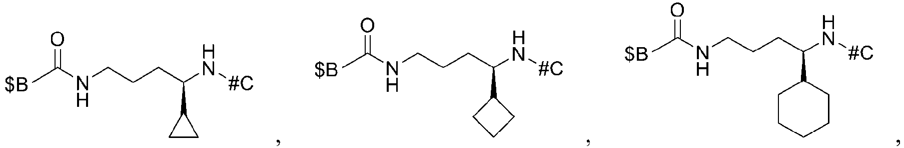 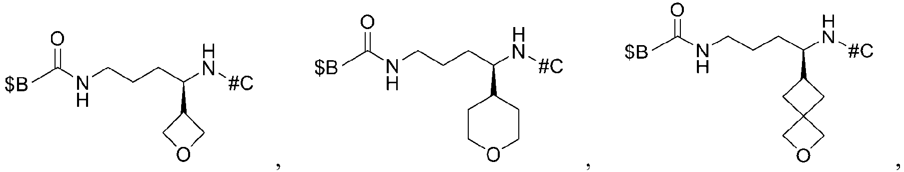 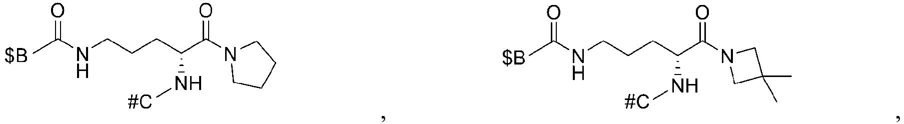 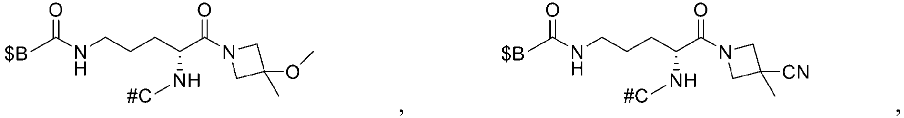 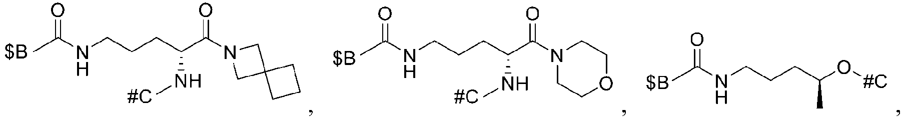 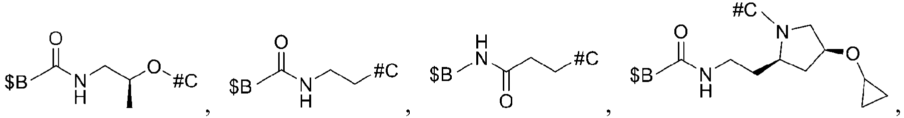 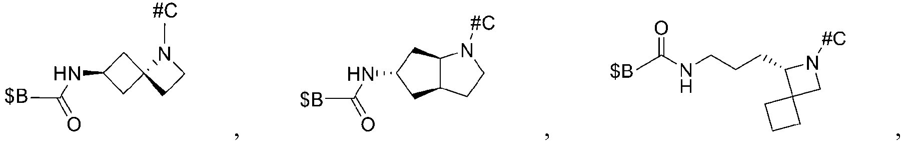 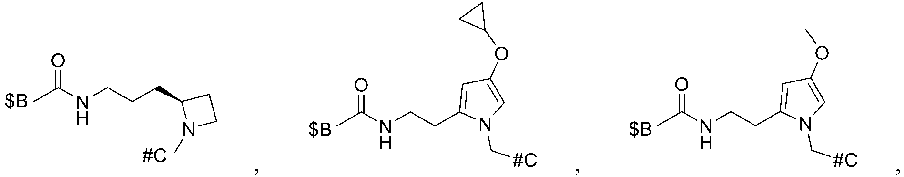 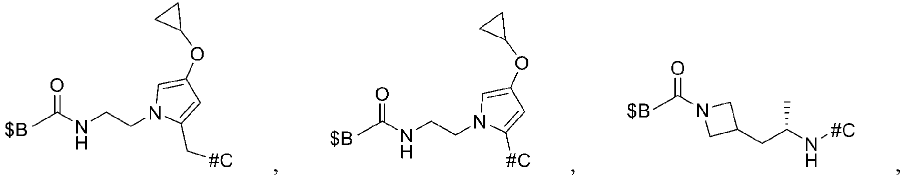 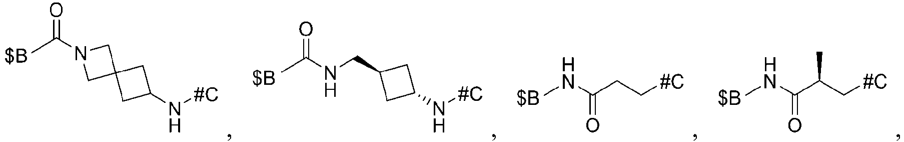 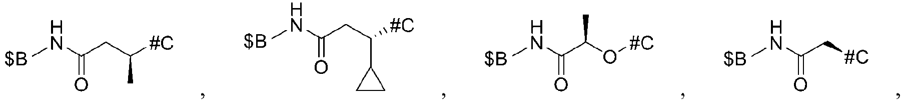 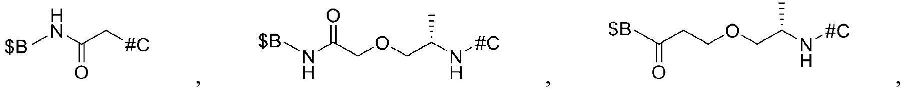 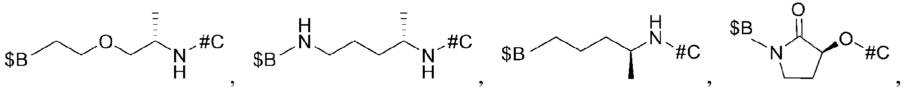 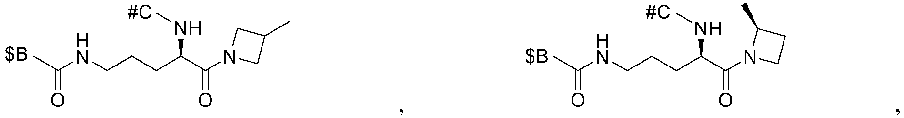 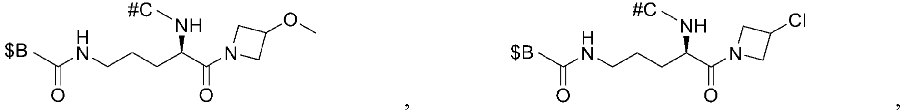 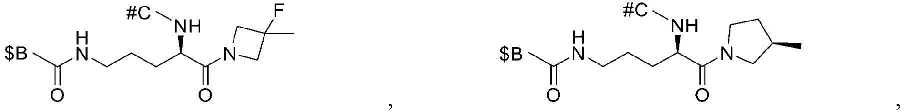 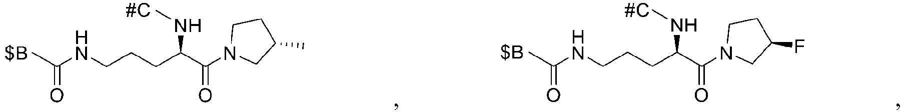 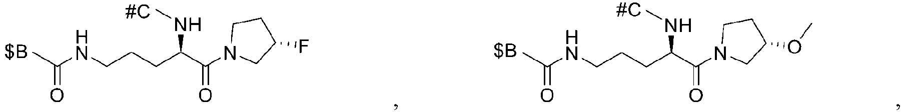 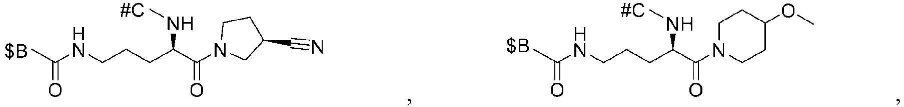 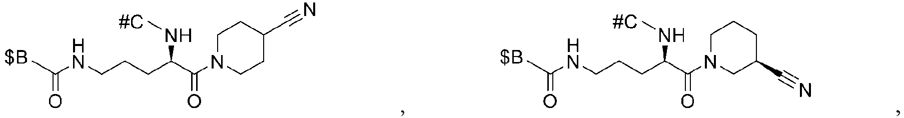 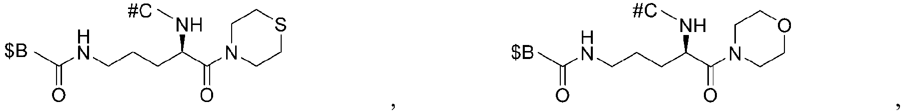 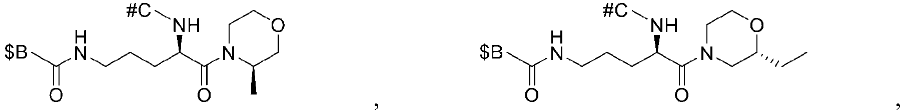 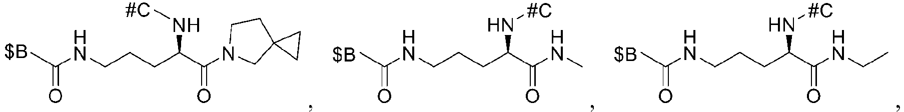 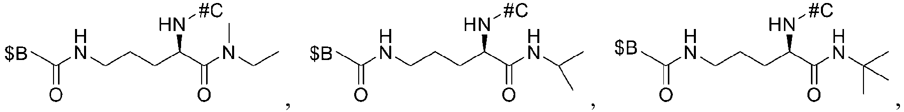 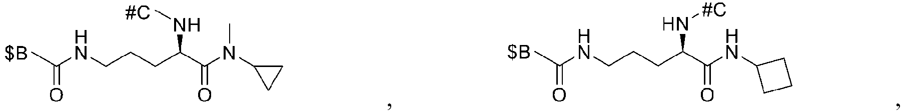 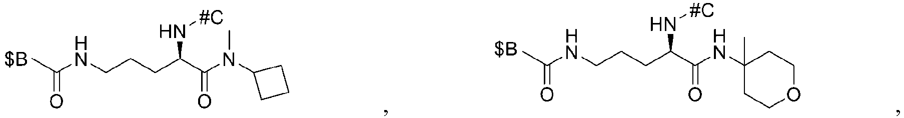 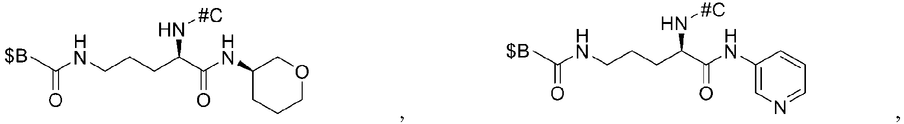 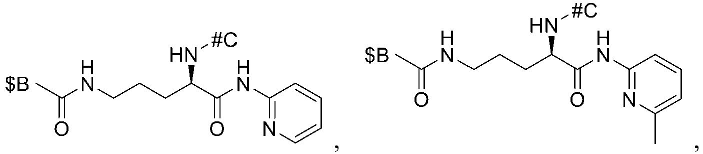 $B-CH₂-CH₂-#C, $B-CH₂-CH₂-CH₂-#C, 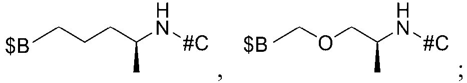 $B represents the site of attachment to ring B, #C represents the site of attachment to ring C;
preferably, L is selected from 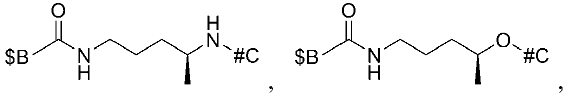 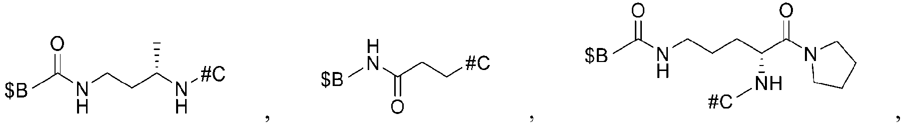 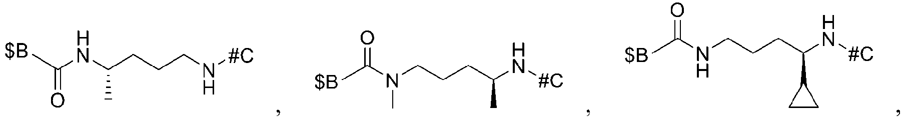 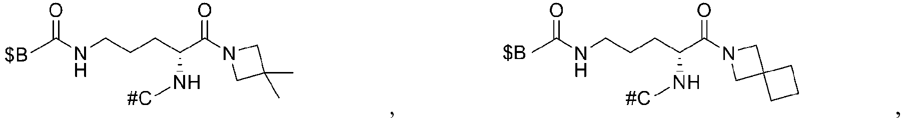 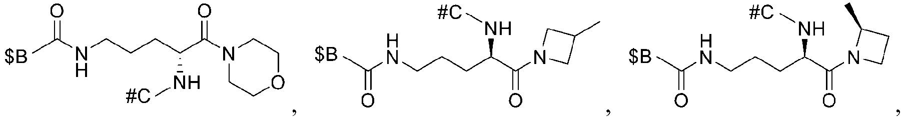 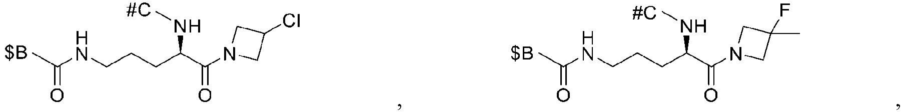 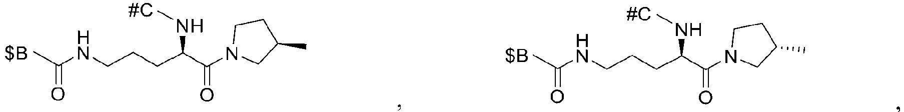 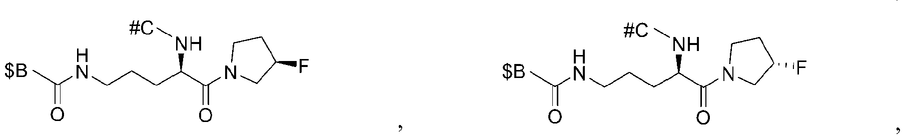 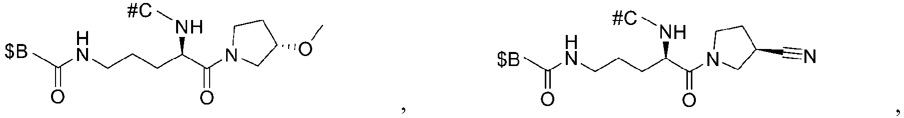 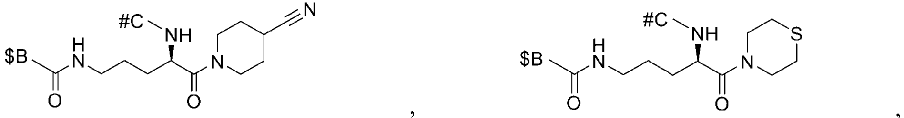 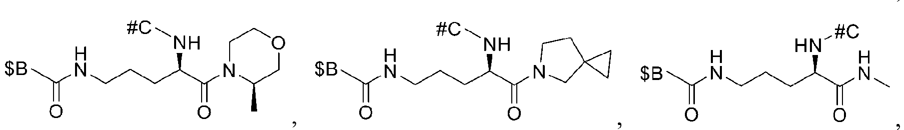 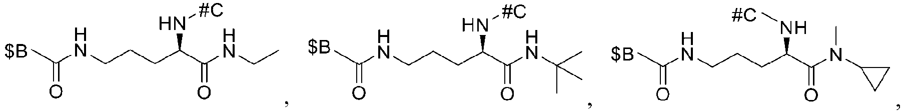 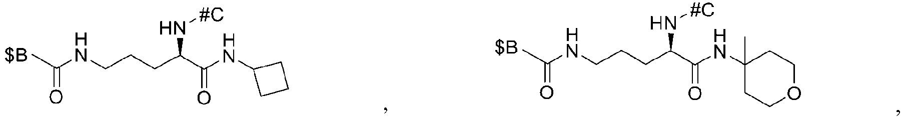 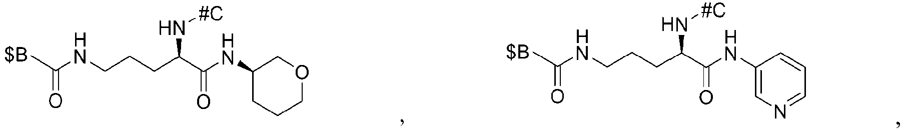 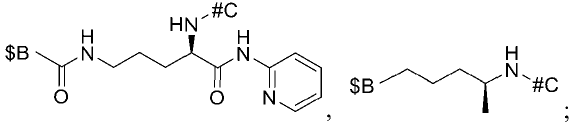
preferably, L is selected from 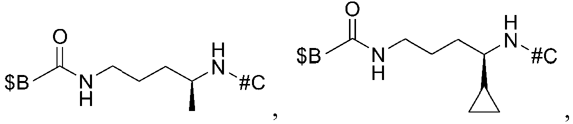 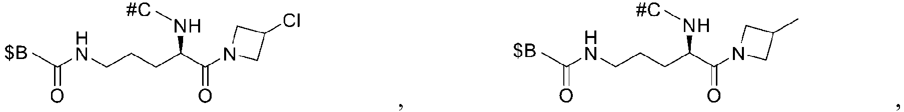 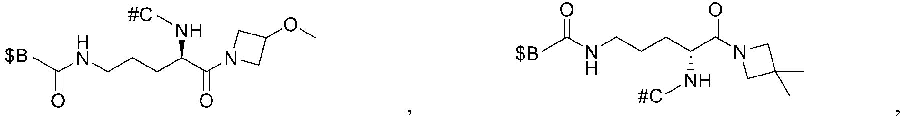 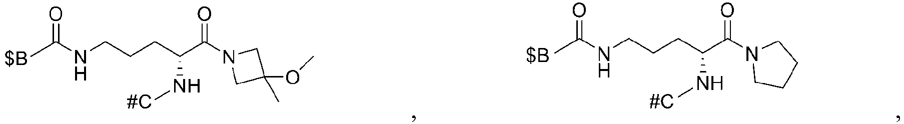 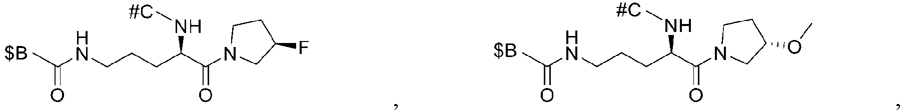 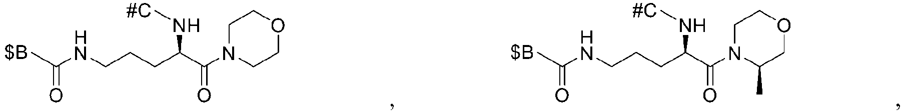 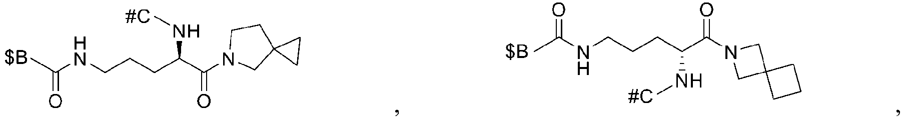 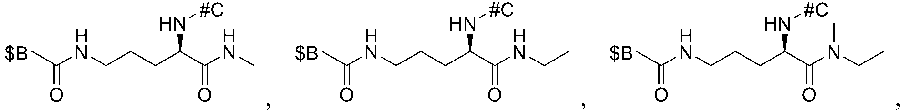 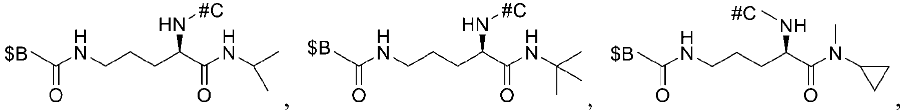 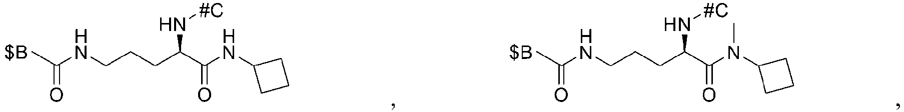 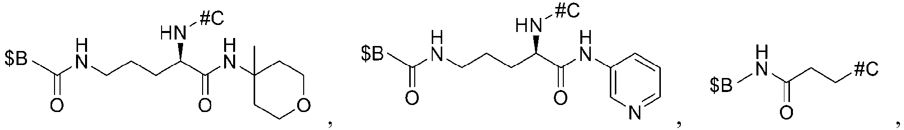 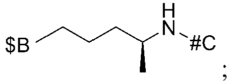
preferably, L is selected from

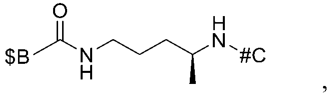

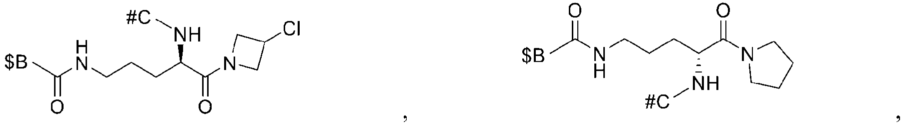

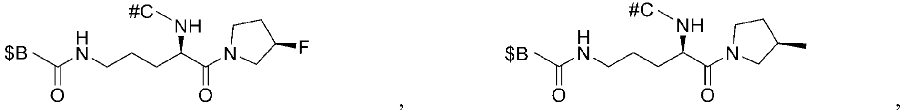

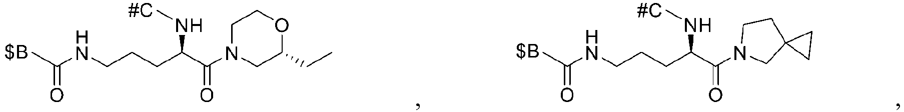

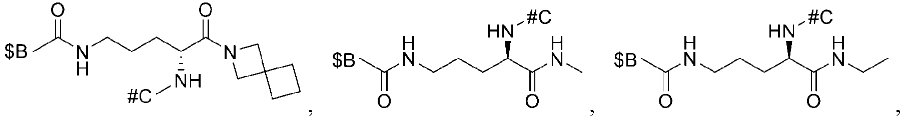

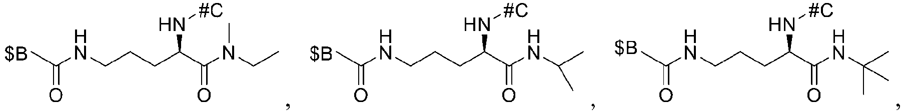

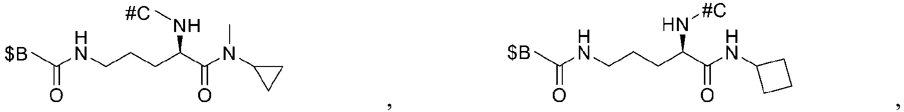

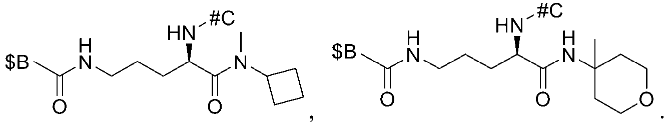

19. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 18, wherein:
ring C is selected from the following groups: 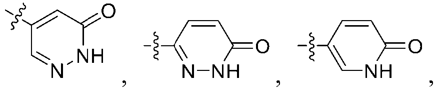 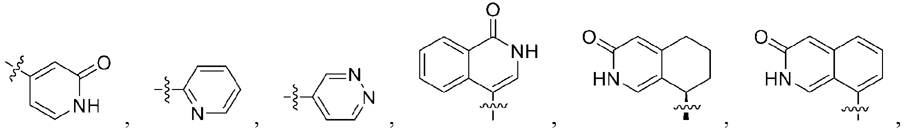 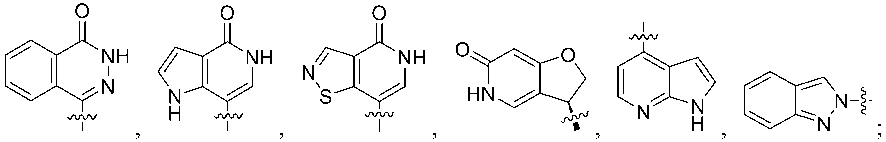
preferably, ring C is selected from: 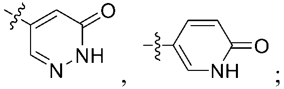
more preferably, ring C is selected from

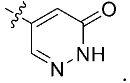

20. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 19, wherein: 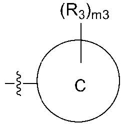 is selected from: 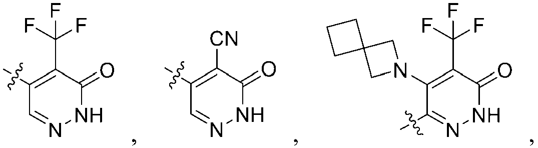 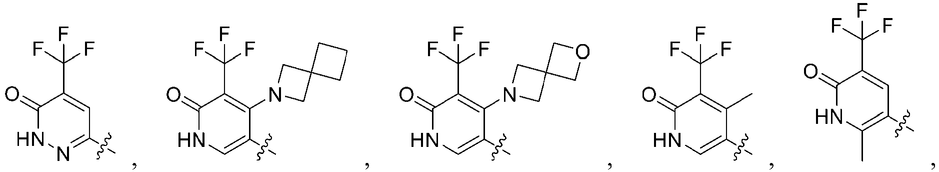 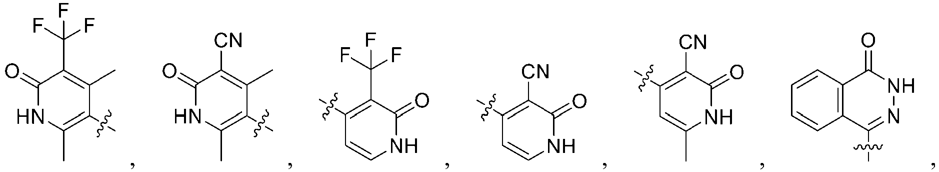 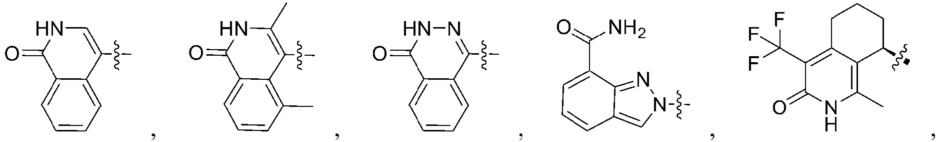 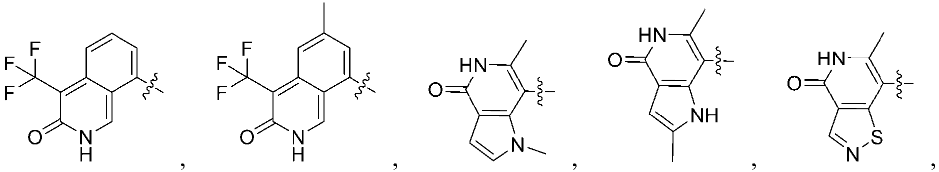 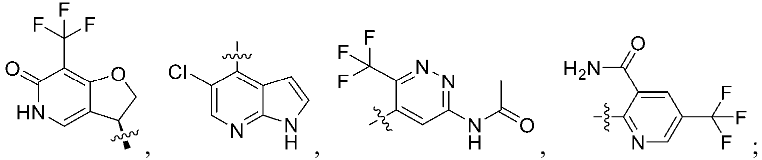
preferably, 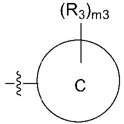 is selected from: 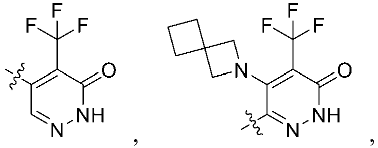 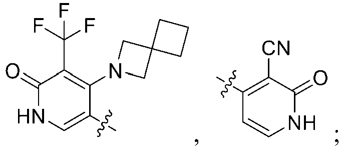
preferably, 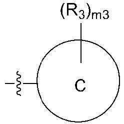 is selected from: 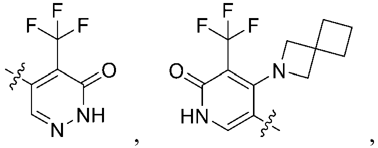 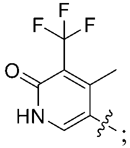
preferably, 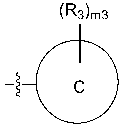 is selected from: 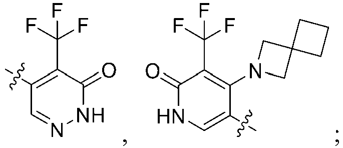
preferably, 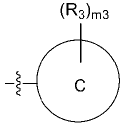 is selected from: 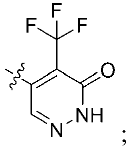
preferably, R₃ is selected from halogen, -CN, C1-3 alkyl, C1-3 haloalkyl, -NReRf, - NReC(=O)Rf, -C(=O)-NReRf, wherein, Re and Rf are each independently selected from H, C1-3 alkyl, or Re and Rf together with the atoms that they are attached to, form 4-7-membered heterocycloalkyl (for example 7-membered nitrogen-containing spiro heterocycloalkyl) ;
further preferably, R₃ is selected from Cl, methyl, -CF₃, -CN, -NHC(=O)CH₃, -C(=O)NH₂, 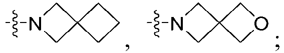
further preferably, R₃ is selected from -CF₃, -CN, 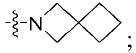
further preferably, R₃ is selected from -CF₃, 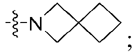
further preferably, R₃ is selected from -CF₃, methyl, 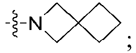 preferably, m3 is selected from 1, 2, 3; more preferably, m1 is selected from 1 or 2.

21. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 20, which has a structure as shown in any one of formulas I-1 to I-4, I-7 to I-10: 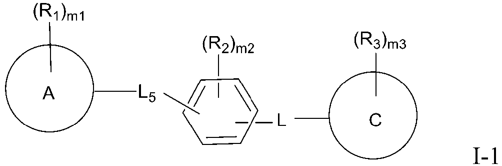 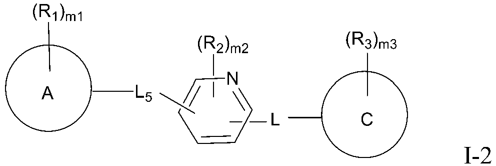 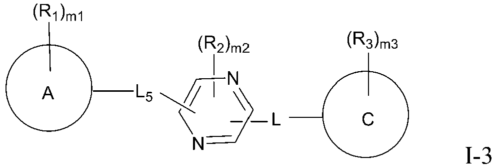 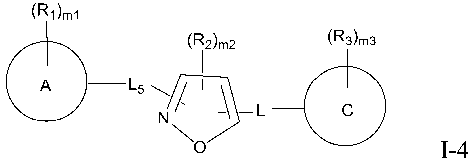 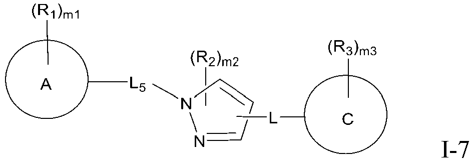 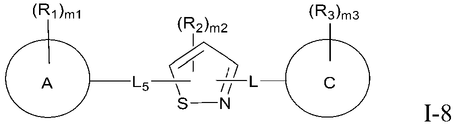 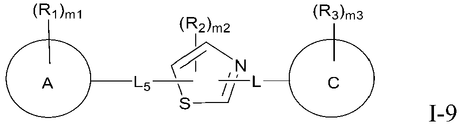 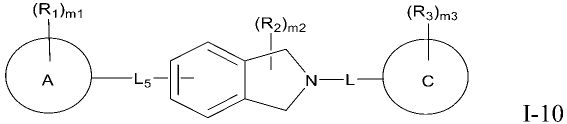 wherein, ring A, ring C, R₁, R₂, R₃, L, L₅, m1, m2, and m3 are as defined in claim 1 or 9.

22. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1-21, which has a structure as shown in formula I-5 or I-6: 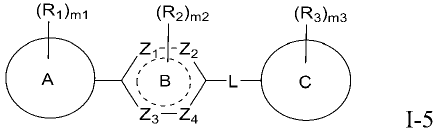 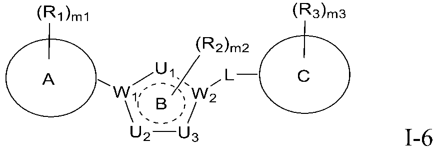 wherein,
in formula I-5, ring B is selected from phenyl or 6-membered heteroaryl, Z₁, Z₂, Z₃, and Z₄ are each independently selected from N or CH, ring A, ring C, R₁, R₂, R₃, L, m1, m2, and m3 are as defined in claim 1;
preferably, at most two of Z₁, Z₂, Z₃, and Z₄ are N;
preferably, ring B is selected from phenyl, pyrimidinyl, pyridinyl, pyrazinyl, pyridazinyl;
preferably, ring B is selected from phenyl, pyridinyl, pyrazinyl;
preferably, ring B is selected from the following groups 1) -5):
1) Z₁, Z₂, Z₃, and Z₄ are all CH;
2) Z₁ and Z₂ are both CH, Z₃ and Z₄ are both N;
3) Z₁ and Z₄ are both CH, Z₂ and Z₃ are both N;
4) only one of Z₁, Z₂, Z₃, and Z₄ is N;
5) Z₁ and Z₃ are both CH, one of Z₂ and Z₄ is N and the other is CH;
in formula I-6, ring B is selected from 5-membered heteroaryl, U₁, U₂, and U₃ are each independently selected from N, O, S, CH, and at most one of U₁, U₂, and U₃ is S, at most one of U₁, U₂, and U₃ is O, W₁ and W₂ are each independently selected from N, CH, ring A, ring C, R₁, R₂, R₃, L, m1, m2, and m3 are as defined in claim 1;
preferably, ring B contains 2 or 3 heteroatoms each independently selected from N, O, S; preferably, ring B is selected from pyrazolyl, thiazolyl, isothiazolyl, isoxazolyl, triazolyl (for example 1,2,3-triazolyl), thiadiazolyl (for example 1,3,4-thiadiazolyl);
preferably, at least one of U₁, U₂, and U₃ is N; at most one of W₁ and W₂ is N; preferably, at most two of U₁, U₂, and U₃ are heteroatoms;
preferably, U₁, U₂, and U₃ are each independently selected from N, O, CH;
preferably, U₁ and W₂ are CH; U₃ and W₁ are CH, N; U₂ is O, N;
preferably, ring B is selected from isoxazolyl, pyrazolyl;
preferably, U₁ is CH, U₂ is O, U₃ is N, W₁ is CH, W₂ is CH; or W₁ and U₂ are N, U₁, W₂, and U₃ are CH.

23. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1-22, which has a structure as shown in any one of formula II-1 or II-2: 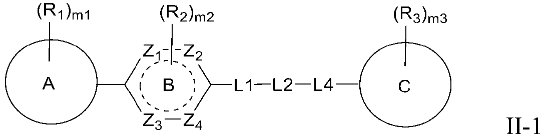 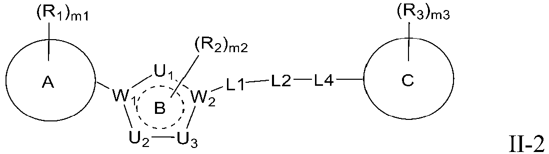
wherein, L1 is selected from $B-C(=O)-NRa-#L2, $B-NRb-C(=O)-#L2; Ra, Rb are as defined in claim 9;
L2 is selected from C1-6 alkylene, wherein, the C1-6 alkylene is optionally substituted by 1 or 2 substituents selected from oxo, C1-3 alkyl, C3-6 cycloalkyl, 4-7-membered nitrogen-containing saturated heterocycloalkyl, -C(=O)-4-7-membered nitrogen-containing saturated heterocycloalkyl, -C(=O)N(R_{L2})₂, the 4-7-membered nitrogen-containing saturated heterocycloalkyl, -C(=O)-4-7-membered nitrogen-containing saturated heterocycloalkyl, as a substituent of C1-6 alkylene, are optionally substituted by a substituent selected from halogen, cyano, C1-3 alkyl, C1-3 alkoxy, C1-3 haloalkyl, C1-3 haloalkoxy; each R_{L2} is independently selected from H, D, C1-6 alkyl, C3-10 cycloalkyl, 4-10-membered heterocycloalkyl, C6-10 aryl, 5-10-membered heteroaryl, and the C1-6 alkyl, C3-10 cycloalkyl, 4-10-membered heterocycloalkyl, C6-10 aryl, 5-10-membered heteroaryl are optionally substituted by a substituent selected from halogen, hydroxyl, cyano, C1-6 alkyl, C1-6 alkoxy;
L4 is selected from a bond, O, -NRc-, wherein, Rc is selected from H, C1-3 alkyl; preferably, L2 is selected from C2-5 alkylene; wherein, the C2-5 alkylene is optionally substituted by a substituent selected from C1-3 alkyl, C3-6 cycloalkyl (for example cyclopropyl, cyclobutyl, cyclohexyl), 4-7-membered heterocycloalkyl (for example oxetanyl, oxanyl, 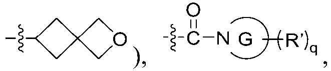 -C(=O)N(R_{L2})₂; wherein, ring G is 4-10-membered nitrogen-containing saturated heterocycloalkyl (for example azetidinyl, azacyclopentyl, morpholinyl, 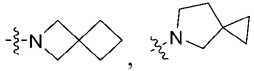 ), R' is selected from halogen, cyano, C1-3 alkyl, C1-3 haloalkyl, C1-3 alkoxy, C1-3 haloalkoxy, q is selected from 0, 1, or 2; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 5-6-membered heteroaryl (for example pyridinyl), and the C1-3 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy.

24. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1-21, which has a structure as shown in any one of formulas III-1 to III-6: 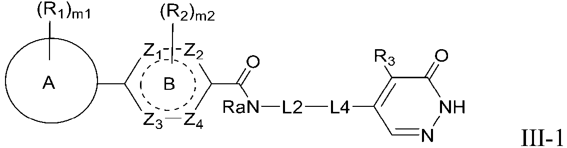 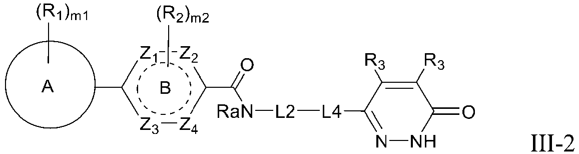 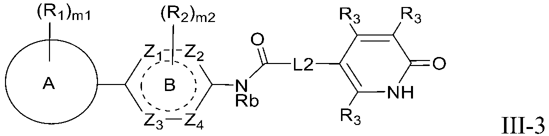 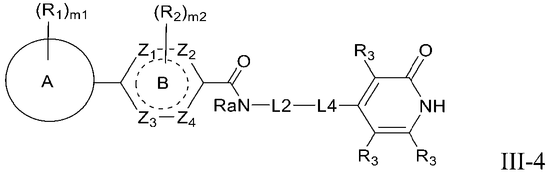 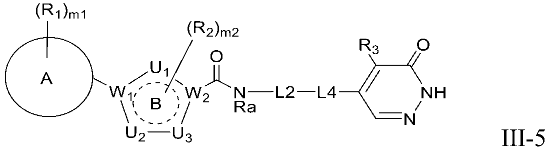 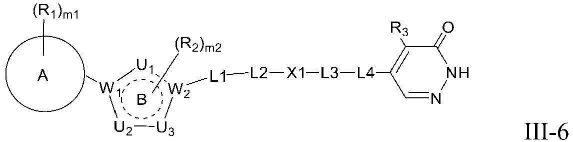 wherein, Z₁, Z₂, Z₃, Z₄, U₁, U₂, U₃, W₁, W₂ are as defined in claim 22; L1, L2, L3, L4, Ra, Rb are as defined in claim 9; ring A, R₁, R₂, R₃, m1, m2, and m3 are as defined in claim 1.

25. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 24, which has a structure as shown in any one of formulas IV-1 to IV-4: 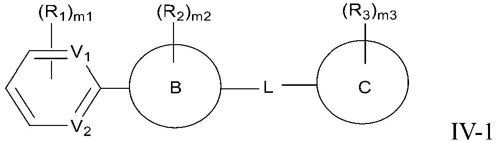 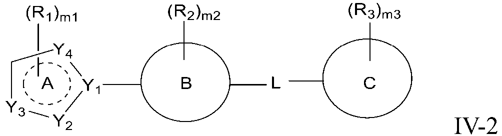 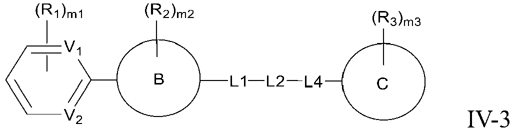 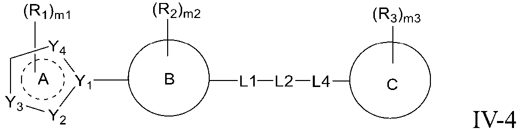
wherein, V₁ and V₂ are each independently selected from N, CH, Y₁ is N or CH, Y₂, Y₃, and Y₄ are each independently selected from N, CH, S, ring A is 5-membered heteroaromatic ring, ring B, ring C, L, R₁, R₂, R₃, m1, m2, and m3 are as defined in claim 1; L1, L2, and L4 are as defined in claim 9;
preferably, at least one of V₁ and V₂ is N; more preferably, V₁ and V₂ are both N; preferably, only two of Y₁, Y₂, Y₃, and Y₄ are heteroatoms;
preferably, Y₂ is N, one of Y₁, Y₃, and Y₄ is a heteroatom;
preferably, Yi and Y₂ are N, Y₃ and Y₄ are CH; or Y₁ and Y₃ are CH, Y₂ and Y₄ are S and N, respectively; or Y₁ and Y₄ are CH, Y₂ is N, Y₃ is S.

26. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 1 or 2, which has a structure as shown in any one of formulas V-1 to V-4: 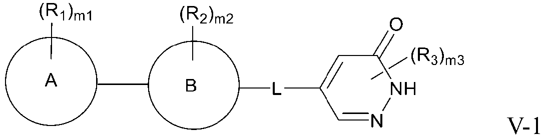 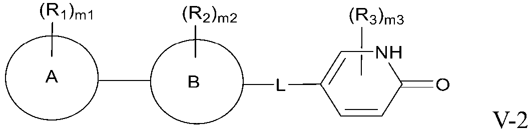 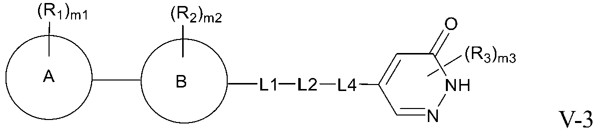 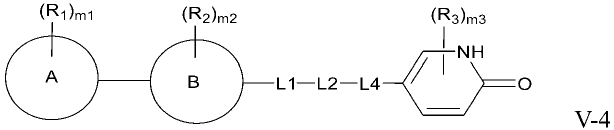 wherein, ring A, ring B, ring C, L, R₁, R₂, R₃, m1, m2, and m3 are as defined in claim 1; L1, L2, and L4 are as defined in claim 9.

27. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 26, which has a structure as shown in any one of formulas VI-1 to VI-6: 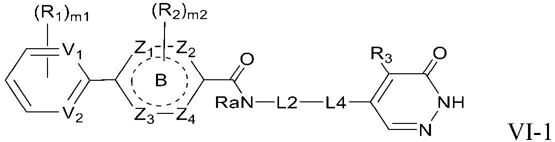 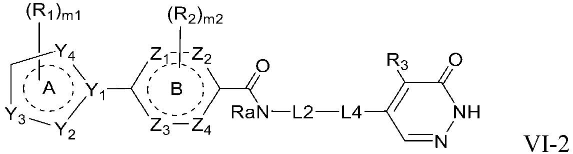 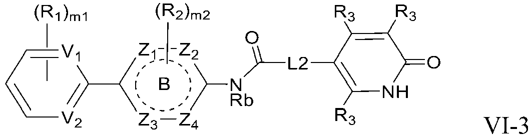 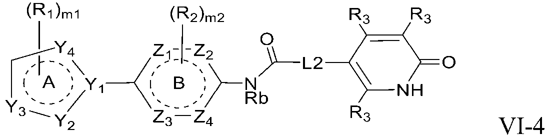 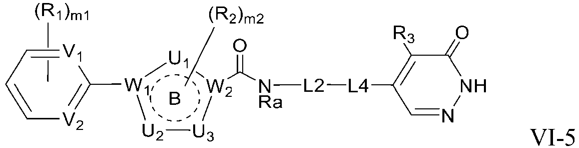 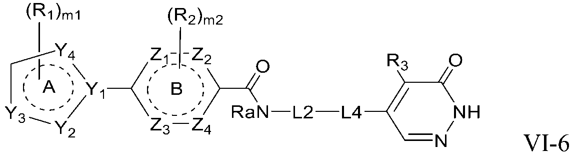 wherein, L1, L2, L3, L4, Ra, and Rb are as defined in claim 9; Z₁, Z₂, Z₃, Z₄, U₁, U₂, U₃, W₁, and W₂ are as defined in claim 22; ring A, V₁, V₂, Y₁, Y₂, Y₃, and Y₄ are as defined in claim 25.

28. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 1, wherein: 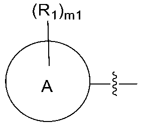 is selected from 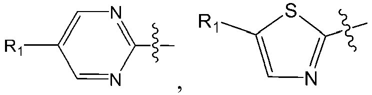 ; and/or 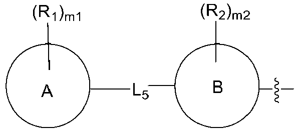 is selected from: 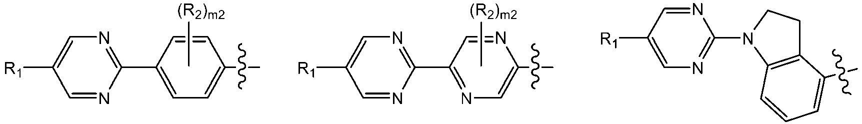 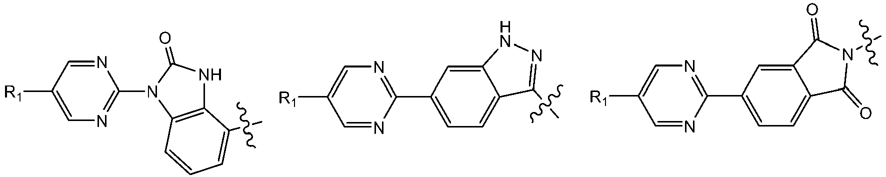 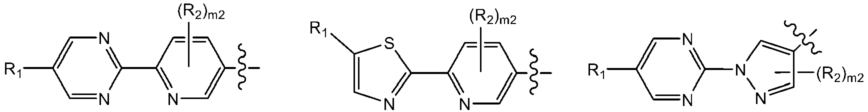 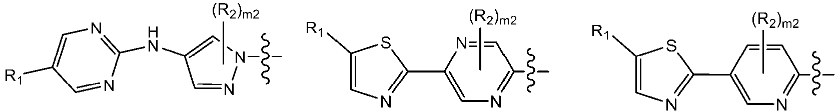 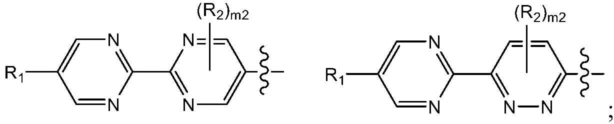 and/or 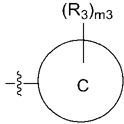 is selected from 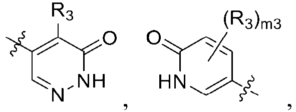
wherein, R₁ is selected from halogen, -OH, -CN, -NReRf, -CF₃, -CHF₂, substituted or unsubstituted C3-6 cycloalkyl, Re and Rf are each independently selected from H, substituted or unsubstituted C1-6 alkyl, substituted or unsubstituted C3-6 cycloalkyl, or Re and Rf together with the atoms that they are attached to, form 4-7-membered heterocycloalkyl;
each R₂ is independently selected from halogen, -OH, -CN, substituted or unsubstituted C1-6 alkyl, substituted or unsubstituted C1-6 alkoxy, substituted or unsubstituted C3-6 cycloalkyl;
each R₃ is independently selected from halogen, -OH, -CN, -NReRf, -CF₃, -CHF₂, substituted or unsubstituted C3-6 cycloalkyl, Re and Rf are each independently selected from H, substituted or unsubstituted C1-6 alkyl, substituted or unsubstituted C3-6 cycloalkyl, or Re and Rf together with the atoms that they are attached to, form 4-7-membered heterocycloalkyl; and/or
m2 and m3 are each independently selected from 0, 1, 2, or 3.

29. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 1, wherein,
ring A is selected from phenyl, pyrimidinyl, thiazolyl, isothiazolyl, imidazolyl, pyrazolyl, pyridinyl, pyridazinyl, oxazolyl, isoxazolyl, 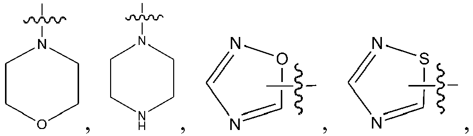 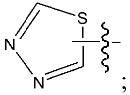
ring B is selected from phenyl, pyridinyl, pyrazolyl, pyrazinyl, oxazolyl, isoxazolyl, benzopyrazolyl, thiazolyl, isothiazole, pyridazinyl, pyrimidinyl, triazolyl, thiadiazole, indolinyl, benzimidazolyl, dihydrobenzimidazolyl, indolinyl, indazolyl, isoindolinyl, pyridopyridinyl, pyrrolopyrimidinyl;
ring C is selected from the following groups: 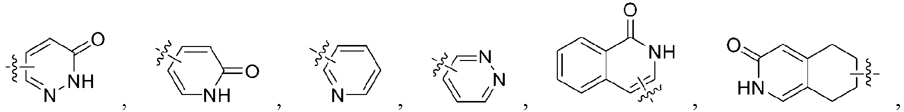 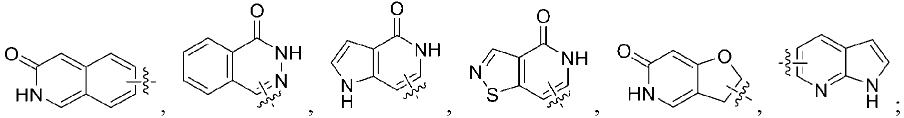 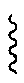 terminus is attached to L; L₅ is selected from a bond; and
L is selected from: 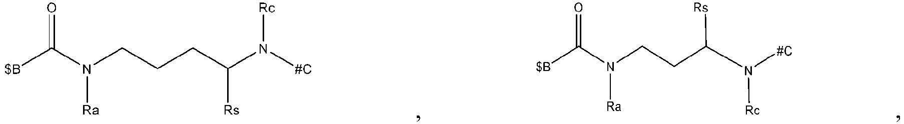 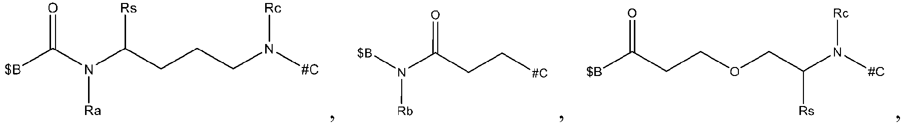 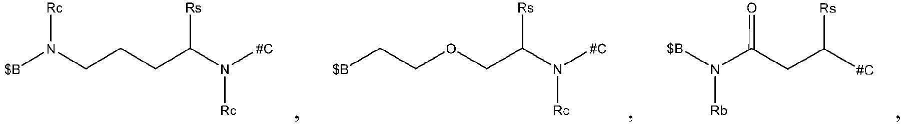 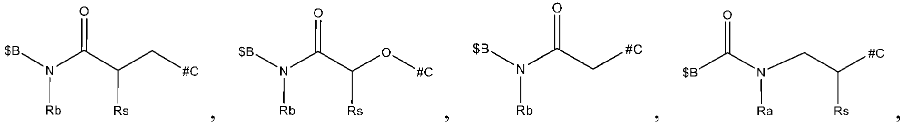 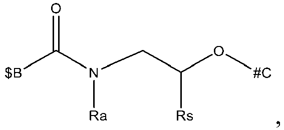
wherein,
$B represents the site of attachment to ring B, #C represents the site of attachment to ring C, Ra, Rb, and Rc are as defined in claim 9, Rs is as defined in claim 11;
preferably, Ra, Rb, and Rc are each independently selected from H or methyl;
preferably, Rs is selected from H or the following groups: methyl, cyclopropyl, cyclobutyl, cyclopentyl, cyclohexyl,

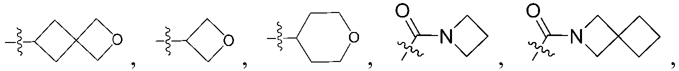

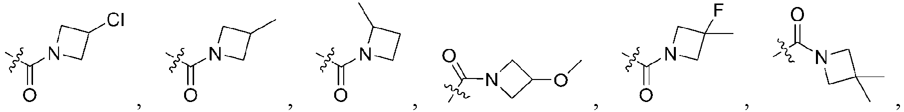

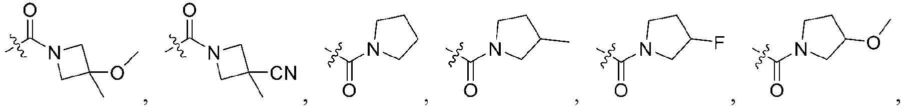

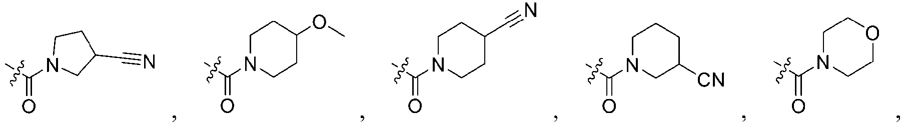

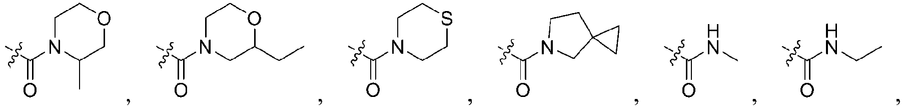

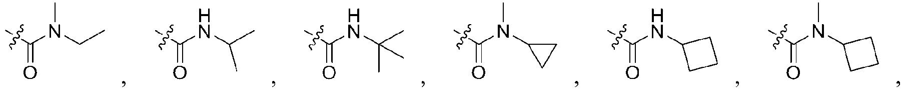

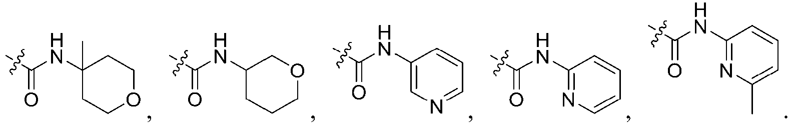

30. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 1, wherein,
ring A is selected from pyrimidinyl, thiazolyl, isothiazolyl, pyrazolyl, pyridinyl;
ring B is selected from: 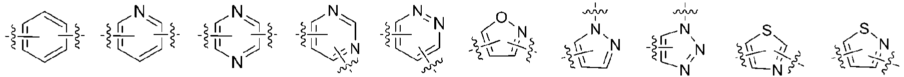 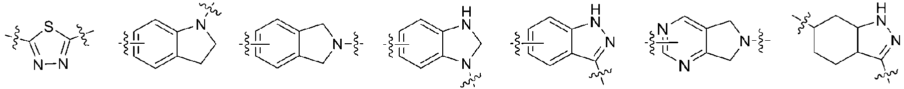 ; 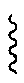 terminus is attached to L terminus or L₅;
ring C is selected from: 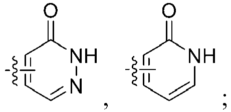
L₅ is a bond;
L is selected from: 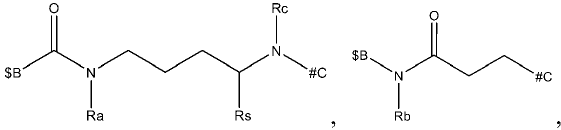 $B represents the site of attachment to ring B, #C represents the site of attachment to ring C;
Ra, Rb, and Rc are as defined in claim 9, Rs is as defined in claim 11;
preferably, Ra, Rb, and Rc are each independently selected from H or methyl;
preferably, Rs is selected from H or the following groups: methyl, cyclopropyl, cyclobutyl, cyclohexyl,

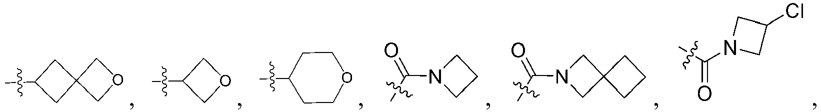

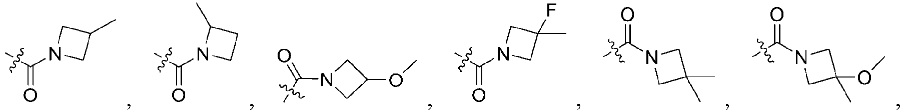

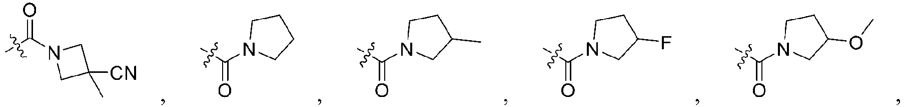

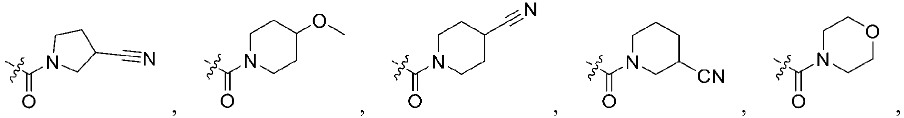

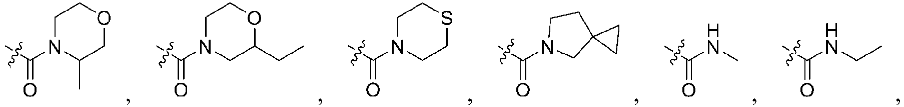

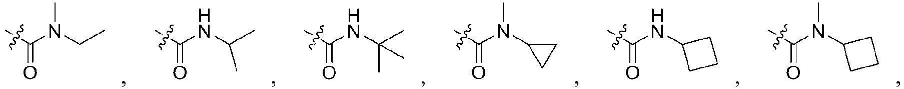

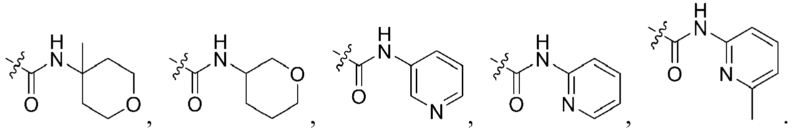

31. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 30, wherein:
m1 is selected from 1, 2, or 3, and each R₁ is independently selected from halogen, -OH, - CN, -NReRf, -C(=O)-NReRf, C1-6 alkyl, C2-6 alkynyl, C1-6 alkoxy, C1-6 haloalkyl, C1-6 haloalkoxy, C3-6 cycloalkyl, -C1-6 alkylene-C3-6 cycloalkyl, 5-6-membered heteroaryl, 4-7-membered heterocycloalkyl, wherein, the C3-6 cycloalkyl, -C1-6 alkylene-C3-6 cycloalkyl, 4-7-membered heterocycloalkyl, 5-6-membered heteroaryl are optionally substituted by 1 to 3 substituents selected from halogen, C1-3 alkyl; Re and Rf are each independently selected from H, C1-3 alkyl, or Re and Rf together with the atoms that they are attached to, form 4-7-membered heterocycloalkyl; preferably, each R₁ is independently selected from C1-3 haloalkyl (for example -CF₃), cyclopropyl, 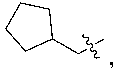 -N(CH₃)₂; and/or
m2 is selected from 0 or 1, and each R₂ is independently selected from -OH, halogen, C1-3 alkyl, C1-3 haloalkyl, C1-3 alkoxy, C1-3 haloalkoxy, preferably -F, hydroxyl; and/or
m3 is selected from 1, 2, or 3, and each R₃ is independently selected from -CN, C1-3 alkyl, C1-3 haloalkyl (for example -CF₃), -NReRf, -C(=O)-NReRf; wherein, Re and Rf are each independently selected from H, C1-3 alkyl, or Re and Rf together with the atoms that they are attached to, form 4-7-membered heterocycloalkyl (for example 7-membered nitrogen-containing spiro heterocycloalkyl) .

32. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 31, wherein:
at least one of the R₁ is selected from C1-6 haloalkyl, and/or at least one of the R₃ is selected from C1-6 haloalkyl;
preferably, at least one of the R₁ is selected from C1-3 haloalkyl, and/or at least one of the R₃ is selected from C1-3 haloalkyl;
preferably, at least one of the R₁ is trifluoromethyl, and/or at least one of the R₃ is trifluoromethyl.

33. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 31, which has a structure as shown in formula VII-1 or VII-2: 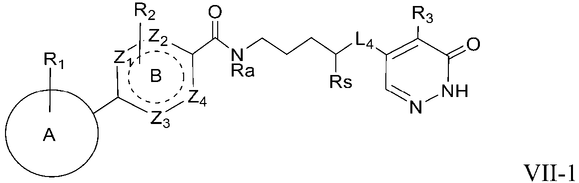 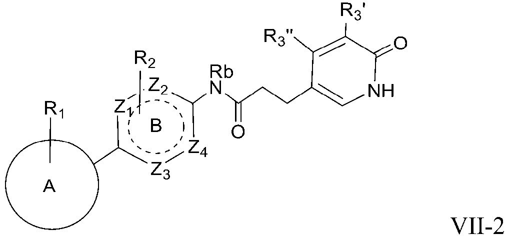 wherein, 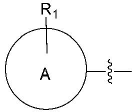 is selected from 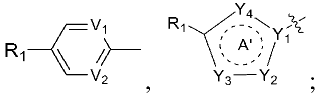
V₁ and V₂ are each independently selected from CH, N; preferably, at least one of V₁ and V₂ is N;
ring A' is 5-membered heteroaromatic ring, Yi is N or CH, Y₂, Y₃, and Y₄ are each independently selected from N, CH, S; preferably, Y₁ and Y₂ are N, Y₃ and Y₄ are CH; or Y₁ is CH, Y₂ is N, one of Y₃ and Y₄ is S and the other is CH; more preferably, Yi and Y₃ are CH, Y₂ is N, Y₄ is S;
R₁ is as defined in claim 1, 3, 28, or 31; preferably, R₁ is selected from C1-3 haloalkyl, - C1-3 alkylene-C3-6 cycloalkyl, -NReRf; Re and Rf are each independently selected from H, C1-3 alkyl; more preferably, R1 is -CF₃;
ring B is phenyl or 6-membered heteroaromatic ring, Z₁, Z₂, Z₃, and Z₄ are each independently selected from N or CH; preferably, ring B is phenyl, or only one of Z₁, Z₂, Z₃, and Z₄ is N, or Z₁ and Z₄ are both CH, Z₂ and Z₃ are both N;
R₂ is as defined in claim 1, 7, 28, or 31; preferably, R₂ is selected from H, halogen, hydroxyl, C1-3 alkyl; more preferably, R₂ is selected from H or F; more preferably, with the proviso that: when Z₁ and Z₄ are both CH, Z₂ and Z₃ are both N, then R₂ is H; ring B is phenyl, or when one of Z₂ or Z₄ is N, Z₁ and Z₃ are both CH, then R₂ is H or F, and F is at the ortho position relative to the -CONRa- in formula VII-1; more preferably, R₂ is H;
Ra, Rb are each independently selected from H or methyl; preferably, Ra is H; preferably, Rb is H;
Rs is as defined in claim 11, 12, 16, or 17, preferably, Rs is selected from C1-3 alkyl, C3-6 cycloalkyl (for example cyclopropyl, cyclobutyl, cyclohexyl), 4-7-membered heterocycloalkyl (for example oxetanyl, oxanyl, 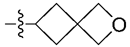 ), 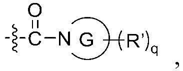 C(=O)N(R_{L2})₂; wherein, ring G is 4-10-membered nitrogen-containing saturated heterocycloalkyl (for example azetidinyl, azacyclopentyl, morpholinyl, 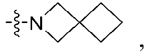 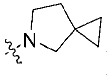
), R' is selected from halogen (for example F, Cl), cyano, C1-3 alkyl (for example methyl, isopropyl), C1-3 haloalkyl, C1-3 alkoxy, C1-3 haloalkoxy, q is selected from 0, 1, or 2; each R_{L2} is independently selected from H, C1-3 alkyl (for example methyl, ethyl, isopropyl), C3-6 cycloalkyl (for example cyclopropyl, cyclobutyl), 4-7-membered heterocycloalkyl (for example oxanyl), 5-6-membered heteroaryl (for example pyridinyl), and the C1-3 alkyl, C3-6 cycloalkyl, 4-7-membered heterocycloalkyl, 5-6-membered heteroaryl are optionally substituted by a substituent selected from halogen, hydroxyl, cyano, C1-3 alkyl, C1-3 alkoxy;
L4 is selected from NH or O, preferably NH;
R₃ is as defined in claim 1, 20, 28, or 31, preferably, R₃ is selected from C1-3 haloalkyl; more preferably, R₃ is selected from -CF₃;
R₃' and R₃'' are each independently R₃; preferably, R₃' is selected from C1-3 haloalkyl, R₃" is selected from -NReRf, and Re and Rf together with the atoms that they are attached to, form 4-7-membered heterocycloalkyl (for example 7-membered nitrogen-containing spiro heterocycloalkyl); more preferably, R₃' is -CF₃, R₃" is

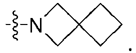

34. The compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of claim 1, wherein, the compound is selected from the following compounds:

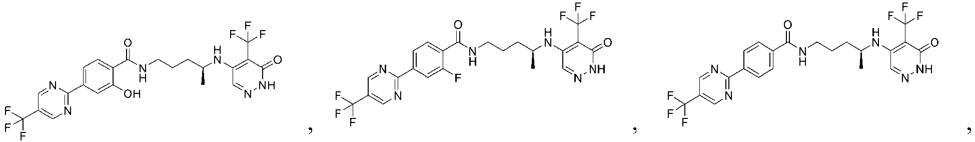

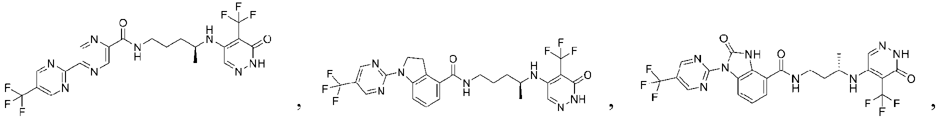

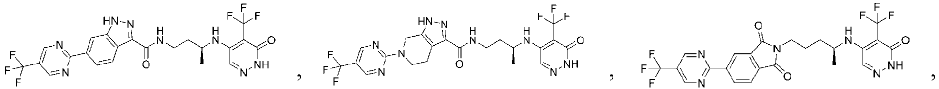

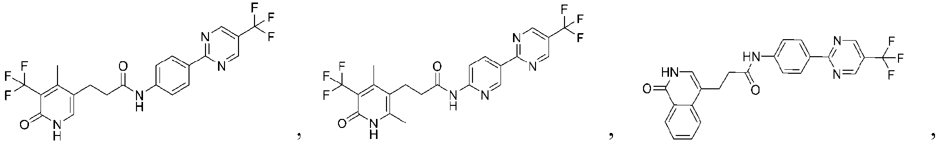

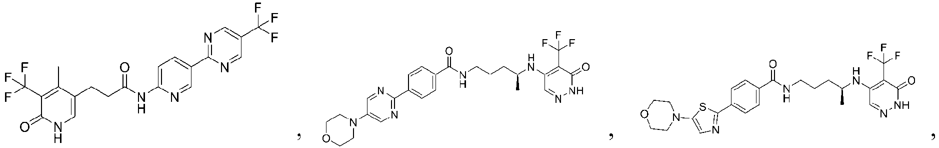

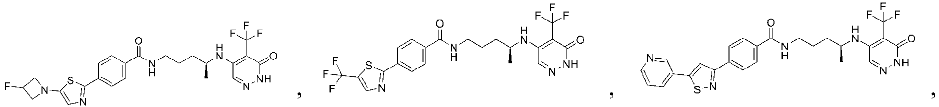

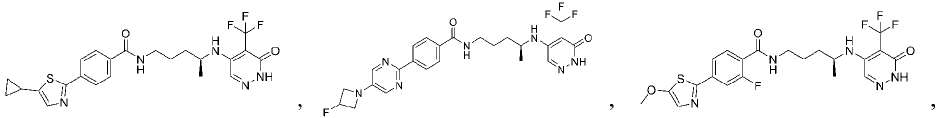

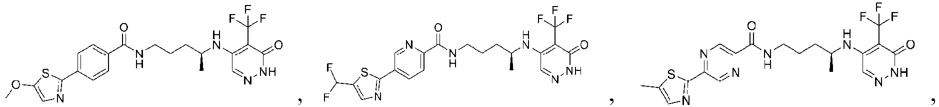

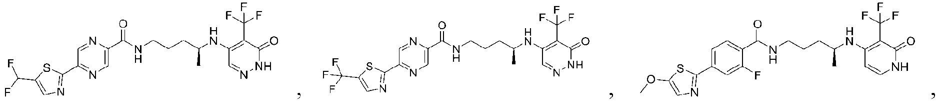

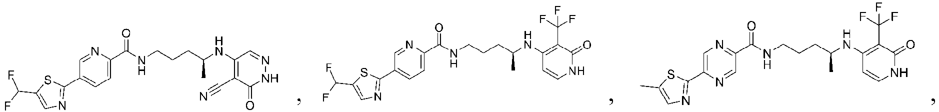

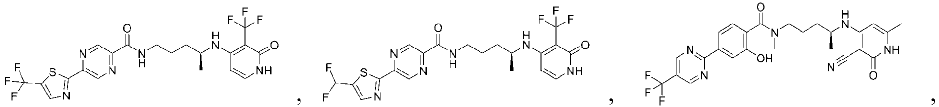

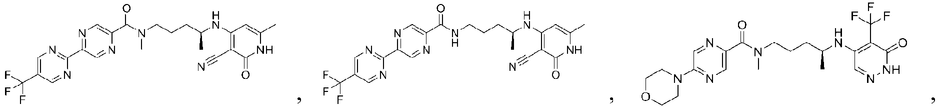

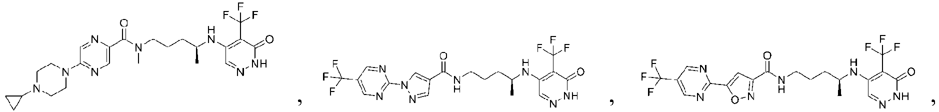

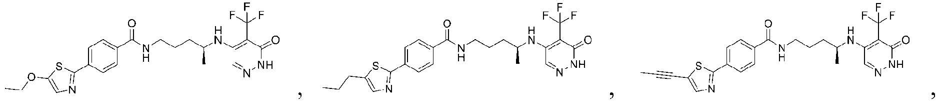

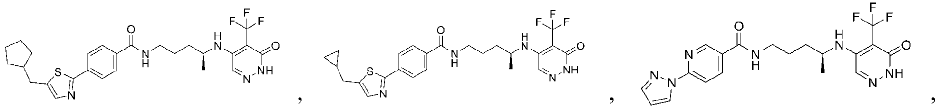

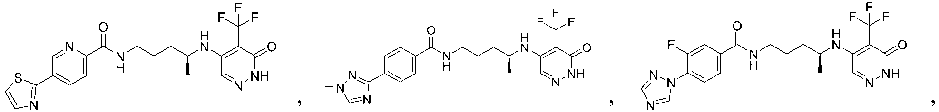

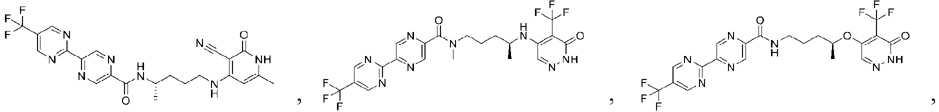

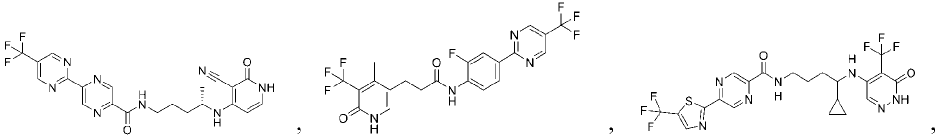

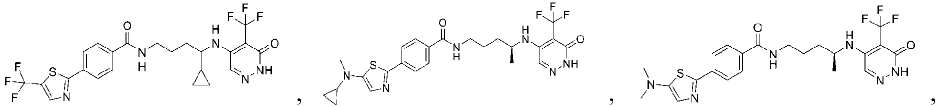

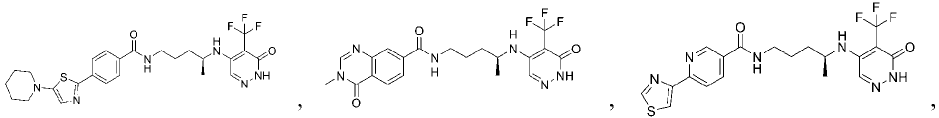

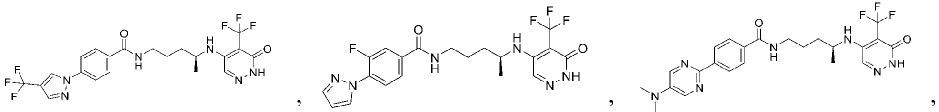

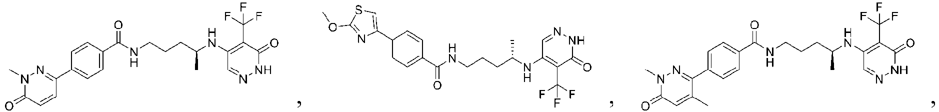

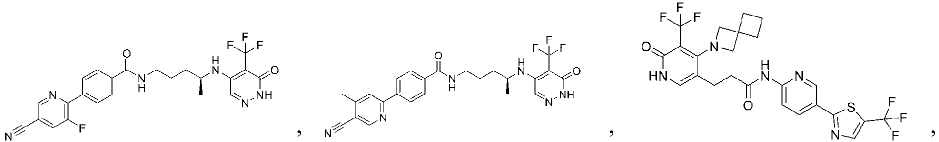

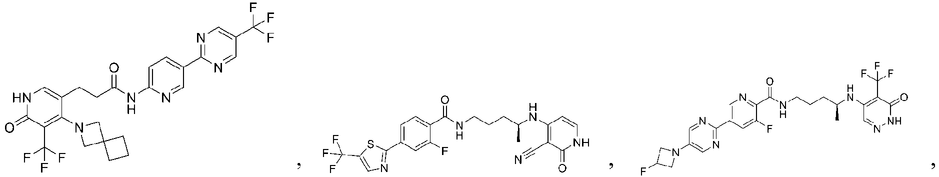

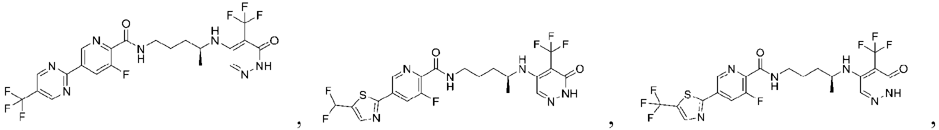

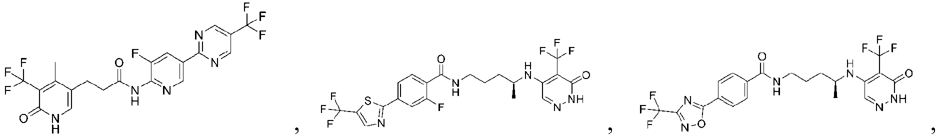

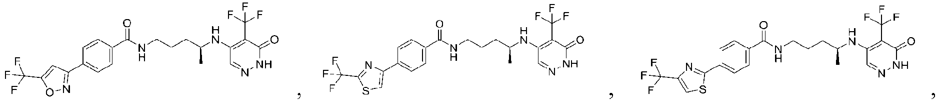

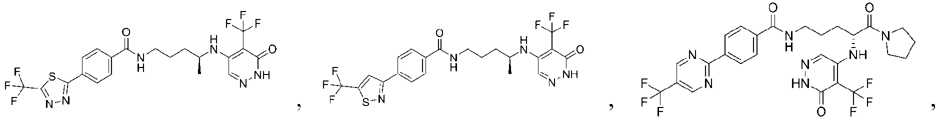

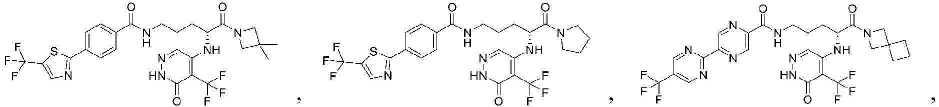

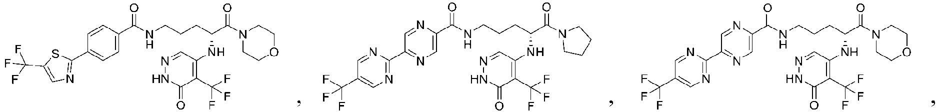

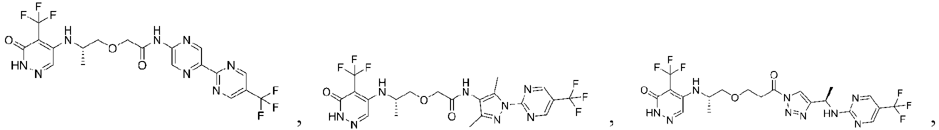

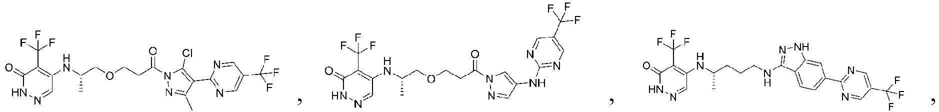

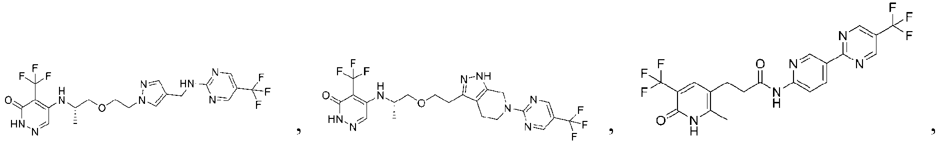

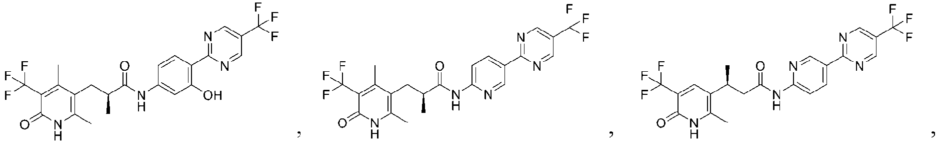

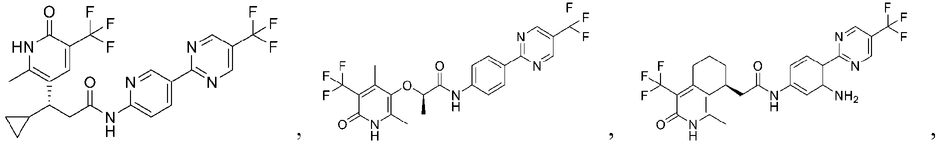

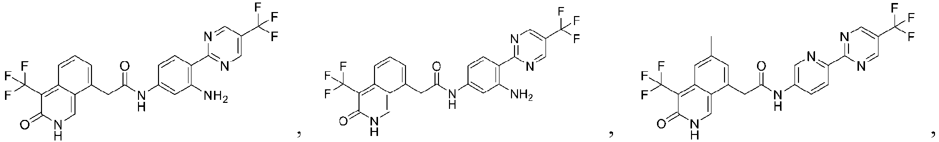

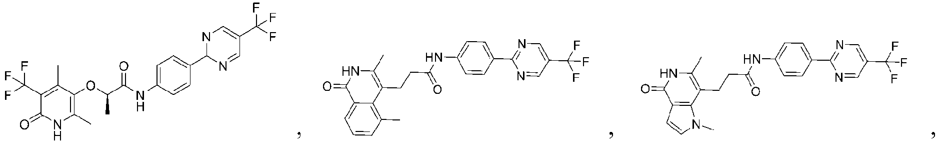

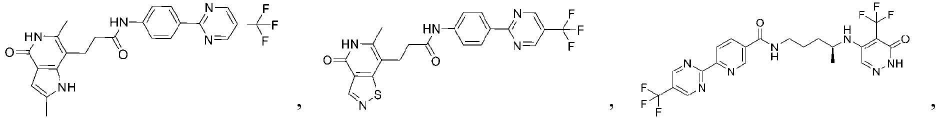

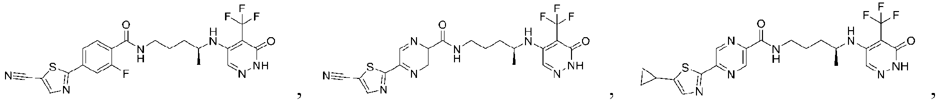

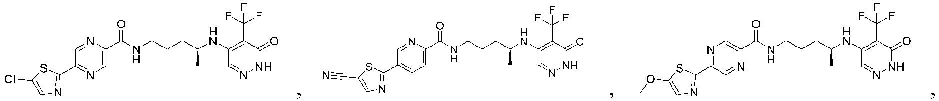

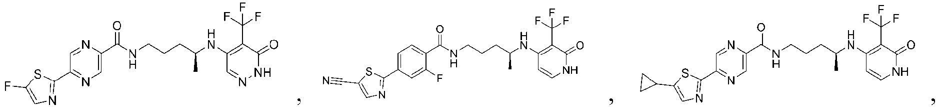

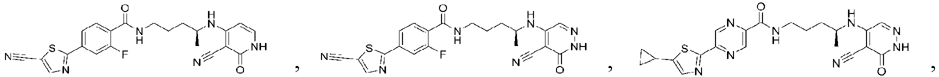

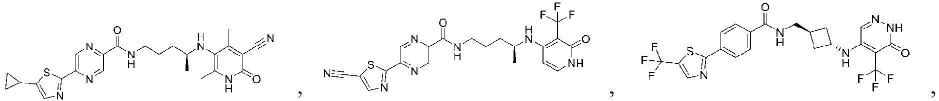

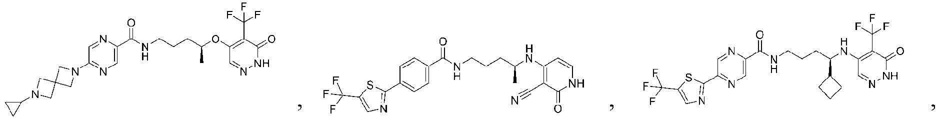

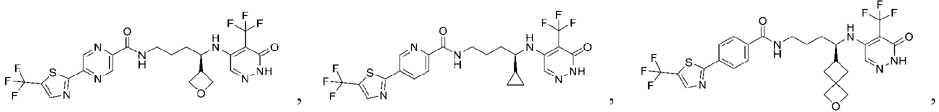

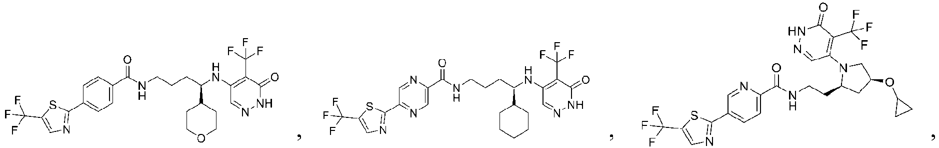

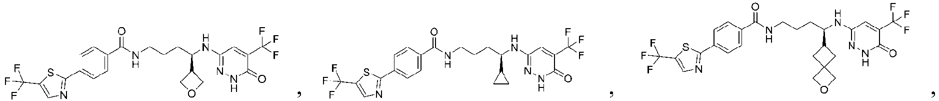

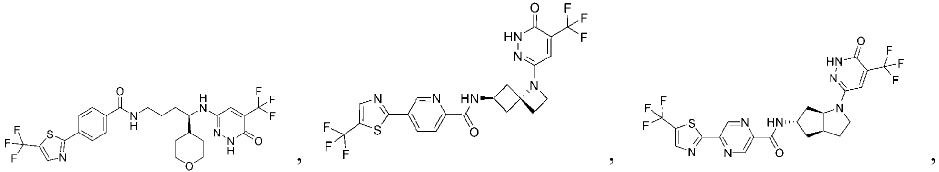

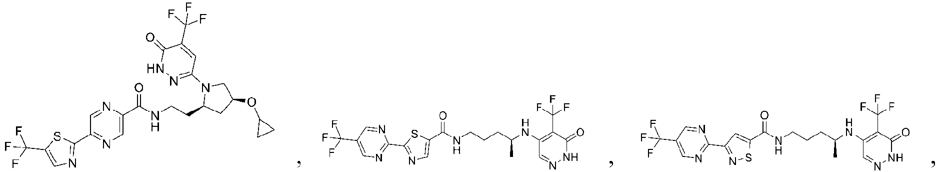

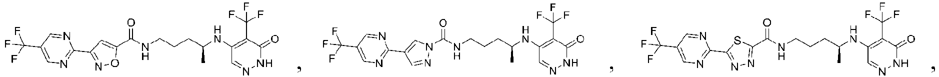

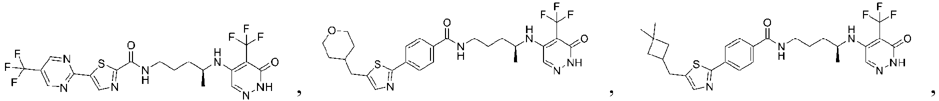

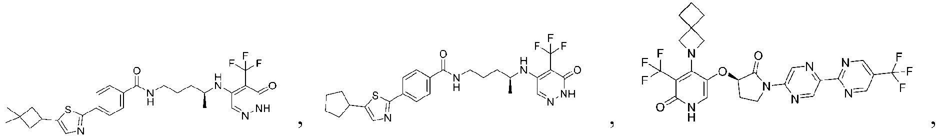

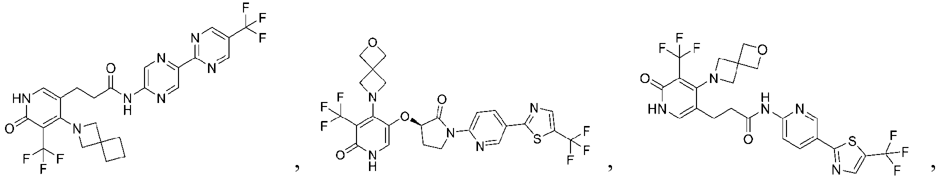

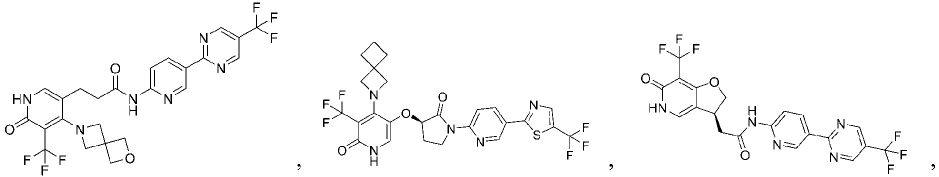

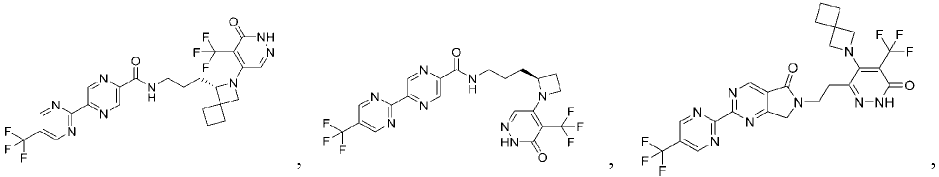

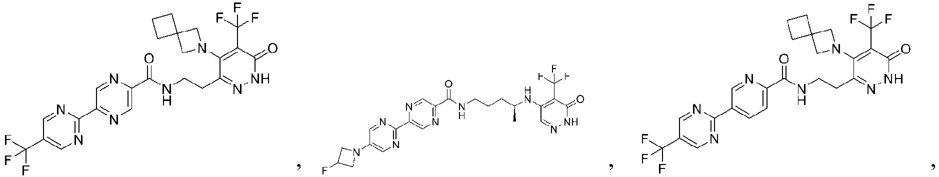

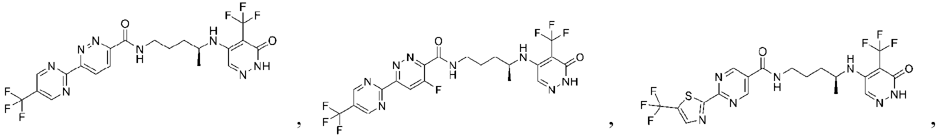

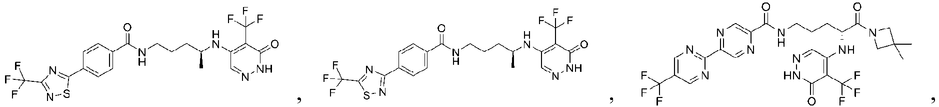

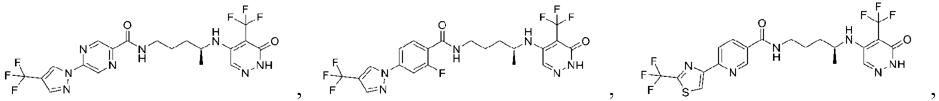

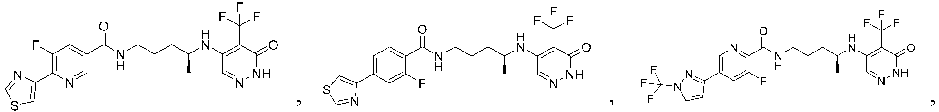

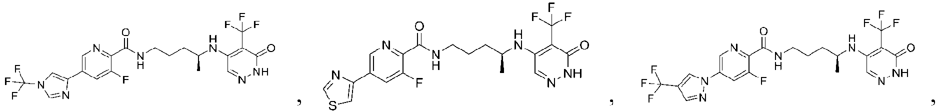

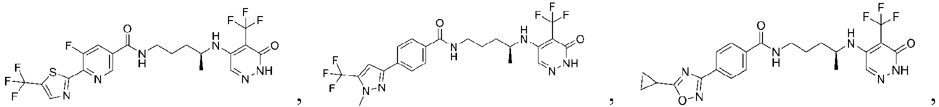

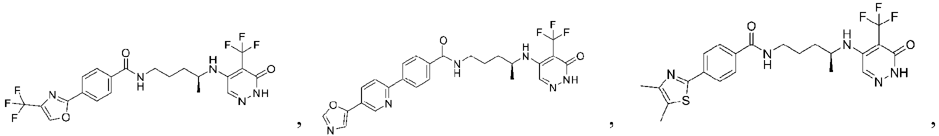

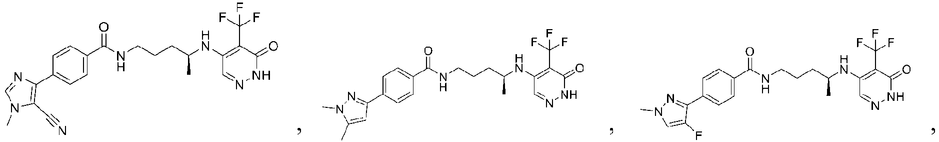

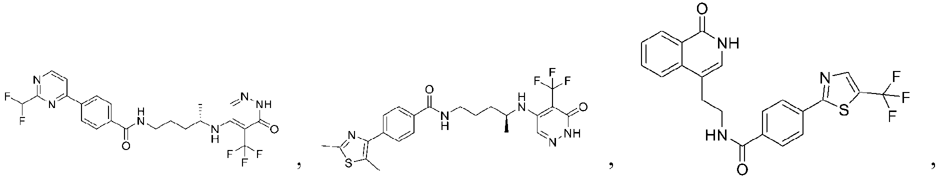

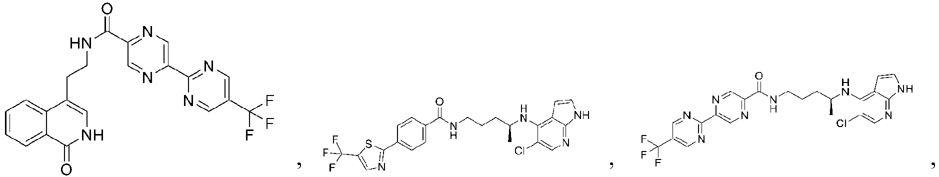

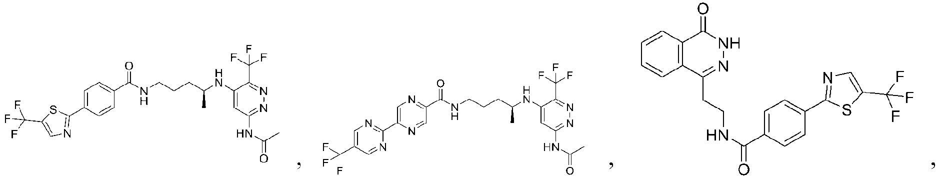

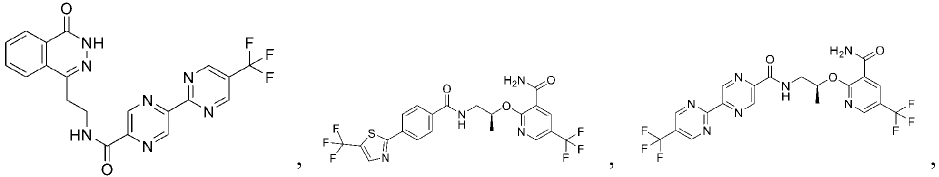

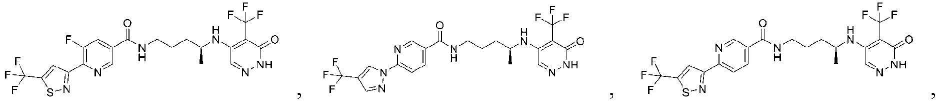

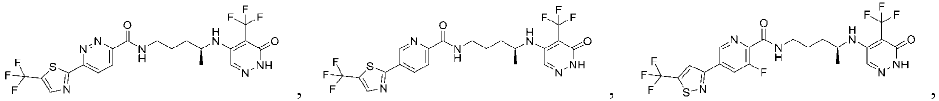

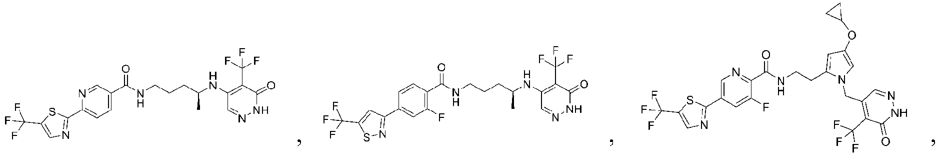

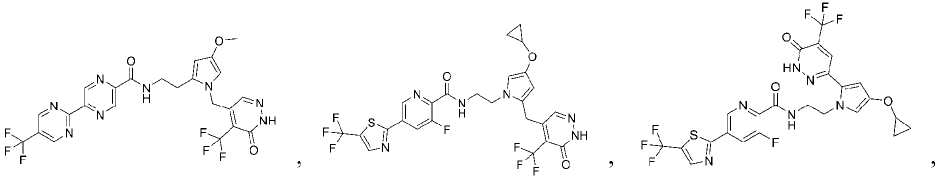

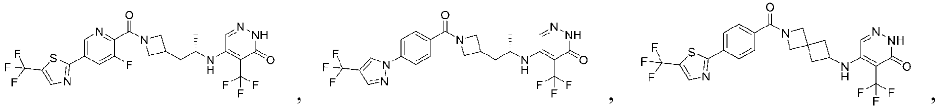

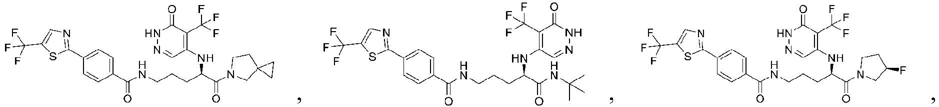

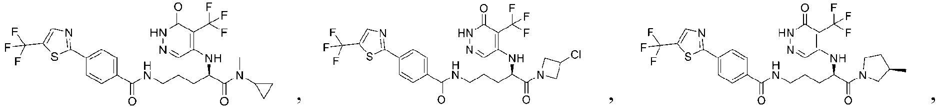

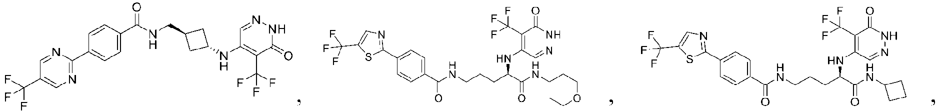

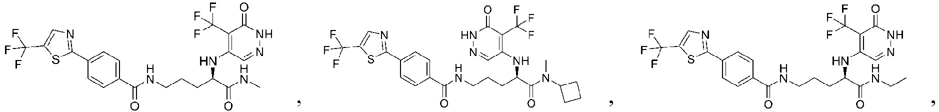

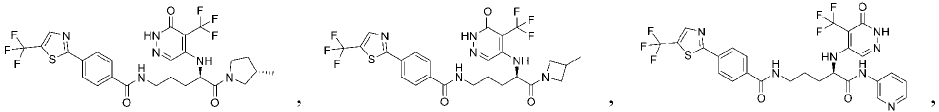

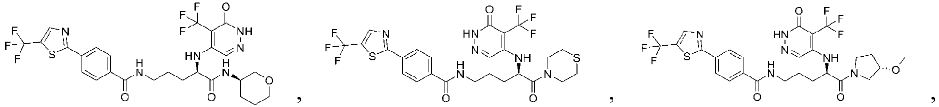

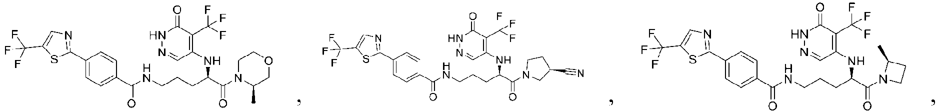

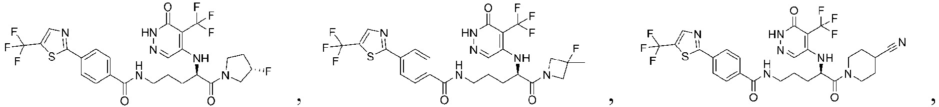

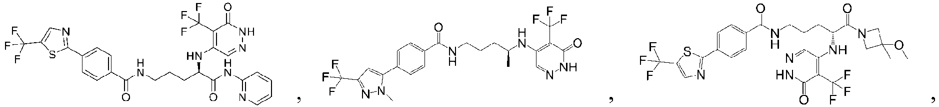

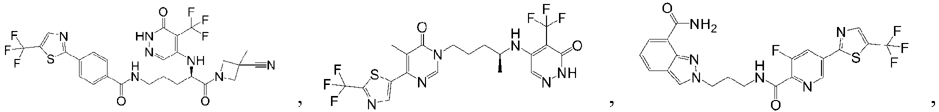

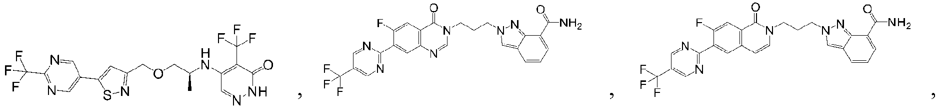

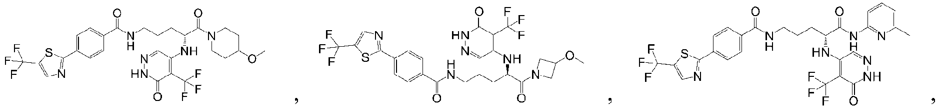

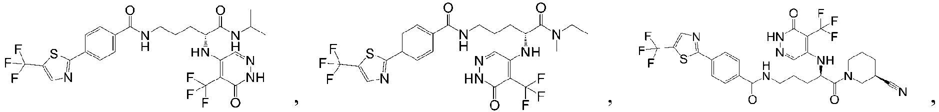

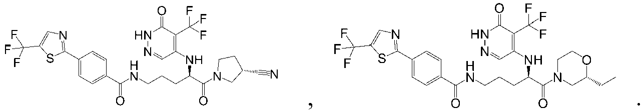

35. A pharmaceutical composition, comprising the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 34, and a pharmaceutically acceptable carrier.

36. Use of the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 34, or the pharmaceutical composition of claim 35 in the preparation of a medicament for inhibiting PARP7 in a subject in need thereof, or for treating or preventing a PARP7-mediated disease; preferably, the PARP7-mediated disease is selected from cancer, immune diseases, inflammation, or viral infections.

37. A method for treating or preventing a PARP7-mediated disease, comprising administering to a subject in need thereof a therapeutically effective amount of the compound, or the enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof of anyone of claims 1 to 34, or the pharmaceutical composition of claim 35, wherein the PARP7-mediated disease is selected from cancer, immune diseases, inflammation, or viral infections.
